# EUROPÄISCHE PATENTANMELDUNG

(11) **EP 2 784 073 A1**
(43) Veröffentlichungstag der Anmeldung: **01.10.2014**
(21) Anmeldenummer: 14172043.3
(22) Anmeldetag: 04.10.2010
(51) Int. Cl.: C07D 401/04, C07D 401/14, C07D 403/04, C07D 403/14, C07D 471/04, C07D 487/04, A01N 43/56

(54) **3-PHENYL-4-PYRI(MI)DINYL-1H-PYRAZOLE UND IHRE VERWENDUNG ALS FUNGIZIDE**

(30) Priorität: 09.10.2009 EP 09172677; 09.10.2009 US 250149 P
(62) Teilanmeldung aus: 10767974.8
(71) Anmelder: Bayer CropScience AG, 40789 Monheim am Rhein (DE)
(72) Erfinder: Die Erfindernennung liegt noch nicht vor
(74) Vertreter: BIP Patents

(57) **Zusammenfassung**

Phenylpyri(mi)dinylazole der Formel **[I-a]** und **[I-b],** 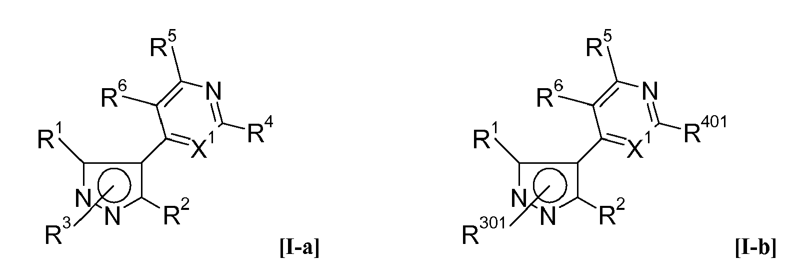 in welcher die Symbole die in der Beschreibung angegebenen Bedeutungen haben, sowie agrochemisch wirksame Salze davon und deren Verwendung zur Bekämpfung unerwünschter Mikroorganismen im Pflanzenschutz und Materialschutz sowie zur Reduktion von Mykotoxinen in Pflanzen und Pflanzenteilen sowie Verfahren zur Herstellung von Verbindungen der Formel **[I-a]** und **[I-b].**

## Beschreibung

Die vorliegende Erfindung betrifft neue Phenylpyri(mi)dinylazole, mehrere Verfahren zu deren Herstellung und deren Verwendung zur Bekämpfung unerwünschter Mikroorganismen im Pflanzenschutz und Materialschutz sowie zur Reduktion von Mykotoxinen in Pflanzen und Pflanzenteilen. Die vorliegende Erfindung betrifft weiterhin ein Verfahren zur Bekämpfung phytopathogener Pilze sowie zur Reduktion von Mykotoxinen in Pflanzen und Pflanzenteilen im Pflanzenschutz sowie Pflanzenschutzmittel enthaltend Phenylpyri(mi)dinylazole.

Es ist bereits bekannt, dass bestimmte Arylpyrazole fungizide Eigenschaften besitzen (vgl. z.B. WO 03/049542, WO 01/030154 und Pharmazie 1999, 54(2),106-11). Die Wirksamkeit der dort beschriebenen Stoffe ist gut, lässt aber in manchen Fällen zu wünschen übrig.

In WO 98/052937 werden bestimmte Heteroaryl-substituierte Pyrazole beschrieben, die medizinisch genutzt werden können, hier zur Inhibierung der Produktion inflammatorischer Cytokine und zur Behandlung humaner p38 Kinase vermittelter Krankheiten. Ähnliche Verbindungen sind auch beschrieben in EP-A-1 553 096, WO 04/029043, WO 98/052940, WO 00/031063, WO 95/031451, WO 02/057265 und WO 00/039116. Eine Wirkung auf pilzliche Pathogene wird jedoch nicht beschrieben.

In WO 07/105058 werden bestimmte Heteroaryl-substituierte Pyrazole beschrieben, die als Modulatoren oder Inhibitoren des humanen Raf Enzyms verwendet werden können. Die Wirkung auf pilzliche Pathogene wird jedoch nicht beschrieben.

Da sich die ökologischen und ökonomischen Anforderungen an moderne Pflanzenschutzmittel laufend erhöhen, beispielsweise was Wirkspektrum, Toxizität, Selektivität, Aufwandmenge, Rückstandsbildung und günstige Herstellbarkeit angeht, und außerdem z.B. Probleme mit Resistenzen auftreten können, besteht die ständige Aufgabe, neue Pflanzenschutzmittel, insbesondere Fungizide zu entwickeln, die zumindest in Teilbereichen Vorteile gegenüber den bekannten aufweisen.

Überraschenderweise wurde nun gefunden, dass die vorliegenden Phenylpyri(mi)dinylazole die genannten Aufgaben zumindest in Teilaspekten lösen und sich als Pflanzenschutzmittel, insbesondere als Fungizide eignen.

Gegenstand der Erfindung sind Verbindungen der Formel [I-a], 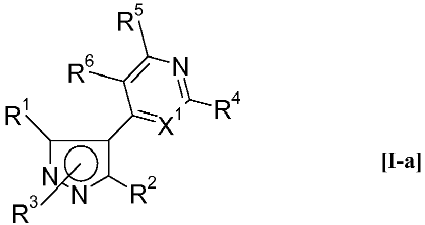 in welcher die Symbole folgende Bedeutungen haben:
- X¹: steht für C-H oder N,
- R¹: steht für jeweils gegebenenfalls einfach oder mehrfach gleich oder verschieden durch R⁷ substituiertes Phenyl, Naphthalenyl, Chinolin-5-yl, Chinolin-8-yl, Isochinolin-5-yl, Isochinolin-8-yl, 1-Benzothiophen-4-yl, 1-Benzothiophen-7-yl, 1-Benzofuran-4-yl, 1-Benzofuran-7-yl, 1,3-Benzodioxol-4-yl oder 1,3-Benzodioxol-5-yl,
- R²: steht für Cyano, Nitro, Halogen, C₁-C₆-Alkyl, C₁-C₆-Alkoxy, C₁-C₆-Halogenalkyl, C₁-C₆-Halogenalkoxy, C₁-C₆-Alkylthio, C₃-C₆-Cycloalkyl, C₃-C₆-Halogencycloalkyl, C₂-C₉-Heterocyclyl oder Wasserstoff,
- R³: steht für jeweils gegebenenfalls einfach oder mehrfach gleich oder verschieden durch Halogen, Cyano, Hydroxy, C₁-C₆-Alkyl, C₁-C₆-Alkoxy, Halogenalkoxy (vorzugsweise C₁-C₆-Halogenalkoxy), Phenyl, Phenoxy substituiertes C₁-C₆-Alkyl, C₂-C₆-Alkenyl, C₂-C₆-Alkinyl, C₃-C₆-Cycloalkyl, C₃-C₆-Cycloalkyl-C₁-C₆-alkyl, C₃-C₆-Cycloalkyl-oxy, C₁-C₆-Alkoxy, C₁-C₆-Alkoxy-C₁-C₆-alkyl, Acyloxy-C₁-C₆-alkyl, Heteroaryl-C₁-C₆-alkyl (vorzugsweise C₂-C₉-Heteroaryl-C₁-C₆-alkyl), Aryl-C₁-C₆-alkyl (vorzugsweise C₆-C₁₄-Aryl-C₁-C₆-alkyl), C₁-C₆-Alkylthio-C₁-C₆-alkyl, C₃-C₆-Cycloalkyl-C(O)-C₁-C₄-alkyl, C₂-C₉-Heterocyclyl-C(O)-C₁-C₄-alkyl, C₁-C₄-Alkyl-C(O)-C₃-C₆-cycloalkyl, C₁-C₄-Alkyl-C(O)-heterocyclyl (vorzugsweise C₁-C₄-Alkyl-C(O)-C₂-C₉-heterocyclyl), C₁-C₄-Alkyl-C(O)O-C₁-C₆-alkyl, Acyloxy-C₃-C₆-cycloalkyl, Acyloxy-heterocyclyl, Heterocyclyl-C₁-C₆-alkyl (vorzugsweise C₂-C₉-Heterocyclyl-C₁-C₆-alkyl), Heterocyclyl (vorzugsweise C₂-C₉-Heterocyclyl), C₂-C₉-OxoHeterocycyl oder Heteroaryl (vorzugsweise C₂-C₉-Heteroaryl),
- R⁴: steht für Wasserstoff, Halogen, Cyano, -C(O)OR¹², -SR¹², -NR¹²R¹³, -C(O)NR¹²R¹³ oder - NR¹²R¹⁴, -N=C=NR²², -N=C(H)OR²², -N=C(OR²²)R²³, -N=C(SR²²)R²³, -C(=NR²²)NR²²R²³, -SO(=NR²²)R²³, -SO₂R²⁰,
oder für jeweils gegebenenfalls einfach oder mehrfach gleich oder verschieden durch R¹¹ substituiertes C₁-C₆-Alkyl, C₃-C₈-Cycloalkyl, C₂-C₆-Alkenyl, C₆-C₁₄-Aryl, C₂-C₉-Heterocyclyl, C₂-C₉-Heteroaryl,
wobei R⁴ bevorzugt für Wasserstoff oder -NHR¹³ steht, in dem R¹³ für jeweils gegebenenfalls einfach oder mehrfach gleich oder verschieden durch Fluor, Chlor, Brom,-OH, Cyano, C₁-C₆-Alkyl, -O-C(O)R¹¹ , -O-P(O)(OR¹¹)₂, -O-B(OR¹¹)₂ oder -O-(C₁-C₄-Alkyl) substituiertes C₁-C₆-Alkyl, C₃-C₆-Cycloalkyl, C₂-C₆-Alkenyl, C₂-C₆-Alkinyl, C₃-C₈-Cycloalkyl, C₆-C₁₄-Aryl, C₂-C₉-Heterocyclyl oder C₂-C₉-Heteroaryl steht,
- R⁵ und R⁶: stehen unabhängig voneinander für Wasserstoff, Fluor, Chlor, Brom, Cyano, Nitro, -OH, -SH,
oder für jeweils gegebenenfalls einfach oder mehrfach gleich oder verschieden durch R¹¹ substituiertes C₁-C₆-Alkyl, C₃-C₆-Cycloalkyl, C₂-C₆-Alkenyl, C₂-C₆-Alkinyl, C₆-C₁₄-Aryl,-O-(C₁-C₄-Alkyl), -O-(C₆-C₁₄-Aryl), -S-(C₁-C₄-Alkyl), -S(O)-(C₁-C₆-Alkyl) oder -C(O)-(C₁-C₆-Alkyl),
oder bilden außerdem gemeinsam mit dem Kohlenstoffatom, an das sie gebunden sind, einen gegebenenfalls einfach oder mehrfach, gleich oder verschieden durch Halogen, Sauerstoff, Cyano oder C₁-C₄-Alkyl substituierten Cyclus (vorzugsweise einen gesättigten, ungesättigten oder partiell ungesättigten Monocyclus) mit 3, vorzugsweise 5 bis 8 Ringatomen, wobei der Cyclus 1 bis 4 Heteroatome aus der Reihe Sauerstoff, Schwefel oder -NR¹⁹ enthalten kann,
- R⁷: steht unabhängig voneinander für eine oder mehrere der folgenden Gruppen: Fluor, Chlor, Brom, Cyano, Nitro, -OH, -SH,
oder für jeweils gegebenenfalls einfach oder mehrfach gleich oder verschieden durch Fluor, Chlor, Brom, -OH, Cyano, C₁-C₆-Alkyl oder -O-(C₁-C₄-Alkyl) substituiertes C₁-C₆-Alkyl, C₃-C₆-Cycloalkyl, C₂-C₆-Alkenyl, C₂-C₆-Alkinyl, Tri(C₁-C₄-alkyl)silyl, C₆-C₁₄-Aryl, -O-(C₁-C₄-Alkyl), -O-(C₆-C₁₄-Aryl), -S-(C₁-C₄-Alkyl), -S(O)-(C₁-C₆-Alkyl), oder -S(O)₂-(C₁-C₆-Alkyl),
- R¹¹: steht für -OH, Fluor, Chlor, Brom, Cyano, -NH-C(O)R²⁰, -NR²⁰R²¹, -C(O)R²⁰, -C(O)OR²⁰, -C(O)NR²⁰R²¹, -SO₂R²⁰,
oder für jeweils gegebenenfalls einfach oder mehrfach gleich oder verschieden durch Fluor, Chlor, Brom, -OH, Carbonyl, Cyano, C₁-C₆-Alkyl oder -O-(C₁-C₄-Alkyl) substituiertes C₁-C₆-Alkyl, C₂-C₆-Alkenyl, C₂-C₆-Alkinyl, C₁-C₁₁-Heteroalkyl, C₃-C₈-Cycloalkyl, -O-(C₁-C₄-Alkyl), -S-(C₁-C₄-Alkyl), -O-(C₃-C₈-Cycloalkyl), -S-(C₃-C₈-Cycloalkyl), C₆-C₁₄-Aryl, -O-(C₆-C₁₄-Aryl), -S-(C₆-C₁₄-Aryl), C₂-C₉-Heterocyclyl oder C₂-C₉-Heteroaryl,
- R¹² und R¹³: stehen unabhängig voneinander für eine oder mehrere der folgenden Gruppen: H, -C(S)R¹⁵, -C(O)R¹⁵, -SO₂R¹⁵, -C(O)OR¹⁵, -OR¹⁵ oder -C(O)NR¹⁵R¹⁶
oder für jeweils gegebenenfalls einfach oder mehrfach gleich oder verschieden durch Fluor, Chlor, Brom, -OH, Cyano, C₁-C₆-Alkyl, -O-C(O)R¹¹, -O-P(O)(OR¹¹)₂, -O-B(OR¹¹)₂ oder - O-(C₁-C₄-Alkyl) substituiertes C₁-C₆-Alkyl, C₃-C₆-Cycloalkyl, C₂-C₆-Alkenyl, C₂-C₆-Alkinyl, C₃-C₈-Cycloalkyl, C₆-C₁₄-Aryl, C₂-C₉-Heterocyclyl oder C₂-C₉-Heteroaryl,
- R¹⁴: steht für -CH₂-NR²²R²³, Piperidin-1-ylmethyl oder Morpholin-4-ylmethyl
oder für jeweils gegebenenfalls einfach oder mehrfach gleich oder verschieden durch Fluor, Chlor, Brom, -OH, Cyano substituiertes C₁-C₆-Alkyl oder -O-(C₁-C₄-Alkyl),
- R¹⁵ und R¹⁶: stehen unabhängig voneinander für Wasserstoff oder -OH
oder für jeweils gegebenenfalls einfach oder mehrfach gleich oder verschieden durch R¹¹ substituiertes C₁-C₆-Alkyl, C₃-C₆-Cycloalkyl, C₂-C₆-Alkenyl, C₂-C₆-Alkinyl, C₃-C₈-Cycloalkyl, C₆-C₁₄-Aryl, C₂-C₉-Heterocyclyl oder C₂-C₉-Heteroaryl
oder bilden (wenn R¹² und/oder R¹³ für -C(O)NR¹⁵R¹⁶ steht) gemeinsam mit dem Stickstoffatom an das sie gebunden sind einen 3 bis 7-gliedrigen Ring, der ein weiteres dem Stickstoff nicht (direkt) benachbartes Heteroatom aus der Reihe N, O enthalten kann,
- R¹⁷ und R¹⁸: stehen unabhängig voneinander für eine oder mehrere der folgenden Gruppen: H, - C(O)OR¹¹
oder für jeweils gegebenenfalls einfach oder mehrfach gleich oder verschieden durch Fluor, Chlor, Brom, -OH, Cyano, C₁-C₆-Alkyl, und -O-(C₁-C₄-Alkyl) substituiertes C₁-C₆-Alkyl, C₃-C₆-Cycloalkyl, C₂-C₆-Alkenyl, C₂-C₆-Alkinyl, C₃-C₈-Cycloalkyl, C₆-C₁₄-Aryl, C₂-C₉-Heterocyclyl oder C₂-C₉-Heteroaryl,
- R¹⁹: steht für H, C₁-C₆-Alkyl, C₃-C₆-Cycloalkyl, C₂-C₆-Alkenyl, C₂-C₆-Alkinyl, -C(S)R¹⁵,-C(O)R¹⁵, -SO₂R¹⁵, -C(O)OR¹⁵,
- R²⁰ und R²¹: stehen unabhängig voneinander für jeweils gegebenenfalls einfach oder mehrfach gleich oder verschieden durch Fluor, Chlor, Brom, -OH, Cyano substituiertes C₁-C₆-Alkyl, C₃-C₆-Cycloalkyl, C₂-C₆-Alkenyl, C₂-C₆-Alkinyl oder Wasserstoff,
- R²² und R²³: stehen unabhängig voneinander für C₁-C₆-Alkyl, C₃-C₆-Cycloalkyl, C₂-C₆-Alkenyl, C₂-C₆-Alkinyl oder Wasserstoff,
sowie agrochemisch wirksame Salze davon.

Ausgenommen von den oben aufgeführten allgemeinen oder in Vorzugsbereichen aufgeführten Restedefinitionen bzw. Erläuterungen sind Kombinationen in denen die Symbole der Formel [I-a] die folgenden Bedeutungen haben:
Verbindungen in denen,
   - X¹: für N steht, und
   - R¹: für ein gegebenenfalls substituiertes Phenyl steht,
   - R³: für Butyl oder Propyn-2-yl steht,
   - R⁴: für NHR₁₂ steht und
   - R¹²: für gegebenenfalls substituiertes C₆-C₁₄-Aryl steht,
   sowie
Verbindungen in denen,
   - X¹: für N steht
   - R², R⁴, R⁵, R⁶: für H stehen,
   - R³: für Methyl, Ethyl, Allyl, 2-Methoxyethyl oder Benzyl steht, wenn R¹ für 4-Chlorphenyl steht, oder
   - R³: für Methyl steht, wenn R¹ für Phenyl, 4-Methoxyphenyl oder 4-Fluorphenyl steht.

Schließlich wurde gefunden, dass die erfindungsgemäßen Phenylpyri(mi)dinylazole der Formel **[I-a]** sehr gute mikrobizide Eigenschaften besitzen und zur Bekämpfung unerwünschter Mikroorganismen im Pflanzenschutz und Materialschutz sowie zur Reduktion von Mykotoxinen in Pflanzen und Pflanzenteilen verwendbar sind.

Die erfindungsgemäßen Phenylpyri(mi)dinylazole sind durch die Formel **[I-a]** allgemein definiert. Bevorzugte Restedefinitionen der vorstehenden und nachfolgend genannten Formeln sind im Folgenden angegeben. Diese Definitionen gelten für die Endprodukte der Formel **[I-a]** wie für alle Zwischenprodukte gleichermaßen.

**Bevorzugte** Verbindungen der Formel **[I-a]** der vorliegenden Erfindung sind solche, in denen ein oder mehrere der Symbole eine der folgenden Bedeutungen haben:
- X¹: steht für C-H oder N,
- R¹: steht für jeweils gegebenenfalls einfach oder mehrfach gleich oder verschieden durch R⁷ substituiertes Phenyl, Naphthalenyl, Chinolin-5-yl, Chinolin-8-yl, Isochinolin-5-yl, Isochinolin-8-yl, 1,3-Benzodioxol-4-yl oder 1,3-Benzodioxol-5-yl,
- R²: steht für Cyano, Nitro, Halogen, C₁-C₆-Alkyl, C₁-C₆-Alkoxy, C₁-C₆-Halogenalkyl, C₁-C₆-Halogenalkoxy, C₁-C₆-Alkylthio, C₃-C₆-Cycloalkyl, C₃-C₆-Halogencycloalkyl, C₂-C₉-Heterocyclyl oder Wasserstoff,
- R³: steht für jeweils gegebenenfalls einfach oder mehrfach gleich oder verschieden durch Halogen, Cyano, Hydroxy, Halogenalkoxy substituiertes C₁-C₆-Alkyl, C₂-C₆-Alkenyl, C₂-C₆-Alkinyl, C₃-C₆-Cycloalkyl, C₃-C₆-Cycloalkyl-C₁-C₆-alkyl, C₆-C₁₄-Aryl-C₁-C₆-alkyl, C₁-C₆-Alkoxy-C₁-C₆-alkyl, C₁-C₆-Alkoxy, C₂-C₉-Heterocyclyl-C₁-C₆-alkyl und C₂-C₉-Heteroaryl oder für Pyridin-2-ylmethyl, Pyridin-3-ylmethyl, Pyridin-4-ylmethyl, 2-Chlor-1,3-thiazol-5-yl)methyl, 5-(Trifluormethyl)-1,3,4-thiadiazol-2-yl, 2-(Methylsulfanyl)ethyl, 2-Cyclohexyl-2-oxoethyl, 2-Cyclopentyl-2-oxoethyl, 1-Acetylpiperidin-4-yl, Tetrahydrofuran-3-yl, 2-Oxotetrahydrofuran-3-yl, 2-Acetoxyethyl, 2-tert-Butoxy-2-oxoethyl, 1-Methoxy-3-methyl-1-oxobutan-2-yl, 1-Methoxy-1-oxopropan-2-yl, 2-[2-(2-Methoxyethoxy)ethoxy]ethyl, 2-(2-Methoxyethoxy)ethyl, Biphenyl-4-ylmethyl, Biphenyl-3-ylmethyl, Biphenyl-2-ylmethyl, 3-Phenoxybenzyl, 4-Fluor-3-phenoxybenzyl, Cyclopentyloxy, 2-(1,5-Dimethyl-1H-pyrazol-3-yl)-2-oxoethyl oder 3-Ethoxy-3-oxopropyl,
- R⁴: steht für Wasserstoff, -NR¹²R¹³, -C(O)NR¹²R¹³,
wobei R⁴ bevorzugt für Wasserstoff oder NHR¹³ steht, wobei dann R¹³ für jeweils gegebenenfalls einfach oder mehrfach gleich oder verschieden durch Fluor, Chlor, Brom,-OH oder Cyano substituiertes C₁-C₆-Alkyl, C₃-C₆-Cycloalkyl, C₂-C₆-Alkenyl, C₂-C₆-Alkinyl, C₃-C₈-Cycloalkyl, C₂-C₉-Heterocyclyl oder C₂-C₉-Heteroaryl steht,
- R⁵ und R⁶: stehen unabhängig voneinander für Wasserstoff, Fluor, Chlor, Brom, Cyano, Nitro, -OH, - SH,
oder für jeweils gegebenenfalls einfach oder mehrfach gleich oder verschieden durch R¹¹ substituiertes C₁-C₆-Alkyl, C₃-C₆-Cycloalkyl, C₂-C₆-Alkenyl, C₂-C₆-Alkinyl, -O-(C₁-C₄-Alkyl), -S-(C₁-C₄-Alkyl) oder -S(O)-(C₁-C₆-Alkyl),
oder bilden außerdem gemeinsam mit dem Kohlenstoffatomen, an das sie gebunden sind, einen gegebenenfalls einfach oder mehrfach, gleich oder verschieden durch Halogen, Sauerstoff, Cyano oder C₁-C₄-Alkyl substituierten, gesättigten, ungesättigten oder partiell ungesättigten Monocyclus mit 3 bis 8 Ringatomen, wobei der Monocyclus Heteroatome aus der Reihe Sauerstoff, Schwefel oder -N-R¹⁹ enthalten kann,
- R⁷: steht unabhängig voneinander für eine oder mehrere der folgenden Gruppen: Fluor, Chlor, Brom, Cyano, Nitro, Methyl, Ethyl, Isopropyl, -CF₃, -CHF₂, -C₂F₅, -CCl₃, -OCH₃, -OC₂H₅, -O-CH(CH₃)₂, -OCF₃, -OCHF₂, -OC₂F₅, -SCH₃, -SO₂CH₃ oder -SCF₃,
- R¹¹: steht für -OH, Fluor, Chlor, Brom, Cyano, -NH-C(O)R²⁰, -C(O)R²⁰, -C(O)OR²⁰,-C(O)NR²⁰R²¹, -SO₂R²⁰
oder für jeweils gegebenenfalls einfach oder mehrfach gleich oder verschieden durch Fluor, Chlor, Brom, -OH, Cyano, C₁-C₆-Alkyl oder O-(C₁-C₄-Alkyl) substituiertes C₁-C₆-Alkyl, C₂-C₆-Alkenyl, C₂-C₆-Alkinyl, C₁-C₁₁-Heteroalkyl, C₃-C₈-Cycloalkyl, -O-(C₁-C₄-Alkyl),-S-(C₁-C₄-Alkyl), -O-(C₃-C₈-Cycloalkyl), -S-(C₃-C₈-Cycloalkyl), C₂-C₉-Heterocyclyl oder C₂-C₉-Heteroaryl,
- R¹² und R¹³: stehen unabhängig voneinander für eine oder mehrere der folgenden Gruppen: H, -C(S)R¹⁵, -C(O)R¹⁵, -SO₂R¹⁵, -C(O)OR¹⁵, -OR¹⁵, -C(O)NR¹⁵R¹⁶
oder für jeweils gegebenenfalls einfach oder mehrfach gleich oder verschieden durch Fluor, Chlor, Brom, -OH oder Cyano substituiertes C₁-C₆-Alkyl, C₃-C₆-Cycloalkyl, C₂-C₆-Alkenyl, C₂-C₆-Alkinyl, C₃-C₈-Cycloalkyl, C₂-C₉-Heterocyclyl oder C₂-C₉-Heteroaryl,
- R¹⁵ und R¹⁶: stehen unabhängig voneinander für H oder -OH
oder für jeweils gegebenenfalls einfach oder mehrfach gleich oder verschieden durch R¹¹ substituiertes C₁-C₆-Alkyl, C₃-C₆-Cycloalkyl, C₂-C₆-Alkenyl, C₂-C₆-Alkinyl, C₃-C₈-Cycloalkyl, C₆-C₁₄-Aryl, C₂-C₉-Heterocyclyl oder C₂-C₉-Heteroaryl
oder bilden, wenn R¹² und/oder R¹³ für -C(O)NR¹⁵R¹⁶ steht, gemeinsam mit dem Stickstoffatom an das sie gebunden sind einen 3 bis 7-gliedrigen Ring, der ein weiteres dem Stickstoff nicht direkt benachbartes Heteroatom aus der Reihe N, O enthalten kann,
- R¹⁹: steht für H, C₁-C₆-Alkyl, C₃-C₆-Cycloalkyl, C₂-C₆-Alkenyl, C₂-C₆-Alkinyl, -C(S)R¹⁵,-C(O)R¹⁵, -SO₂R¹⁵ oder -C(O)OR¹⁵,
- R²⁰ und R²¹: stehen unabhängig voneinander für jeweils gegebenenfalls einfach oder mehrfach gleich oder verschieden durch Fluor, Chlor, Brom, -OH oder Cyano substituiertes C₁-C₆-Alkyl, C₃-C₆-Cycloalkyl, C₂-C₆-Alkenyl, C₂-C₆-Alkinyl oder für Wasserstoff,
- R²² und R²³: stehen unabhängig voneinander für C₁-C₆-Alkyl, C₃-C₆-Cycloalkyl, C₂-C₆-Alkenyl, C₂-C₆-Alkinyl oder Wasserstoff,
sowie agrochemisch wirksame Salze davon.

In einer ersten Ausführungsform der vorliegenden Erfindung sind Verbindungen der Formel **[I-a] besonders bevorzugt,** in denen ein oder mehrere der Symbole eine der folgenden Bedeutungen haben:
- X¹: steht für C-H,
- R¹: steht für jeweils gegebenenfalls einfach oder mehrfach gleich oder verschieden durch R⁷ substituiertes Phenyl oder Naphthalenyl,
- R²: steht für Halogen, C₁-C₆-Alkyl, C₃-C₆-Cycloalkyl oder Wasserstoff,
- R³: steht für Propan-2-yl, Isobutyl, Butan-2-yl, 2-Methylpropyl, 2,2-Dimethylpropyl, 3-Methylbut-2-en-1-yl, But-2-en-1-yl, But-3-en-2-yl, Propadienyl, 4-Methylpent-3-en-2-yl, Prop-2-in-1-yl, But-2-in-1-yl, But-3-in-2-yl, 2-Methylbut-3-in-2-yl, 2-Methylbut-3-in-2-yl, Cyanmethyl, 2-Cyanethyl, 1-Cyanpropan-2-yl, Cyclopropyl, Cyclobutyl, Cyclopentyl, Cyclopentyloxy, Cyclohexyl, (2,2-Dichlorcyclopropyl)methyl, Cyclopropylmethyl, 1-Cyclopropylethyl, Trichlormethyl, Trifluormethyl, 2-Fluorethyl, 2,2-Difluorethyl, 2,2,2-Trifluorethyl, 2,2,2-Trichlorethyl, 2-Chlorethyl, 2-Bromethyl, 2-Fluorpropyl, 3-Fluorpropyl, 2-Chlorpropyl, 3-Chlorpropyl, 1,1,1-Trifluorpropan-2-yl, 1,1-Difluorpropan-2-yl, 1,1,1-Trifluor-2-methylpropan-2-yl, 1,3-Difluorpropan-2-yl, 3,3,3-Trifluor-2-hydroxypropyl, Pyridin-2-ylmethyl, Pyridin-3-ylmethyl, Pyridin-4-ylmethyl, 2-Chlor-1,3-thiazol-5-ylmethyl, Benzyl, 2-Fluorbenzyl, 3-Fluorbenzyl, 4-Fluorbenzyl, 1-(2-Chlorphenyl)ethyl, 2,3-Difluorbenzyl, 2-Chlor-6-fluorbenzyl, 1-(3-Chlorphenyl)ethyl, 1-(4-Chlorphenyl)ethyl, 2-Cyanobenzyl, 3-Cyanobenzyl, 4-Cyanobenzyl, 4-(Difluormethoxy)benzyl, Biphenyl-3-ylmethyl, Biphenyl-4-ylmethyl, Biphenyl-2-ylmethyl, 3-Phenoxybenzyl, 4-Fluoro-3-phenoxybenzyl, 2-(3-Chlorphenyl)ethyl, 2-(2-Chlorphenyl)ethyl, 1-Naphthylmethyl, Methoxymethyl, 2-Methoxyethyl, 2-Methoxypropyl, 2-(Methylsulfanyl)ethyl, 2-(Trifluormethoxy)ethyl, 1-Methoxypropan-2-yl, 2-[2-(2-Methoxyethoxy)ethoxy]ethyl, 2-(2-Methoxyethoxy)ethyl, 1,3-Dimethoxypropan-2-yl, 2-(Cyclopropyloxy)ethyl, Propoxy, (2-Methylprop-2-en-1-yl)oxy, (3-Methylbutanoyl)oxy, 1-Cyanethoxy, 2-Chlorethoxy, But-2-in-1-yloxy, Cyanmethoxy, Prop-2-in-1-yloxy, 2-Cyclohexyl-2-oxoethyl, 2-Cyclopentyl-2-oxoethyl, Tetrahydrofuran-2-ylmethyl, (3-Methyloxetan-3-yl)methyl, 1H-Imidazol-2-ylmethyl, 2-(1,5-Dimethyl-1H-pyrazol-3-yl)-2-oxoethyl, 1-Acetylpiperidin-4-yl, Tetrahydrofuran-3-yl, 2-Oxotetrahydrofuran-3-yl, 5-(Trifluormethyl)pyridin-2-yl, 5-(Trifluormethyl)-1,3,4-thiadiazol-2-yl, 6-(Trifluormethyl)pyrimidin-4-yl, 2-Hydroxypropyl, 2-Hydroxy-2-methylpropyl, 2,3-Dihydroxypropyl, 2-Acetoxyethyl, Cyano, 2-tert-Butoxy-2-oxoethyl, 1-Methoxy-3-methyl-1-oxobutan-2-yl, 1-Methoxy-1-oxopropan-2-yl oder 3-Ethoxy-3-oxopropyl, , Propan-2-yloxy, Methyl, Ethyl, 2-Ethoxyethyl, oder 2-Chlorethyl,
- R⁴: steht für Wasserstoff, -C(O)NR¹²R¹³ oder -NR¹²R¹³,
wobei R⁴ bevorzugt für Wasserstoff steht,
- R⁵ und R⁶: stehen unabhängig voneinander für Wasserstoff, Fluor, Chlor, Brom, Cyano, Nitro,
oder für jeweils gegebenenfalls einfach oder mehrfach gleich oder verschieden durch R¹¹ substituiertes C₁-C₆-Alkyl, C₃-C₆-Cycloalkyl, C₂-C₆-Alkenyl, C₂-C₆-Alkinyl, -O-(C₁-C₄-Alkyl), -S-(C₁-C₄-Alkyl) oder -S(O)-(C₁-C₆-Alkyl),
oder bilden gemeinsam mit dem Kohlenstoffatomen, an das sie gebunden sind, einen Monocyclus, wobei sie gemeinsam für gegebenenfalls einfach oder mehrfach gleich oder verschieden durch R¹¹ substituiertes -(CH=CH-CH=CH)-, -(CH=CH-N(R¹⁹), -(CH=CH-CH=N)-, -(NH-CH=N)- oder -(CH₂-C(O)-N(R¹⁹)- stehen,
- R⁷: steht unabhängig voneinander für eine oder mehrere der folgenden Gruppen: Fluor, Chlor, Cyano, Nitro, Methyl, Ethyl, Isopropyl, -CF₃, -CHF₂, -C₂F₅, -CCl₃,
- R¹¹: steht für -OH, Fluor, Chlor, Brom, Cyano, -NHC(O)R²⁰, -C(O)R²⁰, -C(O)OR²⁰,-C(O)NR²⁰R²¹, -SO₂R²⁰
oder für jeweils gegebenenfalls einfach oder mehrfach gleich oder verschieden durch Fluor, Chlor, Brom, -OH, Cyano, C₁-C₆-Alkyl oder -O-(C₁-C₄-Alkyl) substituiertes C₁-C₆-Alkyl, C₃-C₈-Cycloalkyl, C₂-C₆-Alkenyl, C₂-C₆-Alkinyl, -O-(C₁-C₄-Alkyl), -S-(C₁-C₄-Alkyl), -O-(C₃-C₈-Cycloalkyl) oder -S-(C₃-C₈-Cycloalkyl),
- R¹² und R¹³: stehen unabhängig voneinander für eine oder mehrere der folgenden Gruppen: H, -C(S)R¹⁵, -C(O)R¹⁵, -SO₂R¹⁵, -C(O)OR¹⁵, -OR¹⁵ oder -C(O)NR¹⁵R¹⁶
oder für jeweils gegebenenfalls einfach oder mehrfach gleich oder verschieden durch Fluor, Chlor, Brom, -OH, Cyano substituiertes C₁-C₆-Alkyl, C₃-C₆-Cycloalkyl, C₂-C₆-Alkenyl, C₂-C₆-Alkinyl, C₂-C₉-Heterocyclyl oder C₂-C₉-Heteroaryl,
- R¹⁵ und R¹⁶: stehen unabhängig voneinander für jeweils gegebenenfalls einfach oder mehrfach gleich oder verschieden durch R¹¹ substituiertes C₁-C₆-Alkyl, C₃-C₆-Cycloalkyl, C₂-C₆-Alkenyl, C₂-C₆-Alkinyl, C₃-C₈-Cycloalkyl, C₂-C₉-Heterocyclyl, C₂-C₉-Heteroaryl
oder für Wasserstoff
oder bilden gemeinsam mit dem Stickstoffatom an das sie gebunden sind einen 3 bis 7-gliedrigen Ring, der ein weiteres dem Stickstoff nicht benachbartes Heteroatom aus der Reihe N, O enthalten kann,
- R¹⁹: steht für H, C₂-C₆-Alkinyl, C(O)R¹⁵, SO₂R¹⁵, C(O)OR¹⁵,
- R²⁰ und R²¹: stehen unabhängig voneinander für jeweils gegebenenfalls einfach oder mehrfach gleich oder verschieden durch Fluor, Chlor, Brom, -OH, Cyano substituiertes C₁-C₆-Alkyl, C₃-C₆-Cycloalkyl, C₂-C₆-Alkenyl, C₂-C₆-Alkinyl
oder für Wasserstoff,
sowie agrochemisch wirksame Salze davon.

In dieser ersten Ausführungsform der vorliegenden Erfindung sind Verbindungen der Formel **[I-a] ganz besonders bevorzugt,** in denen ein oder mehrere der Symbole eine der folgenden Bedeutungen haben:
- X¹: steht für C-H
- R¹: steht für gegebenenfalls einfach oder mehrfach gleich oder verschieden durch R⁷ substituiertes Phenyl,
- R²: steht für Methyl, Ethyl, Isopropyl, Cyclopropyl oder Wasserstoff,
- R³: steht für Propan-2-yl, Isobutyl, Butan-2-yl, 2-Methylpropyl, 2,2-Dimethylpropyl, 3-Methylbut-2-en-1-yl, But-2-en-1-yl, But-3-en-2-yl, Propadienyl, Prop-2-in-1-yl, But-2-in-1-yl, But-3-in-2-yl, 2-Methylbut-3-in-2-yl, 2-Methylbut-3-in-2-yl, Cyanmethyl, 2-Cyanethyl, 1-Cyanpropan-2-yl, Cyclopropyl, Cyclobutyl, Cyclopentyl, Cyclohexyl, (2,2-Dichlorcyclopropyl)methyl, Cyclopropylmethyl, 1-Cyclopropylethyl, Trichlormethyl, Trifluormethyl, 2-Fluorethyl, 2,2-Difluorethyl, 2,2,2-Trifluorethyl, 2,2,2-Trichlorethyl, 2-Chlorethyl, 2-Bromethyl, 2-Fluorpropyl, 3-Fluorpropyl, 2-Chlorpropyl, 3-Chlorpropyl, 1,3-Difluorpropan-2-yl, 2-Fluorbenzyl, 3-Fluorbenzyl, 4-Fluorbenzyl, 2,3-Difluorbenzyl, 2-Chlor-6-fluorbenzyl, 1-(2-Chlorphenyl)ethyl, 1-(3-Chlorphenyl)ethyl, 1-(4-Chlorphenyl)ethyl, 3-Cyanobenzyl, 4-Cyanobenzyl, 4-(Difluormethoxy)benzyl, 2-Cyanobenzyl, 2-(3-Chlorphenyl)ethyl, 2-(2-Chlorphenyl)ethyl, 1-Naphthylmethyl,(Pyridin-3-ylmethyl, 2-Chlor-1,3-thiazol-5-yl)methyl, Methoxymethyl, 2-Methoxyethyl, 2-(Methylsulfanyl)ethyl, 2-(Trifluormethoxy)ethyl, 1-Methoxypropan-2-yl, 2-[2-(2-Methoxyethoxy)ethoxy]ethyl, 2-(2-Methoxyethoxy)ethyl, Tetrahydrofuran-2-ylmethyl, (3-Methyloxetan-3-yl)methyl, 1H-Imidazol-2-ylmethyl, Tetrahydrofuran-3-yl, 2-Oxotetrahydrofuran-3-yl, 2-tert-Butoxy-2-oxoethyl, 1-Methoxy-3-methyl-1-oxobutan-2-yl, 1-Methoxy-1-oxopropan-2-yl oder 3-Ethoxy-3-oxopropyl, Propan-2-yloxy, 2-Ethoxyethyl, Methyl, Ethyl, 1-Propyl oder 2-Chlorethyl,
in einer bevorzugten Variante ist R³ wie oben definiert umfasst jedoch nicht Methyl oder Ethyl.
- R⁴: steht für Wasserstoff, -NR¹²R¹³ oder -NHR¹³,
wobei R⁴ bevorzugt für Wasserstoff steht,
- R⁵ und R⁶: stehen unabhängig voneinander für Wasserstoff, Fluor oder Cyano
oder bilden gemeinsam mit dem Kohlenstoffatomen, an das sie gebunden sind, einen Cyclus, wobei sie gemeinsam für gegebenenfalls einfach oder mehrfach gleich oder verschieden durch R¹¹ substituiertes -(CH=CH-CH=CH)-, -(CH=CH-N(R¹⁹), -(CH=CH-CH=N)-, -(NH-CH=N)- oder -(CH₂-C(O)-N(R¹⁹)- stehen,
- R⁷: steht unabhängig voneinander für eine oder mehrere der folgenden Gruppen: Fluor, Chlor, Cyano oder Methyl,
- R¹¹: steht für -OH, Fluor, Chlor, Brom, Cyano, -NH-C(O)R²⁰, -C(O)R²⁰, -C(O)OR²⁰,-C(O)NR²⁰R²¹, -SO₂R²⁰
oder für jeweils gegebenenfalls einfach oder mehrfach gleich oder verschieden durch Fluor, Chlor, Brom, OH, Cyano, C₁-C₆-Alkyl oder -O-(C₁-C₄-Alkyl) substituiertes C₁-C₆-Alkyl, C₃-C₈-Cycloalkyl, C₂-C₆-Alkenyl, C₂-C₆-Alkinyl, -O-(C₁-C₄-Alkyl), -S-(C₁-C₄-Alkyl), -O-(C₃-C₈-Cycloalkyl) oder -S-(C₃-C₈-Cycloalkyl),
- R¹²: steht für -C(S)R¹⁵, -C(O)R¹⁵, -SO₂R¹⁵, -C(O)OR¹⁵, -OR¹⁵ oder -C(O)NR¹⁵R¹⁶,
- R¹³: steht für C₁-C₆-Alkyl, C₂-C₆-Alkenyl, C₂-C₆-Alkinyl, C₂-C₉-Heterocyclyl oder C₂-C₉-Heteroaryl oder Wasserstoff,
- R¹⁵: steht für jeweils gegebenenfalls einfach oder mehrfach gleich oder verschieden durch Halogen, -OH, Cyano oder C₁-C₄-alkyl substituiertes C₁-C₆-Alkyl, C₃-C₆-Cycloalkyl, C₂-C₆-Alkenyl, C₂-C₆-Alkinyl, C₃-C₈-Cycloalkyl, C₂-C₉-Heterocyclyl, C₂-C₉-Heteroaryl
oder für Wasserstoff
- R¹⁶: steht für Wasserstoff, Methyl, Ethyl oder Propyl,
- R¹⁹: steht für H, C₂-C₆-Alkinyl, -C(O)R¹⁵, -SO₂R¹⁵, -C(O)OR¹⁵,
- R²⁰ und R²¹: stehen unabhängig voneinander für jeweils gegebenenfalls einfach oder mehrfach gleich oder verschieden durch Fluor, Chlor, Brom, OH, Cyano substituiertes Methyl, Ethyl, Propyl, iso-Propyl, Cyclopropyl, Cyclobutyl
oder für Wasserstoff,
sowie agrochemisch wirksame Salze davon.

In dieser ersten Ausführungsform der vorliegenden Erfindung sind Verbindungen der Formel [I-a] **insbesondere bevorzugt,** in denen ein oder mehrere der Symbole eine der folgenden Bedeutungen haben:
- X¹: steht für C-H
- R¹: steht für Phenyl, 3-Methylphenyl, 2-Fluorphenyl, 3-Fluorphenyl, 4-Fluorphenyl, 3-Trifluormethylphenyl, 4-Chlorphenyl, 2,5-Difluorphenyl, 2,4-Difluorphenyl, 2,6-Difluorphenyl, 3-Methyl-4-fluorphenyl, 3-Cyan-4-fluorphenyl oder 2,4,6-Trifluorphenyl,
- R²: steht für Methyl, Ethyl, Isopropyl, Cyclopropyl, oder Wasserstoff,
- R³: steht für Propan-2-yl, Isobutyl, Butan-2-yl, 2-Methylpropyl, 2,2-Dimethylpropyl, Prop-2-in-1-yl, But-3-in-2-yl, Cyclopropyl, Cyclobutyl, Cyclopentyl, (2,2-Dichlorcyclopropyl)methyl, Cyclopropylmethyl, 1-Cyclopropylethyl, Trifluormethyl, Trichlormethyl, 2-Fluorethyl, 2,2-Difluorethyl, 2,2,2-Trifluorethyl, 2,2,2-Trichlorethyl, 2-Chlorethyl, 2-Fluorpropyl, 3-Fluorpropyl, 1,3-Difluorpropan-2-yl, 2-Fluorbenzyl, 2,3-Difluorbenzyl, 2-Chlor-6-fluorbenzyl, 1-(2-Chlorphenyl)ethyl, 1-(3-Chlorphenyl)ethyl, 2-(Trifluormethoxy)ethyl, oder 1-Methoxypropan-2-yl, 2,2-Difluorethyl, 2-Cyanethyl, Cyanmethyl, 1-Cyanpropan-2-yl, 1-Propyl, 2-Ethoxyethyl, 2-Chlorethyl oder 2-Methoxyethyl,
- R⁴: steht für Wasserstoff
- R⁵ und R⁶: stehen unabhängig voneinander für Wasserstoff, Fluor oder Cyano
oder bilden gemeinsam mit dem Kohlenstoffatomen, an das sie gebunden sind, einen Cyclus, wobei sie gemeinsam für gegebenenfalls einfach oder mehrfach gleich oder verschieden durch R¹¹ substituiertes -(CH=CH-CH=CH)-, -(CH=CH-N(R¹⁹)-, -(CH=CH-CH=N)-, -(NH-CH=N)- oder -(CH₂-C(O)-N(R¹⁹)- stehen,
- R¹¹: steht für -OH, Fluor, Chlor, Cyano, C₁-C₆-Alkyl, C₂-C₆-Alkenyl oder C₂-C₆-Alkinyl, C₃-C₆-Cycloalkyl,
oder für Wasserstoff,
- R¹⁹: steht für H, Acetyl, Ethoxycarbonyl, Methoxycarbonyl, Prop-2-in-1-yl oder But-2-in-1-yl,
sowie agrochemisch wirksame Salze davon.

In dieser ersten Ausführungsform der vorliegenden Erfindung sind Verbindungen der Formel [I-a] **weiterhin insbesondere bevorzugt,** in denen ein oder mehrere der Symbole eine der folgenden Bedeutungen haben:
- X¹: steht für C-H,
- R⁴: steht für H,
- R³: steht für Propan-2-yl, Isobutyl, Butan-2-yl, 2-Methylpropyl, Prop-2-in-1-yl, Cyclopropyl, Cyclobutyl, Cyclopentyl, (2,2-Dichlorcyclopropyl)methyl, Cyclopropylmethyl, 1-Cyclopropylethyl, 2,2,2-Trifluorethyl, 2-Fluorbenzyl, 2-(Trifluormethoxy)ethyl, 1-Methoxypropan-2-yl, 2,2-Difluorethyl, 2-Cyanethyl, Cyanmethyl, 1-Cyanpropan-2-yl, 1-Propyl, 2-Ethoxyethyl, 2-Chlorethyl oder 2-Methoxyethyl,
wobei die übrigen Substituenten eine oder mehrere der oben genannten Bedeutungen haben,
sowie die agrochemisch wirksamen Salzen davon.

**Weiterhin insbesondere bevorzugt** werden Verbindungen der ersten Ausführungsform der Erfindung der Formel **[I-a],** in denen
- R³: für Propan-2-yl, Isobutyl, Butan-2-yl, 2-Methylpropyl, Prop-2-in-1-yl, Cyclopropyl, Cyclobutyl, Cyclopentyl, (2,2-Dichlorcyclopropyl)methyl, Cyclopropylmethyl, 2,2,2-Trifluorethyl, 2-Fluorbenzyl, 2-(Trifluormethoxy)ethyl, 1 -Methoxypropan-2-yl steht,
wobei die übrigen Substituenten eine oder mehrere der oben genannten Bedeutungen haben,
sowie die agrochemisch wirksamen Salzen davon.

In einer zweiten Ausführungsform der vorliegenden Erfindungen sind Verbindungen der Formel **[I-a] besonders bevorzugt,** in denen ein oder mehrere der Symbole eine der folgenden Bedeutungen haben:
- X¹: steht für N,
- R¹: steht für jeweils gegebenenfalls einfach oder mehrfach gleich oder verschieden durch R⁷ substituiertes Phenyl oder Naphthalenyl,
- R²: steht für Halogen, C₁-C₆-Alkyl, C₃-C₆-Cycloalkyl oder Wasserstoff,
- R³: steht für jeweils gegebenenfalls einfach oder mehrfach gleich oder verschieden durch Halogen, Cyano, Hydroxy, Halogenalkoxy substituiertes C₁-C₆-Alkyl, C₂-C₆-Alkenyl, C₂-C₆-Alkinyl, C₃-C₆-Cycloalkyl, C₃-C₆-Cycloalkyl-C₁-C₆-alkyl, C₆-C₁₄-Aryl-C₁-C₆-alkyl, C₁-C₆-Alkoxy-C₁-C₆-alkyl, C₁-C₆-Alkoxy, C₂-C₉-Heterocyclyl-C₁-C₆-alkyl und C₂-C₉-Heteroaryl
- R⁴: steht für Wasserstoff, -C(O)NR¹²R¹³ oder -NR¹²R¹³,
wobei R⁴ bevorzugt für -NHR¹³ steht, und R¹³ für jeweils gegebenenfalls einfach oder mehrfach gleich oder verschieden durch Fluor, Chlor, Brom, -OH, Cyano substituiertes C₁-C₆-Alkyl, C₃-C₆-Cycloalkyl, C₂-C₆-Alkenyl, C₂-C₆-Alkinyl, C₂-C₉-Heterocyclyl oder C₂-C₉-Heteroaryl steht,
- R⁵ und R⁶: stehen unabhängig voneinander für Wasserstoff, Fluor, Chlor, Brom, Cyano, Nitro,
oder für jeweils gegebenenfalls einfach oder mehrfach gleich oder verschieden durch R¹¹ substituiertes C₁-C₆-Alkyl, C₃-C₆-Cycloalkyl, C₂-C₆-Alkenyl, C₂-C₆-Alkinyl, -O-(C₁-C₄-Alkyl), -S-(C₁-C₄-Alkyl) oder -S(O)-(C₁-C₆-Alkyl),
oder bilden gemeinsam mit dem Kohlenstoffatomen, an das sie gebunden sind, einen Monocyclus, wobei sie gemeinsam für gegebenenfalls einfach oder mehrfach gleich oder verschieden durch R¹¹ substituiertes -(CH=CH-CH=CH)-, -(CH=CH-N(R¹⁹), -(CH=CH-CH=N)-, -(NH-CH=N)- oder -(CH₂-C(O)-N(R¹⁹)- stehen,
- R⁷: steht unabhängig voneinander für eine oder mehrere der folgenden Gruppen: Fluor, Chlor, Cyano, Nitro, Methyl, Ethyl, Isopropyl, -CF₃, -CHF₂, -C₂F₅, -CCl₃,
- R¹¹: steht für -OH, Fluor, Chlor, Brom, Cyano, -NHC(O)R²⁰, -C(O)R²⁰, -C(O)OR²⁰,-C(O)NR²⁰R²¹, -SO₂R²⁰
oder für jeweils gegebenenfalls einfach oder mehrfach gleich oder verschieden durch Fluor, Chlor, Brom, -OH, Cyano, C₁-C₆-Alkyl oder -O-(C₁-C₄-Alkyl) substituiertes C₁-C₆-Alkyl, C₃-C₈-Cycloalkyl, C₂-C₆-Alkenyl, C₂-C₆-Alkinyl, -O-(C₁-C₄-Alkyl), -S-(C₁-C₄-Alkyl), -O-(C₃-C₈-Cycloalkyl) oder -S-(C₃-C₈-Cycloalkyl),
- R¹² und R¹³: stehen unabhängig voneinander für eine oder mehrere der folgenden Gruppen: H, -C(S)R¹⁵, -C(O)R¹⁵, -SO₂R¹⁵, -C(O)OR¹⁵, -OR¹⁵ oder -C(O)NR¹⁵R¹⁶
oder für jeweils gegebenenfalls einfach oder mehrfach gleich oder verschieden durch Fluor, Chlor, Brom, -OH, Cyano substituiertes C₁-C₆-Alkyl, C₃-C₆-Cycloalkyl, C₂-C₆-Alkenyl, C₂-C₆-Alkinyl, C₂-C₉-Heterocyclyl oder C₂-C₉-Heteroaryl,
- R¹⁵ und R¹⁶: stehen unabhängig voneinander für jeweils gegebenenfalls einfach oder mehrfach gleich oder verschieden durch R¹¹ substituiertes C₁-C₆-Alkyl, C₃-C₆-Cycloalkyl, C₂-C₆-Alkenyl, C₂-C₆-Alkinyl, C₃-C₈-Cycloalkyl, C₂-C₉-Heterocyclyl, C₂-C₉-Heteroaryl
oder für Wasserstoff
oder bilden gemeinsam mit dem Stickstoffatom an das sie gebunden sind einen 3- bis 7-gliedrigen Ring, der ein weiteres dem Stickstoff nicht benachbartes Heteroatom aus der Reihe N, O enthalten kann,
- R¹⁹: steht für H, C₂-C₆-Alkinyl, -C(O)R¹⁵, -SO₂R¹⁵, -C(O)OR¹⁵,
- R²⁰ und R²¹: stehen unabhängig voneinander für jeweils gegebenenfalls einfach oder mehrfach gleich oder verschieden durch Fluor, Chlor, Brom, -OH, Cyano substituiertes C₁-C₆-Alkyl, C₃-C₆-Cycloalkyl, C₂-C₆-Alkenyl, C₂-C₆-Alkinyl
oder für Wasserstoff,
sowie agrochemisch wirksame Salze davon.

In dieser zweiten Ausführungsform der Erfindung sind Verbindungen der Formel **[I-a] ganz besonders bevorzugt,** in denen ein oder mehrere der Symbole eine der folgenden Bedeutungen haben:
- X¹: steht für N,
- R¹: steht für gegebenenfalls einfach oder mehrfach gleich oder verschieden durch R⁷ substituiertes Phenyl,
- R²: steht für Methyl, Ethyl, Isopropyl, Cyclopropyl oder Wasserstoff,
- R³: steht für Methyl, Ethyl, 1-Propyl, Propan-2-yl, Isobutyl, Butan-2-yl, 2-Methylpropyl, 2,2-Dimethylpropyl, 3-Methylbut-2-en-1-yl, But-2-en-1-yl, But-3-en-2-yl, Propadienyl, Prop-2-in-1-yl, But-2-in-1-yl, But-3-in-2-yl, 2-Methylbut-3-in-2-yl, 2-Methylbut-3-in-2-yl, Cyanmethyl, 2-Cyanethyl, 1-Cyanpropan-2-yl, Cyclopropyl, Cyclobutyl, Cyclopentyl, Cyclohexyl, (2,2-Dichlorcyclopropyl)methyl, Cyclopropylmethyl, 1-Cyclopropylethyl, Trichlormethyl, Trifluormethyl, 2-Fluorethyl, 2,2-Difluorethyl, 2,2,2-Trifluorethyl, 2,2,2-Trichlorethyl, 2-Chlorethyl, 2-Bromethyl, 2-Fluorpropyl, 3-Fluorpropyl, 2-Chlorpropyl, 3-Chlorpropyl, 1,3-Difluorpropan-2-yl, 2-Fluorbenzyl, 3-Fluorbenzyl, 4-Fluorbenzyl, 2,3-Difluorbenzyl, 2-Chlor-6-fluorbenzyl, 1-(2-Chlorphenyl)ethyl, 1-(3-Chlorphenyl)ethyl, 1-(4-Chlorphenyl)ethyl, 3-Cyanobenzyl, 4-Cyanobenzyl, 4-(Difluormethoxy)benzyl, 2-Cyanobenzyl, 2-(3-Chlorphenyl)ethyl, 2-(2-Chlorphenyl)ethyl, 1-Naphthylmethyl,(Pyridin-3-ylmethyl, 2-Chlor-1,3-thiazol-5-yl)methyl, Methoxymethyl, 2-Methoxyethyl, 2-(Methylsulfanyl)ethyl, 2-(Trifluormethoxy)ethyl, 1-Methoxypropan-2-yl, 2-[2-(2-Methoxyethoxy)ethoxy]ethyl, 2-(2-Methoxyethoxy)ethyl, Tetrahydrofuran-2-ylmethyl, (3-Methyloxetan-3-yl)methyl, 1H-Imidazol-2-ylmethyl, Tetrahydrofuran-3-yl, 2-Oxotetrahydrofuran-3-yl, 2-tert-Butoxy-2-oxoethyl, 1-Methoxy-3-methyl-1-oxobutan-2-yl, 1-Methoxy-1-oxopropan-2-yl oder 3-Ethoxy-3-oxopropyl, Propan-2-yloxy, 2-Ethoxyethyl, oder 2-Chlorethyl,
- R⁴: steht für Wasserstoff, -NR¹²R¹³ oder -NHR¹³,
wobei R⁴ bevorzugt für -NHR¹³ steht, und R¹³ für jeweils gegebenenfalls einfach oder mehrfach gleich oder verschieden durch Fluor, Chlor, Brom, OH, Cyano substituiertes C₁-C₆-Alkyl, C₃-C₆-Cycloalkyl, C₂-C₆-Alkenyl, C₂-C₆-Alkinyl, C₂-C₉-Heterocyclyl oder C₂-C₉-Heteroaryl steht,
- R⁵ und R⁶: stehen unabhängig voneinander für Wasserstoff, Fluor oder Cyano
oder bilden gemeinsam mit dem Kohlenstoffatomen, an das sie gebunden sind, einen Cyclus, wobei sie gemeinsam für gegebenenfalls einfach oder mehrfach gleich oder verschieden durch R¹¹ substituiertes -(CH=CH-CH=CH)-, -(CH=CH-N(R¹⁹), -(CH=CH-CH=N)-, -(NH-CH=N)- oder -(CH₂-C(O)-N(R¹⁹)- stehen,
- R⁷: steht unabhängig voneinander für eine oder mehrere der folgenden Gruppen: Fluor, Chlor, Cyano oder Methyl,
- R¹¹: steht für -OH, Fluor, Chlor, Brom, Cyano, -NH-C(O)R²⁰, -C(O)R²⁰, -C(O)OR²⁰,-C(O)NR²⁰R²¹, -SO₂R²⁰
oder für jeweils gegebenenfalls einfach oder mehrfach gleich oder verschieden durch Fluor, Chlor, Brom, OH, Cyano, C₁-C₆-Alkyl oder -O-(C₁-C₄-Alkyl) substituiertes C₁-C₆-Alkyl, C₃-C₈-Cycloalkyl, C₂-C₆-Alkenyl, C₂-C₆-Alkinyl, -O-(C₁-C₄-Alkyl), -S-(C₁-C₄-Alkyl), -O-(C₃-C₈-Cycloakl) oder -S-(C₃-C₈-Cycloalkyl),
- R¹²: steht für -C(S)R¹⁵, -C(O)R¹⁵, -SO₂R¹⁵, -C(O)OR¹⁵, -OR¹⁵ oder -C(O)NR¹⁵R¹⁶,
- R¹³: steht für C₁-C₆-Alkyl, C₂-C₆-Alkenyl, C₂-C₆-Alkinyl C₂-C₉-Heterocyclyl oder C₂-C₉-Heteroaryl, oder Wasserstoff,
- R¹⁵: steht für jeweils gegebenenfalls einfach oder mehrfach gleich oder verschieden durch Halogen, OH, Cyano oder C₁-C₄-alkyl substituiertes C₁-C₆-Alkyl, C₃-C₆-Cycloalkyl, C₂-C₆-Alkenyl, C₂-C₆-Alkinyl, C₃-C₈-Cycloalkyl, C₂-C₉-Heterocyclyl, C₂-C₉-Heteroaryl
oder für Wasserstoff,
- R¹⁶: steht für Wasserstoff, Methyl, Ethyl oder Propyl,
- R¹⁹: steht für H, C₂-C₆-Alkinyl, -C(O)R¹⁵ -SO₂R¹⁵, -C(O)OR¹⁵,
- R²⁰ und R²¹: stehen unabhängig voneinander für jeweils gegebenenfalls einfach oder mehrfach gleich oder verschieden durch Fluor, Chlor, Brom, -OH, Cyano substituiertes Methyl, Ethyl, Propyl, iso-Propyl, Cyclopropyl, Cyclobutyl
oder für Wasserstoff,
sowie agrochemisch wirksame Salze davon.

In dieser zweiten Ausführungsform der Erfindung sind Verbindungen der Formel **[I-a] insbesondere bevorzugt,** in denen ein oder mehrere der Symbole eine der folgenden Bedeutungen haben:
- X¹: steht für N,
- R¹: steht für Phenyl, 4-Fluorphenyl,
- R²: steht für Methyl, Ethyl, Isopropyl, Cyclopropyl, oder Wasserstoff,
- R³: steht für Methyl, Ethyl, 1-Propyl, Propan-2-yl, Isobutyl, Butan-2-yl, 2-Methylpropyl, 2,2-Dimethylpropyl, Prop-2-in-1-yl, But-3-in-2-yl, Cyclopropyl, Cyclobutyl, Cyclopentyl, (2,2-Dichlorcyclopropyl)methyl, Cyclopropylmethyl, 1-Cyclopropylethyl, Trifluormethyl, Trichlormethyl, 2-Fluorethyl, 2,2-Difluorethyl, 2,2,2-Trifluorethyl, 2,2,2-Trichlorethyl, 2-Chlorethyl, 2-Fluorpropyl, 3-Fluorpropyl, 1,3-Difluorpropan-2-yl, 2-Fluorbenzyl, 2,3-Difluorbenzyl, 2-Chlor-6-fluorbenzyl, 1-(2-Chlorphenyl)ethyl, 1-(3-Chlorphenyl)ethyl, 2-(Trifluormethoxy)ethyl, oder 1-Methoxypropan-2-yl, 2,2-Difluorethyl, 2-Cyanethyl, Cyanmethyl, 1-Cyanpropan-2-yl, 1-Propyl, 2-Ethoxyethyl, 2-Chlorethyl oder 2-Methoxyethyl,
- R⁴: steht für Wasserstoff, -NHR¹² oder für -NHR¹³, bevorzugt für -NHR¹³
- R⁵ und R⁶: stehen unabhängig voneinander für Wasserstoff, Fluor oder Cyano
oder bilden gemeinsam mit dem Kohlenstoffatomen, an das sie gebunden sind, einen Cyclus, wobei sie gemeinsam für gegebenenfalls einfach oder mehrfach gleich oder verschieden durch R¹¹ substituiertes -(CH=CH-CH=CH)-, -(CH=CH-N(R¹⁹)-, -(CH=CH-CH=N)- oder -(CH₂-C(O)-N(R¹⁹)- stehen,
- R¹¹: steht für -OH, Fluor, Chlor, Cyano, C₁-C₆-Alkyl, C₂-C₆-Alkenyl oder C₂-C₆-Alkinyl, C₃-C₆-Cycloalkyl,
- R¹²: steht für -C(S)R¹⁵, -SO₂R¹⁵, -C(O)OR¹⁵ oder -C(O)R¹⁵,
- R¹³: steht für Allyl, Benzyl, Cyclobutyl, Cyclopent-3-en-1-yl, Cyclopentyl, Cyclopropyl, (1-Cyclopropylethyl), (1-Cyclopropylethyl), (Cyclopropylmethyl), (Cyclopropylmethyl), (Dicyclopropylmethyl), (2,2-Difluoroethyl), (2,2-Dimethoxyethyl), [2-(Dimethylamino)-2-oxoethyl], [(2,2-Dimethylcyclopropyl)methyl], (2-Ethoxyethyl), Ethyl, (3-Fluorobenzyl), (4-Fluorobenzyl), (2-Fluorobenzyl), [1-(2-Fluorophenyl)ethyl], (2-Hydroxy-2-methylpropyl), (2-Hydroxyethyl), (2-Hydroxypropyl), (2-Hydroxypropyl), Isopropyl, (2-Methoxyethyl), (1-Methoxypropan-2-yl), (1-Methoxypropan-2-yl), Methyl, [2-(Morpholin-4-yl)ethyl], Oxetan-3-yl, (1-Phenylethyl), Prop-2-yn-1-yl, Propyl, [1-(Pyridin-2-yl)ethyl], (Pyrimidin-2-ylmethyl), sec-Butyl, Tetrahydro-2H-pyran-3-yl, tetrahydro-2H-pyran-4-yl, Tetrahydro-2H-pyran-4-yl,
- R¹⁵: steht für jeweils gegebenenfalls einfach oder mehrfach gleich oder verschieden durch Halogen, -OH, Cyano oder C₁-C₄-alkyl substituiertes Methyl, Ethyl, n-Propyl, Isopropyl, sec-Butyl, Isobutyl, Cyclopropyl, Cyclobutyl, Cyclopentyl, Cyclohexyl, 2-Methoxyethyl, (2-Methoxyethoxy)methyl, Cyclopentenyl, Cyclohexenyl, Oxetanyl, Tetrahydrofuran-2-yl, Ethinyl, Prop-1-in-1-yl, Prop-1-en-1-yl, Aminomethyl, Aminoethyl, Aminopropyl, Aminobutyl, Aminoisopropyl, Aminocyclopropyl, Aminocyclobutyl, Aminocyclopentyl, Dimethylamino, Ethyl(methyl)amino, Pyrrolidinyl, Diethylamino, 2-Pyridyl, 3-Pyridyl, 4-Pyridyl, Ethoxycarbonyl, Benzyl, Phenyl
oder für Wasserstoff,
- R¹⁹: steht für H, Acetyl, Ethoxycarbonyl, Methoxycarbonyl, Prop-2-in-1-yl oder But-2-in-1-yl,
sowie agrochemisch wirksame Salze davon.

**Weiterhin insbesondere bevorzugt** werden Verbindungen zweiten Ausführungsform der Erfindung der Formel **[I-a],** in denen
- R⁴: für -NHR¹³ oder für H steht,
wobei die übrigen Substituenten eine oder mehrere der oben genannten Bedeutungen haben,
sowie die agrochemisch wirksamen Salzen davon.

**Ganz besonders bevorzugte** Verbindungen der Formel **[I-a]** der ersten und zweiten Ausführungsform der oben vorliegenden Erfindung sind solche, in denen ein oder mehrere der Symbole eine der folgenden Bedeutungen haben:
- R¹: steht **ganz besonders bevorzugt** für 4-Fluorphenyl, 3-Chlorphenyl, 2,6-Difluorphenyl, oder 3-Methylphenyl und insbesondere für 4-Fluorphenyl,
- R²: steht **ganz besonders bevorzugt** für Cyclopropyl, Ethyl, Methyl oder Wasserstoff und insbesondere für Wasserstoff,
- R⁵ und R⁶: stehen **ganz besonders bevorzugt** beide für Wasserstoff.

Die oben aufgeführten allgemeinen oder in Vorzugsbereichen aufgeführten Restedefinitionen bzw. Erläuterungen können jedoch auch untereinander, also zwischen den jeweiligen Bereichen und Vorzugsbereichen beliebig kombiniert werden. Sie gelten für die Endprodukte sowie für die Vor- und Zwischenprodukte entsprechend. Außerdem können einzelne Definitionen entfallen.

*Bevorzugt* sind solche Verbindungen der Formel **[I-a],** in welcher alle Reste jeweils die oben genannten bevorzugten Bedeutungen haben.

*Besonders bevorzugt* sind solche Verbindungen der Formel **[I-a],** in welcher alle Reste jeweils die oben genannten besonders bevorzugten Bedeutungen haben.

*Ganz besonders bevorzugt* sind solche Verbindungen der Formel **[I-a],** in welcher alle Reste jeweils die oben genannten ganz besonders bevorzugten Bedeutungen haben.

*Insbesondere bevorzugt* sind solche Verbindungen der Formel **[I-a],** in welcher alle Reste jeweils die oben genannten insbesondere bevorzugten Bedeutungen haben.

Außerdem wurden nun neue Phenylpyri(mi)dinylazole der Formel [I-b] gefunden, 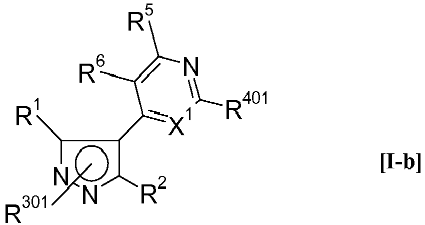 in welcher die Symbole folgende Bedeutungen haben:
- X¹: steht für C-H oder N,
- R¹: steht für jeweils gegebenenfalls einfach oder mehrfach gleich oder verschieden durch R⁷ substituiertes Phenyl, Naphthalenyl, Chinolin-5-yl, Chinolin-8-yl, Isochinolin-5-yl, Isochinolin-8-yl, 1-Benzothiophen-4-yl, 1-Benzothiophen-7-yl, 1-Benzofuran-4-yl, 1-Benzofuran-7-yl, 1,3-Benzodioxol-4-yl oder 1,3-Benzodioxol-5-yl,
- R²: steht für Cyano, Nitro, Halogen, C₁-C₆-Alkyl, C₁-C₆-Alkoxy, C₁-C₆-Halogenalkyl, C₁-C₆-Halogenalkoxy, C₁-C₆-Alkylthio, C₃-C₆-Cycloalkyl, C₃-C₆-Halogencycloalkyl, C₂-C₉-Heterocyclyl oder Wasserstoff,
- R³⁰¹: steht für -C(O)N(R⁹R¹⁰), -C(O)R⁹, -C(O)OR⁹, -S(O)₂R⁹ oder für jeweils gegebenenfalls einfach oder mehrfach gleich oder verschieden durch R⁸ substituiertes C₁-C₆-Alkyl, C₂-C₆-Alkenyl, C₃-C₆-Allenyl, C₂-C₆-Alkinyl, C₃-C₈-Cycloalkyl, C₁-C₆-Alkoxy, C₂-C₉-Heterocyclyl, C₂-C₉-OxoHeterocyclyl, oder Heteroaryl, (vorzugsweise C₂-C₉-Heteroaryl),
- R⁴⁰¹: steht für -NR¹²R¹³, -C(O)NR¹²R¹³ oder für -N(R¹²)₂
- R⁵ und R⁶: stehen unabhängig voneinander für Wasserstoff, Fluor, Chlor, Brom, Cyano, Nitro, -OH, - SH,
oder für jeweils gegebenenfalls einfach oder mehrfach gleich oder verschieden durch R¹¹ substituiertes C₁-C₆-Alkyl, C₃-C₆-Cycloalkyl, C₂-C₆-Alkenyl, C₂-C₆-Alkinyl, C₆-C₁₄-Aryl,-O-(C₁-C₄-Alkyl), -O-(C₆-C₁₄-Aryl), -S-(C₁-C₄-Alkyl), -S(O)-(C₁-C₆-Alkyl) oder -C(O)-(C₁-C₆-Alkyl),
oder bilden außerdem gemeinsam mit dem Kohlenstoffatomen, an das sie gebunden sind, einen gegebenenfalls einfach oder mehrfach, gleich oder verschieden durch Halogen, Sauerstoff, Cyano oder C₁-C₄-Alkyl substituierten Cyclus (vorzugsweise gesättigten, ungesättigten oder partiell ungesättigten Monocyclus) mit 3, vorzugsweise 5 bis 8 Ringatomen, wobei der Cyclus 1 bis 4 Heteroatome aus der Reihe Sauerstoff, Schwefel oder -N-R¹⁹ enthalten kann,
- R⁷: steht unabhängig voneinander für eine oder mehrere der folgenden Gruppen: Fluor, Chlor, Brom, Cyano, Nitro, -OH, -SH,
oder für gegebenenfalls einfach oder mehrfach gleich oder verschieden durch Fluor, Chlor, Brom, OH, Cyano, C₁-C₆-Alkyl oder -O-(C₁-C₄-Alkyl) substituiertes C₁-C₆-Alkyl, C₃-C₆-Cycloalkyl, C₂-C₆-Alkenyl, C₂-C₆-Alkinyl, Tri(C₁-C₄-alkyl)silyl, C₆-C₁₄-Aryl, -O-(C₁-C₄-Alkyl), -O-(C₆-C₁₄-Aryl), -S-(C₁-C₄-Alkyl), -S(O)-(C₁-C₆-Alkyl), -S(O)₂-(C₁-C₆-Alkyl),
- R⁸: steht für -OH, Halogen (vorzugsweise Fluor, Chlor oder Brom), -NO₂, Cyano, -NR⁹R¹⁰,-C(O)N(R⁹R¹⁰), -C(O)R⁹, -C(O)OR⁹, -O-C(O)R⁹, -(CH₂)ₙC(O)R⁹, wobei n = eine ganze Zahl zwischen 1 und 6 ist,
oder für jeweils gegebenenfalls einfach oder mehrfach gleich oder verschieden durch R¹¹ substituiertes C₁-C₆-Alkyl, C₂-C₈-Alkenyl, C₂-C₈-Alkinyl, C₃-C₈-Cycloalkyl, C₁-C₆-Halogenalkyl, C₆-C₁₄-Aryl, C₂-C₉-Heterocyclyl, C₂-C₉-Heteroaryl, -O-(C₁-C₄-Alkyl), -S-(C₁-C₄-Alkyl), -O-(C₃-C₈-Cycloalkyl), -S-(C₃-C₈-Cycloalkyl),
- R⁹ und R¹⁰: stehen unabhängig voneinander für jeweils gegebenenfalls einfach oder mehrfach gleich oder verschieden durch R¹¹ substituiertes C₁-C₆-Alkyl, C₂-C₈-Alkenyl, C₂-C₈-Alkinyl, C₃-C₈-Cycloalkyl, C₆-C₁₄-Aryl, C₂-C₉-Heterocyclyl, C₂-C₉-Heteroaryl,
oder für Wasserstoff,
- R¹¹: steht für -OH, Fluor, Chlor, Brom, Cyano, -NH-C(O)R²⁰, -NR²⁰R²¹, -C(O)R²⁰, -C(O)OR²⁰, -C(O)NR²⁰R²¹, -SO₂R²⁰
oder für gegebenenfalls einfach oder mehrfach gleich oder verschieden durch Fluor, Chlor, Brom, OH, Carbonyl, Cyano, C₁-C₆-Alkyl oder -O-(C₁-C₄-Alkyl) substituiertes C₁-C₆-Alkyl, C₂-C₆-Alkenyl, C₂-C₆-Alkinyl, C₁-C₁₁-Heteroalkyl, C₃-C₈-Cycloalkyl, -O-(C₁-C₄-Alkyl), -S-(C₁-C₄-Alkyl), -O-(C₃-C₈-Cycloalkyl), -S-(C₃-C₈-Cycloalkyl), C₆-C₁₄-Aryl,-O-(C₆-C₁₄-Aryl), -S-(C₆-C₁₄-Aryl), C₂-C₉-Heterocyclyl oder C₂-C₉-Heteroaryl,
- R¹²: steht für -C(S)R¹⁵, -C(O)R¹⁵, -SO₂R¹⁵, -C(O)OR¹⁵, -OR¹⁵ oder -C(O)NR¹⁵R¹⁶,
- R¹³: steht für jeweils gegebenenfalls einfach oder mehrfach gleich oder verschieden durch Fluor, Chlor, Brom, -OH, Cyano, C₁-C₆-Alkyl, -O-C(O)-C₁-C₄-Alkyl, -O-P(O)(O-C₁-C₄-Alkyl)₂, -O-B(O-C₁-C₄-Alkyl)₂ oder -O-(C₁-C₄-Alkyl) substituiertes C₁-C₆-Alkyl, C₃-C₆-Cycloalkyl, C₂-C₆-Alkenyl, C₂-C₆-Alkinyl, C₃-C₈-Cycloalkyl, C₂-C₉-Heterocyclyl, C₂-C₉-Heteroaryl
oder für Wasserstoff,
- R¹⁵ und R¹⁶: stehen unabhängig voneinander für Wasserstoff oder -OH,
oder für jeweils gegebenenfalls einfach oder mehrfach gleich oder verschieden durch R¹¹ substituiertes C₁-C₆-Alkyl, C₃-C₆-Cycloalkyl, C₂-C₆-Alkenyl, C₂-C₆-Alkinyl, C₃-C₈-Cycloalkyl, C₆-C₁₄-Aryl, C₂-C₉-Heterocyclyl oder C₂-C₉-Heteroaryl
oder bilden (wenn R¹² für -C(O)NR¹⁵R¹⁶ steht) gemeinsam mit dem Stickstoffatom an das sie gebunden sind einen 3- bis 7-gliedrigen Ring, der ein weiteres dem Stickstoff nicht direkt benachbartes Heteroatom aus der Reihe N, O enthalten kann,
- R¹⁹: steht für H, C₁-C₆-Alkyl, C₃-C₆-Cycloalkyl, C₂-C₆-Alkenyl, C₂-C₆-Alkinyl, -C(S)R¹⁵,-C(O)R¹⁵, -SO₂R¹⁵, -C(O)OR¹⁵,
- R²⁰ und R²¹: stehen unabhängig voneinander für jeweils gegebenenfalls einfach oder mehrfach gleich oder verschieden durch Fluor, Chlor, Brom, -OH, Cyano substituiertes C₁-C₆-Alkyl, C₃-C₆-Cycloalkyl, C₂-C₆-Alkenyl, C₂-C₆-Alkinyl oder Wasserstoff,
sowie agrochemisch wirksame Salze davon.

Ausgenommen von den oben aufgeführten allgemeinen oder in Vorzugsbereichen aufgeführten Restedefinitionen bzw. Erläuterungen sind Kombinationen in denen die Symbole der Formel **[I-b]** die folgenden Bedeutungen haben:
a) Verbindungen in denen,
   - R³⁰¹: für gegebenenfalls substituiertes [1,2,4]Triazolo[4,3-b]pyridazin-6-yl, 7,8-Dihydro[1,2,4]triazolo[4,3-b]pyridazin 6-yl, 6-Oxo-1,6-dihydropyridazin-3-yl, 6-Oxo-1,4,5,6-tetrahydropyridazin-3-yl oder 6-Chlorpyridazin-3-yl steht und
   - R⁵, R⁶: für H stehen.
b) Verbindungen: 4- {1-[2-(Dimethylamino)ethyl]-3-(4-fluorphenyl)-1H-pyrazol-4-yl} -N,N-dimethylpyridin-2-amin und 1-(4-{4-[1-Ethyl-3-(4-nitrophenyl)-1H-pyrazol-4-yl]-1H-pyrrolo[2,3-b]pyridin-6-yl}phenyl)-N,N-dimethylmethanamin.

Schließlich wurde gefunden, dass die erfindungsgemäßen Phenylpyri(mi)dinylazole der Formel **[I-b]** sehr gute mikrobizide Eigenschaften besitzen und zum Materialschutz sowie zur Reduktion von Mykotoxinen in Pflanzen und Pflanzenteilen verwendbar sind.

Die erfindungsgemäßen Phenylpyri(mi)dinylazole sind durch die Formel [I-b] allgemein definiert. Bevorzugte Restedefinitionen der vorstehenden und nachfolgend genannten Formeln sind im Folgenden angegeben. Diese Definitionen gelten für die Endprodukte der Formel [I-b] wie für alle Zwischenprodukte gleichermaßen.

**Bevorzugt** werden Verbindungen der Formel **[I-b],** in denen ein oder mehrere der Symbole eine der folgenden Bedeutungen haben:
- X¹: steht für C-H oder N,
- R¹: steht für jeweils gegebenenfalls einfach oder mehrfach gleich oder verschieden durch R⁷ substituiertes Phenyl, Naphthalenyl, Chinolin-5-yl, Chinolin-8-yl, Isochinolin-5-yl, Isochinolin-8-yl, 1,3-Benzodioxol-4-yl oder 1,3-Benzodioxol-5-yl,
- R²: steht für Cyano, Halogen, C₁-C₆-Alkyl, C₁-C₆-Alkoxy, C₁-C₆-Halogenalkyl, C₁-C₆-Halogenalkoxy, C₁-C₆-Alkylthio, C₃-C₆-Cycloalkyl, C₃-C₆-Halogencycloalkyl, C₂-C₉-Heterocyclyl oder Wasserstoff,
- R³⁰¹: steht für -C(O)R⁹, -C(O)OR⁹, -S(O)₂R⁹
oder für gegebenenfalls einfach oder mehrfach gleich oder verschieden durch R⁸ substituiertes C₁-C₆-Alkyl, C₂-C₆-Alkenyl, C₃₋₆-Allenyl, C₂₋₆-Alkinyl, C₃-C₈-Cycloalkyl, C₁-C₆-Alkoxy, C₂-C₉-Heterocyclyl, C₂-C₉-Oxoheterocyclyl, oder Heteroaryl,
- R⁴⁰¹: steht für -NR¹²R¹³, -C(O)NR¹²R¹³,
- R⁵ und R⁶: stehen unabhängig voneinander für Wasserstoff, Fluor, Chlor, Brom, Cyano, Nitro, -OH, - SH,
oder für jeweils gegebenenfalls einfach oder mehrfach gleich oder verschieden durch R¹¹ substituiertes C₁-C₆-Alkyl, C₃-C₆-Cycloalkyl, C₂-C₆-Alkenyl, C₂-C₆-Alkinyl, -O-(C₁-C₄-Alkyl), -S-(C₁-C₄-Alkyl) oder -S(O)-(C₁-C₆-Alkyl),
oder bilden außerdem gemeinsam mit dem Kohlenstoffatom, an das sie gebunden sind, einen gegebenenfalls einfach oder mehrfach, gleich oder verschieden durch Halogen, Sauerstoff, Cyano oder C₁-C₄-Alkyl substituierten Cyclus (vorzugsweise einen gesättigten, ungesättigten oder partiell ungesättigten Monocyclus) mit 3, vorzugsweise 5 bis 8 Ringatomen, wobei der Cyclus 1 bis 4 Heteroatome aus der Reihe Sauerstoff, Schwefel oder -NR¹⁹ enthalten kann,
- R⁷: steht für eine oder mehrere der folgenden Gruppen: Fluor, Chlor, Brom, Cyano, Nitro, Methyl, Ethyl, Isopropyl, -CF₃, -CHF₂, -C₂F₅, -CCl₃, -OMe, -OEt, -O-iPr, -OCF₃, -OCHF₂, -OC₂F₅, -SMe oder -SCF₃,
- R⁸: steht für -OH, Fluor, Chlor, Cyano, -NR⁹R¹⁰, -C(O)N(R⁹R¹⁰), -C(O)R⁹, -C(O)OR⁹, -O-C(O)R⁹, -(CH₂)ₙC(O)R⁹, wobei n = eine ganze Zahl zwischen 1 und 6 ist, oder für jeweils gegebenenfalls einfach oder mehrfach gleich oder verschieden durch R¹¹ substituiertes C₁-C₆-Alkyl, C₂-C₈-Alkenyl, C₂-C₈-Alkinyl, C₃-C₈-Cycloalkyl, C₁-C₆-Halogenalkyl, C₆-C₁₄-Aryl, C₂-C₉-Heterocyclyl, C₂-C₉-Heteroaryl, -O-(C₁-C₄-Alkyl), -S-(C₁-C₄-Alkyl), -O-(C₃-C₈-Cycloalkyl), -S-(C₃-C₈-Cycloalkyl),
- R⁹ und R¹⁰: stehen unabhängig voneinander für jeweils gegebenenfalls einfach oder mehrfach gleich oder verschieden durch R¹¹ substituiertes C₁-C₆-Alkyl, C₂-C₈ Alkenyl, C₂-C₈-Alkinyl, C₃-C₈-Cycloalkyl, C₂-C₉-Heterocyclyl, oder C₂-C₉-Heteroaryl, oder für Wasserstoff,
- R¹¹: steht für eine oder mehrere der folgenden Gruppen: -OH, Fluor, Chlor, Brom, Cyano, -NH-C(O)R²⁰, -C(O)R²⁰, -C(O)OR²⁰, -C(O)NR²⁰R²¹, -SO₂R²⁰
oder für jeweils gegebenenfalls einfach oder mehrfach gleich oder verschieden durch Fluor, Chlor, Brom, -OH, Cyano, C₁-C₆-Alkyl oder -O-(C₁-C₄-Alkyl) substituiertes C₁-C₆-Alkyl, C₂-C₆-Alkenyl, C₂-C₆-Alkinyl, C₁-C₁₁-Heteroalkyl, C₃-C₈-Cycloalkyl, -O-(C₁-C₄-Alkyl),-S-(C₁-C₄-Alkyl), -O-(C₃-C₈-Cycloalkyl), -S-(C₃-C₈-Cycloalkyl), C₂-C₉-Heterocyclyl oder C₂-C₉-Heteroaryl,
- R¹²: steht für -C(S)R¹⁵, -C(O)R¹⁵, -SO₂R¹⁵, -C(O)OR¹⁵, -OR¹⁵ oder -C(O)NR¹⁵R¹⁶,
- R¹³: steht für jeweils gegebenenfalls einfach oder mehrfach gleich oder verschieden durch Fluor, Chlor, Brom, -OH, Cyano substituiertes C₁-C₆-Alkyl, C₃-C₆-Cycloalkyl, C₂-C₆-Alkenyl, C₂-C₆-Alkinyl, C₃-C₈-Cycloalkyl, C₂-C₉-Heterocyclyl, C₂-C₉-Heteroaryl oder für Wasserstoff,
- R¹⁵ und R¹⁶: stehen unabhängig voneinander für Wasserstoff oder -OH
oder für jeweils gegebenenfalls einfach oder mehrfach gleich oder verschieden durch R¹¹ substituiertes C₁-C₆-Alkyl, C₃-C₆-Cycloalkyl, C₂-C₆-Alkenyl, C₂-C₆-Alkinyl, C₃-C₈-Cycloalkyl, C₆-C₁₄-Aryl, C₂-C₉-Heterocyclyl oder C₂-C₉-Heteroaryl
oder bilden gemeinsam mit dem Stickstoffatom an das sie gebunden sind einen 3- bis 7-gliedrigen Ring, der ein weiteres dem Stickstoff nicht (direkt) benachbartes Heteroatom aus der Reihe N, O enthalten kann,
- R¹⁹: steht für H, C₁-C₆-Alkyl, C₃-C₆-Cycloalkyl, C₂-C₆-Alkenyl, C₂-C₆-Alkinyl, -C(S)R¹⁵,-C(O)R¹⁵, -SO₂R¹⁵, -C(O)OR¹⁵,
- R²⁰ und R²¹: stehen unabhängig voneinander für jeweils gegebenenfalls einfach oder mehrfach gleich oder verschieden durch Fluor, Chlor, Brom, -OH oder Cyano substituiertes C₁-C₆-Alkyl, C₃-C₆-Cycloalkyl, C₂-C₆-Alkenyl, C₂-C₆-Alkinyl oder für Wasserstoff,
sowie agrochemisch wirksame Salze davon.

**Besonders bevorzugt** werden Verbindungen der Formel **[I-b],** in denen ein oder mehrere der Symbole eine der folgenden Bedeutungen haben:
- X¹: steht für C-H oder N,
- R¹: steht für jeweils gegebenenfalls einfach oder mehrfach gleich oder verschieden durch R⁷ substituiertes Phenyl oder Naphthalenyl,
- R²: steht für Halogen, C₁-C₆-Alkyl, C₃-C₆-Cycloalkyl oder Wasserstoff,
- R³⁰¹: für jeweils gegebenenfalls einfach oder mehrfach gleich oder verschieden durch R⁸ substituiertes C₁-C₆-Alkyl, C₂-C₆-Alkenyl, C₃-₆-Allenyl, C₂-₆-Alkinyl, C₃-C₈-Cycloalkyl, C₁-C₆-Alkoxy, C₂-C₉-Heterocyclyl, C₂-C₉-OxoHeterocyclyl, oder Heteroaryl,
- R⁴⁰¹: steht für -NR¹²R¹³,
- R⁵ und R⁶: stehen unabhängig voneinander für Wasserstoff, Fluor, Chlor, Brom, Cyano, Nitro,
oder für jeweils gegebenenfalls einfach oder mehrfach gleich oder verschieden durch R¹¹ substituiertes C₁-C₆-Alkyl, C₃-C₆-Cycloalkyl, C₂-C₆-Alkenyl, C₂-C₆-Alkinyl, -O-(C₁-C₄-Alkyl), S-(C₁-C₄-Alkyl) oder -S(O)-(C₁-C₆-Alkyl),
oder bilden gemeinsam mit dem Kohlenstoffatomen, an das sie gebunden sind, einen gegebenenfalls einfach oder mehrfach, gleich oder verschieden durch Halogen, Sauerstoff, Cyano oder C₁-C₄-Alkyl substituierten Cyclus mit 5 bis 8 Ringatomen, wobei der Cyclus 1 bis 4 weitere Heteroatome aus der Reihe Sauerstoff, Schwefel oder -N-R¹⁹ enthalten kann,
- R⁷: steht für Fluor, Chlor, Cyano, Nitro, Methyl, Ethyl, Isopropyl, -CF₃, -CHF₂,C₂F₅, CCl₃,
- R⁸: steht für -OH, Fluor, Chlor, Cyano, -C(O)R⁹, -C(O)OR⁹, -O-C(O)R⁹, -(CH₂)ₙC(O)R⁹, wobei n = eine ganze Zahl zwischen 1 und 6 ist, oder für jeweils gegebenenfalls einfach oder mehrfach gleich oder verschieden durch R¹¹ substituiertes C₁-C₆-Alkyl, C₂-C₈-Alkenyl, C₂-C₈-Alkinyl, C₃-C₈-Cycloalkyl, C₁-C₆-Halogenalkyl, C₂-C₉-Heterocyclyl, C₂-C₉-Heteroaryl, -O-(C₁-C₄-Alkyl), -S-(C₁-C₄-Alkyl), -O-(C₃-C₈-Cycloalkyl), -S-(C₃-C₈-Cycloalkyl),
- R⁹ und R¹⁰: stehen unabhängig voneinander für jeweils gegebenenfalls einfach oder mehrfach gleich oder verschieden durch R¹¹ substituiertes C₁-C₆-Alkyl, C₂-C₈-Alkenyl, C₂-C₈-Alkinyl, C₃-C₈-Cycloalkyl
oder für Wasserstoff,
- R¹¹: steht für -OH, Fluor, Chlor, Brom, Cyano, -NH-C(O)R²⁰, -C(O)R²⁰, -C(O)OR²⁰,-C(O)NR²⁰R²¹, -SO₂R²⁰
oder für gegebenenfalls einfach oder mehrfach gleich oder verschieden durch Fluor, Chlor, Brom, -OH, Cyano, C₁-C₆-Alkyl oder -O-(C₁-C₄-Alkyl) substituiertes C₁-C₆-Alkyl, C₃-C₈-Cycloalkyl, C₂-C₆-Alkenyl, C₂-C₆-Alkinyl, -O-(C₁-C₄-Alkyl), -S-(C₁-C₄-Alkyl), -O-(C₃-C₈-Cycloalkyl), -S-(C₃-C₈-Cycloalkyl),
- R¹²: steht für -C(S)R¹⁵, -C(O)R¹⁵, -SO₂R¹⁵, -C(O)OR¹⁵ oder -C(O)NR¹⁵R¹⁶,
- R¹³: steht für jeweils gegebenenfalls einfach oder mehrfach gleich oder verschieden durch Fluor, oder Chlor substituiertes C₁-C₆-Alkyl, C₃-C₆-Cycloalkyl, C₂-C₆-Alkenyl, C₂-C₆-Alkinyl, C₃-C₈-Cycloalkyl, C₂-C₉-Heterocyclyl, C₂-C₉-Heteroaryl
oder für Wasserstoff,
- R¹⁵ und R¹⁶: stehen unabhängig voneinander für jeweils gegebenenfalls einfach oder mehrfach gleich oder verschieden durch R¹¹ substituiertes C₁-C₆-Alkyl, C₃-C₆-Cycloalkyl, C₂-C₆-Alkenyl, C₂-C₆-Alkinyl, C₃-C₈-Cycloalkyl, C₆-C₁₄-Aryl, C₂-C₉-Heterocyclyl, C₂-C₉-Heteroaryl,
oder für Wasserstoff
oder bilden gemeinsam mit dem Stickstoffatom an das sie gebunden sind einen 3- bis 7-gliedrigen Ring, der ein weiteres dem Stickstoff nicht (direkt) benachbartes Heteroatom aus der Reihe N,O enthalten kann,
- R¹⁹: steht für H, C₂-C₆-Alkinyl, -C(O)R¹⁵, -SO₂R¹⁵, -C(O)OR¹⁵,
- R²⁰ und R²¹: stehen unabhängig voneinander für jeweils gegebenenfalls einfach oder mehrfach gleich oder verschieden durch Fluor, Chlor, Brom, -OH, Cyano substituiertes C₁-C₆-Alkyl, C₃-C₆-Cycloalkyl, C₂-C₆-Alkenyl, C₂-C₆-Alkinyl
oder für Wasserstoff,
sowie agrochemisch wirksame Salze davon.

**Ganz besonders bevorzugt** werden Verbindungen der Formel **[I-b],** in denen ein oder mehrere der Symbole eine der folgenden Bedeutungen haben:
- X¹: steht für C-H oder N,
- R¹: steht für gegebenenfalls einfach oder mehrfach gleich oder verschieden durch R⁷ substituiertes Phenyl,
- R²: steht für Methyl, Ethyl, Isopropyl, Cyclopropyl, oder Wasserstoff,
- R³⁰¹: steht für jeweils gegebenenfalls einfach oder mehrfach gleich oder verschieden durch R⁸ substituiertes C₁-C₆-Alkyl, C₂-C₆-Alkenyl, C₃-C₆-Allenyl, C₂-C₆-Alkinyl, C₃-C₈-Cycloalkyl oder C₁-C₆-Alkoxy,
- R⁴⁰¹: steht für -NR¹²R¹³,
- R⁵ und R⁶: stehen unabhängig voneinander für Wasserstoff, Fluor oder Cyano
oder bilden gemeinsam mit dem Kohlenstoffatomen, an das sie gebunden sind, einen Cyclus, wobei sie gemeinsam für gegebenenfalls einfach oder mehrfach gleich oder verschieden durch R¹¹ substituiertes -(CH=CH-CH=CH)-, -(CH=CH-N(R¹⁹)-, -(CH=CH-CH=N)- oder -(CH₂-C(O)-N(R¹⁹)- stehen,
- R⁷: steht für Fluor, Chlor, Cyano oder Methyl,
- R⁸: steht für Fluor, Chlor, Cyano oder für jeweils gegebenenfalls einfach oder mehrfach gleich oder verschieden durch R¹¹ substituiertes C₁-C₆-Alkyl, C₂-C₈-Alkenyl, C₂-C₈-Alkinyl, C₃-C₈-Cycloalkyl, C₆-C₁₄-Aryl, Heterocyclyl, Heteroaryl, -O-(C₁-C₄-Alkyl), -S-(C₁-C₄-Alkyl), -O-(C₃-C₈-Cycloalkyl), -S-(C₃-C₈-Cycloalkyl),
- R¹¹: steht für eine oder mehrere der folgenden Gruppen: -OH, Fluor, Chlor, Brom, Cyano, -NH-C(O)R²⁰, -C(O)R²⁰, -C(O)OR²⁰, -C(O)NR²⁰R²¹, -SO₂R²⁰
oder für jeweils gegebenenfalls einfach oder mehrfach gleich oder verschieden durch Fluor, Chlor, Brom, OH, Cyano, C₁-C₆-Alkyl oder O-(C₁-C₄-Alkyl) substituiertes C₁-C₆-Alkyl, C₃-C₈-Cycloalkyl, C₂-C₆-Alkenyl, C₂-C₆-Alkinyl, -O-(C₁-C₄-Alkyl), -S-(C₁-C₄-Alkyl), -O-(C₃-C₈-Cycloalkyl) oder S-(C₃-C₈-Cycloalkyl),
- R¹²: steht für: -C(S)R¹⁵, -C(O)R¹⁵, -SO₂R¹⁵, -C(O)OR¹⁵, -OR¹⁵ oder -C(O)NR¹⁵R¹⁶,
- R¹³: steht für C₁-C₆-Alkyl, C₂-C₆-Alkenyl, C₂-C₆-Alkinyl oder Wasserstoff,
- R¹⁵: steht für jeweils gegebenenfalls einfach oder mehrfach gleich oder verschieden durch Halogen, -OH, Cyano oder C₁-C₄-Alkyl substituiertes C₁-C₆-Alkyl, C₃-C₆-Cycloalkyl, C₂-C₆-Alkenyl, C₂-C₆-Alkinyl, C₃-C₈-Cycloalkyl, C₆-C₁₄-Aryl, C₂-C₉-Heterocyclyl, C₂-C₉-Heteroaryl,
oder für Wasserstoff,
- R¹⁶: steht für Wasserstoff, Methyl, Ethyl oder Propyl,
- R¹⁹: steht für H, C₂-C₆-Alkinyl, -C(O)R¹⁵, -SO₂R¹⁵, -C(O)OR¹⁵,
- R²⁰ und R²¹: stehen unabhängig voneinander für jeweils gegebenenfalls einfach oder mehrfach gleich oder verschieden durch Fluor, Chlor, Brom, -OH, Cyano substituiertes Methyl, Ethyl, Propyl, iso-Propyl, Cyclopropyl, Cyclobutyl
oder für Wasserstoff,
sowie agrochemisch wirksame Salze davon.

In einer weiteren Ausführungsform der vorliegenden Erfindung sind insbesondere auch Verbindungen der Formel **[I-b]** **bevorzugt**, in denen ein oder mehrere der Symbole eine der folgenden Bedeutungen haben:
- X¹: steht für N,
- R¹: steht für Phenyl, 3-Methylphenyl, 2-Fluorphenyl, 3-Fluorphenyl, 4-Fluorphenyl, 3-Trifluormethylphenyl, 4-Chlorphenyl, 2,5-Difluorphenyl, 2,4-Difluorphenyl, 2,6-Difluorphenyl, 3-Methyl-4-fluorphenyl, 3-Cyan-4-fluorphenyl oder 2,4,6-Trifluorphenyl,
- R²: steht für Methyl, Ethyl, Isopropyl, Cyclopropyl, oder Wasserstoff,
- R³⁰¹: steht für Methyl, Ethyl, 1-Propyl, Propan-2-yl, Isobutyl, Butan-2-yl, 2-Methylpropyl, 2,2-Dimethylpropyl, 2-(Morpholin-4-yl)ethyl, 2-Cyanethyl, Cyanmethyl, 2-Cyan-2-methylpropyl, 3-Methylbut-2-en-1-yl, But-2-en-1-yl, But-3-en-2-yl, Propadienyl, Prop-2-en-1-yl, Prop-2-in-1-yl, But-2-in-1-yl, But-3-in-2-yl, 2-Methylbut-3-in-2-yl, 2-Methylbut-3-in-2-yl, Cyclopropyl, Cyclobutyl, Cyclopentyl, Cyclohexyl, (2,2-Dichlorcyclopropyl)methyl, Cyclopropylmethyl, 1-Cyclopropylethyl, Trichlormethyl, Trifluormethyl, 2-Fluorethyl, 2,2-Difluorethyl, 2,2,2-Trifluorethyl, 2,2,2-Trichlorethyl, 2-Chlorethyl, 2-Bromethyl, 2-Fluorpropyl, 3-Fluorpropyl, 2-Chlorpropyl, 3-Chlorpropyl, 1,3-Difluorpropan-2-yl, 1,1,1-Trifluorpropan-2-yl, 1,1,1-Trifluor-2-methylpropan-2-yl, 3,3,3-Trifluor-2-hydroxypropyl, 2-Fluorbenzyl, 3-Fluorbenzyl, 4-Fluorbenzyl, 2,3-Difluorbenzyl, 2-Chlor-6-fluorbenzyl, 1-(2-Chlorphenyl)ethyl, 1-(3-Chlorphenyl)ethyl, 1-(4-Chlorphenyl)ethyl, 3-Cyanobenzyl, 4-Cyanobenzyl, 4-(Difluormethoxy)benzyl, 2-Cyanobenzyl, 2-(3-Chlorphenyl)ethyl, 2-(2-Chlorphenyl)ethyl, 1-Naphthylmethyl, (Pyridin-3-ylmethyl, 2-Chlor-1,3-thiazol-5-yl)methyl, Methoxymethyl, 2-Methoxyethyl, 2-Methoxypropyl, 2-(Methylsulfanyl)ethyl, 2-(Trifluormethoxy)ethyl, 1-Methoxypropan-2-yl, 2-[2-(2-Methoxyethoxy)ethoxy]ethyl, 2-(2-Methoxyethoxy)ethyl, 1,3-Dimethoxypropan-2-yl, 2-(Cyclopropyloxy)ethyl, Tetrahydrofuran-2-ylmethyl, (3-Methyloxetan-3-yl)methyl, 1H-Imidazol-2-ylmethyl, Tetrahydrofuran-3-yl, 2-Oxotetrahydrofuran-3-yl, 2-tert-Butoxy-2-oxoethyl, 1-Methoxy-3-methyl-1-oxobutan-2-yl, 1-Methoxy-1-oxopropan-2-yl oder 3-Ethoxy-3-oxopropyl, 1-Cyanpropan-2-yl, 1-Propyl, Propan-2-yloxy, 2-Ethoxyethyl,
- R⁴⁰¹: steht für -NHR¹²,
- R⁵ und R⁶: stehen unabhängig voneinander für Wasserstoff, Fluor oder Cyano
oder bilden gemeinsam mit dem Kohlenstoffatomen, an das sie gebunden sind, einen Cyclus, wobei sie gemeinsam für gegebenenfalls einfach oder mehrfach gleich oder verschieden durch R¹¹ substituiertes -(CH=CH-CH=CH)-, -(CH=CH-N(R¹⁹)-, -(CH=CH-CH=N)- oder -(CH₂-C(O)-N(R¹⁹)- stehen,
- R¹¹: steht für eine oder mehrere der folgenden Gruppen: -OH, Fluor, Chlor, Cyano, Methyl, Ethyl, Cyclopropyl,
- R¹²: steht für -C(S)R¹⁵, -SO₁R¹⁵, -C(O)OR¹⁵ oder -C(O)R¹⁵,
- R¹⁵: steht für jeweils gegebenenfalls einfach oder mehrfach gleich oder verschieden durch Halogen, -OH, Cyano oder C₁-C₄-Alkyl substituiertes Methyl, Ethyl, n-Propyl, Isopropyl, sec-Butyl, Isobutyl, Cyclopropyl, Cyclobutyl, Cyclopentyl, Cyclohexyl, 2-Methoxyethyl, (2-Methoxyethoxy)methyl, Cyclopentenyl, Cyclohexenyl, Oxetanyl, Tetrahydrofuran-2-yl, Ethinyl, Prop-1-in-1-yl, Prop-1-en-1-yl, Aminomethyl, Aminoethyl, Aminopropyl, Aminobutyl, Aminoisopropyl, Aminocyclopropyl, Aminocyclobutyl, Aminocyclopentyl, Dimethylamino, Ethyl(methyl)amino, Pyrrolidinyl, Diethylamino, 2-Pyridyl, 3-Pyridyl, 4-Pyridyl, Ethoxycarbonyl, Benzyl, Phenyl, 2-Thienyl, 3-Thienyl
oder für Wasserstoff,
- R¹⁹: steht für H, Acetyl, Ethoxycarbonyl, Methoxycarbonyl, Prop-2-in-1-yl, But-2-in-1-yl,
sowie agrochemisch wirksame Salze davon.

In einer weiteren Ausführungsform der vorliegenden Erfindung sind insbesondere Verbindungen der **Formel [I-b] bevorzugt,** in denen ein oder mehrere der Symbole eine der folgenden Bedeutungen haben:
- X¹: steht für C-H,
- R¹: steht für 4-Fluorphenyl, 3-Chlorphenyl, 2,6-Difluorphenyl, 3-Methylphenyl
- R²: steht für Cyclopropyl, Methyl, H, Difluormethoxy,
- R⁴⁰¹: steht für Acetylamino, n-Propionylamino, Isobutyrylamino, (Cyclopropylcarbonyl)amino, (Methoxyacetyl)amino, 2-Methoxypropanoyl, (2-Methylbutanoyl)amino, But-2-enoylamino, Prop-2-inoylamino, 3-(Dimethylamino)prop-2-enoyl]amino, 3,3,3-Trifluorpropanoyl)amino, 3,3-Difluorpropanoyl)amino, (Cyclopropylacetyl)amino, Lactoylamino, (Cyclobutylcarbonyl)amino, (Cyclopentylacetyl)amino, 2-Methylcyclopropyl)carbonyl] amino, (3-Methylbutanoyl)amino, (Phenylacetyl)amino, Benzoylamino, (3-Thienylcarbonyl)amino, (2-Thienylcarbonyl)amino (2-Hydroxy-2-methylpropanoyl)amino, [(2-Methoxyethoxy)acetyl]amino, 2,3-Dihydroxypropanoyl)amino,
wobei die übrigen Substituenten eine oder mehrere der oben genannten Bedeutungen haben,
sowie die agrochemisch wirksamen Salzen davon.

Weiterhin **insbesondere bevorzugt** werden Verbindungen der Formel **[I-b],** in denen ein oder mehrere der Symbole eine der folgenden Bedeutungen haben:
- X¹: steht für C-H,
- R¹: steht für 4-Fluorphenyl, 3-Chlorphenyl, 2,6-Difluorphenyl, 3-Methylphenyl,
- R²: steht für Cyclopropyl, Methyl, H, Difluormethoxy,
- R³⁰¹: steht für Methyl, Ethyl, 1-Propyl, Propan-2-yl, Isobutyl, Butan-2-yl, 2-Methylpropyl, 2,2-Dimethylpropyl, 2-(Morpholin-4-yl)ethyl, 2-Cyanethyl, Cyanmethyl, 2-Cyan-2-methylpropyl, 3-Methylbut-2-en-1-yl, But-2-en-1-yl, But-3-en-2-yl, Propadienyl, Prop-2-en-1-yl, Prop-2-in-1-yl, But-2-in-1-yl, But-3-in-2-yl, 2-Methylbut-3-in-2-yl, 2-Methylbut-3-in-2-yl, Cyclopropyl, Cyclobutyl, Cyclopentyl, Cyclohexyl, (2,2-Dichlorcyclopropyl)methyl, Cyclopropylmethyl, 1-Cyclopropylethyl, Trichlormethyl, Trifluormethyl, 2-Fluorethyl, 2,2-Difluorethyl, 2,2,2-Trifluorethyl, 2,2,2-Trichlorethyl, 2-Chlorethyl, 2-Bromethyl, 2-Fluorpropyl, 3-Fluorpropyl, 2-Chlorpropyl, 3-Chlorpropyl, 1,3-Difluorpropan-2-yl, 1,1,1-Trifluorpropan-2-yl, 1,1,1-Trifluor-2-methylpropan-2-yl, 3,3,3-Trifluor-2-hydroxypropyl, 2-Fluorbenzyl, 3-Fluorbenzyl, 4-Fluorbenzyl, 2,3-Difluorbenzyl, 2-Chlor-6-fluorbenzyl, 1-(2-Chlorphenyl)ethyl, 1-(3-Chlorphenyl)ethyl, 1-(4-Chlorphenyl)ethyl, 3-Cyanobenzyl, 4-Cyanobenzyl, 4-(Difluormethoxy)benzyl, 2-Cyanobenzyl, 2-(3-Chlorphenyl)ethyl, 2-(2-Chlorphenyl)ethyl, 1-Naphthylmethyl, (Pyridin-3-ylmethyl, 2-Chlor-1,3-thiazol-5-yl)methyl, Methoxymethyl, 2-Methoxyethyl, 2-Methoxypropyl, 2-(Methylsulfanyl)ethyl, 2-(Trifluormethoxy)ethyl, 1-Methoxypropan-2-yl, 2-[2-(2-Methoxyethoxy)ethoxy]ethyl, 2-(2-Methoxyethoxy)ethyl, 1,3-Dimethoxypropan-2-yl, 2-(Cyclopropyloxy)ethyl, Tetrahydrofuran-2-ylmethyl, (3-Methyloxetan-3-yl)methyl, 1H-Imidazol-2-ylmethyl, Tetrahydrofuran-3-yl, 2-Oxotetrahydrofuran-3-yl, 2-tert-Butoxy-2-oxoethyl, 1-Methoxy-3-methyl-1-oxobutan-2-yl, 1-Methoxy-1-oxopropan-2-yl, 3-Ethoxy-3-oxopropyl, 1-Cyanpropan-2-yl, Propan-2-yloxy, 2-Ethoxyethyl, 3-methoxypropyl, 2-(trifluormethoxy)ethyl, 1,3-dioxolan-2-ylmethyl,
- R⁴⁰¹: steht für -NHR¹²,
wobei die übrigen Substituenten eine oder mehrere der oben genannten Bedeutungen haben,
sowie die agrochemisch wirksamen Salzen davon.

Weiterhin insbesondere bevorzugt werden Verbindungen der Formel [I-b], in denen
- X¹: für C-H steht,
wobei die übrigen Substituenten eine oder mehrere der oben genannten Bedeutungen haben,
sowie die agrochemisch wirksamen Salzen davon.

Weiterhin insbesondere bevorzugt werden Verbindungen der Formel **[I-b]**, in denen
- R¹: die gleiche Bedeutung hat wie die allgemeinen oder in Vorzugsbereichen aufgeführten Restedefinition von R¹ bei den Verbindungen der Formel **[I-a],**
wobei die übrigen Substituenten eine oder mehrere der oben genannten Bedeutungen haben, sowie die agrochemisch wirksamen Salzen davon.

Weiterhin insbesondere bevorzugt werden Verbindungen der Formel **[I-b],** in denen
- R²: die gleiche Bedeutung hat wie die allgemeinen oder in Vorzugsbereichen aufgeführten Restedefinition von R² bei den Verbindungen der Formel **[I-a],**
wobei die übrigen Substituenten eine oder mehrere der oben genannten Bedeutungen haben, sowie die agrochemisch wirksamen Salzen davon.

Weiterhin insbesondere bevorzugt werden Verbindungen der Formel **[I-b],** in denen
- R³⁰¹: für Methyl, Ethyl, 1-Propyl, Propan-2-yl, Isobutyl, Butan-2-yl, 2-Methylpropyl, Prop-2-in-1-yl, Cyclopropyl, Cyclobutyl, Cyclopentyl, (2,2-Dichlorcyclopropyl)methyl, 2-Cyanethyl, 2-Chlorethyl, Cyclopropylmethyl, 2,2-Difluorethyl, 2,2,2-Trifluorethyl, 2-Fluorbenzyl, 2-(Trifluormethoxy)ethyl, 1-Methoxypropan-2-yl, 1-Cyclopropylethyl, 1-Cyanpropan-2-yl, Propan-2-yloxy, 2-Ethoxyethyl, 3-methoxypropyl, 2-(trifluormethoxy)ethyl, 1,3-dioxolan-2-ylmethyl steht,
wobei die übrigen Substituenten eine oder mehrere der oben genannten Bedeutungen haben,
sowie die agrochemisch wirksamen Salzen davon.

Weiterhin insbesondere bevorzugt werden Verbindungen der Formel **[I-b],** in denen
- R⁴⁰¹: für -NH-COR¹⁵ steht,
wobei die übrigen Substituenten eine oder mehrere der oben genannten Bedeutungen haben,
sowie die agrochemisch wirksamen Salzen davon.

Weiterhin insbesondere bevorzugt werden Verbindungen der Formel **[I-b],** in denen
- R¹⁵: für C₁-C₆-Alkyl oder C₃-C₆-Cycloalkyl steht,
wobei die übrigen Substituenten eine oder mehrere der oben genannten Bedeutungen haben, sowie die agrochemisch wirksamen Salzen davon.

Weiterhin insbesondere bevorzugt werden Verbindungen der Formel **[I-b],** in denen
- R¹: die gleiche Bedeutung hatwie die allgemeinen oder in Vorzugsbereichen auf-geführten Restedefinitionen von R¹ bei den Verbindungen der Formel **[I-a],**
- R²: die gleiche Bedeutung hat wie die allgemeinen oder in Vorzugsbereichen auf-geführten Restedefinitionen von R² bei den Verbindungen der Formel **[I-a],**
- R⁵ und R⁶: die gleiche Bedeutung haben wie die allgemeinen oder in Vorzugsbereichen auf-geführten Restedefinitionen von R⁵ und R⁶ bei den Verbindungen der Formel **[I-a],**
- R⁷: die gleiche Bedeutung hat wie die allgemeinen oder in Vorzugsbereichen aufgeführten Restedefinition von R⁷ bei den Verbindungen der Formel **[I-a],**
- R¹⁹: die gleiche Bedeutung hat wie die allgemeinen oder in Vorzugsbereichen aufgeführten Restedefinition von R¹⁹ bei den Verbindungen der Formel **[I-a],**
- R²⁰ und R²¹: die gleiche Bedeutung haben wie die allgemeinen oder in Vorzugsbereichen auf-geführten Restedefinitionen von R²⁰ und R²¹ bei den Verbindungen der Formel **[I-a],**
wobei die übrigen Substituenten eine oder mehrere der oben genannten Bedeutungen haben, sowie die agrochemisch wirksamen Salzen davon.

Die zuvor genannten Reste-Definitionen können untereinander in beliebiger Weise kombiniert werden. Außerdem können einzelne Definitionen entfallen.

Die oben aufgeführten allgemeinen oder in Vorzugsbereichen aufgeführten Restedefinitionen bzw. Erläuterungen können jedoch auch untereinander, also zwischen den jeweiligen Bereichen und Vorzugsbereichen beliebig kombiniert werden. Sie gelten für die Endprodukte sowie für die Vor- und Zwischenprodukte entsprechend. Außerdem können einzelne Definitionen entfallen.

*Bevorzugt* sind solche Verbindungen der Formel **[I-b],** in welcher alle Reste jeweils die oben genannten bevorzugten Bedeutungen haben.

*Besonders bevorzugt* sind solche Verbindungen der Formel **[I-b],** in welcher alle Reste jeweils die oben genannten besonders bevorzugten Bedeutungen haben.

*Ganz besonders bevorzugt* sind solche Verbindungen der Formel **[I-b],** in welcher alle Reste jeweils die oben genannten ganz besonders bevorzugten Bedeutungen haben.

*Insbesondere bevorzugt* sind solche Verbindungen der Formel **[I-b],** in welcher alle Reste jeweils die oben genannten insbesondere bevorzugten Bedeutungen haben.

Die erfindungsgemäßen Verbindungen der Formeln **[I-a]** und **[I-b]** können gegebenenfalls als Mischungen verschiedener möglicher isomerer Formen, insbesondere von Stereoisomeren, wie z. B. E- und Z-, threo- und erythro-, sowie optischen Isomeren, gegebenenfalls aber auch von Tautomeren vorliegen. Es werden sowohl die E- als auch die Z-Isomeren, wie auch die threo- und erythro-, sowie die optischen Isomeren, beliebige Mischungen dieser Isomeren, sowie die möglichen tautomeren Formen beansprucht.

Gegebenenfalls substituierte Gruppen können einfach oder mehrfach substituiert sein, wobei bei Mehrfachsubstitutionen die Substituenten gleich oder verschieden sein können.

Je nach Art der oben definierten Substituenten weisen die Verbindungen der Formel **(I)** saure oder basische Eigenschaften auf und können mit anorganischen oder organischen Säuren oder mit Basen oder mit Metallionen Salze, gegebenenfalls auch innere Salze oder Addukte bilden. Tragen die Verbindungen der Formel **(I)** Amino, Alkylamino oder andere, basische Eigenschaften induzierende Gruppen, so können diese Verbindungen mit Säuren zu Salzen umgesetzt werden oder fallen durch die Synthese direkt als Salze an. Tragen die Verbindungen der Formel **(I)** Hydroxy, Carboxy oder andere, saure Eigenschaften induzierende Gruppen, so können diese Verbindungen mit Basen zu Salzen umgesetzt werden. Geeignete Basen sind beispielsweise Hydroxide, Carbonate, Hydrogencarbonate der Alkali- und Erdalkalimetalle, insbesondere die von Natrium, Kalium, Magnesium und Calcium, weiterhin Ammoniak, primäre, sekundäre und tertiäre Amine mit C₁-C₄-Alkyl-Gruppen, Mono-, Di- und Trialkanolamine von C₁-C₄-Alkanolen, Cholin sowie Chlorcholin.

Beispiele für anorganische Säuren sind Halogenwasserstoffsäuren wie Fluorwasserstoff, Chlorwasserstoff, Bromwasserstoff und Iodwasserstoff, Schwefelsäure, Phosphorsäure und Salpetersäure und saure Salze wie NaHSO₄ und KHSO₄. Als organische Säuren kommen beispielsweise Ameisensäure, Kohlensäure und Alkansäuren wie Essigsäure, Trifluoressigsäure, Trichloressigsäure und Propionsäure sowie Glycolsäure, Thiocyansäure, Milchsäure, Bernsteinsäure, Zitronensäure, Benzoesäure, Zimtsäure, Oxal-säure, gesättigte oder einfach oder doppelt ungesättigte C₆-C₂₀-Fettsäuren, gesättigte oder einfach oder doppelt ungesättigte C₆-C₂₀-Alkylendicarbonsäuren, Alkylschwefelsäuremonoester, Alkylsulfonsäuren (Sulfonsäuren mit geradkettigen oder verzweigten Alkylresten mit 1 bis 20 Kohlenstoffatomen), Arylsulfonsäuren oder Aryldisulfonsäuren (aromatische Reste wie Phenyl und Naphthyl welche ein oder zwei Sulfonsäuregruppen tragen), Alkylphosphon-säuren (Phosphonsäuren mit geradkettigen oder verzweigten Alkylresten mit 1 bis 20 Kohlenstoffatomen), Arylphosphonsäuren oder Aryldiphosphonsäuren (aromatische Reste wie Phenyl und Naphthyl welche ein oder zwei Phosphonsäurereste tragen), wobei die Alkyl- bzw. Arylreste weitere Substituenten tragen können, z.B. p-Toluolsulfonsäure, Salicylsäure, p-Aminosalicylsäure, 2-Phenoxybenzoesäure, 2-Acetoxybenzoesäure etc..

Als Metallionen kommen insbesondere die Ionen der Elemente der zweiten Hauptgruppe, insbesondere Calzium und Magnesium, der dritten und vierten Hauptgruppe, insbesondere Aluminium, Zinn und Blei, sowie der ersten bis achten Nebengruppe, insbesondere Chrom, Mangan, Eisen, Kobalt, Nickel, Kupfer, Zink und andere in Betracht. Besonders bevorzugt sind die Metallionen der Elemente der vierten Periode. Die Metalle können dabei in den verschiedenen ihnen zukommenden Wertigkeiten vorliegen.

Die so erhältlichen Salze weisen ebenfalls fungizide und mykotoxinreduzierende Eigenschaften auf.

Bei den in den vorstehenden Formeln angegebenen Definitionen der Symbole wurden Sammelbegriffe verwendet, die allgemein repräsentativ für die folgenden Substituenten stehen:
Alkyl: gesättigte, geradkettige oder verzweigte Kohlenwasserstoffreste mit 1 bis 8 Kohlenstoffatomen, z.B. (aber nicht beschränkt auf) C₁-C₆-Alkyl wie Methyl, Ethyl, Propyl, 1-Methylethyl, Butyl, 1-Methyl-propyl, 2-Methylpropyl, 1,1-Dimethylethyl, Pentyl, 1-Methylbutyl, 2-Methylbutyl, 3-Methylbutyl, 2,2-Dimethylpropyl, 1-Ethylpropyl, Hexyl, 1,1-Dimethylpropyl, 1,2-Dimethylpropyl,1-Methylpentyl, 2-Methylpentyl, 3-Methylpentyl, 4-Methylpentyl, 1,1-Dimethylbutyl, 1,2-Dimethylbutyl, 1,3-Dimethylbutyl, 2,2-Dimethylbutyl, 2,3-Dimethylbutyl, 3,3-Dimethylbutyl, 1-Ethylbutyl, 2-Ethylbutyl, 1,1,2-Trimethylpropyl, 1,2,2-Trimethylpropyl, 1-Ethyl-1-methylpropyl und 1-Ethyl-2-methylpropyl. Vorzugsweise steht Alkyl für gesättigte, geradkettige oder verzweigte Kohlenwasserstoffreste mit 1 bis 6 und bevorzugt 1 bis 4 Kohlenstoffatomen.
Halogenalkyl: geradkettige oder verzweigte Alkylgruppen mit 1 bis 8 (bevorzugt 1 bis 6 und noch bevorzugter 1 bis 4) Kohlenstoffatomen (wie vorstehend genannt), wobei in diesen Gruppen teilweise oder vollständig die Wasserstoffatome durch
Halogenatome wie vorstehend genannt ersetzt sein können, z.B. (aber nicht beschränkt auf) C₁-C₃-Halogenalkyl wie Chlormethyl, Brommethyl, Dichlormethyl, Trichlormethyl, Fluormethyl, Difluormethyl, Trifluormethyl, Chlorfluormethyl, Dichlorfluormethyl, Chlordifluormethyl, 1-Chlorethyl, 1-Bromethyl, 1-Fluorethyl, 2-Fluorethyl, 2,2-Difluorethyl, 2,2,2-Trifluorethyl, 2-Chlor-2-fluorethyl, 2-Chlor-2-difluorethyl, 2,2-Dichlor-2-fluorethyl, 2,2,2-Trichlorethyl, Pentafluorethyl und 1,1,1-Trifluorprop-2-yl;
Cycloalkyl: monocyclische, gesättigte Kohlenwasserstoffgruppen mit 3 bis 8 (bevorzugt 3 bis 6) Kohlenstoffringgliedern, z.B. (aber nicht beschränkt auf) Cyclopropyl, Cyclopentyl und Cyclohexyl; Halogencycloalkyl: monocyclische, gesättigte Kohlenwasserstoffgruppen mit 3 bis 8 (bevorzugt 3 bis 6) Kohlenstoffringgliedern (wie vorstehend genannt), wobei in diesen Gruppen teilweise oder vollständig die Wasserstoffatome durch Halogenatome wie vorstehend genannt ersetzt sein können, z.B. (aber nicht beschränkt auf) 2-Fluorcyclopropyl, 2,2-Difluorcyclopropyl, 3,3-Difluorcyclobutyl, 2-Fluorcyclopentyl, 3-Fluorcyclopentyl;
Heterocyclyl: drei- bis fünfzehngliedriger vorzugsweise drei- bis neungliedriger gesättigter oder partiell ungesättigter Heterocyclus, enthaltend ein bis vier Heteroatome aus der Gruppe Sauerstoff, Stickstoff oder Schwefel: mono-, bi- oder tricyclische Heterocyclen enthaltend neben Kohlenstoffringgliedern ein bis drei Stickstoffatome und/oder ein Sauerstoff- oder Schwefelatom oder ein oder zwei Sauerstoff- und/oder Schwefelatome; enthält der Ring mehrere Sauerstoffatome, so stehen diese nicht direkt benachbart; wie z.B. (aber nicht beschränkt auf) Oxiranyl, Aziridinyl, 2-Tetrahydrofuranyl, 3-Tetrahydrofuranyl, 2-Tetrahydrothienyl, 3-Tetrahydrothienyl, 2-Pyrrolidinyl, 3-Pyrrolidinyl, 3-Isoxazolidinyl, 4-Isoxazolidinyl, 5-Isoxazolidinyl, 3-Isothiazolidinyl, 4-Isothiazolidinyl, 5-Isothiazolidinyl, 3-Pyrazolidinyl, 4-Pyrazolidinyl, 5-Pyrazolidinyl, 2-Oxazolidinyl, 4-Oxazolidinyl, 5-Oxazolidinyl, 2-Thiazolidinyl, 4-thiazolidinyl, 5-Thiazolidinyl, 2-Imidazolidinyl, 4-Imidazolidinyl, 1,2,4-Oxadiazolidin-3-yl, 1,2,4-Oxadiazolidin-5-yl, 1,2,4-Thiadiazolidin-3-yl, 1,2,4-Thiadiazolidin-5-yl, 1,2,4-Triazolidin-3-yl, 1,3,4-Oxadiazolidin-2-yl, 1,3,4-Thiadiazolidin-2-yl, 1,3,4-Triazolidin-2-yl, 2,3-Dihydrofur-2-yl, 2,3-Dihydrofur-3-yl, 2,4-Dihydrofur-2-yl, 2,4-Dihydrofur-3-yl, 2,3-Dihydrothien-2-yl, 2,3-Dihydrothien-3-yl, 2,4-Dihydrothien-2-yl, 2,4-Dihydrothien-3-yl, 2-Pyrrolin-2-yl, 2-Pyrrolin-3-yl, 3-Pyrrolin-2-yl, 3-Pyrrolin-3-yl, 2-Isoxazolin-3-yl, 3-Isoxazolin-3-yl, 4-Isoxazolin-3-yl, 2-Isoxazolin-4-yl, 3-Isoxazolin-4-yl, 4-Isoxazolin-4-yl, 2-Isoxazolin-5-yl, 3-Isoxazolin-5-yl, 4-Isoxazolin-5-yl, 2-Isothiazolin-3-yl, 3-Isothiazolin-3-yl, 4-Isothiazolin-3-yl, 2-Isothiazolin-4-yl, 3-Isothiazolin-4-yl, 4-Isothiazolin-4-yl, 2-Isothiazolin-5-5-yl, 3-Isothiazolin-5-yl, 4-Isothiazolin-5-yl, 2,3-Dihydropyrazol-1-yl, 2,3-Dihydropyrazol-2-yl, 2,3-Dihydropyrazol-3-yl, 2,3-Dihydropyrazol-4-yl, 2,3-Dihydropyrazol-5-yl, 3,4-Dihydropyrazol-1-yl, 3,4-Dihydropyrazol-3-yl, 3,4-Dihydropyrazol-4-yl, 3,4-Dihydropyrazol-5-yl, 4,5-Dihydroopyrazol-1-yl, 4,5-Dihydropyrazol-3-yl, 4,5-Dihydropyrazol-4-yl, 4,5-Dihydropyrazol-5-yl, 2,3-Dihydrooxazol-2-yl, 2,3-Dihydrooxazol-3-yl, 2,3-Dihydrooxazol-4-yl, 2,3-Dihydrooxazol-5-yl, 3,4-Dihydrooxazol-2-yl, 3,4-Dihydrooxazol-3-yl, 3,4-Dihydrooxazol-4-yl, 3,4-Dihydrooxazol-5-yl, 3,4-Dihydrooxazol-2-yl, 3,4-Dihydrooxazol-3-yl, 3,4-Dihydrooxazol-4-yl, 2-Piperidinyl, 3-Piperidinyl, 4-Piperidinyl, 1,3-Dioxan-5-yl, 2-Tetrahydropyranyl, 4-Tetrahydropyranyl, 2-Tetrahydrothienyl, 3-Hexahydro-pyridazinyl, 4-Hexahydropyridazinyl, 2-Hexahydropyrimidinyl, 4-Hexahydropyrimidinyl, 5-Hexahydropyrimidinyl, 2-Piperazinyl, 1,3,5-Hexahydro-triazin-2-yl und 1,2,4-Hexahydrotriazin-3-yl;
OxoHeterocyclyl: drei- bis fünfzehngliedriger vorzugsweise drei- bis neungliedriger gesättigter oder partiell ungesättigter Heterocyclus, (wie vorstehend genannt), wobei in diesen Gruppen die Wasserstoffatome einer oder mehrerer CH₂ Gruppen durch eine oder mehrere Carbonylgruppen ersetzt sein können, z.B. (aber nicht beschränkt auf) 2-Oxooxetan-3-yl, 5-Oxotetrahydrofuran-3-yl, 2-Oxotetrahydrofuran-3-yl, 2,5-Dioxotetrahydrofuran-3-yl, 5-Oxo-2,5-dihydrofuran-3-yl, 2-Oxo-2,5-dihydrofuran-3-yl, 5-Oxopyrrolidin-3-yl, 2-Oxopyrrolidin-3-yl, 5-Oxopyrrolidin-2-yl), 3-Oxopyrrolidin-2-yl und 4-Oxo-3,4-dihydro-2H-pyran-5-yl;
Alkenyl: ungesättigte, geradkettige oder verzweigte Kohlenwasserstoffreste mit 2 bis 8 (vorzugsweise 2 bis 6) Kohlenstoffatomen und einer Doppelbindung in einer beliebigen Position, z.B. (aber nicht beschränkt auf) C2-C6-Alkenyl wie Ethenyl, 1-Propenyl, 2-Propenyl, 1-Methylethenyl, 1-Butenyl, 2-Butenyl, 3-Butenyl, 1-Methyl-1-propenyl, 2-Methyl-1-propenyl, 1-Methyl-2-propenyl, 2-Methyl-2-propenyl, 1-Pentenyl, 2-Pentenyl, 3-Pentenyl, 4-Pentenyl, 1-Methyl-1-butenyl, 2-Methyl-1-butenyl, 3-Methyl-1-butenyl, 1-Methyl-2-butenyl, 2-Methyl-2-butenyl, 3-Methyl-2-butenyl, 1-Methyl-3-butenyl, 2-Methyl-3-butenyl, 3-Methyl-3-butenyl, 1,1-Dimethyl-2-propenyl, 1,2-Dimethyl-1-propenyl, 1,2-Dimethyl-2-propenyl, 1-Ethyl-1-propenyl, 1-Ethyl-2-propenyl, 1-Hexenyl, 2-Hexenyl, 3-Hexenyl, 4-Hexenyl, 5-Hexenyl, 1-Methyl-1-pentenyl, 2-Methyl-1-pentenyl, 3-Methyl-1-pentenyl, 4-Methyl-1-pentenyl, 1-Methyl-2-pentenyl, 2-Methyl-2-pentenyl, 3-Methyl-2-pentenyl, 4-Methyl-2-pentenyl, 1-Methyl-3-pentenyl, 2-Methyl-3-pentenyl, 3-Methyl-3-pentenyl, 4-Methyl-3-pentenyl, 1-Methyl-4-pentenyl, 2-Methyl-4-pentenyl, 3-Methyl-4-pentenyl, 4-Methyl-4-pentenyl, 1,1-Dimethyl-2-butenyl, 1,1-Dimethyl-3-butenyl, 1,2-Dimethyl-1-butenyl, 1,2-Dimethyl-2-butenyl, 1,2-Dimethyl-3-butenyl, 1,3-Dimethyl-1-butenyl, 1,3-Dimethyl-2-butenyl, 1,3-Dimethyl-3-butenyl, 2,2-Dimethyl-3-butenyl, 2,3-Dimethyl-1-butenyl, 2,3-Dimethyl-2-butenyl, 2,3-Dimethyl-3-butenyl, 3,3-Dimethyl-1-butenyl, 3,3-Dimethyl-2-butenyl, 1-Ethyl-1-butenyl, 1-Ethyl-2-butenyl, 1-Ethyl-3-butenyl, 2-Ethyl-1-butenyl, 2-Ethyl-2-butenyl, 2-Ethyl-3-butenyl, 1,1,2-Trimethyl-2-propenyl, 1-Ethyl-1-methyl-2-propenyl, 1-Ethyl-2-methyl-1-propenyl und 1-Ethyl-2-methyl-2-propenyl;
Alkinyl: geradkettige oder verzweigte Kohlenwasserstoffgruppen mit 2 bis 8 (vorzugsweise 2 bis 6) Kohlenstoffatomen und einer Dreifachbindung in einer beliebigen Position, z.B. (aber nicht beschränkt auf) C₂-C₆-Alkinyl wie Ethinyl, 1-Propinyl, 2-Propinyl, 1-Butinyl, 2-Butinyl, 3-Butinyl, 1-Methyl-2-propinyl, 1-Pentinyl, 2-Pentinyl, 3-Pentinyl, 4-Pentinyl, 1-Methyl-2-butinyl, 1-Methyl-3-butinyl, 2-Methyl-3-butinyl, 3-Methyl-1-butinyl, 1,1-Dimethyl-2-propinyl, 1-Ethyl-2-propinyl, 1-Hexinyl, 2-Hexinyl, 3-Hexinyl, 4-Hexinyl, 5-Hexinyl, 1-Methyl-2-pentinyl, 1-Methyl-3-pentinyl, 1-Methyl-4-pentinyl, 2-Methyl-3-pentinyl, 2-Methyl-4-pentinyl, 3-Methyl-1-pentinyl, 3-Methyl-4-pentinyl, 4-Methyl-1-pentinyl, 4-Methyl-2-pentinyl, 1,1-Dimethyl-2-butinyl, 1,1-Dimethyl-3-butinyl, 1,2-Dimethyl-3-butinyl, 2,2-Dimethyl-3-butinyl, 3,3-Dimethyl-1-butinyl, 1-Ethyl-2-butinyl, 1-Ethyl-3-butinyl, 2-Ethyl-3-butinyl und 1-Ethyl-1-methyl-2-propinyl;
Aryl: 6- bis 14-gliedriges, vollständig ungesättigtes carbocyclisches Ringsystem, z.B. (aber nicht beschränkt auf) Phenyl, 1-Naphthyl, 2-Naphthyl, 2-Anthryl, 1-Anthryl;
Heteroaryl: 5 oder 6-gliedriges, vollständig ungesättigtes monocyclisches Ringsystem, enthaltend ein bis vier Heteroatome aus der Gruppe Sauerstoff, Stickstoff oder Schwefel, enthält der Ring mehrere Sauerstoffatome, so stehen diese nicht direkt benachbart;
Alkoxy: einen geradkettigen oder verzweigten Alkoxyrest, bevorzugt C₁-C₆ Alkoxyrest und besonders bevorzugt einen C₁-C₃ Alkoxyrest, wie z.B. (aber nicht beschränkt auf) Methoxy, Ethoxy, n-Propoxy, 1-Methylethoxy, n-Butoxy, 1-Methylpropoxy, 2-Methylpropoxy oder 1,1-Dimethylethoxy, insbesondere Methoxy oder Ethoxy;
Alkylthio: steht für geradkettiges oder verzweigtes Alkylthio z.B. (aber nicht beschränkt auf) Methylthio, Ethylthio, n- und i-Propylthio, n-, i-, sek.- und tert.-Butylthio, n-Pentylthio und seine Isomeren wie 1-, 2-und 3-Methyl-butylthio. Die Alkylthiogruppen können durch 1 bis 3 Halogenatome (vorzugsweise Chlor und/oder Fluor) substituiert sein, z.B. (aber nicht beschränkt auf) Di- und Trifluormethylthio sowie Difluorchlormethylthio.
Halogenalkoxy: steht für einen geradkettigen oder verzweigten Alkoxyrest in dem ein oder mehrere Wasserstoffatome durch Fluor, Chlor oder Brom ersetzt wurden, z.B. (aber nicht beschränkt auf) -OCF₃,-OCHF₂. Bevorzugt wird eine ein bis dreifach Substitution durch Fluor oder Chlor.
Acyloxy: steht für einen über das Sauerstoffatom verbundenen geradkettigen, verzweigten, cyclischen, gesättigten oder ungesättigten Acyloxy rest, z.B. (aber nicht beschränkt auf) Acetyloxy, Propionyloxy, Isobutyryloxy.
Heteroalkyl: gesättigte oder ungesättigte, geradkettige oder verzweigte Kohlenwasserstoffreste mit 2 bis 10 (vorzugsweise 2 bis 8) Kohlenstoffatomen und mindestens einem Heteroatom, wobei nicht zwei Heteroatome direkt benachbart sein dürfen.

Nicht umfasst sind solche Kombinationen, die den Naturgesetzen widersprechen und die der Fachmann daher aufgrund seines Fachwissens ausgeschlossen hätte. Beispielsweise sind Ringstrukturen mit drei oder mehreren benachbarten O-Atomen ausgeschlossen.

### Erläuterung der Verfahren und Zwischenprodukte

Die erfindungsgemäßen Phenylpyri(mi)dinylazole der Formeln **[I-a]** und **[I-b]** lassen sich auf unterschiedliche Weise herstellen. Zum Zwecke der Verfahrensbeschreibung werden die Verbindungen der Formeln [I-a] und [I-b] unter der Formel [I] zusammengefasst, weil die erfindungsgemäßen Verfahren auf beide Formeln angewendet werden können. Im Folgenden sind die möglichen Verfahren zunächst schematisch dargestellt. Wenn nicht anders angegeben, haben die angegebenen Reste die oben angegebenen Bedeutungen.

Die erfindungsgemäßen Phenylpyri(mi)dinylazole der Formel **[I]** lassen sich nach Verfahren A gemäß folgendem Schema herstellen. 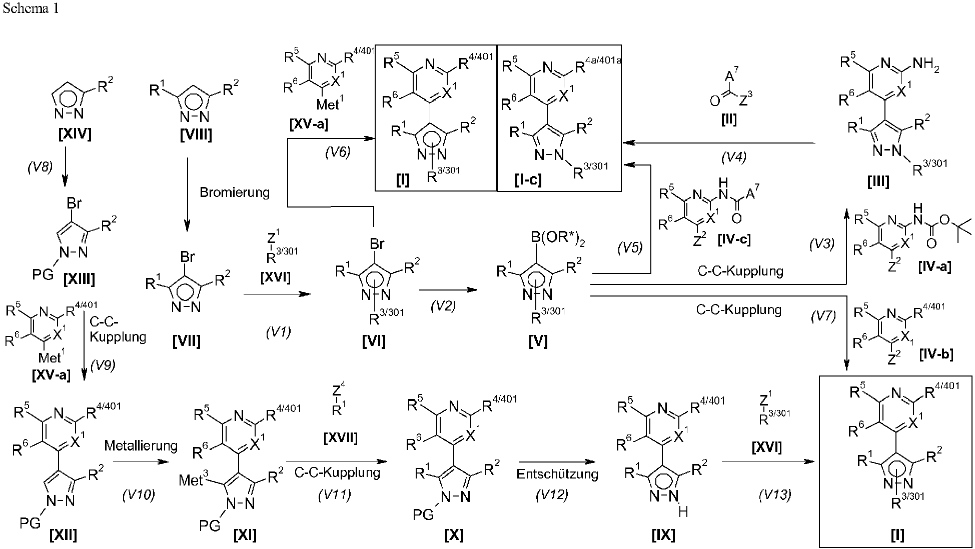
Met¹= z.B. -Sn(Bu)₃, -B(OR*)₂
Met³= z.B. -Sn(Bu)₃, 4,4,5,5-Tetramethyl-1,3,2-dioxaborolan-2-yl
B(OR*)₂= z.B. -B(OiPr)₂, -B(OH)₂
Z¹= z.B. Cl, Br, I, -OTos, -OMs, -OH
Z²= z.B. Cl, Br
Z³= z.B. Cl, -OH
Z⁴= z.B. I, Cl, Br, -OMs, -OTos
A⁷ = R¹⁵, -OR¹⁵
R^{4a/401a}= z.B. -NH-C(S)R¹⁵, -NH-C(O)R¹⁵, -NH C(O)OR¹⁵, -NH-C(O)NR¹⁵R¹⁶,
R^{4b/401b} = z.B. Wasserstoff, Alkyl, Cycloalkyl, Aryl, -NH-C(O)-Alkyl oder -NH-C(O)O-Alkyl
PG = z.B. Tetrahydro-2H-pyran-2-yl, 2-(trimethylsilyl)ethoxy]methyl

Außerdem lassen sich die erfindungsgemäßen Phenylpyri(mi)dinylazole der Formel [I-d] auch durch Verfahren B herstellen (Schema 2)

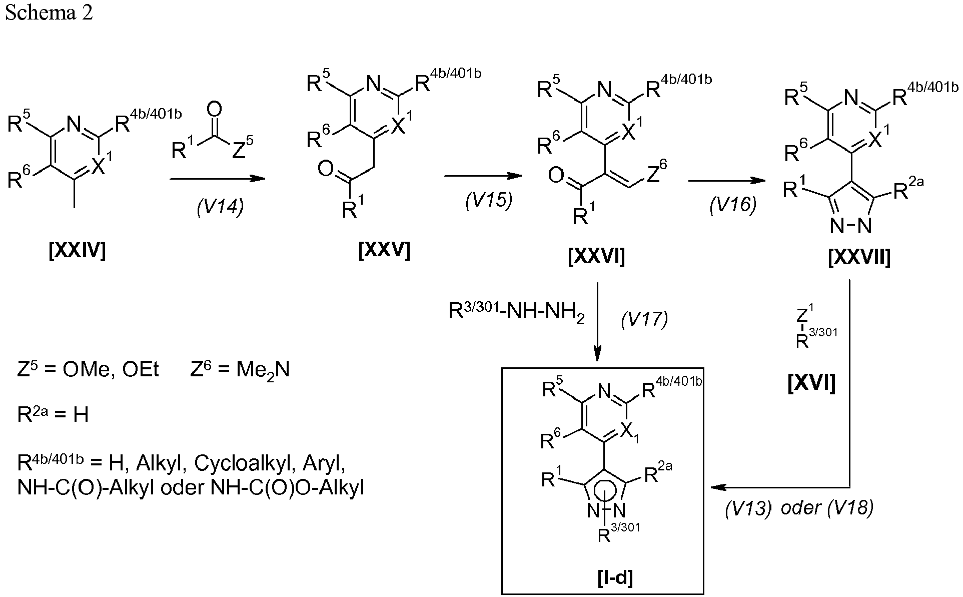

Alternativ lassen sich die erfindungsgemäßen Arylpyrazole der Formel **[I-e]** und Intermediate der Formel **[IX-b]** auch durch Verfahren C herstellen (Schema 3).

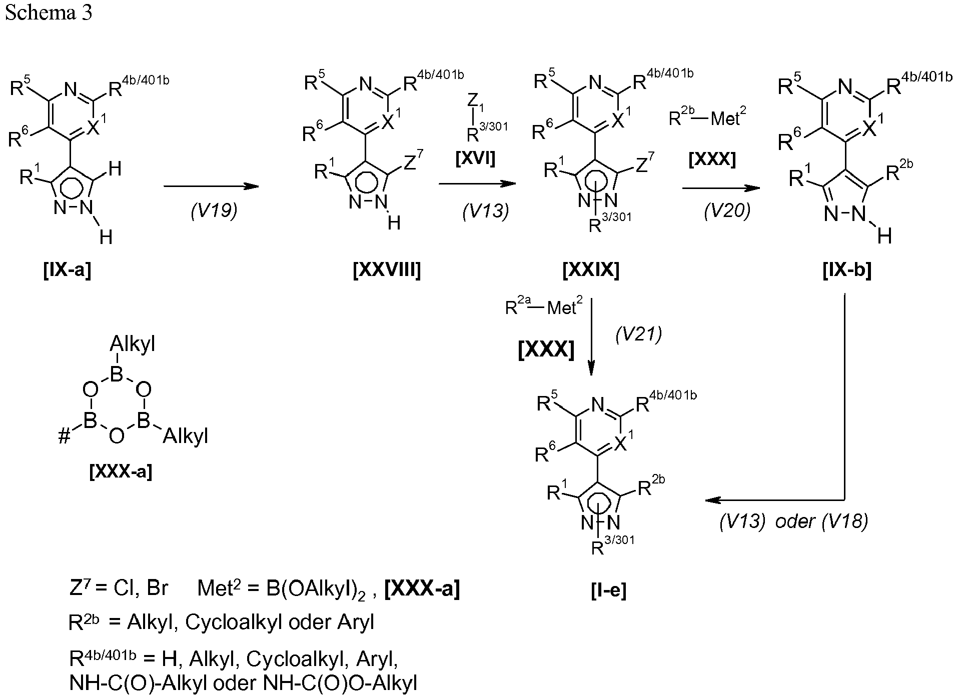

Alternativ lassen sich die Intermediate der Formel **[VI-a]** auch durch Verfahren D herstellen (Schema 4).

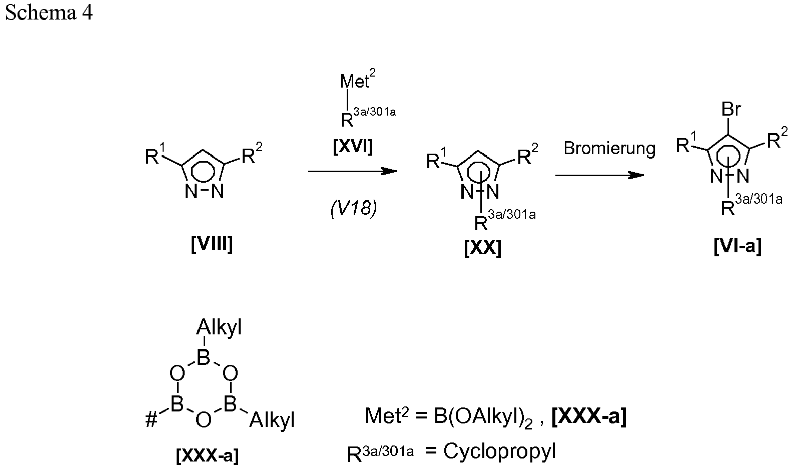

Alternativ lassen sich die Intermediate der Formel **[VI-b]** sowie Intermediate der Formel **[VI-c]** auch durch Verfahren E herstellen (Schema 5).

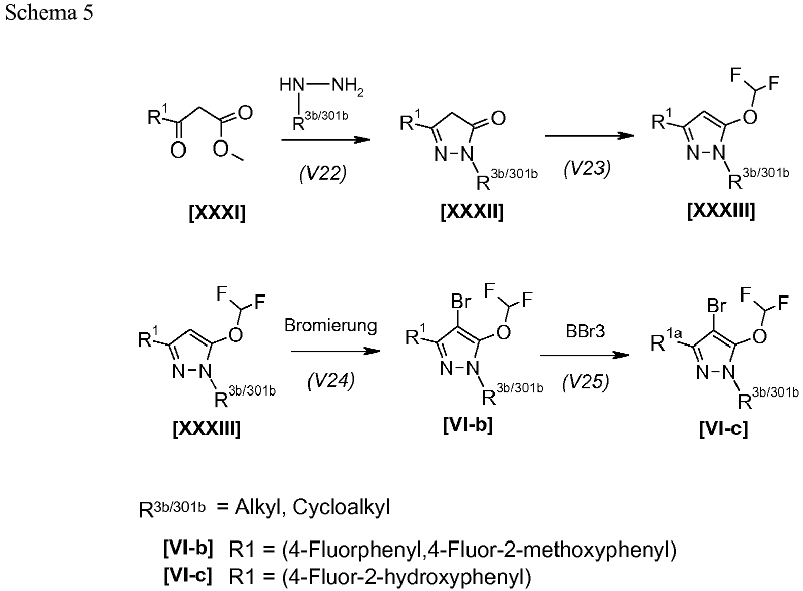

Alternativ lassen sich die Intermediate der Formel **[III]** auch durch Verfahren F herstellen (Schema 6).

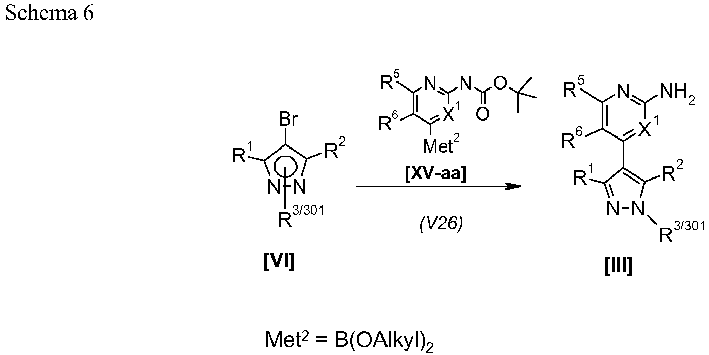

Intermediate des Typs [XV-a] lassen sich durch Verfahren G herstellen (Schema 7).

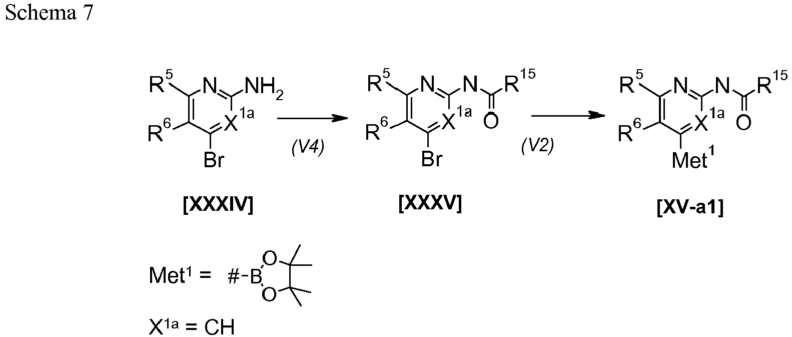

Außerdem lassen sich die erfindungsgemäßen Phenylpyri(mi)dinylazole der Formel **[I-f]** und **[I-g]** auch durch Verfahren H herstellen (Schema 8) 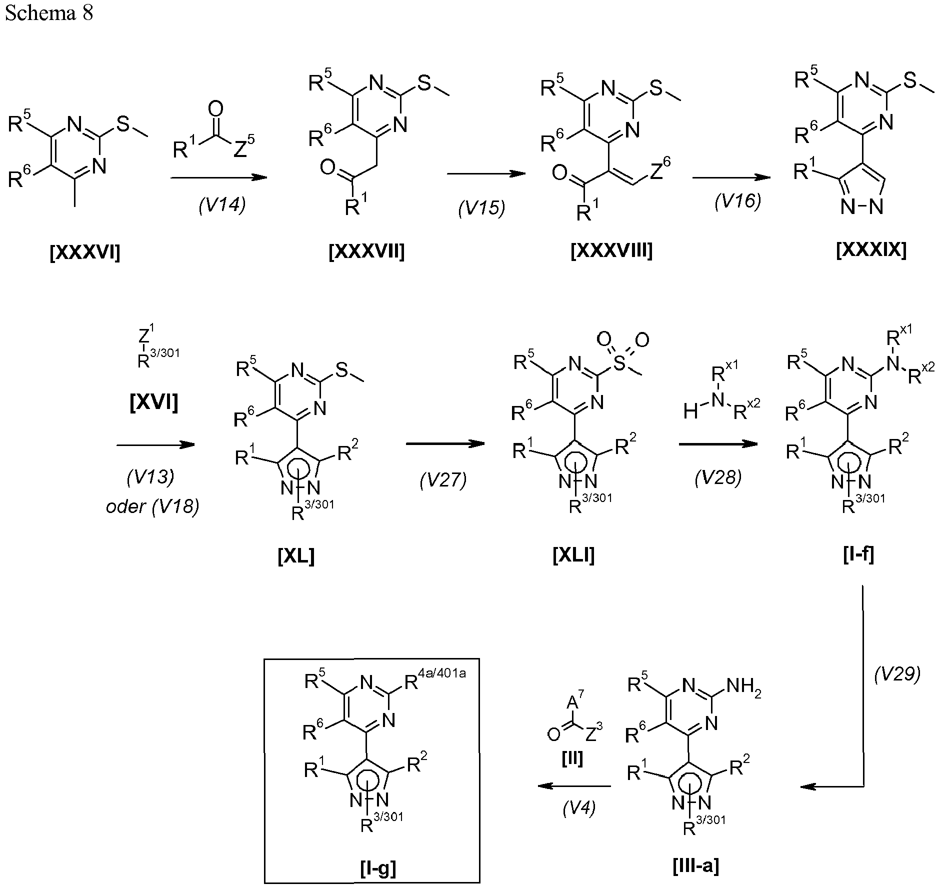
Z¹= z.B. Cl, Br, I, -OTos, OMs, -OH
Z³= z.B. Cl
Z⁵ = -OMe, -OEt
Z⁶ = Me₂N
R^{4a/401a}= z.B. -NH-C(S)R¹⁵, -NH-C(O)R¹⁵, -NH C(O)OR¹⁵, -NH-C(O)NR¹⁵R¹⁶,
R^{x1} = z.B. Wasserstoff, Alkyl, Cycloalkyl, Benzyl
R^{x2} = z.B. Alkyl, Cycloalkyl, Benzyl
A⁷ = -R¹⁵, -OR¹⁵

Außerdem lassen sich die erfindungsgemäßen Phenylpyri(mi)dinylazole der Formel [I-h] durch Verfahren I herstellen (Schema 9) 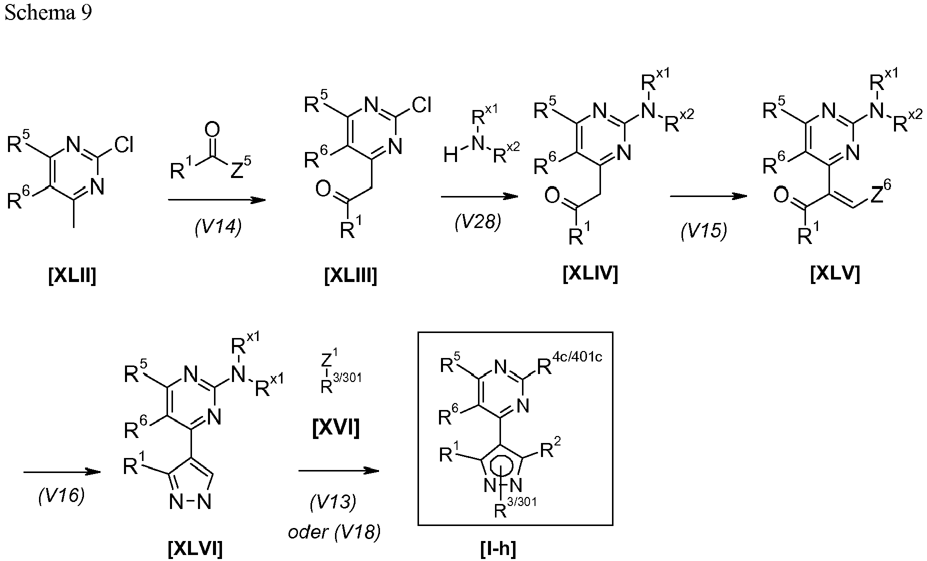
Z¹= z.B. Cl, Br, I, -OTos, OMs, -OH
Z⁵ = -OMe, -OEt
Z⁶ = Me₂N
R^{4c/401c} = z.B. -NR^{12a}R^{13a}, -NHR^{13a}, -NHR^{14a}
R^{x1} = z.B. Wasserstoff, Alkyl, Cycloalkyl, Benzyl, Heterocyclyl
R^{x2}= z.B. Alkyl, Cycloalkyl, Benzyl, Heterocyclyl

Neu sind Verbindungen der Formel **[III]** 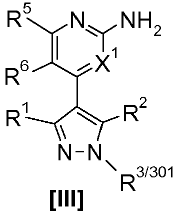 in denen die Symbole R¹, R², R^{3/301}, R⁵, R⁶ und X¹ die oben angegebenen allgemeinen, bevorzugten, besonders bevorzugten, ganz besonders bevorzugten, am meisten bevorzugten oder insbesondere bevorzugten Bedeutungen, sowie Salze davon, haben,

Neu sind zum Beispiel die in der folgenden Tabelle aufgeführten Verbindungen des Typs **[III]** :

| **Nr.** | **Name** | **R¹** | **R²** | **R^{3/301}** | **LogP (pH 2,3)¹** | **[M+H]⁺ Peak²** |
|---|---|---|---|---|---|---|
| **[III-1]** | 4-[3-(4-Fluorphenyl)-1-isopropyl-1H-pyrazol-4-yl]pyridin-2-amin | 4-fluorphenyl | H | Isopropyl | 1.22 | 297.13 |
| **[III-2]** | 4-[3-(4-Fluorphenyl)-1-(2-methoxyethyl)-1H-pyrazol-4-yl]pyridin-2-amin | 4-fluorphenyl | H | 2-Methoxyethyl | 0.98 | 313.15 |
| **[III-3]** | 4-[1-Ethyl-3-(4-fluorphenyl)-1H-pyrazol-4-yl]pyridin-2-amin | 4-fluorphenyl | H | Ethyl | 0.97 | 283.20 |
| **[III-4]** | 4-[1-(2,2-Difluorethyl)-3-(4-fluorphenyl)-1H-pyrazol-4-yl]pyridin-2-amin | 4-fluorphenyl | H | 2,2-Difluorethyl | 1.03 | 319.48 |
| **[III-5]** | 4-[3-(4-Fluorphenyl)-1-methyl-1H-pyrazol-4-yl]pyridin-2-amin | 4-fluorphenyl | H | Methyl | 0.71 | 269.2 |

in denen
X¹ für = C-H und
R⁵, R⁶ für H steht.

Ausgenommen sind die Verbindungen 4-[3-(4-fluorophenyl)-5-methyl-1H-pyrazol-4-yl]pyridin-2-amine; 4-[3-(4-chlorophenyl)-5-methyl-1H-pyrazol-4-yl]pyridin-2-amine, 4-[3-(4-methoxyphenyl)-5-methyl-1 H-pyrazol-4-yl]pyridin-2-amine, 4-[3-(4-fluorophenyl)-1H-pyrazol-4-yl]pyrimidin-2-amine; 4-(5-methyl-3-phenyl-1H-pyrazol-4-yl)pyrimidin-2-amine und [4-(2-aminopyrimidin-4-yl)-3-(3-chloro-5-hydroxyphenyl)-1 H-pyrazol-1-yl] acetonitrile.

Neu sind auch Verbindungen der Formel **[V]** 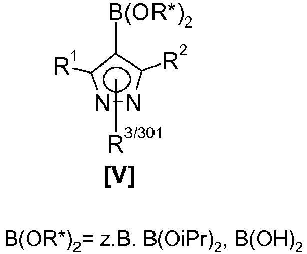 in denen die Symbole R², R^{3/301} die oben angegebenen allgemeinen, bevorzugten, besonders bevorzugten, ganz besonders bevorzugten oder insbesondere bevorzugten Bedeutungen haben,
R¹ hat die oben angegebenen bevorzugten, besonders bevorzugten, ganz besonders bevorzugten, am meisten bevorzugten oder insbesondere bevorzugten Bedeutungen, sowie Salze davon,

Neu sind zum Beispiel die in der folgenden Tabelle aufgeführten Verbindungen des Typs **[V]:**

| **Nr.** | **Name** | **R¹** | **R²** | **R^{3/301}** | **LogP (pH 2,3)¹** | **[M+H]⁺ Peak²** |
|---|---|---|---|---|---|---|
| **[V-1]** | 3-(4-Fluorphenyl)-1-isopropyl-4-(4,4,5,5-tetramethyl-1,3,2-dioxaborolan-2-yl)-1H-pyrazol | 4-fluorphenyl | H | Isopropyl | 4.46 | 331.2 |
| **[V-2]** | 3-(4-Fluorphenyl)-1-(2-methoxyethyl)-4-(4,4,5,5-tetramethyl-1,3,2-dioxaborolan-2-yl)-1H-pyrazol | 4-fluorphenyl | H | 2-methoxyethyl | 3.58 | 347.2 |
| **[V-3]** | 3 -(4-Fluorphenyl)-1-isobutyl-4-(4,4,5,5-tetramethyl-1,3,2-dioxaborolan-2-yl)-1H-pyrazol | 4-fluorphenyl | H | Isobutyl | 4.89 | 345.2 |
| **[V-4]** | 1-(2,2-Difluorethyl)-3-(4-fluorphenyl)-4-(4,4,5,5-tetramethyl-1,3,2-dioxaborolan-2-yl)-1H-pyrazol | 4-fluorphenyl | H | 2,2-Difluorethyl | 3.84 | 353.2 |
| **[V-5]** | 3-(4-Fluorphenyl)-1-isopropoxy-4-(4,4,5,5-tetramethyl-1,3,2-dioxaborolan-2-yl)-1H-pyrazol | 4-fluorphenyl | H | Isopropoxy | 4.81 | 347.2 |
| **[V-6]** | 1-(Cyclopentyloxy)-3-(4-fluorphenyl)-4-(4,4,5,5-tetramethyl-1,3,2-dioxaborolan-2-yl)-1H-pyrazol | 4-fluorphenyl | H | Cyclopentyloxy | 5.51 | 373.2 |
| **[V-7]** | 1-Cyclopropyl-3-(4-fluorphenyl)-4-(4,4,5,5-tetramethyl-1,3,2-dioxaborolan-2-yl)-1H-pyrazol | 4-fluorphenyl | H | Cyclopropyl | 3.47 | 329.2 |
| **[V-8]** | 1-(Cyclopropylmethyl)-3-(4-fluorphenyl)-4-(4,4,5,5-tetramethyl-1,3,2-dioxaborolan-2-yl)-1H-pyrazol | 4-fluorphenyl | H | Cyclopropylmethyl | 4.42 | 343.2 |
| **[V-9]** | 3 -(4-Fluorphenyl)-1-methyl-4-(4,4,5,5-tetramethyl-1,3,2-dioxaborolan-2-yl)-1H-pyrazol | 4-fluorphenyl | H | Methyl | 3.47 | 303.2 |
| **[V-10]** | 1-(2-Chlorethyl)-3-(4-fluorphenyl)-4-(4,4,5,5-tetramethyl-1,3,2-dioxaborolan-2-yl)-1H-pyrazol | 4-fluorphenyl | H | 2-Chlorethyl | 4.06 | 351.1 |

Ausgenommen ist die Verbindung 1-methyl-3-phenyl-4-(4,4,5,5-tetramethyl-1,3,2-dioxaborolan-2-yl)-1H-pyrazole.

Neu sind Verbindungen der Formel **[VI]** 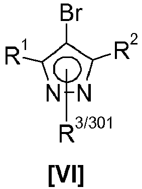 in denen R¹ die oben angegebenen besonders bevorzugten, ganz besonders bevorzugten, am meisten bevorzugten oder insbesondere bevorzugten Bedeutungen hat,
R² und R^{3/301} haben die oben angegebenen bevorzugten, besonders bevorzugten, ganz besonders bevorzugten, am meisten bevorzugten oder insbesondere bevorzugten Bedeutungen hat,
sowie Salze davon.

Neu sind zum Beispiel die in der folgenden Tabelle aufgeführten Verbindungen des Typs **[VI]:**

| **Nr.** | **Name** | **R¹** | **R²** | **R^{3/301}** | **LogP (pH 2,3)¹** | **[M+H]⁺ Peak²** |
|---|---|---|---|---|---|---|
| **[VI-2]** | 4-Brom-1-ethyl-3-(4-fluorphenyl)-1H-pyrazol | 4-fluorphenyl | H | C2H5 | 3.32 | 269.0 |
| **[VI-4]** | 4-Brom-3-(4-fluorphenyl)-1-isobutyl-1H-pyrazol | 4-fluorphenyl | H | isobutyl | 4.34 | 297.0 |
| **[VI-6]** | 4-Brom-3-(4-fluorphenyl)-1-(2-methoxyethyl)-1H-pyrazol | 4-fluorphenyl | H | 2-Methoxyethyl | 3.08 | 299.0 |
| **[VI-9]** | 4-Brom-3-(4-fluorphenyl)-1-[1-(2-fluorphenyl)ethyl]-1H-pyrazol | 4-fluorphenyl | H | 1-(2-Fluorphenyl)ethyl | 4.77 | 363.0 |
| **[VI-10]** | 4-Brom-1-[(2,2-dichlorcyclopropyl)methyl]-3-(4-fluorphenyl)-1H-pyrazol | 4-fluorphenyl | H | (2,2-dichlorcyclopropyl)methyl | 4.43 | 364.9 |
| **[VI-11]** | 5-(4-Brom-1-isobutyl-1H-pyrazol-3-yl)-2-fluorbenzonitril | 3-Cyan-4-fluorphenyl | H | Isobutyl | 4.15 | 324.1 |
| **[VI-12]** | 3-{[4-Brom-3-(4-fluorphenyl)-1H-pyrazol-1-yl]methyl}benzonitril | 4-fluorphenyl | H | 3-Cyanbenzyl | 3.93 | 358.0 |
| **[VI-13]** | 4-Brom-1-(2-fluorbenzyl)-3-(4-fluorphenyl)-1H-pyrazol | 4-fluorphenyl | H | 2-Fluorbenzyl | 4.39 | 349.0 |
| **[VI-14]** | 4-Brom-3-(4-fluorphenyl)-1-propyl-1H-pyrazol | 4-fluorphenyl | H | 1-Propyl | 3.89 | 283.0 |
| **[VI-15]** | 4-Brom-3-(4-fluorphenyl)-1-[2-(methylsulfanyl)ethyl]-1H-pyrazol | 4-fluorphenyl | H | 2-(methylsulfanyl)ethyl | 3.63 | 315.0 |
| **[VI-16]** | Methyl-2-[4-brom-3-(4-fluorphenyl)-1H-pyrazol-1-yl]-3-methylbutanoat | 4-fluorphenyl | H | 1-Methoxy-3-methyl-1-oxobutan-2-yl | 4.27 | 357.1 |
| **[VI-17]** | 4-Brom-1-(1,3-dioxolan-2-ylmethyl)-3-(4-fluorphenyl)-1H-pyrazol | 4-fluorphenyl | H | 1,3-Dioxolan-2-ylmethyl | 3.01 | 329.0 |
| **[VI-18]** | 4-Brom-1-(cyclopropylmethyl)-3-(4-fluorphenyl)-1H-pyrazol | 4-fluorphenyl | H | Cyclopropylmethyl | 3.90 | 297.0 |
| **[VI-19]** | 4-Brom-1-sec-butyl-3-(4-fluorphenyl)-1H-pyrazol | 4-fluorphenyl | H | sec-Butyl | 4.39 | 299.0 |
| **[VI-20]** | 4-Brom-1-(2-ethoxyethyl)-3-(4-fluorphenyl)-1H-pyrazol | 4-fluorphenyl | H | 2-Ethoxyethyl | 3.51 | 313.0 |
| **[VI-21]** | 3-[4-Brom-3-(4-fluorphenyl)-1H-pyrazol-1-yl]butanonitril | 4-fluorphenyl | H | 1-Cyanpropan-2-yl | 3.08 | 310.1 |
| **[VI-22]** | 4-Brom-1-(1-cyclopropylethyl)-3-(4-fluorphenyl)-1H-pyrazol | 4-fluorphenyl | H | 1-cyclopropylethyl | 4.43 | 309.0 |
| **[VI-23]** | 3-[4-Brom-3-(4-fluorphenyl)-1H-pyrazol-1-yl]propanonitril | 4-fluorphenyl | H | 2-Cyanethyl | 2.69 | 296.0 |
| **[VI-24]** | 4-Brom-3-(4-fluorphenyl)-1-isopropyl-5-(trifluormethyl)-1H-pyrazol | 4-fluorphenyl | CF₃ | Isopropyl | 5.51 | 353.1 |
| **[VI-25]** | 4-Brom-3-(4-fluorphenyl)-1-isopropoxy-1H-pyrazol | 4-fluorphenyl | H | Isopropoxy | 4.11 | 301.1 |
| **[VI-26]** | 4-Brom-1-cyclopropyl-3-(4-fluorphenyl)-1H-pyrazol | 4-fluorphenyl | H | Cyclopropyl | 3.59 | 281.0 |
| **[VI-27]** | tert-Butyl-4-[4-brom-3-(4-fluorphenyl)-1H-pyrazol-1-yl]piperidin-1-carboxylat | 4-fluorphenyl | H | 1-(tert-Butoxycarbonyl)piperidin-4-yl | 4.77 | 368 [M+-C4H9]+ |
| **[VI-b-1]** | 4-Brom-5-(difluormethoxy)-3-(4-fluorphenyl)-1-methyl-1H-pyrazol | 4-fluorphenyl | CHF₂ | Methyl | 3.52 | 321.1 |
| **[VI-b-2]** | 4-Brom-5-(difluormethoxy)-3-(4-fluorphenyl)-1-isopropyl-1H-pyrazol | 4-fluorphenyl | CHF₂ | Isopropyl | 4.61 | 351.0 |
| **[VI-b-3]** | 4-Brom-5-(difluormethoxy)-3-(4-fluorphenyl)-1-isobutyl-1H-pyrazol | 4-fluorphenyl | CHF₂ | Isobutyl | 4.91 | 365.0 |
| **[VI-c-1]** | 2-[4-Brom-5-(difluormethoxy)-1-methyl-1H-pyrazol-3-yl]-5-fluorphenol | 4-fluorphenyl | CHF₂ | Methyl | 3.48 | 336.9 |

Ausgenommen sind Verbindungen bei denen R^{3/301} = H, CH₃ oder C(CH₃)₃ ist.

Neu sind Verbindungen der Formel [X] 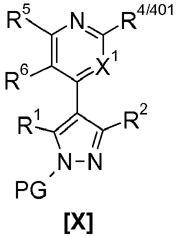 in denen R², R^{4/401}, R⁵, R⁶ und X¹ die oben angegebenen allgemeinen, bevorzugten, besonders bevorzugten, ganz besonders bevorzugten, am meisten bevorzugten oder insbesondere bevorzugten Bedeutungen hat.

Neu sind zum Beispiel Verbindungen:
in denen
R², R^{4/401}, R⁵, R⁶ für H,
X¹ für = C-H und
PG für Tetrahydro-2H-pyran-2-yl steht

| **Nr.** | **Name** | **R¹** | **LogP (pH 2,3)¹** | **[M+H]⁺ Peak²** |
|---|---|---|---|---|
| **[X-1]** | 4-[5-(4-Fluorphenyl)-1-(tetrahydro-2H-pyran-2-yl)-1H-pyrazol-4-yl]pyridin | 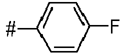 | 1.23 | 324.18 |
| **[X-2]** | 4-[5-(4-Methoxy-1-naphthyl)-1-(tetrahydro-2H-pyran-2-yl)-1H-pyrazol-4-yl]pyridin | 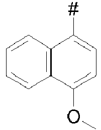 | 1.61 | 386.04 |
| **[X-3]** | 4-[5-(4-Fluor-1-naphthyl)-1-(tetrahydro-2H-pyran-2-yl)-1H-pyrazol-4-yl]pyridin | 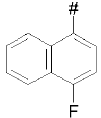 | 1.72 | 374.01 |
| **[X-4]** | 4-[5-(2,2-Difluor-1,3-benzodioxol-4-yl)-1-(tetrahydro-2H-pyran-2-yl)-1H-pyrazol-4-yl]pyridin | 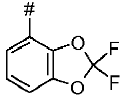 | 1.64 | 386.10 |
| **[X-5]** | 4-[5-(4-Fluor-2-methylphenyl)-1-(tetrahydro-2H-pyran-2-yl)-1H-pyrazol-4-yl]pyridin | 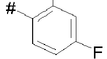 | 1.40 | 338.05 |
| **[X-6]** | 4-[5-(3-Chlor-4-fluorphenyl)-1-(tetrahydro-2H-pyran-2-yl)-1H-pyrazol-4-yl]pyridin | 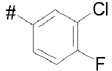 | 1.57 | 357.95 |
| **[X-7]** | 4-[5-(4-Fluor-3-methylphenyl)-1-(tetrahydro-2H-pyran-2-yl)-1H-pyrazol-4-yl]pyridin | 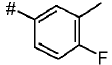 | 1.47 | 338.05 |
| **[X-8]** | 4-[5-(2,4-Difluorphenyl)-1-(tetrahydro-2H-pyran-2-yl)-1H-pyrazol-4-yl]pyridin | 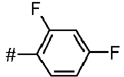 | 1.35 | 342.04 |
| **[X-9]** | 4-[5-(4-tert-Butylphenyl)-1-(tetrahydro-2H-pyran-2-yl)-1H-pyrazol-4-yl]pyridin | 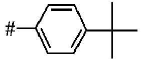 | 2.02 | 362.17 |
| **[X-10]** | 4-[5-(4-Phenoxyphenyl)-1-(tetrahydro-2H-pyran-2-yl)-1H-pyrazol-4-yl]pyridin | 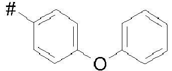 | 1.88 | 398.13 |
| **[X-11]** | 3-[4-(Pyridin-4-yl)-1-(tetrahydro-2H-pyran-2-yl)-1H-pyrazol-5-yl]benzonitril | 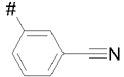 | 1.23 | 331.06 |
| **[X-12]** | 2-Fluor-5-[4-(pyridin-4-yl)-1-(tetrahydro-2H-pyran-2-yl)-1H-pyrazol-5-yl]benzonitril | 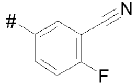 | 1.45 | 349.01 |
| **[X-13]** | N-{4-[4-(Pyridin-4-yl)-1-(tetrahydro-2H-pyran-2-yl)-1H-pyrazol-5-yl]phenyl}acetamid | 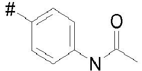 | 0.97 | 363.11 |
| **[X-14]** | 4-[5-(4-Fluor-2-methoxyphenyl)-1-(tetrahydro-2H-pyran-2-yl)-1H-pyrazol-4-yl]pyridin | 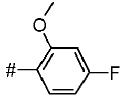 | 1.26 | 354.05 |
| **[X-15]** | 4-{4-[4-(Pyridin-4-yl)-1-(tetrahydro-2H-pyran-2-yl)-1H-pyrazol-5-yl]phenyl}morpholin | 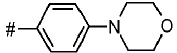 | 1.26 | 391.14 |
| **[X-16]** | 4-[5-(3-Phenoxyphenyl)-1-(tetrahydro-2H-pyran-2-yl)-1H-pyrazol-4-yl]pyridin | 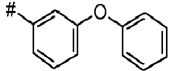 | 1.88 | 398.13 |
| **[X-17]** | 4-{5-[4-(Methylsulfonyl)phenyl]-1-(tetrahydro-2H-pyran-2-yl)-1H-pyrazol-4-yl}pyridin | 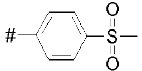 | 0.96 | 384.02 |
| **[X-18]** | 4-[5-(4-Chlorphenyl)-1-(tetrahydro-2H-pyran-2-yl)-1H-pyrazol-4-yl]pyridin | 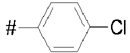 | 1.52 | 340.15 |
| **[X-19]** | 4-{1-(Tetrahydro-2H-pyran-2-yl)-5-[4-(trifluormethoxy)phenyl]-1H-pyrazol-4-yl}pyridin | 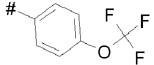 | 1.78 | 390.07 |
| **[X-20]** | 4-[5-(2,3-Dichlorphenyl)-1-(tetrahydro-2H-pyran-2-yl)-1H-pyrazol-4-yl]pyridin | 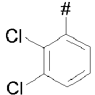 | 1.66 | 374.07 |
| **[X-21]** | N,N-Dimethyl-3-[4-(pyridin-4-yl)-1-(tetrahydro-2H-pyran-2-yl)-1H-pyrazol-5-yl]anilin | 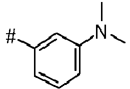 | 1.33 | 349.20 |
| **[X-22]** | 4-{5-[2-(Benzyloxy)phenyl]-1-(tetrahydro-2H-pyran-2-yl)-1H-pyrazol-4-yl}pyridin | 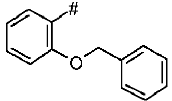 | 1.64 | 412.17 |
| **[X-23]** | 4-[4-(Pyridin-4-yl)-1-(tetrahydro-2H-pyran-2-yl)-1H-pyrazol-5-yl]benzolsulfonamid | 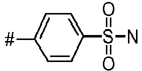 | 0.92 | 385.03 |
| **[X-24]** | 8-[4-(Pyridin-4-yl)-1-(tetrahydro-2H-pyran-2-yl)-1H-pyrazol-5-yl]chinolin | 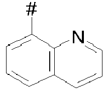 | 1.09 | 357.13 |

| | | | | |
|---|---|---|---|---|
| ¹ Bei der Bestimmung der logP Werte wurden die unten beschriebenen Methoden angewendet. ² Bei der angegebenen Masse handelt es sich um den Peak des Isotopenmusters des [M+H]⁺ Ions mit der höchsten Intensität; falls das [M-H]⁻ Ion detektiert wurde, ist die Massenangabe mit 2 gekennzeichnet. | | | | |

Neu sind Verbindungen der Formel **[XI]** 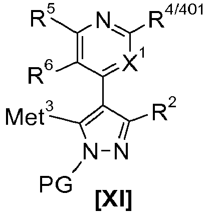 in denen die Symbole R², R^{4/401}, R⁵, R⁶ und X¹ die oben angegebenen allgemeinen, bevorzugten, besonders bevorzugten, ganz besonders bevorzugten, am meisten bevorzugten, oder insbesondere bevorzugten Bedeutungen haben, PG steht für eine Schutzgruppe, wie z.B. Tetrahydro-2H-pyran-2-yl oder 2-(trimethylsilyl)ethoxy]methyl, Met³ steht für ein substituiertes Metallatom, wie z.B. Tributylstannyl, 4,4,5,5-Tetramethyl-1,3,2-dioxaborolan-2-yl, sowie Salze davon,
zum Beispiel [**XI**-**1**]**:**

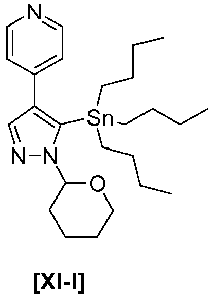

Ausgenommen ist die Verbindung 1-({4-[1-(2,2-Difluorethyl)-3-(trimethylstannyl)-1H-pyrazol-4-yl]pyrimidin-2-yl}amino)propan-2-ol.

Die Herstellung der Verbindungen mit der allgemeinen Formel **[I]** nach Verfahren A kann wie folgt durchgeführt werden:
Eine Verbindung mit der allgemeinen Formel **[XIV]** wird bromiert und anschließend mit einer Schutzgruppe versehen, um eine Verbindung mit der Formel **[XIII]** zu erhalten. Diese Verbindung kann in einer C-C-Kupplungsreaktion mit einem Substrat der Formel **[XV-a]** umgesetzt werden, wobei eine Verbindung mit der Formel **[XII]** entsteht. Diese Verbindung kann durch Umsetzung mit einer starken Base und anschließender Umsetzung mit einer Bor- oder Zinnverbindung zu einer Verbindung der Formel **[XI]** umgewandelt werden. Diese Verbindung wird in einer C-C-Kupplungsreaktion mit Substraten der allgemeinen Formel **[XVII]** zu Verbindungen der Formel **[X]** umgesetzt. Anschließend wird diese Verbindung entschützt, wobei eine Verbindung der allgemeinen Formel [**IX**] erhalten wird. Das erhaltene Pyrazol der Formel [**IX**] wird nun mit Substraten des Typs **[XVI]** umgesetzt, wobei die erfindungsgemäßen Arylpyrazole der Formel **[I]** erhalten werden (Schema 1).

Alternativ kann auch ein anderer Verfahrensweg gewählt werden. Eine Verbindung mit der allgemeinen Formel **[VIII]** wird bromiert und eine Verbindung der Formel **[VII]** wird erhalten. Diese wird durch Umsetzung mit Substraten des Typs **[XVI]** zu einer Verbindung des Typs **[VI]** umgewandelt, wobei Gemische von Pyrazol-Regioisomeren entstehen können. Diese können durch gängige Verfahren z.B. chromatografische Verfahren in die einzelnen Regioisomere aufgetrennt werden. Die Verbindungen der allgemeinen Formel **[VI]** können in einer C-C-Kupplung mit Substraten der Formel **[XV-a]** umgesetzt werden, wobei Verbindungen der Formel **[I]** erhalten werden (Schema 1).

Alternativ können die Pyrazolverbindungen der allgemeinen Formel **[VI]** durch Umsetzung mit einem Boronsäureester in Verbindungen des Typs **[V]** umgewandelt werden. Diese können durch Umsetzung mit einem Substrat der Formel **[IV-c]** in einer C-C-Kupplungsreaktion zu Verbindungen der Formel **[I-c]** umgewandelt werden (Schema 1).

Alternativ können Verbindungen des Typs **[V]** durch Umsetzung mit einem Substrat der Formel **[IV-a]** in einer C-C-Kupplungsreaktion zu Verbindungen der Formel **[III]** umgewandelt werden. Diese Verbindungen werden durch Umsetzung mit Substraten der Formel **[II]** ebenfalls in die Verbindungen des Typs **[I-c]** überführt.

Darüberhinaus können Verbindungen des Typs **[V]** durch Umsetzung mit einem Substrat der Formel **[IV-b]** in einer C-C-Kupplungsreaktion zu den erfindungsgemäßen Arylpyrazolen der Formel **[I]** umgesetzt werden (Schema 1).

Die Herstellung der Verbindungen mit der allgemeinen Formel **[I-d]** wobei R^{4b/401b} für Wasserstoff, Alkyl, Cycloalkyl, Aryl, -NH-C(O)-Alkyl oder -NH-C(O)O-Alkyl steht, kann wie folgt nach Verfahren B durchgeführt werden:
Verbindungen der allgemeinen Formel **[XXIV]** sind entweder kommerziell verfügbar oder können nach literaturbekannten Methoden dargestellt werden.

Verbindungen der allgemeinen Formel **[XXIV]** werden mit einem Carbonsäureester, -nitril, - dialkylamid oder -N,O-dialkylamid der generellen Formel R¹-COZ⁵ umgesetzt, wobei Verbindungen der allgemeinen Formel **[XXV]** erhalten werden. Diese Verbindungen **[XXV]** werden durch Reaktion mit DMF-Dialkylacetal zu Verbindungen der allgemeinen Formel **[XXVI]** umgesetzt. Aus Verbindungen der allgemeinen Formel **[XXVI]** werden dann durch Reaktion mit Hydrazin oder Hydrazinhydrat Verbindungen der Formel **[XXVII]** erhalten. Die erhaltenen Pyrazole der Formel **[XXVII]** werden nun mit Substraten des Typs **[XVI]** umgesetzt, wobei die erfindungsgemäßen Arylpyrazole der Formel **[I-d]** erhalten werden.

Im Falle R^{3/301}=Cyclopropyl kann eine Verbindung der Formel **[I-d]** auch durch C-C-Kupplungsreaktion eines Substrats der Formel **[XXVII]** mit einer Cyclopropylboronsäure erhalten werden

Verbindungen der allgemeinen Formel **[I-d]** können auch durch direkte Umsetzung eines Hydrazinderivates mit Substraten der Formel **[XXVI]** erhalten werden.

Die Herstellung der Verbindungen mit der allgemeinen Formel **[I-e]** kann wie folgt nach Verfahren C durchgeführt werden:
Verbindungen der allgemeinen Formel **[IX-a]** sind entweder kommerziell verfügbar oder können nach Verfahren A dargestellt werden. Die Verbindungen der Formel **[IX-a]** werden durch Halogenierungsreaktion zu Verbindungen der Formel **[XXVIII]** umgesetzt. Die erhaltenen Pyrazole der Formel **[XXVIII]** werden nun mit Substraten des Typs **[XVI]** umgesetzt, wobei Verbindungen der Formel **[XXIX]** erhalten werden. Diese Verbindungen können durch C-C-Kupplungsreaktion mit einem Boronsäurederivat der Formel **[XXX]** und anschließender Entschützungsreaktion durch Abspaltung des R^{3/301}-Rests (z.B. bei Verwendung des p-Methoxybenzylrests) in Intermediate der Formel **[IX-b]** überführt werden. Diese können durch die in Verfahren A und B beschriebenen Methoden am Stickstoffatom des Pyrazols funktionalisiert werden, wobei die erfindungsgemäßen Pyrazole der Formel **[I-e]** erhalten werden.

Alternativ können die Intermediate der Formel **[XXIX]** auch ohne Entschützungsschritt direkt durch C-C-Kupplungsreaktion mit einem Boronsäurederivat der Formel **[XXX]** zu den erfindungsgemäßen Pyrazolen der Formel **[I-e]** umgesetzt werden.

Die Herstellung der Intermediate mit der allgemeinen Formel **[VI-a]** kann wie folgt nach Verfahren D durchgeführt werden:
Pyrazolverbindungen der allgemeinen Formel **[VIII]** können durch Umsetzung mit einem Boronsäureester in Verbindungen des Typs **[XX]** umgewandelt werden. Diese Verbindungen werden durch Bromierung in Intermediate der allgemeinen Formel **[VI-a]** umgewandelt.

Die Herstellung der Intermediate mit der allgemeinen Formel **[VI-b]** und **[VI-c]** kann wie folgt nach Verfahren E durchgeführt werden:
Gemäß literaturbekannten Methoden (WO1996/015115, US5928999) werden Pyrazolinone **[XXXII]** ausgehend von den entsprechenden β-Ketoestern **[XXXI]** durch Umsetzung mit Hydrazinen hergestellt. Diese Pyrazolone werden durch Difluormethylierung gemäß literaturbekannten Methoden *(*Org. Lett. 2006, 8, 17, 3805-3808) in Verbindungen des Typs **[XXXIII]** überführt. Die Verbindungen der Formel **[XXXIII]** werden anschliessend durch Halogenierungsreaktion zu Verbindungen der Formel **[VI-b]** umgesetzt. Verbindungen des Typs **[VI-b]** in denen R¹ für 4-Fluor-2-methoxyphenyl steht, können durch Umsetzung mit BBr₃ in Verbindungen des Typs **[VI-c]** umgewandelt werden, wobei R^{1a} für 4-Fluor-2-hydroxyphenyl steht.

Die Herstellung der Intermediate mit der allgemeinen Formel **[III]** kann alternativ auch wie folgt nach Verfahren F durchgeführt werden:
In einer C-C-Kupplungsreaktion werden Intermediate des Typs **[VI]** mit Substraten der allgemeinen Formel **[XV-aa]** umgesetzt, wobei Met² für einen Boronsäureester steht. Im Verlauf der Reaktion wird durch Abspaltung der Aminoschutzgruppe das freie Amin gebildet, wobei die Intermediate der allgemeinen Formel **[III]** erhalten werden.

Die Herstellung der Intermediate mit der allgemeinen Formel **[XV-a]** kann wie folgt nach Verfahren G durchgeführt werden:
Verbindungen der allgemeinen Formel **[XXXIV]** werden durch Acylierungsreaktion zu Verbindungen der Formel **[XXXV]** umgesetzt. Anschliessend werden diese Verbindungen in einer Kupplungsreaktion zu Boronsäureestern der Formel **[XV-al]** überführt.

Die Herstellung der Verbindungen mit der allgemeinen Formel **[I-f]** und **[I-g]** kann wie folgt nach Verfahren H durchgeführt werden:
Verbindungen der allgemeinen Formel **[XXXVI]** werden gemäß literaturbekannten Methoden (J. Med. Chem. 1999, 42, 12, 2180 - 2190) mit einem Carbonsäureester, -nitril, -dialkylamid oder -N,O-dialkylamid der generellen Formel R¹-COZ⁵ umgesetzt, wobei Verbindungen der allgemeinen Formel **[XXXVII]** erhalten werden. Dabei dürfen die Verbindungen der generellen Formel R¹-COZ⁵ keine Gruppen mit aciden Protonen, wie z.B. NH oder OH Gruppen enthalten. Diese Verbindungen **[XXXVII]** werden durch Reaktion mit DMF-Dialkylacetal zu Verbindungen der allgemeinen Formel **[XXXVIII]** umgesetzt. Aus Verbindungen der allgemeinen Formel **[XXXVIII]** werden dann durch Reaktion mit Hydrazin oder Hydrazinhydrat Verbindungen der Formel **[XXIX]** erhalten. Die erhaltenen Pyrazole der Formel **[XXIX]** werden nun mit Substraten des Typs **[XVI]** umgesetzt, wobei Verbindungen der allgemeinen Formel **[XL]** erhalten werden. Diese werden durch Umsetzung mit Oxidationsmitteln, z.B. m-Chlorperbenzoesäure in Verbindungen der Formel **[XLI]** überführt. Durch Substitutionsreaktion in Gegenwart von primären oder sekundären Aminen werden daraus die erfindungsgemäßen Arylpyrazole der Formel **[I-f]** erhalten. Gegebenenfalls können diese Verbindungen durch Entfernung der Aminsubstituenten (z.B. bei Benzylaminen durch Hydrierungsreaktion) in Verbindungen der allgemeinen Formel **[III-a]** überführt werden. Diese Verbindungen **[III-a]** werden durch Umsetzung mit Substraten der Formel **[II]** in die erfindungsgemäßen Arylpyrazole der Formel **[I-g]** überführt.

Die Herstellung der Verbindungen mit der allgemeinen Formel **[I-h]** kann wie folgt nach Verfahren **I** durchgeführt werden:
Verbindungen der allgemeinen Formel **[XLII]** werden gemäß literaturbekannten Methoden (Tetrahedron Lett. 2009, 50, 21, 2552 - 2554) mit einem Carbonsäureester der generellen Formel R¹-COZ⁵ umgesetzt, wobei Verbindungen der allgemeinen Formel **[XLIII]** erhalten werden. Dabei dürfen die Verbindungen der generellen Formel R¹-COZ⁵ keine Gruppen mit aciden Protonen, wie z.B. NH oder OH Gruppen enthalten. Durch Substitutionsreaktion in Gegenwart von primären oder sekundären Aminen werden daraus Verbindungen der Formel **[XLIV]** erhalten. Diese Verbindungen **[XLIV]** werden durch Reaktion mit DMF-Dialkylacetal zu Verbindungen der allgemeinen Formel **[XLV]** umgesetzt. Aus Verbindungen der allgemeinen Formel **[XLV]** werden dann durch Reaktion mit Hydrazin oder Hydrazinhydrat Verbindungen der Formel **[XLVI]** erhalten. Die erhaltenen Pyrazole der Formel **[XLVI]** werden nun mit Substraten des Typs **[XVI]** umgesetzt, wobei die erfindungsgemäßen Verbindungen der allgemeinen Formel **[I-h]** erhalten werden.

### Schritt (V1)

Eine Möglichkeit zur Synthese von Verbindungen der Formel **[VI]** ist in Schema 1 gezeigt.

Verbindungen der Formel **[VI],** in denen R^{3/301} nicht für Wasserstoff steht, können synthetisiert werden, analog zu in der Literatur beschriebenen Vorschriften (Bioorg. Med. Chem. Lett. 2000, 10, 1351-1356 oder J. Am. Chem. Soc. 2007, 129, 26, 8064-8065), durch Umsetzung von Verbindungen des Typs **[VII]** mit einem Substrat der allgemeinen Formel **[XVI]** (wobei Z¹ eine Fluchtgruppe, wie z.B. Cl, Br, I, -OTos, -OMs oder andere darstellt), gegebenenfalls in der Gegenwart eines Lösungsmittels und eines Säurefängers / Base.

Verbindungen der Formel **[VI],** in denen R^{3/301} nicht für Wasserstoff steht, können darüberhinaus analog zu in der Literatur beschriebenen Vorschriften (Mitsunobu, O. Synthesis 1981, 1-28) synthetisiert werden, z.B. durch Umsetzung von Verbindungen des Typs **[VII]** mit einem Substrat der allgemeinen Formel **[XVI]** (wobei Z¹ für -OH steht) in der Gegenwart eines Phosphanes (z.B. Triphenylphosphan) und eines Azodicarboxylates (z.B. Diethylazodicarboxylat) sowie eines Lösungsmittels (z.B. THF).

Die Brom-substituierten Pyrazole der Formel **[VII]** sind entweder kommerziell verfügbar oder lassen sich nach Literaturvorschriften herstellen. Eine Methode zur Herstellung geeigneter Brompyrazole ist beispielsweise die Bromierung entsprechender Pyrazole **[VIII]** (z.B. beschrieben in EP-A 1382 603) durch Umsetzung mit N-Bromsuccinimid in Essigsäure.

Verbindungen des Typs **[VIII],** wie z.B. 3-(4-Fluorphenyl)-1H-pyrazol, 3-(4-Chlorphenyl)-1H-pyrazol oder 3-(3-Chlorphenyl)-1H-pyrazol sind kommerziell verfügbar oder lassen sich z.B. nach literaturbekannten Methoden (Tetrahedron, 2003, 59, 555-560) aus kommerziellen Acetophenonen durch Umsetzung mit Dimethylformamid-Dimethylacetal und Hydrazin herstellen.

Die für die Umsetzung benötigten Verbindungen der Formel **[XVI]** sind kommerziell verfügbar oder lassen sich nach Literaturvorschriften herstellen (R. C. Larock, Comprehensive Organic Transformations, 2. Ausgabe, 1999, Wiley-VCH, S. 690 ff. und S. 1929 ff. sowie darin zitierte Literatur)

Eine Methode zur Herstellung geeigneter Verbindungen der Formel **[XVI]** (wobei R^{3/301} im Falle einer Alkylierungsreaktion z.B. für einen substituierten oder unsubstituierten Alkyl oder Cycloalkylrest steht), ist beispielsweise die Umsetzung von Alkoholen mit Methansulfonylchlorid und Triethylamin (Org. Lett. 2008, 10, 4425-4428) oder durch Appel-Reaktion mit Triphenylphosphin und CCl₄ (z.B. beschrieben in Tetrahedron 2008, 64, 7247-7251).

Die Herstellung geeigneter Verbindungen der Formel **[XVI]** (wobei in R^{3/301} im Falle einer Acylierungsreaktion eine Carbonylgruppe direkt an Z¹ gebunden ist), erfolgt nach literaturbekannten Verfahren (z.B. Jerry March, Advanced Organic Chemistry, 4. Auflage, John Wiley & Sons, S. 437 ff. und der darin zitierten Literatur).

Aus der chemischen Struktur der Substrate der allgemeinen Formel **[XVI]** können sich bestimmte bevozugte Kombinationen bei der Wahl eines geeigneten Lösungsmittel und einer geeigneten Base ergeben.

Im Falle einer Alkylierungsreaktion mit Substraten der Formel **[XVI]** (wobei R^{3/301} im Falle einer Alkylierungsreaktion z.B. für einen substituierten oder unsubstituierten Alkyl oder Cycloalkylrest steht) können alle üblichen, unter den Reaktionsbedingungen inerten Lösungsmittel, wie z.B. zyklische und azyklische Ether (z.B. Diethylether, Tetrahydrofuran, Dioxane), aromatische Kohlenwasserstoffe (z.B. Benzol, Toluol, Xylol), halogenierte Kohlenwasserstoffe (z.B. Dichlormethan, Chloroform, Tetrachlorkohlenstoff), halogenierte aromatische Kohlenwasserstoffe (z.B. Chlorbenzol, Dichlorbenzol), Nitrile (z.B. Acetonitril), Carbonsäureester (z.B. Essigsäureethylester), Amide (z.B. N,N-Dimethylformamid, N,N-Dimethylacetamid), Dimethylsulfoxid, 1,3-Dimethyl-2-imidazolinon, verwendet werden oder die Reaktion kann in Mischungen von zwei oder mehreren dieser Lösungsmittel ausgeführt werden. Die bevorzugten Lösungsmittel sind Dimethylformamid und Acetonitril.

Im Falle einer Alkylierungsreaktion mit Substraten der Formel **[XVI]** (wobei R^{3/301} im Falle einer Alkylierungsreaktion z.B. für einen substituierten oder unsubstituierten Alkyl oder Cycloalkylrest steht) sind Basen, die für diese Reaktion, verwendet werden können, z.B. Lithiumhexamethyldisilazide (LiHMDS), Kaliumcarbonat, Cesiumcarbonat und Natriumhydrid. Die bevorzugte Base ist Natriumhydrid. Man setzt in der Regel wenigstens 1 Äquivalent Base ein.

Im Falle einer Acylierungsreaktion mit Substraten der Formel **[XVI]** (wobei in R^{3/301} eine Carbonylgruppe direkt an Z¹ gebunden ist) können alle üblichen, unter den Reaktionsbedingungen inerten Lösungsmittel, wie z.B. zyklische und azyklische Ether (z.B. Diethylether, Tetrahydrofuran, Dioxane), aromatische Kohlenwasserstoffe (z.B. Benzol, Toluol, Xylol), halogenierte Kohlenwasserstoffe (z.B. Dichlormethan, Chloroform, Tetrachlorkohlenstoff), halogenierte aromatische Kohlenwasserstoffe (z.B. Chlorbenzol, Dichlorbenzol), Nitrile (z.B. Acetonitril) und aromatische heterocyclische Amine (Pyridin) verwendet werden oder die Reaktion kann in Mischungen von zwei oder mehreren dieser Lösungsmittel ausgeführt werden. Die bevorzugten Lösungsmittel sind Tetrahydrofuran und Dichlormethan.

Im Falle einer Acylierungsreaktion mit Substraten der Formel **[XVI]** (wobei in R^{3/301} eine Carbonylgruppe direkt an Z¹ gebunden ist) kann z.B. ein Äquivalent eines Säurefängers / einer Base (z.B. Pyridin, Diisopropylethylamin, Triethylamin oder kommerziell erhältliche polymere Säurefänger) im Verhältnis zum Startmaterial der allgemeinen Formel **[VII]** verwendet werden. Ist das Startmaterial ein Salz, werden wenigstens zwei Äquivalente des Säurefängers benötigt. Im Falle der Verwendung von Pyridin als Lösungsmittel kann analog zur beschriebenen Literatur gegebenenfalls auf den Zusatz einer weiteren Base verzichtet werden (EP-A-1 000 062).

Die Reaktion wird normalerweise bei Temperaturen von 0 °C -100 °C durchgeführt und vorzugsweise bei 20 °C - 30 °C, aber sie kann auch bei Rückflusstemperatur der Reaktionsmischung durchgeführt werden. Die Reaktionszeit variiert in Abhängigkeit vom Maßstab der Reaktion und der Reaktionstemperatur, aber liegt im Allgemeinen zwischen einigen Minuten und 48 Stunden.

Nach Beendigung der Reaktion, werden die Verbindungen **[VI],** von der Reaktionsmischung durch eine der üblichen Trenntechniken getrennt. Je nach der Art der verwendeten Substrate der Formel **[XVI]** und der Reaktionsbedingungen können die Verbindungen der Formel **[VI],** in denen R^{3/301} nicht für Wasserstoff steht, als reine Regioisomere oder als Mischung beider möglicher Regioisomerer (wobei die Gruppe R^{3/301} beide Positionen am N-Atom des Pyrazols einnehmen kann) erhalten werden. In dem Falle, daß Mischungen an Regioisomeren erhalten werden, können diese durch physikalische Methoden (wie z.B. Kristallisations- oder Chromatographie-Methoden) gereinigt oder können gegebenenfalls auch im nächsten Schritt ohne vorhergehende Reinigung eingesetzt werden.

### Schritt (V2)

Eine Möglichkeit zur Synthese von Verbindungen der Formel **[V]** ist in Schema 1 gezeigt.

Verbindungen der Formel **[V]** lassen sich nach beschriebenen Verfahren z.B. über Umsetzung der Brompyrazole **[VI]** mit Boronsäureestern wie z.B. Bispinacolatodiboron (4,4,4',4',5,5,5',5'-Octamethyl-2,2'-bi-1,3,2-dioxaborolan) in Gegenwart eines Katalysators wie z.B. 1,1'-Bis(diphenyl-phosphino)ferrocen-palladium(II)dichlorid in Gegenwart einer Base und eines geeignetes Lösungsmittels herstellen (vgl. US 0,018,156 A, WO 07/024843 oder EP-A-1 382 603).

Als Lösungsmittel können alle üblichen, unter den Reaktionsbedingungen inerten Lösungsmittel, wie z.B. Sulfoxide (z.B. Dimethylsulfoxid), zyklische Ether (z.B. Dioxane) und Amide (z.B. N,N-Dimethylformamid) verwendet werden und die Reaktion kann in Mischungen von zwei oder mehreren dieser Lösungsmittel ausgeführt werden. Die bevorzugten Lösungsmittel sind Dimethylsulfoxid und Dioxan.

Die Reaktion wird normalerweise bei Temperaturen von 80 °C - 120 °C durchgeführt werden, die bevorzugte Reaktionstemperatur liegt bei 85 °C - 90 °C. Die Reaktionszeit variiert in Abhängigkeit vom Maßstab der Reaktion und der Reaktionstemperatur, aber liegt im Allgemeinen zwischen einer Stunde und 16 Stunden.

Andere literaturbeschriebene Synthesemethoden können ebenfalls zur Herstellung der Verbindungen der Formel **[V]** verwendet werden. Beispielsweise können Verbindungen der Formel [V] durch Metallierung der Brompyrazole **[VI]** mit Basen wie z.B. n-Butyllithium und Umsetzung mit Boronsäureestern wie z.B. Trimethylborat und anschließende Reaktion der erhaltenen Pyrazolboronsäure mit Pinacol hergestellt werden (siehe z.B. J. Het. Chem. 2004, 41, 931-940 oder EP-A-1 382 603 sowie WO2007/16392).

### Schritt (V3)

Eine Möglichkeit zur Synthese von Verbindungen der Formel **[III]** ist in Schema 1 gezeigt.

Verbindungen der Formel **[III]** lassen sich z.B. durch Kupplung der Pyrazolboronsäuren **[V]** mit Heterocyclen der Formel **[IV-a]** (wobei Z² eine Fluchtgruppe wie z.B. Cl oder Br darstellt) in Gegenwart eines Katalysators, einer Base und eines geeigneten Lösungsmittels bei geeigneten Temperaturen nach bekannten Literaturvorschriften herstellen *(*Top. Curr. Chem. 2002, 219, 11; Organomet. Chem. 1999, 28, 147 und darin zitierte Literatur).

Verbindungen der Formel **[IV-a]** (in denen X¹ für C-H steht) sind kommerziell verfügbar oder lassen sich nach Literaturvorschriften herstellen (Schema 10). Eine Methode zur Herstellung geeigneter N-Boc-Halogenheterocyclen [**IV**-**a**-**1**] ist die Reaktion geeigneter Säuren (z.B. 4-Brom-picolinsäure) **[L]** mit Diphenylphosphorylazid und tert-Butanol (Aust. J. Chem. 1982, 35, 2025-2034, J. Med. Chem. 1992, 35, 15, 2761-2768 oder US 5,112,837 A).

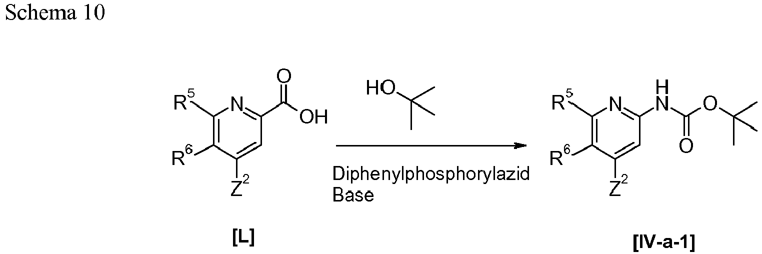

Die Carbonsäuren **[L]** sind bekannt oder können aus kommerziell erhältlichen Vorstufen nach literaturbeschriebenen Vorschriften hergestellt werden (siehe z.B. EP-A-1 650 194), zum Beispiel aus der kommerziell verfügbaren Pyridin-2-carbonsäure durch Umsetzung mit Thionylchlorid in Dimethylformamid. Alternativ können Verbindungen der allgemeinen Formel [L] auch durch Oxidation von kommerziell verfügbaren 4-Halo-2-Methyl-pyridinderivaten nach literaturbekannten Vorschriften hergestellt werden (Aust. J. Chem. 1982, 35, 2025-2034).

Verbindungen der Formel **[IV-a]** (in denen X¹ für N steht) sind kommerziell verfügbar oder lassen sich nach Literaturvorschriften herstellen (Schema 11). Eine Methode zur Herstellung geeigneter N-Boc-Halogenheterocyclen **[IV-a-2]** ist die Chlorierung der Hydroxyverbindungen (z.B. (4-Hydroxy-pyrimidin-2-yl)carbamat) mit Phosphoroxychlorid (Chem. Pharm. Bull. 2003, 51, 8, 975-977).

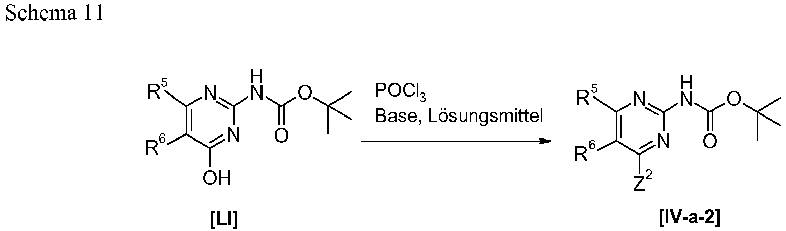

Die Hydroxyverbindungen **[LI]** sind bekannt oder können aus kommerziell erhältlichen Vorstufen nach literaturbeschriebenen Vorschriften hergestellt werden (Chem. Pharm. Bull. 2003, 51, 8, 975-977).

Als Lösungsmittel für die Synthese von Verbindungen der Formel **[III]** können alle üblichen, unter den Reaktionsbedingungen inerten Lösungsmittel, wie z.B. Alkohole (z.B. Methanol, Ethanol, 1-Propanol, 2-Propanol, Ethylenglykol, 1-Butanol, 2-Butanol, tert.-Butanol), zyklische und azyklische Ether (Diethylether, Dimethoxymethan, Diethylenglykoldimethylether, Tetrahydrofuran, Dioxan, Diisopropylether, tert.-Butylmethylether), aromatische Kohlenwasserstoffe (z.B. Benzol, Toluol, Xylol), Kohlenwasserstoffe (z.B. Hexan, iso-Hexan, Heptan, Cyclohexan), Ketone (z.B. Aceton, Ethylmethylketon, iso-Butylmethylketon), Nitrile (z.B. Acetonitril, Propionitril, Butyronitril) und Amide (z.B. Dimethylformamid, Dimethylacetamid, N-Methylpyrrolidon) und Wasser verwendet werden oder die Reaktion kann in Mischungen von zwei oder mehreren dieser Lösungsmittel ausgeführt werden. Das bevorzugte Lösungsmittel ist Dioxan.

Basen, die bei dem erfindungsgemäßen Verfahren vorzugsweise Verwendung finden, sind Alkali- und Erdalkalimetallhydroxide, Alkali- und Erdalkalimetallcarbonate, Alkalimetallhydrogencarbonate, Alkali- und Erdalkalimetallacetate, Alkali- und Erdalkalimetallalkoholate, sowie primäre, sekundäre und tertiäre Amine. Bevorzugte Basen sind Alkalimetallcarbonate wie z.B. Cäsiumcarbonat, Natriumcarbonat, Kaliumcarbonat.

Die Base wird bei dem erfindungsgemäßen Verfahren bevorzugt mit einem Anteil von 100 bis 1000 Mol %, bezogen auf die aromatische Boronsäure, eingesetzt. Der bevorzugte Anteil ist 600 bis 800 Mol%.

Als Katalysatoren können z.B. Palladiummetall, Palladiumverbindungen und/oder Nickelverbindungen eingesetzt werden. Die Katalysatoren können auch auf einem festen Träger, wie Aktivkohle oder Aluminiumoxid, aufgebracht sein. Bevorzugt sind Palladiumkatalysatoren, in denen das Palladium in der Oxidationsstufe (0) oder (II) vorliegt, wie Tetrakis(triphenylphosphin)-palladium, Bis(triphenylphosphin)palladiumdichlorid, Bis(diphenylphosphino)ferrocenpalladium-dichlorid, Palladiumketonate, Palladiumacetylacetonate (wie z.B. Palladiumbisacetylacetonat), Nitrilpalladiumhalogenide (wie z.B. Bis(benzonitril)palladiumdichlorid, Bis(acetonitril)-palladiumdichlorid), Palladiumhalogenide (PdCl₂, Na₂PdCl₄, Na₂PdCl₆), Allylpalladium-halogenide, Palladiumbiscarboxylate (wie z.B. Palladium-II-acetat) und Tetrachlorpalladiumsäure. Besonders bevorzugte Katalysatoren sind Tetrakis(triphenylphosphin)-palladium, Bis(triphenylphosphin)-palladiumdichlorid und Bis(diphenylphosphino)ferrocenpalladium-dichlorid. Die Palladiumverbindung kann auch in situ erzeugt werden, wie beispielsweise Palladium(II)acetat aus Palladium(II)chlorid und Natriumacetat.

Die Menge an Katalysator beträgt, bezogen auf den die Abgangsgruppe Z² tragenden Heteroaromaten **[IV-a],** bevorzugt 0,001 bis 0,5 Mol-% und besonders bevorzugt 0,01 bis 0,2 Mol-%.

Der Katalysator kann phosphorhaltige Liganden enthalten oder es können dem Reaktionsgemisch phosphorhaltige Liganden separat zugesetzt werden. Als phosphorhaltige Liganden eignen sich bevorzugt Tri-n-alkylphosphane, Triarylphosphane, Dialkylarylphosphane, Alkyldiarylphosphane und/oder Heteroarylphosphane, wie Tripyridylphosphan und Trifurylphosphan, wobei die drei Substituenten am Phosphor gleich oder verschieden sein können und wobei einer oder mehrere Substituenten die Phosphorgruppen mehrerer Phosphane verknüpfen können, wobei ein Teil dieser Verknüpfung auch ein Metallatom sein kann. Besonders bevorzugt sind Phosphane wie Triphenylphosphan, Tri-tert-butylphosphan, Tricyclohexylphosphan.

Die Gesamtkonzentration an phosphorhaltigen Liganden beträgt, bezogen auf den die Abgangsgruppe Z² tragenden Heteroaromaten **[IV-a],** bevorzugt bis 1 Mol-%, besonders bevorzugt 0,01 bis 0,5 Mol-%.

Zur Durchführung des erfindungsgemäßen Verfahrens werden zweckmässigerweise die Edukte, das Lösemittel, die Base, der Katalysator und gegebenenfalls der Ligand durchmischt und bevorzugt bei einer Temperatur von 0 °C - 200 °C, besonders bevorzugt bei 100-170 °C umgesetzt. Die Reaktionszeit variiert in Abhängigkeit vom Maßstab der Reaktion und der Reaktionstemperatur, aber liegt im Allgemeinen zwischen einigen Minuten und 48 Stunden. Ausser als Eintopfreaktion kann die Reaktion auch so geführt werden, dass die verschiedenen Reaktanden im Laufe der Reaktion kontrolliert zudosiert werden, wobei unterschiedliche Dosiervarianten möglich sind.

Das molare Reaktandenverhältnis vom Heteroaromat **[IV-a]** zur Organoborverbindung **[V]** beträgt bevorzugt 0.9 bis 1.5.

Die erfindungsgemäßen Verfahren werden im allgemeinen unter Normaldruck durchgeführt. Es ist jedoch auch möglich, unter erhöhtem oder vermindertem Druck zu arbeiten. Die Reaktion wird im allgemeinen unter Verwendung eines Schutzgases wie z.B. Argon oder Stickstoff durchgeführt. Nach beendeter Umsetzung wird der als Feststoff anfallende Katalysator durch Filtration abgetrennt, das Rohprodukt vom Lösungsmittel bzw. den Lösungsmitteln befreit und anschließend nach dem Fachmann bekannten und dem jeweiligen Produkt angemessenen Methoden, z. B. durch Umkristallisation, Destillation, Sublimation, Zonenschmelzen, Schmelzkristallisation oder Chromatographie, gereinigt.

### Schritt (V4)

Eine Möglichkeit zur Synthese von Verbindungen der Formel **[I-c]** ist in Schema 1 gezeigt.

Eine Verbindung mit der allgemeinen Formel **[I-c],** kann synthetisiert werden, analog zu in der Literatur beschriebenen Vorschriften (siehe z.B. WO 04/052880 sowie z.B. T.W. Greene, P. G. M. Wuts, Protective Groups in Organic Synthesis, 1999, John Wiley & Sons, Inc.), durch eine Kupplungsreaktion einer Verbindung mit der entsprechenden allgemeinen Formel **[III]** mit einem Substrat der allgemeinen Formel **[II]** (mit Z³ z.B. = Cl, Br, F, -OH) gegebenenfalls in der Gegenwart eines Säurefängers / Base wobei die Definitionen der Reste R¹, R², R^{3/301}, R^{4a/401a}, R⁵, R⁶ und X¹ in den obigen Schemata den oben angegebenen Definitionen entsprechen.

Säurehalogenide **[II]** (Z³ = Cl) oder die entsprechenden Carbonsäuren **[II]** (Z³ = OH) sind kommerziell erhältlich oder durch in der Literatur beschriebene Prozesse darstellbar. Außerdem kann ein Substrat mit der allgemeinen Formel **[II],** mit Z³ = Cl, aus der entsprechenden Säure (Z³ = OH) durch Chlorierung unter Verwendung literaturbekannter Prozesse dargestellt werden. (R. C. Larock, Comprehensive Organic Transformations, 2. Ausgabe, 1999, Wiley-VCH, Seite 1929 ff. und darin zitierte Literatur).

Als Lösungsmittel können alle üblichen, unter den Reaktionsbedingungen inerten Lösungsmittel, wie z.B. zyklische und azyklische Ether (z.B. Diethylether, Tetrahydrofuran, Dioxane), aromatische Kohlenwasserstoffe (z.B. Benzol, Toluol, Xylol), halogenierte Kohlenwasserstoffe (z.B. Dichlormethan, Chloroform, Tetrachlorkohlenstoff), halogenierte aromatische Kohlenwasserstoffe (z.B. Chlorbenzol, Dichlorbenzol) und Nitrile (z.B. Acetonitril) verwendet werden oder die Reaktion kann in Mischungen von zwei oder mehreren dieser Lösungsmittel ausgeführt werden. Die bevorzugten Lösungsmittel sind Tetrahydrofuran und Dichlormethan.

Wenigstens ein Äquivalent eines Säurefängers / einer Base (z.B. Hünig Base, Triethylamin oder kommerziell erhältliche polymere Säurefänger) im Verhältnis zum Startmaterial der allgemeinen Formel **[III]** wird verwendet. Ist das Startmaterial ein Salz, werden wenigstens zwei Äquivalente des Säurefängers benötigt.

Die Reaktion wird normalerweise bei Temperaturen von 0 °C - 100 °C durchgeführt und vorzugsweise bei 20 °C - 30 °C, aber sie kann auch bei Rückflusstemperatur der Reaktionsmischung durchgeführt werden. Die Reaktionszeit variiert in Abhängigkeit vom Maßstab der Reaktion und der Reaktionstemperatur, aber liegt im Allgemeinen zwischen einigen Minuten und 48 Stunden.

Zur Durchführung des erfindungsgemäßen Verfahrens **(V4)** zur Herstellung der Verbindungen der Formel **[I-c]** setzt man pro Mol des Carbonsäurehalogenids der Formel **[II]** im Allgemeinen 0.2 bis 2 Mol, vorzugsweise 0.5 bis 0.9 Mol an Aminoderivat der Formel **[III]** ein. Die Aufarbeitung erfolgt durch Verdampfen der flüchtigen Bestandteile im Vakuum und Versetzen des Rohmaterial mit ammoniakalischer Methanol-Lösung (7 molar).

Nach Beendigung der Reaktion, werden die Verbindungen **[I-c],** von der Reaktionsmischung durch eine der üblichen Trenntechniken getrennt. Falls notwendig werden die Verbindungen durch Umkristallisation, Destillation oder Chromatographie gereinigt.

Alternativ kann eine Verbindung der Formel **[I-c],** auch aus der entsprechenden Verbindung der Formel **[III]** mit einem Substrat der Formel **[II]** mit Z³ = -OH in Gegenwart eines Kupplungsreagenzes synthetisiert werden analog zu in der Literatur beschriebenen Vorschriften (z.B. Tetrahedron 2005, 61, 10827-10852, und darin zitierte Referenzen).

Geeignete Kupplungsreagenzien sind beispielsweise Peptidkupplungsreagenzien (zum Beispiel, N-(3-Dimethylaminopropyl)-N'-ethyl-carbodiimid gemischt mit 4-Dimethylamino-pyridin, N-(3-Dimethylaminopropyl)-N'-ethyl-carbodiimid gemischt mit 1-Hydroxy-benzotriazol, Brom-tripyrrolidinophosphonium-hexafluorophosphat, O-(7-Azabenzotriazol-1-yl)-N,N,N',N'-tetramethyl-uroniumhexa-fluorophosphat, etc.).

Gegebenenfalls kann eine Base, wie z.B. Triethylamin oder Hünig-Base in der Reaktion verwendet werden.

Als Lösungsmittel können alle üblichen, unter den Reaktionsbedingungen inerten Lösungsmittel, wie z.B. Alkohole (z.B. Methanol, Ethanol, Propanol), zyklische und azyklische Ether (z.B. Diethylether, Tetrahydrofuran, Dioxane), aromatische Kohlenwasserstoffe (z.B. Benzol, Toluol, Xylol), halogenierte Kohlenwasserstoffe (z.B. Dichlormethan, Chloroform, Tetrachlorkohlenstoff), halogenierte aromatische Kohlenwasserstoffe (z.B. Chlorbenzol, Dichlorbenzol), Nitrile (z.B. Acetonitril) und Amide (z.B. N,N-Dimethylformamid, N,N-Dimethylacetamid) verwendet werden oder die Reaktion kann in Mischungen von zwei oder mehreren dieser Lösungsmittel ausgeführt werden. Das bevorzugte Lösungsmittel ist Dichlormethan.

Die Reaktion wird normalerweise bei Temperaturen von 0 °C - 100 °C durchgeführt und vorzugsweise bei 0 °C - 30 °C, aber sie kann auch bei Rückflusstemperatur der Reaktionsmischung durchgeführt werden. Die Reaktionszeit variiert in Abhängigkeit vom Maßstab der Reaktion und der Reaktionstemperatur, aber liegt im Allgemeinen zwischen einigen Minuten und 48 Stunden.

Nach Beendigung der Reaktion, werden die Verbindungen **[I-c]** von der Reaktionsmischung durch eine der üblichen Trenntechniken getrennt. Falls notwendig werden die Verbindungen durch Umkristallisation, Destillation oder Chromatographie gereinigt.

Verbindungen der allgemeinen Formel **[I-c]** bei denen R^{4a/401a} für -NR¹²R^{12'} steht (symmetrisch oder unsymmetrisch bisacylierte Aminopyridine) lassen sich durch die oben beschriebene Methode direkt aus Verbindungen der allgemeinen Formel **[I-c],** bei denen R^{4a/401a} für -NHR¹² steht (monoacylierte Aminopyridine), durch Umsetzung mit Säurehalogeniden der Formel **[II]** herstellen (Z³= z.B. Cl, F).

### Schritt (V5)

Eine weitere Möglichkeit zur Synthese von Verbindungen der Formel **[I-c]** ist in Schema 1 gezeigt.

Verbindungen der Formel **[I-c]** lassen sich z.B. durch Kupplung der Pyrazolboronsäuren **[V]** mit Heterocyclen der Formel **[IV-c]** (wobei Z² eine Fluchtgruppe ist, wie z.B. Cl oder Br) in Gegenwart eines Katalysators, einer Base und eines geeigneten Lösungsmittels bei geeigneten Temperaturen nach bekannten Literaturvorschriften herstellen (Top. Curr. Chem. 2002, 219, 11; Organomet. Chem. 1999, 28, 147 und darin zitierte Literatur).

Verbindungen der Formel **[IV-c]** (in denen X¹ für C-H steht) sind kommerziell verfügbar oder lassen sich nach Literaturvorschriften herstellen (Schema 12). Eine Methode zur Herstellung geeigneter Halogenheterocyclen **[IV-c-1]** ist die Umsetzung von Aminoheterocyclen der Formel **[XX]** mit Säurechloriden in Gegenwart einer Base und eines Lösungsmittels (Synth. Commun. 1997, 27, 5, 861-870). Die Auswahl von Lösungsmittel, Base und Temperatur kann je nach eingesetzten Substrat **[XX]** varriieren und umfasst die unter Schritt **(V4)** beschriebenen Variationsmöglichkeiten für Umsetzung der Aminoheterocyclen der Formel **[III]** mit Substraten der Formel **[II]** zu Herstellung von Verbindungen der Formel **[I-c].**

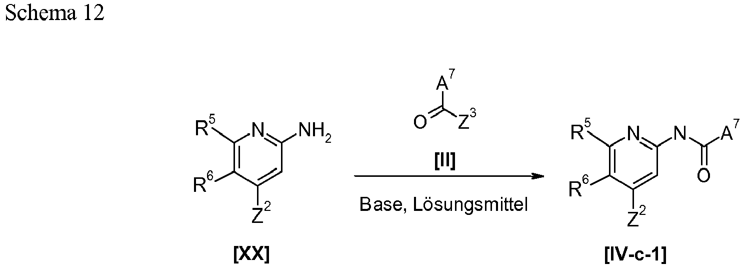

Die Aminoheterocyclen **[XX]** (in denen X¹ für C-H steht) sind bekannt oder können durch Abspaltung der N-BOC Schutzgruppe aus Verbindungen der Formel **[IV-a]** nach literaturbeschriebenen Vorschriften hergestellt werden (Aust. J. Chem. 1982, 35, 10, 2025-2034 und darin enthaltene Referenzen).

Die Aminoheterocyclen **[XX]** (in denen X¹ für N steht) sind bekannt oder können durch Halogenierung der Hydroxyverbindungen (Z²= -OH) nach literaturbeschriebenen Vorschriften hergestellt werden (z.B. nach J. Med. Chem. 2006, 49, 14, 4409-4424).

Die Auswahl von Lösungsmittel, Base, Temperatur, Katalysators und gegebenenfalls zugesetztem Liganden kann je nach eingesetzten Substrat **[IV-c]** varriieren und umfasst die unter Schritt **(V3)** beschriebenen Variationsmöglichkeiten für die C-C-Kupplung von Verbindungen der Formel **[V].**

Nach beendeter Umsetzung wird der als Feststoff anfallende Katalysator durch Filtration abgetrennt, das Rohprodukt vom Lösungsmittel bzw. den Lösungsmitteln befreit und anschließend nach dem Fachmann bekannten und dem jeweiligen Produkt angemessenen Methoden, z. B. durch Umkristallisation, Destillation, Sublimation, Zonenschmelzen, Schmelzkristallisation oder Chromatographie, gereinigt.

### Schritt (V6)

Eine weitere Möglichkeit zur Synthese von Verbindungen der Formel **[I]** ist in Schema 1 gezeigt.

Verbindungen der Formel **[I]** lassen sich z.B. durch Kupplung der Halopyrazole **[VI]** mit metallierten Heterocyclen der Formel **[XV-a]** (wobei Met¹ für einen Boronester oder Boronsäure wie z.B. B(OiPr)₃, B(OH)₂ steht) in Gegenwart eines Katalysators, einer Base, gegebenenfalls eines Liganden und eines geeigneten Lösungsmittels bei geeigneten Temperaturen nach bekannten Literaturvorschriften herstellen *(*Top. Curr. Chem. 2002, 219, 11; Organomet. Chem. 1999, 28, 147 und darin zitierte Literatur, 2005, 7, 21, 4753-4756). (Schema 13)

Verbindungen der Formel **[I]** lassen sich außerdem z.B. durch Kupplung der Halopyrazole **[VI]** mit metallierten Heterocyclen der Formel **[XV-a]** in Gegenwart eines Katalysators, gegebenenfalls eines anorganischen oder organischen Halogenid-Salzes, gegebenenfalls eines Liganden und eines geeigneten Lösungsmittels bei geeigneten Temperaturen nach bekannten Literaturvorschriften herstellen (vgl. Synthesis 1992, 803-815).

Verbindungen der Formel **[XV-al]** (in denen X¹ für C-H steht) sind kommerziell verfügbar oder lassen sich nach Literaturvorschriften herstellen. Eine Methode zur Herstellung geeigneter Halogenheterocyclen **[XV-al]** ist die Umsetzung von Haloheterocyclen der Formel **[XXI]** mit Bispinacolatodiboron in Gegenwart eines Katalysators (wie z.B. Pd(OAc)₂ , PdCl₂(dppf)), gegebenenfalls eines Liganden (wie z.B. 1,3-bis(2,6-diisopropylphenyl)-4,5-dihydroimidazolium-chlorid), einer Base (wie z.B. Kaliumacetat, Natriumacetat) und eines Lösungsmittels (wie z.B. Tetrahydrofuran, Dimethylsulfoxid) nach literaturbeschriebenen Methoden (Bioorg. Med. Chem. Lett. 2006, 16, 5, 1277-1281 und WO 04/014913) (Schema 13).

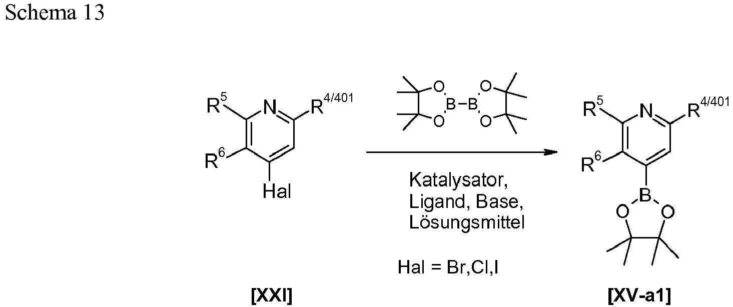

Alternativ können Verbindungen der Formel **[XV-al]** (in denen X¹ für C-H steht) auch durch andere literaturbekannte Methoden dargestellt werden. Eine Methode zur Herstellung geeigneter Heterocyclen **[XV-al]** ist die Metallierung des Halogenpyridins **[XXI]** mit einer Base (wie z.B. n-Butyllithium) in einem Lösungsmittel (wie z.B. Diethylether oder Tetrahydrofuran) und nachfolgende Umsetzung mit einem Boronsäureester (wie z.B. B(i-PrO)₃ oder B(OMe)₃) und Pinakol nach literaturbekannten Methoden (Synthesis 2004, 4, 469-483 und darin beschriebene Literatur) (Schema 14).

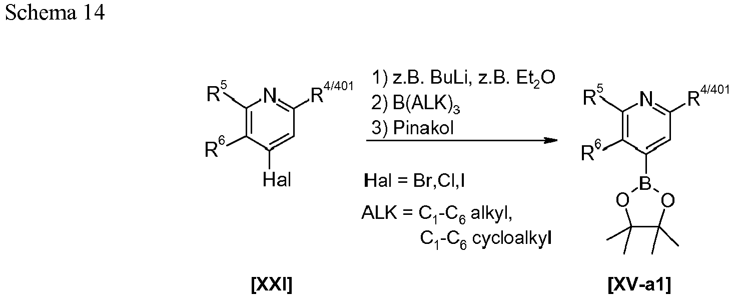

Verbindungen der Formel **[XV-a2]** (in denen X¹ für N steht) sind kommerziell verfügbar oder lassen sich nach Literaturvorschriften herstellen. Eine Methode zur Herstellung geeigneter Halogenheterocyclen [XV-a2] ist die Umsetzung von Haloheterocyclen der Formel **[XXII]** mit Hexaalkyldizinnverbindungen (wie z.B. 1,1,1,2,2,2-Hexabutyldizinn) in Gegenwart eines Katalysators (wie z.B. bis(Triphenylphosphin)palladium(II)acetat), gegebenenfalls einer Fluoridionenquelle (wie z.B. Tetrabutylammoniumfluorid) und eines Lösungsmittels (wie z.B. Tetrahydrofuran, Diethylether) nach literaturbeschriebenen Methoden (WO 03/095455 oder WO 07/104538) (Schema 15).

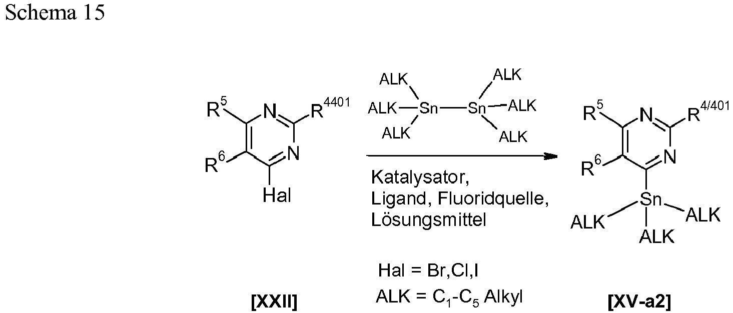

Alternativ können Verbindungen der Formel **[XV-a2]** (in denen X¹ für N steht) auch durch andere literaturbekannte Methoden dargestellt werden. Eine Methode zur Herstellung geeigneter Halogenheterocyclen **[XV-a2]** ist die Metallierung des Halogenpyridins **[XXII]** unter Verwendung eines Metallierungsreagenzes (einer Alkyllithiumverbindung wie z.B. n-Butyllithium oder eines Grignard-Reagenzes wie z.B. Isopropylmagnesiumchlorid) in einem Lösungsmittel (wie z.B. Diethylether oder Tetrahydrofuran) und nachfolgende Umsetzung mit einer Trialkylzinnhalogenverbindung (wie z.B. Bu₃SnCl) nach literaturbekannten Methoden (WO 08/008747 oder Tetrahedron 1994, 275-284 und darin beschriebene Literatur) (Schema 16).

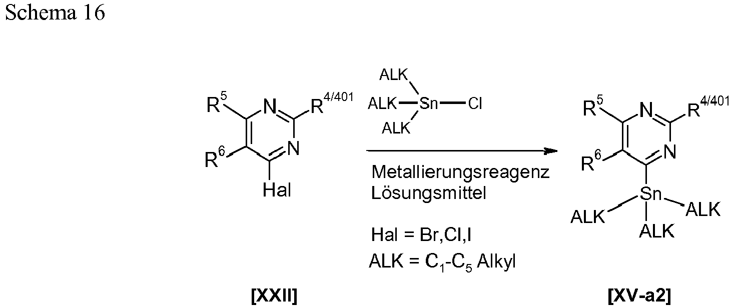

Verbindungen der Formel **[XXI]** und **[XXII]** sind kommerziell verfügbar oder können z.B. durch Acylierung entsprechender Amine (im Falle R^{4/401} = -NH₂) nach literaturbekannten Methoden (z.B. J. Org. Chem. 2004, 69, 543-548) dargestellt werden. Eine andere Methode zur Darstellung der Verbindungen des Typs **[XXI]** und **[XXII]** besteht in der Halogenierung der entsprechenden Hydroxyheterocyclen analog zu den für die Synthese der Verbindungen **[XX]** sowie **[IV-b]** angegebenen Halogenierungsmethoden.

Bei der Kupplung der Halopyrazole **[VI]** mit metallierten Heterocyclen der Formel **[XV-a]** (wobei Met für einen Boronester oder Boronsäure wie z.B. B(OiPr)₃, B(OH)₂ steht) kann die Auswahl von Lösungsmittel, Base, Temperatur, Katalysators und gegebenenfalls zugesetztem Liganden je nach eingesetzten Boronester-Substrat variieren und umfasst die unter Schritt **(V3)** beschriebenen Variationsmöglichkeiten für die C-C-Kupplung von Verbindung der Formel **[V]** mit Substraten der Formel **[IV-a].**

Bei der Kupplung der Halopyrazole **[VI]** mit metallierten Heterocyclen der Formel **[XV-a]** (wobei Met für eine Zinnalkyl tragende Gruppe wie z.B. Sn(Bu)₃ steht) kann die Auswahl eines Katalysators, gegebenenfalls eines anorganischen oder organischen Halogenid-Salzes, gegebenenfalls eines Liganden und eines geeigneten Lösungsmittels bei geeigneten Temperaturen je nach eingesetzten Zinnalkyl-Substrat variieren.

Als Lösungsmittel für die Umsetzung von Verbindungen der Formel **[XV-a]** können alle üblichen, unter den Reaktionsbedingungen inerten Lösungsmittel, wie z.B. zyklische und azyklische Ether (Diethylether, Dimethoxymethan, Diethylenglykoldimethylether, Tetrahydrofuran, Dioxan, Diisopropylether, tert.-Butylmethylether), aromatische Kohlenwasserstoffe (z.B. Benzol, Toluol, Xylol), Amide (z.B. Dimethylformamid, Dimethylacetamid, N-Methylpyrrolidon) und Sulfoxide (z.B. Dimethylsufoxid) verwendet werden oder die Reaktion kann in Mischungen von zwei oder mehreren dieser Lösungsmittel ausgeführt werden. Das bevorzugte Lösungsmittel ist Dimethylformamid.

Halogenidsalze für die Umsetzung von Verbindungen der Formel **[XV-a],** die bei dem erfindungsgemäßen Verfahren vorzugsweise Verwendung finden, sind z.B. Kupferhalogenide (z.B. CuBr oder CuI), Cäsiumhalogenide (CsF) und Tetraalkylammoniumhalogenide (TBAF).

Die Halogenidsalze werden bei dem erfindungsgemäßen Verfahren bevorzugt mit einem Anteil von 1 bis 400 Mol %, bezogen auf die organische Zinnverbindung, eingesetzt. Es können aber auch Mischungen der Halogenidsalze in Anteilen von 1- 400 Mol % eingesetzt werden. Besonders bevorzugt ist die Zugabe einer Mischung aus Kupferiodid und Cäsiumfluorid in Anteilen von 1- 200 Mol%.

Als Katalysatoren für die Umsetzung von Verbindungen der Formel **[XV-a]** können die gleichen Katalysatoren eingesetzt werden, die oben für die Herstellung der Verbindungen der Formel **[III]** durch Reaktion der Verbindungen der Formel **[V]** und **[IV-a]** beschrieben worden sind.

Die Menge an Katalysator beträgt bezogen auf den die Abgangsgruppe Met¹ tragenden Heteroaromaten **[XV-a],** bevorzugt 0,001 bis 0,5 Mol-% und besonders bevorzugt 0,01 bis 0,2 Mol-%.

Der Katalysator kann phosphorhaltige oder arsenhaltige Liganden enthalten oder es können dem Reaktionsgemisch phosphorhaltige oder arsenhaltige Liganden separat zugesetzt werden. Als phosphorhaltige Liganden eignen sich bevorzugt Tri-n-alkylphosphane, Triarylphosphane, Dialkylarylphosphane, Alkyldiarylphosphane und/oder Heteroarylphosphane, wie Tripyridylphosphan und Trifurylphosphan, wobei die drei Substituenten am Phosphor gleich oder verschieden sein können, chiral oder achiral sein können und wobei einer oder mehrere Substituenten die Phosphorgruppen mehrerer Phosphane verknüpfen können, wobei ein Teil dieser Verknüpfung auch ein Metallatom sein kann. Besonders bevorzugt sind Phosphane wie Triphenylphosphan, Tri-tert-butylphosphan, Tricyclohexylphosphan. Als arsenhaltige Liganden eignen sich z.B. Tri-n-alkylarsane und Triarylarsane, wobei die drei Substituenten am Arsen gleich oder verschieden sein können.

Die Gesamtkonzentration an Liganden beträgt, bezogen auf den die Abgangsgruppe Met¹ tragenden Heteroaromaten **[XV-a],** bevorzugt bis 1 Mol-%, besonders bevorzugt 0,01 bis 0,5 Mol-%.

Zur Durchführung des erfindungsgemäßen Verfahrens werden zweckmässigerweise die Edukte, das Lösemittel, das Halogenidsalz, der Katalysator und gegebenenfalls der Ligand durchmischt und bevorzugt bei einer Temperatur von 0 °C - 200 °C, besonders bevorzugt bei 60-150 °C umgesetzt. Die Reaktionszeit variiert in Abhängigkeit vom Maßstab der Reaktion und der Reaktionstemperatur, aber liegt im Allgemeinen zwischen einigen Minuten und 48 Stunden. Ausser als Eintopfreaktion kann die Reaktion auch so geführt werden, dass die verschiedenen Reaktanden im Laufe der Reaktion kontrolliert zudosiert werden, wobei unterschiedliche Dosiervarianten möglich sind.

Die erfindungsgemäßen Verfahren werden im Allgemeinen unter Normaldruck durchgeführt. Es ist jedoch auch möglich, unter erhöhtem oder vermindertem Druck zu arbeiten. Die Reaktion wird im Allgemeinen unter Verwendung eines Schutzgases wie z.B. Argon oder Stickstoff durchgeführt.

Das molare Reaktandenverhältnis vom Halopyrazol **[VI]** zur Organozinnverbindung **[XV-a2]** beträgt bevorzugt 0.9 bis 2.

Nach beendeter Umsetzung wird der als Feststoff anfallende Katalysator durch Filtration abgetrennt, das Rohprodukt vom Lösungsmittel bzw. den Lösungsmitteln befreit und anschließend nach dem Fachmann bekannten und dem jeweiligen Produkt angemessenen Methoden, z. B. durch Umkristallisation, Destillation, Sublimation, Zonenschmelzen, Schmelzkristallisation oder Chromatographie, gereinigt.

### Schritt (V7)

Eine weitere Möglichkeit zur Synthese von Verbindungen der Formel **[I]** ist in Schema 1 gezeigt.

Verbindungen der Formel **[I]** lassen sich durch z.B. durch Kupplung der Pyrazolboronsäuren **[V]** mit Heterocyclen der Formel **[IV-b]** (wobei Z² eine Fluchtgruppe wie z.B. Cl oder Br darstellt) in Gegenwart eines Katalysators, einer Base und eines geeigneten Lösungsmittels bei geeigneten Temperaturen nach bekannten Literaturvorschriften herstellen *(*Top. Curr. Chem. 2002, 219, 11; b - A. Suzuki, Organomet. Chem. 1999, 28, 147 und darin zitierte Literatur).

Verbindungen der Formel **[IV-b1]** (in denen X¹ für C-H steht) sind kommerziell verfügbar oder lassen sich nach Literaturvorschriften herstellen (Schema 17). Eine Methode zur Herstellung geeigneter Halogenheterocyclen **[IV-b1]** ist die Umsetzung der Pyridin-N-oxide mit Halogenierungsmitteln (z.B. PCl₃, POCl₃, SOCl₂ oder Methansulfonylchlorid) (vgl. Bioorg. Med. Chem. Lett. 2007, 17, 7, 1934-1937).

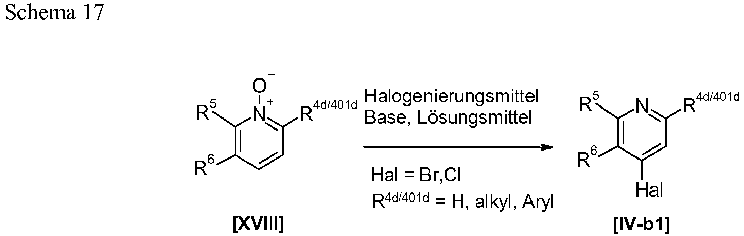

Die Pyridin-N-oxide **[XVIII]** sind bekannt oder können durch Oxidation der entsprechenden Pyridine (z.B. mit H₂O₂, H₂O₂ + Methyltrioxorhenium, m-Chlorperoxybenzoesäure, Dimethyldioxiran oder H₂O₂ + Mangan tetrakis(2,6-dichlorphenyl)porphyrin) nach literaturbeschriebenen Vorschriften hergestellt werden (ARKIVOC 2001 (i) 242-268 und darin enthaltene Referenzen).

Eine weitere Methode zur Herstellung geeigneter Halogenheterocyclen **[IV-b1]** ist die Umsetzung der 4-Hydroxy-Pyridin Verbindungen **[XIX]** mit Halogenierungsmitteln (z.B. PCl₃, POCl₃) nach literaturbekannten Vorschriften (Pol. J. Chem. 1981, 55, 4, 925 - 929) (Schema 18).

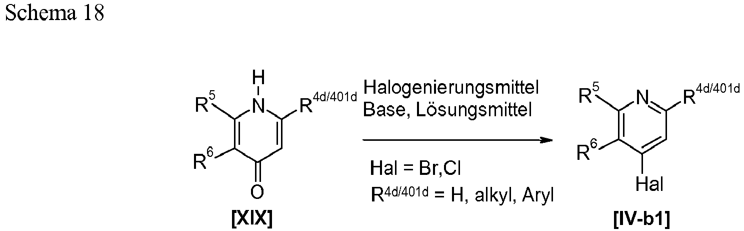

Die Hydroxypyridine **[XIX]** sind bekannt.

Verbindungen der Formel **[IV-b2]** (in denen X¹ für C-H steht) lassen sich nach Literaturvorschriften herstellen (Schema 19). Eine Methode zur Herstellung geeigneter Halogenheterocyclen **[IV-b2]** ist die Umsetzung von Aminoheterocyclen der Formel **[XX]** mit Trifluormethylketonen in Gegenwart von Titan-IV-chlorid, einer Base und eines Lösungsmittels (J. Am. Chem. Soc. 1996, 118, 7134-7138). Das im Verlauf dieser Umsetzung entstehende Imin kann durch Reduktion nach Literaturvorschriften (Tetrahedron 2009, 65, 9807-9813) in das Amin [IV-b2] überführt werden.

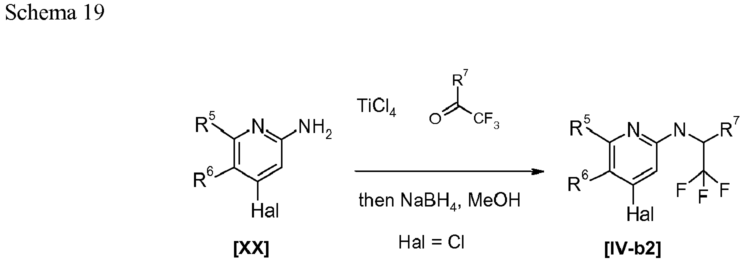

Die Aminoheterocyclen **[XX]** (in denen X¹ für C-H steht) sind bekannt (US2006/189617).

Die Auswahl von Lösungsmittel, Base, Temperatur, Katalysators und gegebenenfalls zugesetztem Liganden kann je nach eingesetzten Substrat **[IV-b]** variieren und umfasst die unter Schritt **(V3)** beschriebenen Variationsmöglichkeiten für die C-C-Kupplung von Verbindung der Formel **[V].**

Nach beendeter Umsetzung wird der als Feststoff anfallende Katalysator durch Filtration abgetrennt, das Rohprodukt vom Lösungsmittel bzw. den Lösungsmitteln befreit und anschließend nach dem Fachmann bekannten und dem jeweiligen Produkt angemessenen Methoden, z. B. durch Umkristallisation, Destillation, Sublimation, Zonenschmelzen, Schmelzkristallisation oder Chromatographie, gereinigt.

### Schritt V8

Eine Möglichkeit zur Synthese von Verbindungen der Formel **[XIII]** ist in Schema 1 gezeigt.

Verbindungen mit der allgemeinen Formel **[XIII]** sind bekannt (R² = H) oder können analog zu in der Literatur beschriebenen Vorschriften (siehe z.B. Acta Chem. Scand., Series B: Organic Chemistry and Biochemistry 1982, 36, 2, 101-108 und EP-A-1 382 603) synthetisiert werden. Eine Möglichkeit zur Herstellung der Verbindungen **[XIII]** ist eine Halogenierung der Pyrazole **[XIV]** mit einem Halogenierungsmittel in einem geeigneten Lösungsmittel zum Pyrazol **[XXIV]** gefolgt von einer Umsetzung des erhaltenen Halogenpyrazols mit einer geeigneten Schutzgruppe PG (z.B. 3,4-Dihydro-2H-pyran) zu Verbindungen der Formel **[XIII]** (Schema 20).

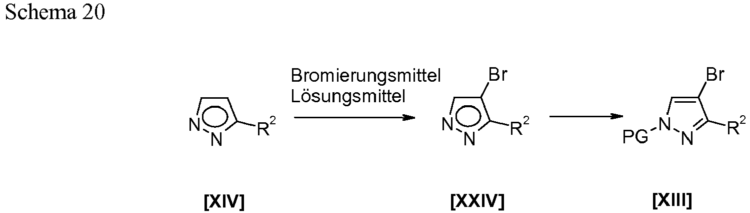

Pyrazole der Formel **[XIV]** (R² = H, CH₃) sind kommerziell erhältlich oder durch in der Literatur beschriebene Prozesse darstellbar. Methoden zur Herstellung geeigneter Pyrazole **[XIV]** sind z.B. die Umsetzung von Alkinen mit TMS-Diazomethan (Schema 21) oder die Reaktion von Methylketonen mit Dimethylformamid-dimethylacetal und Hydrazin (Schema 22) nach beschriebenen Methoden (US 0,063,744 A).

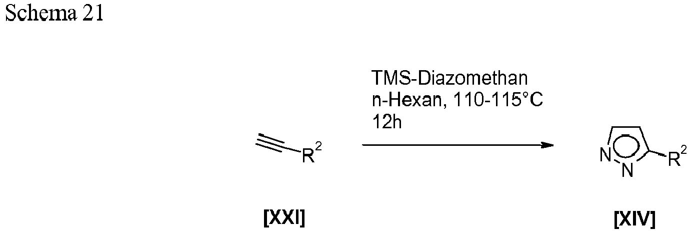

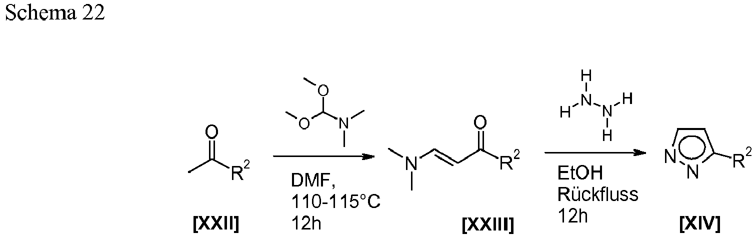

Als Halogenierungsmittel können z.B. N-Bromsuccinimid und Brom verwendet werden.

Als Lösungsmittel für die Halogenierungsreaktion können alle üblichen, unter den Reaktionsbedingungen inerten Lösungsmittel, wie z.B. Amide (z.B. Dimethylformamid, Dimethylacetamid, N-Methylpyrrolidon), halogenierte Kohlenwasserstoffe (z.B. Dichlormethan, Chloroform, Tetrachlorkohlenstoff), und Essigsäure verwendet werden oder die Reaktion kann in Mischungen von zwei oder mehreren dieser Lösungsmittel ausgeführt werden. Die Wahl der Lösungsmittel kann je nach verwendetem Halogenierungsreagenz variieren. Die bevorzugten Lösungsmittel sind Essigsäure und Dimethylformamid.

Die Halogenierungsreaktion wird normalerweise bei Temperaturen von 0 °C - 100 °C durchgeführt und vorzugsweise bei 20 °C - 30 °C. Die Reaktionszeit variiert in Abhängigkeit vom Maßstab der Reaktion und der Reaktionstemperatur, aber liegt im Allgemeinen zwischen einigen Minuten und 48 Stunden.

Nach Beendigung der Halogenierungsreaktion, werden die Rohprodukte von der Reaktionsmischung durch eine der üblichen Trenntechniken getrennt. Falls notwendig werden die Verbindungen durch Umkristallisation, Destillation oder Chromatographie gereinigt oder können gegebenenfalls auch direkt zur weiteren Umsetzung ohne vorhergehende Reinigung eingesetzt werden.

Die erhaltenen Brompyrazole **[XXIV]** werden durch Erhitzen in 3,4-Dihydro-2H-pyran in Gegenwart einer katalytischen Menge Lewissäure (z.B. p-Toluolsulfonsäure) am Stickstoffatom geschützt. Die erhaltenen Produkte können als Regioisomere auftreten. Falls notwendig werden die Verbindungen durch Destillation oder Chromatographie gereinigt oder können gegebenenfalls auch direkt zur weiteren Umsetzung ohne vorhergehende Reinigung eingesetzt werden.

### Schritt V9

Eine Möglichkeit zur Synthese von Verbindungen der Formel **[XII]** ist in Schema 1 gezeigt.

Verbindungen der Formel **[XII]** lassen sich z.B. durch Kupplung der Halopyrazole **[XIII]** mit metallierten Heterocyclen der Formel **[XV-a]** (wobei Met für einen Boronester oder Boronsäure wie z.B. B(OiPr)₃, B(OH)₂ steht) in Gegenwart eines Katalysators, einer Base, gegebenenfalls eines Liganden und eines geeigneten Lösungsmittels bei geeigneten Temperaturen nach bekannten Literaturvorschriften herstellen *(*Top. Curr. Chem. 2002, 219, 11; Organomet. Chem. 1999, 28, 147 und darin zitierte Literatur, Org. Lett. 2005, 7, 21, 4753-4756).

Verbindungen der Formel **[XII]** lassen sich außerdem z.B. durch Kupplung der Halopyrazole **[XIII]** mit metallierten Heterocyclen der Formel **[XV-a],** in Gegenwart eines Katalysators, gegebenenfalls eines anorganischen oder organischen Halogenid-Salzes, gegebenenfalls eines Liganden und eines geeigneten Lösungsmittels bei geeigneten Temperaturen nach bekannten Literaturvorschriften herstellen (vgl. Synthesis 1992, 803-815).

Die Herstellung der Verbindungen des Typs **[XV-a]** ist unter Schritt **(V6)** für die analoge Umsetzung der Halogenpyrazole **[VI]** beschrieben.

Die Auswahl von Lösungsmittel, gegebenenfalls zugesetzter Base oder Halogenidsalz, Temperatur, Katalysators und gegebenenfalls zugesetztem Liganden kann je nach eingesetzten Substrat **[XV-a]** varriieren und umfasst die unter Schritt **(V6)** beschriebenen Variationsmöglichkeiten für die C-C-Kupplung von Verbindungen der Formel **[VI].** Dabei wird üblicherweise bei der Umsetzung von Verbindungen der Formel **[XV-a],** in denen Met¹ für eine Zinnalkyl tragende Gruppe (wie z.B. Sn(Bu)₃) steht, auf den Zusatz einer Base verzichtet und stattdessen ein Halogenidsalz hinzugefügt, wie unter Schritt **(V6)** beschrieben.

### Schritt V10

Eine Möglichkeit zur Synthese von Verbindungen der Formel **[XI]** ist in Schema 1 gezeigt.

Eine Methode zur Herstellung der Verbindungen der Formel **[XI]** ist die Metallierung des geschützten Pyrazoles **[XII]** mit einer Base (wie z.B. n-Butyllithium) in einem Lösungsmittel (wie z.B. Diethylether oder Tetrahydrofuran) und nachfolgende Umsetzung mit einem Boronsäureester (wie z.B. B(i-PrO)₃ oder B(OMe)₃) und Pinakol nach literaturbekannten Methoden (vgl. Tetrahedron Letters 2006, 47; 27; 2006; 4665-4669 und darin beschriebene Literatur) oder mit einem Trialkylzinnhalogenverbindung (wie z.B. Bu₃SnCl) analog zu literaturbekannten Methoden (WO 06/108591)

Als Lösungsmittel können alle üblichen, unter den Reaktionsbedingungen inerten Lösungsmittel, wie z.B. zyklische und azyklische Ether (z.B. Diethylether, Tetrahydrofuran, Dioxane) verwendet werden oder die Reaktion kann in Mischungen von zwei oder mehreren dieser Lösungsmittel ausgeführt werden. Das bevorzugte Lösungsmittel ist Tetrahydrofuran.

Die Reaktion wird normalerweise bei Temperaturen von -80°C bis 0 °C durchgeführt und vorzugsweise bei -78 °C bis -20 °C. Im Verlauf der Reaktionsführung kann eine Änderung der Reaktionstemperatur (z.B. nach dem Metallierungsschritt) günstig oder notwendig sein, um die Reaktion mit dem zweiten Reaktionspartner (z.B. dem Alkylzinnhalogenid oder dem Borsäureester) zu gewährleisten. Die Reaktionszeit variiert in Abhängigkeit vom Maßstab der Reaktion und der Reaktionstemperatur, aber liegt im Allgemeinen zwischen einigen Minuten und 48 Stunden.

Die Aufarbeitung erfolgt üblicherweise durch Zugabe einer Protonenquelle (z.B. einer gesättigten wässrigen Ammoniumchlorid-Lösung) und nachfolgende Phasentrennung. Anschließendwerden die Verbindungen [XI] von der Reaktionsmischung durch eine der üblichen Trenntechniken getrennt.

Alternativ kann die Reaktionmischung aber auch ohne wässrige Aufarbeitung eingeengt und die Rohprodukte [XI] direkt aus der Reaktionsmischung herausdestilliert werden.

Falls notwendig, werden die so erhaltenen Verbindungen durch Umkristallisation, Destillation oder Chromatographie gereinigt.

### Schritt V11

Eine Möglichkeit zur Synthese von Verbindungen der Formel **[X]** ist in Schema 1 gezeigt.

Verbindungen der Formel **[X]** lassen sich z.B. durch Kupplung der Pyrazole der Formel **[XI]** (wobei Met für einen Boronester oder Boronsäure wie z.B. B(OiPr)₃, B(OH)₂ steht) mit Verbindungen der Formel **[XVII]** (wobei Z⁴ eine Fluchtgruppe wie z.B. Cl, Br, I, Mesylat oder Triflat darstellt) in Gegenwart eines Katalysators, einer Base, gegebenenfalls eines Liganden und eines geeigneten Lösungsmittels bei geeigneten Temperaturen nach bekannten Literaturvorschriften herstellen (Top. Curr. Chem. 2002, 219, 11; Organomet. Chem. 1999, 28, 147 und darin zitierte Literatur, Org. Lett. 2005, 7, 21, 4753-4756).

Verbindungen der Formel **[X]** lassen sich außerdem z.B. durch Kupplung der Pyrazole der Formel **[XI]** (wobei Met³ für eine Zinnalkyl tragende Gruppe wie z.B. -Sn(Bu)₃ steht) mit Verbindungen der Formel **[XVII]** (wobei Z⁴ eine Fluchtgruppe wie z.B. Cl, Br, I, Mesylat oder Triflat darstellt) in Gegenwart eines Katalysators, gegebenenfalls eines anorganischen oder organischen Halogenid-Salzes, gegebenenfalls eines Liganden und eines geeigneten Lösungsmittels bei geeigneten Temperaturen nach bekannten Literaturvorschriften herstellen (vgl. Synthesis 1992, 803-815).

Verbindungen der Formel **[XVII]** wie z.B. 4-Brom-1-fluorbenzol sind bekannt und kommerziell verfügbar.

Bei der Kupplung der Pyrazole **[XI]** mit Verbindungen der Formel **[XVII]** kann die Auswahl von Lösungsmittel, Base, Temperatur, Katalysators und gegebenenfalls zugesetztem Liganden je nach eingesetzten Pyrazol **[XI]** variieren und umfasst die unter Schritt **(V6)** beschriebenen Variationsmöglichkeiten.

Bei der Kupplung der Pyrazole **[XI]** mit Verbindungen der Formel **[XVII]** kann die Auswahl eines Katalysators, gegebenenfalls eines anorganischen oder organischen Halogenid-Salzes, gegebenenfalls eines Liganden und eines geeigneten Lösungsmittels bei geeigneten Temperaturen je nach eingesetzten Pyrazol **[XI]** variieren und umfasst die unter Schritt **(V3)** beschriebenen Variationsmöglichkeiten.

Die erfindungsgemäßen Verfahren werden im Allgemeinen unter Normaldruck durchgeführt. Es ist jedoch auch möglich, unter erhöhtem oder vermindertem Druck zu arbeiten.

Die Reaktion wird im Allgemeinen unter Verwendung eines Schutzgases wie z.B. Argon oder Stickstoff durchgeführt.

Das molare Reaktandenverhältnis vom Pyrazol **[XI]** zur Verbindung der Formel **[XVII]** beträgt bevorzugt 0.9 bis 2.

Nach beendeter Umsetzung wird der als Feststoff anfallende Katalysator durch Filtration abgetrennt, das Rohprodukt vom Lösungsmittel bzw. den Lösungsmitteln befreit und anschließend nach dem Fachmann bekannten und dem jeweiligen Produkt angemessenen Methoden, z. B. durch Umkristallisation, Destillation, Sublimation, Zonenschmelzen, Schmelzkristallisation oder Chromatographie, gereinigt.

### Schritt (V12)

Eine Möglichkeit zur Synthese von Verbindungen der Formel **[IX]** ist in Schema 1 gezeigt.

Eine Verbindung der Formel **[X]** wird in eine Verbindung der Formel **[IX]** durch geeignete Methoden zur Entfernung von Schutzgruppen, die in der Literatur beschrieben sind (*"*Protective Groups in Organic Synthesis "; Third Edition; Theodora W. Greene, Peter G. M. Wuts; 1999, Wiley-VCH, S. 494-653, und dort zitierte Literatur), überführt.

2-(Trimethylsilyl-ethoxy)methyl und Tetrahydropyran-2-yl Schutzgruppen können z.B. im sauren Medium (z.B mit methanolischer HCl oder Trifluoressigsäure) nach literaturbekannten Vorschriften entfernt werden (WO 03/099822 und J. Org. Chem. 2008, 73, 4309-4312 und darin enthaltene Literatur). Benzylische Schutzgruppen können hydrogenolytisch mit einer Wasserstoffquelle (z.B. Wasserstoff, Ammoniumformiat, Ameisensäure oder Cyclohexen) in Gegenwart eines Katalysators (z.B. Palladium auf Aktivkohle oder Palladiumhydroxid auf Aktivkohle) nach literaturbekannten Vorschriften entfernt werden (EP-A-1 228 067).

Als Lösungsmittel können alle üblichen, unter den Reaktionsbedingungen inerten Lösungsmittel, wie z.B. Alkohole (z.B. Methanol, Ethanol, Propanol), zyklische und azyklische Ether (z.B. Diethylether, Tetrahydrofuran, Dioxane), aromatische Kohlenwasserstoffe (z.B. Benzol, Toluol, Xylol), halogenierte Kohlenwasserstoffe (z.B. Dichlormethan, Chloroform, Tetrachlorkohlenstoff), halogenierte aromatische Kohlenwasserstoffe (z.B. Chlorbenzol, Dichlorbenzol), Nitrile (z.B. Acetonitril), Carbonsäureester (z.B. Essigsäureethylester), Amide (z.B. N,N-Dimethylformamid, N,N-Dimethylacetamid), Dimethylsulfoxid, 1,3-Dimethyl-2-imidazolinon, Wasser und Essigsäure verwendet werden oder die Reaktion kann in Mischungen von zwei oder mehreren dieser Lösungsmittel ausgeführt werden.

Die Reaktion wird normalerweise bei Temperaturen von 0 °C - 150 °C durchgeführt und vorzugsweise bei Raumtemperatur, aber sie kann auch bei Rückflusstemperatur der Reaktionsmischung durchgeführt werden. Die Reaktionszeit variiert in Abhängigkeit vom Maßstab der Reaktion und der Reaktionstemperatur, aber liegt im Allgemeinen zwischen einer halben Stunde und 72 Stunden.

Nach Beendigung der Reaktion, werden die Verbindungen [**IX**] von der Reaktionsmischung durch eine der üblichen Trenntechniken getrennt. Falls notwendig werden die Verbindungen durch Umkristallisation, Destillation oder Chromatographie gereinigt oder können, wenn gewünscht, auch im nächsten Schritt ohne vorhergehende Reinigung eingesetzt werden. Es ist außerdem möglich, die Verbindung der allgemeinen Formel [**IX**] als Salz zu isolieren, z.B. als Salz der Salzsäure oder der Trifluoressigsäure.

### Schritt (V13)

Eine weitere Möglichkeit zur Synthese von Verbindungen der Formel **[I]** ist in Schema 1 gezeigt.

Verbindungen der Formel **[IX]** lassen sich analog den in Schritt **(V1)** beschriebenen Methoden in Verbindungen der Formel **[I]** überführen (Schema 1) wobei in der Verbindung der Formel **[IX]** in R^{4/401} keine Funktionalität mit reaktiven aciden H-atomen enthalten sein sollte.

Die Auswahl von Lösungsmittel, Base und Temperatur kann je nach eingesetzten Substrat **[IX]** varriieren und umfasst die unter Schritt **(V1)** beschriebenen Variationsmöglichkeiten.

Nach Beendigung der Reaktion, werden die Verbindungen **[I],** von der Reaktionsmischung durch eine der üblichen Trenntechniken getrennt. Je nach der Art der verwendeten Substrate der Formel **[XVI]** und der Reaktionsbedingungen können die Verbindungen des Formel **[I],** in denen R³ nicht für Wasserstoff steht, als reine Regioisomere oder als Mischung beider möglicher Regioisomerer (wobei die Gruppe R^{3/301} beide Positionen am N-Atom des Pyrazols einnehmen kann) erhalten werden. In dem Falle, daß Mischungen an Regioisomeren erhalten werden, können diese durch physikalische Methoden (wie z.B. Kristallisations- oder Chromatographie-Methoden) gereinigt werden.

In Analogie kann die im Schema 2 beschriebene Synthese der Pyrazole **[I-d],** sowie die im Schema 3 beschriebene Synthese der Pyrazole **[I-e]** und **[XXIX]** erfolgen, wobei in den Verbindungen der Formel **[IX-b], [XXVII]** und **[XXVIII]** in R⁴ keine Funktionalität mit reaktiven aciden H-atomen enthalten sein sollte.

### Schritt (V14)

Eine Möglichkeit zur Synthese von Verbindungen der Formel **[XXV]** ist in Schema 2 gezeigt.

Nach literaturbekannten Methoden (J. Med. Chem. 2007, 50, 2732-2736, WO 05/040155, WO 01/74811, US 6,342,608 A) wird ein Carbonsäureester, -nitril, -dialkylamid oder -N,O-dialkylamid in Gegenwart einer starken Base mit einem Alkylpyridin oder Alkylpyrimidin der Formel **[XXIV]** umgesetzt.

Basen, die bei dem erfindungsgemäßen Verfahren vorzugsweise Verwendung finden, sind Alkalimetallalkoxide (wie z.B. KOtBu oder NaOtBu), Lithiumamide (wie z.B. LDA oder LiHMDS) oder Metallhydride (wie z.B. KH oder NaH).

Als Lösungsmittel können alle üblichen, unter den Reaktionsbedingungen inerten Lösungsmittel, wie z.B. zyklische und azyklische Ether (z.B. Diethylether, Tetrahydrofuran, Dioxane, Dimethoxyethan), Amide (z.B. N,N-Dimethylformamid, N,N-Dimethylacetamid), Dimethylsulfoxid oder HMPT verwendet werden oder die Reaktion kann in Mischungen von zwei oder mehreren dieser Lösungsmittel ausgeführt werden. Die Verwendung polarer Lösungsmittel wie N,N-Dimethylformamid, Dimethylsulfoxid oder HMPT ist bevorzugt.

Die Reaktion wird normalerweise bei Temperaturen von -78 °C bis zum Siedepunkt des Lösungsmittels durchgeführt, vorzugsweise im Bereich von -20 °C bis 40 °C. Die Reaktionszeit variiert in Abhängigkeit vom Maßstab der Reaktion und der Reaktionstemperatur, aber liegt im Allgemeinen zwischen einer halben Stunde und 72 Stunden.

Nach Beendigung der Reaktion, werden die Verbindungen **[XXV]** von der Reaktionsmischung durch eine der üblichen Trenntechniken getrennt. Falls notwendig werden die Verbindungen durch Umkristallisation, Destillation oder Chromatographie gereinigt oder können, wenn gewünscht, auch im nächsten Schritt ohne vorhergehende Reinigung eingesetzt werden.

Die Alkylpyridine oder Alkylpyrimidine der Formel **[XXIV]** sind kommerziell erhältlich oder können nach literaturbekannten Methoden (z.B. WO 04/058776 oder WO 04/035545) hergestellt werden.

In Analogie kann die im Schema 8 beschriebene Synthese der Verbindungen der Formel **[XXXVII],** sowie die im Schema 9 beschriebene Synthese der Verbindungen der Formel **[XLIII]** erfolgen, wobei in den Verbindungen der Formel **[XXXVI]** und **[XLII]** in R⁵ und R⁶ keine Funktionalität mit reaktiven aciden H-Atomen enthalten sein sollte.

### Schritt (V15)

Eine Möglichkeit zur Synthese von Verbindungen der Formel **[XXVI]** ist in Schema 2 gezeigt.

Verbindungen der generellen Formel **[XXVI]** werden nach literaturbekannten Methoden (J. Med. Chem. 2007, 50, 2732-2736 und WO 05/040155, für R^{4b/401b} = NHC(O)OAlkyl z.B. EP-A-1 553 096) durch Umsetzung einer Verbindung der Formel **[XXV]** mit DMF-Dialkylacetal erhalten. Die Reaktion kann in Gegenwart eines Lösungsmittels durchgeführt werden, geeignete Lösungsmittel sind Akohole (wie z.B. Ethanol), Ester (wie z. B. Essigsäureethylester), Cyclische Ether (wie z.B. Tetrahydrofuran) oder Amide (z.B. N,N-Dimethylformamid, N-Methylpyrrolidon). Die Reaktion kann in Gegenwart einer Base (z.B. Triethylamin) durchgeführt werden.

Die Reaktion wird normalerweise bei Temperaturen von -78 °C bis zum Siedepunkt des Lösungsmittels durchgeführt.

In Analogie kann die im Schema 8 beschriebene Synthese der Verbindungen der Formel **[XXXVIII],** sowie die im Schema 9 beschriebene Synthese der Verbindungen der Formel **[XLV]** erfolgen.

### Schritt (V16)

Eine Möglichkeit zur Synthese von Verbindungen der Formel **[XXVII]** ist in Schema 2 gezeigt.

Verbindungen der generellen Formel **[XXVII]** werden durch Reaktion von Verbindungen der generellen Formel **[XXVI]** mit Hydrazin oder Hydrazinhydrat nach literaturbekannten Methoden (z.B. EP-A-1 553 096, EP-A-1 188 754) erhalten. Dabei steht die im Schema 2 genannte Gruppe Z⁶ für eine Fluchtgruppe wie z.B. NMe₂.

Die Reaktion kann in Gegenwart einer Base wie z.B. Triethylamin durchgeführt werden.

Als Lösungsmittel können alle üblichen, unter den Reaktionsbedingungen inerten Lösungsmittel, wie z.B. zyklische und azyklische Ether (z.B. Tetrahydrofuran, Dioxane, Dimethoxyethan) oder Alkohole (z.B. Ethanol, Methanol) verwendet werden oder die Reaktion kann in Mischungen von zwei oder mehreren dieser Lösungsmittel ausgeführt werden. Die Verwendung polarer Lösungsmittel wie z.B. Ethanol ist bevorzugt.

Die Reaktion wird normalerweise bei Temperaturen von 0 °C bis zum Siedepunkt des Lösungsmittels durchgeführt, vorzugsweise im Bereich von 25 °C. Die Reaktionszeit variiert in Abhängigkeit vom Maßstab der Reaktion und der Reaktionstemperatur, aber liegt im Allgemeinen zwischen einer halben Stunde und 72 Stunden. Die Reaktion kann in einer Mikrowellenapparatur (z.B. CEM Explorer) bei erhöhter Temperatur durchgeführt werden, wobei sich die benötigte Reaktionszeit verkürzen kann.

In Analogie kann die im Schema 8 beschriebene Synthese der Verbindungen der Formel **[XXXIX],** sowie die im Schema 9 beschriebene Synthese der Verbindungen der Formel **[XLVI]** erfolgen.

### Schritt (V17)

Eine Möglichkeit zur Synthese von Verbindungen der Formel **[I-d]** ist in Schema 2 gezeigt.

Verbindungen der generellen Formel **[I-d]** werden durch Reaktion von Verbindungen der generellen Formel **[XXVI]** mit Alkylhydrazinen der Formel R^{3/301}-NH**-**NH₂ nach literaturbekannten Methoden (z.B. US 6,335,336 A) erhalten. Dabei steht die im Schema 2 genannte Gruppe Z⁶ für eine Fluchtgruppe wie z.B. NMe₂.

Die Reaktion kann in Gegenwart einer Base wie z.B. Triethylamin durchgeführt werden.

Als Lösungsmittel können alle üblichen, unter den Reaktionsbedingungen inerten Lösungsmittel, wie z.B. zyklische und azyklische Ether (z.B. Tetrahydrofuran, Dioxane, Dimethoxyethan) oder Alkohole (z.B. Ethanol, Methanol) verwendet werden oder die Reaktion kann in Mischungen von zwei oder mehreren dieser Lösungsmittel ausgeführt werden. Die Verwendung polarer Lösungsmittel wie z.B. Ethanol ist bevorzugt.

Die Reaktion wird normalerweise bei Temperaturen von 0 °C bis zum Siedepunkt des Lösungsmittels durchgeführt, vorzugsweise im Bereich von 25 °C. Die Reaktionszeit variiert in Abhängigkeit vom Maßstab der Reaktion und der Reaktionstemperatur, aber liegt im Allgemeinen zwischen einer halben Stunde und 72 Stunden. Die Reaktion kann in einer Mikrowellenapparatur (z.B. CEM Explorer) bei erhöhter Temperatur durchgeführt werden, wobei sich die benötigte Reaktionszeit verkürzen kann.

### Schritt (V18)

Eine Möglichkeit zur Synthese von Verbindungen der Formel [I-d] bei denen R^{3/301} für Cyclopropyl steht, ist die Umsetzung von Pyrazolen der Formel **[XXVII]** mit einer Cyclopropylboronsäure nach literaturbekannten Vorschriften (J. Org. Chem. 2008, 73, 6441-6444 oder WO 08/088692).

Die Reaktion wird in Gegenwart einer Base (wie z.B. Triethylamin, Pyridin, Natriumcarbonat, Kaliumphosphat, Cäsiumcarbonat) und eines Cu(II)-Salzes (wie z.B. Cu(OAc)₂ oder CuCl₂) durchgeführt.

Außerdem kann die Reaktion unter Zusatz eines geeigneten Liganden (wie z.B. Pyridin oder 2,2-Bipyridin, N,N,N',N'-Tetramethylethylenediamin oder 1,10-Phenanthridin) erfolgen.

Als Lösungsmittel können alle üblichen, unter den Reaktionsbedingungen inerten Lösungsmittel, wie z.B. zyklische und azyklische Ether (z.B. Tetrahydrofuran, Dioxane, Dimethoxyethan), Halogenalkane (z.B. Dichorethan) oder aromatische Kohlenwasserstoffe (z.B. Benzol,Toluol) verwendet werden oder die Reaktion kann in Mischungen von zwei oder mehreren dieser Lösungsmittel ausgeführt werden. Die Verwendung von Halogenalkanen wie z.B. Dichlorethan ist bevorzugt.

Die Reaktion wird normalerweise bei Temperaturen von 50 °C bis zum Siedepunkt des Lösungsmittels durchgeführt, vorzugsweise im Bereich von 70 °C. Die Reaktionszeit variiert in Abhängigkeit vom Maßstab der Reaktion und der Reaktionstemperatur, aber liegt im Allgemeinen zwischen einer halben Stunde und 72 Stunden. Die Reaktion kann in einer Mikrowellenapparatur (z.B. CEM Explorer) bei erhöhter Temperatur durchgeführt werden, wobei sich die benötigte Reaktionszeit verkürzen kann.

Analog zur im Schema 2 beschriebenen Synthese der Pyrazole [I-d], kann mit diesem Verfahren die im Schema 3 beschriebene Synthese der Pyrazole **[I-e]** sowie die im Schema 4 beschriebene Synthese der Pyrazole **[XX]** erfolgen.

### Schritt (V19)

Eine Möglichkeit zur Synthese von Verbindungen der Formel **[XXVIII]** ist in Schema 3 gezeigt.

Verbindungen der generellen Formel **[XXVIII]** werden durch Halogenierung von Pyrazolen der Formel **[XXVII]** nach literaturbekannten Vorschriften (z.B. Bioorg. Med. Chem. Lett. 2008, 18, 509-512) erhalten.

Als Halogenierungsmittel können z.B. N-Bromsuccinimid und Brom verwendet werden.

Als Lösungsmittel für die Halogenierungsreaktion können alle üblichen, unter den Reaktionsbedingungen inerten Lösungsmittel, wie z.B. Amide (z.B. Dimethylformamid, Dimethylacetamid, N-Methylpyrrolidon), halogenierte Kohlenwasserstoffe (z.B. Dichlormethan, Chloroform, Tetrachlorkohlenstoff), und Essigsäure verwendet werden oder die Reaktion kann in Mischungen von zwei oder mehreren dieser Lösungsmittel ausgeführt werden. Die Wahl der Lösungsmittel kann je nach verwendetem Halogenierungsreagenz variieren. Die bevorzugten Lösungsmittel sind Essigsäure und Dimethylformamid.

Die Halogenierungsreaktion wird normalerweise bei Temperaturen von 0 °C bis 100 °C durchgeführt und vorzugsweise bei 20 °C bis 80 °C. Die Reaktionszeit variiert in Abhängigkeit vom Maßstab der Reaktion und der Reaktionstemperatur, aber liegt im Allgemeinen zwischen einigen Minuten und 48 Stunden.

Nach Beendigung der Halogenierungsreaktion, werden die Rohprodukte von der Reaktionsmischung durch eine der üblichen Trenntechniken getrennt. Falls notwendig werden die Verbindungen durch
Umkristallisation, Destillation oder Chromatographie gereinigt oder können gegebenenfalls auch direkt zur weiteren Umsetzung ohne vorhergehende Reinigung eingesetzt werden.

### Schritt (V20)

Eine Möglichkeit zur Synthese von Verbindungen der Formel **[IX-b]** ist in Schema 3 gezeigt.

Verbindungen der Formel **[IX-b]** bei denen R^{2a} für Alkyl oder Cycloalkyl steht, können durch C-C-Kupplung von Pyrazolen der Formel **[XXIX]** mit Boronsäuren oder Boronsäureestern (z.B. Trimethylboroxin, Cyclopropylboronsäureester) nach literaturbekannten Vorschriften (US 0,018,132) hergestellt werden.

Die Reaktion wird in Gegenwart einer Base (wie z.B. Natriumhydroxid, Kaliumhydroxid, Natriumhydrogencarbonat, Natriumcarbonat oder Cäsiumcarbonat) und eines PalladiumKatalysators (wie z.B. Dichlor[1,1'-ferrocenylbis(diphenylphosphan)]palladium(II)*CH₂Cl₂) durchgeführt.

Als Lösungsmittel können alle üblichen, unter den Reaktionsbedingungen inerten Lösungsmittel, wie z.B. zyklische und azyklische Ether (z.B. Tetrahydrofuran, Dioxane, Dimethoxyethan) verwendet werden oder die Reaktion kann in Mischungen von zwei oder mehreren dieser Lösungsmittel ausgeführt werden.

Die Reaktion wird normalerweise bei Temperaturen von 50 °C bis zum Siedepunkt des Lösungsmittels durchgeführt, vorzugsweise im Bereich von 90 °C. Die Reaktionszeit variiert in Abhängigkeit vom Maßstab der Reaktion und der Reaktionstemperatur, aber liegt im Allgemeinen zwischen einer halben Stunde und 72 Stunden. Die Reaktion kann in einer Mikrowellenapparatur (z.B. CEM Explorer) bei erhöhter Temperatur durchgeführt werden, wobei sich die benötigte Reaktionszeit verkürzen kann.

Bei der C-C-Kupplung der Pyrazolen der Formel **[XXIX]** mit Verbindungen **[XXX]** (wobei Met² für einen Boronester oder Boronsäure wie z.B. B(OiPr)₃, B(OH)₂ steht) kann die Auswahl eines Katalysators, einer Base, eines Liganden und eines geeigneten Lösungsmittels bei geeigneten Temperaturen je nach eingesetzten Pyrazol **[XXIX]** variieren und umfasst ebenfalls die unter Schritt (V3) beschriebenen Variationsmöglichkeiten.

Zur Aufarbeitung wird die Reaktionsmischung mit Wasser versetzt und mit Essigsäureethylester extrahiert. Die organische Phase wird abgetrennt und das Lösungsmittel wird im Vakuum entfernt.

Das erhaltene Rohprodukt wird nach literaturbekannten Methoden (z.B. *"*Protective Groups in Organic Synthesis"; Third Edition; Theodora W. Greene, Peter G. M. Wuts; 1999, Wiley-VCH, S. 639-640, und dort zitierte Literatur) mit Trifluoressigsäure umgesetzt, um die am Pyrazol befindliche Gruppe R³ abzuspalten (z.B. im Falle R³ = p-Methoxybenzyl) wobei die Verbindungen der Formel **[IX-b]** erhalten werden.

Nach Beendigung der Reaktion, werden die Verbindungen **[IX-b]** von der Reaktionsmischung durch eine der üblichen Trenntechniken getrennt. Falls notwendig, werden die Verbindungen durch Umkristallisation, Destillation oder Chromatographie gereinigt.

### Schritt (V21)

Eine Möglichkeit zur Synthese von Verbindungen der Formel **[I-e]** ist in Schema 3 gezeigt.

Verbindungen der Formel **[I-e]** bei denen R^{2a} für Alkyl oder Cycloalkyl steht, können durch C-C-Kupplung von Pyrazolen der Formel **[XXIX]** mit Boronsäuren oder Boronsäureestern (z.B. Trimethylboroxin, Cyclopropylboronsäureester) nach literaturbekannten Vorschriften (US 0,018,132 A) hergestellt werden.

Die Bedingungen für die Kupplung entsprechen den unter obigem Verfahren **(V20)** angegebenen Bedingungen ohne die Entfernung der Gruppe R^{3/301} durch Entschützungsreaktion.

Nach Beendigung der Reaktion, werden die Verbindungen **[I-e]** von der Reaktionsmischung durch eine der üblichen Trenntechniken getrennt. Falls notwendig, werden die Verbindungen durch Umkristallisation, Destillation oder Chromatographie gereinigt.

### Schritt (V22)

Eine Möglichkeit zur Synthese von Verbindungen der Formel **[XXXII]** ist in Schema 4 gezeigt.

Verbindungen der generellen Formel **[XXXII]** werden durch Reaktion von Verbindungen der generellen Formel **[XXXI]** mit Alkylhydrazinen der Formel R^{3/301}-NH-NH₂ nach literaturbekannten Methoden (z.B. US5744426) erhalten.

Die Reaktion kann in Gegenwart einer Säure wie z.B. Essigsäure durchgeführt werden.

Als Lösungsmittel können alle üblichen, unter den Reaktionsbedingungen inerten Lösungsmittel verwendet werden, wie z.B. zyklische und azyklische Ether (z.B. Tetrahydrofuran, Dioxane, Dimethoxyethan), Alkohole (z.B. Ethanol, Methanol) oder Ester (Essigester) verwendet werden oder die Reaktion kann in Mischungen von zwei oder mehreren dieser Lösungsmittel ausgeführt werden. Die Verwendung polarer Lösungsmittel wie z.B. Ethanol ist bevorzugt.

Die Reaktion wird normalerweise bei Temperaturen von 0 °C bis zum Siedepunkt des Lösungsmittels durchgeführt, vorzugsweise unter Rückfluss. Die Reaktionszeit variiert in Abhängigkeit vom Maßstab der Reaktion und der Reaktionstemperatur, aber liegt im Allgemeinen zwischen einer halben Stunde und 72 Stunden. Die Reaktion kann in einer Mikrowellenapparatur (z.B. CEM Explorer) bei erhöhter Temperatur durchgeführt werden, wobei sich die benötigte Reaktionszeit verkürzen kann.

### Schritt (V23)

Eine Möglichkeit zur Synthese von Verbindungen der Formel **[XXXIII]** ist in Schema 4 gezeigt.

Verbindungen der generellen Formel **[XXXIII]** werden durch Reaktion von Verbindungen der generellen Formel **[XXXII]** mit Halogendifluormethanverbindungen (wie z.B. Chlordifluormethan, Natrium chlordifluoracetat) nach literaturbekannten Methoden (z.B. US5861359, Org. Lett. 2006, 8, 17, 3805-3808) erhalten.

Die Reaktion wird in Gegenwart einer Base wie z.B. Kaliumcarbonat durchgeführt.

Als Lösungsmittel können alle üblichen, unter den Reaktionsbedingungen inerten Lösungsmittel, wie z.B. Amide (z.B. Dimethylformamid, Dimethylacetamid, N-Methylpyrrolidon), zyklische und azyklische Ether (z.B. Tetrahydrofuran, Dioxane, Dimethoxyethan) oder Nitrile (z.B. Acetonitril) verwendet werden.

Die Reaktion wird normalerweise bei Temperaturen von 25°C bis zum Siedepunkt des Lösungsmittels durchgeführt. Die Reaktionszeit variiert in Abhängigkeit vom Maßstab der Reaktion und der Reaktionstemperatur, aber liegt im Allgemeinen zwischen einer halben Stunde und 72 Stunden.

### Schritt (V24)

Eine Möglichkeit zur Synthese von Verbindungen der Formel **[VI-b]** ist in Schema 4 gezeigt.

Verbindungen der generellen Formel **[VI-b]** werden durch Halogenierung von Pyrazolen der Formel **[XXXIII]** nach literaturbekannten Vorschriften (z.B. US6482774) erhalten.

Als Halogenierungsmittel können z.B. N-Bromsuccinimid oder Brom verwendet werden.

Als Lösungsmittel für die Halogenierungsreaktion können alle üblichen, unter den Reaktionsbedingungen inerten Lösungsmittel, wie z.B. Amide (z.B. Dimethylformamid, Dimethylacetamid, N-Methylpyrrolidon), halogenierte Kohlenwasserstoffe (z.B. Dichlormethan, Chloroform, Tetrachlorkohlenstoff) oder Essigsäure verwendet werden oder die Reaktion kann in Mischungen von zwei oder mehreren dieser Lösungsmittel ausgeführt werden. Die Wahl der Lösungsmittel kann je nach verwendetem Halogenierungsreagenz variieren. Die bevorzugten Lösungsmittel sind Dichlormethan und Tetrachlormethan.

Die Halogenierungsreaktion wird normalerweise bei Temperaturen von 0 °C - 100 °C durchgeführt und vorzugsweise bei 20 °C - 80 °C. Die Reaktionszeit variiert in Abhängigkeit vom Maßstab der Reaktion und der Reaktionstemperatur, aber liegt im Allgemeinen zwischen einigen Minuten und 48 Stunden.

Nach Beendigung der Halogenierungsreaktion, werden die Rohprodukte von der Reaktionsmischung durch eine der üblichen Trenntechniken getrennt. Falls notwendig werden die Verbindungen durch Umkristallisation, Destillation oder Chromatographie gereinigt oder können gegebenenfalls auch direkt zur weiteren Umsetzung ohne vorhergehende Reinigung eingesetzt werden.

### Schritt (V25)

Eine Möglichkeit zur Synthese von Verbindungen der Formel **[VI-c]** ist in Schema 4 gezeigt.

Verbindungen der generellen Formel **[VI-c]** werden durch Etherspaltung von Pyrazolen der Formel **[VI-b],** wobei R² für 4-Fluor-2-methoxyphenyl steht, nach literaturbekannten Vorschriften (z.B. WO2007/105058) erhalten.

Die Reaktion wird in Gegenwart einer Lewissäure z.B. Bortribromid und eines unter den Reaktionsbedingungen inerten Lösungsmittels (z.B. Dichlormethan) durchgeführt. Die Reaktion wird üblicherweise bei Temperaturen von -20°C +20°C durchgeführt, vorzugsweise bei -5 °C bis 0 °C.

### Schritt (V26)

Eine Möglichkeit zur Synthese von Verbindungen der Formel **[III]** ist in Schema 6 gezeigt.

Verbindungen der Formel **[III]** lassen sich z.B. durch Kupplung der Halopyrazole **[VI]** mit metallierten Heterocyclen der Formel **[XV-a]** (wobei Met für einen Boronester oder Boronsäure wie z.B. B(OiPr)₃, B(OH)₂ steht) in Gegenwart eines Katalysators, einer Base, gegebenenfalls eines Liganden und eines geeigneten Lösungsmittels bei geeigneten Temperaturen nach bekannten Literaturvorschriften herstellen *(*Top. Curr. Chem. 2002, 219, 11; Organomet. Chem. 1999, 28, 147 und darin zitierte Literatur, Org. Lett. 2005, 7, 21, 4753-4756).

Die Herstellung der Verbindungen des Typs **[VI]** ist unter Schritt **(V6)** beschrieben.

Die Auswahl von Lösungsmittel, zugesetzter Base, Temperatur, Katalysators und gegebenenfalls zugesetztem Liganden kann je nach eingesetzten Substrat **[VI]** varriieren und umfasst die unter Schritt **(V6)** beschriebenen Variationsmöglichkeiten für die C-C-Kupplung von Verbindung der Formel **[VI].**

### Schritt (V27)

Eine Möglichkeit zur Synthese von Verbindungen der Formel **[XLI]** ist in Schema 8 gezeigt.

Verbindungen der generellen Formel **[XLI]** werden durch Oxidation von Thioalkylpyrimidinen der Formel **[XL]** nach literaturbekannten Vorschriften (z.B. WO2009/16460 oder WO2007/24843) erhalten.

Als Oxidationsmittel können z.B. m-Chlorperbenzoesäure (m-CPBA) oder Oxone (Kaliumperoxomonosulfat) verwendet werden.

Als Lösungsmittel für die Oxidationsreaktion können alle üblichen, unter den Reaktionsbedingungen inerten Lösungsmittel, wie z.B. halogenierte Kohlenwasserstoffe (z.B. Dichlormethan), Ether (z.B. Tetrahydrofuran), Alkohole (z.B. Methanol) oder Wasser verwendet werden oder die Reaktion kann in Mischungen von zwei oder mehreren dieser Lösungsmittel ausgeführt werden. Die Wahl der Lösungsmittel kann je nach verwendetem Oxidationsreagenz variieren. Die bevorzugten Lösungsmittel sind Dichlormethan (m-CPBA) und Wasser/THF Mischungen (Oxon).

Die Oxidationsreaktion wird normalerweise bei Temperaturen von 0 °C bis 20 °C durchgeführt. Die Reaktionszeit variiert in Abhängigkeit vom Maßstab der Reaktion und der Reaktionstemperatur, aber liegt im Allgemeinen zwischen einigen Stunden und 48 Stunden.

Nach Beendigung der Oxidationsreaktion werden die Rohprodukte von der Reaktionsmischung durch eine der üblichen Trenntechniken getrennt. Falls notwendig, werden die Verbindungen durch Umkristallisation, Destillation oder Chromatographie gereinigt oder können gegebenenfalls auch direkt zur weiteren Umsetzung ohne vorhergehende Reinigung eingesetzt werden.

### Schritt (V28)

Eine Möglichkeit zur Synthese von Verbindungen der Formel **[I-f]** ist in Schema 8 gezeigt.

Verbindungen der generellen Formel **[I-f]** werden durch Reaktion von Verbindungen der generellen Formel **[XLI]** mit primären oder sekundären Aminen nach literaturbekannten Methoden (z.B. WO2007/105058 oder US6423713) erhalten.

Die Reaktion wird gegebenenfalls in Gegenwart eines Salzes wie z.B. Cäsiumfluorid durchgeführt.

Als Lösungsmittel können alle üblichen, unter den Reaktionsbedingungen inerten Lösungsmittel, wie z.B. Amide (z.B. Dimethylformamid, Dimethylacetamid, N-Methylpyrrolidon), zyklische und azyklische Ether (z.B. Tetrahydrofuran, Dioxane, Dimethoxyethan), Nitrile (z.B. Acetonitril), Sulfoxide (z.B. Dimethylsulfoxid) oder Alkohole (z.B. Ethanol, n-Butanol) verwendet werden. Alternativ kann die Reaktion ohne Lösungsmittel durchgeführt werden, z.B. bei Verwendung eines Überschusses an Amin.

Die Reaktion wird normalerweise bei Temperaturen von 50°C bis zum Siedepunkt des Lösungsmittels durchgeführt. Die Reaktionszeit variiert in Abhängigkeit vom Maßstab der Reaktion und der Reaktionstemperatur, aber liegt im Allgemeinen zwischen einer halben Stunde und 72 Stunden. Die Reaktion kann in einer Mikrowellenapparatur (z.B. CEM Explorer) bei erhöhter Temperatur durchgeführt werden, wobei sich die benötigte Reaktionszeit verkürzen kann.

Analog zur im Schema 8 beschriebenen Synthese der Pyrazole **[I-f],** kann mit diesem Verfahren die im Schema 9 beschriebene Synthese der Pyrazole **[XLIV]** aus den Verbindungen des Typs **[XLIII]** erfolgen.

### Schritt (V29)

Eine Möglichkeit zur Synthese von Verbindungen der Formel **[III-a]** ist in Schema 8 gezeigt.

Verbindungen der generellen Formel **[III-a]** werden durch Dealkylierung von Verbindungen der generellen Formel **[I-f]** in denen
- R^{x1}: für H und
- R^{x2}: für Benzyl, 4-Methoxybenzyl, oder 3,4-Dimethoxybenzyl steht,
nach literaturbekannten Methoden (z.B. J. Med. Chem. 1999, 42, 12, 2180-2190 oder Bioorg. Med. Chem. Lett. 2008, 18, 14, 4006-4010) erhalten.

Die Reaktion wird üblicherweise in Gegenwart einer starken Säure z.B. Schwefelsäure, Salzsäure oder Trifluoressigsäure durchgeführt.

Die Reaktion wird normalerweise bei Temperaturen von 0°C bis zu 120°C durchgeführt. Die Reaktionszeit variiert in Abhängigkeit vom Maßstab der Reaktion und der Reaktionstemperatur, aber liegt im Allgemeinen zwischen einer halben Stunde und 72 Stunden. Die Reaktion kann in einer Mikrowellenapparatur (z.B. CEM Explorer) bei erhöhter Temperatur durchgeführt werden, wobei sich die benötigte Reaktionszeit verkürzen kann.

Ein weiterer Gegenstand der Erfindung betrifft die nichtmedizinische Verwendung der erfindungsgemäßen Phenylpyri(mi)dinylazole oder Mischungen dieser zum Bekämpfen unerwünschter Mikroorganismen sowie zur Reduktion von Mykotoxinen in Pflanzen und Pflanzenteilen.

Ein weiterer Gegenstand der Erfindung betrifft ein Mittel zum Bekämpfen unerwünschter Mikroorganismen sowie zur Reduktion von Mykotoxinen in Pflanzen und Pflanzenteilen., umfassend wenigstens ein Phenylpyri(mi)dinylazol gemäß der vorliegenden Erfindung.

Außerdem betrifft die Erfindung ein Verfahren zum Bekämpfen unerwünschter Mikroorganismen sowie zur Reduktion von Mykotoxinen in Pflanzen und Pflanzenteilen, dadurch gekennzeichnet, dass die erfindungsgemäßen Phenylpyri(mi)dinylazole auf die Mikroorganismen und/oder in deren Lebensraum ausgebracht werden.

Die erfindungsgemäßen Stoffe weisen eine starke mikrobizide Wirkung auf und können zur Bekämpfung von unerwünschten Mikroorganismen, wie Fungi und Bakterien, im Pflanzenschutz und im Materialschutz eingesetzt werden.

Die erfindungsgemäßen Phenylpyri(mi)dinylazole der Formel (**Ia**) und (**Ib**) besitzen sehr gute fungizide Eigenschaften und lassen sich im Pflanzenschutz beispielsweise zur Bekämpfung von Plasmodiophoromyceten, Oomyceten, Chytridiomyceten, Zygomyceten, Ascomyceten, Basidiomyceten und Deuteromyceten einsetzen.

Bakterizide lassen sich im Pflanzenschutz beispielsweise zur Bekämpfung von Pseudomonadaceae, Rhizobiaceae, Enterobacteriaceae, Corynebacteriaceae und Streptomycetaceae einsetzen.

Die erfindungsgemäßen fungiziden Mittel können zur Bekämpfung von phytopathogenen Pilzen kurativ oder protektiv eingesetzt werden. Die Erfindung betrifft daher auch kurative und protektive Verfahren zum Bekämpfen von phytopathogenen Pilzen durch die Verwendung der erfindungsgemäßen Wirkstoffe oder Mittel, welche auf das Saatgut, die Pflanze oder Pflanzenteile, die Früchten oder den Boden, in welcher die Pflanzen wachsen, ausgebracht.

Die erfindungsgemäßen Mittel zum Bekämpfen von phytopathogenen Pilzen im Pflanzenschutz umfassen eine wirksame, aber nicht-phytotoxische Menge der erfindungsgemäßen Wirkstoffe. "Wirksame, aber nicht-phytotoxische Menge" bedeutet eine Menge des erfindungsgemäßen Mittels, die ausreichend ist, um die Pilzerkrankung der Pflanze ausreichend zu kontrollieren oder ganz abzutöten und die gleichzeitig keine nennenswerten Symptome von Phytotoxizität mit sich bringt. Diese Aufwandmenge kann im Allgemeinen in einem größeren Bereich variieren. Sie hängt von mehreren Faktoren ab, z.B. vom zu bekämpfenden Pilz, der Pflanze, den klimatischen Verhältnissen und den Inhaltsstoffen der erfindungsgemäßen Mittel.

Erfindungsgemäß können alle Pflanzen und Pflanzenteile behandelt werden. Unter Pflanzen werden hierbei alle Pflanzen und Pflanzenpopulationen verstanden, wie erwünschte und unerwünschte Wildpflanzen oder Kulturpflanzen (einschließlich natürlich vorkommender Kulturpflanzen). Kulturpflanzen können Pflanzen sein, die durch konventionelle Züchtungs- und Optimierungsmethoden oder durch biotechnologische und gentechnologische Methoden oder Kombinationen dieser Methoden erhalten werden können, einschließlich der transgenen Pflanzen und einschließlich der durch Sortenschutzrechte schützbaren oder nicht schützbaren Pflanzensorten. Unter Pflanzenteilen sollen alle oberirdischen und unterirdischen Teile und Organe der Pflanzen, wie Spross, Blatt, Blüte und Wurzel verstanden werden, wobei beispielhaft Blätter, Nadeln, Stängel, Stämme, Blüten, Fruchtkörper, Früchte und Samen sowie Wurzeln, Knollen und Rhizome aufgeführt werden. Zu den Pflanzenteilen gehört auch Erntegut sowie vegetatives und generatives Vermehrungsmaterial, beispielsweise Stecklinge, Knollen, Rhizome, Ableger und Samen.

Als Pflanzen, welche erfindungsgemäß behandelt werden können, seien folgende erwähnt: Baumwolle, Flachs, Weinrebe, Obst, Gemüse, wie *Rosaceae sp.* (beispielsweise Kernfrüchte wie Apfel und Birne, aber auch Steinfrüchte wie Aprikosen, Kirschen, Mandeln und Pfirsiche und Beerenfrüchte wie Erdbeeren), *Ribesioidae sp., Juglandaceae sp., Betulaceae sp., Anacardiaceae sp., Fagaceae sp., Moraceae sp., Oleaceae sp., Actinidaceae sp., Lauraceae sp., Musaceae sp.* (beispielsweise Bananenbäume und -plantagen), *Rubiaceae sp.* (beispielsweise Kaffee), *Theaceae sp., Sterculiceae sp., Rutaceae sp.* (beispielsweise Zitronen, Orangen und Grapefruit); *Solanaceae sp.* (beispielsweise Tomaten), *Liliaceae sp., Asteraceae sp.* (beispielsweise Salat), *Umbelliferae sp., Cruciferae sp., Chenopodiaceae sp., Cucurbitaceae sp.* (beispielsweise Gurke), *Alliaceae sp.* (beispielsweise Lauch, Zwiebel), *Papilionaceae sp.* (beispielsweise Erbsen); Hauptnutzpflanzen, wie *Gramineae sp.* (beispielsweise Mais, Rasen, Getreide wie Weizen, Roggen, Reis, Gerste, Hafer, Hirse und Triticale), *Asteraceae sp.* (beispielsweise Sonnenblume), *Brassicaceae sp.* (beispielsweise Weißkohl, Rotkohl, Brokkoli, Blumenkohl, Rosenkohl, Pak Choi, Kohlrabi, Radieschen sowie Raps, Senf, Meerrettich und Kresse), *Fabacae sp.* (beispielsweise Bohne, Erdnüsse), *Papilionaceae sp.* (beispielsweise Sojabohne), *Solanaceae sp.* (beispielsweise Kartoffeln), *Chenopodiaceae sp.* (beispielsweise Zuckerrübe, Futterrübe, Mangold, Rote Rübe); Nutzpflanzen und Zierpflanzen in Garten und Wald; sowie jeweils genetisch modifizierte Arten dieser Pflanzen. Bevorzugt werden Getreidepflanzen erfindungsgemäß behandelt.

Beispielhaft, aber nicht begrenzend, seien einige Erreger von pilzlichen Erkrankungen, die erfindungsgemäß behandelt werden können, genannt:
Erkrankungen, hervorgerufen durch Erreger des Echten Mehltaus wie z.B. Blumeria-Arten, wie beispielsweise Blumeria graminis; Podosphaera-Arten, wie beispielsweise Podosphaera leucotricha; Sphaerotheca-Arten, wie beispielsweise Sphaerotheca fuliginea; Uncinula-Arten, wie beispielsweise Uncinula necator;
Erkrankungen, hervorgerufen durch Erreger von Rostkrankheiten wie z.B. Gymnosporangium-Arten, wie beispielsweise Gymnosporangium sabinae; Hemileia-Arten, wie beispielsweise Hemileia vastatrix; Phakopsora-Arten, wie beispielsweise Phakopsora pachyrhizi und Phakopsora meibomiae; Puccinia-Arten, wie beispielsweise Puccinia recondita oder Puccinia triticina; Uromyces-Arten, wie beispielsweise Uromyces appendiculatus;
Erkrankungen, hervorgerufen durch Erreger der Gruppe der Oomyceten wie z.B. Bremia-Arten, wie beispielsweise Bremia lactucae; Peronospora-Arten, wie beispielsweise Peronospora pisi oder P. brassicae; Phytophthora-Arten, wie beispielsweise Phytophthora infestans; Plasmopara-Arten, wie beispielsweise Plasmopara viticola; Pseudoperonospora-Arten, wie beispielsweise Pseudoperonospora humuli oder Pseudoperonospora cubensis; Pythium-Arten, wie beispielsweise Pythium ultimum;
Blattfleckenkrankheiten und Blattwelken, hervorgerufen durch z.B. Alternaria-Arten, wie beispielsweise Alternaria solani; Cercospora-Arten, wie beispielsweise Cercospora beticola; Cladiosporum-Arten, wie beispielsweise Cladiosporium cucumerinum; Cochliobolus-Arten, wie beispielsweise Cochliobolus sativus (Konidienform: Drechslera, Syn: Helminthosporium); Colletotrichum-Arten, wie beispielsweise Colletotrichum lindemuthanium; Cycloconium-Arten, wie beispielsweise Cycloconium oleaginum; Diaporthe-Arten, wie beispielsweise Diaporthe citri; Elsinoe-Arten, wie beispielsweise Elsinoe fawcettii; Gloeosporium-Arten, wie beispielsweise Gloeosporium laeticolor; Glomerella-Arten, wie beispielsweise Glomerella cingulata; Guignardia-Arten, wie beispielsweise Guignardia bidwelli; Leptosphaeria-Arten, wie beispielsweise Leptosphaeria maculans; Magnaporthe-Arten, wie beispielsweise Magnaporthe grisea; Microdochium-Arten, wie beispielsweise Microdochium nivale; Mycosphaerella-Arten, wie beispielsweise Mycosphaerella graminicola und M. fijiensis; Phaeosphaeria-Arten, wie beispielsweise Phaeosphaeria nodorum; Pyrenophora-Arten, wie beispielsweise Pyrenophora teres; Ramularia-Arten, wie beispielsweise Ramularia collo-cygni; Rhynchosporium-Arten, wie beispielsweise Rhynchosporium secalis; Septoria-Arten, wie beispielsweise Septoria apii; Typhula-Arten, wie beispielsweise Typhula incarnata; Venturia-Arten, wie beispielsweise Venturia inaequalis;
Wurzel- und Stängelkrankheiten, hervorgerufen durch z.B. Corticium-Arten, wie beispielsweise Corticium graminearum; Fusarium-Arten, wie beispielsweise Fusarium oxysporum; Gaeumannomyces-Arten, wie beispielsweise Gaeumannomyces graminis; Rhizoctonia-Arten, wie beispielsweise Rhizoctonia solani; Tapesia-Arten, wie beispielsweise Tapesia acuformis; Thielaviopsis-Arten, wie beispielsweise Thielaviopsis basicola;
Ähren- und Rispenerkrankungen (inklusive Maiskolben), hervorgerufen durch z.B. Alternaria-Arten, wie beispielsweise Alternaria spp.; Aspergillus-Arten, wie beispielsweise Aspergillus flavus; Cladosporium-Arten, wie beispielsweise Cladosporium cladosporioides; Claviceps-Arten, wie beispielsweise Claviceps purpurea; Fusarium-Arten, wie beispielsweise Fusarium culmorum; Gibberella-Arten, wie beispielsweise Gibberella zeae; Monographella-Arten, wie beispielsweise Monographella nivalis; Septoria-Arten, wie beispielsweise Septoria nodorum;
Erkrankungen, hervorgerufen durch Brandpilze wie z.B. Sphacelotheca-Arten, wie beispielsweise Sphacelotheca reiliana; Tilletia-Arten, wie beispielsweise Tilletia caries, T. controversa; Urocystis-Arten, wie beispielsweise Urocystis occulta; Ustilago-Arten, wie beispielsweise Ustilago nuda, U. nuda tritici;
Fruchtfäule hervorgerufen durch z.B. Aspergillus-Arten, wie beispielsweise Aspergillus flavus; Botrytis-Arten, wie beispielsweise Botrytis cinerea; Penicillium-Arten, wie beispielsweise Penicillium expansum und P. purpurogenum; Sclerotinia-Arten, wie beispielsweise Sclerotinia sclerotiorum;
Verticilium-Arten, wie beispielsweise Verticilium alboatrum;
Samen- und bodenbürtige Fäulen und Welken, sowie Sämlingserkrankungen, hervorgerufen durch z.B. Fusarium-Arten, wie beispielsweise Fusarium culmorum; Phytophthora Arten, wie beispielsweise Phytophthora cactorum; Pythium-Arten, wie beispielsweise Pythium ultimum; Rhizoctonia-Arten, wie beispielsweise Rhizoctonia solani; Sclerotium-Arten, wie beispielsweise Sclerotium rolfsii;
Krebserkrankungen, Gallen und Hexenbesen, hervorgerufen durch z.B. Nectria-Arten, wie beispielsweise Nectria galligena;
Welkeerkrankungen hervorgerufen durch z.B. Monilinia-Arten, wie beispielsweise Monilinia laxa;
Deformationen von Blättern, Blüten und Früchten, hervorgerufen durch z.B. Taphrina-Arten, wie beispielsweise Taphrina deformans;
Degenerationserkrankungen holziger Pflanzen, hervorgerufen durch z.B. Esca-Arten, wie beispielsweise Phaemoniella clamydospora und Phaeoacremonium aleophilum und Fomitiporia mediterranea;
Blüten- und Samenerkrankungen, hervorgerufen durch z.B. Botrytis-Arten, wie beispielsweise Botrytis cinerea;
Erkrankungen von Pflanzenknollen, hervorgerufen durch z.B. Rhizoctonia-Arten, wie beispielsweise Rhizoctonia solani; Helminthosporium-Arten, wie beispielsweise Helminthosporium solani;
Erkrankungen, hervorgerufen durch bakterielle Erreger wie z.B. Xanthomonas-Arten, wie beispielsweise Xanthomonas campestris pv. oryzae; Pseudomonas-Arten, wie beispielsweise Pseudomonas syringae pv. lachrymans; Erwinia-Arten, wie beispielsweise Erwinia amylovora;
Bevorzugt können die folgenden Krankheiten von Soja-Bohnen bekämpft werden:
   Pilzkrankheiten an Blättern, Stängeln, Schoten und Samen verursacht durch z.B. Alternaria leaf spot (Alternaria spec. atrans tenuissima), Anthracnose (Colletotrichum gloeosporoides dematium var. truncatum), Brown spot (Septoria glycines), Cercospora leaf spot and blight (Cercospora kikuchii), Choanephora leaf blight (Choanephora infundibulifera trispora (Syn.)), Dactuliophora leaf spot (Dactuliophora glycines), Downy Mildew (Peronospora manshurica), Drechslera blight (Drechslera glycini), Frogeye Leaf spot (Cercospora sojina), Leptosphaerulina Leaf Spot (Leptosphaerulina trifolii), Phyllostica Leaf Spot (Phyllosticta sojaecola), Pod and Stem Blight (Phomopsis sojae), Powdery Mildew (Microsphaera diffusa), Pyrenochaeta Leaf Spot (Pyrenochaeta glycines), Rhizoctonia Aerial, Foliage, and Web Blight (Rhizoctonia solani), Rust (Phakopsora pachyrhizi, Phakopsora meibomiae), Scab (Sphaceloma glycines), Stemphylium Leaf Blight (Stemphylium botryosum), Target Spot (Corynespora cassiicola).

Pilzkrankheiten an Wurzeln und der Stängelbasis verursacht durch z.B. Black Root Rot (Calonectria crotalariae), Charcoal Rot (Macrophomina phaseolina), Fusarium Blight or Wilt, Root Rot, and Pod and Collar Rot (Fusarium oxysporum, Fusarium orthoceras, Fusarium semitectum, Fusarium equiseti), Mycoleptodiscus Root Rot (Mycoleptodiscus terrestris), Neocosmospora (Neocosmopspora vasinfecta), Pod and Stem Blight (Diaporthe phaseolorum), Stem Canker (Diaporthe phaseolorum var. caulivora), Phytophthora Rot (Phytophthora megasperma), Brown Stem Rot (Phialophora gregata), Pythium Rot (Pythium aphanidermatum, Pythium irregulare, Pythium debaryanum, Pythium myriotylum, Pythium ultimum), Rhizoctonia Root Rot, Stem Decay, and Damping-Off (Rhizoctonia solani), Sclerotinia Stem Decay (Sclerotinia sclerotiorum), Sclerotinia Southern Blight (Sclerotinia rolfsii), Thielaviopsis Root Rot (Thielaviopsis basicola).

Unter unerwünschten Mikroorganismen sind im vorliegenden Fall phytopathogene Pilze und Bakterien zu verstehen. Die erfindungsgemäßen Stoffe können also eingesetzt werden, um Pflanzen innerhalb eines gewissen Zeitraumes nach der Behandlung gegen den Befall durch die genannten Schaderreger zu schützen. Der Zeitraum, innerhalb dessen Schutz herbeigeführt wird, erstreckt sich im allgemeinen von 1 bis 10 Tage, vorzugsweise 1 bis 7 Tage nach der Behandlung der Pflanzen mit den Wirkstoffen.

Die gute Pflanzenverträglichkeit der Wirkstoffe in den zur Bekämpfung von Pflanzenkrankheiten notwendigen Konzentrationen erlaubt eine Behandlung von oberirdischen Pflanzenteilen, von Pflanz- und Saatgut, und des Bodens.

Dabei lassen sich die erfindungsgemäßen Wirkstoffe mit besonders gutem Erfolg zur Bekämpfung von Getreidekrankheiten, wie beispielsweise gegen Erysiphe-Arten, gegen Puccinia und gegen Fusarien-Arten, von Reiskrankheiten, wie beispielsweise gegen Pyricularia und Rhizoctonia und von Krankheiten im Wein-, Obst-und Gemüseanbau, wie beispielsweise gegen Botrytis-, Venturia-, Sphaerotheca-und Podosphaera-Arten, einsetzen.

Die erfindungsgemäßen Wirkstoffe eignen sich auch zur Steigerung des Ernteertrages. Sie sind außerdem mindertoxisch und weisen eine gute Pflanzenverträglichkeit auf.

Die erfindungsgemäßen Verbindungen können gegebenenfalls in bestimmten Konzentrationen bzw. Aufwandmengen auch als Herbizide, Safener, Wachstumsregulatoren oder Mittel zur Verbesserung der Pflanzeneigenschaften, oder als Mikrobizide, beispielsweise als Fungizide, Antimykotika, Bakterizide, Virizide (einschließlich Mittel gegen Viroide) oder als Mittel gegen MLO (Mycoplasma-like-organism) und RLO (Rickettsia-like-organism) verwendet werden. Sie lassen sich gegebenenfalls auch als Insektizide verwenden. Sie lassen sich gegebenenfalls auch als Zwischen- oder Vorprodukte für die Synthese weiterer Wirkstoffe einsetzen.

Die erfindungsgemäßen Wirkstoffe können gegebenenfalls in bestimmten Konzentrationen und Aufwandmengen auch als Herbizide, zur Beeinflussung des Pflanzenwachstums, sowie zur Bekämpfung von tierischen Schädlingen verwendet werden. Sie lassen sich gegebenenfalls auch als Zwischen-und Vorprodukte für die Synthese weiterer Wirkstoffe einsetzen.

Die erfindungsgemäßen Wirkstoffe eignen sich bei guter Pflanzenverträglichkeit, günstiger Warmblütertoxizität und guter Umweltverträglichkeit zum Schutz von Pflanzen und Pflanzenorganen, zur Steigerung der Ernteerträge, Verbesserung der Qualität des Erntegutes. Sie können vorzugsweise als Pflanzenschutzmittel eingesetzt werden. Sie sind gegen normal sensible und resistente Arten sowie gegen alle oder einzelne Entwicklungsstadien wirksam.

Die erfindungsgemäße Behandlung der Pflanzen und Pflanzenteile mit den Wirkstoffen bzw. Mitteln erfolgt direkt oder durch Einwirkung auf deren Umgebung, Lebensraum oder Lagerraum nach den üblichen Behandlungsmethoden, z.B. durch Tauchen, (Ver-)Spritzen, (Ver-)Sprühen, Berieseln, Verdampfen, Zerstäuben, Vernebeln, (Ver-)Streuen, Verschäumen, Bestreichen, Verstreichen, Gießen (drenchen), Tröpfchenbewässerung und bei Vermehrungsmaterial, insbesondere bei Samen, weiterhin durch Trockenbeizen, Nassbeizen, Schlämmbeizen, Inkrustieren, ein- oder mehrschichtiges Umhüllen usw. Es ist ferner möglich, die Wirkstoffe nach dem Ultra-Low-Volume-Verfahren auszubringen oder die Wirkstoffzubereitung oder den Wirkstoff selbst in den Boden zu injizieren.

Die angewandte Wirkstoffmenge kann in einem größeren Bereich schwanken. Sie hängt im wesentlichen von der Art des gewünschten Effektes ab. Im Allgemeinen liegen die Aufwandmengen zwischen 1 g und 10 kg Wirkstoff pro Hektar Bodenfläche, vorzugsweise zwischen 5 g und 5 kg pro ha.

Der vorteilhafte Effekt der Kulturpflanzen-Verträglichkeit der erfindungsgemäßen Wirkstoffe ist bei bestimmten Konzentrationsverhältnissen besonders stark ausgeprägt. Jedoch können die Gewichtsverhältnisse der Wirkstoffe in den Wirkstoffkombinationen in relativ großen Bereichen variiert werden. Im allgemeinen entfallen auf 1 Gewichtsteil Wirkstoff der Formel (I) 0,001 bis 1000 Gewichtsteile, vorzugsweise 0,01 bis 100 Gewichtsteile, besonders bevorzugt 0,05 bis 20 Gewichtsteile einer der oben unter (b') genannten, die Kulturpflanzen Verträglichkeit verbessernden Verbindungen (Antidots/Safener).

Die erfindungsgemäßen Wirkstoffe werden im allgemeinen in Form von Fertigformulierungen zur Anwendung gebracht. Die in den Wirkstoffkombinationen enthaltenen Wirkstoffe können aber auch in Einzelformulierungen bei der Anwendung gemischt, d.h. in Form von Tankmischungen zur Anwendung gebracht werden.

Darüber hinaus kann durch die erfindungsgemäße Behandlung der Mykotoxingehalt im Erntegut und den daraus hergestellten Nahrungs- und Futtermitteln verringert werden. Besonders, aber nicht ausschließlich sind hierbei folgende Mykotoxine zu nennen: Deoxynivalenol (DON), Nivalenol, 15-Ac-DON, 3-Ac-DON, T2- und HT2- Toxin, Fumonisine, Zearalenon, Moniliformin, Fusarin, Diacetoxyscirpenol (DAS), Beauvericin, Enniatin, Fusaroproliferin, Fusarenol, Ochratoxine, Patulin, Mutterkornalkaloide und Aflatoxine, die beispielsweise von den folgenden Pilzen verursacht werden können: Fusarium spec., wie Fusarium acuminatum, F. avenaceum, F. crookwellense, F. culmorum, F. graminearum (Gibberella zeae), F. equiseti, F. fujikoroi, F. musarum, F. oxysporum, F. proliferatum, F. poae, F. pseudograminearum, F. sambucinum, F. scirpi, F. semitectum, F. solani, F. sporotrichoides, F. langsethiae, F. subglutinans, F. tricinctum, F. verticillioides u.a. sowie auch von Aspergillus spec., Penicillium spec., Claviceps purpurea, Stachybotrys spec. u.a.

Die erfindungsgemäßen Wirkstoffe bzw. Mittel können außerdem im Materialschutz zum Schutz von technischen Materialien gegen Befall und Zerstörung durch unerwünschten Mikroorganismen, wie z.B. Pilzen, eingesetzt werden.

Unter technischen Materialien sind im vorliegenden Zusammenhang nichtlebende Materialien zu verstehen, die für die Verwendung in der Technik zubereitet worden sind. Beispielsweise können technische Materialien, die durch erfindungsgemäße Wirkstoffe vor mikrobieller Veränderung oder Zerstörung geschützt werden sollen, Klebstoffe, Leime, Papier und Karton, Textilien, Leder, Holz, Anstrichmittel und Kunststoffartikel, Kühlschmierstoffe und andere Materialien sein, die von Mikroorganismen befallen oder zersetzt werden können. Im Rahmen der zu schützenden Materialien seien auch Teile von Produktionsanlagen, beispielsweise Kühlwasserkreisläufe, genannt, die durch Vermehrung von Mikroorganismen beeinträchtigt werden können. Im Rahmen der vorliegenden Erfindung seien als technische Materialien vorzugsweise Klebstoffe, Leime, Papiere und Kartone, Leder, Holz, Anstrichmittel, Kühlschmiermittel und Wärmeübertragungsflüssigkeiten genannt, besonders bevorzugt Holz. Die erfindungsgemäßen Wirkstoffe bzw. Mittel können nachteilige Effekte wie Vermodern, Verfall, Ver-, Entfärbung oder Verschimmeln verhindern.

Das erfindungsgemäße Verfahren zum Bekämpfen von unerwünschten Pilzen kann auch zum Schutz von so genannte Storage Goods verwendet werden. Unter "Storage Goods" werden dabei natürliche Substanzen pflanzlichen oder tierischen Ursprungs oder deren Verarbeitungsprodukte, welche der Natur entnommen wurden und für die Langzeitschutz gewünscht ist, verstanden. Storage Goods pflanzlichen Ursprungs, wie z.B. Pflanzen oder Pflanzenteile, wie Stiele, Blätter, Knollen, Samen, Früchte, Körner, können in frisch geerntetem Zustand oder nach Verarbeitung durch (Vor-)Trocknen, Befeuchten, Zerkleinern, Mahlen, Pressen oder Rösten, geschützt werden. Storage Goods umfasst auch Nutzholz, sei es unverarbeitet, wie Bauholz, Stromleitungsmasten und Schranken, oder in Form fertiger Produkte, wie Möbel. Storage Goods tierischen Ursprungs sind beispielsweise Felle, Leder, Pelze und Haare. Die erfindungsgemäßen Wirkstoffe können nachteilige Effekte wie Vermodern, Verfall, Ver-, Entfärbung oder Verschimmeln verhindern.

Als Mikroorganismen, die einen Abbau oder eine Veränderung der technischen Materialien bewirken können, seien beispielsweise Bakterien, Pilze, Hefen, Algen und Schleimorganismen genannt. Vorzugsweise wirken die erfindungsgemäßen Wirkstoffe gegen Pilze, insbesondere Schimmelpilze, Holz verfärbende und Holz zerstörende Pilze (Basidiomyceten) sowie gegen Schleimorganismen und Algen. Es seien beispielsweise Mikroorganismen der folgenden Gattungen genannt: Alternaria, wie Alternaria tenuis; Aspergillus, wie Aspergillus niger; Chaetomium, wie Chaetomium globosum; Coniophora, wie Coniophora puetana; Lentinus, wie Lentinus tigrinus; Penicillium, wie Penicillium glaucum; Polyporus, wie Polyporus versicolor; Aureobasidium, wie Aureobasidium pullulans; Sclerophoma, wie Sclerophoma pityophila; Trichoderma, wie Trichoderma viride; Escherichia, wie Escherichia coli; Pseudomonas, wie Pseudomonas aeruginosa; Staphylococcus, wie Staphylococcus aureus.

Die vorliegende Erfindung betrifft weiterhin ein Mittel zum Bekämpfen unerwünschter Mikroorganismen, umfassend wenigstens eines der erfindungsgemäßen Thienylaminopyrimidine. Vorzugsweise handelt es sich um fungizide Mittel, welche landwirtschaftlich verwendbare Hilfsmittel, Solventien, Trägerstoffe, oberflächenaktive Stoffe oder Streckmittel enthalten.

Erfindungsgemäß bedeutet Trägerstoff eine natürliche oder synthetische, organische oder anorganische Substanz, mit welchen die Wirkstoffe zur besseren Anwendbarkeit, v.a. zum Aufbringen auf Pflanzen oder Pflanzenteile oder Saatgut, gemischt oder verbunden sind. Der Trägerstoff, welcher fest oder flüssig sein kann, ist im Allgemeinen inert und sollte in der Landwirtschaft verwendbar sein.

Als feste Trägerstoffe kommen infrage: z.B. Ammoniumsalze und natürliche Gesteinsmehle, wie Kaoline, Tonerden, Talkum, Kreide, Quarz, Attapulgit, Montmorillonit oder Diatomeenerde und synthetische Gesteinsmehle, wie hochdisperse Kieselsäure, Aluminiumoxid und Silikate, als feste Trägerstoffe für Granulate kommen in Frage: z.B. gebrochene und fraktionierte natürliche Gesteine wie Calcit, Marmor, Bims, Sepiolith, Dolomit sowie synthetische Granulate aus anorganischen und organischen Mehlen sowie Granulate aus organischem Material wie Papier, Sägemehl, Kokosnußschalen, Maiskolben und Tabakstengeln; als Emulgier- und/oder schaumerzeugende Mittel kommen in Frage: z.B. nichtionogene und anionische Emulgatoren, wie Polyoxyethylen-Fettsäure-Ester, Polyoxyethylen-Fettalkohol-Ether, z.B. Alkylaryl-polyglykolether, Alkylsulfonate, Alkylsulfate, Arylsulfonate sowie Eiweißhydrolysate; als Dispergiermittel kommen in Frage nicht-ionische und/oder ionische Stoffe, z.B. aus den Klassen der Alkohol-POE- und/oder POP-Ether, Säure- und/oder POP-POE-Ester, Alkyl-Aryl- und/oder POP- POE-Ether, Fett- und/oder POP- POE-Addukte, POE- und/oder POP-Polyol Derivate, POE- und/oder POP-Sorbitan- oder-Zucker-Addukte, Alky- oder Aryl-Sulfate, Sulfonate und Phosphate oder die entsprechenden PO-Ether-Addukte. Ferner geeignet sind Oligo- oder Polymere, z.B. ausgehend von vinylischen Monomeren, von Acrylsäure, aus EO und/oder PO allein oder in Verbindung mit z.B. (poly-) Alkoholen oder (poly-) Aminen. Ferner können Einsatz finden Lignin und seine Sulfonsäure-Derivate, einfache und modifizierte Cellulosen, aromatische und/oder aliphatische Sulfonsäuren sowie deren Addukte mit Formaldehyd.

Die Wirkstoffe können in die üblichen Formulierungen überführt werden, wie Lösungen, Emulsionen, Spritzpulver, wasser- und ölbasierte Suspensionen, Pulver, Stäubemittel, Pasten, lösliche Pulver, lösliche Granulate, Streugranulate, Suspensions-Emulsions-Konzentrate, Wirkstoff-imprägnierte Naturstoffe, Wirkstoff-imprägnierte synthetische Stoffe, Düngemittel sowie Feinstverkapselungen in polymeren Stoffen.

Die Wirkstoffe können als solche, in Form ihrer Formulierungen oder den daraus bereiteten Anwendungsformen, wie gebrauchsfertige Lösungen, Emulsionen, wasser- oder ölbasierte Suspensionen, Pulver, Spritzpulver, Pasten, lösliche Pulver, Stäubemittel, lösliche Granulate, Streugranulate, Suspensions-Emulsions-Konzentrate, Wirkstoff-imprägnierte Naturstoffe, Wirkstoff-imprägnierte synthetische Stoffe, Düngemittel sowie Feinstverkapselungen in polymeren Stoffen angewendet werden. Die Anwendung geschieht in üblicher Weise, z.B. durch Gießen, Verspritzen, Versprühen, Verstreuen, Verstäuben, Verschäumen, Bestreichen usw. Es ist ferner möglich, die Wirkstoffe nach dem Ultra-Low-Volume-Verfahren auszubringen oder die Wirkstoffzubereitung oder den Wirkstoff selbst in den Boden zu injizieren. Es kann auch das Saatgut der Pflanzen behandelt werden.

Die genannten Formulierungen können in an sich bekannter Weise hergestellt werden, z.B. durch Vermischen der Wirkstoffe mit mindestens einem üblichen Streckmittel, Lösungs- bzw. Verdünnungsmittel, Emulgator, Dispergier- und/oder Binde- oder Fixiermittels, Netzmittel, Wasser-Repellent, gegebenenfalls Sikkative und UV-Stabilisatoren und gegebenenfalls Farbstoffen und Pigmenten, Entschäumer, Konservierungsmittel, sekundäre Verdickungsmittel, Kleber, Gibberelline sowie weiteren Verarbeitungshilfsmitteln.

Die erfindungsgemäßen Mittel umfassen nicht nur Formulierungen, welche bereits anwendungsfertig sind und mit einer geeigneten Apparatur auf die Pflanze oder das Saatgut ausgebracht werden können, sondern auch kommerzielle Konzentrate, welche vor Gebrauch mit Wasser verdünnt werden müssen.

Die erfindungsgemäßen Wirkstoffe können als solche oder in ihren (handelsüblichen) Formulierungen sowie in den aus diesen Formulierungen bereiteten Anwendungsformen in Mischung mit anderen (bekannten) Wirkstoffen, wie Insektiziden, Lockstoffen, Sterilantien, Bakteriziden, Akariziden, Nematiziden, Fungiziden, Wachstumsregulatoren, Herbiziden, Düngemitteln, Safener bzw. Semiochemicals vorliegen.

Als Hilfsstoffe können solche Stoffe Verwendung finden, die geeignet sind, dem Mittel selbst oder und/oder davon abgeleitete Zubereitungen (z.B. Spritzbrühen, Saatgutbeizen) besondere Eigenschaften zu verleihen, wie bestimmte technische Eigenschaften und/oder auch besondere biologische Eigenschaften. Als typische Hilfsmittel kommen in Frage: Streckmittel, Lösemittel und Trägerstoffe.

Als Streckmittel eignen sich z.B. Wasser, polare und unpolare organische chemische Flüssigkeiten z.B. aus den Klassen der aromatischen und nicht-aromatischen Kohlenwasserstoffe (wie Paraffine, Alkylbenzole, Alkylnaphthaline, Chlorbenzole), der Alkohole und Polyole (die ggf. auch substituiert, verethert und/oder verestert sein können), der Ketone (wie Aceton, Cyclohexanon), Ester (auch Fette und Öle) und (poly-)Ether, der einfachen und substituierten Amine, Amide, Lactame (wie N-Alkylpyrrolidone) und Lactone, der Sulfone und Sulfoxide (wie Dimethylsulfoxid).

Mit verflüssigten gasförmigen Streckmitteln oder Trägerstoffen sind solche Flüssigkeiten gemeint, welche bei normaler Temperatur und unter Normaldruck gasförmig sind, z.B. Aerosol-Treibgase, wie Halogenkohlenwasserstoffe sowie Butan, Propan, Stickstoff und Kohlendioxid.

Es können in den Formulierungen Haftmittel wie Carboxymethylcellulose, natürliche und synthetische pulverige, körnige oder latexförmige Polymere verwendet werden, wie Gummiarabicum, Polyvinylalkohol, Polyvinylacetat, sowie natürliche Phospholipide, wie Kephaline und Lecithine, und synthetische Phospholipide. Weitere Additive können mineralische und vegetabile Öle sein.

Im Falle der Benutzung von Wasser als Streckmittel können z.B. auch organische Lösungsmittel als Hilfslösungsmittel verwendet werden. Als flüssige Lösungsmittel kommen im Wesentlichen infrage: Aromaten, wie Xylol, Toluol oder Alkylnaphthaline, chlorierte Aromaten oder chlorierte aliphatische Kohlenwasserstoffe, wie Chlorbenzole, Chlorethylene oder Methylenchlorid, aliphatische Kohlenwasserstoffe, wie Cyclohexan oder Paraffine, z.B. Erdölfraktionen, Alkohole, wie Butanol oder Glycol sowie deren Ether und Ester, Ketone, wie Aceton, Methylethylketon, Methylisobutylketon oder Cyclohexanon, stark polare Lösungsmittel, wie Dimethylformamid und Dimethylsulfoxid, sowie Wasser.

Die erfindungsgemäßen Mittel können zusätzlich weitere Bestandteile enthalten, wie z.B. oberflächenaktive Stoffe. Als oberflächenaktive Stoffe kommen Emulgier- und/oder Schaum erzeugende Mittel, Dispergiermittel oder Benetzungsmittel mit ionischen oder nicht-ionischen Eigenschaften oder Mischungen dieser oberflächenaktiven Stoffe infrage. Beispiele hierfür sind Salze von Polyacrylsäure, Salze von Lignosulphonsäure, Salze von Phenolsulphonsäure oder Naphthalinsulphonsäure, Polykondensate von Ethylenoxid mit Fettalkoholen oder mit Fettsäuren oder mit Fettaminen, substituierten Phenolen (vorzugsweise Alkylphenole oder Arylphenole), Salze von Sulphobernsteinsäureestern, Taurinderivate (vorzugsweise Alkyltaurate), Phosphorsäureester von polyethoxylierten Alkoholen oder Phenole, Fettsäureester von Polyolen, und Derivate der Verbindungen enthaltend Sulphate, Sulphonate und Phosphate, z.B. Alkylarylpolyglycolether, Alkylsulfonate, Alkylsulfate, Arylsulfonate, Eiweißhydrolysate, Lignin-Sulfitablaugen und Methylcellulose. Die Anwesenheit einer oberflächenaktiven Substanz ist notwendig, wenn einer der Wirkstoffe und/oder einer der inerten Trägerstoffe nicht in Wasser löslich ist und wenn die Anwendung in Wasser erfolgt. Der Anteil an oberflächenaktiven Stoffen liegt zwischen 5 und 40 Gewichtsprozent des erfindungsgemäßen Mittels.

Es können Farbmittel wie anorganische Pigmente, z.B. Eisenoxid, Titanoxid, Ferrocyanblau und organische Farbstoffe, wie Alizarin-, Azo- und Metallphthalocyaninfarbstoffe und Spurennährstoffe, wie Salze von Eisen, Mangan, Bor, Kupfer, Kobalt, Molybdän und Zink verwendet werden.

Weitere Additive können Duftstoffe, mineralische oder vegetabile gegebenenfalls modifizierte Öle, Wachse und Nährstoffe (auch Spurennährstoffe), wie Salze von Eisen, Mangan, Bor, Kupfer, Kobalt, Molybdän und Zink sein.

Weiterhin enthalten sein können Stabilisatoren wie Kältestabilisatoren, Konservierungsmittel, Oxidationsschutzmittel, Lichtschutzmittel oder andere die chemische und / oder physikalische Stabilität verbessernde Mittel.

Gegebenenfalls können auch andere zusätzliche Komponenten enthalten sein, z.B. schützende Kolloide, Bindemittel, Klebstoffe, Verdicker, thixotrope Stoffe, Penetrationsförderer, Stabilisatoren, Sequestiermittel, Komplexbildner. Im Allgemeinen können die Wirkstoffe mit jedem festen oder flüssigen Additiv, welches für Formulierungszwecke gewöhnlich verwendet wird, kombiniert werden.

Die Formulierungen enthalten im Allgemeinen zwischen 0,05 und 99 Gew.-%, 0,01 und 98 Gew.-%, vorzugsweise zwischen 0,1 und 95 Gew.-%, besonders bevorzugt zwischen 0,5 und 90 % Wirkstoff, ganz besonders bevorzugt zwischen 10 und 70 Gewichtsprozent.

Die zuvor beschriebenen Formulierungen können in einem erfindungsgemäßen Verfahren zum Bekämpfen unerwünschter Mikroorganismen verwendet werden, bei dem die erfindungsgemäßen Thienylaminopyrimidine auf die Mikroorganismen und/oder in deren Lebensraum ausgebracht werden.

Die erfindungsgemäßen Wirkstoffe können als solche oder in ihren Formulierungen auch in Mischung mit bekannten Fungiziden, Bakteriziden, Akariziden, Nematiziden oder Insektiziden verwendet werden, um so z.B. das Wirkungsspektrum zu verbreitern oder Resistenzentwicklungen vorzubeugen.

Als Mischpartner kommen zum Beispiel bekannte Fungizide, Insektizide, Akarizide, Nematizide oder auch Bakterizide (siehe auch Pesticide Manual, 13th ed.) infrage.

Auch eine Mischung mit anderen bekannten Wirkstoffen, wie Herbiziden, oder mit Düngemitteln und Wachstumsregulatoren, Safenern bzw. Semiochemicals ist möglich.

Die Anwendung geschieht in einer den Anwendungsformen angepaßten üblichen Weise.

Die Bekämpfung von pflanzenpathogenen Schadpilzen erfolgt in erster Linie durch die Behandlung des Bodens und der oberirdischen Pflanzenteile mit Pflanzenschutzmitteln. Aufgrund der Bedenken hinsichtlich eines möglichen Einflusses der Pflanzenschutzmittel auf die Umwelt und die Gesundheit von Menschen und Tieren gibt es Anstrengungen, die Menge der ausgebrachten Wirkstoffe zu vermindern.

Die Wirkstoffe können als solche, in Form ihrer Formulierungen oder den daraus bereiteten Anwendungsformen, wie gebrauchsfertige Lösungen, Suspensionen, Spritzpulver, Pasten, lösliche Pulver, Stäubemittel und Granulate angewendet werden. Die Anwendung geschieht in üblicher Weise, z.B. durch Gießen, Verspritzen, Versprühen, Verstreuen, Verstäuben, Verschäumen, Bestreichen usw. Es ist ferner möglich, die Wirkstoffe nach dem Ultra-Low-Volume-Verfahren auszubringen oder die Wirkstoffzubereitung oder den Wirkstoff selbst in den Boden zu injizieren. Es kann auch das Saatgut der Pflanzen behandelt werden.

Beim Einsatz der erfindungsgemäßen Wirkstoffe als Fungizide können die Aufwandmengen je nach Applikationsart innerhalb eines größeren Bereiches variiert werden. Die Aufwandmenge der erfindungsgemäßen Wirkstoffe beträgt
- bei der Behandlung von Pflanzenteilen, z.B. Blättern: von 0,1 bis 10 000 g/ha, bevorzugt von 10 bis 1000 g/ha, besonders bevorzugt von 50 bis 300g/ha (bei Anwendung durch Gießen oder Tropfen kann die Aufwandmenge sogar verringert werden, vor allem wenn inerte Substrate wie Steinwolle oder Perlit verwendet werden);
- bei der Saatgutbehandlung: von 2 bis 200 g pro 100 kg Saatgut, bevorzugt von 3 bis 150 g pro 100 kg Saatgut, besonders bevorzugt von 2,5 bis 25 g pro 100 kg Saatgut, ganz besonders bevorzugt von 2,5 bis 12,5 g pro 100 kg Saatgut;
- bei der Bodenbehandlung: von 0,1 bis 10 000 g/ha, bevorzugt von 1 bis 5 000 g/ha.

Diese Aufwandmengen seien nur beispielhaft und nicht limitierend im Sinne der Erfindung genannt.

Zugleich können die erfindungsgemäßen Verbindungen zum Schutz vor Bewuchs von Gegenständen, insbesondere von Schiffskörpern, Sieben, Netzen, Bauwerken, Kaianlagen und Signalanlagen, welche mit See- oder Brackwasser in Verbindung kommen, eingesetzt werden.

Weiter können die erfindungsgemäßen Verbindungen allein oder in Kombinationen mit anderen Wirkstoffen als Antifouling-Mittel eingesetzt werden.

Das erfindungsgemäße Behandlungsverfahren kann für die Behandlung von genetisch modifizierten Organismen (GMOs), z. B. Pflanzen oder Samen, verwendet werden. Genetisch modifizierte Pflanzen (oder transgene Pflanzen) sind Pflanzen, bei denen ein heterologes Gen stabil in das Genom integriert worden ist. Der Begriff "heterologes Gen" bedeutet im wesentlichen ein Gen, das außerhalb der Pflanze bereitgestellt oder assembliert wird und das bei Einführung in das Zellkerngenom, das Chloroplastengenom oder das Hypochondriengenom der transformierten Pflanze dadurch neue oder verbesserte agronomische oder sonstige Eigenschaften verleiht, daß es ein interessierendes Protein oder Polypeptid exprimiert oder daß es ein anderes Gen, das in der Pflanze vorliegt bzw. andere Gene, die in der Pflanze vorliegen, herunterreguliert oder abschaltet (zum Beispiel mittels Antisense-Technologie, Cosuppressionstechnologie oder RNAi-Technologie [RNA Interference]). Ein heterologes Gen, das im Genom vorliegt, wird ebenfalls als Transgen bezeichnet. Ein Transgen, das durch sein spezifisches Vorliegen im Pflanzengenom definiert ist, wird als Transformations- bzw. transgenes Event bezeichnet.

In Abhängigkeit von den Pflanzenarten oder Pflanzensorten, ihrem Standort und ihren Wachstumsbedingungen (Böden, Klima, Vegetationsperiode, Ernährung) kann die erfindungsgemäße Behandlung auch zu überadditiven ("synergistischen") Effekten führen. So sind zum Beispiel die folgenden Effekte möglich, die über die eigentlich zu erwartenden Effekte hinausgehen: verringerte Aufwandmengen und/oder erweitertes Wirkungsspektrum und/oder erhöhte Wirksamkeit der Wirkstoffe und Zusammensetzungen, die erfindungsgemäß eingesetzt werden können, besseres Pflanzenwachstum, erhöhte Toleranz gegenüber hohen oder niedrigen Temperaturen, erhöhte Toleranz gegenüber Trockenheit oder Wasser- oder Bodensalzgehalt, erhöhte Blühleistung, Ernteerleichterung, Reifebeschleunigung, höhere Erträge, größere Früchte, größere Pflanzenhöhe, intensiver grüne Farbe des Blatts, frühere Blüte, höhere Qualität und/oder höherer Nährwert der Ernteprodukte, höhere Zuckerkonzentration in den Früchten, bessere Lagerfähigkeit und/oder Verarbeitbarkeit der Ernteprodukte.

Im vorliegenden Fall versteht man unter unerwünschten phytopathogenen Pilzen und/oder Mikroorganismen und/oder Viren phytopathogene Pilze, Bakterien und Viren. Die erfindungsgemäßen Substanzen lassen sich daher zum Schutz von Pflanzen gegen Angriff durch die erwähnten Pathogene innerhalb eines gewissen Zeitraums nach der Behandlung einsetzen. Der Zeitraum, über den eine Schutzwirkung erzielt wird, erstreckt sich im allgemeinen von 1 bis 10 Tagen, vorzugsweise 1 bis 7 Tagen, nach der Behandlung der Pflanzen mit den Wirkstoffen.

Zu Pflanzen und Pflanzensorten, die vorzugsweise erfindungsgemäß behandelt werden, zählen alle Pflanzen, die über Erbgut verfügen, das diesen Pflanzen besonders vorteilhafte, nützliche Merkmale verleiht (egal, ob dies durch Züchtung und/oder Biotechnologie erzielt wurde).

Pflanzen und Pflanzensorten, die ebenfalls vorzugsweise erfindungsgemäß behandelt werden, sind gegen einen oder mehrere biotische Streßfaktoren resistent, d. h. diese Pflanzen weisen eine verbesserte Abwehr gegen tierische und mikrobielle Schädlinge wie Nematoden, Insekten, Milben, phytopathogene Pilze, Bakterien, Viren und/oder Viroide auf.

Pflanzen und Pflanzensorten, die ebenfalls erfindungsgemäß behandelt werden können, sind solche Pflanzen, die gegen einen oder mehrere abiotische Streßfaktoren resistent sind. Zu den abiotischen Streßbedingungen können zum Beispiel Dürre, Kälte- und Hitzebedingungen, osmotischer Streß, Staunässe, erhöhter Bodensalzgehalt, erhöhtes Ausgesetztsein an Mineralien, Ozonbedingungen, Starklichtbedingungen, beschränkte Verfügbarkeit von Stickstoffnährstoffen, beschränkte Verfügbarkeit von Phosphornährstoffen oder Vermeidung von Schatten zählen.

Pflanzen und Pflanzensorten, die ebenfalls erfindungsgemäß behandelt werden können, sind solche Pflanzen, die durch erhöhte Ertragseigenschaften gekennzeichnet sind. Ein erhöhter Ertrag kann bei diesen Pflanzen z. B. auf verbesserter Pflanzenphysiologie, verbessertem Pflanzenwuchs und verbesserter Pflanzenentwicklung, wie Wasserverwertungseffizienz, Wasserhalteeffizienz, verbesserter Stickstoffverwertung, erhöhter Kohlenstoffassimilation, verbesserter Photosynthese, verstärkter Keimkraft und beschleunigter Abreife beruhen. Der Ertrag kann weiterhin durch eine verbesserte Pflanzenarchitektur (unter Streß- und nicht-Streß-Bedingungen) beeinflußt werden, darunter frühe Blüte, Kontrolle der Blüte für die Produktion von Hybridsaatgut, Keimpflanzenwüchsigkeit, Pflanzengröße, Internodienzahl und -abstand, Wurzelwachstum, Samengröße, Fruchtgröße, Schotengröße, Schoten- oder Ährenzahl, Anzahl der Samen pro Schote oder Ähre, Samenmasse, verstärkte Samenfüllung, verringerter Samenausfall, verringertes Schotenplatzen sowie Standfestigkeit. Zu weiteren Ertragsmerkmalen zählen Samenzusammensetzung wie Kohlenhydratgehalt, Proteingehalt, Ölgehalt und Ölzusammensetzung, Nährwert, Verringerung der nährwidrigen Verbindungen, verbesserte Verarbeitbarkeit und verbesserte Lagerfähigkeit.

Pflanzen, die erfindungsgemäß behandelt werden können, sind Hybridpflanzen, die bereits die Eigenschaften der Heterosis bzw. des Hybrideffekts exprimieren, was im allgemeinen zu höherem Ertrag, höherer Wüchsigkeit, besserer Gesundheit und besserer Resistenz gegen biotische und abiotische Streßfaktoren führt. Solche Pflanzen werden typischerweise dadurch erzeugt, daß man eine ingezüchtete pollensterile Elternlinie (den weiblichen Kreuzungspartner) mit einer anderen ingezüchteten pollenfertilen Elternlinie (dem männlichen Kreuzungspartner) kreuzt. Das Hybridsaatgut wird typischerweise von den pollensterilen Pflanzen geerntet und an Vermehrer verkauft. Pollensterile Pflanzen können manchmal (z. B. beim Mais) durch Entfahnen (d. h. mechanischem Entfernen der männlichen Geschlechtsorgane bzw. der männlichen Blüten), produziert werden; es ist jedoch üblicher, daß die Pollensterilität auf genetischen Determinanten im Pflanzengenom beruht. In diesem Fall, insbesondere dann, wenn es sich bei dem gewünschten Produkt, da man von den Hybridpflanzen ernten will, um die Samen handelt, ist es üblicherweise günstig, sicherzustellen, daß die Pollenfertilität in Hybridpflanzen, die die für die Pollensterilität verantwortlichen genetischen Determinanten enthalten, völlig restoriert wird. Dies kann erreicht werden, indem sichergestellt wird, daß die männlichen Kreuzungspartner entsprechende Fertilitätsrestorergene besitzen, die in der Lage sind, die Pollenfertilität in Hybridpflanzen, die die genetischen Determinanten, die für die Pollensterilität verantwortlich sind, enthalten, zu restorieren. Genetische Determinanten für Pollensterilität können im Cytoplasma lokalisiert sein. Beispiele für cytoplasmatische Pollensterilität (CMS) wurden zum Beispiel für Brassica-Arten beschrieben. Genetische Determinanten für Pollensterilität können jedoch auch im Zellkerngenom lokalisiert sein. Pollensterile Pflanzen können auch mit Methoden der pflanzlichen Biotechnologie, wie Gentechnik, erhalten werden. Ein besonders günstiges Mittel zur Erzeugung von pollensterilen Pflanzen ist in WO 89/10396 beschrieben, wobei zum Beispiel eine Ribonuklease wie eine Barnase selektiv in den Tapetumzellen in den Staubblättern exprimiert wird. Die Fertilität kann dann durch Expression eines Ribonukleasehemmers wie Barstar in den Tapetumzellen restoriert werden.

Pflanzen oder Pflanzensorten (die mit Methoden der Pflanzenbiotechnologie, wie der Gentechnik, erhalten werden), die erfindungsgemäß behandelt werden können, sind herbizidtolerante Pflanzen, d. h. Pflanzen, die gegenüber einem oder mehreren vorgegebenen Herbiziden tolerant gemacht worden sind. Solche Pflanzen können entweder durch genetische Transformation oder durch Selektion von Pflanzen, die eine Mutation enthalten, die solch eine Herbizidtoleranz verleiht, erhalten werden.

Herbizidtolerante Pflanzen sind zum Beispiel glyphosatetolerante Pflanzen, d. h. Pflanzen, die gegenüber dem Herbizid Glyphosate oder dessen Salzen tolerant gemacht worden sind. So können zum Beispiel glyphosatetolerante Pflanzen durch Transformation der Pflanze mit einem Gen, das für das Enzym 5-Enolpyruvylshikimat-3-phosphatsynthase (EPSPS) kodiert, erhalten werden. Beispiele für solche EPSPS-Gene sind das AroA-Gen (Mutante CT7) des Bakterium *Salmonella typhimurium,* das CP4-Gen des Bakteriums *Agrobacterium sp.,* die Gene, die für eine EPSPS aus der Petunie, für eine EPSPS aus der Tomate oder für eine EPSPS aus Eleusine kodieren. Es kann sich auch um eine mutierte EPSPS handeln. Glyphosatetolerante Pflanzen können auch dadurch erhalten werden, daß man ein Gen exprimiert, das für ein Glyphosate-Oxidoreduktase-Enzym kodiert. Glyphosatetolerante Pflanzen können auch dadurch erhalten werden, daß man ein Gen exprimiert, das für ein Glyphosateacetyltransferase-Enzym kodiert. Glyphosatetolerante Pflanzen können auch dadurch erhalten werden, daß man Pflanzen, die natürlich vorkommende Mutationen der oben erwähnten Gene selektiert.

Sonstige herbizidresistente Pflanzen sind zum Beispiel Pflanzen, die gegenüber Herbiziden, die das Enzym Glutaminsynthase hemmen, wie Bialaphos, Phosphinotricin oder Glufosinate, tolerant gemacht worden sind. Solche Pflanzen können dadurch erhalten werden, daß man ein Enzym exprimiert, das das Herbizid oder eine Mutante des Enzyms Glutaminsynthase, das gegenüber Hemmung resistent ist, entgiftet. Solch ein wirksames entgiftendes Enzym ist zum Beispiel ein Enzym, das für ein Phosphinotricin-acetyltransferase kodiert (wie zum Beispiel das bar- oder pat-Protein aus Streptomyces-Arten). Pflanzen, die eine exogene Phosphinotricin-acetyltransferase exprimieren, sind beschrieben.

Weitere herbizidtolerante Pflanzen sind auch Pflanzen, die gegenüber den Herbiziden, die das Enzym Hydroxyphenylpyruvatdioxygenase (HPPD) hemmen, tolerant gemacht worden sind. Bei den Hydroxyphenylpyruvatdioxygenasen handelt es sich um Enzyme, die die Reaktion, in der para-Hydroxyphenylpyruvat (HPP) zu Homogentisat umgesetzt wird, katalysieren. Pflanzen, die gegenüber HPPD-Hemmern tolerant sind, können mit einem Gen, das für ein natürlich vorkommendes resistentes HPPD-Enzym kodiert, oder einem Gen, das für ein mutiertes HPPD-Enzym kodiert, transformiert werden. Eine Toleranz gegenüber HPPD-Hemmern kann auch dadurch erzielt werden, daß man Pflanzen mit Genen transformiert, die für gewisse Enzyme kodieren, die die Bildung von Homogentisat trotz Hemmung des nativen HPPD-Enzyms durch den HPPD-Hemmer ermöglichen. Die Toleranz von Pflanzen gegenüber HPPD-Hemmern kann auch dadurch verbessert werden, daß man Pflanzen zusätzlich zu einem Gen, das für ein HPPD-tolerantes Enzym kodiert, mit einem Gen transformiert, das für ein Prephenatdehydrogenase-Enzym kodiert.

Weitere herbizidresistente Pflanzen sind Pflanzen, die gegenüber Acetolactatsynthase (ALS)-Hemmern tolerant gemacht worden sind. Zu bekannten ALS-Hemmern zählen zum Beispiel Sulfonylharnstoff, Imidazolinon, Triazolopyrimidine, Pyrimidinyloxy(thio)benzoate und/oder Sulfonylaminocarbonyltriazolinon-Herbizide. Es ist bekannt, daß verschiedene Mutationen im Enzym ALS (auch als Acetohydroxysäure-Synthase, AHAS, bekannt) eine Toleranz gegenüber unterschiedlichen Herbiziden bzw. Gruppen von Herbiziden verleihen. Die Herstellung von sulfonylharnstofftoleranten Pflanzen und imidazolinontoleranten Pflanzen ist in der internationalen Veröffentlichung WO 96/033270 beschrieben. Weitere sulfonylharnstoff- und imidazolinontolerante Pflanzen sind auch in z.B. WO 07/024782 beschrieben.

Weitere Pflanzen, die gegenüber Imidazolinon und/oder Sulfonylharnstoff tolerant sind, können durch induzierte Mutagenese, Selektion in Zellkulturen in Gegenwart des Herbizids oder durch Mutationszüchtung erhalten werden.

Pflanzen oder Pflanzensorten (die nach Methoden der pflanzlichen Biotechnologie, wie der Gentechnik, erhalten wurden), die ebenfalls erfindungsgemäß behandelt werden können, sind insektenresistente transgene Pflanzen, d.h. Pflanzen, die gegen Befall mit gewissen Zielinsekten resistent gemacht wurden. Solche Pflanzen können durch genetische Transformation oder durch Selektion von Pflanzen, die eine Mutation enthalten, die solch eine Insektenresistenz verleiht, erhalten werden.

Der Begriff "insektenresistente transgene Pflanze" umfaßt im vorliegenden Zusammenhang jegliche Pflanze, die mindestens ein Transgen enthält, das eine Kodiersequenz umfaßt, die für folgendes kodiert:
1) ein insektizides Kristallprotein aus *Bacillus thuringiensis* oder einen insektiziden Teil davon, wie die insektiziden Kristallproteine, die online bei: http://www.lifesci.sussex.ac.uk/Home/Neil_Crickmore/Bt/ beschrieben sind, zusammengestellt wurden, oder insektizide Teile davon, z.B. Proteine der Cry-Proteinklassen Cry1Ab, Cry1Ac, Cry1F, Cry2Ab, Cry3Ae oder Cry3Bb oder insektizide Teile davon; oder
2) ein Kristallprotein aus *Bacillus thuringiensis* oder einen Teil davon, der in Gegenwart eines zweiten, anderen Kristallproteins als *Bacillus thuringiensis* oder eines Teils davon insektizid wirkt, wie das binäre Toxin, das aus den Kristallproteinen Cy34 und Cy35 besteht; oder
3) ein insektizides Hybridprotein, das Teile von zwei unterschiedlichen insektiziden Kristallproteinen aus *Bacillus thuringiensis* umfaßt, wie zum Beispiel ein Hybrid aus den Proteinen von 1) oben oder ein Hybrid aus den Proteinen von 2) oben, z. B. das Protein Cry1A.105, das von dem Mais-Event MON98034 produziert wird (WO 07/027777); oder
4) ein Protein gemäß einem der Punkte 1) bis 3) oben, in dem einige, insbesondere 1 bis 10, Aminosäuren durch eine andere Aminosäure ersetzt wurden, um eine höhere insektizide Wirksamkeit gegenüber einer Zielinsektenart zu erzielen und/oder um das Spektrum der entsprechenden Zielinsektenarten zu erweitern und/oder wegen Veränderungen, die in die Kodier- DNA während der Klonierung oder Transformation induziert wurden, wie das Protein Cry3Bb1 in Mais-Events MON863 oder MON88017 oder das Protein Cry3A im Mais-Event MIR 604;
5) ein insektizides sezerniertes Protein aus *Bacillus thuringiensis* oder *Bacillus cereus* oder einen insektiziden Teil davon, wie die vegetativ wirkenden insektentoxischen Proteine (vegetative insekticidal proteins, VIP), die unter http://www.lifesci.sussex.ac.uk/Home/Neil_Crickmore/Bt/vip.html angeführt sind, z. B. Proteine der Proteinklasse VIP3Aa; oder
6) ein sezerniertes Protein aus *Bacillus thuringiensis* oder *Bacillus cereus,* das in Gegenwart eines zweiten sezernierten Proteins aus *Bacillus thuringiensis* oder *B. cereus* insektizid wirkt, wie das binäre Toxin, das aus den Proteinen VIP1A und VIP2A besteht.
7) ein insektizides Hybridprotein, das Teile von verschiedenen sezernierten Proteinen von *Bacillus thuringiensis* oder *Bacillus cereus* umfaßt, wie ein Hybrid der Proteine von 1) oder ein Hybrid der Proteine von 2) oben; oder
8) ein Protein gemäß einem der Punkte 1) bis 3) oben, in dem einige, insbesondere 1 bis 10, Aminosäuren durch eine andere Aminosäure ersetzt wurden, um eine höhere insektizide Wirksamkeit gegenüber einer Zielinsektenart zu erzielen und/oder um das Spektrum der entsprechenden Zielinsektenarten zu erweitern und/oder wegen Veränderungen, die in die Kodier- DNA während der Klonierung oder Transformation induziert wurden (wobei die Kodierung für ein insektizides Protein erhalten bleibt), wie das Protein VIP3Aa im Baumwoll-Event COT 102.

Natürlich zählt zu den insektenresistenten transgenen Pflanzen im vorliegenden Zusammenhang auch jegliche Pflanze, die eine Kombination von Genen umfaßt, die für die Proteine von einer der oben genannten Klassen 1 bis 8 kodieren. In einer Ausführungsform enthält eine insektenresistente Pflanze mehr als ein Transgen, das für ein Protein nach einer der oben genannten 1 bis 8 kodiert, um das Spektrum der entsprechenden Zielinsektenarten zu erweitern oder um die Entwicklung einer Resistenz der Insekten gegen die Pflanzen dadurch hinauszuzögern, daß man verschiedene Proteine einsetzt, die für dieselbe Zielinsektenart insektizid sind, jedoch eine unterschiedliche Wirkungsweise, wie Bindung an unterschiedliche Rezeptorbindungsstellen im Insekt, aufweisen.

Pflanzen oder Pflanzensorten (die nach Methoden der pflanzlichen Biotechnologie, wie der Gentechnik, erhalten wurden), die ebenfalls erfindungsgemäß behandelt werden können, sind gegenüber abiotischen Streßfaktoren tolerant. Solche Pflanzen können durch genetische Transformation oder durch Selektion von Pflanzen, die eine Mutation enthalten, die solch eine Streßresistenz verleiht, erhalten werden. Zu besonders nützlichen Pflanzen mit Streßtoleranz zählen folgende:
a. Pflanzen, die ein Transgen enthalten, das die Expression und/oder Aktivität des Gens für die Poly(ADP-ribose)polymerase (PARP) in den Pflanzenzellen oder Pflanzen zu reduzieren vermag.
b. Pflanzen, die ein streßtoleranzförderndes Transgen enthalten, das die Expression und/oder Aktivität der für PARG kodierenden Gene der Pflanzen oder Pflanzenzellen zu reduzieren vermag;
c. Pflanzen, die ein streßtoleranzförderndes Transgen enthalten, das für ein in Pflanzen funktionelles Enzym des Nicotinamidadenindinukleotid-Salvage-Biosynthesewegs kodiert, darunter Nicotinamidase, Nicotinatphosphoribosyltransferase, Nicotinsäuremononukleotidadenyltransferase, Nicotinamidadenindinukleotidsynthetase oder Nicotinamidphosphoribosyltransferase.

Pflanzen oder Pflanzensorten (die nach Methoden der pflanzlichen Biotechnologie, wie der Gentechnik, erhalten wurden), die ebenfalls erfindungsgemäß behandelt werden können, weisen eine veränderte Menge, Qualität und/oder Lagerfähigkeit des Ernteprodukts und/oder veränderte Eigenschaften von bestimmten Bestandteilen des Ernteprodukts auf, wie zum Beispiel:
1) Transgene Pflanzen, die eine modifizierte Stärke synthetisieren, die bezüglich ihrer chemisch-physikalischen Eigenschaften, insbesondere des Amylosegehalts oder des Amylose/Amylopektin-Verhältnisses, des Verzweigungsgrads, der durchschnittlichen Kettenlänge, der Verteilung der Seitenketten, des Viskositätsverhaltens, der Gelfestigkeit, der Stärkekorngröße und/oder Stärkekornmorphologie im Vergleich mit der synthetisierten Stärke in Wildtyppflanzenzellen oder -pflanzen verändert ist, so daß sich diese modifizierte Stärke besser für bestimmte Anwendungen eignet.
2) Transgene Pflanzen, die Nichtstärkekohlenhydratpolymere synthetisieren, oder Nichtstärkekohlenhydratpolymere, deren Eigenschaften im Vergleich zu Wildtyppflanzen ohne genetische Modifikation verändert sind. Beispiele sind Pflanzen, die Polyfructose, insbesondere des Inulin- und Levantyps, produzieren, Pflanzen, die alpha-1,4-Glucane produzieren, Pflanzen, die alpha-1,6-verzweigte alpha-1,4-Glucane produzieren und Pflanzen, die Alternan produzieren.
3) Transgene Pflanzen, die Hyaluronan produzieren.

Pflanzen oder Pflanzensorten (die nach Methoden der pflanzlichen Biotechnologie, wie der Gentechnik, erhalten wurden), die ebenfalls erfindungsgemäß behandelt werden können, sind Pflanzen wie Baumwollpflanzen mit veränderten Fasereigenschaften. Solche Pflanzen können durch genetische Transformation oder durch Selektion von Pflanzen, die eine Mutation enthalten, die solche veränderten Fasereigenschaften verleiht, erhalten werden; dazu zählen:
a) Pflanzen wie Baumwollpflanzen, die eine veränderte Form von Cellulosesynthasegenen enthalten,
b) Pflanzen wie Baumwollpflanzen, die eine veränderte Form von rsw2- oder rsw3-homologen Nukleinsäuren enthalten;
c) Pflanzen wie Baumwollpflanzen mit einer erhöhten Expression der Saccharosephosphatsynthase;
d) Pflanzen wie Baumwollpflanzen mit einer erhöhten Expression der Saccharosesynthase;
e) Pflanzen wie Baumwollpflanzen bei denen der Zeitpunkt der Durchlaßsteuerung der Plasmodesmen an der Basis der Faserzelle verändert ist, z. B. durch Herunterregulieren der faserselektiven β-1,3-Glucanase;
f) Pflanzen wie Baumwollpflanzen mit Fasern mit veränderter Reaktivität, z. B. durch Expression des N-Acetylglucosamintransferasegens, darunter auch nodC, und von Chitinsynthasegenen.

Pflanzen oder Pflanzensorten (die nach Methoden der pflanzlichen Biotechnologie, wie der Gentechnik, erhalten wurden), die ebenfalls erfindungsgemäß behandelt werden können, sind Pflanzen wie Raps oder verwandte Brassica-Pflanzen mit veränderten Eigenschaften der Ölzusammensetzung. Solche Pflanzen können durch genetische Transformation oder durch Selektion von Pflanzen, die eine Mutation enthalten, die solche veränderten Öleigenschaften verleiht, erhalten werden; dazu zählen:
a) Pflanzen wie Rapspflanzen, die Öl mit einem hohen Ölsäuregehalt produziere;
b) Pflanzen wie Rapspflanzen, die Öl mit einem niedrigen Linolensäuregehalt produzieren.
c) Pflanzen wie Rapspflanzen, die Öl mit einem niedrigen gesättigten Fettsäuregehalt produzieren.

Besonders nützliche transgene Pflanzen, die erfindungsgemäß behandelt werden können, sind Pflanzen mit einem oder mehreren Genen, die für ein oder mehrere Toxine kodieren, und sind die transgenen Pflanzen, die unter den folgenden Handelsbezeichnungen angeboten werden: YIELD GARD® (zum Beispiel Mais, Baumwolle, Sojabohnen), KnockOut® (zum Beispiel Mais), BiteGard® (zum Beispiel Mais), BT-Xtra® (zum Beispiel Mais), StarLink® (zum Beispiel Mais), Bollgard® (Baumwolle), Nucotn® (Baumwolle), Nucotn 33B® (Baumwolle), NatureGard® (zum Beispiel Mais), Protecta® und NewLeaf® (Kartoffel). Herbizidtolerante Pflanzen, die zu erwähnen sind, sind zum Beispiel Maissorten, Baumwollsorten und Sojabohnensorten, die unter den folgenden Handelsbezeichnungen angeboten werden: Roundup Ready® (Glyphosatetoleranz, zum Beispiel Mais, Baumwolle, Sojabohne), Liberty Link® (Phosphinotricintoleranz, zum Beispiel Raps), IMI® (Imidazolinontoleranz) und SCS® (Sulfonylharnstofftoleranz), zum Beispiel Mais. Zu den herbizidresistenten Pflanzen (traditionell auf Herbizidtoleranz gezüchtete Pflanzen), die zu erwähnen sind, zählen die unter der Bezeichnung Clearfield® angebotenen Sorten (zum Beispiel Mais).

Besonders nützliche transgene Pflanzen, die erfindungsgemäß behandelt werden können, sind Pflanzen, die Transformations-Events, oder eine Kombination von Transformations-Events, enthalten und die zum Beispiel in den Dateien von verschiedenen nationalen oder regionalen Behörden angeführt sind (siehe zum Beispiel http://gmoinfo.jrc.it/gmp_browse.aspx und http://www.agbios.com/dbase.php).

Die aufgeführten Pflanzen können besonders vorteilhaft erfindungsgemäß mit den Verbindungen der allgemeinen Formel (I) behandelt werden. Die bei den Wirkstoffen bzw. Mischungen oben angegebenen Vorzugsbereiche gelten auch für die Behandlung dieser Pflanzen. Besonders hervorgehoben sei die Pflanzenbehandlung mit den im vorliegenden Text speziell aufgeführten Verbindungen bzw. Mischungen.

Die erfindungsgemäßen Wirkstoffe bzw. Mittel können also eingesetzt werden, um Pflanzen innerhalb eines gewissen Zeitraumes nach der Behandlung gegen den Befall durch die genannten Schaderreger zu schützen. Der Zeitraum, innerhalb dessen Schutz herbeigeführt wird, erstreckt sich im Allgemeinen auf 1 bis 28 Tage, bevorzugt auf 1 bis 14 Tage, besonders bevorzugt auf 1 bis 10 Tage, ganz besonders bevorzugt auf 1 bis 7 Tage nach der Behandlung der Pflanzen mit den Wirkstoffen bzw. auf bis zu 200 Tage nach einer Saatgutbehandlung.

Die Herstellung und die Verwendung der erfindungsgemäßen Wirkstoffe der Formeln [I] und [I-c] geht aus den folgenden Beispielen hervor. Die Erfindung ist jedoch nicht auf diese Beispiele beschränkt.

### Herstellung von Ausgangsstoffen der Formel [VII]:

### 4-Brom-3-(4-fluorphenyl)-1H-pyrazol [VII-1]

14.9 g (92 mmol) 3-(4-Fluorphenyl)-1H-pyrazol (Synthese beschrieben in EP-A-1 382 603) werden in 45 mL Essigsäure gelöst. Dazu wird bei 3-5 °C eine Lösung von 5.7 mL Brom (110 mmol) in 9 mL Essigsäure zugegeben. Es bildet sich ein Niederschlag zu dem weitere 130 mL Essigsäure gegeben werden. Danach wird die Reaktionsmischung noch 4 h bei Raumtemperatur nachgerührt. Anschließend werden alle flüchtigen Komponenten im Hochvakuum entfernt. Der Rückstand wird in 1 molarer Natriumcarbonatlösung gelöst und mehrmals mit Essigsäureethylester extrahiert. Anschließend wird die organische Phase getrocknet (Na₂SO₄) und eingeengt. Man erhält 21.8 g 4-Brom-3-(4-fluorphenyl)-1H-pyrazol (Ausbeute 98%) als farblosen Feststoff. Das Produkt wird ohne weitere Reinigung weiter umgesetzt.
logP (pH2.7): 2.29
MS (ESI): 241.0 ([M+H]⁺)
1H-NMR (400 MHz, d₆-DMSO): δ *=* 13.3 (s, 1H, br), 7.82 (m, 3H), 7.30 (m, 2H) ppm

### Herstellung von Ausgangsstoffen der Formel [VI]:

### Mischung von 4-Brom-3-(4-fluorphenyl)-1-isopropyl-1H-pyrazol und 4-Brom-5-(4-fluorphenyl)-1-isopropyl-1H-pyrazol [VI-1]

3.6 g (14.9 mmol) 4-Brom-3-(4-fluorphenyl)-1H-pyrazol werden in 6 mL N-N-Dimethylformamid gelöst. Dazu werden 0.43 g Natriumhydrid (17.9 mmol) als 60 %ige Suspension in Öl gegeben und die Mischung wird 20 min bei 25 °C gerührt. Anschließend werden 3.8 g Isopropyliodid (22.4mmol) zugegeben und die Reaktionsmischung über Nacht bei 25 °C gerührt. Zur Aufarbeitung wird Essigsäure (konzentriert) langsam zugegeben (0.2 eq) und anschließend alle flüchtigen Komponenten im Hochvakuum entfernt. Der Rückstand wird in Wasser gelöst und mehrmals mit Essigsäureethylester extrahiert. Anschließend wird die organische Phase getrocknet (Na₂SO₄) und eingeengt. Die Reinigung erfolgt mittels Kieselgelchromatographie mit dem Eluent Cyclohexan (A) / Essigsäureethylester (B) (0% B auf 40% B). Man erhält 3.54 g eines (84:16) Gemisches aus 4-Brom-3-(4-fluorphenyl)-1-isopropyl-1H-pyrazol und 4-Brom-5-(4-fluorphenyl)-1-isopropyl-1H-pyrazol (minor) als farblosen Feststoff.. Das Produkt wird ohne weitere Reinigung weiter umgesetzt.
logP (pH2.7): 3.96 und 3.68^{minor}
MS (ESI): 285.0 ([M+H]⁺) 1H-NMR (400 MHz, d6-DMSO): δ = 8.04 (s, 1H), 7.85 (dd, 2H), 7.64 (s, 1H^{minor}, 7.43 (dd, 2H^{minor}), 7.36 (t, 2H^{minor}), 7.25 (t, 2H), 4.51 (m, 1H), 4.36 (m, 1H^{minor}), 1.45 (d, 6H), 1.33 (d, 6H^{minor}) ppm

### 4-Brom-1-ethyl-3-(4-fluorphenyl)-1H-pyrazol [VI-2]

### 4-Brom-1-ethyl-5-(4-fluorphenyl)-1H-pyrazol [VI-3]

3.0 g (12.3 mmol) 4-Brom-3-(4-fluorphenyl)-1H-pyrazol werden in 6 mL N-N-Dimethylformamid gelöst. Dazu werden 0.59 g Natriumhydrid (14.7 mmol) als 60%ige Suspension in Öl gegeben und die Mischung wird 20 min bei 25 °C gerührt. Anschließend werden 2.9 g Iodethan (18.4 mmol) zugegeben und die Reaktionsmischung über Nacht bei 25 °C gerührt. Zur Aufarbeitung wird Essigsäure (konzentriert) langsam zugegeben (0.2 eq) und anschließend alle flüchtigen Komponenten im Hochvakuum entfernt. Der Rückstand wird in Wasser gelöst und mehrmals mit Essigsäureethylester extrahiert. Anschließend wird die organische Phase getrocknet (Na₂SO₄) und eingeengt. Die Reinigung erfolgt mittels Kieselgelchromatographie mit dem Eluent Cyclohexan (A) / Essigsäureethylester (B) (0% B auf 40% B). Man erhält 2.52 g eines (75:25) Gemisches der Pyrazol-Isomeren als farblosen Feststoff. Die Mischung wird durch preparative HPLC (Kromasil 100 C18 16µm 250*100 mm, 60/40 Methanol/H₂O isokratisch, Fluß 800 ml/min) getrennt und es werden 1.58 g (48% Ausbeute) 4-Brom-1-ethyl-3-(4-fluorphenyl)-1H-pyrazol und 0.41 g (12% Ausbeute) 4-Brom-1-ethyl-5-(4-fluorphenyl)-1H-pyrazol erhalten.

### Hauptisomer: 4-Brom-1-ethyl-3-(4-fluorphenyl)-1H-pyrazol [VI-2]

logP (pH2.7): 3.37
MS (ESI): 269.0 ([M+H]⁺)
¹H-NMR (400 MHz, d₆-DMSO): δ = 8.02 (s, 1H), 7.85 (dd, 2H), 7.25 (t, 2H), 4.16 (q, 2H), 1.41 (t, 3H) ppm

### Minderisomer: 4-Brom-1-ethyl-5-(4-fluorphenyl)-1H-pyrazol [VI-3]

logP (pH2.7): 3.15
MS (ESI): 269.0 ([M+H]⁺)
¹H-NMR (400 MHz, d₆-DMSO): δ = 7.62 (s, 1H), 7.48 (dd, 2H), 7.36 (t, 2H), 4.02 (q, 2H), 1.24 (t, 3H) ppm

### 4-Brom-3-(4-fluorphenyl)-1-isobutyl-1H-pyrazol [VI-4]

### 4-Brom-5-(4-fluorphenyl)-1-isobutyl-1H-pyrazol [VI-5]

3.62 g (14.9 mmol) 4-Brom-3-(4-fluorphenyl)-1H-pyrazol werden in 6 mL N-N-Dimethylformamid gelöst. Dazu werden 0.43 g Natriumhydrid (17.9 mmol) als 60%ige Suspension in Öl gegeben und die Mischung wird 20 min bei 25 °C gerührt. Anschließend werden 4.1 g 1-Iod-2-methylpropan (22.4 mmol) zugegeben und die Reaktionsmischung über Nacht bei 25 °C gerührt. Zur Aufarbeitung wird Essigsäure (konzentriert) langsam zugegeben (0.2 eq) und anschließend alle flüchtigen Komponenten im Hochvakuum entfernt. Der Rückstand wird in Wasser gelöst und mehrmals mit Essigsäureethylester extrahiert. Anschließend wird die organische Phase getrocknet (Na₂SO₄) und eingeengt. Die Reinigung erfolgt mittels Kieselgelchromatographie mit dem Eluent Cyclohexan (A) / Essigsäureethylester (B) (0% B auf 40% B). Man erhält 3.74 g eines (71:29) Gemisches der Pyrazol-Isomeren als farblosen Feststoff. Die Mischung wird durch preparative HPLC (Kromasil 100 C18 16 µm 250*100 mm, 70/30 Methanol/H₂O isokratisch, Fluß 800ml/min) getrennt und es werden 2.51 g (56%) 4-Brom-3-(4-fluorphenyl)-1-isobutyl-1H-pyrazol und 0.59 g (13% Ausbeute) 4-Brom-5-(4-fluorphenyl)-1-isobutyl-1H-pyrazol erhalten.

### Hauptisomer: 4-Brom-3-(4-fluorphenyl)-1-isobutyl-1H-pyrazol [VI-4]

logP (pH2.7): 4.34
MS (ESI): 299.0 ([M+H]⁺)
¹H-NMR (400 MHz, d₆-DMSO): δ = 7.99 (s, 1H), 7.85 (dd, 2H), 7.25 (t, 2H), 3.94 (d, 2H), 2.18 (m, 1H), 0.88 (d, 6H) ppm

### Minderisomer: 4-Brom-5-(4-fluorphenyl)-1-isobutyl-1H-pyrazol [VI-5]

logP (pH2.7): 4.04
MS (ESI): 299.0 ([M+H]⁺)
¹H-NMR (400 MHz, d₆-DMSO): δ = 7.64 (s, 1H), 7.48 (dd, 2H), 7.36 (t, 2H), 3.84 (d, 2H), 1.98 (m, 1 H), 0.69 (d, 6H) ppm

### 4-Brom-3-(4-fluorphenyl)-1-(2-methoxyethyl)-1H-pyrazol [VI-6]

### 4-Brom-5-(4-fluorphenyl)-1-(2-methoxyethyl)-1H-pyrazol [VI-7]

3.62 g (14.9 mmol) 4-Brom-3-(4-fluorphenyl)-1H-pyrazol werden in 6 mL N-N-Dimethylformamid gelöst. Dazu werden 0.43 g Natriumhydrid (17.9 mmol) als 60%ige Suspension in Öl gegeben und die Mischung wird 20min bei 25 °C gerührt. Anschließend werden 3.1 g 1-Brom-2-methoxyethan (22.4 mmol) zugegeben und die Reaktionsmischung über Nacht bei 25 °C gerührt. Zur Aufarbeitung wird Essigsäure (konzentriert) langsam zugegeben (0.2 eq) und anschließend alle flüchtigen Komponenten im Hochvakuum entfernt. Der Rückstand wird in Wasser gelöst und mehrmals mit Essigsäureethylester extrahiert. Anschließend wird die organische Phase getrocknet (Na₂SO₄) und eingeengt. Die Reinigung erfolgt mittels Kieselgelchromatographie mit dem Eluent Cyclohexan (A) / Essigsäureethylester (B) (0% B auf 40% B). Man erhält 2.91 g eines (76:23) Gemisches der Pyrazol-Isomeren als farblosen Feststoff. Die Mischung wird durch preparative HPLC (Kromasil 100 C18 16 µm 250*100 mm, 62/38 Methanol/H₂O isokratisch, Fluß 800ml/min) getrennt und es werden 2.92 g (61% Ausbeute) 4-Brom-3-(4-fluorphenyl)-1-(2-methoxyethyl)-1H-pyrazol und 0.43 g (9% Ausbeute) 4-Brom-5-(4-fluorphenyl)-1-(2-methoxyethyl)-1H-pyrazol erhalten.

### Hauptisomer: 4-Brom-3-(4-fluorphenyl)-1-(2-methoxyethyl)-1H-pyrazol [VI-6]

logP (pH2.7): 3.08
MS (ESI): 299.0 ([M+H]⁺)¹H-NMR (400 MHz, d₆-DMSO): δ = 7.98 (s, 1H), 7.85 (dd, 2H), 7.23 (t, 2H), 4.29 (t, 2H), 3.73 (t, 2H), 3.26 (s, 3H) ppm

### Minderisomer: 4-Brom-5-(4-fluorphenyl)-1-(2-methoxyethyl)-1H-pyrazol [VI-7]

logP (pH2.7): 2.90
MS (ESI): 299.0 ([M+H]⁺)
¹H-NMR (400 MHz, d₆-DMSO): δ = 7.65 (s, 1H), 7.48 (dd, 2H), 7.36 (t, 2H), 4.13 (t, 2H), 3.63 (t, 2H), 3.10 (s, 3H) ppm
Nach demselben Verfahren lassen sich herstellen

### 4-Brom-3-(4-fluorphenyl)-1-methyl-1H-pyrazol [VI-8]

logP (pH2.7): 2.86
MS (ESI): 257.0 ([M+H]⁺)
¹H-NMR (400 MHz, CD₃CN): δ = 7.88-7,84 (m, 2H), 7.65 (s, 1H), 7.21-7.16 (m, 2H), 3.87(s, 3H) ppm

### 4-Brom-3-(4-fluorphenyl)-1-[1-(2-fluorphenyl)ethyl]-1H-pyrazol [VI-9]

logP (pH2.7): 3.63
MS (ESI): 477.0 ([M+H]⁺)
¹H-NMR (400 MHz, CD3CN): δ = 8.15 (s, 1H), 7.88-7.78 (m, 2H), 7.38-7.28 (m, 2H), 4.37 (dd, 1H), 4,31 (dd, 1H), 2.33 (m, 1H), 1.87 (dd, 1H), 1.65 (t, 1H) ppm

### 4-Brom-1-[(2,2-dichlorcyclopropyl)methyl]-3-(4-fluorphenyl)-1H-pyrazol [VI-10]

logP (pH2.7): 4.43
MS (ESI): 364.9 ([M+H]⁺)
¹H-NMR (400 MHz, CD₃CN): δ = 7.88-7,83 (m, 2H), 7.82 (s, 1H), 7.31-7.29 (m, 2H), 7.27-7.10 (m, 4H), 5.86 (q, 1H), 1.88 (d, 3H) ppm

### 5-(4-Brom-1-isobutyl-1H-pyrazol-3-yl)-2-fluorbenzonitril [VI-11]

logP (pH2.7): 4.15
MS (ESI): 324.1 ([M+H]⁺)
¹H-NMR (400 MHz, d6-DMSO): δ = 8.23-8.22 (m, 1H), 8.20-8.17 (m, 1H), 8.16 (s, 1H), 7.65 (t, 1H), 3.97 (d, 2H), 2.15 (q, 1H) ppm

### 3-{[4-Brom-3-(4-fluorphenyl)-1H-pyrazol-1-yl]methyl}benzonitril [VI-12]

logP (pH2.7): 3.93
MS (ESI): 358.0 ([M+H]⁺)
¹H-NMR (400 MHz, CD₃CN): δ = 7.88-7,81 (m, 2H), 7.78 (s, 1H), 7.70-7.65 (m, 2H), 7.60-7.51 (m, 2 H), 7.22-7.16 (m, 2H), 5.35 (s, 2H) ppm

### 4-Brom-1-(2-fluorbenzyl)-3-(4-fluorphenyl)-1H-pyrazol [VI-13]

logP (pH2.7): 4.39
MS (ESI): 349.0 ([M+H]⁺)
¹H-NMR (400 MHz, d6-DMSO): δ = 8.18 (s, 1H), 7.83-7.43 (m, 2H), 7.42-7.33 (m, 1H), 7.31-7.19 (m, 5H), 5.43 (s, 2H) ppm

### 4-Brom-3-(4-fluorphenyl)-1-propyl-1H-pyrazol [VI-14]

logP (pH2.7): 3.89
MS (ESI): 283.0 ([M+H]⁺)
¹H-NMR (400 MHz, CD₃CN): δ = 7.89-7,84 (m, 2H), 7.69 (s, 1H), 7.21-7.15 (m, 2H), 4.08 (t, 2 H), 1.90-1.81 (m, 2H), 0.89 (t, 3 H) ppm

### 4-Brom-3-(4-fluorphenyl)-1-[2-(methylsulfanyl)ethyl]-1H-pyrazol [VI-15]

logP (pH2.7): 3.63
MS (ESI): 315.0 ([M+H]⁺)
¹H-NMR (400 MHz, CD₃CN): δ = 7.89-7,84 (m, 2H), 7.75 (s, 1H), 7.22-7.16 (m, 2H), 4.31 (t, 2 H), 2.95 (t, 2H), 2.04 (s, 3H) ppm

### Methyl-2-[4-brom-3-(4-fluorphenyl)-1H-pyrazol-1-yl]-3-methylbutanoat [VI-16]

logP (pH2.7): 4.27
MS (ESI): 357.0 ([M+H]⁺)
¹H-NMR (400 MHz, CD₃CN): δ = 7.88 (s, 1H), 7.87-7.84 (m, 2H), 7.22-7.17 (m, 2H), 4.70 (d, 1 H), 3.73 (s, 1H), 2.53 (m, 1H), 1.01 (d, 3H), 0.86 (d, 3.03) ppm

### 4-Brom-1-(1,3-dioxolan-2-ylmethyl)-3-(4-fluorphenyl)-1H-pyrazol [VI-17]

logP (pH2.7): 3.01
MS (ESI): 329.0 ([M+H]⁺)
¹H-NMR (400 MHz, CD₃CN): δ = 7.89-7.84 (m, 2H), 7.73 (s, 1H), 7.22-7.16 (m, 2H), 5.20 (t, 1H), 4.26 (d, 2H), 3.87 (m, 4H) ppm

### 4-Brom-1-(cyclopropylmethyl)-3-(4-fluorphenyl)-1H-pyrazol [VI-18]

logP (pH2.7): 3.90
MS (ESI): 297.0 ([M+H]⁺)
¹H-NMR (400 MHz, CD₃CN): δ = 7.90-7.85 (m, 2H), 7.78 (s, 1H), 7.28-7.16 (m, 2H), 3.98 (d, 2H), 1.27 (m,1H), 0.62 (m, 2H), 0.40 (m, 2H) ppm

### 4-Brom-1-sec-butyl-3-(4-fluorphenyl)-1H-pyrazol [VI-19]

logP (pH2.7): 4.39
MS (ESI): 299.0 ([M+H]⁺)
¹H-NMR (400 MHz, CD₃CN): δ = 7.90-7,85 (m, 2H), 7.71 (s, 1H), 7.21-7.15 (m, 2H), 4.2 (m, 1 H), 1.94-1.86 (m, 1H), 1.84-1.74 (m, 1H), 1.45 (d, 3H), 0.70 (t, 3H) ppm

### 4-Brom-1-(2-ethoxyethyl)-3-(4-fluorphenyl)-1H-pyrazol [VI-20]

logP (pH2.7): 3.51
MS (ESI): 313.0 ([M+H]⁺)
¹H-NMR (400 MHz, d6-DMSO): δ = 8.04 (s, 1H), 7.87-7.82 (m, 2H), 7.32-7.26 (m,2H), 4.28 (t, 2H), 3.78 (t, 2H), 3.44 (q, 2H), 1.08 (t, 3H) ppm

### 3-[4-Brom-3-(4-fluorphenyl)-1H-pyrazol-1-yl]butanonitril [VI-21]

logP (pH2.7): 3.08
MS (ESI): 310.1 ([M+H]⁺)
¹H-NMR (400 MHz, CD₃CN): δ = 7.91-7.88 (m, 2H), 7.83 (s, 1H), 7.23-7.19 (m, 2H), 4.72-4.69 (m, 1H), 3.03-2.95 (m, 2H), 1.60-1.59 (d, 3H) ppm

### tert-Butyl-4-[4-brom-3-(4-fluorphenyl)-1H-pyrazol-1-yl]piperidin-1-carboxylat [VI-27]

logP (pH2.7): 4.77
MS (ESI): 368.0 ([M-C₄H₉]⁺)
¹H-NMR (400 MHz, CD₃CN): δ = 7.88-7.86 (m, 2H), 7.75 (s, 1H), 7.20-7.17 (m, 2H), 4.35-4.30 (m, 1H), 4.20-4.10 (m, 2H), 3.00-2.85 (m, 2H, br), 2.08-2.05 (m, 2H), 1.88-1.83 (m, 2H), 1.44 (s, 9H) ppm

### Weitere Methoden zur Herstellung von Ausgangsstoffen der Formel [VI]:

### 4-Brom-1-(1-cyclopropylethyl)-3-(4-fluorphenyl)-1H-pyrazol [VI-22]

21.7g (0.082mol, 3eq) Triphenylphosphin werden in 70mL Tetrahydrofuran gelöst und mittels Eiskühlung auf 0°C gekühlt. Unter Argon werden langsam 25mL einer Lösung von 23.8g (2eq, 0.055mol) Diethyl azodicarboxylat (DEAD) in Toluol zugegeben, wobei die Innentemperatur 20°C nicht übersteigt. Nach 10min Rühren werden 6.9g (1eq, 0.027 mol) 4-Brom-3-(4-fluorphenyl)-1H-pyrazol und 4.9g (2eq, 0.055mol) Cyclopropylmethyl carbinol, gelöst in 20mL Tetrahydrofuran, langsam bei 0°C zugegeben. Die Reaktionsmischung wird über Nacht bei RT gerührt, anschliessend eingedampft und über Säulenchromatografie gereinigt. Es werden 1.92g (22.7%) 4-Brom-1-(1-cyclopropylethyl)-3-(4-fluorphenyl)-1H-pyrazol erhalten.
logP (pH2.7): 4.43
MS (ESI): 309.0 ([M+H]⁺)
¹H-NMR (400 MHz, CD₃CN): δ = 7.91-7,85 (m, 2H), 7.80 (s, 1H), 7.22-7.16 (m, 2H), 3.64 (m, 1 H), 1.57 (d, 3H), 1.25 (m, 1H) 0.67 (m,1H), 0.50 (m, 1H), 0.44 (m, 2H) ppm

### 3-[4-Brom-3-(4-fluorphenyl)-1H-pyrazol-1-yl]propanonitril [VI-23]

Zu einer Lösung von 2.5g 4-Brom-5-(4-fluorphenyl)-1H-pyrazol (10.4mmol) in 25mL DMF werden 5.07g Cs₂CO₃ (15.6 mmol) und 3-Brompropanonitril (2.08 g, 15.6 mmol) zugegeben und die Reaktionsmischung wird über Nacht bei 70°C gerührt. Danach wird die Mischung auf Raumtemperatur abgekühlt, in Wasser gegeben und mit Essigester extrahiert. Die organische Phase wird getrocknet, eingedampft und über preparative HPLC gereinigt. Es werden 2.60 g (85%) 3-[4-Brom-3 -(4-fluorphenyl)-1H-pyrazol-1-yl]propanonitril erhalten.
logP (pH2.7): 2.69
MS (ESI): 296.0 ([M+H]⁺)
¹H-NMR (400 MHz, CD₃CN): δ = 7.91-7,86 (m, 2H), 7.79 (s, 1H), 7.23-7.17 (m, 2H), 4.38 (t, 2H), 3.73 (t, 2H) ppm

### 4-Brom-3-(4-fluorphenyl)-1-isopropyl-5-(trifluormethyl)-1H-pyrazol [VI-24]

Zu einer Lösung von 1.4g (4.5mmol) 4-Brom-3-(4-fluorphenyl)-5-(trifluormethyl)-1H-pyrazol in 5mL DMF werden 0.63g (4.5 mmol) K₂CO₃ und 0.66g (5.4 mmol) 3-Brompropanonitril zugegeben und die Reaktionsmischung wird über Nacht bei 80°C gerührt. Danach wird die Mischung auf Raumtemperatur abgekühlt, in Wasser gegeben und mit Diethylether extrahiert. Die organische Phase wird getrocknet, eingedampft und über Chromatografie an Kieselgel (Eluent Petrolether) gereinigt. Es werden 0.5 g (32%) 4-Brom-3-(4-fluorphenyl)-1-isopropyl-5-(trifluormethyl)-1H-pyrazol erhalten.
logP (pH2.7): 5.51
MS (ESI): 353.1 ([M+H]⁺)
¹H-NMR (400 MHz, CD₃CN): δ = 7.84-7,82 (m, 2H), 7.25-7.22 (m, 2H), 4.78 (q, 1H), 1.51 (d, 6H) ppm

### 4-Brom-3-(4-fluorphenyl)-1-isopropoxy-1H-pyrazol [VI-25]

Zu einer Suspension von 1.26g (52.7mmol) Natriumhydrid in DMF werden 7.5g (29.3 mmol) 4-Brom-3-(4-fluorphenyl)-1H-pyrazol-1-ol gelöst in 30mL DMF bei 0°C zugegeben. Nach der Zugabe wird die Reaktionsmischung 20min bei Raumtemperatur gerührt. Dann wird die Mischung auf 0°C gekühlt und 4.1mL (43.9mmol) 2-Brompropan werden zugegeben. Danach wird die Reaktionsmischung 15h bei Raumtemperatur gerührt, anschliessend in 500mL Eiswasser gegeben und 3x mit 150mL Essigester extrahiert. Die vereinigten organischen Phasen werden mit Wasser gewaschen, über Na₂SO₄ getrocknet und im Vacuum eingeengt. Das Rohmaterial wird über Säulenchromatografie an Kieselgel (Eluent 2% Essigester / Petrolether) und anschliessend über päparative HPLC gereinigt. Es werden 1.2g 4-Brom-3-(4-fluorphenyl)-1-isopropoxy-1H-pyrazol (13.7%) erhalten.
logP (pH2.7): 4.11
MS (ESI): 301.1 ([M+H]⁺)
¹H-NMR (400 MHz, d6-DMSO): δ = 8.23 (s, 1H), 7.84-7.80 (m, 2H), 7.32-7.28 (m, 2H), 4.68 (q, 1H), 1.27 (d, 6H) ppm

### 4-Brom-1-cyclopropyl-3-(4-fluorphenyl)-1H-pyrazol [VI-26]

Zu einer Suspension von 4.8g (27.2mmol) N-Bromsuccinimid in 250mL Dichlormethan werden 5g (20.2 mmol) 1-Cyclopropyl-3-(4-fluorphenyl)-1H-pyrazol 10°C zugegeben. Nach der Zugabe wird die Reaktionsmischung 1h bei Raumtemperatur gerührt. Danach wird die Reaktionsmischung mit Wasser versetzt und mit Dichlormethan extrahiert. Die vereinigten organischen Phasen werden mit Wasser gewaschen, über Na₂SO₄ getrocknet und im Vacuum eingeengt. Das Rohmaterial wird über Säulenchromatografie an Kieselgel gereinigt. Es werden 5g 4-Brom-1-cyclopropyl-3-(4-fluorphenyl)-1H-pyrazol (73%) erhalten.
logP (pH2.7): 3.59
MS (ESI): 281.0 ([M+H]⁺)
¹H-NMR (400 MHz, CD₃CN): δ = 7.88-7.84 (m, 2H), 7.75 (s, 1H), 7.20-7.16 (m, 2H), 3.68-3.65 (m, 1H), 1.12-1.09 (m, 2H), 1.04-1.01 (m, 2H) ppm

### Herstellung von Ausgangsstoffen der Formel [V] nach Verfahren V2:

### 3-(4-Fluorphenyl)-1-isopropyl-4-(4,4,5,5-tetramethyl-1,3,2-dioxaborolan-2-yl)-1H-pyrazol [V-1]

3.0 g (10.5 mmol) 4-Brom-3-(4-fluorphenyl)-1-isopropyl-1H-pyrazol und 5.38 g (21.1 mmol) Bis-(pinacolato)-diborane werden in 30 mL Dimethylsulfoxid gelöst. Dazu werden 3.1 g Kaliumacetat (31.8 mmol) und 0.86 g (1.06 mmol) 1,1'-Bis(Diphenylphosphino)ferrocene]Dichlorpalladium(II) *CH₂Cl₂ gegeben und die Reaktionsmischung unter Argonfluss 5 h bei 85 °C erhitzt. Nach nochmaligem Erhitzen für 2 h bei 80 °C wird die Reaktionsmischung abgekühlt und das Dimethylsulfoxid am Hochvakuum entfernt. Der Rückstand wird in Wasser gelöst und mehrmals mit Essigsäureethylester extrahiert. Anschließend wird die organische Phase getrocknet (Na₂SO₄) und eingeengt. Die Reinigung erfolgt mittels Kieselgelchromatographie mit dem Eluent Cyclohexan (A) / 20%Essigsäureethylester in Cyclohexan (B) (0% B auf 70% B). Man erhält 3.85 g 3-(4-Fluorphenyl)-1-isopropyl-4-(4,4,5,5-tetramethyl-1,3,2-dioxaborolan-2-yl)-1H-pyrazol als farblosen Feststoff (40% Reinheit nach NMR). Die Verbindung wird ohne weitere Reinigung weiter umgesetzt.
logP (pH2.7): 4.51
MS (ESI): 331.20 ([M+H]⁺)
¹H-NMR (400 MHz, d₃-CD₃CN): δ = 7.90 (dd, 2H), 7.78 (s, 1H), 7.10 (dd, 1H), 4.52 (m, 1H), 1.49 (d, 6H), 1.25 (s, 12H) ppm

### 3-(4-Fluorphenyl)-1-(2-methoxyethyl)-4-(4,4,5,5-tetramethyl-1,3,2-dioxaborolan-2-yl)-1H-pyrazol [V-2]

1.0 g (3.3mmol) 4-Brom-3-(4-fluorphenyl)-1-(2-methoxyethyl)-1H-pyrazol und 1.69 g (2eq, 6.7 mmol) Bis-(pinacolato)-diborane werden in 15m L Dimethylsulfoxid gelöst. Dazu werden 0.98 g Kaliumacetat (10 mmol) und 0.27 g (0.3 mmol) 1,1'-Bis(Diphenylphosphino)ferrocene]Dichlor-palladium(II)*CH₂Cl₂ gegeben und die Reaktionsmischung unter Argonfluß 7 h bei 85 °C erhitzt. Danach wird die Reaktionsmischung abgekühlt und das Dimethylsulfoxid am Hochvakuum entfernt. Der Rückstand wird in Wasser gelöst und mehrmals mit Essigsäureethylester extrahiert. Anschließend wird die organische Phase getrocknet (Na₂SO₄) und eingeengt. Die Reinigung erfolgt mittels Kieselgelchromatographie mit dem Eluent Cyclohexan (A) / 20%Essigsäureethylester in Cyclohexan (B) (0% B auf 70% B). Man erhält 0.39 g 3-(4-Fluorphenyl)-1-(2-methoxyethyl)-4-(4,4,5,5-tetramethyl-1,3,2-dioxaborolan-2-yl)-1H-pyrazol als farblosen Feststoff (60% Reinheit nach NMR). Die Verbindung wird ohne weitere Reinigung weiter umgesetzt.
logP (pH2.7): 3.58
MS (ESI): 347.21 ([M+H]⁺)
¹H-NMR (400 MHz, d₃-CD₃CN): δ = 7.80 (dd, 2H), 7.58 (s, 1H), 7.15 (dd, 1H), 4.30 (m, 2H), 3.75 (m, 2H), 3.30 (s, 3H), 1.28 (s, 12H) ppm

### 3-(4-Fluorphenyl)-1-isobutyl-4-(4,4,5,5-tetramethyl-1,3,2-dioxaborolan-2-yl)-1H-pyrazol [V-3]

2.0 g (6.7 mmol) 4-Brom-3-(4-fluorphenyl)-1-isobutyl-1H-pyrazol und 3.4 g (13.4 mmol) Bis-(pinacolato)-diborane werden in 30 mL Dimethylsulfoxid gelöst. Dazu werden 1.98 g Kaliumacetat (20 mmol) und 0.55 g (0.67 mmol) 1,1'-Bis(Diphenylphosphino)ferrocene]Dichlorpalladium(II)*CH₂Cl₂ gegeben und die Reaktionsmischung unter Argonfluß 7 h bei 85 °C erhitzt. Danach wird die Reaktionsmischung abgekühlt und das Dimethylsulfoxid am Hochvakuum entfernt. Der Rückstand wird in Wasser gelöst und mehrmals mit Essigsäureethylester extrahiert. Anschließend wird die organische Phase getrocknet (Na₂SO₄) und eingeengt. Die Reinigung erfolgt in mittels Kieselgelchromatographie mit dem Eluent Cyclohexan (A) / 20%Essigsäureethylester in Cyclohexan (B) (0% B auf 70% B). Man erhält 1.1 g 3-(4-Fluorphenyl)-1-isobutyl-4-(4,4,5,5-tetramethyl-1,3,2-dioxaborolan-2-yl)-1H-pyrazol als farblosen Feststoff (23% Reinheit nach NMR). Die Verbindung wird ohne weitere Reinigung weiter umgesetzt.
logP (pH2.7): 4.76
MS (ESI): 345.17 ([M+H]⁺)
¹H-NMR (400 MHz, d₃-CD₃CN): δ = 7.90 (dd, 2H), 7.72 (s, 1H), 7.10 (dd, 1H), 3.95 (d, 2H), 2.20 (m, 1H), 1.30 (s, 12H), 0.90 (d, 6H) ppm

Analog zu dem oben beschriebenen Verfahren lassen sich auch folgende Verbindungen des Typs **[III]** darstellen:

### 1-(2,2-Difluorethyl)-3-(4-fluorphenyl)-4-(4,4,5,5-tetramethyl-1,3,2-dioxaborolan-2-yl)-1H-pyrazol [V-4]

logP (pH2.7): 3.84
MS (ESI): 353.2 ([M+H]⁺)
¹H-NMR (400 MHz, d6-DMSO): δ = 8.06 (s, 1H), 7.90-7.87 (m, 2H), 7.21 (m,2H), 6.41(m, 1H), 4.68 (m, 2H), 1.27 (s, 12H) ppm

### 3-(4-Fluorphenyl)-1-isopropoxy-4-(4,4,5,5-tetramethyl-1,3,2-dioxaborolan-2-yl)-1H-pyrazol [V-5]

logP (pH2.7): 4.81
MS (ESI): 347.2 ([M+H]⁺)
¹H-NMR (400 MHz, CD₃CN): δ = 7.89-7.85 (m, 2H), 7.68 (s, 1H), 7.15-7.10 (m, 2H), 4.71 (m, 1H), 1.29 (m, 18H) ppm

### 1-(Cyclopentyloxy)-3-(4-fluorphenyl)-4-(4,4,5,5-tetramethyl-1,3,2-dioxaborolan-2-yl)-1H-pyrazol [V-6]

logP (pH2.7): 5.51
MS (ESI): 373.2 ([M+H]⁺)
¹H-NMR (400 MHz, CD₃CN): δ = 7.89-7.85 (m, 2H), 7.67 (s, 1H), 7.15-7.10 (m, 2H), 1.95-1.93 (m, 7H), 1.80-1.79(m, 2H) ppm

### 1-Cyclopropyl-3-(4-fluorphenyl)-4-(4,4,5,5-tetramethyl-1,3,2-dioxaborolan-2-yl)-1H-pyrazol [V-7]

Zu einer Lösung von 2g (7.11 mmol) 4-Brom-1-cyclopropyl-3-(4-fluorphenyl)-1H-pyrazol und 1.98g (10.67mmol) 2-isopropoxy-4,4,5,5-tetramethyl-1,3,2-dioxaborolan in trockenem Tetrahydrofuran (40 mL) wird unter Argon eine Lösung von n-Butyllithium in n-Hexan (1eq) bei -78°C langsam zugegeben. Die Reaktionsmischung wird 5min bei -78°C gerührt und dann mit wässriger NH₄Cl Lösung versetzt. Nach Erwärmen des Reaktionsgemisches auf Raumtemperatur wird die Reaktionsmischung mit Essigester extrahiert. Die vereinigten organischen Extrakte werden getrocknet und im Vacuum eingeengt. Das erhaltene Rohmaterial wird durch Chromatografie an Kieselgel (Eluent n-hexan/Dichlormethan 2:1) gereinigt. Es werden 900mg (39%) 1-Cyclopropyl-3-(4-fluorphenyl)-4-(4,4,5,5-tetramethyl-1,3,2-dioxaborolan-2-yl)-1H-pyrazol erhalten.
logP (pH2.7): 3.47
MS (ESI): 329.2 ([M+H]⁺)
¹H-NMR (400 MHz, CD₃CN): δ = 7.89-7.86 (m, 2H), 7.81 (s, 1H), 7.13-7.09 (m, 2H), 3.65 (m, 1H), 1.28 (s, 12H), 1.10 (m, 2H), 1.00 (m, 2H) ppm

Analog zu dem oben beschriebenen Verfahren über Metallierung des Pyrazoles lassen sich auch folgende Verbindungen des Typs **[III]** darstellen:

### 1-(Cyclopropylmethyl)-3-(4-fluorphenyl)-4-(4,4,5,5-tetramethyl-1,3,2-dioxaborolan-2-yl)-1H-pyrazol [V-8]

logP (pH2.7): 4.42
MS (ESI): 343.2 ([M+H]⁺)
¹H-NMR (400 MHz, d6-DMSO): δ = 8.01 (s, 1H), 7.90-7.87 (m, 2H), 7.19 (m,2H), 4.00(d, 2H), 0.39-0.55 (m, 5H) ppm

### 3-(4-Fluorphenyl)-1-methyl-4-(4,4,5,5-tetramethyl-1,3,2-dioxaborolan-2-yl)-1H-pyrazol [V-9]

logP (pH2.7): 3.47
MS (ESI): 303.2 ([M+H]⁺)
¹H-NMR (400 MHz, d6-DMSO): δ = 7.95 (s, 1H), 7.90-7.86 (m, 2H), 7.19 (m, 2H), 3.87 (s, 3H), 1.26 (s, 12H) ppm

### 1-(2-Chlorethyl)-3-(4-fluorphenyl)-4-(4,4,5,5-tetramethyl-1,3,2-dioxaborolan-2-yl)-1H-pyrazol [V-10]

logP (pH2.7): 4.06
MS (ESI): 351.1 ([M+H]⁺)
¹H-NMR (400 MHz, d6-DMSO): δ = 8.06 (s, 1H), 7.91-7.87 (m, 2H), 7.22-7.18 (m, 2H), 4.49 (t, 2H), 4.03 (t, 2H), 1.27 (s, 12H) ppm

### Herstellung von Ausgangsstoffen der Formel [IV-c]: 4-[3-(4-Fluorphenyl)-1-isopropyl-1H-pyrazol-4-yl]pyridin-2-amin [IV-c-1]

500 mg (2.9 mmol) 4-Brompyridin-2-amin und 450 µL (3.2 mmol) Triethylamin in 25 mL Tetrahydrofuran gelöst. Dazu werden 338 µL 2-Methylpropanoylchlorid (2.9 mmol) gegeben und die Reaktionsmischung wird bei Raumtemperatur 16 h gerührt. Anschließend werden die flüchtigen Bestandteile im Vakuum entfernt und das Rohmaterial mit 3 mL NH₃ in Methanol (7 molar) versetzt. Die Mischung wird 16 h bei Raumtemperatur gerührt und anschließend eingedampft. Das Rohprodukt wird über Kieselgelchromatographie (Eluent Cyclohexan / Essigsäureethylester) gereinigt. Man erhält 382 mg (47% Ausbeute) N-(4-Brompyridin-2-yl)-2-methylpropanamid als farblosen Feststoff.
logP (pH2.7): 2.09
MS (ESI): 244.9 ([M+H]⁺)
¹H-NMR (400 MHz, d₃-CD₃CN): δ = 8.70 (s, 1H, br), 8.40 (d, 1H), 8.12 (d, 1H), 7.25 (dd, 1H), 2.65 (m, 1H), 1.15 (d, 6H) ppm

### Herstellung von Ausgangsstoffen der Formel [III] nach Verfahren (V3):

### 4-[3-(4-Fluorphenyl)-1-isopropyl-1H-pyrazol-4-yl]pyridin-2-amin [III-1]

200 mg (0.6 mmol) 3-(4-Fluorphenyl)-1-isopropyl-4-(4,4,5,5-tetramethyl-1,3,2-dioxaborolan-2-yl)-1H-pyrazol und 166 mg (0.72mmol) tert-Butyl-(4-chlorpyridin-2-yl)carbamat werden in 3 mL 1,4-Dioxan gelöst. Dazu werden 44.7 mg Bis(tricyclohexylphosphin)palladium(II)-dichlorid (0.06 mmol) und 2 mL Natriumcarbonat-lösung (2 molar) gegeben. Die Reaktionsmischung wird 5 min mit Argon gespült und danach verschlossen. Anschließend wird die Mischung 12 min bei 150 °C in der Mikrowelle erhitzt (CEM Explorer). Nach dem Abkühlen wird von unlöslichen Bestandteilen abfiltriert und der Salzrückstand mit 1,4-Dioxan gewaschen. Die organische Phase wird eingedampft und das Rohprodukt über Kieselgelchromatographie (Eluent Cyclohexan / Essigsäureethylester) gereinigt. Man erhält 45.4 mg (25% Ausbeute) 4-[3-(4-Fluorphenyl)-1-isopropyl-1H-pyrazol-4-yl]pyridin-2-amin als farblosen Feststoff.
logP (pH2.7): 1.22
MS (ESI): 297.13 ([M+H]⁺)
¹H-NMR (400 MHz, d₃-CD₃CN): δ = 7.80 (m, 2H), 7.50 (dd, 2H), 7.10 (dd, 1H), 6.47 (d, 1H), 6.39 (s, 1H), 5.10 (s, 2H, br), 4.53 (m, 1H), 1.20 (d, 6H) ppm

### Herstellung von Ausgangsstoffen der Formel [III] nach Verfahren (V26):

### 4-[3-(4-Fluorphenyl)-1-(2-methoxyethyl)-1H-pyrazol-4-yl]pyridin-2-amin [III-2]

257 mg (0.86mmol) 4-Brom-3-(4-fluorphenyl)-1-(2-methoxyethyl)-1H-pyrazol und 303 mg (0.94mmol) tert-Butyl-[4-(4,4,5,5-tetramethyl-1,3,2-dioxaborolan-2-yl)pyridin-2-yl]carbamat werden in 4 mL 1,4-Dioxan gelöst. Dazu werden 50.8 mg Bis(tricyclohexylphosphin)palladium(II)-dichlorid (0.04 mmol) und 2 mL Natriumcarbonat-lösung (2 M in H2O) gegeben. Die Reaktionsmischung wird 5 min mit Argon gespült und danach verschlossen. Anschließend wird die Mischung 12 min bei 150 °C in der Mikrowelle erhitzt (CEM Explorer). Nach dem Abkühlen wird von unlöslichen Bestandteilen abfiltriert und der Salzrückstand mit 1,4-Dioxan gewaschen. Die organische Phase wird eingedampft und das Rohprodukt über Kieselgelchromatographie (Eluent Dichlormethan / 10%Methanol - Dichlormethan) gereinigt. Man erhält 255 mg (86 % Ausbeute) 4-[3-(4-Fluorphenyl)-1-(2-methoxyethyl)-1H-pyrazol-4-yl]pyridin-2-amin als farblosen Feststoff.
logP (pH2.7): 0.98
MS (ESI): 313.15 ([M+H]⁺)
¹H-NMR (400 MHz, d₃-CD₃CN): δ = 7.85 (d, 1H), 7.77 (s, 1H), 7.48-7.46 (m, 2H), 7.12-7.09 (m, 2H), 6.44 (dd, 1H), 6.37 (s, 1H), 4.79 (s, 2H, br), 4.29 (t, 2H), 3.77 (t, 2H), 3.31 (s, 3H) ppm

Analog zu den beschriebenen Verfahren lassen sich auch folgende Verbindungen des Typs **[III]** darstellen:

### 4-[1-Ethyl-3-(4-fluorphenyl)-1H-pyrazol-4-yl]pyridin-2-amin [III-3]

logP (pH2.7): 0.97
MS (ESI): 283.32 ([M+H]⁺)
¹H-NMR (400 MHz, d₃-CD₃CN): δ = 7.85 (m, 1H), 7.77 (s, 1H), 7.48-7.46 (m, 2H), 7.12-7.09 (m, 2H), 6.45 (dd, 1H), 6.37 (s, 1H), 4.82 (s, 2H, br), 4.20 (q, 2H), 1.49 (t, 3H) ppm

### 4-[1-(2,2-Difluorethyl)-3-(4-fluorphenyl)-1H-pyrazol-4-yl]pyridin-2-amin [III-4]

logP (pH2.7): 1.03
MS (ESI): 319.48 ([M+H]⁺)
¹H-NMR (400 MHz, d₃-CD₃CN): δ = 7.86 (d, 1H), 7.82 (s, 1H), 7.48-7.46 (m, 2H), 7.12-7.09 (m, 2H), 6.44 (dd, 1H), 6.37 (s, 1H), 6.26 (td, 1H), 4.87 (s, 1H), br), 4.57 (dt, 2H) ppm

### 4-[3-(4-Fluorphenyl)-1-methyl-1H-pyrazol-4-yl]pyridin-2-amin [III-5]

logP (pH2.7): 0.71
MS (ESI): 269.2 ([M+H]⁺)
¹H-NMR (400 MHz, d6-DMSO): δ = 7.95 (s, 1H), 7.81 (m, 1H), 7.45-7.42 (m, 2H), 7.21-7.18 (m, 2H), 6.31-6.29 (m, 2H), 5.80 (s, 2H, br), 3.90 (s, 3H) ppm

### Herstellung von Zwischenverbindungen der Formel [XV-a] :

### 2-Methoxy-N-[4-(4,4,5,5-tetramethyl-1,3,2-dioxaborolan-2-yl)pyridin-2-yl]acetamid [XV-a-1]

2.0 g (8.5 mmol) N-(4-Brompyridin-2-yl)-2-methoxyacetamid und 2.4 g (9.3 mmol) Bis-(pinacolato)-diborane werden in 50 mL trockenem Dioxan gelöst. Dazu werden 2.50 g Kaliumacetat (25.5 mmol) und 0.31 g (0.38 mmol) 1,1'-Bis(Diphenylphosphino)ferrocene]Dichlorpalladium(II)*CH₂Cl₂ gegeben und die Reaktionsmischung wird unter Argonfluß 3 h bei 80 °C erhitzt. Danach wird die Reaktionsmischung abgekühlt, Wasser zugegeben und mehrmals mit Essigsäureethylester extrahiert. Anschließend wird die organische Phase getrocknet (Na₂SO₄) und eingeengt. Die Reinigung erfolgt in mittels Kieselgelchromatographie mit dem Eluent Hexan / Ether (3:1). Man erhält 1.32 g (53% Ausbeute) 2-Methoxy-N-[4-(4,4,5,5-tetramethyl-1,3,2-dioxaborolan-2-yl)pyridin-2-yl]acetamid als farblosen Feststoff.
logP (pH2.7): -0.18
MS (ESI): 211.13 ([M(-pinakol)+H]⁺)
¹H-NMR (400 MHz, d₃-CD₃CN): δ = 8.75 (s, 1H, br), 8.38 (s, 1H), 8.33 (d, 1H), 7.33 (m, 1H), 4.00 (s, 2H), 3.46 (s, 3H), 1.34 (s, 12H) ppm

### N-[4-(4,4,5,5-Tetramethyl-1,3,2-dioxaborolan-2-yl)pyridin-2-yl]propanamid [XV-a-2]

1.80 g (7.9 mmol) N-(4-Brompyridin-2-yl)propanamid und 2.19 g (8.6 mmol) Bis-(pinacolato)-diborane werden in 50 mL trockenem Dioxan gelöst. Dazu werden 2.31 g Kaliumacetat (23.6mmol) und 0.35g (0.43 mmol) 1,1'-Bis(Diphenylphosphino)ferrocene]Dichlorpalladium(II)*CH₂Cl₂ gegeben und die Reaktionsmischung wird unter Argonfluß 3 h bei 80 °C erhitzt. Danach wird die Reaktionsmischung abgekühlt, Wasser zugegeben und mehrmals mit Essigsäureethylester extrahiert. Anschließend wird die organische Phase getrocknet (Na₂SO₄) und eingeengt. Die Reinigung erfolgt in mittels Kieselgelchromatographie mit dem Eluent Hexan / Ether (3:1). Man erhält 0.870 g (36% Ausbeute) N-[4-(4,4,5,5-Tetramethyl-1,3,2-dioxaborolan-2-yl)pyridin-2-yl]propanamid als farblosen Feststoff.
¹H-NMR (400 MHz, d₃-CD₃CN): δ = 8.55 (s, 1H, br), 8.37 (s, 1H), 8.28 (d, 1H), 7.27 (m, 1H), 2.43 (q, 2H), 1.34 (s, 12H), 1.15 (t, 3H) ppm

### 2-Phenyl-N-[4-(4,4,5,5-tetramethyl-1,3,2-dioxaborolan-2-yl)pyridin-2-yl]acetamid [XV-a-3]

1.40 g (4.81mmol) N-(4-Brompyridin-2-yl)-2-phenylacetamid und 1.34 g (5.3mmol) Bis-(pinacolato)-diborane werden in 50 mL trockenem Dioxan gelöst. Dazu werden 1.42 g Kaliumacetat (14.3 mmol) und 0.18 g (0.22 mmol) 1,1'-Bis(Diphenylphosphino)ferrocene]Dichlorpalladium-(II)*CH₂Cl₂ gegeben und die Reaktionsmischung wird unter Argonfluß 3 h bei 80 °C erhitzt. Danach wird die Reaktionsmischung abgekühlt, Wasser zugegeben und mehrmals mit Essigsäureethylester extrahiert. Anschließend wird die organische Phase getrocknet (Na₂SO₄) und eingeengt. Die Reinigung erfolgt durch Triturieren des Produkts mit Hexan / Ether (3:1). Man erhält 0.87 g (54% Ausbeute) 2-Phenyl-N-[4-(4,4,5,5-tetramethyl-1,3,2-dioxaborolan-2-yl)pyridin-2-yl]acetamid als farblosen Feststoff..
¹H-NMR (400 MHz, d₃-CD₃CN): δ = 8.68 (s, 1H, br), 8.33 (s, 1H), 8.28 (d, 1H), 7.36 (m, 5H), 7.27 (m, 1H), 3.72 (s, 2H), 1.32 (s, 12H) ppm

Auf analoge Weise lassen sich auch die folgenden Intermediate des Typs [XV-a] herstellen:

### 2-Methyl-N-[4-(4,4,5,5-tetramethyl-1,3,2-dioxaborolan-2-yl)pyridin-2-yl]propanamid [XV-a-4]

logP (pH2.7): 0.04
MS (ESI): 209.1 ([M-C6H12]⁺)
¹H-NMR (400 MHz, CD₃CN): δ = 8.39 (s, 1H), 8.29(d, 1H), 7.28(d, 1H), 1.94(m, 1H), 1.34 (s, 12H), 1.17 (d, 6H) ppm

### 2-Cyclopropyl-N-[4-(4,4,5,5-tetramethyl-1,3,2-dioxaborolan-2-yl)pyridin-2-yl]acetamid [XV-a-5]

logP (pH2.7): 0.27
MS (ESI): 221.1 ([M-C6H12]⁺)
¹H-NMR (400 MHz, CD₃CN): δ = 8.60 (s, 1H, br), 8.39 (s, 1H), 8.29(d, 1H), 7.28 (d, 1H) 2.29 (d, 2H), 1.34 (s, 12H), 1.10 (m, 1H), 0.57 (m, 2H), 0.25 (m, 2H) ppm

### Ethyl-[4-(4,4,5,5-tetramethyl-1,3,2-dioxaborolan-2-yl)pyridin-2-yl]carbamat [XV-a-6]

logP (pH2.7): 0.00
MS (ESI): 211.1 ([M-C6H12]⁺)
¹H-NMR (400 MHz, CD₃CN): δ = 8.28 (m, 2H), 8.18(s, 1H), 7.24(d, 1H), 1.94(m, 1H), 4.21 (q, 2H), 1.34 (s, 12H), 1.29 (t, 3H) ppm

### N-[4-(4,4,5,5-Tetramethyl-1,3,2-dioxaborolan-2-yl)pyridin-2-yl]cyclopropancarboxamid [XV-a-7]

logP (pH2.7): 0.00
MS (ESI): 207.1 ([M-C6H12]⁺)
¹H-NMR (400 MHz, CD₃CN): δ = 8.36 (s, 1H), 8.29 (d, 1H), 7.27 (d, 1H), 1.80(m, 1H), 1.33 (s, 12H), 0.93 (m, 3H), 0.84 (m, 2H) ppm

### Herstellung von Zwischenverbindungen der Formel [XIII] :

### 4-Brom-1-(tetrahydro-2H-pyran-2-yl)-1H-pyrazol [XIII-1]

Zu einer Lösung von Pyrazol (100 g, 1.47 mol) in auf 40 °C vorgewärmten H₂O (400 ml) werden 234.7 g Brom (75.26 ml, 1.473 mol) tropfenweise zugegeben. Die Reaktionslösung wird 30 min unter Rückfluß erhitzt und gerührt (TLC, Hexan:EtOAc 1:1, *R_{f}* = 0.6). Nach Abkühlung der Reaktionslösung (pH = 3) auf Raumtemperatur wird konz. NaOH_{(aq)} tropfenweise zugegeben und der pH auf 8 eingestellt (Ausfällung eines weißen Niederschlags). Die erhaltene Suspension wird abfiltriert, der Rückstand mit eiskaltem H₂O (150 ml) gewaschen und anschließend im Vacuum getrocknet. Man erhält 195.47 g des Intermediats 4-Brom-1H-pyrazol (91% Ausbeute, Reinheitsgrad von 99%) als weißen Feststoff der ohne weitere Reinigung weiter umgesetzt wird.

Eine Suspension von 4-Brom-1H-pyrazol (181 g, 1.23 mol), 3,4-Dihydro-*2H-*pyran (155.5 g, 168.6 ml, 1.85 mol) und Trifluoressigsäure (0.84 g, 0.57 ml, 7.40 mmol) wird 5 h unter Rückfluß erhitzt und gerührt. Anschließend wird das erhaltene Rohprodukt nach Zugabe von NaH (1.18 g, 0.05 mol) fraktionierend destilliert. Man erhält 253 g 4-Brom-1-(tetrahydro-2H-pyran-2-yl)-1H-pyrazol (89%) als farblose Flüssigkeit (Sdp. 88-90 °C bei einem Druck von 0.02 mmHg).

Die spektroskopischen Daten entsprechen den in der Literatur beschriebenen Daten (Acta Chem. Scand. Series B: Organic Chemistry and Biochemistry 1982, 36, 2, 101-108)
1H-NMR (400 MHz, d₃-CD₃CN): δ = 7.78 (s, 1H), 7.47 (s, 1H), 5.33 (dd, 1H), 3.95 (m, 1H), 3.65 (m, 1H), 2.10-2.00 (m, 2H), 1.70-1.50 (m, 4H) ppm

### Herstellung von Zwischenverbindungen der Formel [XII] :

### 4-[1-(Tetrahydro-2H-pyran-2-yl)-1H-pyrazol-4-yl]pyridin [XII-1]

Zu einer Suspension von 39.0 g 4-Brom-1-(tetrahydro-2H-pyran-2-yl)-1H-pyrazol (0.17 mol), 675 mL wässriges Na₂CO₃ (2.0 molar in H₂O, 1.35 mol) und 25.1 g 4-Pyridinboronsäure (0.21 mol) in Dioxan (2000 mL) werden 4.88 g Pd(PPh₃)₄ (4.22 mmol, 2.5 Mol%) gegeben. Die Reaktionsmischung wird unter Schutzgasatmosphäre und unter Rückfluß bei 80 °C erhitzt und 41 h gerührt. Anschließend wird die Reaktion mit H₂O (50 mL) versetzt. Die Reaktionslösung wird auf ¼ des Volumens eingeengt und mit Essigsäureethylester (3 x 300 mL) extrahiert. Die vereinigten organischen Phasen werden mit gesättigter NaCl-Lösung gewaschen und anschließend mit MgSO₄ getrocknet. Das erhaltene Rohprodukt wird über eine Kugelrohr-Destillation gereinigt (Sdp. 130-135 °C bei p = 0.02 mmHg). Man erhält 26.37 g (, bis ein Reinigungsgrad von 97.3 % erreicht werden konnte. Man erhielt 26.37 g 4-[1-(Tetrahydro-2H-pyran-2-yl)-1H-pyrazol-4-yl]pyridin (68%) als gelbes hochviskoses Öl.
logP (pH2.7): 0.38
MS (ESI): 230.1 ([M+H]⁺)
1H-NMR (400 MHz, d₃-CD₃CN): δ = 8.49 (d, 2H), 8.20 (s, 1H), 7.94 (s, 1H), 7.48 (d, 2H), 5.39 (dd, 1H), 4.00 (m, 1H), 3.69 (m, 1H), 2.10-2.00 (m, 2H), 1.70-1.50 (m, 4H) ppm

### Herstellung von Zwischenverbindungen der Formel [XI] :

### 4-[1-(Tetrahydro-2H-pyran-2-yl)-5-(tributylstannyl)-1H-pyrazol-4-yl]pyridin [XI-1]

Zu einer Lösung von 7.0 g 4-[1-(Tetrahydro-2H-pyran-2-yl)-1H-pyrazol-4-yl]pyridin (30.5 mmol) in trockenem THF (200 mL) werden bei -70 °C unter einer Schutzgasathmosphäre und Rühren 13.5 mL n-Butyllithium (2.5 molar in n-Hexan, 33.75 mmol) zugegeben. Nach beendeter Zugabe wird noch eine Stunde bei dieser Temperatur gerührt. Danach werden 9.5 g Tri-n-Butylzinnchlorid (29.2 mmol) zugegeben. Anschließend lässt man die Reaktionsmischung wird auf Raumtemperatur erwärmen und rührt noch 15 min bei dieser Temperatur nach. Alle flüchtigen Komponenten werden im Vakuum verdampft und der Rückstand wird im Hochvakuum (<0.1 mbar) destilliert. Die Fraktion mit einem Siedepunkt oberhalb 130 °C wird isoliert und mittels Chromatografie weiter gereinigt (n-Hexane/Diethyl Ether = 1:4 eluent). Es werden 7.5 g 4-[1-(Tetrahydro-2H-pyran-2-yl)-5-(tributylstannyl)-1H-pyrazol-4-yl]pyridin (45%) erhalten
logP (pH2.7): 5.09
MS (ESI): 520.1 ([M+H]⁺)
¹H-NMR (400 MHz, d₃-CD₃CN): δ = 8.50 (d, 2H), 8.20 (s, 1H), 7.94 (s, 1H), 7.48 (d, 2H), 5.40 (dd, 1H), 4.00 (m, 1H), 3.68 (m, 1H), 2.10-2.00 (m, 2H), 1.70-1.50 (m, 10H), 1.40-1.30 (m, 6H), 1.10-1.00 (m, 6H), 0.89 (t, 9H) ppm

### Herstellung von Zwischenverbindungen der Formel [X] :

### 4-[5-(4-Fluorphenyl)-1-(tetrahydro-2H-pyran-2-yl)-1H-pyrazol-4-yl]pyridin [X-1]

250 mg (0.48 mmol) 4-[1-(Tetrahydro-2H-pyran-2-yl)-5-(tributylstannyl)-1H-pyrazol-4-yl]pyridin und 126 mg (0.72 mmol) 4-Bromfluorbenzol werden in 3mL Dimethylformamid verrührt. Dazu werden 146 mg Cäsiumfluorid (0.96 mmol), 84 mg Tetrakis(triphenylphosphin)palladium(0) (0.07 mmol, 15 Mol%) und 9 mg Kupfer(I)iodid (0.05 mmol, 10 Mol %) gegeben und die Mischung wird für 5 min mit Schutzgas entgast. Danach wird die Mischung bei 150 °C für 20 min in der Mikrowelle erhitzt (CEM Discover). Anschließend wird die Rohmischung über eine Kartusche mit Celite filtriert und die flüchtigen Komponenten im Vakuum entfernt. Das Rohprodukt wird über Chromatografie an Kieselgel (Cyclohexan /Essigsäureethylester) gereinigt und es werden 47.4 mg 4-[5-(4-Fluorphenyl)-1-(tetrahydro-2H-pyran-2-yl)-1H-pyrazol-4-yl]pyridin (30%) sowie 41 mg des abgespaltenen Produkts 4-[3-(4-Fluorphenyl)-1H-pyrazol-4-yl]pyridin **[IX-1]** (35%) als farbloses Öl erhalten.

### 4-[5-(4-Fluorphenyl)-1-(tetrahydro-2H-pyran-2-yl)-1H-pyrazol-4-yl]pyridin [X-1]

logP (pH2.7): 1.23
MS (ESI): 324.18 ([M+H]⁺)
¹H-NMR (400 MHz, d₃-CD₃CN): δ = 8.36 (dd, 2H), 7.90 (s, 1H), 7.42 (dd, 2H), 7.26 (dd, 2H), 7.09 (dd, 2H), 5.01 (dd, 1H), 3.96 (m, 1H), 2.40 (m, 1H), 1.82 (m, 1H), 1.70-1.45 (m, 3H), 1.35-1.25 (m, 1H) ppm

### Herstellung von Zwischenverbindungen der Formel [IX] :

### 4-[3-(4-Fluorphenyl)-1H-pyrazol-4-yl]pyridin [IX-1]

Die in der allgemeinen Vorschrift VII hergestellten Zwischenprodukte können auch ohne weitere Reinigung in der Entschützungsreaktion eingesetzt werden.

Analog zu oben beschriebenem Verfahren (VII) werden 750 mg (1.45 mmol) 4-[1-(Tetrahydro-2H-pyran-2-yl)-5-(tributylstannyl)-1H-pyrazol-4-yl]pyridin, 380 mg (2.17 mmol) 4-Bromfluorbenzol, 440 mg Cäsiumfluorid (2.89 mmol), 250 mg Tetrakis(triphenylphosphin)-palladium(0) (0.28 mmol, 15 Mol%) und 28 mg Kupfer(I)iodid (0.15 mmol, 10 Mol%) umgesetzt. Nach Abfiltrieren der unlöslichen Bestandteile über Celite und Entfernen der flüchtigen Komponenten im Vakuum werden 950 mg eines Rohprodukts erhalten.

Das Rohprodukt wird in 5 mL Methanol gelöst und mit 5.8 mL HCl in Dioxan (4 molar) versetzt. Die Lösung wird bei Raumtemperatur 1.5 h gerührt und anschließend eingeengt. Der erhaltene Feststoff wird mehrmals mit Diethylether trituriert und es werden 387 mg 4-[3-(4-Fluorphenyl)-1H-pyrazol-4-yl]pyridin hydrochlorid (75%) als weisser Feststoff erhalten. Aus diesem kann das freie 4-[3-(4-Fluorphenyl)-1H-pyrazol-4-yl]pyridin durch Auflösen des Hydrochlorids in Essigsäureester und waschen mit Natriumcarbonat in Form des salzfreien Pyrazols gewonnen werden.

### 4-[3-(4-Fluorphenyl)-1H-pyrazol-4-yl]pyridin hydrochlorid [IX-1]

logP (pH2.7): 0.65
MS (ESI): 240.11 ([M+H]⁺)
¹H-NMR (400 MHz, d₃-CD₃CN): δ = 11.3 (s, 0.5H), 8.44 (dd, 2H), 7.89 (s, 1H, br), 7.45 (dd, 2H), 7.40 (s, 0.5H, br), 7.23 (dd, 2H), 7.15 (m, 2H) ppm

### Herstellung von Verbindungen der Formel [I] nach Verfahren (V7):

### 4-[3-(4-Fluorphenyl)-1-isopropyl-1H-pyrazol-4-yl]chinolin [I-1]

79 mg (0.24 mmol) 3-(4-Fluorphenyl)-1-isopropyl-4-(4,4,5,5-tetramethyl-1,3,2-dioxaborolan-2-yl)-1H-pyrazol und 59 mg (0.36mmol) 4-Chlorchinolin werden in 2.5 mL 1,4-Dioxan gelöst. Dazu werden 17.7 mg 1,1'-Bis(Diphenylphosphino)ferrocene]Dichlorpalladium(II)*CH₂Cl₂ (0.01 mmol) und 0.5 mL Natriumcarbonatlösung (2 molar) gegeben. Die Reaktionsmischung wird 5min mit Argon gespült und danach verschlossen. Anschließend wird die Mischung 12 min bei 150 °C in der Mikrowelle erhitzt (CEM Explorer). Nach dem Abkühlen wird von unlöslichen Bestandteilen über Celite abfiltriert und der Rückstand mit 1,4-Dioxan gewaschen. Die organische Phase wird eingedampft und das Rohprodukt über präparative HPLC (XTerra 125x19mm, 5µm, Gradient: 0-1.5 min 80% Wasser, 15% Methanol, 5% wässrige 10%-ige NH₄HCO₃-Lsg, 1.5-10.0 min linearer Gradient auf 0% Wasser, 95% Methanol, 5% wässrige 10%-ige NH₄HCO₃-Lsg, 10.0-15.0 min 0% Wasser, 95% Methanol, 5% wässrige 10%-ige NH₄HCO₃-Lsg) gereinigt. Man erhält 25 mg (22%) 4-[3-(4-Fluorphenyl)-1-isopropyl-1H-pyrazol-4-yl]chinolin als farblosen Feststoff.
logP (pH2.7): 2.23
MS (ESI): 332.07 ([M+H]⁺)
¹H-NMR (400 MHz, d₆-DMSO): δ = 8.86 (d, 1H), 8.15 (s, 1H), 8.05 (d, 1H), 7.75-7.70 (m, 2H), 7.48 (m, 1H), 7.35 (d, 1H), 7.28 (dd, 2H), 7.03 (t, 2H), 4.65 (m, 1H), 1.56 (d, 6H) ppm

### Herstellung von Verbindungen der Formel [I] nach Verfahren (V6):

### N-{4-[3-(4-Fluorphenyl)-1-(2-methoxyethyl)-1H-pyrazol-4-yl]pyridin-2-yl}propanamid [I-2]

50 mg (0.18mmol) N-[4-(4,4,5,5-Tetramethyl-1,3,2-dioxaborolan-2-yl)pyridin-2-yl]propanamid und 42 mg (0.13mmol) 4-Brom-3-(4-fluorphenyl)-1-(2-methoxyethyl)-1H-pyrazol werden in 2.5 mL 1,4-Dioxan gelöst. Dazu werden 11.3 mg 1,1'-Bis(Diphenylphosphino)ferrocene]Dichlorpalladium(II) *CH₂Cl₂ (0.01 mmol) und 1 mL Cäsiumcarbonatlösung (2 molar) gegeben. Die Reaktionsmischung wird 5 min mit Argon gespült und danach verschlossen. Anschließend wird die Mischung 25 min bei 90 °C in der Mikrowelle erhitzt (CEM Explorer). Nach dem Abkühlen wird von unlöslichen Bestandteilen über Celite abfiltriert und der Rückstand mit 1,4-Dioxan gewaschen. Die organische Phase wird eingedampft und das Rohprodukt über präparative HPLC (Macherey Nagel, Nucleodur C18 100-5 ec, VP50x21 mm, Gradient: 0-1.5 min 90% Wasser, 10% Methanol, 1.5-10.0 min linearer Gradient auf 5% Wasser, 95% Methanol, 10.0-15.0 min 0% Wasser, 95% Methanol, Modifier 20% HCOOH in H2O, Zugabe des Modifiers mit 2.0 mL/min während der gesamten Trennung) gereinigt. Man erhält 46 mg (69%) N-{4-[3-(4-Fluorphenyl)-1-(2-methoxyethyl)-1H-pyrazol-4-yl]pyridin-2-yl}propanamid als farblosen Feststoff.
logP (pH2.7): 1.61
MS (ESI): 369.22 ([M+H]⁺)
1 H-NMR (400 MHz, d₃-CD₃CN): δ = 8.54 (s, 1 H, br), 8.10 (m, 2H), 7.86 (s, 1 H), 7.46 (dd, 2H), 7.09 (t, 2H), 6.87 (dd, 1H), 4.31 (t, 2H), 3.78 (t, 2H), 3.32 (s, 3H), 2.38 (q, 2H), 1.11 (t, 2H) ppm

### Herstellung von Verbindungen der Formel [I] nach Verfahren (V13):

### 3-{[3-(4-Fluorphenyl)-4-(pyridin-4-yl)-1H-pyrazol-1-yl]methyl}benzonitril [I-3]

### 3-{[5-(4-Fluorphenyl)-4-(pyridin-4-yl)-1H-pyrazol-1-yl]methyl}benzonitril [I-4]

60 mg (0.25 mmol) 4-[3-(4-Fluorphenyl)-1H-pyrazol-4-yl]pyridin wird in 2 mL Dimethylformamid gelöst. Dazu werden 12.7 mg Natriumhydrid (0.32 mol) als 60%-ige Suspension in Mineralöl gegeben und 10 min bei Raumtemperatur verrührt. Anschließend werden 74 mg (0.38 mmol) 3-(Brommethyl)benzonitril zugegeben und die Reaktionsmischung wird 1 h bei Raumtemperatur gerührt. Zur Aufarbeitung werden ca 2 µL Essigsäure (0.03 mmol) zugegeben. Die erhaltene Suspension wird filtriert und das Rohprodukt wird über präparative HPLC (XTerra 125x19 mm, 5µm, Gradient: 0-1.5 min 80% Wasser, 15% Methanol, 5% wässrige 10%-ige NH₄HCO₃-Lsg, 1.5-10.0 min linearer Gradient auf 15% Wasser, 80% Methanol, 5% wässrige 10%-ige NH₄HCO₃-Lsg, 10.0-15.0 min 15% Wasser, 80% Methanol, 5% wässrige 10%-ige NH₄HCO₃-Lsg) gereinigt. Man erhält 27 mg (30%) des Hauptisomers 3-{[3-(4-Fluorphenyl)-4-(pyridin-4-yl)-1H-pyrazol-1-yl]methyl}benzonitril **[I-3]** als Mischung (im Verhältnis 58:37) mit dem Minder-Regioisomeren 3-{[5-(4-Fluorphenyl)-4-(pyridin-4-yl)-1H-pyrazol-1-yl]methyl}benzonitril **[I-4]** als farblosen Feststoff.
logP (pH2.7): 1.51 Hauptisomer
logP (pH2.7): 1.38 Minderisomer
MS (ESI): 355.2 ([M+H]⁺) für beide Isomere
¹H-NMR (400 MHz, d₃-CD₃CN): δ = 8.47 (dd), 8.37 (m), 8.16 (s), 7.86 (s), 7.81 (d), 7.70 (m), 7.50 (t), 7.45-7.30 (m), 7.25-7.10 (m), 7.12 (dd), 5.47 (s, 2H, CH₂-Hauptisomer), 5.27 (s, 2H, CH₂-Nebenisomer) ppm

Analog obigem Beispiel sowie entsprechend den allgemeinen Beschreibungen der erfindungsgemäßen Verfahren können die in der folgenden Tabelle 1 genannten Verbindungen der Formel [I] erhalten werden. Diese können in Form von Isomerenmischung gebildet werden, wobei sich der Anteil des Haupt und Nebenisomers je nach verwendetem Substrat unterscheiden kann.

### Herstellung von Verbindungen der Formel [I-c] nach Verfahren (V4):

### N-{4-[3-(4-Fluorphenyl)-1-isopropyl-1H-pyrazol-4-yl]pyridin-2-yl}cyclopropancarboxamid [I-c-1]

22 mg (0.077 mmol) 4-[3-(4-Fluorphenyl)-1-isopropyl-1H-pyrazol-4-yl]pyridin-2-amin und 12 µL (0.084mmol) Triethylamin werden in 2 mL Tetrahydrofuran gelöst. Dazu werden 8.8 mg Cyclopropancarbonsäurechlorid (0.084 mmol) gegeben und die Reaktionsmischung wird bei Raumtemperatur 2 Tage gerührt. Anschließend werden die flüchtigen Bestandteile im Vakuum entfernt und das Rohmaterial mit 3 mL NH₃ in Methanol (7 molar) versetzt. Die Mischung wird 2 h bei Raumtemperaur verrührt und anschließend eingedampft. Das Rohprodukt wird über Kieselgelchromatographie (Eluent Cyclohexan / Essigsäureethylester) gereinigt. Man erhält 11.2 mg (40%) N-{4-[3-(4-Fluorphenyl)-1-isopropyl-1H-pyrazol-4-yl]pyridin-2-yl}cyclopropancarboxamid als farblosen Feststoff.
logP (pH2.7): 2.07
MS (ESI): 365.13 ([M+H]⁺)
1H-NMR (400 MHz, d₃-CD₃CN): δ = 8.82 (s, 1H, br), 8.11 (d, 1H), 8.07 (s, 1H), 7.85 (s, 1H), 7.44 (dd, 2H), 7.06 (t, 2H), 6.86 (dd, 1H), 4.54 (m, 1H), 1.78 (m, 1H), 1.51 (d, 6H), 0.90-0.80 (m, 4H) ppm

### Herstellung von Verbindungen der Formel [I-c] nach Verfahren (V5):

### 4-[3-(4-Fluorphenyl)-1-isopropyl-1H-pyrazol-4-yl]chinolin [I-c-2]

80 mg (0.18 mmol) 3-(4-Fluorphenyl)-1-isobutyl-4-(4,4,5,5-tetramethyl-1,3,2-dioxaborolan-2-yl)-1H-pyrazol und 68 mg (0.27 mmol) N-(4-Brompyridin-2-yl)-2-methylpropanamid werden in 2.5 mL 1,4-Dioxan gelöst. Dazu werden 15 mg 1,1'-Bis(Diphenylphosphino)ferrocene]Dichlorpalladium-(II)*CH₂Cl₂ (0.01mmol) und 0.5 mL Natriumcarbonatlösung (2 molar) gegeben. Die Reaktionsmischung wird 5 min mit Argon gespült und danach verschlossen. Anschließend wird die Mischung 25 min bei 80 °C in der Mikrowelle erhitzt (CEM Explorer). Nach dem Abkühlen wird von unlöslichen Bestandteilen über Celite abfiltriert und der Rückstand mit 1,4-Dioxan gewaschen. Die organische Phase wird eingedampft und das Rohprodukt Kieselgelchromatographie (Eluent Cyclohexan / Essigsäureethylester) gereinigt. Man erhält 29 mg (40% Ausbeute) N-{4-[3-(4-Fluorphenyl)-1-isobutyl-1H-pyrazol-4-yl]pyridin-2-yl}-2-methylpropanamid als farblosen Feststoff.
logP (pH2.7): 2.89
MS (ESI): 381.19 ([M+H]⁺)
1H-NMR (400 MHz, d₃-CD₃CN): δ = 8.59 (s, 1H, br), 8.10 (m, 2H), 7.83 (s, 1H), 7.46 (dd, 2H), 7.09 (dd, 2H), 6.86 (m, 1H), 3.96 (d, 2H), 2.62 (m, 1H), 2.25 (m, 1H), 1.13 (d, 6H), 0.93 (d, 6H) ppm

### Herstellung von Verbindungen der Formel [I-d] nach Verfahren (V17):

### 4-[3-(2,6-Difluorphenyl)-1-isopropyl-1H-pyrazol-4-yl]pyridin [I-d-1] und

### 4-[5-(2,6-Difluorphenyl)-1-isopropyl-1H-pyrazol-4-yl]pyridin [I-d-2]

Eine Mischung aus 1-(2,6-Difluorphenyl)-3-(dimethylamino)-2-(pyridin-4-yl)prop-2-en-1-on (0.86 mmol), Isopropylhydrazin (1.3 mmol) und Triethylamin (1.3 mmol) in 5 ml Ethanol wird 15 min bei 120 °C in der Mikrowelle bestrahlt. Das Lösungsmittel wird im Vakuum evaporiert und der Rückstand über Kieselgel chromatographiert (Gradient Heptan/EE 20:1 bis 5:1). Man erhält 69 mg of 4-[3-(2,6-Difluorphenyl)-1-isopropyl-1H-pyrazol-4-yl]pyridin (25% Ausbeute) und 34mg (12% Ausbeute) 4-[5-(2,6-Difluorphenyl)-1-isopropyl-1H-pyrazol-4-yl]pyridin.
4-[3-(2,6-Difluorphenyl)-1-isopropyl-1H-pyrazol-4-yl]pyridine
logP (pH2.7): 1.40
MS (ESI): 300.2 ([M+H]⁺)
¹H-NMR (400 MHz, d₆-DMSO): δ = 8.43 (d, 2H), 8.21 (s, 1H), 7.78 (m, 1H), 7.30 (t, 2H), 7.10 (d, 2H), 4.19 (m, 1H), 1.38 (d, 6H) ppm

### 4-[5-(2,6-Difluorphenyl)-1-isopropyl-1H-pyrazol-4-yl]pyridine

logP (pH2.7): 1.54
MS (ESI): 300.3 ([M+H]⁺)
¹H-NMR (400 MHz, d₆-DMSO): δ = 8.53 (s, 1H), 8.42 (d, 2H), 7.60 (m, 1H), 7.24 (t, 2H), 7.15 (d, 2H), 4.60 (m, 1H), 1.51 (d, 6H) ppm

### Herstellung von Verbindungen der Formel [XX] nach Verfahren (V18):

### 1-Cyclopropyl-3-(4-fluorphenyl)-1H-pyrazol [XX-1]

Eine Mischung aus 10g 3-(4-Fluorphenyl)-1H-pyrazol (62mmol), 10.59g Cyclopropylboronsäure (123mmol), 44mL Triethylamin (308mmol) und 40mL Pyridin (493mmol) in trockenem THF wird unter Rückfluss 18h erhitzt. Anschliessend wird die Reaktionsmischung abgekühlt, über Celite filtriert und eingeengt. Der Rückstand wird in Essigester aufgenommen, mit Na2CO3 Lösung gewaschen, getrocknet und im Vacuum eingeengt. Das Rohprodukt wird über Kieselgel chromatographiert und es werden 5g (40%) 1-Cyclopropyl-3-(4-fluorphenyl)-1H-pyrazol erhalten.
MS (ESI): 203.0 ([M+H]+)
1H-NMR (400MHz, CDCl3) δ = 7.76-7.73 (m, 2H) 7.435 (d, J=2.04Hz, 1H), 7.05 (t, J=8.6Hz, 2H), 6.44 (s, 1H), 3.64-3.58 (m, 1H), 1.24-1.14 (m, 2H), 1.06-1.01 (m, 2H) ppm

### Herstellung von Verbindungen der Formel [XXVII] nach Verfahren (V16):

### 4-[3-(2,6-Difluorphenyl)-1H-pyrazol-4-yl]pyridin [XXVII-1]

Eine Mischung aus 1-(2,6-Difluorphenyl)-3-(dimethylamino)-2-(pyridin-4-yl)prop-2-en-1-on (0.86 mmol), Hydrazinhydrat (1.3 mmol) und Triethylamin (1.3 mmol) in 5 ml Ethanol wird 15 min bei 120 °C in der Mikrowelle bestrahlt. Das Lösungsmittel wird im Vakuum evaporiert, der Rückstand in Dichlormethan (DCM) aufgenommen und die Suspension filtriert. Der Feststoff wird im Vacuumofen getrocknet bei 50 °C getrocknet. Man erhält 0.18 g (76% Ausbeute) 4-[3-(2,6-Difluorphenyl)-1 H-pyrazol-4-yl]pyridin.
logP (pH2.7): 1.54
MS (ESI): 258.1 ([M+H]⁺)
¹H-NMR (400 MHz, d₆-DMSO): δ = 13.59 (bs, 1H), 8.46 (m, 3H), 7.60(m, 1H), 7.29 (m, 2H), 7.18 (m, 2H) ppm

### Herstellung von Verbindungen der Formel [XXVI] nach Verfahren (V15):

### 1-(2,6-Difluorphenyl)-3-(dimethylamino)-2-(pyridin-4-yl)prop-2-en-1-on [XXVI-1]

Zu einer Suspension aus 4-(2,6-Difluorophenyl)-2-pyrid-4-ylethanon (17.7 mmol) in 20 ml N,N-Dimethylformamid (DMF) wird mit N,N-Dimethylformamid-dimethylacetal (60.3 mmol) versetzt und 3 h unter Rückfluss erhitzt. Nach dem Abkühlen auf Raumtemperatur wird das Lösungsmittel im Vakuum evaporiert, der Rückstand in Essigsäureethylester aufgenommen und die wässrige Phase dreimal mit EE extrahiert. Die gesammelten Extrakte werden über MgSO₄ getrocknet und im Vakuum eingeengt. Der Rückstand wird über Kieselgel chromatographiert (Gradient Heptan/EE 2:1 bis 0:1). Man erhält 3.1 g (58%) 1-(2,6-Difluorphenyl)-3-(dimethylamino)-2-(pyridin-4-yl)prop-2-en-1-on.
logP (pH2.7): 0.60
MS (ESI): 289.2 ([M+H]⁺)

### Herstellung von Verbindungen der Formel [XXV] nach Verfahren (V14):

### 1-(2,6-Difluorphenyl)-2-(pyridin-4-yl)ethanon [XXV-1]

Eine Lösung aus 4-Methylpyridin (24.6 mmol) und 2,6-Difluorbenzoesaureethylester (27.1mmol) in 58 ml wasserfreiem Tetrahydrofuran (THF) wird auf 0 °C gekühlt und tropfenweise mit 24.6 ml Lithiumbistrimethylsilylamid (LiHMDS, 1 molareLösung in Hexan) versetzt. Nach 3 Stunden bei 5-10 °C wird Wasser zugegeben und mit Essigsäureethylester (Essigsäureethylester) extrahiert. Die organische Phase wird mit gesättigter Natriumchlorid- Lösung (gesättigte NaCl) gewaschen, über Magnesiumsulfat (MgSO₄) getrocknet und im Vakuum eingeengt. Der Rückstand wird über Kieselgel chromatographiert (Gradient Heptan/EE 3:1 bis 1:1). Man erhält 4.1 g (54% Ausbeute) 4-(2,6-difluorophenyl)-2-pyrid-4-ylethanon.
logP (pH2.7): 0.62
MS (ESI): 234.1 ([M+H]⁺)
¹H-NMR (400 MHz, d₆-DMSO): δ = 8.53 (d, 2H), 7.65 (m, 1H), 7.27 (m, 4H), 4.34 (s, 2H) ppm

### Herstellung von Verbindungen der Formel [XXVIII] nach Verfahren (V19):

### 4-[5-Brom-3-(4-fluorphenyl)-1H-pyrazol-4-yl]pyridin [XXVIII-1]

Eine Lösung aus 4-[3-(4-Fluorphenyl)-1H-pyrazol-4-yl]pyridin (0.58 mmol) in 2 ml of *N*,*N-*dimethylformamid und N-Bromsuccinimid (0.58 mmol) wird 2 h auf 80 °C erhitzt. Nach dem Abkühlen auf Raumtemperatur wird mit Wasser und Essigsäureethylester versetzt. Die organische Phase wird mit Wasser gewaschen, über MgSO₄ getrocknet und nach dem Filtrieren im Vakuum eingeengt. Der Rückstand wird in Diisopropylether suspendiert, filtriert und im Vacuumofen bei 50 °C getrocknet. Man erhielt 0.13 g (64% Ausbeute) 4-[5-Brom-3-(4-fluorphenyl)-1H-pyrazol-4-yl]pyridin.
logP (pH2.7): 0.92
MS (ESI): 318.0 ([M+H]⁺)
¹H-NMR (400 MHz, d₆-DMSO): δ = 13.88 (bs, 1H), 8.60 (d, 2H), 7.38 (m, 2H), 7.29 (m, 4H) ppm

### Herstellung von Verbindungen der Formel [IX-b] nach Verfahren (V20):

### 4-[3-(4-Fluorphenyl)-5-methyl-1H-pyrazol-4-yl]pyridin [IX-b-1]

Unter Argon wird zu einer Lösung aus Natriumhydrogencarbonat (NaHCO₃, 2.1 mmol) und Dichlor[1.1'-ferrocenylbis(diphenylphosphan)]palladium(II)dichloromethan (0.03mmol) eine entgaste Lösung aus 4-[5-Brom-3-(4-fluorphenyl)-1-(4-methoxybenzyl)-1H-pyrazol-4-yl]pyridin und 4-[3-Brom-5-(4-fluorphenyl)-1-(4-methoxybenzyl)-1H-pyrazol-4-yl]pyridin (Mischung zweier Regioisomere, 1:1, 0.68 mmol) in 10.5 ml Dimethoxyethan und 3 ml Wasser gegeben. Es folgt die Zugabe einer 50%-igen Lösung von Trimethylboroxin (1,36 mmol) in THF. Das Gemisch wird 3 h auf 90 °C erhitzt, auf Raumtemperatur abgekühlt und mit Wasser und Essigsäureethylester versetzt. Die wässrige Lösung wird mit Essigsäureethylester extrahiert, die organische Phase mit gesättigter wässriger NaCl-Lösung gewaschen, über MgSO₄ getrocknet und im Vakuum eingeengt.

Abspaltung des N-Substituenten am Pyrazol: Der Rückstand wird in 3 ml Trifluoressigsäure (TFA) aufgenommen und 2 h bei 65 °C gerührt. Nach Zugabe von Wasser und Essigsäureethylester wird die organische Phase mit Essigsäureethylester extrahiert, mit gesättigter wässriger NaCl-Lösung gewaschen, über MgSO₄ getrocknet und im Vakuum eingeengt.

Der Rückstand wird an Kieselgel chromatographiert (Gradient DCM/Methanol (MeOH) 20:1 bis 10:1). Man erhält 88 mg (43%Ausbeute) 4-[3-(4-Fluorphenyl)-5-methyl-1H-pyrazol-4-yl]pyridin.
logP (pH2.7): 0.60
MS (ESI): 254.1 ([M+H]⁺)
¹H-NMR (400 MHz, d₆-DMSO): δ = 13.03 (s, 1H), 8.52 (d, 2H), 7.35 (m, 2H), 7.16 (m, 4H), 2.29 (s, 3H) ppm

### 4-[3-(4-Fluorphenyl)-5-cyclopropyl-1H-pyrazol-4-yl]pyridin [IX-b-2]

Unter Argon wird zu einer Lösung aus Natriumhydrogencarbonat (NaHCO₃, 2.1 mmol), Dichlor[1.1'-ferrocenylbis(diphenylphosphan)]palladium(II)dichloromethan (0.03 mmol) und Cyclopropylboronsäure (1,63 mmol) eine entgaste Lösung aus 4-[5-Brom-3-(4-fluorphenyl)-1-(4-methoxybenzyl)-1H-pyrazol-4-yl]pyridin und 4-[3-Brom-5-(4-fluorphenyl)-1-(4-methoxybenzyl)-1H-pyrazol-4-yl]pyridin (Mischung zweier Regioisomere, 1:1, 0.68 mmol) in 10.5 ml Dimethoxyethan und 3 ml Wasser gegeben. Das Gemisch wird 3 h auf 90 °C und 16 h auf 65 °C erhitzt, auf Raumtemperatur abgekühlt und mit Wasser und Essigsäureethylester versetzt. Die wässrige Lösung wird mit Essigsäureethylester extrahiert, die organische Phase mit ges. NaCl gewaschen, über MgSO₄ getrocknet und im Vakuum eingeengt.

Abspaltung des N-Substituenten am Pyrazol: Der Rückstand wird in 3 ml Trifluoressigsäure (TFA) aufgenommen und 2 h bei 65 °C gerührt. Nach Zugabe von Wasser und EE wird die organische Phase mit Essigsäureethylester extrahiert, mit ges. NaCl gewaschen, über MgSO₄ getrocknet und im Vakuum eingeengt.

Der Rückstand wird an Kieselgel chromatographiert (Gradient DCM/Methanol (MeOH) 20:1 bis 10:1). Man erhielt 75.8 mg (38% Ausbeute) 4-[3-(4-Fluorphenyl)-5-cyclopropyl-1H-pyrazol-4-yl]pyridin.
logP (pH2.7): 0.920
MS (ESI): 280 ([M+H]⁺)

### Herstellung von Verbindungen der Formel [XXXII] nach Verfahren (V22):

### 5-(4-Fluorphenyl)-2-methyl-2,4-dihydro-3H-pyrazol-3-on [XXXII-1]

Zu einer Lösung von 8.00g Methyl-3-(4-fluorphenyl)-3-oxopropanoat (40.8mmol) in 45mL Ethylacetat werden langsam 2.43g (53.0mmol) Methylhydrazin zugegeben. Anschliessend wird die Reaktionsmischung unter Rückfluss bis zur kompletten Umsetzung des Startmaterials erhitzt. Nach Abkühlen auf Raumtemperatur wird die Reaktionsmischung mit Diethylether und Wasser versetzt. Der gebildete Niederschlag wird abgesaugt, mit einer Petrolether/Diethylether Mischung (60mL, 1:1) gewaschen und getrocknet. Man erhält 5.33 g (68%) 5-(4-Fluorphenyl)-2-methyl-2,4-dihydro-3H-pyrazol-3-on.
¹H-NMR (400 MHz, CDCl₃): δ = 7.69-7.63 (m, 2 H); 7.12 (t, 2 H); 3.59 (s, 2 H), 3.41 (s, 3H) ppm

### Herstellung von Verbindungen der Formel [XXXIII] nach Verfahren (V23):

### 5-(Difluormethoxy)-3-(4-fluorphenyl)-1-methyl-1H-pyrazol [XXXIII-1]

Zu einer Lösung von 4.40g (22.9 mmol) 5-(4-Fluorphenyl)-2-methyl-2,4-dihydro-3H-pyrazol-3-on in wasserfreiem Acetonitril (100 mL) werden 3.16g (22.9mmol) K₂CO₃ und 4.19g (27.5 mmol) Natriumchlordifluoracetat gegeben und die Mischung wird 5h in einer N₂-Athmosphäre unter Rückfluss erhitzt. Nach Abkühlen der Reaktionsmischung wird wässrige NH4Cl Lösung (85mL) zugegeben und die organische Phase mit Essigester extrahiert (3x50 mL). Die vereinigten organischen Extrakte werden mit NaCl Lösung gewaschen, getrocknet und im Vacuum eingeengt. Das Rohprodukt wird durch Chromatografie an Kieselgel (Eluent Petrolether/Essigester 8/2) gereinigt. Es werden 1.26g (23%) 5-(Difluormethoxy)-3-(4-fluorphenyl)-1-methyl-1H-pyrazol erhalten.
¹H-NMR (400 MHz, CDCl₃): δ = 7.74-7.68 (m, 2 H); 7.08 (t, 2 H); 6.56 (t, 2JHF = 72.2 Hz, 1 H, CHF2); 6.14 (s, 1H); 3.78 (s, 3 H) ppm

### Herstellung von Verbindungen der Formel [VI-b] nach Verfahren (V24):

### 4-Brom-5-(difluormethoxy)-3-(4-fluorphenyl)-1-methyl-1H-pyrazol [VI-b-1]

Zu einer Lösung von 1.16g (7.28mmol) 5-(Difluormethoxy)-3-(4-fluorphenyl)-1-methyl-1H-pyrazol in Dichlormethan (14mL) werden tropfenweise 0.914g (5.72mmol) Brom zugegeben. Die Reaktionsmischung wird 26h bei Raumtemperatur gerührt. Anschliessend wird das Reaktionsgemisch mit Na₂S₂O₃-Lösung (3x 20mL) und mit NaHCO3-Lösung (3x30mL) gewaschen. Die organische Phase wird getrocknet und im Vacuum eingeengt. Es werden 1.34g (80%) 4-Brom-5-(difluormethoxy)-3-(4-fluorphenyl)-1-methyl-1H-pyrazol erhalten.
logP (pH2.7): 3.52
MS(ESI):321.1 ([M+H]⁺)
¹H-NMR (400 MHz, d6-DMSO): δ = 7.86-7.81 (m, 2H), 7.50-7.15 (m, 3H), 3.80 (s, 3H) ppm

Analog obigem Beispiel können ebenfalls die folgenden Verbindungen der Formel [VI-b] erhalten werden:

### 4-Brom-5-(difluormethoxy)-3-(4-fluorphenyl)-1-isopropyl-1H-pyrazol [VI-b-2]

logP (pH2.7): 4.61
MS (ESI): 351.0 ([M+H]⁺)
¹H-NMR (400 MHz, d6-DMSO): δ = 7.87-7.82 (m, 2H), 7.52-7.16 (m, 3H), 4.64-4.57 (m, 1H), 1.42 (d, 6H) ppm

### 4-Brom-5-(difluormethoxy)-3-(4-fluorphenyl)-1-isobutyl-1H-pyrazol [VI-b-3]

logP (pH2.7): 4.91
MS (ESI): 365.0 ([M+H]⁺)
¹H-NMR (400 MHz, d6-DMSO): δ = 7.87-7.83 (m, 2H), 7.51-7.16 (m, 3H), 3.90 (d, 2H), 2.22-2.15 (m, 6H) ppm

### 4-Brom-5-(difluormethoxy)-3-(4-fluor-2-methoxyphenyl)-1-methyl-1H-pyrazol [VI-b-4]

logP (pH2.7): 3.15
MS (ESI): 353.0 ([M+H]⁺)
¹H-NMR (400 MHz, d6-DMSO): δ = 7.50-7.13 (m, 3H), 7.05-7.02 (m, 1H), 6.86-6.81 (m, 1H) 3.80 (s, 3H), 3.78 (s, 3H) ppm

### Herstellung von Verbindungen der Formel [VI-c] nach Verfahren (V25):

### 2-[4-Brom-5-(difluormethoxy)-1-methyl-1H-pyrazol-3-yl]-5-fluorphenol [VI-c-1]

Zu einer Lösung von 3.0g (8.6mmol) 4-Brom-5-(difluormethoxy)-3-(4-fluor-2-methoxyphenyl)-1-methyl-1H-pyrazol in Dichlormethan (68mL) werden tropfenweise 5.8mL BBr₃ (1M Lösung in Dichlormethan, 5.8mmol) bei 0°C zugegeben. Die Reaktionsmischung wird langsam auf Raumtemperatur erwärmt und 23h gerührt. Anschliessend werden 150mL Diethylether zugegeben und die erhaltene Mischung wird zwischen gesättigter NaHCO₃-Lösung (100mL) und Essigester (200mL) verteilt. Der erhaltene Niederschlag wird durch Zugabe von 100mL Wasser gelöst und die Phasen getrennt. Die wässrige Phase wird mit Essigester extrahiert (3x 200mL). Die vereinigten organischen Extrakte werden mit Wasser, gesättigter NaCl Lösung gewaschen und getrocknet. Nach Entfernen des Lösungsmittels im Vacuum wird das erhaltene Rohprodukt durch Chromatografie an Kieselgel (Eluent Petrolether/Essigester 95/5) gereinigt. Es werden 1.6g (55%) 2-[4-Brom-5-(difluormethoxy)-1-methyl-1H-pyrazol-3-yl]-5-fluorphenol erhalten.
logP (pH2.7): 3.48
MS (ESI): 336.9 ([M+H]⁺)
1H-NMR (400 MHz, d6-DMSO): δ = 7.49-7.13 (m, 2H), 6.74-6.68 (m, 2H), 3.78 (s, 3H) ppm

### Herstellung von Ausgangsstoffen der Formel [XXXVII] nach Verfahren V14:

### 1-(4-Fluorphenyl)-2-[2-(methylsulfanyl)pyrimidin-4-yl]ethanon [XXXVII-1]

Eine Lösung aus 4-Methyl-2-(methylsulfanyl)pyrimidin (1eq, 41mmol) und Ethyl-4-fluorbenzoat (1.1eq, 45mmol) in 50 mL wasserfreiem Tetrahydrofuran (THF) wird auf 0°C gekühlt und tropfenweise mit Lithiumbistrimethylsilylamid (2eq, 82mmol, 1 molare Lösung von LiHMDS in n-Hexan) versetzt. Nach 3 Stunden bei 5-10 °C wird Wasser zugegeben und mit Essigsäureethylester (Essigsäureethylester) extrahiert. Die organische Phase wird mit gesättigter Natriumchlorid- Lösung (NaCl) gewaschen, über Magnesiumsulfat (MgSO₄) getrocknet und im Vakuum eingeengt. Der Rückstand wird durch Kristallisation aus 100mL Cyclohexan gereinigt und im Vacuum getrocknet. Man erhält 8.9 g (83% Ausbeute) 1-(4-Fluorphenyl)-2-[2-(methylsulfanyl)pyrimidin-4-yl]ethanon.

### Herstellung von Ausgangsstoffen der Formel [XXXVIII] nach Verfahren V15:

### 3-(Dimethylamino)-1-(4-fluorphenyl)-2-[2-(methylsulfanyl)pyrimidin-4-yl]prop-2-en-1-on [XXXVIII-1]

Eine Lösung von 1-(4-Fluorphenyl)-2-[2-(methylsulfanyl)pyrimidin-4-yl]ethanon (1eq, 30mmol) in 40mL N,N-Dimethylformamid-dimethylacetal wird 1h bei 75-80°C erhitzt. Nach dem Abkühlen auf Raumtemperatur wird das Lösungsmittel im Vakuum entfernt und der Rückstand durch Chromatografie an Kieselgel (Gradient Heptan/Ethylacetat 1:1 auf 2:8) gereinigt. Man erhält 9.3 g (97%) (2Z)-3-(Dimethylamino)-1-(4-fluorphenyl)-2-[2-(methylsulfanyl)pyrimidin-4-yl]prop-2-en-1-on.

### Herstellung von Ausgangsstoffen der Formel [XXXIX] nach Verfahren V16:

### 4-[3-(4-Fluorphenyl)-1H-pyrazol-4-yl]-2-(methylsulfanyl)pyrimidin [XXXIX-1]

Eine Mischung aus 3-(Dimethylamino)-1-(4-fluorphenyl)-2-[2-(methylsulfanyl)pyrimidin-4-yl]prop-2-en-1-on (1eq, 29 mmol), Hydrazinhydrat (1.5eq, 44 mmol) und Triethylamin (1.5eq, 44 mmol) in 186 mL Ethanol wird 3h unter Rückfluss erhitzt. Das Lösungsmittel wird im Vakuum evaporiert, Wasser wird hinzugegeben und mit Ethylacetat extrahiert. Die organische Phase wird abgetrennt, getrocknet (MgSO₄) und im Vacuum eingeengt. Man erhält 7.9 g (94% Ausbeute) 4-[3-(4-Fluorphenyl)-1H-pyrazol-4-yl]-2-(methylsulfanyl)pyrimidin.
logP (pH2.7): 2.28
MS (ESI): 287.0 ([M+H]⁺)
¹H-NMR (400 MHz, d₆-DMSO): δ = 13.48 (bs, 1H), 8.44 (d, 1H), 8.38 (bs, 1H), 7.56 (m, 2H), 7.27 (t, 2H), 7.12 (d, 1H), 2.21 (s, 3H) ppm

### Herstellung von Ausgangsstoffen der Formel [XL] nach Verfahren V13:

### 4-[3-(4-Fluorphenyl)-1-isopropyl-1H-pyrazol-4-yl]-2-(methylsulfanyl)pyrimidin [XL-1]

Zu einer Lösung von 4-[3-(4-Fluorphenyl)-1H-pyrazol-4-yl]-2-(methylsulfanyl)pyrimidin (1eq, 29.3mmol) in 75mL *N,N*-Dimethylformamid werden Cs₂CO₃ (2.5eq, 73.3 mmol) und 2-Iodpropan (1.5 eq, 44 mmol) zugegeben und die Reaktionsmischung wird über Nacht bei Raumtemperatur gerührt. Danach wird die Mischung mit Wasser versetzt und mit Essigester extrahiert. Die organische Phase wird getrocknet, eingedampft und durch Chromatografie an Kieselgel (Gradient Dichlormethan/Ethylacetat 20:1 auf 5:1) gereinigt. Es werden 6.5 g (64%) 4-[3-(4-Fluorphenyl)-1-isopropyl-1H-pyrazol-4-yl]-2-(methylsulfanyl)pyrimidin erhalten.
logP (pH2.7): 3.62
MS (ESI): 329.0 ([M+H]⁺)
¹H-NMR (400 MHz, CDCl₃): δ = 8.28 (d, 1H), 8.10 (s, 1H), 7.52 (m, 2H), 7.11 (t, 2H), 6.73 (d, 1H), 4.57 (m, 1H), 2.50 (s, 3H), 1.60 (d, 6H) ppm

### Herstellung von Ausgangsstoffen der Formel [XLI] nach Verfahren V27:

### 4-[3-(4-Fluorphenyl)-1-isopropyl-1H-pyrazol-4-yl]-2-(methylsulfonyl)pyrimidin [XLI-1]

Eine Lösung von 4-[3-(4-Fluorphenyl)-1-isopropyl-1H-pyrazol-4-yl]-2-(methylsulfanyl)pyrimidin (1 eq, 19.7 mmol) und m-Chlorperbenzoesäure (2eq, 40 mmol, 70%) in 520 mL Dichlormethan wird über Nacht bei Raumtemperatur gerührt. Danach wird die Reaktionsmischung mit Wasser und Natriumsulfit (2.1eq, 41.5mmol) versetzt und die Phasen getrennt. Die organische Phase wird 2x mit einer 2M K₂CO₃ Lösung gewaschen, getrocknet und im Vakuum eingeengt. Es werden 6.3g (84%) 4-[3 -(4-Fluorphenyl)-1-isopropyl-1H-pyrazol-4-yl]-2-(methylsulfonyl)pyrimidin erhalten.
logP (pH2.7): 2.90
MS (ESI): 375.1 ([M+H]+)
¹H-NMR (400 MHz, d₆-DMSO): δ = 8.87 (d, 1H), 8.70 (s, 1H), 7.62 (d, 1H), 7.61 (m, 2H), 7.25 (t, 2H), 4.05 (d, 2H), 3.17 (s, 3H), 2.22 (m, 1H), 0.92 (d, 6H) ppm

### Herstellung von Verbindungen der Formel [I-f] nach Verfahren V28:

### N-Benzyl-4-[3-(4-fluorphenyl)-1-isopropyl-1H-pyrazol-4-yl]pyrimidin-2-amin [I-f-1]

Eine Mischung von 4-[3-(4-Fluorphenyl)-1-isopropyl-1H-pyrazol-4-yl]-2-(methylsulfonyl)pyrimidin (1eq, 9.15 mmol) in 43 mL Benzylamin wird 4h bei Raumtemperatur gerührt. Anschliessend wird das Benzylamin im Vakuum entfernt und das Rohprodukt durch Chromatografie an Kieselgel (Gradient Dichlormethane / Ethylacetat 20:1 auf 5:1) gereinigt. Man erhält 1.77 g (47%) N-Benzyl-4-[3-(4-fluorphenyl)-1-is opropyl-1 H-pyrazol-4-yl]pyrimidin-2-amin.
logP (pH2.7): 3.31
MS (ESI): 385.1 ([M+H]⁺)

### Herstellung von Ausgangsstoffen der Formel [III-a] nach Verfahren V29:

### 4-[3-(4-Fluorphenyl)-1-isopropyl-1H-pyrazol-4-yl]pyrimidin-2-amin [III-a-1]

Eine Lösung von N-Benzyl-4-[3-(4-fluorphenyl)-1-isopropyl-1H-pyrazol-4-yl]pyrimidin-2-amin (1eq, 5.21 mmol) in Schwefelsäure (100 eq, 521 mmol) wird über Nacht bei Raumtemperatur gerührt. Anschliessend wird die Reaktionsmischung zunächst mit Eis, dann mit Wasser versetzt und vorsichtig mit 30% NaOH bis zum pH=9 neutralisiert. Die wässrige Phase wird mehrmals mit Dichlormethan extrahiert. Die vereinigten organischen Extrakte werden getrocknet und im Vakuum eingeengt. Es werden 1.1 g (67%) 4-[3-(4-Fluorphenyl)-1-isopropyl-1H-pyrazol-4-yl]pyrimidin-2-amin erhalten.
logP (pH2.7): 1.54
MS (ESI): 298.2 ([M+H]+)
¹H-NMR (400 MHz, d₆-DMSO): δ = 8.23 (s, 1H), 8.08 (d, 1H), 7.55 (m, 2H), 7.23 (t, 2H), 6.47 (s, 1H)6.76 (d, 1H), 6.35 (d, 1H), 4.57 (m, 1H), 1.48 (d, 6H) ppm

### Herstellung von Verbindungen der Formel [I-g] nach Verfahren V4:

### N-{4-[3-(4-Fluorphenyl)-1-isopropyl-1H-pyrazol-4-yl]pyrimidin-2-yl}propanamid [I-g-1]

Zu einer Lösung von 4-[3-(4-Fluorphenyl)-1-isopropyl-1H-pyrazol-4-yl]pyrimidin-2-amin (4eq, 1.34mmol) und Triethylamin (4eq, 1.34mmol) in 6 mL Tetrahydrofuran wird Propionylchlorid (2eq, 0.67 mmol) gegeben. Die Reaktionsmischung wird bei Raumtemperatur über Nacht gerührt. Anschließend werden die flüchtigen Bestandteile im Vakuum entfernt und das Rohmaterial mit 6 mL NH₃ in Methanol (7 molar) versetzt. Die Mischung wird 2 h bei Raumtemperatur verrührt und anschließend eingedampft. Das Rohprodukt wird mit Wasser versetzt und 2x mit Dichlormethan extrahiert. Die organischen Extrakte werden getrocknet und im Vakuum eingeengt. Das Rohprodukt wird über Chromatographie an Kieselgel (Eluent Heptan / Essigsäureethylester) gereinigt. Man erhält 70 mg (59%) N-{4-[3-(4-Fluorphenyl)-1-isopropyl-1H-pyrazol-4-yl]pyrimidin-2-yl}propanamid.
logP (pH2.7): 2.35
MS (ESI): 354.2 ([M+H]⁺)
¹H-NMR (400 MHz, d₆-DMSO): δ = 10.27 (bs, 1H), 8.46 (d, 1H), 8.38 (s, 1H), 7.60 (m, 2H), 7.23 (t, 2H), 6.94 (m, 1H), 4.61 (m, 1H), 2.40 (q, 2H), 1.50 (d, 6H), 0.98 (t, 3H) ppm

### Herstellung von Ausgangsstoffen der Formel [XLIII] nach Verfahren V14:

### 2-(2-Chlorpyrimidin-4-yl)-1-(4-fluorphenyl)ethanon [XLIII-1]

Eine Lösung aus 2-Chlor-4-methylpyrimidin (1eq, 15.5mmol) und Ethyl-4-fluorbenzoat (1.1eq, 17.1mmol) in 17 mL wasserfreiem Tetrahydrofuran (THF) wird auf 0°C gekühlt und tropfenweise mit Lithiumbistrimethylsilylamid (2eq, 31mmol, 1 molare Lösung von LiHMDS in n-Hexan) versetzt. Nach 3 Stunden bei 5-10 °C wird Wasser zugegeben und mit Dichlormethan extrahiert. Die organische Phase wird mit gesättigter Natriumchlorid- Lösung (NaCl) gewaschen, über Magnesiumsulfat (MgSO₄) getrocknet und im Vakuum eingeengt. Man erhält 3.8 g (83% Ausbeute) 2-(2-Chlorpyrimidin-4-yl)-1-(4-fluorphenyl)ethanon (Mischung aus Keto- und Enolform 4/9).
logP (pH2.7): 2.27
MS (ESI): 251.0 ([M+H]⁺)
¹H-NMR (400 MHz, d₆-DMSO): δ = 13.58 (s, 1H, enol), 8.75 (d, 1H), 8.57 (bs, 1H, enol), 8.11 (m, 2H), 7.92 (m, 2H, enol), 7.60 (d, 1H), 7.41 (m, 2H), 7.34 (m, 3H, enol), 6.51 (bs, 1H, enol), 4.70 (s, 2H) ppm

### Herstellung von Ausgangsstoffen der Formel [XLIV] nach Verfahren V28:

### N-Benzyl-4-[3-(4-fluorphenyl)-1-isopropyl-1H-pyrazol-4-yl]pyrimidin-2-amin [XLIV-1]

Eine Mischung von 2-(2-Chlorpyrimidin-4-yl)-1-(4-fluorphenyl)ethanon (1eq, 16 mmol) in 27 mL Isopropylamin wird 10min bei 110°C im Mikrowellenofen erhitzt (CEM Explorer). Anschliessend wird das Amin im Vakuum entfernt und das Rohprodukt mit Dichlormethan (25mL) und 1M HCl (7mL) versetzt. Die Lösung wird über Nacht bei Raumtemperatur gerührt und und anschliessend mit 1M NaOH neutralisiert. Die Phasen werden getrennt und die wässrige Phase wird 2x mit Dichlormethan extrahiert. Die vereinigten organischen Extrakte werden getrocknet (MgSO₄) und im Vakuum eingeengt. Man erhält 4.0 g (77%) 1-(4-Fluorphenyl)-2-[2-(isopropylamino)pyrimidin-4-yl]ethanon.
logP (pH2.7): 2.05
MS (ESI): 274.2 ([M+H]⁺)

### Herstellung von Ausgangsstoffen der Formel [XLV] nach Verfahren V15:

### 3-(Dimethylamino)-1-(4-fluorphenyl)-2-[2-(isopropylamino)pyrimidin-4-yl]prop-2-en-1-on [XLV-1]

Eine Lösung von 1-(4-Fluorphenyl)-2-[2-(isopropylamino)pyrimidin-4-yl]ethanon (1eq, 14.6mmol) in einer Mischung von 6.5mL N,N-Dimethylformamid und 6.5mL N,N-Dimethylformamid-dimethylacetal wird 2.5h bei 100°C erhitzt. Nach dem Abkühlen auf Raumtemperatur wird das Lösungsmittel im Vakuum entfernt. Man erhält 5.4 g (65%) 3-(Dimethylamino)-1-(4-fluorphenyl)-2-[2-(isopropylamino)pyrimidin-4-yl]prop-2-en-1 -on.
logP (pH2.7): 1.45
MS (ESI): 329.2 ([M+H]⁺)

### Herstellung von Ausgangsstoffen der Formel [XLVI] nach Verfahren V16:

### 4-[3-(4-Fluorphenyl)-1H-pyrazol-4-yl]-N-isopropylpyrimidin-2-amin [XLVI-1]

Eine Mischung aus 3-(Dimethylamino)-1-(4-fluorphenyl)-2-[2-(isopropylamino)pyrimidin-4-yl]prop-2-en-1-on (1eq, 16.4 mmol), Hydrazinhydrat (1.1eq, 18 mmol) in 100 mL Ethanol wird 16h bei Raumtemperatur gerührt. Das Lösungsmittel wird im Vakuum evaporiert und der Rückstand durch Chromatografie an Kieselgel (Gradient Heptan/Ethylacetat 1:0 auf 6:4) gereinigt. Man erhält 3.5 g (65% Ausbeute) 4-[3-(4-Fluorphenyl)-1H-pyrazol-4-yl]-N-isopropylpyrimidin-2-amin.
logP (pH2.7): 1.37
MS (ESI): 298.2 ([M+H]⁺)
¹H-NMR (400 MHz, d₆-DMSO): δ = 13.28 (bs, 1H), 8.12 (d, 1H), 7.59(bs, 2H), 7.28 (bs, 2H), 6.77 (d, 1H), 6.52 (bs, 1H), 3.81 (bs, 1H), 1.09 (bs, 6H) ppm.

### Herstellung von Verbindungen der Formel [I-h] nach Verfahren V13:

### 4-[3-(4-Fluorphenyl)-1-isobutyl-1H-pyrazol-4-yl]-N-isopropylpyrimidin-2-amin [I-h-1]

Zu einer Lösung von 4-[3-(4-Fluorphenyl)-1H-pyrazol-4-yl]-N-isopropylpyrimidin-2-amin (1eq, 0.82 mmol) in 8mL *N,N*-Dimethylformamid werden Cs₂CO₃ (1.1eq, 0.9 mmol) und 2-Iodpropan (1.5 eq, 1.23 mmol) zugegeben und die Reaktionsmischung wird über Nacht bei Raumtemperatur gerührt und anschliessend 4h bei 80°C erhitzt. Danach wird das Lösungsmittel entfernt, das Rohprodukt mit Wasser versetzt und 3x mit Dichlormethan extrahiert. Die organische Phase wird getrocknet, eingeengt und durch Chromatografie an Kieselgel (Gradient Heptan/Ethylacetat 1:0 auf 1:1) gereinigt. Es werden 124 mg (40%) 4-[3-(4-Fluorphenyl)-1-isobutyl-1H-pyrazol-4-yl]-N-isopropylpyrimidin-2-amin erhalten.
logP (pH2.7): 1.81
MS (ESI): 312.2 ([M+H]⁺)
¹H-NMR (400 MHz, CDCl₃): δ = 8.01 (d, 1H), 7.89 (s, 1H), 7.45 (m, 2H), 7.02 (t, 2H), 6.36 (d, 1H), 4.94 (bs, 2H), 3.89 (d, 2H), 2.23 (m, 1H), 0.91 (d, 6H) ppm

Nach den zuvor angegebenen Methoden werden auch die in den nachstehenden Tabellen I-III genannten Verbindungen der Formel **[I-a]** und **[I-b]** erhalten. 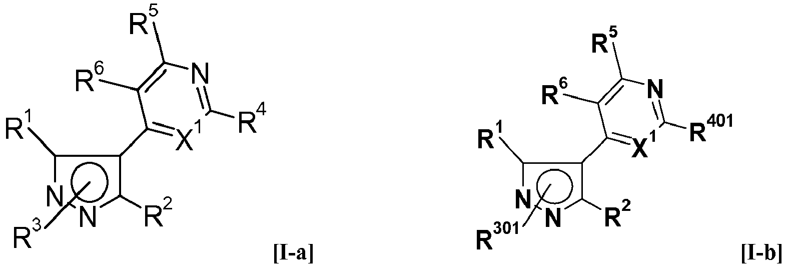 **Tabelle** 1

**Tabelle 1**

| Bsp | X¹ | R¹ | R² | R^{3/301} | R^{4/401} | R⁵ | R⁶ |
|---|---|---|---|---|---|---|---|
| 1 | CH | 4-Fluorphenyl | H | 2-Methylpropyl | H | H | H |
| 2 | CH | 2,6-Difluorphenyl | H | 2-Methylpropyl | H | H | H |
| 3 | CH | 2,6-Difluorphenyl | H | Butan-2-yl | H | H | H |
| 4^{[s]} | CH | 4-Fluorphenyl | H | H | H | H | H |
| 5 | N | 3-Chlorphenyl | H | 2-Methylpropyl | H | H | H |
| 6 | CH | 2,6-Difluorphenyl | H | 2,2-Dimethylpropyl | H | H | H |
| 7 | CH | 2,6-Difluorphenyl | H | 3-Methylbut-2-en-1-yl | H | H | H |
| 8 | CH | 2,6-Difluorphenyl | H | Cyclobutyl | H | H | H |
| 9 | CH | 4-Fluorphenyl | H | Tetrahydro-2H-pyran-2-yl | H | H | H |
| 10 | CH | 2,6-Difluorphenyl | H | Propan-2-yl | H | H | H |
| 11 | CH | 2,6-Difluorphenyl | H | 2,2-Dimethylpropyl | H | H | H |
| 12 | CH | 2,6-Difluorphenyl | H | 2,2,2-Trifluorethyl | H | H | H |
| 13 | CH | 2,6-Difluorphenyl | H | 2-Methylpropyl | H | H | H |
| 14 | CH | 2,6-Difluorphenyl | H | Propan-2-yl | H | H | H |
| 15 | CH | 2,6-Difluorphenyl | H | Cyclobutyl | H | H | H |
| 16 | CH | 2,6-Difluorphenyl | H | Cyclopentyl | H | H | H |
| 17 | CH | 2,6-Difluorphenyl | H | Cyclopentyl | H | H | H |
| 18^{[s]} | N | 4-Fluorphenyl | H | H | CH₃ | H | H |
| 19 | CH | 4-Fluorphenyl | H | Tetrahydro-2H-pyran-2-yl | Propanoylamino | H | H |
| 20 | CH | 4-Fluorphenyl | H | Tetrahydro-2H-pyran-2-yl | H | -NHCH=CH- | |
| 21 | CH | 4-Fluorphenyl | H | Tetrahydro-2H-pyran-2-yl | (2-Methylpropanoyl)amino | H | H |
| 22 | CH | 2,6-Difluorphenyl | H | 3-Methylbut-2-en-1-yl | H | H | H |
| 23 | CH | 4-Fluorphenyl | H | Tetrahydro-2H-pyran-2-yl | (Methoxyacetyl)amino | H | H |
| 24 | CH | 4-Fluorphenyl | H | 2-Methylpropyl | H | H | H |
| 25 | CH | 4-Fluorphenyl | H | 3-Methylbut-2-en-1-yl | H | H | H |
| 26 | CH | 2,6-Difluorphenyl | H | Tetrahydrofuran-2-ylmethyl | H | H | H |
| 27 | CH | 2,6-Difluorphenyl | H | Tetrahydrofuran-2-ylmethyl | H | H | H |
| 28 | CH | 3-Chlorphenyl | H | Tetrahydro-2H-pyran-2-yl | H | H | H |
| 29 | N | 4-Fluorphenyl | H | Tetrahydro-2H-pyran-2-yl | CH₃ | H | H |
| 30 | CH | 4-Fluorphenyl | H | 3-Methylbut-2-en-1-yl | H | H | H |
| 31 | CH | 4-Fluorphenyl | H | Tetrahydro-2H-pyran-2-yl | CH₃ | H | H |
| 32 | CH | 3-Chlorphenyl | H | Tetrahydro-2H-pyran-2-yl | H | -NHCH=CH- | |
| 33 | CH | 2,2-Difluor-1,3-benzodioxol-4-yl | H | Tetrahydro-2H-pyran-2-yl | H | H | H |
| 34 | N | 3-Chlorphenyl | H | 3-Methylbut-2-en-1-yl | H | H | H |
| 35 | CH | 4-Fluorphenyl | Brom | Tetrahydro-2H-pyran-2-yl | H | H | H |
| 36 | N | 3-(Trifluormethyl)phenyl | H | Tetrahydro-2H-pyran-2-yl | Morpholin-4-yl | H | H |
| 37 | CH | 4-Fluor-2-methylphenyl | H | Tetrahydro-2H-pyran-2-yl | H | H | H |
| 38 | CH | 2,6-Difluorphenyl | H | Butan-2-yl | H | H | H |
| 39 | CH | 4-Fluorphenyl | H | Tetrahydro-2H-pyran-2-yl | Amino | H | H |
| 40 | CH | 4-Fluorphenyl | H | Propan-2-yl | (Methoxyacetyl)amin o | H | H |
| 41 | CH | 4-Fluorphenyl | H | Tetrahydro-2H-pyran-2-yl | (2,2-Dimethylpropanoyl)amino | H | H |
| 42 | CH | 3-(Trifluormethyl)phenyl | H | Tetrahydro-2H-pyran-2-yl | H | H | H |
| 43 | CH | 4-Methoxyphenyl | H | Tetrahydro-2H-pyran-2-yl | H | H | H |
| 44 | CH | 4-Fluorphenyl | H | Propan-2-yl | H | (1Z,3Z)-buta-1,3-dien-1,4-diyl | |
| 45 | CH | 4-Methoxynaphthalen-1-yl | H | Tetrahydro-2H-pyran-2-yl | H | H | H |
| 46 | CH | 4-Fluorphenyl | H | Propan-2-yl | Amino | H | H |
| 47 | CH | 4-Fluorphenyl | H | Propan-2-yl | H | -(prop-2-yn-1-yl)N-CH=CH- | |
| 48 | CH | 3-(Trifluormethyl)phenyl | H | Tetrahydro-2H-pyran-2-yl | (2,2-Dimethylpropanoyl)amino | H | H |
| 49 | CH | 4-Fluorphenyl | H | Tetrahydro-2H-pyran-2-yl | H | -(prop-2-yn-1-yl)N-CH=CH- | |
| 50 | CH | 3-(Trifluormethyl)phenyl | H | Tetrahydro-2H-pyran-2-yl | H | -NHCH=CH- | |
| 51 | CH | 3-Chlor-4-fluorphenyl | H | Tetrahydro-2H-pyran-2-yl | H | H | H |
| 52 | N | 4-Fluorphenyl | H | Tetrahydro-2H-pyran-2-yl | Morpholin-4-yl | H | H |
| 53 | CH | 4-Fluorphenyl | H | Propan-2-yl | Methylcarbamoyl | H | H |
| 54 | CH | 4-Fluorphenyl | H | Tetrahydro-2H-pyran-2-yl | H | (1Z,3Z)-buta-1,3-dien-1,4-diyl | |
| 55 | CH | 4-Fluor-3-methylphenyl | H | Tetrahydro-2H-pyran-2-yl | H | H | H |
| 56 | CH | 2,4-Difluorphenyl | H | Tetrahydro-2H-pyran-2-yl | H | H | H |
| 57 | CH | 4-tert-Butylphenyl | H | Tetrahydro-2H-pyran-2-yl | H | H | H |
| 58 | CH | 4-Fluorphenyl | H | tert-Butyl | H | H | H |
| 59 | CH | 4-Phenoxyphenyl | H | Tetrahydro-2H-pyran-2-yl | H | H | H |
| 60 | CH | 3-Cyanphenyl | H | Tetrahydro-2H-pyran-2-yl | H | H | H |
| 61 | CH | 3-Cyan-4-fluorphenyl | H | Tetrahydro-2H-pyran-2-yl | H | H | H |
| 62 | N | 4-Methoxyphenyl | H | Tetrahydro-2H-pyran-2-yl | 1H-Pyrazol-1-yl | H | H |
| 63 | N | 4-Fluorphenyl | H | Tetrahydro-2H-pyran-2-yl | Ethylamino | H | H |
| 64 | CH | 4-Fluor-2-methoxyphenyl | H | Tetrahydro-2H-pyran-2-yl | H | H | H |
| 65 | N | 4-Methoxyphenyl | H | Tetrahydro-2H-pyran-2-yl | H | -NHCH=CH- | |
| 66 | N | 4-Fluorphenyl | H | Tetrahydro-2H-pyran-2-yl | H | -NHCH=CH- | |
| 67 | CH | 3-Phenoxyphenyl | H | Tetrahydro-2H-pyran-2-yl | H | H | H |
| 68 | CH | 4-(Methylsulfonyl)phenyl | H | Tetrahydro-2H-pyran-2-yl | H | H | H |
| 69 | CH | 4-Methoxyphenyl | H | Tetrahydro-2H-pyran-2-yl | Amino | H | H |
| 70 | CH | 4-Chlorphenyl | H | Tetrahydro-2H-pyran-2-yl | H | H | H |
| 71 | CH | 4-(Trifluormethoxy)phenyl | H | Tetrahydro-2H-pyran-2-yl | H | H | H |
| 72 | CH | 4-Fluorphenyl | H | Propan-2-yl | (2,2-Dimethylpropanoyl)amino | H | H |
| 73 | CH | 4-Methoxyphenyl | H | Tetrahydro-2H-pyran-2-yl | CH₃ | H | H |
| 74 | N | 4-Fluorphenyl | H | Tetrahydro-2H-pyran-2-yl | Methylsulfanyl | H | H |
| 75 | N | 4-Fluorphenyl | H | Tetrahydro-2H-pyran-2-yl | 1H-Pyrazol-1-yl | H | H |
| 76 | CH | 2,3-Dichlorphenyl | H | Tetrahydro-2H-pyran-2-yl | H | H | H |
| 77 | N | 4-Methoxyphenyl | H | Tetrahydro-2H-pyran-2-yl | CH₃ | H | H |
| 78 | N | 4-Methoxyphenyl | H | Tetrahydro-2H-pyran-2-yl | Ethylamino | H | H |
| 79 | CH | 4-Fluorphenyl | H | 5-(Trifluormethyl)pyridin-2-yl | H | H | H |
| 80 | CH | 4-Methoxyphenyl | H | Tetrahydro-2H-pyran-2-yl | H | -NHCH=CH- | |
| 81 | CH | 4-Fluorphenyl | H | Tetrahydro-2H-pyran-2-yl | H | -N(COCH₃)-CH=CH- | |
| 82 | CH | 3-(Trifluormethyl)phenyl | H | Tetrahydro-2H-pyran-2-yl | (2,2-Dimethylpropanoyl)amino | H | H |
| 83 | CH | 3-Chlorphenyl | H | Tetrahydro-2H-pyran-2-yl | Methylcarbamoyl | H | H |
| 84 | CH | 4-Fluorphenyl | H | Propan-2-yl | (2-Methylpropanoyl)amino | H | H |
| 85 | CH | 4-Fluorphenyl | H | Propan-2-yl | Propanoylamino | H | H |
| 86 | CH | 4-Fluorphenyl | H | Propan-2-yl | (Cyclopropylcarbonyl )amino | H | H |
| 87 | CH | 4-Fluorphenyl | H | 2-Methylpropyl | (2-Methylpropanoyl)amino | H | H |
| 88 | CH | 4-Fluorphenyl | H | 2-Methoxyethyl | H | H | H |
| 89 | CH | 4-Fluorphenyl | H | Propan-2-yl | H | H | H |
| 90 | CH | 4-Fluorphenyl | H | Tetrahydro-2H-pyran-2-yl | Methylcarbamoyl | H | H |
| 91 | CH | 4-Fluorphenyl | H | Butan-2-yl | H | H | H |
| 92 | CH | 4-Fluorphenyl | H | Cyclopentyl | H | H | H |
| 93 | CH | 4-Fluorphenyl | H | Tetrahydro-2H-pyran-2-yl | Carbamoyl | H | H |
| 94 | CH | 4-Fluorphenyl | H | Tetrahydro-2H-pyran-2-yl | Phenylcarbamoyl | H | H |
| 95 | CH | 3-Chlorphenyl | H | Tetrahydro-2H-pyran-2-yl | Carbamoyl | H | H |
| 96 | CH | 3-Chlorphenyl | H | Tetrahydro-2H-pyran-2-yl | Phenylcarbamoyl | H | H |
| 97 | CH | 4-Fluorphenyl | H | Propan-2-yl | Amino | H | H |
| 98 | CH | 2-(Benzyloxy)phenyl | H | Tetrahydro-2H-pyran-2-yl | H | H | H |
| 99 | CH | Chinolin-8-yl | H | Tetrahydro-2H-pyran-2-yl | H | H | H |
| 100 | CH | 4-Fluorphenyl | H | Pyridin-3-ylmethyl | H | H | H |
| 101 | CH | 4-Fluorphenyl | H | 1-(3-Chlorphenyl)ethyl | H | H | H |
| 102 | CH | 4-Fluorphenyl | H | 1-(2-Chlorphenyl)ethyl | H | H | H |
| 103 | CH | 4-Fluorphenyl | H | 2,2,2-Trifluorethyl | H | H | H |
| 104 | CH | 4-Fluorphenyl | H | 2,3-Difluorbenzyl | H | H | H |
| 105 | CH | 4-Fluorphenyl | H | 2-tert-Butoxy-2-oxoethyl | H | H | H |
| 106 | CH | 4-Fluorphenyl | H | 1-Methoxy-3-methyl-1-oxobutan-2-yl | H | H | H |
| 107 | CH | 4-Fluorphenyl | H | 1-Methoxy-1-oxopropan-2-yl | H | H | H |
| 108 | CH | 4-Fluorphenyl | H | 2-Chlor-6-fluorbenzyl | H | H | H |
| 109 | CH | 4-Fluorphenyl | H | 4-(Difluormethoxy)benzyl | H | H | H |
| 110 | CH | 4-Fluorphenyl | H | (2-Chlor-1,3-thiazol-5-yl)methyl | H | H | H |
| 111 | CH | 4-Fluorphenyl | H | 5-(Trifluormethyl)-1,3,4-thiadiazol-2-yl | H | H | H |
| 112 | CH | 4-Fluorphenyl | H | 6-(Trifluormethyl)pyrimidin-4-yl | H | H | H |
| 113 | CH | 4-Fluorphenyl | H | 2-Cyclohexyl-2-oxoethyl | H | H | H |
| 114 | CH | 4-Fluorphenyl | H | 2-Cyclopentyl-2-oxoethyl | H | H | H |
| 115 | CH | 4-Fluorphenyl | H | Pyridin-2-ylmethyl | H | H | H |
| 116 | CH | 4-Fluorphenyl | H | (2,2-Dichlorcyclopropyl)methyl | H | H | H |
| 117 | CH | 4-Fluorphenyl | H | 2-(Methylsulfanyl)ethyl | H | H | H |
| 118 | CH | 4-Fluorphenyl | H | 2-(3-Chlorphenyl)ethyl | H | H | H |
| 119 | CH | 4-Fluorphenyl | H | 2-(2-Chlorphenyl)ethyl | H | H | H |
| 120 | CH | 4-Fluorphenyl | H | 1-(4-Chlorphenyl)ethyl | H | H | H |
| 121 | CH | 4-Fluorphenyl | H | Pyridin-4-ylmethyl | H | H | H |
| 122 | CH | 4-Fluorphenyl | H | 2-(Trifluormethoxy)ethyl | H | H | H |
| 123 | CH | 4-Fluorphenyl | H | Tetrahydrofuran-2-ylmethyl | H | H | H |
| 124 | CH | 4-Fluorphenyl | H | 2,3-Dihydroxypropyl | H | H | H |
| 125 | CH | 4-Fluorphenyl | H | Biphenyl-3-ylmethyl | H | H | H |
| 126 | CH | 4-Fluorphenyl | H | Cyclopropylmethyl | H | H | H |
| 127 | CH | 4-Fluorphenyl | H | Benzyl | H | H | H |
| 128 | CH | 4-Fluorphenyl | H | 2-Fluorbenzyl | H | H | H |
| 129 | CH | 4-Fluorphenyl | H | 3-Fluorbenzyl | H | H | H |
| 130 | CH | 4-Fluorphenyl | H | 4-Fluorbenzyl | H | H | H |
| 131 | CH | 4-Fluorphenyl | H | Biphenyl-4-ylmethyl | H | H | H |
| 132 | CH | 4-Fluorphenyl | H | 3-Ethoxy-3-oxopropyl | H | H | H |
| 133 | CH | 4-Fluorphenyl | H | 2-[2-(2-Methoxyethoxy)ethoxy] ethyl | H | H | H |
| 134 | CH | 4-Fluorphenyl | H | 2-(2-Methoxyethoxy)ethyl | H | H | H |
| 135 | CH | 4-Fluorphenyl | H | Biphenyl-2-ylmethyl | H | H | H |
| 136 | CH | 4-Fluorphenyl | H | 2-Cyanbenzyl | H | H | H |
| 137 | CH | 4-Fluorphenyl | H | Naphthalen-1-ylmethyl | H | H | H |
| 138 | CH | 4-Fluorphenyl | H | 3-Phenoxybenzyl | H | H | H |
| 139 | CH | 4-Fluorphenyl | H | 4-Fluor-3-phenoxybenzyl | H | H | H |
| 140 | CH | 4-Fluorphenyl | H | 3-Cyanbenzyl | H | H | H |
| 141 | CH | 4-Fluorphenyl | H | 4-Cyanbenzyl | H | H | H |
| 142 | CH | 4-Fluorphenyl | H | 2-Oxotetrahydrofuran-3-yl | H | H | H |
| 143 | CH | 4-Fluorphenyl | H | 1H-Imidazol-2-ylmethyl | H | H | H |
| 144 | CH | 4-Fluorphenyl | H | 2-(1,5-Dimethyl-1H-pyrazol-3-yl)-2-oxoethyl | H | H | H |
| 145 | CH | 4-Fluorphenyl | H | 2-(Acetyloxy)ethyl | H | H | H |
| 146 | CH | 4-Fluorphenyl | H | 2-Cyanethyl | H | H | H |
| 147 | CH | 4-Fluorphenyl | H | 2-Methylpropyl | H | -NHCOCH₂- | |
| 148 | CH | 4-Fluorphenyl | H | 2-Methylpropyl | H | -N(CH₃)COC(CH₃)₂- | |
| 149 | N | 4-Fluorphenyl | H | 2-Methylpropyl | Prop-2-en-1-ylamino | H | H |
| 150 | N | 4-Fluorphenyl | H | 2-Methylpropyl | Prop-2-en-1-ylamino | H | H |
| 151^{[s]} | CH | 4-Methoxyphenyl | H | H | H | H | H |
| 152^{[s]} | CH | 3-(Trifluormethyl)phenyl | H | H | H | H | H |
| 153 | CH | 4-Fluorphenyl | H | 2-Methoxyethyl | Propanoylamino | H | H |
| 154 | CH | 3-Cyan-4-fluorphenyl | H | 2-Methylpropyl | H | H | H |
| 155 | CH | 4-Fluorphenyl | H | 2-Methylpropyl | Propanoylamino | H | H |
| 156 | CH | 4-Fluorphenyl | H | Propan-2-yl | (Cyclobutylcarbonyl)amino | H | H |
| 157 | CH | 4-Fluorphenyl | H | Butan-2-yl | (2-Methylpropanoyl)amino | H | H |
| 158 | CH | 4-Fluorphenyl | H | 2-Methylpropyl | (Cyclopropylacetyl)amino | H | H |
| 159 | CH | 4-Fluorphenyl | H | Butan-2-yl | (Cyclopropylacetyl)amino | H | H |
| 160 | CH | 4-Fluorphenyl | H | Propan-2-yl | (3-Methylbutanoyl)amino | H | H |
| 161 | CH | 4-Fluorphenyl | H | Propan-2-yl | Acetylamino | H | H |
| 162 | CH | 4-Fluorphenyl | H | Butan-2-yl | (Methoxyacetyl)amino | H | H |
| 163 | CH | 4-Fluorphenyl | H | 2-Methoxyethyl | (Methoxyacetyl)amino | H | H |
| 164 | CH | 4-Fluorphenyl | H | Butan-2-yl | Propanoylamino | H | H |
| 165 | CH | 4-Fluorphenyl | H | 2-Methylpropyl | (Cyclopropylcarbonyl )amino | H | H |
| 166 | CH | 4-Fluorphenyl | H | 2-Methylpropyl | (Methoxyacetyl)amino | H | H |
| 167 | CH | 4-Fluorphenyl | H | Butan-2-yl | (Cyclopropylcarbonyl )amino | H | H |
| 168 | CH | 4-Fluorphenyl | H | Cyclohexyl | H | H | H |
| 169 | CH | 4-Fluorphenyl | H | 2,2-Difluorethyl | H | H | H |
| 170 | CH | 4-Fluorphenyl | H | 2-Ethoxy-2-oxoethyl | H | H | H |
| 171 | CH | 4-Fluorphenyl | CH₃ | Prop-2-in-1-yl | H | H | H |
| 172 | N | 4-Fluorphenyl | H | H | Propan-2-ylamino | H | H |
| 173 | N | 4-Fluorphenyl | H | H | Prop-2-in-1-ylamino | H | H |
| 174 | N | 4-Fluorphenyl | H | 2-Methylpropyl | Propan-2-ylamino | H | H |
| 175 | CH | 4-Fluorphenyl | Cyan | H | H | H | H |
| 176 | CH | 4-Fluorphenyl | H | 1-(2-Chlor-1,3-thiazol-5-yl)ethyl | H | H | H |
| 177 | CH | 4-Fluorphenyl | H | 1,3-Difluorpropan-2-yl | H | H | H |
| 178 | CH | 4-Fluorphenyl | H | 3-Methyl-2-oxobutyl | H | H | H |
| 179 | CH | 4-Fluorphenyl | H | 2-Ethoxyethyl | H | H | H |
| 180 | CH | 4-Fluorphenyl | H | Cyanmethyl | H | H | H |
| 181 | CH | 4-Fluorphenyl | H | Propan-2-yl | [(Propan-2-yloxy)carbonyl]amino | H | H |
| 182 | N | 4-Fluorphenyl | H | 2-Methylpropyl | Benzylamino | H | H |
| 183 | N | 4-Fluorphenyl | H | 2-Methylpropyl | Prop-2-in-1-ylamino | H | H |
| 184 | N | 4-Fluorphenyl | H | 2-Methylpropyl | Amino | H | H |
| 185 | N | 4-Fluorphenyl | H | 2-Methylpropyl | (2-Methylpropanoyl)(pr opan-2-yl)amino | H | H |
| 186 | N | 4-Fluorphenyl | H | 2-Methylpropyl | Propan-2-yl(2,2,4-trimethyl-3-oxopentanoyl)amino | H | H |
| 187 | CH | 4-Fluorphenyl | H | 2-Methylpropyl | 2-Oxopyrrolidin-1-yl | H | H |
| 188 | N | 4-Fluorphenyl | H | 2-Methylpropyl | Bis(2-methylpropanoyl)ami no | H | H |
| 189 | N | 4-Fluorphenyl | H | 2-Methylpropyl | (2-Methylpropanoyl)amino | H | H |
| 190 | CH | 4-Fluorphenyl | H | Ethyl | (Phenylacetyl)amino | H | H |
| 191 | CH | 4-Fluorphenyl | H | 2-Methylpropyl | (Phenylacetyl)amino | H | H |
| 192 | CH | 4-Fluorphenyl | H | Propan-2-yl | [(2S)-2-Hydroxypropanoyl]amino | H | H |
| 193 | CH | 4-Fluorphenyl | H | 1-(tert-Butoxycarbonyl)piperidin-4-yl | Propanoylamino | H | H |
| 194 | CH | 4-Cyan-2,5-difluorphenyl | Difluormethoxy | CH₃ | Propanoylamino | H | H |
| 195 | CH | 4-Chlor-2-fluorphenyl | Methylsulfanyl | CH₃ | Propanoylamino | H | H |
| 196 | CH | 4-Fluor-2-hydroxyphenyl | Difluormethoxy | CH₃ | Propanoylamino | H | H |
| 197 | CH | 4-Chlor-2-fluorphenyl | CF₃ | CH₃ | Propanoylamino | H | H |
| 198 | CH | 4-Fluorphenyl | H | Ethyl | Propanoylamino | H | H |
| 199 | CH | 4-Cyan-2,5-difluorphenyl | Difluormethoxy | CH₃ | (Methoxyacetyl)amino | H | H |
| 200 | CH | 4-Chlor-2-fluorphenyl | Methylsulfanyl | CH₃ | (Methoxyacetyl)amino | H | H |
| 201 | CH | 4-Fluor-2-hydroxyphenyl | Difluormethoxy | CH₃ | (Methoxyacetyl)amino | H | H |
| 202 | CH | 4-Chlor-2-fluorphenyl | CF₃ | CH₃ | (Methoxyacetyl)amino | H | H |
| 203 | CH | 4-Fluorphenyl | H | Ethyl | (Methoxyacetyl)amino | H | H |
| 204 | CH | 4-Fluorphenyl | H | 1-(tert-Butoxycarbonyl)piperidin-4-yl | (Methoxyacetyl)amino | H | H |
| 205 | CH | 4-Fluorphenyl | H | Propan-2-yl | Benzoylamino | H | H |
| 206 | CH | 4-Fluorphenyl | H | Propan-2-yl | (Thiophen-2-ylcarbonyl)amino | H | H |
| 207 | CH | 4-Fluorphenyl | H | Propan-2-yl | (Thiophen-3-ylcarbonyl)amino | H | H |
| 208 | CH | 4-Fluorphenyl | H | Propan-2-yl | [(2-Methoxyethoxy)acety l]amino | H | H |
| 209 | CH | 4-Fluorphenyl | H | Propan-2-yl | (2-Hydroxy-2-methylpropanoyl)amino | H | H |
| 210 | CH | 4-Fluorphenyl | H | Propan-2-yl | (2,3-Dihydroxypropanoyl)amino | H | H |
| 211 | CH | 4-Fluorphenyl | H | Propan-2-yl | (Ethylcarbamoyl)ami no | H | H |
| 212 | CH | 4-Fluorphenyl | H | 2-Methoxyethyl | (Phenylacetyl)amino | H | H |
| 213 | CH | 4-Fluorphenyl | H | 2-Fluorbenzyl | Propanoylamino | H | H |
| 214 | CH | 4-Fluorphenyl | H | Cyclopropylmethyl | Propanoylamino | H | H |
| 215 | CH | 4-Fluorphenyl | H | 2,2-Difluorethyl | Propanoylamino | H | H |
| 216 | CH | 4-Fluorphenyl | H | 2,2-Difluorethyl | (Methoxyacetyl)amino | H | H |
| 217 | CH | 4-Fluorphenyl | H | 2,2-Difluorethyl | (Cyclopropylcarbonyl )amino | H | H |
| 218 | CH | 4-Fluorphenyl | H | 2,2-Difluorethyl | (2-Methylpropanoyl)amino | H | H |
| 219 | CH | 4-Fluorphenyl | H | 2-Chlorethyl | Propanoylamino | H | H |
| 220 | CH | 4-Fluorphenyl | H | 2-Chlorethyl | (Methoxyacetyl)amino | H | H |
| 221 | CH | 4-Fluorphenyl | H | 2-Chlorethyl | (Cyclopropylcarbonyl )amino | H | H |
| 222 | CH | 4-Fluorphenyl | H | 2-Chlorethyl | (2-Methylpropanoyl)amino | H | H |
| 223 | CH | 4-Fluorphenyl | H | Cyclopropylmethyl | (Methoxyacetyl)amino | H | H |
| 224 | CH | 4-Fluorphenyl | H | Cyclopropylmethyl | (Cyclopropylcarbonyl )amino | H | H |
| 225 | CH | 4-Fluorphenyl | H | Cyclopropylmethyl | (2-Methylpropanoyl)amino | H | H |
| 226 | CH | 4-Fluorphenyl | H | Propan-2-yloxy | Propanoylamino | H | H |
| 227 | CH | 4-Fluorphenyl | H | Propan-2-yloxy | H | H | H |
| 228 | CH | 4-Fluorphenyl | H | 2-Methylpropyl | H | H | 4-Fluorphenyl |
| 229 | CH | 4-Fluorphenyl | H | 2-Methylpropyl | H | H | 3-Fluorphenyl |
| 230 | CH | 3-Cyan-4-fluorphenyl | H | 2-Methylpropyl | Propanoylamino | H | H |
| 231 | CH | 4-Fluorphenyl | H | 2,2-Difluorethyl | (Ethylcarbamoyl)amino | H | H |
| 232 | CH | 4-Fluorphenyl | H | 1-(2-Fluorphenyl)ethyl | (2-Methylpropanoyl)amino | H | H |
| 233 | CH | 4-Fluorphenyl | H | Propan-2-yl | (Dimethylcarbamoyl)amino | H | H |
| 234 | CH | 4-Fluorphenyl | H | 2,2-Difluorethyl | (Propan-2-ylsulfonyl)amino | H | H |
| 235 | CH | 4-Fluorphenyl | H | 2,2-Difluorethyl | (Thiophen-3-ylcarbonyl)amino | H | H |
| 236 | CH | 4-Fluorphenyl | H | 2,2-Difluorethyl | (Thiophen-2-ylcarbonyl)amino | H | H |
| 237 | CH | 4-Fluorphenyl | H | 2,2-Difluorethyl | (Phenylcarbonyl)amino | H | H |
| 238 | CH | 4-Fluorphenyl | H | 2,2-Difluorethyl | (3-Methylbutanoyl)amino | H | H |
| 239 | CH | 4-Fluorphenyl | H | 2,2-Difluorethyl | (2-Methylbutanoyl)amino | H | H |
| 240 | CH | 4-Fluorphenyl | H | 2,2-Difluorethyl | (Cyclobutylcarbonyl)amino | H | H |
| 241 | CH | 4-Fluorphenyl | H | 2,2-Difluorethyl | (Ethoxycarbonyl)amino | H | H |
| 242 | CH | 4-Fluorphenyl | H | 2,2-Difluorethyl | Acetylamino | H | H |
| 243 | CH | 4-Fluorphenyl | H | 2,2-Difluorethyl | (2-Hydroxy-2-methylpropanoyl)amino | H | H |
| 244 | CH | 4-Fluorphenyl | H | 1-(2-Fluorphenyl)ethyl | (Methoxyacetyl)amino | H | H |
| 245 | CH | 4-Fluorphenyl | H | (2,2-Dichlorcyclopropyl)methyl | (Methoxyacetyl)amino | H | H |
| 246 | CH | 3-Cyan-4-fluorphenyl | H | 2-Methylpropyl | (Methoxyacetyl)amino | H | H |
| 247 | CH | 4-Fluorphenyl | H | 2-Fluorbenzyl | (Methoxyacetyl)amino | H | H |
| 248 | CH | 4-Fluorphenyl | H | 1-(2-Fluorphenyl)ethyl | Propanoylamino | H | H |
| 249 | CH | 4-Fluorphenyl | H | (2,2-Dichlorcyclopropyl)methyl | Propanoylamino | H | H |
| 250 | CH | 4-Fluorphenyl | H | 1-(2-Fluorphenyl)ethyl | (Cyclopropylacetyl)amino | H | H |
| 251 | CH | 4-Fluorphenyl | H | (2,2-Dichlorcyclopropyl)methyl | (Cyclopropylacetyl)amino | H | H |
| 252 | CH | 3-Cyan-4-fluorphenyl | H | 2-Methylpropyl | (Cyclopropylacetyl)amino | H | H |
| 253 | CH | 4-Fluorphenyl | H | 2-Fluorbenzyl | (Cyclopropylacetyl)amino | H | H |
| 254 | CH | 4-Fluorphenyl | H | (2,2-Dichlorcyclopropyl)methyl | (2-Methylpropanoyl)amino | H | H |
| 255 | CH | 3-Cyan-4-fluorphenyl | H | 2-Methylpropyl | (2-Methylpropanoyl)amino | H | H |
| 256 | CH | 4-Fluorphenyl | H | 2-Fluorbenzyl | (2-Methylpropanoyl)amino | H | H |
| 257 | CH | 4-Fluor-2-hydroxyphenyl | Difluormethoxy | CH₃ | (2-Methylpropanoyl)amino | H | H |
| 258 | CH | 4-Fluor-2-hydroxyphenyl | Difluormethoxy | CH₃ | (Cyclopropylcarbonyl )amino | H | H |
| 259 | CH | 4-Fluorphenyl | H | 1-(2-Fluorphenyl)ethyl | (Cyclopropylcarbonyl )amino | H | H |
| 260 | CH | 4-Fluorphenyl | H | (2,2-Dichlorcyclopropyl)methyl | (Cyclopropylcarbonyl )amino | H | H |
| 261 | CH | 3-Cyan-4-fluorphenyl | H | 2-Methylpropyl | (Cyclopropylcarbonyl )amino | H | H |
| 262 | CH | 4-Fluorphenyl | H | 2-Fluorbenzyl | (Cyclopropylcarbonyl )amino | H | H |
| 263 | CH | 4-Fluorphenyl | H | 1-(2-Fluorphenyl)ethyl | (Ethoxycarbonyl)amino | H | H |
| 264 | CH | 4-Fluorphenyl | H | (2,2-Dichlorcyclopropyl)methyl | (Ethoxycarbonyl)amino | H | H |
| 265 | CH | 3-Cyan-4-fluorphenyl | H | 2-Methylpropyl | (Ethoxycarbonyl)amino | H | H |
| 266 | CH | 4-Fluorphenyl | H | 2-Fluorbenzyl | (Ethoxycarbonyl)amino | H | H |
| 267 | CH | 4-Fluorphenyl | H | 2-Methylpropyl | (Ethoxycarbonyl)amino | H | H |
| 268 | CH | 4-Fluorphenyl | H | Butan-2-yl | (Ethoxycarbonyl)amino | H | H |
| 269 | CH | 4-Fluorphenyl | H | Cyclopentyloxy | (Cyclopropylcarbonyl )amino | H | H |
| 270 | CH | 4-Fluorphenyl | H | Cyclopentyloxy | (2-Methylpropanoyl)amino | H | H |
| 271 | CH | 4-Fluorphenyl | H | Propan-2-yloxy | (Cyclopropylcarbonyl )amino | H | H |
| 272 | CH | 4-Fluorphenyl | H | Propan-2-yloxy | (2-Methylpropanoyl)amino | H | H |
| 273 | N | 4-Fluorphenyl | H | 2-Methylpropyl | (Cyclopropylmethyl)amino | H | H |
| 274 | CH | 4-Fluorphenyl | H | 1-Methoxy-3-methyl-1-oxobutan-2-yl | Propanoylamino | H | H |
| 275 | CH | 4-Fluorphenyl | H | 1,3-Dioxolan-2-ylmethyl | H | H | H |
| 276 | CH | 4-Fluorphenyl | H | 1,3-Dioxolan-2-ylmethyl | (2-Methylpropanoyl)amino | H | H |
| 277 | CH | 4-Fluorphenyl | H | 1-Methoxy-3-methyl-1-oxobutan-2-yl | (2-Methylpropanoyl)amino | H | H |
| 278 | CH | 4-Fluorphenyl | H | 1,3-Dioxolan-2-ylmethyl | Propanoylamino | H | H |
| 279 | N | 4-Fluorphenyl | H | 2-Methylpropyl | Oxetan-3-ylamino | H | H |
| 280 | CH | 4-Fluorphenyl | H | Propan-2-yl | H | -NHCH=N- | |
| 281 | N | 4-Fluorphenyl | H | 2-Methylpropyl | Cyclopropylamino | H | H |
| 282 | N | 4-Fluorphenyl | H | Propan-2-yl | H | -NHCH=N- | |
| 283 | CH | 4-Fluorphenyl | H | Propan-2-yl | 4-Methyl-1H-imidazol-1-yl | H | H |
| 284 | N | 4-Fluorphenyl | H | 2-Methylpropyl | tert-Butylamino | H | H |
| 285 | N | 4-Fluorphenyl | H | 2-Methylpropyl | (1-Methoxypropan-2-yl)amino | H | H |
| 286 | N | 4-Fluorphenyl | H | 2-Methylpropyl | Butan-2-ylamino | H | H |
| 287 | N | 4-Fluorphenyl | H | 2-Methylpropyl | Methylsulfonyl | H | H |
| 288 | N | 4-Fluorphenyl | H | 2-Methylpropyl | H | -C(CH₃)=CHS- | |
| 289 | N | 4-Fluorphenyl | H | Cyclopropylmethyl | H | -C(CH₃)=CHS- | |
| 290 | CH | 4-Fluorphenyl | H | Ethyl | (2-Methylpropanoyl)amino | H | H |
| 291 | CH | 4-Fluorphenyl | H | Ethyl | (Cyclopropylacetyl)amino | H | H |
| 292 | CH | 4-Fluorphenyl | H | Propyl | Propanoylamino | H | H |
| 293 | CH | 4-Fluorphenyl | H | Propyl | (Methoxyacetyl)amino | H | H |
| 294 | CH | 4-Fluorphenyl | H | Propyl | (2-Methylpropanoyl)amino | H | H |
| 295 | CH | 4-Fluorphenyl | H | Propyl | (Cyclopropylacetyl)amino | H | H |
| 296 | CH | 4-Fluorphenyl | H | CH₃ | Propanoylamino | H | H |
| 297 | CH | 4-Fluorphenyl | H | CH₃ | (Methoxyacetyl)amino | H | H |
| 298 | CH | 4-Fluorphenyl | H | CH₃ | (2-Methylpropanoyl)amino | H | H |
| 299 | CH | 4-Fluorphenyl | H | CH₃ | (Cyclopropylacetyl)amino | H | H |
| 300 | CH | 4-Fluorphenyl | H | 2-(Methylsulfanyl)ethyl | Propanoylamino | H | H |
| 301 | CH | 4-Fluorphenyl | H | 1-Cyclopropylethyl | Propanoylamino | H | H |
| 302 | CH | 4-Fluorphenyl | H | 3-Cyanbenzyl | Propanoylamino | H | H |
| 303 | CH | 4-Fluorphenyl | H | 2-Cyanethyl | Propanoylamino | H | H |
| 304 | CH | 4-Fluorphenyl | CF₃ | Propan-2-yl | Propanoylamino | H | H |
| 305 | CH | 4-Fluorphenyl | H | 1-Cyclopropylethyl | H | H | H |
| 306 | CH | 4-Fluorphenyl | H | 2-(Methylsulfanyl)ethyl | (2-Methylpropanoyl)amino | H | H |
| 307 | CH | 4-Fluorphenyl | H | 1-Cyclopropylethyl | (2-Methylpropanoyl)amino | H | H |
| 308 | CH | 4-Fluorphenyl | H | 3-Cyanbenzyl | (2-Methylpropanoyl)amino | H | H |
| 309 | CH | 4-Fluorphenyl | H | 2-Cyanethyl | (2-Methylpropanoyl)amino | H | H |
| 310 | N | 4-Fluorphenyl | H | Propan-2-yl | H | -NHCH=CH- | |
| 311 | N | 4-Fluorphenyl | H | Propan-2-yl | H | -NHCH₂CH₂CH₂- | |
| 312 | CH | 4-Fluorphenyl | H | 1-Cyanpropan-2-yl | Propanoylamino | H | H |
| 313 | CH | 4-Fluorphenyl | H | 1-Cyanpropan-2-yl | (Methoxyacetyl)amino | H | H |
| 314 | CH | 4-Fluorphenyl | H | 1-Cyanpropan-2-yl | (2-Methylpropanoyl)amino | H | H |
| 315 | CH | 4-Fluorphenyl | H | 1-Cyanpropan-2-yl | (Cyclopropylacetyl)amino | H | H |
| 316 | CH | 4-Fluorphenyl | Difluormethoxy | CH₃ | Propanoylamino | H | H |
| 317 | CH | 4-Fluorphenyl | H | Cyclopropylmethyl | (1,1,1-Trifluorpropan-2-yl)amino | H | H |
| 318 | CH | 4-Fluorphenyl | H | Propan-2-yl | (1,1,1-Trifluorpropan-2-yl)amino | H | H |
| 319 | CH | 4-Fluorphenyl | H | CH₃ | (1,1,1-Trifluorpropan-2-yl)amino | H | H |
| 320 | CH | 4-Fluorphenyl | H | Cyclopropyl | Propanoylamino | H | H |
| 321 | CH | 4-Fluorphenyl | H | Cyclopropyl | (Methoxyacetyl)amino | H | H |
| 322 | CH | 4-Fluorphenyl | H | Cyclopropyl | (2-Methylpropanoyl)amino | H | H |
| 323 | CH | 4-Fluorphenyl | H | Cyclopropyl | (Cyclopropylacetyl)amino | H | H |
| 324 | CH | 4-Fluorphenyl | H | Propan-2-yl | [2,2,2-Trifluor-1-(4-fluorphenyl)ethyl]amino | H | H |
| 325 | CH | 4-Fluorphenyl | H | Propan-2-yl | (2,2,2-Trifluor-1-phenylethyl)amino | H | H |
| 326 | CH | 4-Fluorphenyl | H | 2-Ethoxyethyl | Propanoylamino | H | H |
| 327 | CH | 4-Fluorphenyl | H | 2-Ethoxyethyl | (Methoxyacetyl)amino | H | H |
| 328 | CH | 4-Fluorphenyl | H | 2-Ethoxyethyl | (2-Methylpropanoyl)amino | H | H |
| 329 | CH | 4-Fluorphenyl | H | 2-Ethoxyethyl | (Cyclopropylacetyl)amino | H | H |
| 330 | CH | 4-Fluorphenyl | H | Ethyl | (6-Methoxy-6-oxohexanoyl)amino | H | H |
| 331 | CH | 4-Fluorphenyl | H | Ethyl | (5-Methoxy-5-oxopentanoyl)amino | H | H |
| 332 | CH | 4-Fluorphenyl | H | Ethyl | (5-Ethoxy-3-ethyl-5-oxopentanoyl)amino | H | H |
| 333 | CH | 4-Fluorphenyl | H | Ethyl | {[3-(Difluormethyl)-1-methyl-1H-pyrazol-4-yl]carbonyl} amino | H | H |
| 334 | CH | 4-Fluorphenyl | H | Ethyl | {[2-(Difluormethyl)thiop hen-3-yl]carbonyl}amino | H | H |
| 335 | CH | 4-Fluorphenyl | H | Ethyl | [(4-Methyl-1,2,3-thiadiazol-5-yl)carbonyl] amino | H | H |
| 336 | CH | 4-Fluorphenyl | H | Ethyl | [3-(4-Chlorphenyl)propanoyl] amino | H | H |
| 337 | CH | 4-Fluorphenyl | H | Ethyl | {[(1R,2R)-2-Phenylcyclopropyl]ca rbonyl}amino | H | H |
| 338 | CH | 4-Fluorphenyl | H | Ethyl | (Cyclopentylacetyl)amino | H | H |
| 339 | CH | 4-Fluorphenyl | H | Ethyl | (3-Methylbutanoyl)amino | H | H |
| 340 | CH | 4-Fluorphenyl | H | Ethyl | (2-Methylbutanoyl)amino | H | H |
| 341 | CH | 4-Fluorphenyl | H | Ethyl | [(2-Methylcyclopropyl)c arbonyl]amino | H | H |
| 342 | CH | 4-Fluorphenyl | H | Ethyl | Acetylamino | H | H |
| 343 | CH | 4-Fluorphenyl | H | CH₃ | (3-Phenylpropanoyl)amino | H | H |
| 344 | CH | 4-Fluorphenyl | H | CH₃ | (4-Fluorbenzoyl)amino | H | H |
| 345 | CH | 4-Fluorphenyl | H | CH₃ | [(2-Methoxyethoxy)acety l]amino | H | H |
| 346 | CH | 4-Fluorphenyl | H | CH₃ | (Phenylcarbonyl)amino | H | H |
| 347 | CH | 4-Fluorphenyl | H | CH₃ | (3-Methylbutanoyl)amino | H | H |
| 348 | CH | 4-Fluorphenyl | H | CH₃ | (2-Methylbutanoyl)amino | H | H |
| 349 | CH | 4-Fluorphenyl | H | CH₃ | (Cyclobutylcarbonyl)amino | H | H |
| 350 | CH | 4-Fluorphenyl | H | CH₃ | Acetylamino | H | H |
| 351 | CH | 4-Fluorphenyl | H | Ethyl | [Amino(oxo)acetyl]amino | H | H |
| 352 | CH | 4-Fluorphenyl | H | Ethyl | [(2S)-2-Hydroxypropanoyl]amino | H | H |
| 353 | CH | 4-Fluorphenyl | H | CH₃ | [(2S)-2-Hydroxypropanoyl]amino | H | H |
| 354 | N | 4-Fluorphenyl | H | Propan-2-yl | (Methoxyacetyl)amino | H | H |
| 355 | N | 4-Fluorphenyl | H | Propan-2-yl | (2-Methylpropanoyl)amino | H | H |
| 356 | N | 4-Fluorphenyl | H | Propan-2-yl | (Cyclopropylcarbonyl )amino | H | H |
| 357 | N | 4-Fluorphenyl | H | 2-Methylpropyl | Acetylamino | H | H |
| 358 | N | 4-Fluorphenyl | H | 2-Methoxyethyl | Propan-2-ylamino | H | H |
| 359 | N | 4-Fluorphenyl | H | 2-Chlorethyl | Propan-2-ylamino | H | H |
| 360 | N | 4-Fluorphenyl | H | Butan-2-yl | Propan-2-ylamino | H | H |
| 361 | N | 4-Fluorphenyl | H | CH₃ | Propan-2-ylamino | H | H |
| 362 | N | 4-Fluorphenyl | H | 2-Cyanethyl | Propan-2-ylamino | H | H |
| 363 | N | 4-Fluorphenyl | H | Prop-2-in-1-yl | Propan-2-ylamino | H | H |
| 364 | N | 4-Fluorphenyl | H | 2,2-Difluorethyl | Propan-2-ylamino | H | H |
| 365 | N | 4-Fluorphenyl | H | 2-Methylpropyl | Propanoylamino | H | H |
| 366 | N | 4-Fluorphenyl | H | Propan-2-yl | Amino | H | H |
| 367 | CH | 4-Fluorphenyl | CH₃ | 2,2-Difluorethyl | H | H | H |
| 368 | CH | 4-Fluorphenyl | CH₃ | 2-Methylpropyl | H | H | H |
| 369 | N | 4-Fluorphenyl | H | Propan-2-yl | Benzylamino | H | H |
| 370 | N | 4-Fluorphenyl | H | Ethyl | Propan-2-ylamino | H | H |
| 371 | N | 4-Fluorphenyl | H | Propan-2-yl | Propan-2-ylamino | H | H |
| 372 | N | 4-Fluorphenyl | H | 2-Methylpropyl | (1-Cyclopropylethyl)amino | H | H |

| | | | | | | | |
|---|---|---|---|---|---|---|---|
| ^{[s]} Folgende Beispiele liegen in Form von folgenden Salzen vor: Beispiel 4: Hydrochlorid; Beispiel 18: Trihydrochlorid; Beispiel 151: Hydrochlorid; Beispiel 152: Hydrochlorid. | | | | | | | |

**Tabelle 2 NMR-Daten von Verbindungen des Typs [I-a] und [I-b]**

| **Nr.** | **Name** | **NMR** | **Isomeren-verhältnis^{[1]}** |
|---|---|---|---|
| 1 | 4-[3-(4-Fluorphenyl)-1-(2-methylpropyl)-1H-pyrazol-4-yl]pyridin | 1H-NMR (400 MHz, d₆-DMSO): δ = 8.47 (d, 2H), 8.22 (s, 1H), 7.43 (m, 2H), 7.22 (m, 4H), 3.99 (d, 2H), 2.20 (m, 1H), 0.92 (d, 6H) ppm | |
| 2 | 4-[5-(2,6-Difluorphenyl)-1-(2-methylpropyl)-1H-pyrazol-4-yl]pyridin | 1H-NMR (400 MHz, d₆-DMSO): δ = 8.47 (s, 1H), 8.42 (d, 2H), 7.59 (m, 1H), 7.24 (m, 2H), 7.13 (m, 2H), 4.03 (d, 2H), 2.19 (m, 1H), 0.91 (d, 6H) ppm | - |
| 3 | 4-[1-(Butan-2-yl)-3-(2,6-difluorphenyl)-1H-pyrazol-4-yl]pyridin | 1H-NMR (400 MHz, d₆-DMSO): δ = 8.53 (s, 1H), 8.42 (d, 2H), 7.59 (m, 1H), 7.25 (m, 2H), 7.14 (d, 2H), 4.36 (m, 1H), 1.84 (m, 2H), 1.50 (d, 3H), 0.79 (d, 3H) ppm | - |
| 5 | 4-[3-(3-Chlorphenyl)-1-isobutyl-1H-pyrazol-4-yl]pyrimidin | 1H-NMR (400 MHz, CDCl₃): δ = 9.12 (s, 1H), 8.48 (s, 1H), 8.08 (s, 1H), 7.58 (s, 1H), 7.36 (m, 3H), 7.10 (d, 1H), 3.99 (d, 2H), 2.32 (m, 1H), 0.99 (d, 6H) ppm | - |
| 6 | 4-[5-(2,6-Difluorphenyl)-1-(2,2-dimethylpropyl)-1H-pyrazol-4-yl]pyridin | 1H-NMR (400 MHz, d₆-DMSO): δ = 8.43 (s, 1H), 8.42 (d, 2H), 7.58 (m, 1H), 7.23 (m, 2H), 7.13 (d, 2H), 4.02 (s, 2H), 0.96 (s, 9H) ppm | - |
| 7 | 4-[3-(2,6-Difluorphenyl)-1-(3-methylbut-2-en-1-yl)-1H-pyrazol-4-yl]pyridin | 1H-NMR (400 MHz, d₆-DMSO): δ = 8.42 (d, 2H), 8.17 (s, 1H), 7.75 (m, 1H), 7.38 (m, 2H), 7.13 (m, 2H), 5.14 (m, 1H), 4.59 (d, 2H), 1.59 (s, 3H), 1.36 (s, 3H) ppm | - |
| 8 | 4-[1-Cyclobutyl-3-(2,6-difluorphenyl)-1H-pyrazol-4-yl]pyridin | 1H-NMR (400 MHz, d₆-DMSO): δ = 8.42 (d, 2H), 8.24 (s, 1H), 7.76 (m, 1H), 7.38 (m, 2H), 7.13 (d, 2H), 4.50 (m, 1H), 2.58 (m, 2H), 2.23 (m, 2H), 1.75 (m, 2H) ppm | - |
| 9 | 4-[3-(4-Fluorphenyl)-1-(tetrahydro-2H-pyran-2-yl)-1H-pyrazol-4-yl]pyridin | 1H-NMR (400 MHz, d3-CD3CN): δ = 8.45 (dd, 2H), 8.02 (s, 1H), 7.44 (dd, 2H), 7.20 (dd, 2H), 7.10 (t, 2H), 5.44 (dd, 1H), 4.05-4.00 (m, 1H), 3.75-3.70 (m, 1H), 2.20-2.10 (m, 1H), 2.05-1.95 (m, 2H), 1.70-1.50 (m, 3H) ppm | - |
| 10 | 4-[5-(2,6-Difluorphenyl)-1-(propan-2-yl)-1 H-pyrazol-4-yl]pyridin | 1H-NMR (400 MHz, d₆-DMSO): δ = 8.53 (s, 1H), 8.42 (d, 2H), 7.59 (m, 1H), 7.24 (m, 2H), 7.15 (m, 2H), 4.60 (m, 1H), 1.51 (d, 6H) ppm | - |
| 11 | 4-[3-(2,6-Difluorphenyl)-1-(2,2-dimethylpropyl)-1H-pyrazol-4-yl]pyridin | 1H-NMR (400 MHz, d₆-DMSO): δ = 8.42 (d, 2H), 8.20 (s, 1H), 7.74 (m, 1H), 7.38 (m, 2H), 7.12 (d, 2H), 3.33 (s, 2H), 0.79 (s, 9H) ppm | - |
| 13 | 4-[3-(2,6-Difluorphenyl)-1-(2-methylpropyl)-1H-pyrazol-4-yl]pyridin | 1H-NMR (400 MHz, d₆-DMSO): δ = 8.42 (d, 2H), 8.21 (s, 1H), 7.77 (m, 1H), 7.39 (m, 2H), 7.13 (d, 2H), 3.75 (d, 2H), 2.05 (m, 1H), 0.73 (d, 6H) ppm | - |
| 15 | 4-[1-Cyclobutyl-5-(2,6-difluorphenyl)-1H-pyrazol-4-yl]pyridin | 1H-NMR (400 MHz, d₆-DMSO): δ = 8.59 (s, 1H), 8.42 (d, 2H), 7.60 (m, 1H), 7.24 (m, 2H), 7.14 (d, 2H), 4.94 (m, 1H), 2.60-2.45 (m, 4H), 1.83 (m, 2H) ppm | - |
| 16 | 4-[1-Cyclopentyl-3-(2,6-difluorphenyl)-1H-pyrazol-4-yl]pyridin | 1H-NMR (400 MHz, d₆-DMSO): δ = 8.42 (d, 2H), 8.20 (s, 1H), 7.76 (m, 1H), 7.39 (m, 2H), 7.11 (d, 2H), 4.37 (m, 1H), 1.95 (m, 4H), 1.84 (m, 2H), 1.58 (m, 2H) ppm | - |
| 19 | N-{4-[3-(4-Fluorphenyl)-1-(tetrahydro-2H-pyran-2-yl)-1H-pyrazol-4-yl]pyridin-2-yl}propanamid | 1H-NMR (400 MHz, d₆-DMSO): δ = 10.38 (s, 1H), 8.29 (s, 1H), 8.18 (d, 1H), 8.07 (s, 1H), 7.44 (dd, 2H), 7.20 (t, 2H), 6.85 (dd, 1H), 5.49 (dd, 1H), 3.96 (m, 1H), 3.68 (m, 1H), 2.35 (q, 2H), 2.18 (m, 2H), 2.00-1.90 (m, 2H), 1.70 (m, 1H), 1.58 (m, 1H), 1.03 (t, 3H) ppm | - |
| 20 | 4-[3-(4-Fluorphenyl)-1-(tetrahydro-2H-pyran-2-yl)-1H-pyrazol-4-yl]-1H-pyrrolo[2,3-b]pyridin | 1H-NMR (400 MHz, d₃-CD3CN): δ = 9.70 (s, 1H, br), 8.16 (d, 1H), 8.02 (s, 1H), 7.42 (dd, 2H), 7.25 (m, 1H), 6.99 (t, 2H), 6.89 (d, 1H), 6.15 (d, 1H), 5.46 (dd, 1H), 4.04 (m, 1H), 3.76 (m, 1H), 2.25-2.15 (m, 1H), 2.10-2.00 (m, 2H), 1.80-1.60 (m, 3H) ppm | - |
| 33 | 4-[5-(2,2-Difluor-1,3-benzodioxol-4-yl)-1-(tetrahydro-2H-pyran-2-yl)-1H-pyrazol-4-yl]pyridin | 1H-NMR (400 MHz, d₃-CD3CN): δ = 8.39 (m, 2H), 7.97 (s, 1H), 7.70-7.60 (m, 1H), 7.53-7.50 (m, 1H), 7.35-7.30 (m, 2H), 7.12 (m, 2H), 5.10 (m, 1H), 3.90 (m, 1H), 3.40 (m, 1H), 2.50-2.40 (m, 1H), 2.00 (m, 1H), 1.90 (m, 1H), 1.60-1.40 (m, 3H) ppm | - |
| 38 | 4-[1-(Butan-2-yl)-5-(2,6-difluorphenyl)-1H-pyrazol-4-yl]pyridin | 1H-NMR (400 MHz, d₆-DMSO): δ = 8.42 (d, 2H), 8,23 (s, 1H), 7,77 (m, 1H), 7,41 (t, 2H), 7,13 (d, 2H), 3,85 (m, 1H), 1,89 (m, 1H), 1,68 (m, 1H), 1,38 (d, 3H), 0,59 (t, 3H) ppm | |
| 39 | 4-[3-(4-Fluorphenyl)-1-(tetrahydro-2H-pyran-2-yl)-1H-pyrazol-4-yl]pyridin-2-amin | 1H-NMR (400 MHz, d₃-CD3CN): δ = 7.91 (s, 1H), 7.87 (d, 1H), 7.47 (dd, 2H), 7.10 (t, 2H), 6.45 (dd, 1H), 6.37 (s, 1H), 5.42 (dd, 1H), 4.79 (s, 2H, br), 4.05-4.00 (m, 1H), 3.75-3.65 (m, 1H), 2.20-2.10 (m, 1H), 2.05-1.95 (m, 2H), 1.70-1.50 (m, 3H) ppm | - |
| 40 | N-{4-[3-(4-Fluorphenyl)-1-(propan-2-yl)-1H-pyrazol-4-yl]pyridin-2-yl}-2-methoxyacetamid | 1H-NMR (400 MHz, d₆-DMSO): δ = 9.90 (s, 1H), 8.22 (s, 1H), 8.19 (d, 1H), 8.02 (s, 1H), 7.44 (dd, 2H), 7.20 (t, 2H), 6.93 (d, 1H), 4.58 (m, 1H), 4.02 (s, 3H), 3.30 (m, 2H), 1.50 (d, 6H) ppm | - |
| 43 | 4-[3-(4-Methoxyphenyl)-1-(tetrahydro-2H-pyran-2-yl)-1H-pyrazol-4-yl]pyridin | 1H-NMR (400 MHz, d₃-CD3CN): δ = 8.44 (dd, 2H), 8.00 (s, 1H), 7.34 (d, 2H), 7.20 (dd, 2H), 6.92 (d, 2H), 5.43 (dd, 1H), 4.04 (m, 1H), 3.80 (s, 3H), 3.60 (m, 1H), 2.15 (m, 1H), 2.05-2.00 (m, 2H), 1.70-1.50 (m, 3H) ppm | - |
| 44 | 4-[3-(4-Fluorphenyl)-1-(propan-2-yl)-1H-pyrazol-4-yl]chinolin | 1H-NMR (400 MHz, d₆-DMSO): δ = 8.86 (d, 1H), 8.15 (s, 1H), 8.05 (d, 1H), 7.75-7.70 (m, 2H), 7.48 (m, 1H), 7.35 (d, 1H), 7.28 (dd, 2H), 7.03 (t, 2H), 4.65 (m, 1H), 1.56 (d, 6H) ppm | - |
| 46 | 4-[3-(4-Fluorphenyl)-1-(propan-2-yl)-1H-pyrazol-4-yl]pyridin-2-amin | 1H-NMR (400 MHz, d₃-CD3CN): δ = 7.80 (m, 2H), 7.50 (dd, 2H), 7.10 (dd, 1H), 6.47 (d, 1H), 6.39 (s, 1H), 5.10 (s, 2H, br), 4.53 (m, 1H), 1.20 (d, 6H) ppm | - |
| 47 | 4-[3-(4-Fluorphenyl)-1-(propan-2-yl)-1H-pyrazol-4-yl]-1-(prop-2-in-1-yl)-1H-pyrrolo[2,3-b]pyridin | 1H-NMR (400 MHz, d₃-CD3CN): δ = 8.18 (d, 1H), 8.05 (d, 1H, Isomer), 7.96 (s, 1H, Isomer), 7.93 (s, 1H), 7.43 (m, 3H), 7.38 (dd, 2H, Isomer), 7.19 (t, 2H, Isomer), 7.01 (t, 2H), 6.89 (d, 1H), 6.64 (d, 1H, Isomer), 6.48 (d, 1H, Isomer), 6.29 (d, 1H), 5.08 (d, 1H), 5.04 (d, 1H, Isomer), 4.60 (m, 1H), 4.48 (m, 1H, Isomer), 2.63 (t, 1H), 2.60 (t, 1H, Isomer), 1.56 (d, 6H), 1.42 (d, 6H, Isomer) ppm | 80:20 |
| 53 | 4-[3-(4-Fluorphenyl)-1-(propan-2-yl)-1H-pyrazol-4-yl]-N-methylpyridin-2-carboxamid | 1H-NMR (400 MHz, d₆-DMSO): δ = 8.74 (d, 1H), 8.50 (d, 1H), 8.41 (s, 1H), 7.90 (s, 1H), 7.45-7.40 (dd, 2H), 7.37 (d, 1H), 7.25-7.20 (t, 2H), 4.57 (m, 1H), 2.79 (d, 3H), 1.51 (d, 6H) ppm | - |
| 70 | 4-[5-(4-Chlorphenyl)-1-(tetrahydro-2H-pyran-2-yl)-1H-pyrazol-4-yl]pyridin | 1H-NMR (400 MHz, d₃-CD3CN): δ = 8.37 (m, 2H), 7.90 (s, 1H), 7.53 (d, 2H), 7.37 (d, 2H), 7.09 (m, 2H), 5.02 (dd, 1H), 3.96 (m, 1H), 3.46 (m, 1H), 2.50-2.35 (m, 1H), 2.00 (m, 1H), 1.80 (m, 1H), 1.60-1.40 (m, 3H) ppm | - |
| 76 | 4-[5-(2,3-Dichlorphenyl)-1-(tetrahydro-2H-pyran-2-yl)-1H-pyrazol-4-yl]pyridin | 1H-NMR (400 MHz, d₃-CD3CN): δ = 8.37 (m, 2H), 8.01 (s, 1H), 7.75 (m, 1H), 7.50-7.45 (m, 1H), 7.43-7.38 (m, 1H), 7.05 (m, 2H), 5.08 (dd, 1H), 4.90 (dd, 1H, Isomer), 3.90-3.80 (m, 1H), 3.40-3.30 (m, 1H), 2.50-2.40 (m, 1H), 2.00 (m, 1H), 1.80 (m, 1H), 1.60-1.40 (m, 3H) ppm | 40:60 |
| 81 | 1-{4-[3-(4-Fluorphenyl)-1-(tetrahydro-2H-pyran-2-yl)-1H-pyrazol-4-yl]-1H-pyrrolo[2,3-b]pyridin-1-yl}ethanon | 1H-NMR (400 MHz, d₃-CD3CN): δ = 8.29 (d, 1H), 8.03 (s, 1H), 7.85 (d, 1H), 7.40-7.35 (dd, 2H), 7.09 (d, 1H), 7.02 (t, 2H), 6.34 (d, 1H), 5.49 (dd, 1H), 4.05-4.00 (m, 1H), 3.75-3.70 (m, 1H), 3.00 (s, 3H), 2.30-2.00 (m, 3H), 1.80-1.60 (m, 3H) ppm | - |
| 84 | N-{4-[3-(4-Fluorphenyl)-1-(propan-2-yl)-1H-pyrazol-4-yl]pyridin-2-yl}-2-methylpropanamid | 1H-NMR (400 MHz, d₆-DMSO): δ = 10.36 (s, 1H), 8.18 (s, 1H), 8.16 (d, 1H), 8.10 (s, 1H), 7.44 (dd, 2H), 7.19 (t, 2H), 6.80 (dd, 1H), 4.57 (m, 1H), 2.73 (m, 1H), 1.50 (d, 6H), 1.06 (d, 6H) ppm | - |
| 85 | N-{4-[3-(4-Fluorphenyl)-1-(propan-2-yl)-1H-pyrazol-4-yl]pyridin-2-yl}propanamid | 1H-NMR (400 MHz, d₃-CD3CN): δ = 8.55 (s, 1H, br), 8.10 (m, 2H), 7.88 (s, 1H), 7.46 (dd, 2H), 7.09 (t, 2H), 6.87 (dd, 1H), 4.55 (m, 1H), 2.38 (q, 2H), 1.51 (d, 6H), 1.10 (t, 2H) ppm | - |
| 86 | N-{4-[3-(4-Fluorphenyl)-1-(propan-2-yl)-1H-pyrazol-4-yl]pyridin-2-yl}cyclopropancarboxamid | 1H-NMR (400 MHz, d₃-CD3CN): δ = 8.82 (s, 1H, br), 8.11 (d, 1H), 8.07 (s, 1H), 7.85 (s, 1H), 7.44 (dd, 2H), 7.06 (t, 2H), 6.86 (dd, 1H), 4.54 (m, 1H), 1.78 (m, 1H), 1.51 (d, 6H), 0.90-0.80 (m, 4H) ppm | - |
| 87 | N-{4-[3-(4-Fluorphenyl)-1-(2-methylpropyl)-1H-pyrazol-4-yl]pyridin-2-yl}-2-methylpropanamid | 1H-NMR (400 MHz, d₃-CD3CN): δ = 8.59 (s, 1H, br), 8.12 (m, 2H), 7.83 (s, 1H), 7.45 (dd, 2H), 7.09 (t, 2H), 6.86 (dd, 1H), 3.96 (d, 2H), 2.62 (m, 1H), 2.21 (m, 1H), 1.14 (d, 6H), 0.93 (d, 6H) ppm | - |
| 88 | 4-[3-(4-Fluorphenyl)-1-(2-methoxyethyl)-1H-pyrazol-4-yl]pyridin | 1H-NMR (400 MHz, d₆-DMSO): δ = 8.45 (d, 2H), 8.37 (d, 2H, Isomer), 8.20 (s, 1H), 8.08 (s, 1H, Isomer), 7.48-7.38 (m, 2H), 7.25-7.20 (m, 4H), 7.08 (d, 2H, Isomer), 4.33 (t, 2H), 4.06 (t, 2H, Isomer), 3.77 (t, 2H), 3.65 (t, 2H, Isomer), 3.27 (s, 3H), 3.11 (s, 3H, Isomer) ppm | 70:30 |
| 92 | 4-[1-Cyclopentyl-3-(4- fluorphenyl)-1H-pyrazol-4-yl]pyridin | 1H-NMR (400 MHz, d₆-DMSO): δ = 8.45 (d, 2H), 8.36 (d, 2H, Isomer), 8.28 (s, 1H), 8.07 (s, 1H, Isomer), 7.48-7.38 (m, 2H), 7.25-7.20 (m, 4H), 7.07 (d, 2H, Isomer), 4.75 (m, 1H), 4.38 (m, 1H, Isomer), 2.15-2.10 (m, 2H), 2.05-2.00 (m, 2H), 2.00-1.90 (m, 2H, Isomer), 1.80 (m, 2H), 1.70-1.65 (m, 2H), 1.55 (m, 2H, Isomer) ppm | 85:15 |
| 92 | 4-[1-Cyclopentyl-3-(4- fluorphenyl)-1H-pyrazol-4-yl]pyridin | 1H-NMR (400 MHz, d₆-DMSO): δ = 8.45 (d, 2H), 8.36 (d, 2H, Isomer), 8.28 (s, 1H), 8.07 (s, 1H, Isomer), 7.48-7.38 (m, 2H), 7.25-7.20 (m, 4H), 7.07 (d, 2H, Isomer), 4.75 (m, 1H), 4.38 (m, 1H, Isomer), 2.15-2.10 (m, 2H), 2.05-2.00 (m, 2H), 2.00-1.90 (m, 2H, Isomer), 1.80 (m, 2H), 1.70-1.65 (m, 2H), 1.55 (m, 2H, Isomer) ppm | 80:20 |
| 100 | 3-{[3-(4-Fluorphenyl)-4-(pyridin-4-yl)-1H-pyrazol-1-yl]methyl}pyridin | 1H-NMR (400 MHz, d₆-DMSO): δ = 8.63 (s, 1H), 8.54 (d, 1H), 8.47 (d, 2H), 8.38 (s, 1H), 7.78 (d, 1H), 7.40 (m, 3H), 7.25-7.20 (m, 4H), 5.46 (s, 2H) ppm | - |
| 101 | 4-{1-[1-(3-Chlorphenyl)ethyl]-3-(4-fluorphenyl)-1H-pyrazol-4-yl}pyridin | 1H-NMR (400 MHz, d₆-DMSO): δ = 8.47 (d, 2H), 8.38 (d, 2H, Isomer), 7.50-7.10 (m, 11H), 7.11 (m, 2H, Isomer), 5.72 (m, 1 H), 5.30 (m, 1 H, Isomer), 1.88 (d, 3H), 1.79 (d, 3H, Isomer) ppm | 80:20 |
| 102 | 4-{1-[1-(2-Chlorphenyl)ethyl]-3-(4-fluorphenyl)-1H-pyrazol-4-yl}pyridin | 1H-NMR (400 MHz, d₆-DMSO): δ = 8.46 (m, 3H), 8.37 (d, 2H, Isomer), 7.50 (d, 1H), 7.40-7.20 (m, 10H), 7.11 (d, 2H, Isomer), 6.03 (q, 2H), 5.65 (q, 2H, Isomer), 1.88 (d, 3H), 1.79 (d, 3H, Isomer) ppm | 91:9 |
| 103 | 4-[3-(4-Fluorphenyl)-1-(2,2,2-trifluorethyl)-1H-pyrazol-4-yl]pyridin | 1H-NMR (400 MHz, d₆-DMSO): δ = 8.50 (d, 2H), 8.40 (d, 2H, Isomer), 8.33 (s, 1H), 8.23 (s, 1H, Isomer), 7.48-7.38 (m, 2H), 7.25-7.20 (m, 4H), 7.11 (d, 2H, Isomer), 5.25 (q, 2H), 4.89 (q, 2H, Isomer) ppm | 91:9 |
| 104 | 4-[1-(2,3-Difluorbenzyl)-3-(4-fluorphenyl)-1H-pyrazol-4-yl]pyridin | 1H-NMR (400 MHz, d₆-DMSO): δ = 8.47 (d, 2H), 8.38 (d, 2H, Isomer), 8.35 (s, 1H), 8.14 (s, 1H, Isomer), 7.45-7.30 (m, 4H), 7.25-7.20 (m, 5H), 7.11 (d, 2H, Isomer), 5.52 (s, 2H), 5.29 (s, 1H, Isomer) ppm | 75:25 |
| 105 | tert-Butyl- [3-(4-fluorphenyl)-4-(pyridin-4-yl)-1H-pyrazol-1-yl]acetat | 1H-NMR (400 MHz, d₆-DMSO): δ = 8.48 (d, 2H), 8.39 (d, 2H, Isomer), 8.21 (s, 1H), 8.10 (s, 1H, Isomer), 7.40 (m, 2H), 7.25-7.20 (m, 4H), 7.12 (d, 2H, Isomer), 5.04 (s, 2H), 4.79 (s, 2H, Isomer), 1.45 (s, 9H), 1.30 (s, 9H, Isomer) ppm | 78:22 |
| 106 | Methyl-2-[3-(4-fluorphenyl)-4-(pyridin-4-yl)-1H-pyrazol-1-yl]-3-methylbutanoat | 1H-NMR (400 MHz, d₆-DMSO): δ = 8.48 (d, 2H), 8.37 (d, 2H, Isomer), 8.36 (s, 1H), 8.17 (s, 1H, Isomer), 7.43 (m, 2H), 7.25-7.20 (m, 4H), 7.09 (d, 2H, Isomer), 4.89 (d, 1H), 4.30 (d, 1H, Isomer), 3.72 (s, 3H), 3.64 (s, 3H, Isomer), 1.00 (d, 3H), 0.87 (d, 3H), 0.71 (d, 3H, Isomer) ppm | 93:7 |
| 107 | Methyl-2-[3-(4-fluorphenyl)-4-(pyridin-4-yl)-1H-pyrazol-1-yl]propanoat | 1H-NMR (400 MHz, d₆-DMSO): δ = 8.48 (d, 2H), 8.36 (s, 1H), 7.43 (dd, 2H), 7.25-7.20 (m, 4H), 5.36 (q, 1H), 3.70 (s, 3H), 1.75 (d, 3H) ppm | - |
| 108 | 4-[1-(2-Chlor-6-fluorbenzyl)-3-(4-fluorphenyl)-1H-pyrazol-4-yl]pyridin | 1H-NMR (400 MHz, d₆-DMSO): δ = 8.46 (d, 2H), 8.37 (d, 2H, Isomer), 8.29 (s, 1H), 8.00 (s, 1H, Isomer), 7.55-7.45 (m, 1H), 7.40-7.30 (m, 3H), 7.25-7.20 (m, 4H), 7.09 (d, 2H, Isomer), 5.53 (s, 2H), 5.26 (s, 1H, Isomer) ppm | 87:13 |
| 109 | 4-{1-[4-(Difluormethoxy)benzyl]-3-(4-fluorphenyl)-1H-pyrazol-4-yl}pyridin | 1H-NMR (400 MHz, d₆-DMSO): δ = 8.46 (d, 2H), 8.38 (d, 2H, Isomer), 8.35 (s, 1H), 8.14 (s, 1H, Isomer), 7.45-7.35 (m, 4H), 7.25-7.20 (m, 6H), 7.15 (m, 1H), 7.05 (d, 2H, Isomer), 5.39 (s, 2H), 5.18 (s, 1H, Isomer) ppm | 74:26 |
| 110 | 4-{1-[(2-Chlor-1,3-thiazol-5-yl)methyl]-3-(4-fluorphenyl)-1H-pyrazol-4-yl}pyridin | 1H-NMR (400 MHz, d₆-DMSO): δ = 8.47 (d, 2H), 8.32 (s, 1H), 7.78 (s, 1H), 7.45-7.40 (dd, 2H), 7.25-7.20 (m, 4H), 5.66 (s, 2H) ppm | - |
| 111 | 4-{3-(4-Fluorphenyl)-1-[5-(trifluormethyl)-1,3,4-thiadiazol-2-yl]-1H-pyrazol-4-yl}pyridin | 1H-NMR (400 MHz, d₆-DMSO): δ = 9.32 (s, 1H), 8.58 (d, 2H), 7.53 (dd, 2H), 7.37 (d, 2H), 7.34 (t, 2H) ppm | - |
| 112 | 4-[3-(4-Fluorphenyl)-4-(pyridin-4-yl)-1H-pyrazol-1-yl]-6-(trifluormethyl)pyrimidin | 1H-NMR (400 MHz, d₆-DMSO): δ = 9.39 (s, 1H), 9.26 (s, 1H), 8.57 (d, 2H), 8.35 (s, 1H), 7.59 (dd, 2H), 7.38 (d, 2H), 7.33 (t, 2H) ppm | - |
| 113 | 1-Cyclohexyl-2-[3-(4-fluorphenyl)-4-(pyridin-4-yl)-1H-pyrazol-1-yl]ethanon | 1H-NMR (400 MHz, d₆-DMSO): δ = 8.47 (d, 2H), 8.38 (d, 2H, Isomer), 8.12 (s, 1H), 8.09 (s, 1H, Isomer), 7.43-7.35 (m, 2H), 7.25-7.20 (m, 4H), 7.11 (d, 2H, Isomer), 5.32 (s, 2H), 5.09 (s, 1H, Isomer), 2.60 (m, 1H), 1.90 (m, 2H), 1.70 (m, 2H), 1.65-1.60 (m, 2H), 1.30-1.10 (m, 4H) ppm | 80:20 |
| 114 | 1-Cyclopentyl-2-[3-(4-fluorphenyl)-4-(pyridin-4-yl)-1H-pyrazol-1-yl]ethanon | 1H-NMR (400 MHz, d₆-DMSO): δ = 8.47 (d, 2H), 8.38 (d, 2H, Isomer), 8.15 (s, 1H), 8.10 (s, 1H, Isomer), 7.43-7.35 (m, 2H), 7.25-7.20 (m, 4H), 7.11 (d, 2H, Isomer), 5.30 (s, 2H), 5.08 (s, 1H, Isomer), 3.05 (m, 1H), 2.90 (m, 1H, Isomer), 1.90-1.20 (m, 8H) ppm | 75:25 |
| 115 | 2-{[3-(4-Fluorphenyl)-4-(pyridin-4-yl)-1H-pyrazol-1-yl]methyl}pyridin | 1H-NMR (400 MHz, d₆-DMSO): δ = 8.56 (d, 1H), 8.47 (d, 2H), 8.22 (s, 1H), 7.88 (t, 1H), 7.58 (d, 2H), 7.52 (d, 1H), 7.46 (dd, 2H), 7.38 (m, 1H), 7.25-7.20 (m, 2H), 5.63 (s, 2H) ppm | - |
| 115 | 2-{[3-(4-Fluorphenyl)-4-(pyridin-4-yl)-1H-pyrazol-1-yl]methyl}pyridin | 1H-NMR (400 MHz, d₆-DMSO): δ = 8.57 (d, 1H), 8.47 (d, 2H), 8.38 (m, 1H), 7.88 (m, 1H), 7.72 (m, 1H, Isomer), 7.45-7.40 (m, 3H), 7.30 (m, 1H), 7.25 (m, 1H), 7.20 (m, 2H), 7.11 (d, 2H, Isomer), 5.51 (s, 2H), 5.27 (s, 1H, Isomer) ppm | 75:25 |
| 116 | 4-{1-[(2,2-Dichlorcyclopropyl)methyl]-3-(4-fluorphenyl)-1H-pyrazol-4-yl}pyridin | 1H-NMR (400 MHz, d₆-DMSO): δ = 8.48 (d, 2H), 8.39 (d, 2H, Isomer), 8.30 (s, 1H), 8.15 (s, 1H, Isomer), 7.50 (m, 2H, Isomer), 7.48-7.40 (m, 2H), 7.25-7.20 (m, 4H), 7.11 (d, 2H, Isomer), 4.44 (dd, 1H), 4.34 (dd, 1H), 4.13 (m, 2H, Isomer), 2.38 (m, 1H), 2.21 (m, 1H, Isomer), 1.89 (dd, 1H), 1.77 (dd, 1H, Isomer), 1.70 (dd, 1H), 1.37 (dd, 1H, Isomer) ppm | 75:25 |
| 117 | 4-{3-(4-Fluorphenyl)-1-[2-(methylsulfanyl)ethyl]-1H-pyrazol-4-yl}pyridin | 1H-NMR (400 MHz, d₆-DMSO): δ = 8.46 (d, 2H), 8.38 (d, 2H, Isomer), 8.28 (s, 1H), 8.10 (s, 1H, Isomer), 7.48-7.38 (m, 2H), 7.25-7.20 (m, 4H), 7.08 (d, 2H, Isomer), 4.37 (t, 2H), 4.10 (t, 2H, Isomer), 2.98 (t, 2H), 2.82 (t, 2H, Isomer), 2.07 (s, 3H), 1.81 (s, 3H, Isomer) ppm | 80:20 |
| 120 | 4-{1-[1-(4-Chlorphenyl)ethyl]-3-(4-fluorphenyl)-1H-pyrazol-4-yl}pyridin | 1H-NMR (400 MHz, d₆-DMSO): δ = 8.46 (d, 2H), 8.42 (s, 1H), 8.37 (d, 2H, Isomer), 8.17 (s, 1H, Isomer), 7.45-7.35 (m, 6H), 7.25-7.20 (m, 4H), 7.08 (m, 2H, Isomer), 5.70 (q, 1H), 5.30 (q, 1H, Isomer), 1.86 (d, 3H), 1.78 (d, 3H, Isomer) ppm | 80:20 |
| 121 | 4-[3-(4-Fluorphenyl)-1-(pyridin-4-ylmethyl)-1H-pyrazol-4-yl]pyridin | 1H-NMR (400 MHz, d₆-DMSO): δ = 8.62 (d, 2H), 8.59 (d, 2H), 8.32 (s, 1H), 7.67 (d, 2H), 7.50 (m, 2H), 7.30 (d, 2H), 7.27 (t, 2H), 5.59 (s, 2H) ppm | - |
| 122 | 4-{3-(4-Fluorphenyl)-1-[2-(trifluormethoxy)ethyl]-1H-pyrazol-4-yl}pyridin | 1H-NMR (400 MHz, d₆-DMSO): δ = 8.48 (d, 2H), 8.38 (d, 2H, Isomer), 8.29 (s, 1H), 8.16 (s, 1H, Isomer), 7.43 (m, 2H), 7.25-7.20 (m, 4H), 7.10 (d, 2H, Isomer), 4.51 (m, 4H), 4.40 (t, 2H, Isomer), 4.24 (t, 2H, Isomer) ppm | 88:12 |
| 122 | 4-{3-(4-Fluorphenyl)-1-[2-(trifluormethoxy)ethyl]-1H-pyrazol-4-yl}pyridin | 1H-NMR (400 MHz, d₆-DMSO): δ = 8.48 (d, 2H), 8.38 (d, 2H, Isomer), 8.29 (s, 1H), 8.16 (s, 1H, Isomer), 7.43 (m, 2H), 7.25-7.20 (m, 4H), 7.10 (d, 2H, Isomer), 4.51 (m, 4H), 4.40 (t, 2H, Isomer), 4.24 (t, 2H, Isomer) ppm | 66:33 |
| 123 | 4-[3-(4-Fluorphenyl)-1-(tetrahydrofuran-2-ylmethyl)-1H-pyrazol-4-yl]pyridin | 1H-NMR (400 MHz, d₆-DMSO): δ = 8.45 (d, 2H), 8.37 (d, 2H, Isomer), 8.18 (s, 1H), 8.07 (s, 1H, Isomer), 7.40 (m, 2H), 7.25-7.20 (m, 4H), 7.08 (d, 2H, Isomer), 4.25 (m, 2H), 4.20 (m, 1H), 3.99 (dd, 1H, Isomer), 3.89 (dd, 1H, Isomer), 3.79 (m, 1H), 3.66 (m, 1H), 3.55 (m, 1H, Isomer), 2.00 (m, 1H), 1.83 (m, 2H), 1.56 (m, 1H) ppm | 89:11 |
| 124 | 3-[3-(4-Fluorphenyl)-4-(pyridin-4-yl)-1H-pyrazol-1-yl]propan-1,2-diol | 1H-NMR (400 MHz, d₆-DMSO): δ = 8.65 (d, 2H), 8.51 (s, 1H), 8.47 (s, 1H), 7.78 (d, 2H), 7.53 (m, 2H), 7.30 (t, 2H), 5.70 (s, 1H, br), 5.15 (s, 1H, br), 4.57 (dd, 1H), 4.28 (dd, 1H), 3.80 (m, 1H), 3.48 (m, 2H) ppm | - |
| 128 | 4-[1-(2-Fluorbenzyl)-3-(4-fluorphenyl)-1H-pyrazol-4-yl]pyridin | 1H-NMR (400 MHz, d₆-DMSO): δ = 8.47 (d, 2H), 8.38 (d, 2H, Isomer), 8.32 (s, 1H), 8.13 (s, 1H, Isomer), 7.40-7.30 (m, 5H), 7.25-7.20 (m, 5H), 7.12 (m, 2H, Isomer), 5.47 (s, 2H), 5.24 (s, 2H, Isomer) ppm | 80:20 |
| 134 | 4-{3-(4-Fluorphenyl)-1-[2-(2-methoxyethoxy)ethyl]-1H-pyrazol-4-yl}pyridin | 1H-NMR (400 MHz, d₃-CD₃CN): δ = 8.44 (d, 2H), 7.91 (s, 1H), 7.45 (m, 2H), 7.18 (d, 2H), 7.12 (t, 2H), 4.31 (t, 2H), 3.86 (t, 2H), 3.57 (t, 2H), 3.46 (t, 2H), 3.26 (s, 3H) ppm | - |
| 140 | 3-{[3-(4-Fluorphenyl)-4-(pyridin-4-yl)-1H-pyrazol-1-yl]methyl}benzonitril | 1H-NMR (400 MHz, d₃-CD₃CN): δ = 8.47 (dd), 8.37 (m), 8.16 (s), 7.86 (s), 7.81 (d), 7.70 (m), 7.50 (t), 7.45-7.30 (m), 7.25-7.10 (m), 7.12 (dd), 5.47 (s, 2H), 5.27 (s, 2H, Isomer) ppm | 60:40 |
| 141 | 4-{[3-(4-Fluorphenyl)-4-(pyridin-4-yl)-1H-pyrazol-1-yl]methyl}benzonitril | 1H-NMR (400 MHz, d₆-DMSO): δ = 8.48 (d, 2H), 8.39 (d, 2H, Isomer), 8.37 (s, 1H), 8.17 (s, 1H, Isomer), 7.84 (d, 2H), 7.74 (d, 2H, Isomer), 7.50 (d, 2H), 7.40 (m, 2H), 7.33 (m, 1H), 7.25-7.20 (m, 2H), 7.08 (m, 2H, Isomer), 5.52 (s, 2H), 5.30 (s, 2H, Isomer) ppm | 70:30 |
| 142 | 3-[3-(4-Fluorphenyl)-4-(pyridin-4-yl)-1H-pyrazol-1-yl]dihydrofuran-2(3H)-on | 1H-NMR (400 MHz, d₃-CD₃CN): δ = 8.46 (m, 2H), 8.03 (s, 1H), 7.46 (dd, 2H), 7.24 (dd, 2H), 7.10 (t, 2H), 5.33 (t, 1H), 4.60 (m, 1H), 4.40 (m, 1H), 2.90-2.80 (m, 2H) ppm | - |
| 143 | 4-[3-(4-Fluorphenyl)-1-(1H-imidazol-2-ylmethyl)-1H-pyrazol-4-yl]pyridin | 1H-NMR (400 MHz, d₃-CD₃CN): δ = 8.43 (d, 2H), 7.94 (s, 1H), 7.44 (dd, 2H), 7.17 (d, 2H), 7.10 (t, 2H), 6.99 (s, 2H), 5.40 (s, 2H), 2.90 (s, 1H, br) ppm | - |
| 144 | 1-(1,5-Dimethyl-1H-pyrazol-3-yl)-2-[3-(4-fluorphenyl)-4-(pyridin-4-yl)-1H-pyrazol-1-yl]ethanon | 1H-NMR (400 MHz, d₃-CD₃CN): δ = 8.45 (d, 2H), 8.35 (d, 2H, Isomer), 7.94 (s, 1H, Isomer), 7.91 (s, 1H), 7.44 (dd, 2H), 7.36 (dd, 2H, Isomer), 7.20 (m, 2H), 7.10 (m, 2H), 6.57 (s, 1H), 6.47 (s, 1H, Isomer), 5.62 (s, 2H), 5.40 (s, 2H, Isomer), 3.85 (s, 3H), 3.77 (s, 3H, Isomer), 2.31 (s, 3H), 2.26 (s, 3H, Isomer) ppm | 62:38 |
| 144 | 1-(1,5-Dimethyl-1H-pyrazol-3-yl)-2-[3-(4-fluorphenyl)-4-(pyridin-4-yl)-1H-pyrazol-1-yl]ethanon | 1H-NMR (400 MHz, d₃-CD₃CN): δ = 8.45 (d, 2H), 7.91 (s, 1H), 7.44 (dd, 2H), 7.20 (d, 2H), 7.10 (t, 2H), 6.57 (s, 1H), 5.62 (s, 2H), 3.85 (s, 3H), 2.31 (s, 3H) ppm | - |
| 145 | 2-[3-(4-Fluorphenyl)-4-(pyridin-4-yl)-1H-pyrazol-1-yl]ethylacetat | 1H-NMR (400 MHz, d₃-CD₃CN): δ = 8.50 (m, 2H, br), 7.89 (s, 1H), 7.45 (dd, 2H), 7.28 (m, 2H, br), 7.10 (t, 2H), 4.46 (m, 2H), 4.39 (m, 2H), 4.28 (t, 2H, Isomer), 3.90 (t, 2H), 2.00 (s, 3H), 1.90 (s, 3H, Isomer) ppm | 91:9 |
| 146 | 3-[3-(4-Fluorphenyl)-4-(pyridin-4-yl)-1H-pyrazol-1-yl]propanonitril | 1H-NMR (400 MHz, d₃-CD₃CN): δ = 8.50 (m, 2H, br), 7.94 (s, 1H), 7.46 (dd, 2H), 7.28 (m, 2H, br), 7.14 (t, 2H), 4.45 (t, 2H), 3.02 (t, 2H) ppm | - |
| 147 | 4-[3-(4-Fluorphenyl)-1-isobutyl-1H-pyrazol-4-yl]-1,3-dihydro-2H-pyrrolo[2,3-b]pyridin-2-on | ¹H-NMR (400 MHz, d₆-DMSO): δ = 11.02 (s, 1H), 8.21 (s, 1H), 7.91 (d, 1H), 7.42 (m, 2H), 7.21 (m, 2H), 6.60 (d, 1H), 4.00 (d, 2H), 3.42 (s, 2H), 2.20 (m, 1H), 0.92 (d, 6H) ppm | - |
| 148 | 4-[3-(4-Fluorphenyl)-1-isobutyl-1H-pyrazol-4-yl]-1,3,3-trimethyl-1,3-dihydro-2H-pyrrolo[2,3-b]pyridin-2-on | ¹H-NMR (400 MHz, d₆-DMSO): δ = 8.06 (d, 1H), 7.30 (m, 3H), 6.85 (t, 2H), 6.67 (d, 1H), 3.92 (d, 2H), 3.27 (s, 3H), 2.26 (m, 1H), 1.13 (s, 6H), 0.92(d, 6H) ppm | - |
| 149 | 4-[5-(4-Fluorphenyl)-1-(2-methylpropyl)-1H-pyrazol-4-yl]-N-(prop-2-en-1-yl)pyrimidin-2-amin | 1H-NMR (400 MHz, CDCl₃): δ = 8,11 (s, 1H), 8,03 (d, 1H), 7,32 (m, 2H), 7,19 (t, 2H), 6,18 (bs, 1H), 5,87 (m, 1H), 5,20 (d, 1H), 5,09 (d, 1H), 5,02 (m, 1H), 3,85 (bs, 2H), 3,74 (d, 2H), 2,18 (m, 1H), 0,79 (d, 6H) ppm | - |
| 150 | 4-[3-(4-Fluorphenyl)-1-(2-methylpropyl)-1H-pyrazol-4-yl]-N-(prop-2-en-1-yl)pyrimidin-2-amin | 1H-NMR (400 MHz, CDCl₃): δ = 8,10 (d, 1H), 7,93 (s, 1H), 7,54 (m, 2H), 7,07 (t, 2H), 6,39 (d, 1H), 5,95 (m, 1H), 5,26 (d, 1H), 5,12 (d, 2H), 4,04 (bs 1H), 3,96 (d, 2H), 2,30 (m, 1H), 0,98 (d, 6H) ppm | - |
| 153 | N-{4-[3-(4-Fluorphenyl)-1-(2-methoxyethyl)-1 H-pyrazol-4-yl]pyridin-2-yl}propanamid | 1H-NMR (400 MHz, d₃-CD₃CN): δ = 8.54 (s, 1H, br), 8.10 (m, 2H), 7.86 (s, 1H), 7.46 (dd, 2H), 7.09 (t, 2H), 6.87 (dd, 1H), 4.31 (t, 2H), 3.78 (t, 2H), 3.32 (s, 3H), 2.38 (q, 2H), 1.11 (t, 2H) ppm | - |
| 154 | 2-Fluor-5-[1-(2-methylpropyl)-4-(pyridin-4-yl)-1H-pyrazol-3-yl]benzonitril | 1H-NMR (400 MHz, d₃-CD₃CN): δ = 8.47-8.46 (m, 2H), 7.86 (s, 1H), 7.81-7.79 (m, 1H), 7.73-7.71 (m, 1H), 7.33-7.30 (m, 1H), 7.19-7.18 (m, 2H), 3.99 (d, 2H), 2.23 (m, 1H), 0.94 (d, 6H) ppm | - |
| 155 | N-{4-[3-(4-Fluorphenyl)-1-(2-methylpropyl)-1H-pyrazol-4-yl]pyridin-2-yl}propanamid | 1H-NMR (400 MHz, d₆-DMSO): δ = 10.40 (s, 1H), 8.16 (d, 1H), 8.14 (s, 1H), 8.08 (s, 1H), 7.42-7.40 (m, 2H), 7.20-7.18 (m, 2H), 6.82 (dd, 1H), 3.98 (d, 2H), 2.38 (q, 2H), 2.20 (m, 1H), 1.05 (t, 3H), 0.92 (d, 6H) ppm | - |
| 156 | N-{4-[3-(4-Fluorphenyl)-1-(propan-2-yl)-1H-pyrazol-4-yl]pyridin-2-yl}cyclobutancarboxamid | 1H-NMR (400 MHz, d₃-CD₃CN): δ = 8.35 (s, 1H, br), 8.12 (s, 1H), 8.10 (m, 1H), 7.88 (s, 1H), 7.48-7.45 (m, 2H), 7.12-7.08 (m, 2H), 6.86 (dd, 1H), 4.55 (m, 1H), 3.25 (m, 1H), 2.03-2.15 (m, 6H), 1.53 (d, 6H) ppm | - |
| 157 | N-{4-[1-(Butan-2-yl)-3-(4-fluorphenyl)-1H-pyrazol-4-yl]pyridin-2-yl} -2-methylpropanamid | 1H-NMR (400 MHz, d₆-DMSO): δ = 10.39 (s, 1H), 8.19 (s, 1H), 8.15 (m, 1H), 8.11 (s, 1H), 7.44-7.42 (m, 2H), 7.20-7.18 (m, 2H), 6.82-6.81 (dd, 1H), 4.35 (m, 1H), 2.74 (m, 1H), 1.90-1.80 (m, 1H), 1.48 (d, 2H), 1.06 (d, 6H), 0.80 (t, 3H) ppm | - |
| 158 | 2-Cyclopropyl-N-{4-[3-(4-fluorphenyl)-1-(2-methylpropyl)-1H-pyrazol-4-yl]pyridin-2-yl}acetamid | 1H-NMR (400 MHz, d₆-DMSO): δ = 10.32 (s, 1H), 8.16-8.14 (m, 2H), 8.09 (s, 1H), 7.44-7.42 (m, 2H), 7.20-7.18 (m, 2H), 6.83-6.82 (dd, 1H), 3.99 (d, 2H), 2.24 (d, 2H), 2.20 (m, 1H), 1.05 (m, 1H), 0.91 (d, 6H), 0.45 (m, 2H), 0.16 (m, 2H) ppm | - |
| 159 | N-{4-[1-(Butan-2-yl)-3-(4-fluorphenyl)-1H-pyrazol-4-yl]pyridin-2-yl}-2-cyclopropylacetamid | 1H-NMR (400 MHz, d₆-DMSO): δ = 10.31 (s, 1H), 8.19 (s, 1H), 8.16 (d, 1H), 8.09 (s, 1H), 7.44-7.42 (dd, 2H), 7.20-7.18 (m, 2H), 6.84-6.83 (dd, 1H), 4.33 (m, 1H), 2.23 (d, 2H), 1.90 (m, 1H), 1.80 (m, 1H), 1.48 (d, 3H), 1.05 (m, 1H), 0.80 (t, 3H), 0.45 (m, 2H), 0.17 (m, 2H) ppm | - |
| 160 | N-{4-[3-(4-Fluorphenyl)-1-(propan-2-yl)-1H-pyrazol-4-yl]pyridin-2-yl}-3-methylbutanamid | 1H-NMR (400 MHz, d₃-CD₃CN): δ = 8.50 (s, 1H, br), 8.10 (m, 2H), 7.89 (s, 1H), 7.47-7.45 (m, 2H), 7.09-7.08 (m, 2H), 6.88 (dd, 1H), 4.55 (m, 1H), 2.21 (d, 2H), 2.10 (m, 1H), 1.53 (d, 6H), 0.95 (d, 6H) ppm | - |
| 161 | N- {4-[3-(4-Fluorphenyl)-1-(propan-2-yl)-1H-pyrazol-4-yl]pyridin-2-yl}acetamid | 1H-NMR (400 MHz, d₃-CD₃CN): δ = 8.59 (s, 1H, br), 8.11 (m, 1H), 8.05 (s, 1H), 7.88 (s, 1H), 7.47-7.45 (m, 2H), 7.11-7.08 (m, 2H), 6.88 (dd, 1H), 4.55 (m, 1H), 2.08 (s, 3H), 1.52 (d, 6H) ppm | - |
| 162 | N-{4-[1-(Butan-2-yl)-3-(4-fluorphenyl)-1H-pyrazol-4-yl]pyridin-2-yl}-2-methoxyacetamid | 1H-NMR (400 MHz, d₆-DMSO): δ = 9.93 (s, 1H), 8.23 (s, 1H), 8.20 (d, 1H), 8.03 (s, 1H), 7.44-7.40 (m, 2H), 7.22-7.19 (m, 2H), 6.94 (d, 1H), 4.35 (m, 1H), 4.03 (s, 2H), 1.90 (m, 1H), 1.80 (m, 1H), 1.49 (d, 3H), 1.04 (t, 3H), 0.80 (t, 3H) ppm | - |
| 163 | N-{4-[3-(4-Fluorphenyl)-1-(2-methoxyethyl)-1H-pyrazol-4-yl]pyridin-2-yl}-2-methoxyacetamid | 1H-NMR (400 MHz, d₃-CD₃CN): δ = 8.70 (s, 1H, br), 8.15 (d, 1H), 8.08 (s, 1H), 7.88 (s, 1H), 7.47-7.45 (m, 2H), 7.12-7.10 (m, 2H), 6.95 (dd, 1H), 4.32 (t, 2H), 3.96 (s, 2H), 3.78 (t, 2H), 3.45 (s, 3H), 3.32 (s, 3H) ppm | - |
| 164 | N-{4-[1-(Butan-2-yl)-3-(4-fluorphenyl)-1H-pyrazol-4-yl]pyridin-2-yl}propanamid | 1H-NMR (400 MHz, d₆-DMSO): δ = 10.39 (s, 1H), 8.19 (s, 1H), 8.17 (d, 1H), 8.09 (s, 1H), 7.44-7.42 (m, 2H), 7.22-7.19 (m, 2H), 6.84-6.83 (dd, 1H), 4.34 (m, 1H), 2.38 (q, 2H), 1.90 (m, 1H), 1.80 (m, 1H), 1.49 (d, 3H), 1.04 (t, 3H), 0.80 (t, 3H) ppm | - |
| 165 | N-{4-[3-(4-Fluorphenyl)-1-(2-methylpropyl)-1H-pyrazol-4-yl]pyridin-2-yl}cyclopropancarboxamid | 1H-NMR (400 MHz, d₆-DMSO): δ = 10.76 (s, 1H), 8.17 (d, 1H), 8.13 (s, 1H), 8.08 (s, 1H), 7.44-7.42 (m, 2H), 7.22-7.19 (m, 2H), 6.83-6.81 (dd, 1H), 3.99 (d, 2H), 2.20 (m, 1H), 1.98 (m, 1H), 0.91 (m, 6H), 0.78 (m, 4H) ppm | - |
| 166 | N-{4-[3-(4-Fluorphenyl)-1-(2-methylpropyl)-1H-pyrazol-4-yl]pyridin-2-yl}-2-methoxyacetamid | 1H-NMR (400 MHz, d₆-DMSO): δ = 9.94 (s, 1H), 8.21-8.19 (m, 1H), 8.04 (s, 1H), 7.45-7.43 (m, 2H), 7.22 (m, 2H), 6.83-6.81 (dd, 1H), 4.03 (s, 2H), 3.99 (d, 2H), 2.20 (m, 1H), 0.91 (m, 6H) ppm | - |
| 167 | N-{4-[1-(Butan-2-yl)-3-(4-fluorphenyl)-1H-pyrazol-4-yl]pyridin-2-yl}cyclopropancarboxamid | 1H-NMR (400 MHz, d₆-DMSO): δ = 10.76 (s, 1H), 8.17 (m, 2H), 8.07 (s, 1H), 7.44-7.42 (dd, 2H), 7.24-7.20 (m, 2H), 6.83-6.82 (dd, 1H), 4.35 (m, 1H), 2.00 (m, 1H), 1.90 (m, 1H), 1.80 (m, 1H), 1.40 (d, 3H), 0.80 (m, 7H) ppm | - |
| 169 | 4-[1-(2,2-Difluorethyl)-3-(4-fluorphenyl)-1H-pyrazol-4-yl]pyridin | 1H-NMR (400 MHz, d₃-CD₃CN): δ = 8.46 (dd, 1H), 7.93 (s, 1H), 7.46-7.44 (m, 2H), 7.19 (m, 1H), 7.14-7.10 (m, 2H), 6.27 (tt, 2J(H,F)= 55Hz, 1H), 4.59 (dt, 3J(H,F)= 15Hz, 2H) ppm | - |
| 170 | Ethyl-[3-(4-fluorphenyl)-4-(pyridin-4-yl)-1H-pyrazol-1-yl]acetat | 1H-NMR (400 MHz, d₃-CD₃CN): δ = 8.45 (d, 2H), 7.91 (s, 1H), 7.45 (m, 2H), 7.21 (d, 2H), 7.14-7.08 (m, 2H), 4.99 (s, 2H), 4.21 (q, 2H), 1.28 (t, 3H) ppm | - |
| 171 | 4-[3-(4-Fluorphenyl)-5-methyl-1-(prop-2-in-1-yl)-1H-pyrazol-4-yl]pyridin | 1H-NMR (400 MHz, d₆-DMSO): δ = 8.56 (d, 2H), 8.46 (d, 2H, isomer), 7.58 (t, 2H), 7.32 (m, 2H), 7.17 (m, 2H), 5.77 (d, 2H), 5.50 (d, 2H, isomer), 2.34 (s, 3H), 2.30 (s, 1H) ppm | 62/19 |
| 172 | 4-[3-(4-Fluorphenyl)-1H-pyrazol-4-yl]-N-(propan-2-yl)pyrimidin-2-amin | ¹H-NMR (400 MHz, d₆-DMSO): δ = 13.28 (bs, 1H), 8.12 (d, 1H), 7.59(bs, 2H), 7.28 (bs, 2H), 6.77 (d, 1H), 6.52 (bs, 1H), 3.81 (bs, 1H), 1.09 (bs, 6H) ppm. | - |
| 173 | 4-[3-(4-Fluorphenyl)-1H-pyrazol-4-yl]-N-(prop-2-in-1-yl)pyrimidin-2-amin | 1 H-NMR (400 MHz, CDCl₃): δ = 8.15 (s, 1H), 8.06 (d, 1H), 7.46 (m, 2H), 7.09 (t, 2H), 6.49 (d, 1H), 4.05 (bs, 2H), 2.15 (s, 1H) ppm | - |
| 174 | 4-[3-(4-Fluorphenyl)-1-(2-methylpropyl)-1H-pyrazol-4-yl]-N-(propan-2-yl)pyrimidin-2-amin | 1H-NMR (400 MHz, d₆-DMSO): δ = 8.11 (s, 1H), 8.00 (d, 1H), 7.43 (m, 2H), 7.33 (t, 2H), 6.65 (d, 1H), 6.40 (bs, 1H), 3.70 (d, 2H), 2.02 (m, 1H), 0.98 (bs, 6H), 0.72 (d, 6H) ppm | - |
| 175 | 3-(4-Fluorphenyl)-4-(pyridin-4-yl)-1 H-pyrazol-5-carbonitril | 1H-NMR (400 MHz, d₆-DMSO): δ = 8.62 (d, 2H), 7.46 (m, 2H), 7.631 (m, 4H) ppm | - |
| 176 | 4-{1-[1-(2-Chlor-1,3-thiazol-5-yl)ethyl]-3-(4-fluorphenyl)-1H-pyrazol-4-yl}pyridin | 1H-NMR (400 MHz, d₃-CD₃CN): δ = 8.45 (m, 2H), 7.98 (s, 1H), 7.57 (s, 1H), 7.48-7.43 (m, 2H), 7.18 (m, 2H), 7.14-7.08 (m, 2H), 5.88 (q, 1H), 2.15 (m, 3H) ppm | - |
| 178 | 1-[3-(4-Fluorphenyl)-4-(pyridin-4-yl)-1H-pyrazol-1-yl]-3-methylbutan-2-on | 1H-NMR (400 MHz, d₃-CD₃CN): δ = 8.45 (m, 2H), 8.10 (s, 1H), 7.45 (m, 2H), 7.25 (m, 2H), 7.15 (m, 2H), 1.75 (d, 6H) ppm | - |
| 179 | 4-[1-(2-Ethoxyethyl)-3-(4-fluorphenyl)-1H-pyrazol-4-yl]pyridin | 1H-NMR (400 MHz, d₃-CD₃CN): δ = 8.45 (d, 2H), 7.89 (s, 1H), 7.45 (m, 2H), 7.18 (d, 2H), 7.14-7.08 (m, 2H), 4.30 (t, 2H), 3.82 (t, 2H), 3.49 (q, 2H), 1.12 (t, 3H) ppm | - |
| 180 | [3-(4-Fluorphenyl)-4-(pyridin-4-yl)-1H-pyrazol-1-yl] acetonitril | 1H-NMR (400 MHz, d₃-CD₃CN): δ = 8.46 (dd, 2H), 7.96 (s, 1H), 7.47-7.44 (m, 2H), 7.20 (d, 2H), 7.14-7.11 (m, 2H), 5.23 (s, 2H) ppm | - |
| 181 | Propan-2-yl-{4-[3-(4-fluorphenyl)-1-(propan-2-yl)-1H-pyrazol-4-yl]pyridin-2-yl} carbamat | 1H-NMR (400 MHz, d7-DMF): δ = 8.16 (d, 1H), 8.02 (m, 2H), 7.93 (s, 1H), 7.57-7.53 (m, 2H), 7.25-7.21 (m, 2H), 6.92 (dd, 1H), 4.90 (m, 1H), 4.67 (m, 1H), 3.20 (m, 6H), 1.57 (d, 6H) ppm | - |
| 182 | N-Benzyl-4-[3-(4-fluorphenyl)-1-(2-methylpropyl)-1H-pyrazol-4-yl]pyrimidin-2-amin | 1H-NMR (400 MHz, d₆-DMSO): δ = 8.30 (bs, 1H), 8.12 (d, 1H), 7.56 (m, 3H), 7.25 (t, 7H), 6.49 (bs, 1H), 4.37 (bs, 2H), 4.01 (d, 2H), 2.18 (m, 1H), 0.89 (d, 6H) ppm | - |
| 184 | 4-[3-(4-Fluorphenyl)-1-(2-methylpropyl)-1H-pyrazol-4-yl]pyrimidin-2-amin | 1H-NMR (400 MHz, CDCl₃): δ = 8.01 (d, 1H), 7.89 (s, 1H), 7.45 (m, 2H), 7.02 (t, 2H), 6.36 (d, 1H), 4.94 (bs, 2H), 3.89 (d, 2H), 2.23 (m, 1H), 0.91 (d, 6H) ppm | - |
| 185 | N- {4-[3-(4-Fluorphenyl)-1-(2-methylpropyl)-1H-pyrazol-4-yl]pyrimidin-2-yl} -2-methyl-N-(propan-2-yl)propanamid | 1H-NMR (400 MHz, CDCl₃): δ = 8.37 (d, 2H), 7.98 (s, 1H), 7.44 (m, 2H), 7.07 (d, 2H), 6.87 (d, 1H), 4.85 (m, 1H), 3.92 (d, 2H), 2.48 (m, 1H), 2.24 (m, 1H), 1.18 (d, 6H), 1.03 (d, 6H), 0.92 (d, 6H) ppm | - |
| 186 | N-{4-[3-(4-Fluorphenyl)-1-(2-methylpropyl)-1H-pyrazol-4-yl]pyrimidin-2-yl}-2,2,4-trimethyl-3-oxo-N-(propan-2-yl)pentanamid | 1H-NMR (400 MHz, CDCl₃): δ = 8.09 (bs, 1H), 7.93 (s, 1H), 7.41 (m, 2H), 7.05 (t, 2H), 6.8 (d, 1H), 4.85 (m, 1H), 3.92 (d, 2H), 3.05 (m, 1H), 2.25 (m, 1H), 1.23 (d, 6H), 1.1 (d, 6H), 1.04 (s, 6H), 0.93 (d, 6H) ppm | - |
| 187 | 1-{4-[3-(4-Fluorphenyl)-1-(2-methylpropyl)-1H-pyrazol-4-yl]pyridin-2-yl}pyrrolidin-2-on | 1H-NMR (400 MHz, d₆-DMSO): δ = 8.26 (d, 2H), 8.16 (s, 1H), 7.43 (m, 2H), 7.20 (t, 2H), 6.93 (m, 1H), 3.98 (m, 4H), 2.54 (m, 2H), 2,20 (m, 1H), 2.02 (m, 2H), 0.91 (d, 6H) ppm | - |
| 188 | N-{4-[3-(4-Fluorphenyl)-1-(2-methylpropyl)-1H-pyrazol-4-yl]pyrimidin-2-yl}-2-methyl-N-(2-methylpropanoyl)propanamid | 1H-NMR (400 MHz, CDCl₃): δ = 8.44 (d, 1H), 7.98 (s, 1H), 7.44 (m, 2H), 7.06 (m, 2H), 6.95 (d, 1H), 3.90 (d, 2H), 2.87 (m, 2H), 2.23 (m, 1H), 1.14 (d, 12H), 0.91 (d, 6H) ppm | - |
| 189 | N-{4-[3-(4-Fluorphenyl)-1-(2-methylpropyl)-1H-pyrazol-4-yl]pyrimidin-2-yl}-2-methylpropanamid | 1H-NMR (400 MHz, CDCl₃): δ = 8.25 (d, 2H), 8.05 (s, 1H), 8.00 (s, 1H), 7.42 (m, 2H), 7.04 (d, 2H), 6.98 (d, 1H), 3.90 (d, 2H), 2.9 (m, 1H), 2.24 (m, 1H), 1.2 (d, 6H), 0.91 (d, 6H) ppm | - |
| 191 | N-{4-[3-(4-Fluorphenyl)-1-(2-methylpropyl)-1H-pyrazol-4-yl]pyridin-2-yl}-2-phenylacetamid | 1H-NMR (400 MHz, d₃-CD₃CN): δ = 8.61 (s, 1H, br), 8.10 (d, 1H), 8.09 (s, 1H), 7.82 (s, 1H), 7.45-7.43 (m, 2H), 7.35-7.28 (m, 5H), 7.09-7.06 (m, 2H), 6.88 (d, 1H), 3.96 (d, 2H), 3.69 (s, 2H), 2.20 (m, 1H), 0.93 (d, 6H) ppm | - |
| 192 | (2S)-N-{4-[3-(4-Fluorphenyl)-1-(propan-2-yl)-1H-pyrazol-4-yl]pyridin-2-yl}-2-hydroxypropanamid | 1H-NMR (400 MHz, d₃-CD₃CN): δ = 8.98 (s, 1H, br), 8.15-8.10 (m, 2H), 7.90 (s, 1H), 7.48-7.45 (m, 2H), 7.11-7.08 (m, 2H), 6.92 (dd, 1H), 4.56 (m, 1H), 4.22 (m, 1H), 4.05 (s, 1H), 1.53 (d, 6H) ppm | - |
| 193 | tert-Butyl-4-{3-(4-fluorphenyl)-4-[2-(propanoylamino)pyridin-4-yl]-1H-pyrazol-1-yl}piperidin-1-carboxylat | 1H-NMR (400 MHz, d₃-CD₃CN): δ = 8.50 (s, 1H, br), 8.10 (m, 2H), 7.89 (s, 1H), 7.47-7.45 (m, 2H), 7.11-7.08 (m, 2H), 6.92 (dd, 1H), 4.38 (m, 1H), 4.18 (m, 2H), 2.90 (m, 2H), 2.37 (q, 2H), 2.10 (m, 2H), 1.90 (m, 2H), 1.45 (s, 9H), 1.10 (t, 3H) ppm | - |
| 195 | N-{4-[3-(4-Chlor-2-fluorphenyl)-1-methyl-5-(methylsulfanyl)-1H-pyrazol-4-yl]pyridin-2-yl}propanamid | 1H-NMR (400 MHz, d₃-CD₃CN): δ = 8.53 (s, 1H, br), 8.15-8.13 (m, 3H), 7.42-7.40 (m, 2H), 7.25 (d, 1H), 7.15 (d, 1H), 6.85 (dd, 1H), 4.05 (s, 3H), 2.38 (q, 2H), 2.30 (s, 3H), 1.10 (t, 3H) ppm | - |
| 196 | N-{4-[5-(Difluormethoxy)-3-(4-fluor-2-hydroxyphenyl)-1-methyl-1H-pyrazol-4-yl]pyridin-2-yl}propanamid | 1H-NMR (400 MHz, d₃-CD₃CN): δ = 10.2 (s, 1H), 8.63 (s, 1H, br), 8.26 (d, 1H), 8.13 (s, 1H), 7.03 (dd, 1H), 6.95 (m, 1H), 6.70 (m, 1H), 6.55 (t, 1H), 6.45 (m, 1H), 3.82 (s, 3H), 2.41 (q, 2H), 1.12 (t, 3H) ppm | - |
| 197 | N-{4-[3-(4-Chlor-2-fluorphenyl)-1-methyl-5-(trifluormethyl)-1H-pyrazol-4-yl]pyridin-2-yl}propanamid | 1H-NMR (400 MHz, d₃-CD₃CN): δ = 8.55 (s, 1H, br), 8.17 (d, 1H), 8.01 (s, 1H), 7.36 (dd, 1H), 7.21 (d, 1H), 7.18 (d, 1H), 6.82 (dd, 1H), 4.10 (s, 3H), 2.37 (q, 2H), 1.09 (t, 3H) ppm | - |
| 198 | N-{4-[1-Ethyl-3-(4-fluorphenyl)-1H-pyrazol-4-yl]pyridin-2-yl}propanamid | 1H-NMR (400 MHz, d₃-CD₃CN): δ = 8.50 (s, 1H, br), 8.10 (m, 2H), 7.86 (s, 1H), 7.47-7.45 (m, 2H), 7.11-7.08 (m, 2H), 6.86 (dd, 1H), 4.21 (q, 2H), 2.38 (q, 2H), 1.49 (t, 3H), 1.10 (t, 3H) ppm | - |
| 199 | N-{4-[3-(4-Cyan-2,5-difluorphenyl)-5-(difluormethoxy)-1-methyl-1H-pyrazol-4-yl]pyridin-2-yl}-2-methoxyacetamid | 1H-NMR (400 MHz, d₃-CD₃CN): δ = 8.70 (s, 1H, br), 8.20 (d, 1H), 7.99 (s, 1H), 7.50-7.45 (m, 2H), 6.94 (dd, 1H), 6.65 (t, 2J(H,F), 1H), 3.96 (s, 2H), 3.86 (s, 3H), 3.44 (s, 3H) ppm | - |
| 200 | N-{4-[3-(4-Chlor-2-fluorphenyl)-1-methyl-5-(methylsulfanyl)-1H-pyrazol-4-yl]pyridin-2-yl}-2-methoxyacetamid | 1H-NMR (400 MHz, d₃-CD₃CN): δ = 8.73 (s, 1H, br), 8.18-8.15 (m, 2H), 7.40 (m, 1H), 7.25 (d, 1H), 7.15 (d, 1H), 6.90 (m, 1H), 4.03 (s, 3H), 3.97 (s, 2H), 3.45 (s, 3H), 2.30 (s, 3H) ppm | - |
| 201 | N-{4-[5-(Difluormethoxy)-3-(4-fluor-2-hydroxyphenyl)-1-methyl-1H-pyrazol-4-yl]pyridin-2-yl}-2-methoxyacetamid | 1H-NMR (400 MHz, d₃-CD₃CN): δ = 8.80 (s, 1H, br), 8.30 (d, 1H), 8.12 (s, 1H), 7.03-7.00 (m, 2H), 6.70 (m, 1H), 6.58 (t, 2J(H,F), 1H), 6.48 (m, 1H), 3.99 (s, 2H), 3.84 (s, 3H), 3.46 (s, 3H) ppm | - |
| 202 | N-{4-[3-(4-Chlor-2-fluorphenyl)-1-methyl-5-(trifluormethyl)-1H-pyrazol-4-yl]pyridin-2-yl}-2-methoxyacetamid | 1H-NMR (400 MHz, d₃-CD₃CN): δ = 8.78 (s, 1H, br), 8.21 (d, 1H), 8.01 (s, 1H), 7.36 (dd, 1H), 7.21 (d, 1H), 7.16 (d, 1H), 6.89 (dd, 1H), 4.10 (s, 3H), 3.96 (s, 2H), 3.45 (s, 3H) ppm | - |
| 203 | N-{4-[1-Ethyl-3-(4-fluorphenyl)-1H-pyrazol-4-yl]pyridin-2-yl}-2-methoxyacetamid | 1H-NMR (400 MHz, d₃-CD₃CN): δ = 8.70 (s, 1H, br), 8.14 (d, 1H), 8.08 (s, 1H), 7.88 (s, 1H), 7.47-7.45 (m, 2H), 7.12-7.10 (m, 2H), 6.94 (dd, 1H), 4.21 (q, 2H), 3.96 (s, 2H), 3.45 (s, 3H), 1.50 (t, 3H) ppm | - |
| 204 | tert-Butyl-4-[3-(4-fluorphenyl)-4-{2-[(methoxyacetyl)amino]pyridin-4-yl}-1H-pyrazol-1-yl]piperidin-1-carboxylat | 1H-NMR (400 MHz, d₃-CD₃CN): δ = 8.70 (s, 1H, br), 8.12 (d, 1H), 8.07(s, 1H), 7.92 (s, 1H), 7.45-7.41 (m, 2H), 7.08 (m, 2H), 6.95 (d, 1H), 4.38 (m, 1H), 4.15 (m, 2H), 3.96 (s, 2H), 3.45 (s, 3H), 2.1 (m, 2H), 1.90 (m, 2H), 1.45 (s, 9H) ppm | - |
| 205 | N-{4-[3-(4-Fluorphenyl)-1-(propan-2-yl)-1H-pyrazol-4-yl]pyridin-2-yl}benzamid | 1H-NMR (400 MHz, d₃-CD₃CN): δ = 8.95 (s, 1H, br), 8.26 (s, 1H), 8.18 (d, 1H), 7.93 (m, 3H), 7.60 (m, 1H), 7.51-7.49 (m, 4H), 7.13-7.10 (m, 1H), 6.96-6.95 (dd, 1H), 4.57 (m, 1H), 1.54 (d, 6H) ppm | - |
| 206 | N-{4-[3-(4-Fluorphenyl)-1-(propan-2-yl)-1H-pyrazol-4-yl]pyridin-2-yl}thiophen-2-carboxamid | 1H-NMR (400 MHz, d₃-CD₃CN): δ = 8.93 (s, 1H, br), 8.18 (m, 2H), 7.93 (s, 1H), 7.84 (m, 1H), 7.70 (dd, 1H), 7.50-7.48 (m, 2H), 7.18-7.16 (dd, 1H), 7.12-7.10 (m, 2H), 6.95 (dd, 1H), 4.56 (m, 1H), 1.54 (d, 6H) ppm | - |
| 207 | N-{4-[3-(4-Fluorphenyl)-1-(propan-2-yl)-1H-pyrazol-4-yl]pyridin-2-yl}thiophen-3 -carboxamid | 1H-NMR (400 MHz, d₃-CD₃CN): δ = 8.80 (s, 1H, br), 8.21 (s, 1H), 8.18 (m, 1H), 7.93 (s, 1H), 7.56 (m, 1H), 7.50-7.48 (m, 3H), 7.12-7.09 (m, 2H), 6.94 (dd, 1H), 4.55 (m, 1H), 1.53 (d, 6H) ppm | - |
| 208 | N-{4-[3-(4-Fluorphenyl)-1-(propan-2-yl)-1H-pyrazol-4-yl]pyridin-2-yl}-2-(2-methoxyethoxy)acetamid | 1H-NMR (400 MHz, d₃-CD₃CN): δ = 9.18 (s, 1H, br), 8.16 (d, 1H), 8.11 (s, 1H), 7.90 (s, 1H), 7.48-7.46 (m, 2H), 7.11-7.08 (m, 2H), 6.94 (dd, 1H), 4.55 (m, 1H), 4.04 (s, 2H), 3.71 (t, 2H), 3.56 (t, 2H), 3.38 (s, 3H), 1.53 (d, 6H) ppm | - |
| 209 | N-{4-[3-(4-Fluorphenyl)-1-(propan-2-yl)-1H-pyrazol-4-yl]pyridin-2-yl}-2-hydroxy-2-methylpropanamid | 1H-NMR (400 MHz, d₃-CD₃CN): δ = 9.12 (s, 1H, br), 8.13 (m, 2H), 7.90 (s, 1H), 7.48-7.46 (m, 2H), 7.11-7.09 (m, 2H), 6.91 (dd, 1H), 4.55 (m, 1H), 3.90 (s, 1H, br), 1.53 (d, 6H), 1.40 (s, 6H) ppm | - |
| 210 | N-{4-[3-(4-Fluorphenyl)-1-(propan-2-yl)-1H-pyrazol-4-yl]pyridin-2-yl}-2,3-dihydroxypropanamid | 1H-NMR (400 MHz, d₃-CD₃CN): δ = 9.01 (s, 1H, br), 8.14 (m, 2H), 7.91 (s, 1H), 7.48-7.46 (m, 2H), 7.11-7.09 (m, 2H), 6.93 (dd, 1H), 4.56 (m, 1H), 4.15 (m, 1H), 4.10 (s, 1H, br), 3.74 (m, 2H), 3.10 (s, 1H, br), 1.53 (d, 6H) ppm | - |
| 211 | 1-Ethyl-3-}4-[3-(4-fluorphenyl)-1-(propan-2-yl)-1H-pyrazol-4-yl]pyridin-2-yl}harnstoff | 1H-NMR (400 MHz, d₃-CD₃CN): δ = 8.82 (s, 1H, br), 8.05 (d, 1H), 7.86 (s, 1H), 7.49-7.45 (m, 2H), 7.13-7.10 (m, 2H), 6.81 (s, 1H), 6.78 (m, 1H), 4.56 (m, 1H), 3.25 (q, 2H), 1.52 (d, 6H), 1.15 (t, 3H) ppm | - |
| 212 | N-{4-[3-(4-Fluorphenyl)-1-(2-methoxyethyl)-1H-pyrazol-4-yl]pyridin-2-yl}-2-phenylacetamid | 1H-NMR (400 MHz, d₃-CD₃CN): δ = 8.60 (s, 1H, br), 8.10 (d, 1H), 8.09 (s, 1H), 7.84 (s, 1H), 7.45 (dd, 2H), 7.38-7.30 (m, 5H), 7.10 (t, 2H), 6.88 (d, 1H), 4.29 (t, 2H), 3.77 (t, 2H), 3.69 (s, 2H), 3.30 (s, 3H) ppm | - |
| 213 | N-{4-[1-(2-Fluorbenzyl)-3-(4-fluorphenyl)-1H-pyrazol-4-yl]pyridin-2-yl}propanamid | 1H-NMR (400 MHz, d₆-DMSO): δ = 10.41 (s, 1H), 8.27 (s, 1H), 8.18 (d, 1H), 8.09 (s, 1H), 7.43-7.37 (m, 4H), 7.30-7.18 (m, 4H), 6.83 (dd, 1H), 5.48 (s, 2H), 2.38 (q, 2H), 1.05 (t, 3H) ppm | - |
| 214 | N-{4-[1-(Cyclopropylmethyl)-3-(4-fluorphenyl)-1H-pyrazol-4-yl]pyridin-2-yl}propanamid | 1H-NMR (400 MHz, d₃-CD₃CN): δ = 8.57 (s, 1H, br), 8.10 (m, 2H), 7.93 (s, 1H), 7.48-7.45 (m, 2H), 7.12-7.09 (m, 2H), 6.87 (dd, 1H), 4.03 (d, 2H), 2.38 (q, 2H), 1.36 (m, 1H), 1.11 (t, 3H), 0.62 (m, 2H), 0.44 (m, 2H) ppm | - |
| 215 | N-{4-[1-(2,2-Difluorethyl)-3-(4-fluorphenyl)-1H-pyrazol-4-yl]pyridin-2-yl}propanamid | 1H-NMR (400 MHz, d₃-CD₃CN): δ = 8.60 (s, 1H, br), 8.12-8.10 (m, 2H), 7.93 (s, 1H), 7.48-7.45 (m, 2H), 7.13-7.10 (m, 2H), 6.86 (dd, 1H), 6.28 (tt, 2J(F,H)=54 Hz, 1H), 4.59 (dt, 3J(F,H)=14Hz, 2H), 2.39 (q, 2H), 1.11 (t, 3H) ppm | - |
| 216 | N-{4-[1-(2,2-Difluorethyl)-3-(4-fluorphenyl)-1H-pyrazol-4-yl]pyridin-2-yl}-2-methoxyacetamid | 1H-NMR (400 MHz, d₃-CD₃CN): δ = 8.76 (s, 1H, br), 8.17 (d, 2H), 8.09 (s, 1H), 7.96 (s, 1H), 7.48-7.46 (m, 2H), 7.13-7.10 (m, 2H), 6.95 (dd, 1H), 6.28 (tt, 2J(F,H)=54 Hz, 1H), 4.59 (dt, 3J(F,H)=14Hz, 2H), 3.97 (s, 2H), 3.45 (s, 3H) ppm | - |
| 217 | N-{4-[1-(2,2-Difluorethyl)-3-(4-fluorphenyl)-1H-pyrazol-4-yl]pyridin-2-yl}cyclopropancarboxamid | 1H-NMR (400 MHz, d₃-CD₃CN): δ = 8.91 (s, 1H, br), 8.13 (dd, 2H), 8.09 (s, 1H), 7.91 (s, 1H), 7.47-7.44 (m, 2H), 7.11-7.08 (m, 2H), 6.85 (dd, 1H), 6.27 (tt, 2J(F,H)=59 Hz, 1H), 4.59 (dt, 3J(F,H)=14Hz, 2H), 1.77 (m, 1H), 0.90-0.80 (m, 4H) ppm | - |
| 218 | N-{4-[1-(2,2-Difluorethyl)-3-(4-fluorphenyl)-1H-pyrazol-4-yl]pyridin-2-yl} -2-methylpropanamid | 1H-NMR (400 MHz, d₃-CD₃CN): δ = 8.58 (s, 1H, br), 8.12 (m, 2H), 7.93 (s, 1H), 7.47-7.45 (m, 2H), 7.12-7.09 (m, 2H), 6.86 (dd, 1H), 6.27 (tt, 2J(F,H)=55 Hz, 1H), 4.58 (dt, 3J(F,H)=14Hz, 2H), 2.62 (m, 1H), 1.14 (d, 6H) ppm | - |
| 219 | N-{4-[1-(2-Chlorethyl)-3-(4-fluorphenyl)-1H-pyrazol-4-yl]pyridin-2-yl}propanamid | 1H-NMR (400 MHz, d₃-CD₃CN): δ = 8.58 (s, 1H, br), 8.11 (m, 2H), 7.93 (s, 1H), 7.49-7.45 (m, 2H), 7.15-7.08 (m, 2H), 6.86 (dd, 1H), 4.48 (t, 2H), 4.02 (t, 2H), 2.38 (q, 2H), 1.11 (t, 3H) ppm | - |
| 220 | N-{4-[1-(2-Chlorethyl)-3-(4-fluorphenyl)-1H-pyrazol-4-yl]pyridin-2-yl}-2-methoxyacetamid | 1H-NMR (400 MHz, d₃-CD₃CN): δ = 8.74 (s, 1H, br), 8.16 (dd, 1H), 8.09 (s, 1H), 7.95 (s, 1H), 7.49-7.46 (m, 2H), 7.12-7.09 (m, 2H), 6.95 (dd, 1H), 4.49 (t, 2H), 4.02 (t, 2H), 3.96 (s, 2H), 3.45 (s, 3H) ppm | - |
| 221 | N-{4-[1-(2-Chlorethyl)-3-(4-fluorphenyl)-1H-pyrazol-4-yl]pyridin-2-yl}cyclopropancarboxamid | 1H-NMR (400 MHz, d₃-CD₃CN): δ = 8.90 (s, 1H, br), 8.12 (d, 1H), 8.09 (s, 1H), 7.90 (s, 1H), 7.47-7.45 (m, 2H), 7.12-7.08 (m, 2H), 6.86 (dd, 1H), 4.48 (t, 2H), 4.01 (t, 2H), 1.77 (m, 1H), 0.88-0.81 (m, 4H) ppm | - |
| 222 | N-{4-[1-(2-Chlorethyl)-3-(4-fluorphenyl)-1H-pyrazol-4-yl]pyridin-2-yl} -2-methylpropanamid | 1H-NMR (400 MHz, d₃-CD₃CN): δ = 8.58 (s, 1H, br), 8.13-8.11 (m, 2H), 7.93 (s, 1H), 7.48-7.46 (m, 2H), 7.12-7.09 (m, 2H), 6.86 (dd, 1H), 4.49 (t, 2H), 4.02 (t, 2H), 2.62 (m, 1H), 1.14 (d, 6H) ppm | - |
| 223 | N-{4-[1-(Cyclopropylmethyl)-3-(4-fluorphenyl)-1H-pyrazol-4-yl]pyridin-2-yl}-2-methoxyacetamid | 1H-NMR (400 MHz, d₃-CD₃CN): δ = 8.73 (s, 1H, br), 8.15 (d, 1H), 8.09 (s, 1H), 7.95 (s, 1H), 7.48-7.46 (m, 2H), 7.12-7.09 (m, 2H), 6.96 (dd, 1H), 4.03 (d, 2H), 3.97 (s, 2H), 3.45 (s, 3H), 1.36 (m, 1H), 0.63 (m, 2H), 0.43 (m, 2H) ppm | - |
| 224 | N-{4-[1-(Cyclopropylmethyl)-3-(4-fluorphenyl)-1H-pyrazol-4-yl]pyridin-2-yl}cyclopropancarboxamid | 1H-NMR (400 MHz, d₃-CD₃CN): δ = 8.92 (s, 1H, br), 8.11 (d, 1H), 8.10 (s, 1H), 7.91 (s, 1H), 7.47-7.45 (m, 2H), 7.11-7.08 (m, 2H), 6.86 (dd, 1H), 4.02 (d, 2H), 1.78 (m, 1H), 1.34 (m, 1H), 0.85-0.80 (m, 4H), 0.62 (m, 2H), 0.43 (m, 2H) ppm | - |
| 225 | N-{4-[1-(Cyclopropylmethyl)-3-(4-fluorphenyl)-1H-pyrazol-4-yl]pyridin-2-yl} -2-methylpropanamid | 1H-NMR (400 MHz, d₃-CD₃CN): δ = 8.60 (s, 1H, br), 8.12 (s, 1H), 8.10 (d, 1H), 7.93 (s, 1H), 7.48-7.45 (m, 2H), 7.11-7.08 (m, 2H), 6.86 (dd, 1H), 4.03 (d, 2H), 2.63 (m, 1H), 1.38 (m, 1H), 1.14 (d, 6H), 0.62 (m, 2H), 0.43 (m, 2H) ppm | - |
| 226 | N-{4-[3-(4-Fluorphenyl)-1-(propan-2-yloxy)-1H-pyrazol-4-yl]pyridin-2-yl}propanamid | 1H-NMR (400 MHz, d₃-CD₃CN): δ = 8.54 (s, 1H, br), 8.13 (d, 1H), 8.09 (s, 1H), 7.80 (s, 1H), 7.47-7.44 (m, 2H), 7.11-7.08 (m, 2H), 6.89 (dd, 1H), 4.74 (m, 1H), 2.38 (q, 2H), 1.33 (d, 6H), 1.10 (t, 3H) ppm | - |
| 227 | 4-[3-(4-Fluorphenyl)-1-(propan-2-yloxy)-1H-pyrazol-4-yl]pyridin | 1H-NMR (400 MHz, d₃-CD₃CN): δ = 8.45 (dd, 2H), 7.82 (s, 1H), 7.45-7.43 (m, 2H), 7.20-7.19 (dd, 2H), 7.13-7.10 (m, 2H), 4.77 (m, 1H), 1.33 (d, 6H) ppm | - |
| 228 | 3-(4-Fluorphenyl)-4-[3-(4-fluorphenyl)-1-(2-methylpropyl)-1H-pyrazol-4-yl]pyridin | 1H-NMR (400 MHz, d₆-DMSO): δ = 8.51 (d, 1H), 8.45 (s, 1H), 7.45 (s, 1H), 7.29 (d, 1H), 7.04-7.03 (dd, 2H), 6.94-6.91 (dd, 2H), 6.90-6.80 (m, 4H), 3.89 (d, 2H), 2.20 (m, 1H), 0.87 (d, 6H) ppm | - |
| 229 | 3-(3-Fluorphenyl)-4-[3-(4-fluorphenyl)-1-(2-methylpropyl)-1H-pyrazol-4-yl]pyridin | 1H-NMR (400 MHz, d₆-DMSO): δ = 8.54-8.53 (d, 1H), 8.46 (s, 1H), 7.46 (s, 1H), 7.32 (d, 1H), 7.13 (m, 1H), 7.05 (m, 2H), 6.90-6.86 (m, 2H), 6.75 (m, 1H), 6.65 (m, 1H), 3.89 (d, 2H), 2.20 (m, 1H), 0.87 (d, 6H) ppm | - |
| 230 | N-{4-[3-(3-Cyan-4-fluorphenyl)-1-(2-methylpropyl)-1H-pyrazol-4-yl]pyridin-2-yl}propanamid | 1H-NMR (400 MHz, d₆-DMSO): δ = 10.41 (s, 1H), 8.24 (s, 1H), 8.20 (m, 1H), 8.06 (s, 1H), 7.92 (m, 1H), 7.78 (m, 1H), 7.56 (t, 1H), 6.92 (m, 1H), 4.02 (d, 2H), 2.39 (q, 2H), 2.20 (m, 1H), 1.05 (t, 3H), 0.92 (d, 6H) ppm | - |
| 231 | 1-{4-[1-(2,2-Difluorethyl)-3-(4-fluorphenyl)-1H-pyrazol-4-yl]pyridin-2-yl}-3-ethylharnstoff | 1H-NMR (400 MHz, d₃-CD₃CN): δ = 8.80 (s, 1H, br), 8.06 (m, 1H), 7.88 (s, 1H), 7.53-7.45 (m, 3H), 7.14-7.10 (m, 2H), 6.84 (s, 1H), 6.77 (m, 1H), 6.27 (tt, 2J(F,H)=55 Hz, 1H), 4.59 (dt, 3J(F,H)=14Hz, 2H), 3.27 (q, 2H), 1.14 (t, 3H) ppm | - |
| 232 | N-(4-{3-(4-Fluorphenyl)-1-[1-(2-fluorphenyl)ethyl]-1H-pyrazol-4-yl}pyridin-2-yl)-2-methylpropanamid | 1H-NMR (400 MHz, d₆-DMSO): δ **=** 10.40 (s, 1H), 8.34 (s, 1H), 8.17 (d, 1H), 8.10 (s, 1H), 7.45-7.35 (m, 4H), 7.25-7.18 (m, 4H), 6.82 (dd, 1H), 5.96 (q, 1H), 2.72 (m, 1H), 1.06 (d, 6H) ppm | - |
| 233 | 3-{4-[3-(4-Fluorphenyl)-1-(propan-2-yl)-1H-pyrazol-4-yl]pyridin-2-yl}-1,1-dimethylharnstoff | 1H-NMR (400 MHz, d₃-CD₃CN): δ = 8.05 (m, 1H), 7.90 (m, 1H), 7.85 (s, 1H), 7.48-7.46 (m, 2H), 7.36 (s, 1H, br), 7.11-7.09 (m, 2H), 6.77 (dd, 1H), 4.55 (m, 1H), 2.95 (s, 6H), 1.53 (d, 6H) ppm | - |
| 234 | N-{4-[1-(2,2-Difluorethyl)-3-(4-fluorphenyl)-1H-pyrazol-4-yl]pyridin-2-yl} propan-2-sulfonamid | 1H-NMR (400 MHz, d₃-CD₃CN): δ = 8.01 (s, 1H), 7.90 (d, 1H), 7.85 (m, 1H), 7.50-7.45 (m, 3H), 7.13-7.05 (m, 2H), 6.35 (m, 1H), 6.27 (tt, 1H), 4.60 (m, 2H), 3.05 (m, 1H), 1.34 (d, 6H) ppm | - |
| 235 | N-{4-[1-(2,2-Difluorethyl)-3-(4-fluorphenyl)-1H-pyrazol-4-yl]pyridin-2-yl}thiophen-3-carboxamid | 1H-NMR (400 MHz, d₃-CD₃CN): δ = 8.91 (s, 1H, br), 8.23-8.17 (m, 3H), 7.97 (s, 1H), 7.57 (m, 1H), 7.49-7.48 (m, 2H), 7.13-7.11 (m, 2H), 6.94 (dd, 1H), 6.28 (tt, 2J(F,H)=55 Hz, 1H), 4.60 (dt, 3J(F,H)=14Hz, 2H) ppm | - |
| 236 | N-{4-[1-(2,2-Difluorethyl)-3-(4-fluorphenyl)-1H-pyrazol-4-yl]pyridin-2-yl}thiophen-2-carboxamid | 1H-NMR (400 MHz, d₃-CD₃CN): δ = 9.04 (s, 1H, br), 8.24 (d, 1H), 8.20 (s, 1H), 7.87 (dd, 1H), 7.74 (d, 1H), 7.53-7.51 (m, 2H), 7.21-7.20 (dd, 1H), 7.17-7.14 (m, 2H), 6.98 (dd, 1H), 6.31 (tt, 2J(F,H)=55 Hz, 1H), 4.63 (dt, 3J(F,H)=14Hz, 2H) ppm | - |
| 237 | N-{4-[1-(2,2-Difluorethyl)-3-(4-fluorphenyl)-1H-pyrazol-4-yl]pyridin-2-yl}benzamid | 1H-NMR (400 MHz, d₃-CD₃CN): δ = 9.08 (s, 1H, br), 8.30 (m, 1H), 8.24 (m, 1H), 8.01 (s, 1H), 7.97 (d, 2H), 7.64 (m, 1H), 7.56-7.50 (m, 4H), 7.17-7.14 (m, 2H), 6.98 (dd, 1H), 6.32 (tt, 2J(F,H)=55 Hz, 1H), 4.64 (dt, 3J(F,H)=14Hz, 2H) ppm | - |
| 238 | N-{4-[1-(2,2-Difluorethyl)-3-(4-fluorphenyl)-1H-pyrazol-4-yl]pyridin-2-yl}-3-methylbutanamid | 1H-NMR (400 MHz, d₃-CD₃CN): δ = 8.57 (s, 1H, br), 8.13-8.10 (m, 2H), 7.93 (s, 1H), 7.47-7.45 (m, 2H), 7.12-7.09 (m, 2H), 6.88 (dd, 1H), 6.27 (tt, 2J(F,H)=55 Hz, 1H), 4.58 (dt, 3J(F,H)=14Hz, 2H), 2.22 (m, 2H), 2.10 (m, 1H), 0.95 (d, 6H) ppm | - |
| 239 | N-{4-[1-(2,2-Difluorethyl)-3-(4-fluorphenyl)-1H-pyrazol-4-yl]pyridin-2-yl}-2-methylbutanamid | 1H-NMR (400 MHz, d₃-CD₃CN): δ = 8.57 (s, 1H, br), 8.13-8.10 (m, 2H), 7.93 (s, 1H), 7.47-7.45 (m, 2H), 7.12-7.09 (m, 2H), 6.86 (dd, 1H), 6.27 (tt, 2J(F,H)=55 Hz, 1H), 4.59 (dt, 3J(F,H)=14Hz, 2H), 2.42 (m, 1H), 1.65 (m, 1H), 1.45 (m, 1H), 1.12 (d, 3H), 0.89 (t, 3H) ppm | - |
| 240 | N-{4-[1-(2,2-Difluorethyl)-3-(4-fluorphenyl)-1H-pyrazol-4-yl]pyridin-2-yl}cyclobutancarboxamid | 1H-NMR (400 MHz, d₃-CD₃CN): δ = 8.44 (s, 1H, br), 8.16-8.14 (m, 2H), 7.96 (s, 1H), 7.51-7.49 (m, 2H), 7.16-7.13 (m, 2H), 6.89 (dd, 1H), 6.31 (tt, 2J(F,H)=55 Hz, 1H), 4.62 (dt, 3J(F,H)=14Hz, 2H), 2.30-2.20 (m, 6H), 1.85 (m, 1H) ppm | - |
| 241 | Ethyl-{4-[1-(2,2-difluorethyl)-3-(4-fluorphenyl)-1H-pyrazol-4-yl]pyridin-2-yl}carbamat | 1H-NMR (400 MHz, d₃-CD₃CN): δ = 8.16 (s, 1H, br), 8.12 (d, 1H), 7.92 (s, 1H), 7.85 (d, 1H), 7.48-7.46 (m, 2H), 7.12-7.10 (m, 2H), 6.86 (dd, 1H), 6.27 (tt, 2J(F,H)=55 Hz, 1H), 4.59 (dt, 3J(F,H)=14Hz, 2H), 4.14 (q, 2H), 1.24 (t, 3H) ppm | - |
| 242 | N-{4-[1-(2,2-Difluorethyl)-3-(4-fluorphenyl)-1H-pyrazol-4-yl]pyridin-2-yl}acetamid | 1H-NMR (400 MHz, d₃-CD₃CN): δ = 8.63 (s, 1H, br), 8.13 (dd, 1H), 8.06 (s, 1H), 7.92 (s, 1H), 7.48-7.46 (m, 2H), 7.13-7.09 (m, 2H), 6.88 (dd, 1H), 6.27 (tt, 2J(F,H)=55 Hz, 1H), 4.59 (dt, 3J(F,H)=14Hz, 2H), 2.10 (s, 3H) ppm | - |
| 243 | N-{4-[1-(2,2-Difluorethyl)-3-(4-fluorphenyl)-1H-pyrazol-4-yl]pyridin-2-yl}-2-hydroxy-2-methylpropanamid | 1H-NMR (400 MHz, d₃-CD₃CN): δ = 9.20 (s, 1H, br), 8.12 (m, 2H), 7.97 (s, 1H), 7.51-7.49 (m, 2H), 7.15-7.13 (m, 2H), 6.94 (dd, 1H), 6.30 (tt, 2J(F,H)=55 Hz, 1H), 4.62 (dt, 2H), 1.43 (s, 6H) ppm | - |
| 244 | N-(4-{3-(4-Fluorphenyl)-1-[1-(2-fluorphenyl)ethyl]-1H-pyrazol-4-yl}pyridin-2-yl)-2-methoxyacetamid | 1H-NMR (400 MHz, d₆-DMSO): δ = 9.95 (s, 1H), 8.38 (s, 1H), 8.20 (dd, 1H), 8.07 (s, 1H), 7.43-7.36 (m, 4H), 7.25-7.15 (m, 4H), 6.96 (dd, 1H), 4.03 (s, 2H), 3.40 (s, 3H), 1.90 (d, 3H) ppm | - |
| 245 | N-(4-{1-[(2,2-Dichlorcyclopropyl)methyl]-3-(4-fluorphenyl)-1H-pyrazol-4-yl}pyridin-2-yl)-2-methoxyacetamid | 1H-NMR (400 MHz, d₆-DMSO): δ = 9.96 (s, 1H), 8.26 (s, 1H), 8.22 (d, 1H), 8.06 (s, 1H), 7.47 (m, 2H), 7.23 (m, 2H), 6.91 (dd, 1H), 4.45 (dd, 1H), 4.34 (dd, 1H), 4.03 (s, 2H), 3.40 (s, 3H), 2.40 (m, 1H), 1.90 (dd, 1H), 1.70 (dd, 1H) ppm | - |
| 246 | N-{4-[3-(3-Cyan-4-fluorphenyl)-1-(2-methylpropyl)-1H-pyrazol-4-yl]pyridin-2-yl}-2-methoxyacetamid | 1H-NMR (400 MHz, d₆-DMSO): δ = 9.97 (s, 1H), 8.27 (s, 1H), 8.24 (d, 1H), 7.98 (s, 1H), 7.92 (dd, 1H), 7.78 (m, 1H), 7.55 (t, 1H), 6.99 (d, 1H), 4.03 (m, 4H), 3.40 (s, 3H), 2.20 (m, 1H), 0.91 (d, 6H) ppm | - |
| 247 | N-{4-[1-(2-Fluorbenzyl)-3-(4-fluorphenyl)-1H-pyrazol-4-yl]pyridin-2-yl}-2-methoxyacetamid | 1H-NMR (400 MHz, d₆-DMSO): δ = 9.96 (s, 1H), 8.30 (s, 1H), 8.21 (d, 1H), 8.04 (s, 1H), 7.45-7.38 (m, 4H), 7.30-7.18 (m, 4H), 6.92 (dd, 1H), 5.49 (s, 2H), 4.03 (s, 2H), 3.4 (s, 3H) ppm | - |
| 248 | N-(4-{3-(4-Fluorphenyl)-1-[1-(2-fluorphenyl)ethyl]-1H-pyrazol-4-yl} pyridin-2-yl)propanamid | 1H-NMR (400 MHz, d₆-DMSO): δ = 10.40 (s, 1H), 8.34 (s, 1H), 8.18 (d, 1H), 8.08 (s, 1H), 7.42-7.35 (m, 4H), 7.25-7.18 (m, 4H), 6.86 (dd, 1H), 5.96 (q, 1H), 2.38 (q, 2H), 1.90 (d, 3H), 1.05 (t, 3H) ppm | - |
| 249 | N-(4-{1-[(2,2-Dichlorcyclopropyl)methyl]-3-(4-fluorphenyl)-1H-pyrazol-4-yl}pyridin-2-yl)propanamid | 1H-NMR (400 MHz, d₆-DMSO): δ = 10.42 (s, 1H), 8.24 (s, 1H), 8.18 (d, 1H), 8.11 (s, 1H), 7.47 (m, 2H), 7.23 (m, 2H), 6.84 (dd, 1H), 4.45 (dd, 1H), 4.33 (dd, 1H), 2.40-2.35 (m, 3H), 1.90 (dd, 1H), 1.70 (dd, 1H), 1.04 (q, 3H) ppm | - |
| 250 | 2-Cyclopropyl-N-(4-{3-(4-fluorphenyl)-1-[1-(2-fluorphenyl)ethyl]-1H-pyrazol-4-yl}pyridin-2-yl)acetamid | 1H-NMR (400 MHz, d₆-DMSO): δ = 10.33 (s, 1H), 8.33 (s, 1H), 8.16 (d, 1H), 8.09 (s), 7.42-7.35 (m, 4H), 7.25-7.15 (m, 4H), 6.84 (dd, 1H), 5.95 (q, 1H), 2.23 (d, 2H), 1.90 (d, 3H), 1.00 (m, 1H), 0.45 (m, 2H), 0.16 (m, 2H) ppm | - |
| 251 | 2-Cyclopropyl-N-(4-{1-[(2,2-dichlorcyclopropyl)methyl]-3-(4-fluorphenyl)-1H-pyrazol-4-yl}pyridin-2-yl)acetamid | 1H-NMR (400 MHz, d₆-DMSO): δ = 10.35 (s, 1H), 8.24 (s, 1H), 8.18 (dd, 1H), 8.11 (s, 1H), 7.45 (m, 2H), 7.22 (m, 2H), 6.83 (dd, 1H), 4.45 (dd, 1H), 4.33 (dd, 1H), 2.40 (m, 1H), 2.23 (d, 2H), 1.90 (dd, 1H), 1.70 (dd, 1H), 1.00 (m, 1H), 0.45 (d, 2H), 0.17 (d, 2H) ppm | - |
| 252 | N-{4-[3-(3-Cyan-4-fluorphenyl)-1-(2-methylpropyl)-1H-pyrazol-4-yl]pyridin-2-yl}-2-cyclopropylacetamid | 1H-NMR (400 MHz, d₆-DMSO): δ = 10.34 (s, 1H), 8.24 (s, 1H), 8.20 (d, 1H), 8.06 (s, 1H), 7.90 (dd, 1H), 7.78 (m, 1H), 7.56 (t, 1H), 6.92 (dd, 1H), 4.00 (d, 2H), 2.23 (d, 2H), 2.20 (m, 1H), 1.05 (m, 1H), 0.91 (d, 6H), 0.45 (d, 2H), 0.16 (d, 2H) ppm | - |
| 253 | 2-Cyclopropyl-N-{4-[1-(2-fluorbenzyl)-3-(4-fluorphenyl)-1H-pyrazol-4-yl]pyridin-2-yl}acetamid | 1H-NMR (400 MHz, d₆-DMSO): δ = 10.33 (s, 1H), 8.27 (s, 1H), 8.16 (dd, 1H), 8.09 (s, 1H), 7.42-7.35 (m, 4H), 7.25-7.15 (m, 4H), 6.82-6.80 (dd, 1H), 5.47 (s, 2H), 2.24 (d, 2H), 1.00 (m, 1H), 0.44 (m, 2H), 0.18 (m, 2H) ppm | - |
| 254 | N-(4-{1-[(2,2-Dichlorcyclopropyl)methyl]-3-(4-fluorphenyl)-1H-pyrazol-4-yl}pyridin-2-yl)-2-methylpropanamid | 1H-NMR (400 MHz, d₆-DMSO): δ = 10.41 (s, 1H), 8.24 (s, 1H), 8.18 (d, 1H), 8.13 (s, 1H), 7.45 (dd, 2H), 7.22 (m, 2H), 6.81 (dd, 1H), 4.45 (dd, 1H), 4.35 (dd, 1H), 2.75 (m, 1H), 2.43 (m, 1H), 1.89 (dd, 1H), 1.70 (dd, 1H), 1.05 (d, 6H) ppm | - |
| 255 | N-{4-[3-(3-Cyan-4-fluorphenyl)-1-(2-methylpropyl)-1H-pyrazol-4-yl]pyridin-2-yl}-2-methylpropanamid | 1H-NMR (400 MHz, d₆-DMSO): δ = 10.41 (s, 1H), 8.25 (s, 1H), 8.20 (d, 1H), 8.09 (s, 1H), 7.90 (dd, 1H), 7.78 (m, 1H), 7.55 (t, 1H), 6.90 (dd, 1H), 4.00 (d, 2H), 2.72 (m, 1H), 2.20 (m, 1H), 1.05 (d, 6H), 0.91 (d, 6H) ppm | - |
| 256 | N-{4-[1-(2-Fluorbenzyl)-3-(4-fluorphenyl)-1H-pyrazol-4-yl]pyridin-2-yl} -2-methylpropanamid | 1H-NMR (400 MHz, d₆-DMSO): δ = 10.40 (s, 1H), 8.27 (s, 1H), 8.16 (dd, 1H), 8.11 (s, 1H), 7.45-7.35 (m, 3H), 7.25-7.15 (m, 5H), 6.80-6.79 (dd, 1H), 5.47 (s, 2H), 2.61 (m, 1H), 1.05 (d, 6H) ppm | - |
| 258 | N-{4-[5-(Difluormethoxy)-3-(4-fluor-2-hydroxyphenyl)-1-methyl-1H-pyrazol-4-yl]pyridin-2-yl}cyclopropancarboxamid | 1H-NMR (400 MHz, d₆-DMSO): δ = 10.69 (s, 1H), 9.92 (s, 1H), 8.15 (d, 1H), 8.03 (s, 1H), 7.20 (m, 1H), 7.13 (t, 2J(H,F)=71 Hz, 1H), 6.73 (dd, 1H), 6.67-6.64 (m, 1H), 6.58-6.55 (dd, 1H), 3.79 (s, 3H), 1.95 (m, 1H), 0.76 (m, 4H) ppm | - |
| 259 | N-(4-{3-(4-Fluorphenyl)-1-[1-(2-fluorphenyl)ethyl]-1H-pyrazol-4-yl}pyridin-2-yl)cyclopropancarboxamid | 1H-NMR (400 MHz, d₆-DMSO): δ = 10.76 (s, 1H), 8.32 (s, 1H), 8.18 (d, 1H), 8.07 (s), 7.44-7.42 (m, 4H), 7.25-7.15 (m, 4H), 6.83 (dd, 1H), 5.95 (q, 1H), 2.00 (m, 2H), 1.80 (d, 3H), 0.80 (m, 4H) ppm | - |
| 260 | N-(4-{1-[(2,2-Dichlorcyclopropyl)methyl]-3-(4-fluorphenyl)-1H-pyrazol-4-yl}pyridin-2-yl)cyclopropancarboxamid | 1H-NMR (400 MHz, d₆-DMSO): δ = 10.78 (s, 1H), 8.22 (s, 1H), 8.19 (d, 1H), 8.09 (s, 1H), 7.44 (dd, 2H), 7.23 (m, 2H), 6.82 (dd, 1H), 4.45 (dd, 1H), 4.34 (dd, 1H), 2.40 (m, 1H), 1.98 (m, 1H), 1.70 (dd, 1H), 0.78 (m, 4H) ppm | - |
| 261 | N-{4-[3-(3-Cyan-4-fluorphenyl)-1-(2-methylpropyl)-1H-pyrazol-4-yl]pyridin-2-yl}cyclopropancarboxamid | 1H-NMR (400 MHz, d₆-DMSO): δ = 10.78 (s, 1H), 8.23 (s, 1H), 8.21 (d, 1H), 8.05 (s, 1H), 7.90 (dd, 1H), 7.77 (m, 1H), 7.55 (t, 1H), 6.90 (dd, 1H), 4.00 (d, 2H), 2.20 (m, 1H), 1.98 (m, 1H), 0.90 (d, 6H), 0.78 (m, 4H) ppm | - |
| 262 | N-{4-[1-(2-Fluorbenzyl)-3-(4-fluorphenyl)-1H-pyrazol-4-yl]pyridin-2-yl}cyclopropancarboxamid | 1H-NMR (400 MHz, d₆-DMSO): δ = 10.78 (s, 1H), 8.25 (s, 1H), 8.17 (d, 1H), 8.08 (s, 1H), 7.45-7.30 (m, 3H), 7.30-7.15 (m, 5H), 6.81 (m, 1H), 5.47 (s, 2H), 1.98 (m, 1H), 0.78 (m, 4H) ppm | - |
| 263 | Ethyl-(4-{3-(4-fluorphenyl)-1-[1-(2-fluorphenyl)ethyl]-1H-pyrazol-4-yl}pyridin-2-yl)carbamat | 1H-NMR (400 MHz, d₆-DMSO): δ = 10.05 (s, 1H), 8.35 (s, 1H), 8.15 (d, 1H), 7.77 (s, 1H), 7.45-7.35 (m, 3H), 7.25-7.15 (m, 5H), 6.83 (dd, 1H), 5.94 (q, 1H), 1.90 (d, 3H), 1.22 (t, 3H) ppm | - |
| 264 | Ethyl-(4-{1-[(2,2-dichlorcyclopropyl)methyl]-3-(4-fluorphenyl)-1H-pyrazol-4-yl}pyridin-2-yl)carbamat | 1H-NMR (400 MHz, d₆-DMSO): δ = 10.07 (s, 1H), 8.24 (s, 1H), 8.15 (d, 1H), 7.78 (s, 1H), 7.47-7.45 (dd, 2H), 7.22 (dd, 2H), 6.83 (dd, 1H), 4.47-4.44 (dd, 1H), 4.35-4.32 (dd, 1H), 4.09 (q, 2H), 2.40 (m, 1H), 1.90 (dd, 1H), 1.70 (dd, 1H), 1.22 (t, 3H) ppm | - |
| 265 | Ethyl- {4-[3-(3-cyan-4-fluorphenyl)-1 - (2-methylpropyl)-1H-pyrazol-4-yl]pyridin-2-yl} carbamat | 1H-NMR (400 MHz, d₆-DMSO): δ = 10.08 (s, 1H), 8.25 (s, 1H), 8.16 (m, 1H), 7.92 (m, 1H), 7.78-7.70 (m, 2H), 7.56-7.53 (m, 2H), 7.40 (m, 1H), 6.90 (dd, 1H), 4.09 (q, 2H), 4.00 (d, 2H), 2.20 (m, 1H), 1.22 (m, 6H), 0.80 (d, 3H) ppm | - |
| 266 | Ethyl-{4-[1-(2-fluorbenzyl)-3-(4-fluorphenyl)-1H-pyrazol-4-yl]pyridin-2-yl}carbamat | 1H-NMR (400 MHz, d₆-DMSO): δ = 10.06 (s, 1H), 8.27 (s, 1H), 8.14-8.13 (d, 1H), 7.78 (d, 1H), 7.44-7.42 (m, 4H), 7.35 (m, 1H), 7.25-7.15 (m, 4H), 5.47 (s, 2H), 4.10 (q, 2H), 1.22 (t, 3H) ppm | - |
| 267 | Ethyl-{4-[3-(4-fluorphenyl)-1-(2-methylpropyl)-1H-pyrazol-4-yl]pyridin-2-yl} carbamat | 1H-NMR (400 MHz, d₆-DMSO): δ = 10.05 (s, 1H), 8.19 (s, 1H), 8.13 (d, 1H), 7.78 (d, 1H), 7.44-7.42 (m, 2H), 7.22-7.19 (dd, 2H), 6.83-6.82 (dd, 1H), 4.10 (q, 2H), 3.99 (d, 2H), 2.18 (m, 1H), 1.22 (m, 6H), 0.80 (d, 3H) ppm | - |
| 268 | Ethyl-{4-[1-(butan-2-yl)-3-(4-fluorphenyl)-1H-pyrazol-4-yl]pyridin-2-yl}carbamat | 1H-NMR (400 MHz, d₆-DMSO): δ = 10.04 (s, 1H), 8.19 (s, 1H), 8.13 (d, 1H), 7.77 (s, 1H), 7.45-7.42 (m, 2H), 7.22 (dd, 2H), 6.84-6.83 (dd, 1H), 4.35 (m, 1H), 4.09 (q, 2H), 1.90 (m, 1H), 1.80 (m, 1H), 1.22 (m, 6H), 0.80 (t, 3H) ppm | |
| 269 | N-{4-[1-(Cyclopentyloxy)-3-(4-fluorphenyl)-1H-pyrazol-4-yl]pyridin-2-yl}cyclopropancarboxamid | 1H-NMR (400 MHz, d₃-CD₃CN): δ = 8.86 (s, 1H, br), 8.14 (d, 1H), 8.07 (s, 1H), 7.80 (s, 1H), 7.47-7.43 (m, 2H), 7.10-7.06 (m, 2H), 6.88 (dd, 1H), 5.09 (m, 1H), 1.85-1.78 (m, 5H), 1.70-1.63 (m, 2H), 0.85-0.75 (m, 4H) ppm | - |
| 270 | N-{4-[1-(Cyclopentyloxy)-3-(4-fluorphenyl)-1H-pyrazol-4-yl]pyridin-2-yl} -2-methylpropanamid | 1H-NMR (400 MHz, d₃-CD₃CN): δ = 8.53 (s, 1H, br), 8.13-8.11 (m, 2H), 7.81 (s, 1H), 7.47-7.44 (m, 2H), 7.11-7.08 (m, 2H), 6.87 (dd, 1H), 5.11 (m, 1H), 2.63 (m, 1H), 1.85-1.78 (m, 4H), 1.70-1.63 (m, 2H), 1.13 (d, 6H) ppm | - |
| 271 | N-{4-[3-(4-Fluorphenyl)-1-(propan-2-yloxy)-1H-pyrazol-4-yl]pyridin-2-yl}cyclopropancarboxamid | 1H-NMR (400 MHz, d₃-CD₃CN): δ = 8.87 (s, 1H, br), 8.13-8.12 (d, 1H), 8.07 (s, 1H), 7.78 (s, 1H), 7.46-7.44 (m, 2H), 7.10-7.08 (m, 2H), 6.87 (dd, 1H), 4.75 (m, 1H), 1.76 (m, 1H), 1.32 (d, 6H), 0.87-0.81 (m, 4H) ppm | - |
| 272 | N-{4-[3-(4-Fluorphenyl)-1-(propan-2-yloxy)-1H-pyrazol-4-yl]pyridin-2-yl}-2-methylpropanamid | 1H-NMR (400 MHz, d₃-CD₃CN): δ = 8.53 (s, 1H, br), 8.13-8.11 (m, 2H), 7.80 (s, 1H), 7.47-7.44 (m, 2H), 7.10-7.08 (m, 2H), 6.87 (dd, 1H), 4.75 (m, 1H), 2.60 (m, 1H), 1.32 (d, 6H), 1.13 (d, 6H) ppm | - |
| 273 | N-(Cyclopropylmethyl)-4-[3-(4-fluorphenyl)-1-(2-methylpropyl)-1H-pyrazol-4-yl]pyrimidin-2-amin | 1H-NMR (400 MHz, d₆-DMSO): δ = 8.30 (bs, 1H), 8.11 (d, 1H), 7.56 (m, 2H), 7.21 (t, 2H), 7.00 (m, 1H), 6.47 (bs, 1H), 3.99 (d, 2H), 2.94 (bs, 2H), 2.18 (m, 1H), 0.90 (d, 6H), 0.35 (m, 2H), 0.10 (m, 2H) ppm | - |
| 274 | Methyl-2-{3-(4-fluorphenyl)-4-[2-(propionylamino)pyridin-4-yl]-1H-pyrazol-1-yl}-3-methylbutanoat | 1H-NMR (400 MHz, d₆-DMSO): δ = 10.41 (s, 1H), 8.27 (s, 1H), 8.18 (dd, 1H), 8.09 (s, 1H), 7.44-7.41 (m, 2H), 7.22-7.20 (m, 2H), 6.85 (dd, 1H), 4.92 (d, 1H), 3.72 (s, 3H), 2.60 (m, 1H), 2.37 (q, 2H), 1.03 (t, 3H), 1.00 (d, 3H), 0.86 (d, 3H) ppm | - |
| 275 | 4-[1-(1,3-Dioxolan-2-ylmethyl)-3-(4-fluorphenyl)-1H-pyrazol-4-yl]pyridin | 1H-NMR (400 MHz, d₆-DMSO): δ = 8.47 (dd, 2H), 8.19 (s, 1H), 7.44-7.42 (m, 2H), 7.25-7.15 (m, 4H), 5.27 (t, 1H), 4.32 (d, 2H), 3.95-3.85 (m, 4H) ppm | - |
| 276 | N-{4-[1-(1,3-Dioxolan-2-ylmethyl)-3-(4-fluorphenyl)-1H-pyrazol-4-yl]pyridin-2-yl}-2-methylpropanamid | 1H-NMR (400 MHz, d₆-DMSO): δ = 10.41 (s, 1H), 8.16 (dd, 1H), 8.12 (s, 2H), 7.44-7.42 (m, 2H), 7.22-7.19 (m, 2H), 6.78 (dd, 1H), 5.27 (t, 1H), 4.32 (d, 2H), 3.95-3.80 (m, 4H), 2.72 (m, 1H), 1.06 (d, 6H) ppm | - |
| 277 | Methyl-2-{3-(4-fluorphenyl)-4-[2-(isobutyrylamino)pyridin-4-yl]-1H-pyrazol-1-yl}-3-methylbutanoat | 1H-NMR (400 MHz, d₆-DMSO): δ = 10.42 (s, 1H), 8.27 (s, 1H), 8.18 (dd, 1H), 8.12 (s, 1H), 7.44-7.41 (m, 2H), 7.22-7.20 (m, 2H), 6.83 (dd, 1H), 4.92 (d, 1H), 3.72 (s, 3H), 2.73 (m, 1H), 2.62 (m, 1H), 1.06 (d, 6H), 1.00 (d, 3H), 0.86 (d, 3H) ppm | - |
| 278 | N-{4-[1-(1,3-Dioxolan-2-ylmethyl)-3-(4-fluorphenyl)-1H-pyrazol-4-yl]pyridin-2-yl}propanamid | 1H-NMR (400 MHz, d₆-DMSO): δ = 10.41 (s, 1H), 8.16 (dd, 1H), 8.11-8.09 (m, 2H), 7.44-7.42 (m, 2H), 7.22-7.19 (m, 2H), 6.81 (dd, 1H), 5.27 (t, 1H), 4.32 (d, 2H), 3.95-3.80 (m, 4H), 2.37 (q, 2H), 1.04 (t, 3H) ppm | - |
| 279 | 4-[3-(4-Fluorphenyl)-1-(2-methylpropyl)-1H-pyrazol-4-yl]-N-(oxetan-3-yl)pyrimidin-2-amin | 1H-NMR (400 MHz, d₆-DMSO): δ = 8.31 (bs, 1H), 8.16 (d, 1H), 7.73 (d, 1H), 7.54 (m, 2H), 7.27 (t, 2H), 6.52 (bs, 1H), 4.54 (bs, 2H), 4.43 (bs, 2H), 3.99 (d, 2H), 3.75 (bs, 1H), 2.18 (m, 1H), 0.90 (d, 6H) ppm | - |
| 280 | 7-[3-(4-fluorophenyl)-1-isopropyl-1H-pyrazol-4-yl]-3H-imidazo[4,5-b]pyridine | 1H-NMR (400 MHz, CDCl₃): δ = 12.35 (bs, 1H), 8.78 (s, 1H), 8.69 (s, 1H), 8.12 (s, 1H), 7.65 (m, 2H), 7.00 (t, 2H), 4.58 (m, 1H), 1.57 (d, 6H) ppm | - |
| 281 | N-Cyclopropyl-4-[3-(4-fluorphenyl)-1-(2-methylpropyl)-1H-pyrazol-4-yl]pyrimidin-2-amin | 1H-NMR (400 MHz, d₆-DMSO): δ = 8.26 (bs, 1H), 8.14 (d, 1H), 7.63 (m, 2H), 7.21 (t, 2H), 6.48 (d, 1H), 4.0 (d, 2H), 2.62 (m, 1H), 2.018 (m, 1H), 0.9 (d, 6H), 0.55 (m, 2H), 0.43 (m, 2H) ppm | - |
| 282 | 6-[3-(4-Fluorphenyl)-1-isopropyl-1H-pyrazol-4-yl]-9H-purin | 1H-NMR (400 MHz, d₃-CD₃CN): δ = 11.00 (s, 1H), 8.85 (s, 1H), 8.60 (s, 1H), 8.21 (s, 1H), 7.74-7.73 (m, 2H), 7.12-7.09 (m, 2H), 4.65 (m, 1H), 1.57 (d, 6H) ppm | - |
| 283 | 4-[3-(4-fluorophenyl)-1-isopropyl-1H-pyrazol-4-yl]-2-(4-methyl-1H-imidazol-1-yl)pyridine | 1H-NMR (250 MHz, CDCl₃): δ = 8.24 (d, 1H), 8.04 (s, 1H), 7.65 (s, 1H), 7.41 (m, 2H), 7.12 (d, 1H), 7.06-6.98 (m, 4H), 4.52 (m, 1H), 2.20 (s, 3H), 1.54 (d, 6H) ppm | - |
| 284 | N-tert-Butyl-4-[3-(4-fluorphenyl)-1-(2-methylpropyl)-1H-pyrazol-4-yl]pyrimidin-2-amin | 1H-NMR (400 MHz, d₆-DMSO): δ = 8.18 (s, 1H), 8.10 (d, 1H), 7.5 (m, 2H), 7.22 (t, 2H), 6.45 (s, 1H), 6.33 (d, 1H), 4.0 (d, 2H), 2.17 (m, 1H), 1.3 (bs, 9H), 0.9 (d, 6H) ppm | - |
| 285 | 4-[3-(4-Fluorphenyl)-1-(2-methylpropyl)-1H-pyrazol-4-yl]-N-(1-methoxypropan-2-yl)pyrimidin-2-amin | 1H-NMR (400 MHz, d₆-DMSO): δ = 8.04 (bs, 1H), 7.88 (d, 1H), 7.31 (m, 2H), 6.97 (t, 2H), 6.48 (d, 1H), 6.19 (bs, 1H), 3.76 (m, 3H), 3.10 (s, 3H), 2.96 (bs, 2H), 1.94 (m, 1H), 0.80 (bs, 3H), 0.66 (d, 6H) ppm | - |
| 286 | N-(Butan-2-yl)-4-[3-(4-fluorphenyl)-1-(2-methylpropyl)-1H-pyrazol-4-yl]pyrimidin-2-amin | 1H-NMR (400 MHz, d₆-DMSO): δ = 8.25 (bs, 1H), 8.10 (d, 1H), 7.55 (m, 2H), 7.20 (t, 2H), 6.72 (d, 1H), 3.99 (d, 2H), 2.18 (m, 1H), 1.00 (bs, 2H), 0.9 (d, 6H), 0.78 (bs, 2H) ppm | - |
| 287 | 4-[3-(4-Fluorphenyl)-1-(2-methylpropyl)-1H-pyrazol-4-yl] -2-(methylsulfonyl)pyrimidin | 1H-NMR (400 MHz, d₆-DMSO): δ = 8.87 (d, 1H), 8.70 (s, 1H), 7.62 (d, 1H), 7.61 (m, 2H), 7.25 (t, 2H), 4.05 (d, 2H), 3.17 (s, 3H), 2.22 (m, 1H), 0.92 (d, 6H) ppm | - |
| 288 | 4-[3-(4-Fluorphenyl)-1-isobutyl-1H-pyrazol-4-yl]-7-methylthieno[3,2-d]pyrimidin | 1H-NMR (400 MHz, d₃-CD3CN): δ = 9.02 (s, 1H), 8.17 (s, 1H), 7.77 (s, 1H), 7.52-7.50 (m, 2H), 7.05-7.02 (m, 2H), 4.06 (d, 2H), 2.46 (s, 3H), 2.27 (m, 1H), 0.96 (d, 6H) ppm | - |
| 289 | 4-[1-(Cyclopropylmethyl)-3-(4-fluorphenyl)-1H-pyrazol-4-yl]-7-methylthieno [3,2-d]pyrimidin | 1H-NMR (400 MHz, d₃-CD₃CN): δ = 9.02 (s, 1H), 8.26 (s, 1H), 7.77 (m, 1H), 7.53-7.50 (m, 2H), 7.06-7.02 (m, 2H), 4.10 (d, 2H), 2.46 (d, 3H), 1.42-1.37 (m, 1H), 0.66-0.64 (m, 2H), 0.48-0.43 (m, 2H) ppm | - |
| 290 | N-{4-[1-Ethyl-3-(4-fluorphenyl)-1H-pyrazol-4-yl]pyridin-2-yl}-2-methylpropanamid | 1H-NMR (400 MHz, d₃-CD₃CN): δ = 8.61 (s, 1H, br), 8.11-8.09 (m, 2H), 7.87 (s, 1H), 7.47-7.45 (m, 2H), 7.11-7.09 (m, 2H), 6.86 (dd, 1H), 4.20 (q, 2H), 2.62 (m, 1H), 1.50 (t, 3H), 1.14 (d, 6H) ppm | - |
| 291 | 2-Cyclopropyl-N-{4-[1-ethyl-3-(4-fluorphenyl)-1H-pyrazol-4-yl]pyridin-2-yl}acetamid | 1H-NMR (400 MHz, d₃-CD₃CN): δ = 8.60 (s, 1H, br), 8.11 (m, 2H), 7.88 (s, 1H), 7.47-7.45 (m, 2H), 7.11-7.08 (m, 2H), 6.88 (dd, 1H), 4.21 (q, 2H), 2.26 (d, 2H), 1.50 (t, 3H), 1.05 (m, 1H), 0.54 (m, 2H), 0.21 (m, 2H) ppm | - |
| 292 | N-{4-[3-(4-Fluorphenyl)-1-propyl-1H-pyrazol-4-yl]pyridin-2-yl}propanamid | 1H-NMR (400 MHz, d₃-CD₃CN): δ = 8.61 (s, 1H, br), 8.10 (m, 2H), 7.86 (s, 1H), 7.47-7.45 (m, 2H), 7.11-7.08 (m, 2H), 6.87 (dd, 1H), 4.12 (t, 2H), 2.37 (q, 2H), 1.90 (m, 2H), 1.11 (t, 3H), 0.93 (t, 3H) ppm | - |
| 293 | N-{4-[3-(4-Fluorphenyl)-1-propyl-1H-pyrazol-4-yl]pyridin-2-yl}-2-methoxyacetamid | 1H-NMR (400 MHz, d₃-CD₃CN): δ = 8.72 (s, 1H, br), 8.14 (dd, 1H), 8.08 (s, 1H), 7.88 (s, 1H), 7.47-7.45 (m, 2H), 7.11-7.09 (m, 2H), 6.93 (dd, 1H), 4.13 (t, 2H), 3.96 (s, 3H), 3.45 (s, 3H), 0.93 (t, 3H) ppm | - |
| 294 | N-{4-[3-(4-Fluorphenyl)-1-propyl-1H-pyrazol-4-yl]pyridin-2-yl}-2-methylpropanamid | 1H-NMR (400 MHz, d₃-CD₃CN): δ = 8.60 (s, 1H, br), 8.12-8.10 (m, 2H), 7.86 (s, 1H), 7.47-7.45 (m, 2H), 7.11-7.08 (m, 2H), 6.86 (dd, 1H), 4.12 (t, 2H), 2.62 (m, 1H), 1.90 (m, 2H), 1.14 (d, 6H), 0.93 (t, 3H) ppm | - |
| 295 | 2-Cyclopropyl-N-{4-[3-(4-fluorphenyl)-1-propyl-1H-pyrazol-4-yl]pyridin-2-yl} acetamid | 1H-NMR (400 MHz, d₃-CD₃CN): δ = 8.60 (s, 1H, br), 8.11 (m, 2H), 7.87 (s, 1H), 7.47-7.45 (m, 2H), 7.11-7.08 (m, 2H), 6.87 (dd, 1H), 4.13 (t, 2H), 2.26 (d, 2H), 1.05 (m, 1H), 0.93 (t, 3H), 0.54 (m, 2H), 0.21 (m, 2H) ppm | - |
| 296 | N-{4-[3-(4-Fluorphenyl)-1-methyl-1H-pyrazol-4-yl]pyridin-2-yl}propanamid | 1H-NMR (400 MHz, d₃-CD₃CN): δ = 8.61 (s, 1H, br), 8.10 (m, 2H), 7.82 (s, 1H), 7.47-7.45 (m, 2H), 7.11-7.08 (m, 2H), 6.85 (dd, 1H), 3.91 (s, 3H), 2.37 (q, 2H), 1.11 (t, 3H) ppm | - |
| 297 | N-{4-[3-(4-Fluorphenyl)-1-methyl-1H-pyrazol-4-yl]pyridin-2-yl}-2-methoxyacetamid | 1H-NMR (400 MHz, d₃-CD₃CN): δ = 8.72 (s, 1H, br), 8.14 (dd, 1H), 8.08 (s, 1H), 7.84 (s, 1H), 7.47-7.45 (m, 2H), 7.11-7.09 (m, 2H), 6.93 (dd, 1H), 3.96 (s, 2H), 3.91 (s, 3H), 3.45 (s, 3H) ppm | - |
| 298 | N-{4-[3-(4-Fluorphenyl)-1-methyl-1H-pyrazol-4-yl]pyridin-2-yl}-2-methylpropanamid | 1H-NMR (400 MHz, d₃-CD₃CN): δ = 8.60 (s, 1H, br), 8.10 (m, 2H), 7.82 (s, 1H), 7.47-7.45 (m, 2H), 7.11-7.09 (m, 2H), 6.95 (dd, 1H), 3.91 (s, 3H), 2.62 (m, 1H), 1.14 (d, 6H) ppm | - |
| 299 | 2-Cyclopropyl-N-{4-[3-(4-fluorphenyl)-1-methyl-1H-pyrazol-4-yl]pyridin-2-yl} acetamid | 1H-NMR (400 MHz, d₃-CD₃CN): δ = 8.60 (s, 1H, br), 8.11 (m, 2H), 7.83 (s, 1H), 7.47-7.45 (m, 2H), 7.11-7.08 (m, 2H), 6.86 (dd, 1H), 3.91 (s, 3H), 2.33 (d, 2H), 1.05 (m, 1H), 0.54 (m, 2H), 0.21 (m, 2H) ppm | - |
| 300 | N-(4-{3-(4-Fluorphenyl)-1-[2-(methylsulfanyl)ethyl]-1H-pyrazol-4-yl} pyridin-2-yl)propanamid | 1H-NMR (400 MHz, d₆-DMSO): δ = 10.40 (s, 1H), 8.21 (s, 1H), 8.16 (dd, 1H), 8.10 (s, 1H), 7.44-7.42 (m, 2H), 7.22-7.19 (m, 2H), 6.81-6.80 (dd, 1H), 4.37 (t, 2H), 2.98 (t, 2H), 2.36 (q, 2H), 2.07 (s, 3H), 1.03 (t, 3H) ppm | - |
| 301 | N-{4-[1-(1-Cyclopropylethyl)-3-(4-fluorphenyl)-1H-pyrazol-4-yl]pyridin-2-yl}propanamid | 1H-NMR (400 MHz, d₆-DMSO): δ = 10.39 (s, 1H), 8.21 (s, 1H), 8.16 (dd, 1H), 8.09 (s, 1H), 7.45-7.43 (m, 2H), 7.21-7.18 (m, 2H), 6.84-6.83 (dd, 1H), 3.71 (m, 1H), 2.36 (q, 2H), 1.58 (d, 3H), 1.32 (m, 1H), 1.03 (t, 3H), 0.63 (m, 1H), 0.50 (m, 1H), 0.42-0.38 (m, 2H) ppm | - |
| 302 | N-{4-[1-(3-Cyanbenzyl)-3-(4-fluorphenyl)-1H-pyrazol-4-yl]pyridin-2-yl}propanamid | 1H-NMR (400 MHz, d₆-DMSO): δ = 10.40 (s, 1H), 8.35 (s, 1H), 8.17 (dd, 1H), 8.10 (s, 1H), 7.88 (d, 1H), 7.80 (m, 1H), 7.70 (d, 1H), 7.62-7.58 (m, 1H), 7.43-7.41 (m, 2H), 7.21-7.19 (m, 2H), 6.83-6.82 (dd, 1H), 5.48 (s, 2H), 2.36 (q, 2H), 1.03 (t, 3H) ppm | - |
| 303 | N-{4-[1-(2-Cyanethyl)-3-(4-fluorphenyl)-1H-pyrazol-4-yl]pyridin-2-yl}propanamid | 1H-NMR (400 MHz, d₆-DMSO): δ = 10.42 (s, 1H), 8.25 (s, 1H), 8.18 (dd, 1H), 8.11 (s, 1H), 7.46-7.43 (m, 2H), 7.25-7.21 (m, 2H), 6.82-6.81 (dd, 1H), 4.48 (t, 2H), 3.13 (t, 2H), 2.38 (q, 2H), 1.04 (t, 3H) ppm | - |
| 304 | N-{4-[3-(4-Fluorphenyl)-1-isopropyl-5-(trifluormethyl)-1H-pyrazol-4-yl]pyridin-2-yl}propanamid | 1H-NMR (400 MHz, d₆-DMSO): δ = 10.58 (s, 1H), 8.32 (dd, 1H), 8.03 (s, 1H), 7.33-7.30 (m, 2H), 7.18-7.15 (m, 2H), 7.00 (dd, 1H), 4.75 (m, 1H), 2.38 (q, 2H), 1.56 (d, 6H), 1.02 (t, 3H) ppm | - |
| 305 | 4-[1-(1-Cyclopropylethyl)-3-(4-fluorphenyl)-1H-pyrazol-4-yl]pyridin | 1H-NMR (400 MHz, d₆-DMSO): δ = 8.46 (dd, 2H), 8.31 (s, 1H), 7.45-7.43 (m, 2H), 7.24-7.21 (m, 4H), 3.71 (m, 1H), 1.57 (d, 3H), 1.32 (m, 1H), 0.63 (m, 1H), 0.50 (m, 1H), 0.42-0.38 (m, 2H) ppm | - |
| 306 | N-(4- {3-(4-Fluorphenyl)-1-[2-(methylsulfanyl)ethyl]-1H-pyrazol-4-yl}pyridin-2-yl)-2-methylpropanamid | 1H-NMR (400 MHz, d₆-DMSO): δ = 10.41 (s, 1H), 8.22 (s, 1H), 8.16 (dd, 1H), 8.13 (s, 1H), 7.44-7.42 (m, 2H), 7.22-7.19 (m, 2H), 6.78 (dd, 1H), 4.37 (t, 2H), 2.98 (t, 2H), 2.73 (m, 1H), 2.07 (s, 3H), 1.06 (d, 6H) ppm | - |
| 307 | N- {4-[1-(1-Cyclopropylethyl)-3-(4-fluorphenyl)-1H-pyrazol-4-yl]pyridin-2-yl} -2-methylpropanamid | 1H-NMR (400 MHz, d₆-DMSO): δ = 10.40 (s, 1H), 8.22 (s, 1H), 8.16 (dd, 1H), 8.12 (s, 1H), 7.45-7.42 (m, 2H), 7.21-7.18 (m, 2H), 6.81 (dd, 1H), 3.71 (m, 1H), 2.73 (m, 1H), 1.58 (d, 3H), 1.32 (m, 1H), 1.05 (d, 6H), 0.63 (m, 1H), 0.50 (m, 1H), 0.42-0.38 (m, 2H) ppm | - |
| 308 | N-{4-[1-(3-Cyanbenzyl)-3-(4-fluorphenyl)-1H-pyrazol-4-yl]pyridin-2-yl} -2-methylpropanamid | 1H-NMR (400 MHz, d₆-DMSO): δ = 10.41 (s, 1H), 8.36 (s, 1H), 8.17 (dd, 1H), 8.13 (s, 1H), 7.88 (s, 1H), 7.82 (m, 1H), 7.69 (m, 1H), 7.62-7.58 (m, 1H), 7.43-7.41 (m, 2H), 7.21-7.18 (m, 2H), 6.81-6.79 (dd, 1H), 5.48 (s, 2H), 2.73 (m, 1H), 1.06 (d, 6H) ppm | - |
| 309 | N- {4-[1-(2-Cyanethyl)-3-(4-fluorphenyl)-1H-pyrazol-4-yl]pyridin-2-yl} -2-methylpropanamid | 1H-NMR (400 MHz, d₆-DMSO): δ = 10.42 (s, 1H), 8.26 (s, 1H), 8.18 (dd, 1H), 8.14 (s, 1H), 7.46-7.44 (m, 2H), 7.24-7.21 (m, 2H), 6.79 (dd, 1H), 4.48 (t, 2H), 3.17 (t, 2H), 2.73 (m, 1H), 1.07 (d, 6H) ppm | - |
| 310 | 4-[3-(4-Fluorphenyl)-1-isopropyl-1H-pyrazol-4-yl]-7H-pyrrolo[2,3-d]pyrimidin | 1H-NMR (400 MHz, d₃-CD3CN): δ = 9.90 (s, 1H), 8.63 (s, 1H), 8.11 (s, 1H), 7.55-7.53 (m, 2H), 7.28 (dd, 1H), 7.05-7.02 (m, 2H), 6.27 (dd, 1H), 4.62 (m, 1H), 1.57 (d, 6H) ppm | - |
| 311 | 4-[3-(4-Fluorphenyl)-1-isopropyl-1H-pyrazol-4-yl]-5,6,7,8-tetrahydropyrido[2,3-d]pyrimidin | 1H-NMR (400 MHz, d₃-CD3CN): δ = 8.15 (s, 1H), 7.69 (s, 1H), 7.47-7.44 (m, 2H), 7.05-7.02 (m, 2H), 5.89 (s, 1H), 4.55 (m, 1H), 3.28 (m, 1H), 2.35 (m, 2H), 1.67 (m, 2H), 1.52 (d, 6H) ppm | - |
| 312 | N-{4-[1-(1-Cyanpropan-2-yl)-3-(4-fluorphenyl)-1H-pyrazol-4-yl]pyridin-2-yl}propanamid | 1H-NMR (400 MHz, d₃-CD3CN): δ = 8.54 (s, 1H, br), 8.12-8.10 (m, 2H), 7.50-7.47 (m, 2H), 7.13-7.10 (m, 2H), 7.13-7.7.10 (m, 1H), 6.87 (dd, 1H), 4.77-4.74 (m, 1H), 3.08-2.98 (m, 2H), 2.39 (q, 2H), 1.64 (d, 3H),1.10 (t, 3H) ppm | - |
| 313 | N-{4-[1-(1-Cyanpropan-2-yl)-3-(4-fluorphenyl)-1H-pyrazol-4-yl]pyridin-2-yl}-2-methoxyacetamid | 1H-NMR (400 MHz, d₃-CD3CN): δ = 8.71 (s, 1H, br), 8.17-8.10 (m, 1H), 7.99 (s, 1H), 7.50-7.48 (m, 2H), 7.13-7.7.10 (m, 1H), 6.96 (dd, 1H), 4.78-4.75 (m, 1H), 3.08-2.99 (m, 2H), (m, 1H), 2.27 (d, 2H),1.64 (d, 3H), 1.14 (d, 2H) ppm | - |
| 314 | N-{4-[1-(1-Cyanpropan-2-yl)-3-(4-fluorphenyl)-1H-pyrazol-4-yl]pyridin-2-yl} -2-methylpropanamid | 1H-NMR (400 MHz, d₃-CD3CN): δ = 8.56 (s, 1H, br), 8.15-8.11 (m, 2H), 7.96 (s, 1H), 7.50-7.47 (m, 2H), 7.13-7.10 (m, 1H), 6.86 (dd, 1H), 4.76 (m, 2H), 3.08-2.99 (m, 2H), 2.65-2.60 (m, 1H), 2.27 (d, 2H),1.64 (d, 3H), 1.19 (d, 1H), 0.56 (m, 6H) ppm | - |
| 315 | N-{4-[1-(1-Cyanpropan-2-yl)-3-(4-fluorphenyl)-1H-pyrazol-4-yl]pyridin-2-yl} -2-cyclopropylacetamid | 1H-NMR (400 MHz, d₃-CD3CN): δ = 8.56 (s, 1H, br), 8.15-8.13 (m, 1H), 7.98 (s, 1H), 7.51-7.48 (m, 2H), 7.14-7.11 (m, 2H), 6.90 (dd, 1H), 5.29 (m, 1H), 4.78-4.75 (m, 1H), 3.09-3.00 (m, 2H), 2.27 (d, 2H),1.65 (d, 3H), 1.12-1.05 (m, 1H), 0.56 (m, 2H), 0.22 (m, 2H) ppm | - |
| 316 | N- {4-[5-(Difluormethoxy)-3-(4-fluorphenyl)-1-methyl-1H-pyrazol-4-yl]pyridin-2-yl}propanamid | 1H-NMR (400 MHz, d₃-CD3CN): δ = 8.58(s, 1H, br), 8.09 (s, 1H), 7.42-7.40 (m, 2H), 7.08-7.05 (m, 2H), 6.89 (dd, 1H), 3.82 (s, 3H), 2.50 (s, 1H), 2.39 (q, 2H), 1.11 (t, 2H) ppm | - |
| 317 | 4-[1-(Cyclopropylmethyl)-3-(4-fluorphenyl)-1H-pyrazol-4-yl]-N-(1,1,1-trifluorpropan-2-yl)pyridin-2-amin | 1H-NMR (400 MHz, d₃-CD3CN): δ = 7.92 (d, 1H), 7.86 (s, 1H), 7.49-7.44 (m, 2H), 7.10(m, 2H), 6.53 (dd, 1H), 6.42(m, 1H), 5.29 (m, 1H), 4.54 (d, 1H, br), 4.95-4.88 (m, 1H), 4.01 (d, 2H),1.36-1.29 (m, 4H), 0.63 (m, 2H), 0.42 (m, 2H) ppm | - |
| 318 | 4-[3-(4-Fluorphenyl)-1-isopropyl-1H-pyrazol-4-yl]-N-(1,1,1-trifluorpropan-2-yl)pyridin-2-amin | 1H-NMR (400 MHz, d₃-CD3CN): δ = 7.92 (d, 1H), 7.48-7.46 (m, 1H), 7.13-7.10 (m, 2H), 6.52 (dd, 2H), 6.50 (dd, 1H), 6.41 (m, 1H), 5.28(d, 1H, br), 4.90 (m, 1H), 4.54 (m, 1H), 1.50 (d, 6H), 1.31 (d, 3H) ppm | - |
| 319 | 4-[3-(4-Fluorphenyl)-1-methyl-1H-pyrazol-4-yl]-N-(1,1,1-trifluorpropan-2-yl)pyridin-2-amin | 1H-NMR (400 MHz, d₃-CD3CN): δ = 7.92 (d, 1H), 7.74 (s, 1H), 7.47-7.44 (m, 2H), 7.12-7.10 (m, 2H), 6.50 (dd, 1H), 6.40 (m, 1H), 5.30 (d, 1H, br), 4.91 (m, 1H), 3.89 (s, 3H), 1.30 (d, 2H) ppm | - |
| 320 | N-{4-[1-Cyclopropyl-3-(4-fluorphenyl)-1H-pyrazol-4-yl]pyridin-2-yl}propanamid | 1H-NMR (400 MHz, d₃-CD3CN): δ = 8.52 (s, 1H, br), 8.11 (d, 1H), 8.08 (s, 1H), 7.90 (s, 1H), 7.46-7.44 (m, 2H), 7.11-7.08 (m, 2H), 6.86 (dd, 1H), 3.71 (m, 1H), 2.37 (q, 2H), 1.16-1.15 (m, 2H), 1.10 (t, 3H), 1.04-1.02 (m, 2H) ppm | |
| 321 | N-{4-[1-Cyclopropyl-3-(4-fluorphenyl)-1H-pyrazol-4-yl]pyridin-2-yl}-2-methoxyacetamid | 1H-NMR (400 MHz, d₃-CD3CN): δ = 8.70 (s, 1H, br), 8.15 (d, 1H), 8.07 (s, 1H), 7.92 (m, 2H), 7.47-7.44 (m, 2H), 7.12-7.08 (m, 2H), 6.94 (dd, 1H), 3.72(m, 1H), 3.45 (s, 3H), 1.17-1.14 (m, 2H), 1.05-1.02 (m, 2H) ppm | - |
| 322 | N-{4-[1-Cyclopropyl-3-(4-fluorphenyl)-1H-pyrazol-4-yl]pyridin-2-yl} -2-methylpropanamid | 1H-NMR (400 MHz, d₃-CD3CN): δ = 8.51 (s, 1H, br), 8.11 (d, 2H), 7.90 (s, 1H), 7.46-7.44 (m, 2H), 7.10-7.07 (m, 2H), 6.85 (dd, 1H), 3.71 (m, 1H), 2.61(m, 1H), 1.17-1.13 (m, 8H), 1.05-1.02 (m, 2H) ppm | - |
| 323 | 2-Cyclopropyl-N- {4-[1-cyclopropyl-3-(4-fluorphenyl)-1H-pyrazol-4-yl]pyridin-2-yl} acetamid | 1H-NMR (400 MHz, d₃-CD3CN): δ = 8.53 (s, 1H, br), 8.12-8.10 (m, 2H), 7.90 (s, 1H), 7.47-7.44 (m, 2H), 7.11-7.07 (m, 2H), 6.88 (dd, 1H), 3.71 (m, 1H), 2.50(d, 2H), 1.17-1.14 (m, 2H), 1.11-1.60 (m, 3H) 0.55 (m, 2H), 0.22 (m, 2H) ppm | - |
| 324 | 4-[3-(4-Fluorphenyl)-1-isopropyl-1H-pyrazol-4-yl]-N-[2,2,2-trifluor-1-(4-fluorphenyl)ethyl]pyridin-2-amin | 1H-NMR (400 MHz, d₃-CD3CN): δ = 7.90(d, 1H), 7.82 (s, 1H), 7.53-7.50 (m, 2H), 7.46-7.44 (m, 2H), 7.17-7.09 (m, 4H), 6.56-6.53 (m, 2H), 6.09-6.04 (m, 1H), 4.53(q, 1H), 1.51 (d, 6H) ppm | - |
| 325 | 4-[3-(4-Fluorphenyl)-1-isopropyl-1H-pyrazol-4-yl]-N-(2,2,2-trifluor-1-phenylethyl)pyridin-2-amin | 1H-NMR (400 MHz, d₃-CD3CN): δ = 7.91(d, 1H), 7.82 (s, 1H), 7.50-7.38 (m, 7H), 7.12-7.09 (m, 2H), 6.54 (d, 2H), 6.09-6.01 (m, 2H), 4.53 (m, 1H), 1.51 (d, 6H), ppm | - |
| 326 | N-{4-[1-(2-Ethoxyethyl)-3-(4-fluorphenyl)-1H-pyrazol-4-yl]pyridin-2-yl}propanamid | 1H-NMR (400 MHz, d₃-CD3CN): δ = 8.52 (s, 1H, br), 8.11 (m, 2H), 7.87 (m, 1H), 7.90 (s, 1H), 7.48-7.43 (m, 2H), 7.12-7.08 (m, 2H), 6.74 (dd, 1H), 4.30 (t, 2H), 3.83 (t, 2H), 3.83 (t, 2H), 3.47 (q, 2H), 2.37 (q, 2H), 1.14-1.04 (t, 6H) ppm | - |
| 327 | N- {4-[1-(2-Ethoxyethyl)-3-(4-fluorphenyl)-1H-pyrazol-4-yl]pyridin-2-yl}-2-methoxyacetamid | 1H-NMR (400 MHz, d₃-CD3CN): δ = 8.70 (s, 1H, br), 8.15 (d, 1H), 8.09 (s, 1H), 7.90 (s, 1H), 7.48-7.46 (m, 2H), 7.12-7.09 (m, 2H), 6.94 (dd, 1H), 4.31 (t, 2H), 3.96 (s, 2H), 3.83 (t, 2H), 3.49 (q, 2H), 3.45 (s, 3H), 1.12 (t, 3H) ppm | - |
| 328 | N- {4-[-(2-Ethoxyethyl)-3-(4-fluorphenyl)-1H-pyrazol-4-yl]pyridin-2-yl} -2-methylpropanamid | 1H-NMR (400 MHz, d₃-CD3CN): δ = 8.51 (s, 1H, br), 8.11 (m, 2H), 7.88 (s, 1H), 7.48-7.45 (m, 2H), 7.11-7.09 (m, 2H), 6.86 (dd, 1H), 4.30 (t, 2H), 3.83 (t, 2H), 3.49 (q, 2H), 1.14-1.10 (m, 10H) ppm | - |
| 329 | 2-Cyclopropyl-N- {4-[1-(2-ethoxyethyl)-3-(4-fluorphenyl)-1H-pyrazol-4-yl]pyridin-2-yl}acetamid | 1H-NMR (400 MHz, d₃-CD3CN): δ = 8.54 (s, 1H, br), 8.11 (m, 2H), 7.88 (s, 1H), 7.48-7.41 (m, 2H), 7.12-7.09 (m, 2H), 6.77 (dd, 1H), 4.30 (t, 2H), 4.07 (2H, isomer), 3.83 (t, 2H), 3.73 (2H, isomer), 3.49 (q, 2H), 3.32 (2H, isomer), 2.41 (d, 2H), 2.22 (2H, isomer), 1.13 (t, 3H), 1.07 (m, 1H), 0.54 (m, 2H), 0.21 (m, 2H) ppm | - |
| 330 | Methyl-6-(4-[1-ethyl-3-(4-fluorphenyl)-1H-pyrazol-4-yl]pyridin-2-yl} amino)-6-oxohexanoat | 1H-NMR (400 MHz, d₃-CD3CN): δ = 8.18-8.16 (m, 2H), 8.08 (s, 1H), 7.44-7.42 (m, 2H), 7.21-7.18 (m, 2H), 6.77 (dd, 1H), 4.21 (q, 2H), 3.58 (s, 1H), 2.39-2.31 (m, 4H), 1.56-1.52 (m, 4H), 1.45 (t, 3H) ppm | - |
| 331 | Methyl-5-({4-[1-ethyl-3-(4-fluorphenyl)-1H-pyrazol-4-yl]pyridin-2-yl}amino)-5-oxopentanoat | 1H-NMR (400 MHz, d₃-CD3CN): δ = 8.18-8.16 (m, 2H), 8.07 (s, 1H), 7.44-7.42 (m, 2H), 7.21-7.18 (m, 2H), 6.83 (dd, 1H), 4.21 (q, 2H), 3.59 (s, 1H), 2.41-2.39 (m, 2H), 2.33 (t, 2H), 1.82-1.77 (t, 3H) ppm | - |
| 332 | Ethyl-3-ethyl-5-({4-[1-ethyl-3-(4-fluorphenyl)-1H-pyrazol-4-yl]pyridin-2-yl}amino)-5-oxopentanoat | 1H-NMR (400 MHz, d₃-CD3CN): δ = 8.18-8.16 (m, 2H), 8.07 (s, 1H), 7.44-7.41 (m, 2H), 7.20-7.17 (m, 2H), 6.84 (dd, 1H), 4.21 (q, 2H), 4.03 (q, 2H), 2.33-2.19(m, 4H), 1.45 (t, 3H), 1.34-1.29 (m, 2H), 1.16 (t, 3H), 0.84 (t, 3H) ppm | - |
| 333 | 3-(Difluormethyl)-N- {4-[1-ethyl-3-(4-fluorphenyl)-1H-pyrazol-4-yl]pyridin-2-yl} -1-methyl-1H-pyrazol-4-carboxamid | 1H-NMR (400 MHz, d₃-CD3CN): δ = 8.67 (s, 1H, br), 8.23 (m, 2H), 8.14 (m, 1H), 7.46-7.43 (m, 2H), 7.23-7.20 (m, 2H), 6.89-6.88 (dd, 1H), 4.22 (q, 2H), 3.64(s, 3H), 1.46 (t, 3H) ppm | - |
| 334 | 2-(Difluormethyl)-N-{4-[1-ethyl-3-(4-fluorphenyl)-1H-pyrazol-4-yl]pyridin-2-yl}thiophen-3-carboxamid | 1H-NMR (400 MHz, d₃-CD3CN): δ = 8.28 (d, 1H), 8.24 (s, 1H), 8.22 (m, 1H), 8.13 (m, 1H), 7.55-7.54 (m, 1H), 7.47-7.44 (m, 2H), 7.24-7.21 (m, 2H), 6.94(dd, 1H), 4.24 (q, 2H), 1.46 (t, 3H) ppm | - |
| 335 | N- {4-[1-Ethyl-3-(4-fluorphenyl)-1H-pyrazol-4-yl]pyridin-2-yl}-4-methyl-1,2,3-thiadiazol-5-carboxamid | 1H-NMR (400 MHz, d₃-CD3CN): δ = 8.27 (m, 2H), 8.08 (s, 1H), 7.47-7.44 (m, 2H), 7.24-7.21 (m, 2H), 7.00 (dd, 1H), 4.22 (q, 2H), 2.78 (s, 3H), 1.46 (t, 3H) ppm | - |
| 336 | 3-(4-Chlorphenyl)-N-{4-[1-ethyl-3-(4-fluorphenyl)-1H-pyrazol-4-yl]pyridin-2-yl}propanamid | 1H-NMR (400 MHz, d₃-CD3CN): δ = 8.17 (m, 2H), 8.06 (s, 1H, br), 7.44-7.41 (m, 2H), 7.43-7.32 (m, 2H), 7.26 (m, 2H), 7.22-7.19 (m, 2H), 6.85 (dd, 1H), 4.21 (q, 2H), 2.87 (t, 2H), 2.64 (t, 2H), 1.45 (t, 3H) ppm | - |
| 337 | (1R,2R)-N-{4-[1-Ethyl-3-(4-fluorphenyl)-1H-pyrazol-4-yl]pyridin-2-yl}-2-phenylcyclopropancarboxamid | 1H-NMR (400 MHz, d₆-DMSO): δ = 8.18-8.15 (m, 2H), 8.10 (s, 1H), 7.44-7.41 (m, 2H), 7.32-7.28 (m, 2H), 7.23-7.15 (m, 5H), 6.82-6.81 (dd, 1H), 4.23-4.19 (q, 2H), 2.35 (m, 1H), 1.43 (t, 3H), 1.43 (m, 1H), 1.35 (m, 1H) ppm | - |
| 338 | 2-Cyclopentyl-N- {4-[1-ethyl-3-(4-fluorphenyl)-1H-pyrazol-4-yl]pyridin-2-yl}acetamid | 1H-NMR (400 MHz, d₆-DMSO): δ = 8.18 (s, 1H), 8.16-8.15 (m, 1H), 8.07 (s, 1H), 7.43-7.41 (m, 2H), 7.21-7.18 (m, 2H), 6.83-6.82 (dd, 1H), 4.22-4.18 (m, 2H), 2.32 (d, 2H), 2.18 (m, 1H), 1.70 (m, 1H), 1.60 (m, 1H), 1.50 (m, 1H), 1.43 (t, 3H), 1.15 (m, 1H) ppm | - |
| 339 | N-{4-[1-Ethyl-3-(4-fluorphenyl)-1H-pyrazol-4-yl]pyridin-2-yl}-3-methylbutanamid | 1H-NMR (400 MHz, d₃-CD3CN): δ = 8.18-8.16 (m, 2H), 8.08 (s, 1H, br), 7.44-7.41 (m, 2H), 7.21-7.18 (m, 2H), 6.84 (dd, 1H), 4.21 (m, 2H), 2.23 (d, 2H), 2.05-2.00 (m, 1H), 1.45 (t, 3H), 0.90 (d, 6H) ppm | - |
| 340 | N-{4-[1-Ethyl-3-(4-fluorphenyl)-1H-pyrazol-4-yl]pyridin-2-yl}-2-methylbutanamid | 1H-NMR (400 MHz, d₃-CD3CN): δ = 8.19-8.16 (m, 2H), 8.12 (s, 1H, br), 7.44-7.41 (m, 2H), 7.22-7.18 (m, 2H), 6.82 (dd, 1H), 4.21 (m, 1H), 1.45 (t, 3H), 1.38-1.34 (m, 1H), 1.21 (m, 1H), 1.04 (d, 3H), 0.83 (t, 3H) ppm | - |
| 341 | N- {4-[1-Ethyl-3-(4-fluorphenyl)-1H-pyrazol-4-yl]pyridin-2-yl}-2-methylcyclopropancarboxamid | 1H-NMR (400 MHz, d₆-DMSO): δ = 10.67 (m, 1H), 8.17-8.14 (m, 2H), 8.05 (s, 1H), 7.43-7.40 (m, 2H), 7.21-7.17 (m, 2H), 6.82-6.79 (dd, 1H), 4.22-4.18 (m, 2H), 1.72 (m, 1H), 1.45 (m, 2H), 1.20 (m, 1H), 1.07 (d, 3H), 0.90 (m, 1H), 0.60 (m, 1H) ppm | - |
| 342 | N-{4-[1-Ethyl-3-(4-fluorphenyl)-1H-pyrazol-4-yl]pyridin-2-yl}acetamid | 1H-NMR (400 MHz, d₃-CD3CN): δ = 8.17 (m, 2H), 8.03(s, 1H, br), 7.44-7.41 (m, 2H), 7.22-7.18 (m, 2H), 6.86 (dd, 1H), 4.21 (m, 1H), 4.21 (q, 2H), 2.05 (s, 3H), 1.45 (t, 3H) ppm | - |
| 343 | N-{4-[3-(4-Fluorphenyl)-1-methyl-1H-pyrazol-4-yl]pyridin-2-yl}-3-phenylpropanamid | 1H-NMR (400 MHz, d₃-CD3CN): δ = 8.16 (d, 1H), 8.13(s, 1H), 8.07 (s, 1H, br), 7.44-7.41 (m, 2H), 7.29-7.26 (m, 2H), 7.23-7.17 (m, 5H), 6.82 (dd, 1H), 3.92 (s, 3H), 2.86 (t, 2H), 2.67 (t, 2H) ppm | - |
| 344 | 4-Fluor-N- {4-[3-(4-fluorphenyl)-1-methyl-1H-pyrazol-4-yl]pyridin-2-yl}benzamid | 1H-NMR (400 MHz, d₃-CD3CN): δ = 8.27 (d, 1H), 8.19(s, 1H), 8.16 (s, 1H), 8.09-8.06 (m, 2H), 7.47-7.45 (m, 2H), 7.35-7.32 (m, 2H), 7.24-7.21 (m, 2H), 6.92 (dd, 1H), 3.94 (s, 3H) ppm | - |
| 345 | N-{4-[3-(4-Fluorphenyl)-1-methyl-1H-pyrazol-4-yl]pyridin-2-yl}-2-(2-methoxyethoxy)acetamid | 1H-NMR (400 MHz, d₃-CD3CN): δ = 8.20 (d, 1H), 8.19(s, 1H), 8.15 (s, 1H), 8.05 (s, 1H, br), 7.44-7.41 (m, 2H), 7.22-7.20 (m, 2H), 6.90 (dd, 1H), 4.10 (s, 2H), 3.91 (s, 3H), 3.66-3.64 (m, 2H), 3.51-3.50 (m, 2H), 3.29 (s, 3H) ppm | - |
| 346 | N-{4-[3-(4-Fluorphenyl)-1-methyl-1H-pyrazol-4-yl]pyridin-2-yl}benzamid | 1H-NMR (400 MHz, d₃-CD3CN): δ = 8.24 (m, 1H), 8.18(s, 2H), 8.00 (m, 2H), 7.60-7.58 (m, 1H), 7.52-7.45 (m, 4H), 7.23-7.21 (m, 2H), 6.92 (dd, 1H), 3.94 (s, 3H) ppm | - |
| 347 | N-{4-[3-(4-Fluorphenyl)-1-methyl-1H-pyrazol-4-yl]pyridin-2-yl}-3-methylbutanamid | 1H-NMR (400 MHz, d₃-CD3CN): δ = 8.17 (m, 1H), 8.14 (s, 1H), 8.07 (s, 1H, br), 7.43-7.41 (m, 2H), 7.21-7.18 (m, 2H), 6.83 (dd, 1H), 3.92 (s, 3H), 2.23 (d, 2H), 2.05-2.00 (m, 1H) ppm | - |
| 348 | N-{4-[3-(4-Fluorphenyl)-1-methyl-1H-pyrazol-4-yl]pyridin-2-yl}-2-methylbutanamid | 1H-NMR (400 MHz, d₃-CD3CN): δ = 8.16 (m, 1H), 8.14 (s, 1H), 8.11 (s, 1H), 7.44-7.41 (m, 2H), 7.21-7.18 (m, 2H), 6.80 (dd, 1H), 3.92 (s, 3H), 1.59-1.55 (m, 1H), 1.38-1.35 (m, 1H), 1.23 (s, 1H, br), 1.04(d, 3H), 0.84 (t, 3H) ppm | - |
| 349 | N-{4-[3-(4-Fluorphenyl)-1-methyl-1H-pyrazol-4-yl]pyridin-2-yl}cyclobutancarboxamid | 1H-NMR (400 MHz, d₆-DMSO): δ = 10.27 (s, 1H), 8.15-8.11 (m, 3H), 7.43-7.41 (m, 2H), 7.22-7.19 (m, 2H), 6.80-6.78 (dd, 1H), 3.92 (s, 3H), 2.15 (m, 2H), 2.05 (m, 2H), 1.90 (m, 1H), 1.23 (m, 2H) ppm | - |
| 350 | N-{4-[3-(4-Fluorphenyl)-1-methyl-1H-pyrazol-4-yl]pyridin-2-yl}acetamid | 1H-NMR (400 MHz, d₃-CD3CN): δ = 8.12 (d, 1H), 8.12 (s, 1H), 8.03 (s, 1H,br), 7.43-7.41 (m, 2H), 7.21-7.18 (m, 2H), 6.84 (dd, 1H), 3.92 (s, 3H), 2.05 (s, 3H) ppm | - |
| 351 | N-{4-[1-Ethyl-3-(4-fluorphenyl)-1H-pyrazol-4-yl]pyridin-2-yl}ethandiamid | 1H-NMR (400 MHz, d₃-CD3CN): δ = 8.42 (s, 1H, br), 8.28 (d, 1H), 8.24 (s, 1H), 8.12 (s, 1H, br), 7.99 (s, 1H, br), 7.47-7.43 (m, 2H), 7.23-7.20 (m, 2H), 7.05-7.04 (m, 1H), 4.22(q, 2H), 1.46 (t, 3H) ppm | - |
| 352 | (2S)-N-{4-[1-Ethyl-3-(4-fluorphenyl)-1H-pyrazol-4-yl]pyridin-2-yl}-2-hydroxypropanamid | 1H-NMR (400 MHz, d₃-CD3CN): δ = 9.56 (s, 1H), 8.21 (s, 1H), 8.19 (d, 1H), 8.07 (s, 1H, br), 7.45-7.42 (m, 2H), 7.22-7.19 (m, 2H), 6.90 (dd, 1H), 5.90 (d, 1H), 4.23-4.16(m, 3H), 1.45 (t, 3H), 1.28 (d, 3H) ppm | - |
| 353 | (2S)-N-{4-[3-(4-Fluorphenyl)-1-methyl-1H-pyrazol-4-yl]pyridin-2-yl}-2-hydroxypropanamid | 1H-NMR (400 MHz, d₃-CD3CN): δ = 9.56 (s, 1H), 8.20 (d, 1H), 8.16 (s, 1H), 8.07 (s, 1H, br), 7.44-7.41 (m, 2H), 7.22-7.19 (m, 2H), 6.90 (dd, 1H), 4.18 (m, 1H), 3.92 (s, 1H), 1.45 (t, 3H), 1.28 (d, 3H) ppm | - |
| 354 | N-{4-[3-(4-Fluorphenyl)-1-(propan-2-yl)-1H-pyrazol-4-yl]pyrimidin-2-yl}-2-methoxyacetamid | 1H-NMR (400 MHz, d₆-DMSO): δ = 10.15 (bs, 1H), 8.49 (d, 1H), 8.44 (s, 1H), 7.60 (m, 2H), 7.24 (t, 1H), 6.99 (d, 1H), 4.61 (m, 1H), 4.07 (s, 2H), 3.33 (s, 3H), 1.51 (d, 6H) ppm | - |
| 355 | N-{4-[3-(4-Fluorphenyl)-1-(propan-2-yl)-1H-pyrazol-4-yl]pyrimidin-2-yl}-2-methylpropanamid | 1H-NMR (400 MHz, d₆-DMSO): δ = 10.33 (bs, 1H), 8.47 (d, 1H), 8.37 (s, 1H), 7.66 (m, 2H), 7.23 (t, 1H), 6.92 (d, 1H), 4.62 (m, 1H), 2.84 (m, 1H), 1.50 (d, 6H), 1.05 (d, 6H) ppm | - |
| 356 | N-{4-[3-(4-Fluorphenyl)-1-(propan-2-yl)-1H-pyrazol-4-yl]pyrimidin-2-yl}cyclopropancarboxamid | 1H-NMR (400 MHz, d₆-DMSO): δ = 10.63 (s, 1H), 8.47 (d, 1H), 8.36 (s, 1H), 7.66 (m, 2H), 7.22 (t, 2H), 6.94 (d, 1H), 4.61 (m, 1H), 2.16 (m, 1H), 1.5 (d, 6H), 0.8 (m, 2H), 0.74 (m, 2H) ppm | - |
| 357 | N-{4-[3-(4-Fluorphenyl)-1-(2-methylpropyl)-1H-pyrazol-4-yl]pyrimidin-2-yl} acetamid | 1H-NMR (400 MHz, d₃-CD3CN): δ = 8.25 (d, 2H), 7.96 (s, 1H), 7.43 (m, 2H), 7.05 (t, 2H), 6.69 (d, 1H), 3.92 (d, 2H), 2.39 (s, 3H), 2.24 (m, 1H), 0.92 (d, 6H), ppm | - |
| 358 | 4-[3-(4-Fluorphenyl)-1-(2-methoxyethyl)-1H-pyrazol-4-yl]-N-(propan-2-yl)pyrimidin-2-amin | 1H-NMR (400 MHz, d₆-DMSO): δ = 8.26 (bs, 1H), 8.11 (d, 1H), 7.56 (m, 2H), 7.22 (t, 2H), 6.78 (d, 1H), 6.41 (bs, 1H), 4.34 (t, 2H), 4.01 (t, 2H, isomer), 3.76 (t, 2H), 3.65 (t, 2H, isomer), 3.28 (s, 3H), 3.12 (s, 3H, isomer), 1.06 (bs, 6H), 0.96 (bs, 6H, isomer) ppm | 76/23 |
| 359 | 4-[1-(2-Chlorethyl)-3-(4-fluorphenyl)-1H-pyrazol-4-yl]-N-(propan-2-yl)pyrimidin-2-amin | 1H-NMR (400 MHz, d₆-DMSO): δ = 8.36 (bs, 1H), 8.12 (d, 1H), 7.56 (m, 2H), 7.23 (t, 2H), 6.80 (d, 1H), 6.40 (bs, 1H), 4.54 (t, 2H), 4.20 (t, 2H, isomer), 4.08 (t, 2H), 3.97 (t, 2H, isomer), 1.06 (bs, 6H), 0.95 (bs, 6H, isomer) ppm | 70/30 |
| 360 | 4-[1-(Butan-2-yl)-3-(4-fluorphenyl)-1H-pyrazol-4-yl] -N-(propan-2-yl)pyrimidin-2-amin | 1H-NMR (400 MHz, CDCl₃): δ = 7.99 (d, 1H), 7.89 (s, 1H), 7.46 (m, 2H), 7.01 (t, 2H), 6.26 (d, 1H), 4.87 (bd, 1H), 4.19 (m, 1H), 4.03 (m, 1H), 1.79(m, 2H), 1.49 (d, 3H), 1.15 (d, 6H), 0.83 (t, 3H) ppm | - |
| 361 | 4-[3-(4-Fluorphenyl)-1-methyl-1H-pyrazol-4-yl]-N-(propan-2-yl)pyrimidin-2-amin | 1H-NMR (400 MHz, d₆-DMSO): δ = 8.25 (bs, 1H), 7.55 (m, 2H), 7.21 (t, 2H), 6.76 (d, 1H), 6.38 (bs, 1H), 3.23 (s, 3H), 1,06 (bs, 6H) ppm | - |
| 362 | 3-{3-(4-Fluorphenyl)-4-[2-(propan-2-ylamino)pyrimidin-4-yl]-1H-pyrazol-1-yl}propanonitril | 1H-NMR (400 MHz, d₆-DMSO): δ = 8.38 (bs, 1H), 8.13 (d, 1H), 7.56 (m, 2H), 7.24 (t, 2H), 6.82 (d, 1H), 6.39 (bs, 1H), 4.48 (t, 2H), 3.75 (bs, 1H), 3.16 (t, 2H), 1.07 (bs, 6H) ppm | - |
| 363 | 4-[3-(4-Fluorphenyl)-1-(prop-2-in-1-yl)-1H-pyrazol-4-yl]-N-(propan-2-yl)pyrimidin-2-amin | 1H-NMR (400 MHz, d₆-DMSO): δ = 8.34 (bs, 1H), 8.12 (d, 1H), 7.55 (m, 2H), 7.23 (t, 2H), 6.83 (d, 1H), 6.38 (bs, 1H), 5.14 (s, 2H), 3.59 (m, 1H), 1.07 (bs, 6H) ppm | - |
| 364 | 4-[1-(2,2-Difluorethyl)-3-(4-fluorphenyl)-1H-pyrazol-4-yl]-N-(propan-2-yl)pyrimidin-2-amin | 1H-NMR (400 MHz, d₆-DMSO): δ = 8.34 (bs, 1H), 8.12 (d, 1H), 7.55 (m, 2H), 7.24 (t, 2H), 6.84 (d, 1H), 6.46 (tt, 1H), 6.40 (bs, 1H), 4.73 (td, 2H), 3.85 (bs, 1H), 1.07 (bs, 6H) ppm | - |
| 365 | N-{4-[3-(4-Fluorphenyl)-1-(2-methylpropyl)-1H-pyrazol-4-yl]pyrimidin-2-yl}propanamid | 1H-NMR (400 MHz, d₆-DMSO): δ = 10.30 (s, 1H), 8.45 (d, 1H), 8.34 (s, 1H), 7.62 (m, 2H), 7.24 (t, 2H), 6.91 (d, 1H), 4.03 (d, 2H), 2.41 (q, 2H), 2.28 (m, 1H), 0.99 (t, 3H), 0.91 (d, 6H) ppm | - |
| 366 | 4-[3-(4-Fluorphenyl)-1-(propan-2-yl)-1H-pyrazol-4-yl]pyrimidin-2-amin | 1H-NMR (400 MHz, d₆-DMSO): δ = 8.23 (s, 1H), 8.08 (d, 1H), 7.55 (m, 2H), 7.23 (t, 2H), 6.47 (s, 1H)6.76 (d, 1H), 6.35 (d, 1H), 4.57 (m, 1H), 1.48 (d, 6H) ppm | - |
| 367 | 4-[1-(2,2-Difluorethyl)-3-(4-fluorphenyl)-5-methyl-1H-pyrazol-4-yl]pyridin | 1H-NMR (400 MHz, d₆-DMSO): δ = 8.55 (d, 2H), 8.44 (d, 2H, isomer), 7.35 (m, 4H), 7.17 (m, 2H), 7.07 (dd, 2H, isomer), 6.47 (tt, 1H), 4.72 (td, 2H), 4.38 (td, 2H, isomer), 2.31 (s, 3H) ppm | 66/23 |
| 368 | 4-[3-(4-Fluorphenyl)-5-methyl-1-(2-methylpropyl)-1H-pyrazol-4-yl]pyridin | 1H-NMR (400 MHz, d₆-DMSO): δ = 8.53 (d, 2H), 8.41 (d, 2H, isomer), 7.33 (m, 4H), 7.15 (m, 4H), 7.03 (d, 2H, isomer), 3.96 (d, 2H), 3.74 (d, 2H, isomer), 2.28 (m, 4H), 0.94 (d, 6H), 0.72 (d, 6H,isomer) ppm | 64/28 |
| 369 | N-Benzyl-4-[3-(4-fluorphenyl)-1-(propan-2-yl)-1H-pyrazol-4-yl]pyrimidin-2-amin | 1H-NMR (400 MHz, d₆-DMSO): δ = 8.47 (bs, 1H), 8.18 (bs, 1H), 7.57 (m, H), 7.22 (m, 8H), 6.72 (bs, 1H), 4.58 (m, 1H), 4.32 (bs, 2H), 1.47 (d, 6H) ppm | - |
| 370 | 4-[1-Ethyl-3-(4-fluorphenyl)-1H-pyrazol-4-yl]-N-(propan-2-yl)pyrimidin-2-amin | 1H-NMR (400 MHz, d₆-DMSO): δ = 8.29 (bs, 1H), 8.10 (d, 1H), 7.56 (m, 2H), 7.21 (t, 2H), 6.76 (d, 1H), 6.41 (bs, 1H), 4.21 (q, 2H), 3.90 (q, 2H, isomer), 3.68 (bs, 1H), 1.44 (t, 3H), 1.24 (t, 3H, isomer), 1.06 (bs, 6H), 0.95 (bs, 6H, isomer) ppm | - |
| 371 | 4-[3-(4-Fluorphenyl)-1-(propan-2-yl)-1H-pyrazol-4-yl]-N-(propan-2-yl)pyrimidin-2-amin | 1H-NMR (400 MHz, d₃-CD3CN): δ = 8.06 (d, 1H), 7.97 (s, 1H), 7.54 (m, 2H), 7.08 (t, 2H), 6.33 (d, 1H), 4.95 (d, 1H), 4.55 (m, 1H), 4.11 (m, 1H), 1.58 (d, 6H), 1.22 (d, 6H), ppm | - |
| 372 | N-(1-Cyclopropylethyl)-4-[3-(4-fluorphenyl)-1-(2-methylpropyl)-1H-pyrazol-4-yl]pyrimidin-2-amin | 1H-NMR (400 MHz, d₃-CD3CN): δ = 8.08 (d, 1H), 7.95 (s, 1H), 7.54 (m, 2H), 7.11 (t, 2H), 6.36 (d, 1H), 5.25 (bs, 1H), 3.98 (d, 2H), 3.45 (m, 1H), 2.33 (m, 1H), 127 (d, 3H),1.02 (d, 6H), 0.92 (m, 1H), 0.5 (m, 2H), 0.37 (m, 1H), 0.26 (m, 1H) ppm | - |

| | | | |
|---|---|---|---|
| ¹ Das Isomerenverhältnis bezieht sich auf eine gegebenenfalls erhaltene Mischung verschiedener Pyrazolisomerer (Das Hauptisomer ist das 1-substituierte-3-Aryl-1H-pyrazol, das Minderisomer ist das entsprechende 1-substituierte-5-Aryl-1H-pyrazol). Wenn kein Isomerenverhältnis angegeben wurde, liegt ausschliesslich das Hauptisomer vor. | | | |

**Tabelle 3 - Massenspektroskopische / logP Daten der hergestellten Verbindungen des Typs [I-a] und [I-b]**

| **Nr.** | **Name** | **LogP_{A}¹** | **[M+H]_{A}⁺²** | **LogP_{B}³** | **[M+H]_{B}⁺²** | **Isomerenverhäl tnis⁴** | **Methode** ⁵ |
|---|---|---|---|---|---|---|---|
| 1 | 4-[3-(4-Fluorphenyl)-1-(2-methylpropyl)-1H-pyrazol-4-yl]pyridin | 1.63 | 296 | - | - | - | C |
| 2 | 4-[5-(2,6-Difluorphenyl)-1-(2-methylpropyl)-1H-pyrazol-4-yl]pyridin | 1.86 | 314 | - | - | - | C |
| 3 | 4-[1-(Butan-2-yl)-3-(2,6-difluorphenyl)-1H-pyrazol-4-yl]pyridin | 1.82 | 314 | - | - | - | C |
| 5 | 4-[3-(3-Chlorphenyl)-1-(2-methylpropyl)-1H-pyrazol-4-yl]pyrimidin | 3.27 | 313 | - | - | - | C |
| 6 | 4-[5-(2,6-Difluorphenyl)-1-(2,2-dimethylpropyl)-1H-pyrazol-4-yl]pyridin | 1.79 | 328 | - | - | - | C |
| 7 | 4-[3-(2,6-Difluorphenyl)-1-(3-methylbut-2-en-1-yl)-1H-pyrazol-4-yl]pyridin | 1.69 | 326 | - | - | - | C |
| 8 | 4-[1-Cyclobutyl-3-(2,6-difluorphenyl)-1H-pyrazol-4-yl]pyridin | 1.56 | 312 | - | - | - | C |
| 9 | 4-[3-(4-Fluorphenyl)-1-(tetrahydro-2H-pyran-2-yl)-1H-pyrazol-4-yl]pyridin | 1.29 | 324 | - | - | - | B |
| 10 | 4-[5-(2,6-Difluorphenyl)-1-(propan-2-yl)-1H-pyrazol-4-yl]pyridin | 1.54 | 300 | - | - | - | C |
| 11 | 4-[3-(2,6-Difluorphenyl)-1-(2,2-dimethylpropyl)-1H-pyrazol-4-yl]pyridin | 1.84 | 328 | - | - | - | C |
| 12 | 4-[3-(2,6-Difluorphenyl)-1-(2,2,2-trifluorethyl)-1H-pyrazol-4-yl]pyridin | 1.39 | 340 | - | - | - | C |
| 13 | 4-[3-(2,6-Difluorphenyl)-1-(2-methylpropyl)-1H-pyrazol-4-yl]pyridin | 1.62 | 314 | - | - | - | C |
| 14 | 4-[3-(2,6-Difluorphenyl)-1-(propan-2-yl)-1H-pyrazol-4-yl]pyridin | 1.4 | 300 | - | - | - | C |
| 15 | 4-[1-Cyclobutyl-5-(2,6-difluorphenyl)-1H-pyrazol-4-yl]pyridin | 1.49 | 312 | - | - | - | C |
| 16 | 4-[1-Cyclopentyl-3-(2,6-difluorphenyl)-1H-pyrazol-4-yl]pyridin | 1.79 | 326 | - | - | - | C |
| 17 | 4-[1-Cyclopentyl-5-(2,6-difluorphenyl)-1H-pyrazol-4-yl]pyridin | 1.74 | 326 | - | - | - | C |
| 19 | N-{4-[3-(4-Fluorphenyl)-1-(tetrahydro-2H-pyran-2-yl)-1H-pyrazol-4-yl]pyridin-2-yl}propanamid | 2.32 | 395 | - | - | - | A |
| 20 | 4-[3-(4-Fluorphenyl)-1-(tetrahydro-2H-pyran-2-yl)-1H-pyrazol-4-yl]-1H-pyrrolo[2,3-b]pyridin | 2.08 | 363 | - | - | - | B |
| 21 | N-{4-[3-(4-Fluorphenyl)-1-(tetrahydro-2H-pyran-2-yl)-1H-pyrazol-4-yl]pyridin-2-yl}-2-methylpropanamid | 2.76 | 409 | - | - | - | B |
| 22 | 4-[5-(2,6-Difluorphenyl)-1-(3-methylbut-2-en-1-yl)-1H-pyrazol-4-yl]pyridin | 1.72 | 326 | - | - | - | C |
| 23 | N-{4-[3-(4-Fluorphenyl)-1-(tetrahydro-2H-pyran-2-yl)-1H-pyrazol-4-yl]pyridin-2-yl}-2-methoxyacetamid | 2.64 | 411 | - | - | - | B |
| 24 | 4-[5-(4-Fluorphenyl)-1-(2-methylpropyl)-1H-pyrazol-4-yl]pyridin | 1.59 | 296 | - | - | - | C |
| 25 | 4-[5-(4-Fluorphenyl)-1-(3-methylbut-2-en-1-yl)-1H-pyrazol-4-yl]pyridin | 2.68 | 308 | - | - | - | C |
| 26 | 4- [3-(2,6-Difluorphenyl)-1-(tetrahydrofuran-2-ylmethyl)-1H-pyrazol-4-yl]pyridin | 1.16 | 342 | - | - | - | C |
| 27 | 4-[5-(2,6-Difluorphenyl)-1-(tetrahydrofuran-2-ylmethyl)-1H-pyrazol-4-yl]pyridin | 1.21 | 342 | - | - | - | C |
| 28 | 4-[3-(3-Chlorphenyl)-1-(tetrahydro-2H-pyran-2-yl)-1H-pyrazol-4-yl]pyridin | 1.55 | 340 | - | - | - | B |
| 29 | 4-[3-(4-Fluorphenyl)-1-(tetrahydro-2H-pyran-2-yl)-1H-pyrazol-4-yl]-2-methylpyrimidin | 2.53 | 339 | - | - | - | B |
| 30 | 4-[3-(4-Fluorphenyl)-1-(3-methylbut-2-en-1-yl)-1H-pyrazol-4-yl]pyridin | 2.11 | 308 | - | - | - | C |
| 31 | 4-[3-(4-Fluorphenyl)-1-(tetrahydro-2H-pyran-2-yl)-1H-pyrazol-4-yl]-2-methylpyridin | 1.38 | 338 | - | - | - | B |
| 32 | 4-[3-(3-Chlorphenyl)-1-(tetrahydro-2H-pyran-2-yl)-1H-pyrazol-4-yl]-1H-pyrrolo[2,3-b]pyridin | 2.46 | 379 | - | - | - | B |
| 33 | 4-[5-(2,2-Difluor-1,3-benzodioxol-4-yl)-1-(tetrahydro-2H-pyran-2-yl)-1H-pyrazol-4-yl]pyridin | 1.64 | 386 | - | - | - | B |
| 34 | 4-[3-(3-Chlorphenyl)-1-(3-methylbut-2-en-1-yl)-1H-pyrazol-4-yl]pyrimidin | 3.5 | 325 | - | - | - | C |
| 36 | 4-(6-{1-(Tetrahydro-2H-pyran-2-yl)-3-[3-(trifluormethyl)phenyl]-1H-pyrazol-4-yl}pyrimidin-4-yl)morpholin | 2.1 | 460 | - | - | - | B |
| 37 | 4-[5-(4-Fluor-2-methylphenyl)-1-(tetrahydro-2H-pyran-2-yl)-1H-pyrazol-4-yl]pyridin | 1.40 | 338 | - | - | - | B |
| 38 | 4-[1-(Butan-2-yl)-5-(2,6-difluorphenyl)-1H-pyrazol-4-yl]pyridin | 1.9 | 314 | - | - | - | C |
| 39 | 4-[3-(4-Fluorphenyl)-1-(tetrahydro-2H-pyran-2-yl)-1H-pyrazol-4-yl]pyridin-2-amin | 1.38 | 339 | - | - | - | B |
| 40 | N-{4-[3-(4-Fluorphenyl)-1-(propan-2-yl)-1H-pyrazol-4-yl]pyridin-2-yl}-2-methoxyacetamid | 2.59 | 369 | - | - | - | A |
| 41 | N-{4-[3-(4-Fluorphenyl)-1-(tetrahydro-2H-pyran-2-yl)-1H-pyrazol-4-yl]pyridin-2-yl}-2,2-dimethylpropanamid | 3.19 | 423 | - | - | - | B |
| 42 | 4-{1-(Tetrahydro-2H-pyran-2-yl)-3-[3-(trifluormethyl)phenyl]-1H-pyrazol-4-yl}pyridin | 1.84 | 374 | - | - | - | B |
| 43 | 4-[3-(4-Methoxyphenyl)-1-(tetrahydro-2H-pyran-2-yl)-1H-pyrazol-4-yl]pyridin | 1.24 | 336 | - | - | - | B |
| 44 | 4-[3-(4-Fluorphenyl)-1-(propan-2-yl)-1H-pyrazol-4-yl]chinolin | 2.24 | 332 | - | - | - | B |
| 45 | 4-[5-(4-Methoxynaphthalen-1-yl)-1-(tetrahydro-2H-pyran-2-yl)-1H-pyrazol-4-yl]pyridin | 1.69 | 386 | - | - | - | B |
| 46 | 4-[3-(4-Fluorphenyl)-1-(propan-2-yl)-1H-pyrazol-4-yl]pyridin-2-amin | 1.18 | 297 | - | - | - | A |
| 47 | 4-[3-(4-Fluorphenyl)-1-(propan-2-yl)-1H-pyrazol-4-yl]-1-(prop-2-in-1-yl)-1H-pyrrolo[2,3-b]pyridin | 3.63 | 359 | - | - | 80:20 | B |
| 48 | 2,2-Dimethyl-N-(4-{1-(tetrahydro-2H-pyran-2-yl)-3-[3-(trifluormethyl)phenyl]-1H-pyrazol-4-yl}pyridin-2-yl)propanamid | 4.1 | 473 | - | - | - | B |
| 49 | 4-[3-(4-Fluorphenyl)-1-(tetrahydro-2H-pyran-2-yl)-1H-pyrazol-4-yl]-1-(prop-2-in-1-yl)-1H-pyrrolo[2,3-b]pyridin | 3.47 | 401 | - | - | - | B |
| 50 | 4-{1-(Tetrahydro-2H-pyran-2-yl)-3-[3-(trifluormethyl)phenyl]-1H-pyrazol-4-yl}-1H-pyrrolo[2,3-b]pyridin | 2.93 | 413 | - | - | - | B |
| 51 | 4-[5-(3-Chlor-4-fluorphenyl)-1-(tetrahydro-2H-pyran-2-yl)-1H-pyrazol-4-yl]pyridin | 1.57 | 359 | - | - | - | B |
| 52 | 4-{6-[3-(4-Fluorphenyl)-1-(tetrahydro-2H-pyran-2-yl)-1H-pyrazol-4-yl]pyrimidin-4-yl}morpholin | 1.49 | 410 | - | - | - | B |
| 53 | 4-[3-(4-Fluorphenyl)-1-(propan-2-yl)-1H-pyrazol-4-yl]-N-methylpyridin-2-carboxamid | 2.74 | 339 | - | - | - | A |
| 54 | 4-[3-(4-Fluorphenyl)-1-(tetrahydro-2H-pyran-2-yl)-1H-pyrazol-4-yl]chinolin | 2.46 | 374 | - | - | - | B |
| 55 | 4-[5-(4-Fluor-3-methylphenyl)-1-(tetrahydro-2H-pyran-2-yl)-1H-pyrazol-4-yl]pyridin | 1.47 | 338 | - | - | - | B |
| 56 | 4-[5-(2,4-Difluorphenyl)-1-(tetrahydro-2H-pyran-2-yl)-1H-pyrazol-4-yl]pyridin | 1.35 | 342 | - | - | - | B |
| 57 | 4-[5-(4-tert-Butylphenyl)-1-(tetrahydro-2H-pyran-2-yl)-1H-pyrazol-4-yl]pyridin | 2.02 | 362 | - | - | - | B |
| 58 | 4-[1-tert-Butyl-3-(4-fluorphenyl)-1H-pyrazol-4-yl]pyridin | 2.62 | 296 | - | - | - | C |
| 59 | 4-[5-(4-Phenoxyphenyl)-1-(tetrahydro-2H-pyran-2-yl)-1H-pyrazol-4-yl]pyridin | 1.88 | 398 | - | - | - | B |
| 60 | 3-[4-(Pyridin-4-yl)-1-(tetrahydro-2H-pyran-2-yl)-1H-pyrazol-5-yl]benzonitril | 1.23 | 331 | - | - | - | B |
| 61 | 2-Fluor-5-[4-(pyridin-4-yl)-1-(tetrahydro-2H-pyran-2-yl)-1H-pyrazol-5-yl]benzonitril | 1.45 | 349 | - | - | - | B |
| 62 | 4-[3-(4-Methoxyphenyl)-1-(tetrahydro-2H-pyran-2-yl)-1H-pyrazol-4-yl]-6-(1H-pyrazol-1-yl)pyrimidin | 3.32 | 403 | - | - | - | B |
| 63 | N-Ethyl-6-[3-(4-fluorphenyl)-1-(tetrahydro-2H-pyran-2-yl)-1H-pyrazol-4-yl]pyrimidin-4-amin | 1.42 | 368 | - | - | - | B |
| 64 | 4-[5-(4-Fluor-2-methoxyphenyl)-1-(tetrahydro-2H-pyran-2-yl)-1H-pyrazol-4-yl]pyridin | 1.26 | 354 | - | - | - | B |
| 65 | 4-[3-(4-Methoxyphenyl)-1-(tetrahydro-2H-pyran-2-yl)-1H-pyrazol-4-yl]-7H-pyrrolo[2,3-d]pyrimidin | 1.54 | 376 | - | - | - | B |
| 66 | 4-[3-(4-Fluorphenyl)-1-(tetrahydro-2H-pyran-2-yl)-1H-pyrazol-4-yl]-7H-pyrrolo[2,3-d]pyrimidin | 1.69 | 364 | - | - | - | B |
| 67 | 4-[5-(3-Phenoxyphenyl)-1-(tetrahydro-2H-pyran-2-yl)-1H-pyrazol-4-yl]pyridin | 1.88 | 398 | - | - | - | B |
| 68 | 4-{5-[4-(Methylsulfonyl)phenyl]-1-(tetrahydro-2H-pyran-2-yl)-1H-pyrazol-4-yl}pyridin | 0.96 | 384 | - | - | - | B |
| 69 | 4-[3-(4-Methoxyphenyl)-1-(tetrahydro-2H-pyran-2-yl)-1H-pyrazol-4-yl]pyridin-2-amin | 1.31 | 351 | - | - | - | B |
| 70 | 4-[5-(4-Chlorphenyl)-1-(tetrahydro-2H-pyran-2-yl)-1H-pyrazol-4-yl]pyridin | 1.52 | 340 | - | - | - | B |
| 71 | 4-{1-(Tetrahydro-2H-pyran-2-yl)-5-[4-(trifluormethoxy)phenyl]-1H-pyrazol-4-yl}pyridin | 1.78 | 390 | - | - | - | B |
| 72 | N-{4-[5-(4-Fluorphenyl)-1-(propan-2-yl)-1H-pyrazol-4-yl]pyridin-2-yl}-2,2-dimethylpropanamid | 2.99 | 381 | - | - | - | B |
| 73 | 4-[3-(4-Methoxyphenyl)-1-(tetrahydro-2H-pyran-2-yl)-1H-pyrazol-4-yl]-2-methylpyridin | 1.33 | 350 | - | - | - | B |
| 74 | 4-[3-(4-Fluorphenyl)-1-(tetrahydro-2H-pyran-2-yl)-1H-pyrazol-4-yl]-2-(methylsulfanyl)pyrimidin | 3.58 | 371 | - | - | - | B |
| 75 | 4-[3-(4-Fluorphenyl)-1-(tetrahydro-2H-pyran-2-yl)-1H-pyrazol-4-yl]-6-(1H-pyrazol-1-yl)pyrimidin | 3.58 | 391 | - | - | - | B |
| 76 | 4-[5-(2,3-Dichlorphenyl)-1-(tetrahydro-2H-pyran-2-yl)-1H-pyrazol-4-yl]pyridin | 1.66 | 374 | 1.60 | 374 | 40:60 | B |
| 77 | 4-[3-(4-Methoxyphenyl)-1-(tetrahydro-2H-pyran-2-yl)-1H-pyrazol-4-yl]-2-methylpyrimidin | 2.27 | 351 | - | - | - | B |
| 78 | N-Ethyl-6-[3-(4-methoxyphenyl)-1-(tetrahydro-2H-pyran-2-yl)-1H-pyrazol-4-yl]pyrimidin-4-amin | 1.28 | 380 | - | - | - | B |
| 79 | 2-[3-(4-Fluorphenyl)-4-(pyridin-4-yl)-1H-pyrazol-1-yl]-5-(trifluormethyl)pyridin | 2.64 | 385 | - | - | - | B |
| 80 | 4-[3-(4-Methoxyphenyl)-1-(tetrahydro-2H-pyran-2-yl)-1H-pyrazol-4-yl]-1H-pyrrolo[2,3-b]pyridin | 1.87 | 375 | - | - | - | B |
| 81 | 1-{4-[3-(4-Fluorphenyl)-1-(tetrahydro-2H-pyran-2-yl)-1H-pyrazol-4-yl]-1H-pyrrolo[2,3-b]pyridin-1-yl} ethanon | 3.85 | 405 | - | - | - | B |
| 82 | 2,2-Dimethyl-N-(4-{1-(tetrahydro-2H-pyran-2-yl)-5-[3-(trifluormethyl)phenyl]-1H-pyrazol-4-yl}pyridin-2-yl)propanamid | 3.84 | 473 | - | - | - | B |
| 83 | 4-[3-(3-Chlorphenyl)-1-(tetrahydro-2H-pyran-2-yl)-1H-pyrazol-4-yl]-N-methylpyridin-2-carboxamid | 3.15 | 397 | - | - | - | B |
| 84 | N-{4-[3-(4-Fluorphenyl)-1-(propan-2-yl)-1H-pyrazol-4-yl]pyridin-2-yl}-2-methylpropanamid | 2.50 | 367 | - | - | - | A |
| 85 | N-{4-[3-(4-Fluorphenyl)-1-(propan-2-yl)-1H-pyrazol-4-yl]pyridin-2-yl}propanamid | 2.15 | 353 | - | - | - | A |
| 86 | N-{4-[3-(4-Fluorphenyl)-1-(propan-2-yl)-1H-pyrazol-4-yl]pyridin-2-yl}cyclopropancarboxamid | 2.07 | 365 | - | - | - | B |
| 87 | N-{4-[3-(4-Fluorphenyl)-1-(2-methylpropyl)-1H-pyrazol-4-yl]pyridin-2-yl}-2-methylpropanamid | 2.89 | 381 | - | - | - | B |
| 88 | 4-[3-(4-Fluorphenyl)-1-(2-methoxyethyl)-1H-pyrazol-4-yl]pyridin | 1.11 | 298 | 1.05 | 298 | 70:30 | A |
| 89 | 4-[3-(4-Fluorphenyl)-1-(propan-2-yl)-1H-pyrazol-4-yl]pyridin | 1.16 | 282 | - | - | - | B |
| 90 | 4-[3-(4-Fluorphenyl)-1-(tetrahydro-2H-pyran-2-yl)-1H-pyrazol-4-yl]-N-methylpyridin-2-carboxamid | 2.72 | 381 | - | - | - | B |
| 91 | 4-[1-(Butan-2-yl)-3-(4-fluorphenyl)-1H-pyrazol-4-yl]pyridin | 1.5 | 296 | - | - | - | A |
| 92 | 4-[1-Cyclopentyl-3-(4-fluorphenyl)-1H-pyrazol-4-yl]pyridin | 1.65 | 308 | 1.65 | 308 | 85:15 | A |
| 92 | 4-[1-Cyclopentyl-3-(4-fluorphenyl)-1H-pyrazol-4-yl]pyridin | 1.65 | 308 | 1.65 | 308 | 80:20 | A |
| 93 | 4-[3-(4-Fluorphenyl)-1-(tetrahydro-2H-pyran-2-yl)-1 H-pyrazol-4-yl]pyridin-2-carboxamid | 2.46 | 367 | - | - | - | B |
| 94 | 4-[3-(4-Fluorphenyl)-1-(tetrahydro-2H-pyran-2-yl)-1H-pyrazol-4-yl]-N-phenylpyridin-2-carboxamid | 4.34 | 443 | - | - | - | B |
| 95 | 4-[3-(3-Chlorphenyl)-1-(tetrahydro-2H-pyran-2-yl)-1H-pyrazol-4-yl]pyridin-2-carboxamid | 2.93 | 383 | - | - | - | B |
| 96 | 4-[3-(3-Chlorphenyl)-1-(tetrahydro-2H-pyran-2-yl)-1H-pyrazol-4-yl]-N-phenylpyridin-2-carboxamid | 4.89 | 459 | - | - | - | B |
| 97 | 4-[5-(4-Fluorphenyl)-1-(propan-2-yl)-1H-pyrazol-4-yl]pyridin-2-amin | 1.25 | 297 | - | - | - | B |
| 98 | 4-{5-[2-(Benzyloxy)phenyl]-1-(tetrahydro-2H-pyran-2-yl)-1H-pyrazol-4-yl}pyridin | 1.64 | 412 | - | - | - | B |
| 99 | 8-[4-(Pyridin-4-yl)-1-(tetrahydro-2H-pyran-2-yl)-1H-pyrazol-5-yl]chinolin | 1.09 | 357 | - | - | - | B |
| 100 | 3-{[3-(4-Fluorphenyl)-4-(pyridin-4-yl)-1H-pyrazol-1-yl]methyl}pyridin | 0.78 | 331 | - | - | - | B |
| 101 | 4-{1-[1-(3-Chlorphenyl)ethyl]-3-(4-fluorphenyl)-1H-pyrazol-4-yl}pyridin | 2.17 | 378 | 2.1 | 378 | 80:20 | A |
| 102 | 4-{1-[1-(2-Chlorphenyl)ethyl]-3-(4-fluorphenyl)-1H-pyrazol-4-yl}pyridin | 2.16 | 378 | 2.13 | 378 | 91:9 | A |
| 103 | 4-[3-(4-Fluorphenyl)-1-(2,2,2-trifluorethyl)-1H-pyrazol-4-yl]pyridin | 1.41 | 322 | 1.33 | 322 | 91:9 | A |
| 104 | 4-[1-(2,3-Difluorbenzyl)-3-(4-fluorphenyl)-1H-pyrazol-4-yl]pyridin | 1.80 | 366 | 1.7 | 366 | 75:25 | A |
| 105 | tert-Butyl-[3-(4-fluorphenyl)-4-(pyridin-4-yl)-1H-pyrazol-1-yl]acetat | 1.67 | 354 | 1.59 | 354 | 78:22 | A |
| 106 | Methyl-2-[3-(4-fluorphenyl)-4-(pyridin-4-yl)-1H-pyrazol-1-yl]-3-methylbutanoat | 1.67 | 354 | 1.6 | 354 | 93:7 | A |
| 107 | Methyl-2-[3-(4-fluorphenyl)-4-(pyridin-4-yl)-1H-pyrazol-1 -yl]propanoat | 1.20 | 326 | - | - | - | A |
| 108 | 4-[1-(2-Chlor-6-fluorbenzyl)-3-(4-fluorphenyl)-1H-pyrazol-4-yl]pyridin | 1.90 | 382 | 1.82 | 382 | 87:13 | A |
| 109 | 4- {1-[4-(Difluormethoxy)benzyl]-3-(4-fluorphenyl)-1H-pyrazol-4-yl}pyridin | 1.88 | 396 | 1.79 | 396 | 74:26 | A |
| 110 | 4-{1-[(2-Chlor-1,3-thiazol-5-yl)methyl]-3-(4-fluorphenyl)-1H-pyrazol-4-yl}pyridin | 1.54 | 371 | - | - | - | A |
| 111 | 4-{3-(4-Fluorphenyl)-1-[5-(trifluormethyl)-1,3,4-thiadiazol-2-yl]-1H-pyrazol-4-yl}pyridin | 2.90 | 392 | - | - | - | A |
| 112 | 4-[3-(4-Fluorphenyl)-4-(pyridin-4-yl)-1H-pyrazol-1-yl]-6-(trifluormethyl)pyrimidin | 2.73 | 386 | - | - | - | A |
| 113 | 1-Cyclohexyl-2-[3-(4-fluorphenyl)-4-(pyridin-4-yl)-1H-pyrazol-1-yl]ethanon | 1.86 | 364 | 1.76 | 364 | 80:20 | A |
| 114 | 1-Cyclopentyl-2-[3-(4-fluorphenyl)-4-(pyridin-4-yl)-1H-pyrazol-1-yl]ethanon | 1.63 | 350 | 1.54 | 350 | 75:25 | A |
| 115 | 2-{[3-(4-Fluorphenyl)-4-(pyridin-4-yl)-1H-pyrazol-1-yl]methyl}pyridin | 0.99 | 331 | - | - | - | A |
| 115 | 2-{[3-(4-Fluorphenyl)-4-(pyridin-4-yl)-1H-pyrazol-1-yl]methyl}pyridin | 1.08 | 331 | 0.97 | 331 | 75:25 | A |
| 116 | 4-{1-[(2,2-Dichlorcyclopropyl)methyl]-3-(4-fluorphenyl)-1H-pyrazol-4-yl}pyridin | 1.76 | 362 | 1.68 | 362 | 75:25 | A |
| 117 | 4-{3-(4-Fluorphenyl)-1-[2-(methylsulfanyl)ethyl]-1H-pyrazol-4-yl}pyridin | 1.29 | 314 | 1.25 | 314 | 80:20 | A |
| 118 | 4-{1-[2-(3-Chlorphenyl)ethyl]-3-(4-fluorphenyl)-1H-pyrazol-4-yl}pyridin | 2.04 | 378 | 1.9 | 378 | 70:30 | A |
| 119 | 4-{1-[2-(2-Chlorphenyl)ethyl]-3-(4-fluorphenyl)-1H-pyrazol-4-yl}pyridin | 2.04 | 378 | 1.9 | 378 | 72:28 | A |
| 120 | 4-{1-[1-(4-Chlorphenyl)ethyl]-3-(4-fluorphenyl)-1H-pyrazol-4-yl}pyridin | 2.18 | 378 | 2.12 | 378 | 80:20 | A |
| 121 | 4-[3-(4-Fluorphenyl)-1-(pyridin-4-ylmethyl)-1H-pyrazol-4-yl]pyridin | 0.25 | 331 | - | - | - | B |
| 122 | 4-{3-(4-Fluorphenyl)-1-[2-(trifluormethoxy)ethyl]-1H-pyrazol-4-yl}pyridin | 1.66 | 352 | 1.59 | 352 | 88:12 | A |
| 122 | 4-{3-(4-Fluorphenyl)-1-[2-(trifluormethoxy)ethyl]-1H-pyrazol-4-yl}pyridin | 1.66 | 352 | 1.59 | 352 | 66:33 | A |
| 123 | 4-[3-(4-Fluorphenyl)-1-(tetrahydrofuran-2-ylmethyl)-1H-pyrazol-4-yl]pyridin | 1.19 | 324 | 1.16 | 324 | 89:11 | A |
| 124 | 3-[3-(4-Fluorphenyl)-4-(pyridin-4-yl)-1H-pyrazol-1-yl]propan-1,2-diol | 0.17 | 314 | - | - | - | A |
| 125 | 4-[1-(Biphenyl-3-ylmethyl)-3-(4-fluorphenyl)-1H-pyrazol-4-yl]pyridin | 2.38 | 406 | 2.25 | 406 | 91:9 | A |
| 126 | 4-[-(Cyclopropylmethyl)-3-(4-fluorphenyl)-1H-pyrazol-4-yl]pyridin | 1.33 | 294 | - | - | - | A |
| 127 | 4-[1-Benzyl-3-(4-fluorphenyl)-1H-pyrazol-4-yl]pyridin | 1.63 | 330 | 1.57 | 330 | 77:23 | A |
| 128 | 4-[1-(2-Fluorbenzyl)-3-(4-fluorphenyl)-1H-pyrazol-4-yl]pyridin | 1.84 | 348 | 1.76 | 348 | 80:20 | A |
| 129 | 4-[1-(3-Fluorbenzyl)-3-(4-fluorphenyl)-1H-pyrazol-4-yl]pyridin | 1.71 | 348 | 1.61 | 348 | 75:25 | A |
| 130 | 4-[1-(4-Fluorbenzyl)-3-(4-fluorphenyl)-1H-pyrazol-4-yl]pyridin | 1.69 | 348 | 1.61 | 348 | 75:25 | A |
| 131 | 4-[-(Biphenyl-4-ylmethyl)-3-(4-fluorphenyl)-1H-pyrazol-4-yl]pyridin | 2.39 | 406 | 2.3 | 406 | 95:5 | A |
| 132 | Ethyl-3-[3-(4-fluorphenyl)-4-(pyridin-4-yl)-1H-pyrazol-1-yl]propanoat | 1.29 | 340 | - | - | - | A |
| 133 | 4-[3-(4-Fluorphenyl)-1-{2-[2-(2-methoxyethoxy)ethoxy]ethyl}-1H-pyrazol-4-yl]pyridin | 1.11 | 386 | 1.06 | 386 | 70:30 | A |
| 134 | 4-{3-(4-Fluorphenyl)-1-[2-(2-methoxyethoxy)ethyl]-1H-pyrazol-4-yl}pyridin | 1.04 | 342 | - | - | - | A |
| 134 | 4-{3-(4-Fluorphenyl)-1-[2-(2-methoxyethoxy)ethyl]-1H-pyrazol-4-yl}pyridin | 1.11 | 342 | - | - | - | A |
| 135 | 4-[1-(Biphenyl-2-ylmethyl)-3-(4-fluorphenyl)-1H-pyrazol-4-yl]pyridin | 2.34 | 406 | - | - | - | B |
| 136 | 2-{[3-(4-Fluorphenyl)-4-(pyridin-4-yl)-1H-pyrazol-1-yl]methyl}benzonitril | 1.49 | 355 | - | - | - | A |
| 137 | 4-[3-(4-Fluorphenyl)-1-(naphthalen-1-ylmethyl)-1H-pyrazol-4-yl]pyridin | 2.12 | 380 | 1.99 | 380 | 84:16 | A |
| 138 | 4-[3-(4-Fluorphenyl)-1-(3-phenoxybenzyl)-1H-pyrazol-4-yl]pyridin | 2.36 | 422 | - | - | - | A |
| 139 | 4-[1-(4-Fluor-3-phenoxybenzyl)-3-(4-fluorphenyl)-1H-pyrazol-4-yl]pyridin | 2.40 | 440 | - | - | - | A |
| 140 | 3-{[3-(4-Fluorphenyl)-4-(pyridin-4-yl)-1H-pyrazol-1-yl]methyl}benzonitril | 1.50 | 355 | - | - | - | A |
| 140 | 3-{[3-(4-Fluorphenyl)-4-(pyridin-4-yl)-1H-pyrazol-1-yl]methyl}benzonitril | 1.51 | 355 | 1.38 | 355 | 60:40 | A |
| 141 | 4-{[3-(4-Fluorphenyl)-4-(pyridin-4-yl)-1H-pyrazol-1-yl]methyl}benzonitril | 1.48 | 355 | - | - | - | A |
| 141 | 4-{[3-(4-Fluorphenyl)-4-(pyridin-4-yl)-1H-pyrazol-1-yl]methyl}benzonitril | 1.50 | 355 | 1.38 | 355 | 70:30 | A |
| 142 | 3-[3-(4-Fluorphenyl)-4-(pyridin-4-yl)-1H-pyrazol-1-yl]dihydrofuran-2(3H)-on | 0.95 | 324 | - | - | - | A |
| 143 | 4-[3-(4-Fluorphenyl)-1-(1H-imidazol-2-ylmethyl)-1H-pyrazol-4-yl]pyridin | 0.41 | 320 | - | - | - | B |
| 144 | 1-(1,5-Dimethyl-1H-pyrazol-3-yl)-2-[3-(4-fluorphenyl)-4-(pyridin-4-yl)-1H-pyrazol-1-yl]ethanon | 1.31 | 376 | 1.18 | 376 | 62:38 | A |
| 144 | 1-(1,5-Dimethyl-1H-pyrazol-3-yl)-2-[3-(4-fluorphenyl)-4-(pyridin-4-yl)-1H-pyrazol-1-yl]ethanon | 1.30 | 376 | - | - | - | A |
| 145 | 2-[3-(4-Fluorphenyl)-4-(pyridin-4-yl)-1H-pyrazol-1-yl]ethylacetat | 1.05 | 326 | 0.97 | 326 | 91:9 | A |
| 146 | 3-[3-(4-Fluorphenyl)-4-(pyridin-4-yl)-1H-pyrazol-1-yl]propanonitril | 0.81 | 293 | - | - | - | A |
| 147 | 4-[3-(4-Fluorphenyl)-1-(2-methylpropyl)-1H-pyrazol-4-yl]-1,3-dihydro-2H-pyrrolo[2,3-b]pyridin-2-on | 2.6 | 351 | - | - | - | C |
| 148 | 4-[3-(4-Fluorphenyl)-1-(2-methylpropyl)-1H-pyrazol-4-yl]-1,3,3-trimethyl-1,3-dihydro-2H-pyrrolo[2,3-b]pyridin-2-on | 3.68 | 393 | - | - | - | C |
| 149 | 4-[5-(4-Fluorphenyl)-1-(2-methylpropyl)-1H-pyrazol-4-yl]-N-(prop-2-en-1-yl)pyrimidin-2-amin | 2.37 | 352 | - | - | - | C |
| 150 | 4-[3-(4-Fluorphenyl)-1-(2-methylpropyl)-1H-pyrazol-4-yl]-N-(prop-2-en-1-yl)pyrimidin-2-amin | 2.46 | 352 | - | - | - | C |
| 153 | N-{4-[3-(4-Fluorphenyl)-1-(2-methoxyethyl)-1H-pyrazol-4-yl]pyridin-2-yl}propanamid | 1.61 | 369 | - | - | - | B |
| 154 | 2-Fluor-5-[1-(2-methylpropyl)-4-(pyridin-4-yl)-1H-pyrazol-3-yl]benzonitril | 1.65 | 321 | - | - | - | A |
| 155 | N-{4-[3-(4-Fluorphenyl)-1-(2-methylpropyl)-1H-pyrazol-4-yl]pyridin-2-yl}propanamid | 2.51 | 367 | - | - | - | A |
| 156 | N-{4-[3-(4-Fluorphenyl)-1-(propan-2-yl)-1H-pyrazol-4-yl]pyridin-2-yl}cyclobutancarboxamid | 2.57 | 379 | - | - | - | A |
| 157 | N-{4-[1-(Butan-2-yl)-3-(4-fluorphenyl)-1H-pyrazol-4-yl]pyridin-2-yl}-2-methylpropanamid | 2.82 | 381 | - | - | - | B |
| 158 | 2-Cyclopropyl-N-{4-[3-(4-fluorphenyl)-1-(2-methylpropyl)-1 H-pyrazol-4-yl]pyridin-2-yl}acetamid | 3.03 | 393 | - | - | - | B |
| 159 | N-{4-[1-(Butan-2-yl)-3-(4-fluorphenyl)-1H-pyrazol-4-yl]pyridin-2-yl}-2-cyclopropylacetamid | 3 | 393 | - | - | - | B |
| 160 | N-{4-[3-(4-Fluorphenyl)-1-(propan-2-yl)-1H-pyrazol-4-yl]pyridin-2-yl}-3-methylbutanamid | 2.82 | 381 | - | - | - | A |
| 161 | N-{4-[3-(4-Fluorphenyl)-1-(propan-2-yl)-1H-pyrazol-4-yl]pyridin-2-yl}acetamid | 1.85 | 339 | - | - | - | A |
| 162 | N-{4-[1-(Butan-2-yl)-3-(4-fluorphenyl)-1H-pyrazol-4-yl]pyridin-2-yl}-2-methoxyacetamid | 2.95 | 383 | - | - | - | B |
| 163 | N-{4-[3-(4-Fluorphenyl)-1-(2-methoxyethyl)-1H-pyrazol-4-yl]pyridin-2-yl}-2-methoxyacetamid | 2.05 | 385 | - | - | - | A |
| 164 | N-{4-[1-(Butan-2-yl)-3-(4-fluorphenyl)-1H-pyrazol-4-yl]pyridin-2-yl}propanamid | 2.46 | 367 | - | - | - | B |
| 165 | N-{4-[3-(4-Fluorphenyl)-1-(2-methylpropyl)-1H-pyrazol-4-yl]pyridin-2-yl}cyclopropancarboxamid | 2.6 | 379 | - | - | - | B |
| 166 | N-{4-[3-(4-Fluorphenyl)-1-(2-methylpropyl)-1H-pyrazol-4-yl]pyridin-2-yl}-2-methoxyacetamid | 2.96 | 383 | - | - | - | B |
| 167 | N-{4-[1-(Butan-2-yl)-3-(4-fluorphenyl)-1H-pyrazol-4-yl]pyridin-2-yl}cyclopropancarboxamid | 2.56 | 379 | - | - | - | B |
| 168 | 4-[1-Cyclohexyl-3-(4-fluorphenyl)-1H-pyrazol-4-yl]pyridin | 1.91 | 322 | 1.82 | 322 | 69:31 | A |
| 169 | 4-[1-(2,2-Difluorethyl)-3-(4-fluorphenyl)-1H-pyrazol-4-yl]pyridin | 1 | 304 | - | - | - | A |
| 170 | Ethyl-[3-(4-fluorphenyl)-4-(pyridin-4-yl)-1H-pyrazol-1-yl]acetat | 1.31 | 326 | - | - | - | B |
| 171 | 4-[3-(4-Fluorphenyl)-5-methyl-1-(prop-2-in-1-yl)-1H-pyrazol-4-yl]pyridin | 2.51 | 292 | 2.33 | 292 | 62:19 | C |
| 172 | 4-[3-(4-Fluorphenyl)-1H-pyrazol-4-yl]-N-(propan-2-yl)pyrimidin-2-amin | 1.37 | 298 | - | - | - | A |
| 173 | 4-[3-(4-Fluorphenyl)-1H-pyrazol-4-yl]-N-(prop-2-in-1-yl)pyrimidin-2-amin | 1.44 | 294 | - | - | - | A |
| 174 | 4-[3-(4-Fluorphenyl)-1-(2-methylpropyl)-1H-pyrazol-4-yl]-N-(propan-2-yl)pyrimidin-2-amin | 2.39 | 354 | - | - | - | C |
| 175 | 3-(4-Fluorphenyl)-4-(pyridin-4-yl)-1H-pyrazol-5-carbonitril | 1.08 | 265 | - | - | - | A |
| 176 | 4-{1-[1-(2-Chlor-1,3-thiazol-5-yl)ethyl]-3-(4-fluorphenyl)-1H-pyrazol-4-yl}pyridin | 1.82 | 385 | - | - | - | B |
| 177 | 4-[1-(1,3-Difluorpropan-2-yl)-3-(4-fluorphenyl)-1H-pyrazol-4-yl]pyridin | 1.16 | 318 | - | - | - | B |
| 178 | 1-[3-(4-Fluorphenyl)-4-(pyridin-4-yl)-1H-pyrazol-1-yl]-3-methylbutan-2-on | 1.37 | 324 | - | - | - | A |
| 179 | 4-[1-(2-Ethoxyethyl)-3-(4-fluorphenyl)-1H-pyrazol-4-yl]pyridin | 1.25 | 312 | - | - | - | B |
| 180 | [3-(4-Fluorphenyl)-4-(pyridin-4-yl)-1H-pyrazol-1-yl] acetonitril | 0.89 | 279 | - | - | - | A |
| 181 | Propan-2-yl-{4-[3-(4-fluorphenyl)-1-(propan-2-yl)-1H-pyrazol-4-yl]pyridin-2-yl} carbamat | 2.99 | 383 | - | - | - | A |
| 182 | N-Benzyl-4-[3-(4-fluorphenyl)-1-(2-methylpropyl)-1H-pyrazol-4-yl]pyrimidin-2-amin | 3.15 | 402 | - | - | - | C |
| 183 | 4-[3-(4-Fluorphenyl)-1-(2-methylpropyl)-1H-pyrazol-4-yl]-N-(prop-2-in-1-yl)pyrimidin-2-amin | 2.76 | 350 | - | - | - | C |
| 184 | 4-[3-(4-Fluorphenyl)-1-(2-methylpropyl)-1H-pyrazol-4-yl]pyrimidin-2-amin | 1.81 | 312 | - | - | - | C |
| 185 | N-{4-[3-(4-Fluorphenyl)-1-(2-methylpropyl)-1H-pyrazol-4-yl]pyrimidin-2-yl}-2-methyl-N-(propan-2-yl)propanamid | 4.25 | 424 | - | - | - | C |
| 186 | N-{4-[3-(4-Fluorphenyl)-1-(2-methylpropyl)-1H-pyrazol-4-yl]pyrimidin-2-yl}-2,2,4-trimethyl-3-oxo-N-(propan-2-yl)pentanamid | 3.02 | 494 | - | - | - | A |
| 187 | 1-{4-[3-(4-Fluorphenyl)-1-(2-methylpropyl)-1H-pyrazol-4-yl]pyridin-2-yl}pyrrolidin-2-on | 3.19 | 379 | - | - | - | C |
| 188 | N-{4-[3-(4-Fluorphenyl)-1-(2-methylpropyl)-1H-pyrazol-4-yl]pyrimidin-2-yl}-2-methyl-N-(2-methylpropanoyl)propanamid | 4.74 | 452 | - | - | - | C |
| 189 | N-{4-[3-(4-Fluorphenyl)-1-(2-methylpropyl)-1H-pyrazol-4-yl]pyrimidin-2-yl}-2-methylpropanamid | 2.92 | 382 | - | - | - | C |
| 190 | N-{4-[1-Ethyl-3-(4-fluorphenyl)-1H-pyrazol-4-yl]pyridin-2-yl}-2-phenylacetamid | 2.72 | 401 | - | - | - | B |
| 191 | N-{4-[3-(4-Fluorphenyl)-1-(2-methylpropyl)-1H-pyrazol-4-yl]pyridin-2-yl}-2-phenylacetamid | 3.54 | 429 | - | - | - | A |
| 192 | (2S)-N-{4-[3-(4-Fluorphenyl)-1-(propan-2-yl)-1H-pyrazol-4-yl]pyridin-2-yl}-2-hydroxypropanamid | 2.14 | 369 | - | - | - | B |
| 193 | tert-Butyl-4-{3-(4-fluorphenyl)-4-[2-(propanoylamino)pyridin-4-yl]-1H-pyrazol-1-yl}piperidin-1-carboxylat | 3.03 | 494 | - | - | - | B |
| 194 | N-{4-[3-(4-Cyan-2,5-difluorphenyl)-5-(difluormethoxy)-1-methyl-1H-pyrazol-4-yl]pyridin-2-yl}propanamid | | 0 | - | - | - | B |
| 195 | N-{4-[3-(4-Chlor-2-fluorphenyl)-1-methyl-5-(methylsulfanyl)-1H-pyrazol-4-yl]pyridin-2-yl}propanamid | 2.70 | 405 | - | - | - | A |
| 196 | N-{4-[5-(Difluormethoxy)-3-(4-fluor-2-hydroxyphenyl)-1-methyl-1H-pyrazol-4-yl]pyridin-2-yl}propanamid | 2.12 | 407 | - | - | - | A |
| 197 | N-{4-[3-(4-Chlor-2-fluorphenyl)-1-methyl-5-(trifluormethyl)-1H-pyrazol-4-yl]pyridin-2-yl}propanamid | 3.32 | 427 | - | - | - | A |
| 198 | N-{4-[1-Ethyl-3-(4-fluorphenyl)-1H-pyrazol-4-yl]pyridin-2-yl}propanamid | 1.82 | 339 | - | - | - | B |
| 199 | N-{4-[3-(4-Cyan-2,5-difluorphenyl)-5-(difluormethoxy)-1-methyl-1H-pyrazol-4-yl]pyridin-2-yl}-2-methoxyacetamid | 2.59 | 450 | - | - | - | B |
| 200 | N-{4-[3-(4-Chlor-2-fluorphenyl)-1-methyl-5-(methylsulfanyl)-1H-pyrazol-4-yl]pyridin-2-yl}-2-methoxyacetamid | 3.00 | 421 | - | - | - | B |
| 201 | N-{4-[5-(Difluormethoxy)-3-(4-fluor-2-hydroxyphenyl)-1-methyl-1H-pyrazol-4-yl]pyridin-2-yl}-2-methoxyacetamid | 2.38 | 423 | - | - | - | B |
| 202 | N-{4-[3-(4-Chlor-2-fluorphenyl)-1-methyl-5-(trifluormethyl)-1H-pyrazol-4-yl]pyridin-2-yl} -2-methoxyacetamid | 3.45 | 443 | - | - | - | B |
| 203 | N-{4-[1-Ethyl-3-(4-fluorphenyl)-1H-pyrazol-4-yl]pyridin-2-yl}-2-methoxyacetamid | 2.20 | 355 | - | - | - | A |
| 204 | tert-Butyl-4-[3-(4-fluorphenyl)-4-{2-[(methoxyacetyl)amino]pyridin-4-yl}-1H-pyrazol-1-yl]piperidin-1-carboxylat | 3.51 | 510 | - | - | - | A |
| 205 | N-{4-[3-(4-Fluorphenyl)-1-(propan-2-yl)-1H-pyrazol-4-yl]pyridin-2-yl}benzamid | 3.20 | 401 | - | - | - | A |
| 206 | N-{4-[3-(4-Fluorphenyl)-1-(propan-2-yl)-1H-pyrazol-4-yl]pyridin-2-yl}thiophen-2-carboxamid | 3.14 | 407 | - | - | - | A |
| 207 | N-{4-[3-(4-Fluorphenyl)-1-(propan-2-yl)-1H-pyrazol-4-yl]pyridin-2-yl}thiophen-3-carboxamid | 2.96 | 407 | - | - | - | A |
| 208 | N-{4-[3-(4-Fluorphenyl)-1-(propan-2-yl)-1H-pyrazol-4-yl]pyridin-2-yl}-2-(2-methoxyethoxy)acetamid | 2.62 | 413 | - | - | - | A |
| 209 | N-{4-[3-(4-Fluorphenyl)-1-(propan-2-yl)-1H-pyrazol-4-yl]pyridin-2-yl}-2-hydroxy-2-methylpropanamid | 2.42 | 383 | - | - | - | A |
| 210 | N-{4-[3-(4-Fluorphenyl)-1-(propan-2-yl)-1H-pyrazol-4-yl]pyridin-2-yl}-2,3-dihydroxypropanamid | 1.76 | 385 | - | - | - | A |
| 211 | 1-Ethyl-3-{4-[3-(4-fluorphenyl)-1-(propan-2-yl)-1H-pyrazol-4-yl]pyridin-2-yl}harnstoff | 2.02 | 368 | - | - | - | A |
| 212 | N-{4-[3-(4-Fluorphenyl)-1-(2-methoxyethyl)-1H-pyrazol-4-yl]pyridin-2-yl}-2-phenylacetamid | 2.6 | 431 | - | - | - | A |
| 213 | N- {4-[1-(2-Fluorbenzyl)-3-(4-fluorphenyl)-1H-pyrazol-4-yl]pyridin-2-yl}propanamid | 2.69 | 419 | - | - | - | B |
| 214 | N-{4-[1-(Cyclopropylmethyl)-3-(4-fluorphenyl)-1H-pyrazol-4-yl]pyridin-2-yl}propanamid | 2.20 | 366 | - | - | - | B |
| 215 | N-{4-[1-(2,2-Difluorethyl)-3-(4-fluorphenyl)-1H-pyrazol-4-yl]pyridin-2-yl}propanamid | 1.99 | 375 | - | - | - | B |
| 216 | N-{4-[1-(2,2-Difluorethyl)-3-(4-fluorphenyl)-1H-pyrazol-4-yl]pyridin-2-yl}-2-methoxyacetamid | 2.27 | 391 | - | - | - | B |
| 217 | N-{4-[1-(2,2-Difluorethyl)-3-(4-fluorphenyl)-1H-pyrazol-4-yl]pyridin-2-yl}cyclopropancarboxamid | 2.07 | 388 | - | - | - | B |
| 218 | N-{4-[1-(2,2-Difluorethyl)-3-(4-fluorphenyl)-1H-pyrazol-4-yl]pyridin-2-yl}-2-methylpropanamid | 2.29 | 389 | - | - | - | B |
| 219 | N-{4-[1-(2-Chlorethyl)-3-(4-fluorphenyl)-1H-pyrazol-4-yl]pyridin-2-yl}propanamid | 2.01 | 373 | - | - | - | B |
| 220 | N-{4-[1-(2-Chlorethyl)-3-(4-fluorphenyl)-1H-pyrazol-4-yl]pyridin-2-yl}-2-methoxyacetamid | 2.36 | 389 | - | - | - | B |
| 221 | N-{4-[1-(2-Chlorethyl)-3-(4-fluorphenyl)-1H-pyrazol-4-yl]pyridin-2-yl}cyclopropancarboxamid | 2.10 | 385 | - | - | - | B |
| 222 | N-{4-[1-(2-Chlorethyl)-3-(4-fluorphenyl)-1H-pyrazol-4-yl]pyridin-2-yl}-2-methylpropanamid | 2.33 | 387 | - | - | - | B |
| 223 | N-{4-[1-(Cyclopropylmethyl)-3-(4-fluorphenyl)-1H-pyrazol-4-yl]pyridin-2-yl}-2-methoxyacetamid | 2.61 | 382 | - | - | - | B |
| 224 | N-{4-[1-(Cyclopropylmethyl)-3-(4-fluorphenyl)-1H-pyrazol-4-yl]pyridin-2-yl}cyclopropancarboxamid | 2.29 | 377 | - | - | - | B |
| 225 | N-{4-[1-(Cyclopropylmethyl)-3-(4-fluorphenyl)-1H-pyrazol-4-yl]pyridin-2-yl}-2-methylpropanamid | 2.54 | 380 | - | - | - | B |
| 226 | N-{4-[3-(4-Fluorphenyl)-1-(propan-2-yloxy)-1H-pyrazol-4-yl]pyridin-2-yl}propanamid | 2.58 | 369 | - | - | - | A |
| 227 | 4-[3-(4-Fluorphenyl)-1-(propan-2-yloxy)-1H-pyrazol-4-yl]pyridin | 1.58 | 298 | - | - | - | A |
| 228 | 3-(4-Fluorphenyl)-4-[3-(4-fluorphenyl)-1-(2-methylpropyl)-1H-pyrazol-4-yl]pyridin | 2.96 | 390 | - | - | - | B |
| 229 | 3-(3-Fluorphenyl)-4-[3-(4-fluorphenyl)-1-(2-methylpropyl)-1H-pyrazol-4-yl]pyridin | 3.13 | 390 | - | - | - | B |
| 230 | N-{4-[3-(3-Cyan-4-fluorphenyl)-1-(2-methylpropyl)-1H-pyrazol-4-yl]pyridin-2-yl}propanamid | 2.67 | 392 | - | - | - | B |
| 231 | 1-{4-[1-(2,2-Difluorethyl)-3-(4-fluorphenyl)-1H-pyrazol-4-yl]pyridin-2-yl}-3-ethylharnstoff | 1.91 | 390 | - | - | - | A |
| 232 | N-(4-{3-(4-Fluorphenyl)-1-[1-(2-fluorphenyl)ethyl]-1H-pyrazol-4-yl}pyridin-2-yl)-2-methylpropanamid | 3.41 | 447 | - | - | - | B |
| 233 | 3-{4-[3-(4-Fluorphenyl)-1-(propan-2-yl)-1H-pyrazol-4-yl]pyridin-2-yl} -1,1-dimethylharnstoff | 1.60 | 368 | - | - | - | A |
| 234 | N-{4-[1-(2,2-Difluorethyl)-3-(4-fluorphenyl)-1H-pyrazol-4-yl]pyridin-2-yl}propan-2-sulfonamid | 3.96 | 425 | - | - | - | B |
| 235 | N-{4-[1-(2,2-Difluorethyl)-3-(4-fluorphenyl)-1H-pyrazol-4-yl]pyridin-2-yl}thiophen-3-carboxamid | 2.69 | 429 | - | - | - | B |
| 236 | N-{4-[1-(2,2-Difluorethyl)-3-(4-fluorphenyl)-1H-pyrazol-4-yl]pyridin-2-yl}thiophen-2-carboxamid | 2.82 | 429 | - | - | - | B |
| 237 | N-{4-[1-(2,2-Difluorethyl)-3-(4-fluorphenyl)-1H-pyrazol-4-yl]pyridin-2-yl}benzamid | 2.90 | 423 | - | - | - | B |
| 238 | N-{4-[1-(2,2-Difluorethyl)-3-(4-fluorphenyl)-1H-pyrazol-4-yl]pyridin-2-yl}-3-methylbutanamid | 2.63 | 403 | - | - | - | B |
| 239 | N-{4-[1-(2,2-Difluorethyl)-3-(4-fluorphenyl)-1H-pyrazol-4-yl]pyridin-2-yl}-2-methylbutanamid | 2.62 | 403 | - | - | - | B |
| 240 | N-{4-[1-(2,2-Difluorethyl)-3-(4-fluorphenyl)-1H-pyrazol-4-yl]pyridin-2-yl}cyclobutancarboxamid | 2.42 | 401 | - | - | - | B |
| 241 | Ethyl-{4-[1-(2,2-difluorethyl)-3-(4-fluorphenyl)-1H-pyrazol-4-yl]pyridin-2-yl}carbamat | 2.45 | 391 | - | - | - | B |
| 242 | N-{4-[1-(2,2-Difluorethyl)-3-(4-fluorphenyl)-1H-pyrazol-4-yl]pyridin-2-yl}acetamid | 1.74 | 361 | - | - | - | B |
| 243 | N-{4-[1-(2,2-Difluorethyl)-3-(4-fluorphenyl)-1H-pyrazol-4-yl]pyridin-2-yl}-2-hydroxy-2-methylpropanamid | 2.18 | 405 | - | - | - | B |
| 244 | N-(4-{3-(4-Fluorphenyl)-1-[1-(2-fluorphenyl)ethyl]-1H-pyrazol-4-yl}pyridin-2-yl)-2-methoxyacetamid | 3.50 | 449 | - | - | - | B |
| 245 | N-(4-{1-[(2,2-Dichlorcyclopropyl)methyl]-3-(4-fluorphenyl)-1H-pyrazol-4-yl}pyridin-2-yl)-2-methoxyacetamid | 3.22 | 449 | - | - | - | B |
| 246 | N-{4-[3-(3-Cyan-4-fluorphenyl)-1-(2-methylpropyl)-1H-pyrazol-4-yl]pyridin-2-yl}-2-methoxyacetamid | 2.98 | 408 | - | - | - | B |
| 247 | N-{4-[1-(2-Fluorbenzyl)-3-(4-fluorphenyl)-1H-pyrazol-4-yl]pyridin-2-yl}-2-methoxyacetamid | 3.09 | 435 | - | - | - | B |
| 248 | N-(4-{3-(4-Fluorphenyl)-1-[1-(2-fluorphenyl)ethyl]-1H-pyrazol-4-yl}pyridin-2-yl)propanamid | 3.03 | 433 | - | - | - | B |
| 249 | N-(4-{1-[(2,2-Dichlorcyclopropyl)methyl]-3-(4-fluorphenyl)-1H-pyrazol-4-yl}pyridin-2-yl)propanamid | 2.80 | 433 | - | - | - | B |
| 250 | 2-Cyclopropyl-N-(4-{3-(4-fluorphenyl)-1-[1-(2-fluorphenyl)ethyl]-1H-pyrazol-4- | 3.54 | 459 | - | - | - | B |
| 251 | yl}pyridin-2-yl)acetamid 2-Cyclopropyl-N-(4-{1-[(2,2-dichlorcyclopropyl)methyl]-3-(4-fluorphenyl)-1H-pyrazol-4-yl}pyridin-2-yl)acetamid | 3.30 | 459 | - | - | - | B |
| 252 | N-{4-[3-(3-Cyan-4-fluorphenyl)-1-(2-methylpropyl)-1H-pyrazol-4-yl]pyridin-2-yl} -2-cyclopropylacetamid | 3.14 | 418 | - | - | - | B |
| 253 | 2-Cyclopropyl-N-{4-[1-(2-fluorbenzyl)-3-(4-fluorphenyl)-1H-pyrazol-4-yl]pyridin-2-yl}acetamid | 3.17 | 445 | - | - | - | B |
| 254 | N-(4-{1-[(2,2-Dichlorcyclopropyl)methyl]-3-(4-fluorphenyl)-1H-pyrazol-4-yl}pyridin-2-yl)-2-methylpropanamid | 3.17 | 447 | - | - | - | B |
| 255 | N-{4-[3-(3-Cyan-4-fluorphenyl)-1-(2-methylpropyl)-1H-pyrazol-4-yl]pyridin-2-yl} -2-methylpropanamid | 3.03 | 406 | - | - | - | B |
| 256 | N-{4-[1-(2-Fluorbenzyl)-3-(4-fluorphenyl)-1H-pyrazol-4-yl]pyridin-2-yl}-2-methylpropanamid | 3.03 | 433 | - | - | - | B |
| 257 | N-{4-[5-(Difluormethoxy)-3-(4-fluor-2-hydroxyphenyl)-1-methyl-1H-pyrazol-4-yl]pyridin-2-yl}-2-methylpropanamid | 2.44 | 421 | - | - | - | B |
| 258 | N-{4-[5-(Difluormethoxy)-3-(4-fluor-2-hydroxyphenyl)-1-methyl-1H-pyrazol-4-yl]pyridin-2-yl}cyclopropancarboxamid | 2.22 | 419 | - | - | - | B |
| 259 | N-(4-{3-(4-Fluorphenyl)-1-[1-(2-fluorphenyl)ethyl]-1H-pyrazol-4-yl}pyridin-2-yl)cyclopropancarboxamid | *3.13* | 445 | - | - | - | B |
| 260 | N-(4-{1-[(2,2-Dichlorcyclopropyl)methyl]-3-(4-fluorphenyl)-1H-pyrazol-4-yl}pyridin-2-yl)cyclopropancarboxamid | 2.67 | 445 | - | - | - | B |
| 261 | N-{4-[3-(3-Cyan-4-fluorphenyl)-1-(2-methylpropyl)-1H-pyrazol-4-yl]pyridin-2-yl}cyclopropancarboxamid | 2.77 | 404 | - | - | - | B |
| 262 | N-{4-[1-(2-Fluorbenzyl)-3-(4-fluorphenyl)-1H-pyrazol-4-yl]pyridin-2-yl}cyclopropancarboxamid | 2.77 | 431 | - | - | - | B |
| 263 | Ethyl-(4-{3-(4-fluorphenyl)-1-[1-(2-fluorphenyl)ethyl]-1H-pyrazol-4-yl}pyridin-2-yl)carbamat | *3.65* | 449 | - | - | - | B |
| 264 | Ethyl-(4-{1-[(2,2-dichlorcyclopropyl)methyl]-3-(4-fluorphenyl)-1H-pyrazol-4-yl}pyridin-2-yl)carbamat | *3.37* | 451 | - | - | - | B |
| 265 | Ethyl-{4-[3-(3-cyan-4-fluorphenyl)-1-(2-methylpropyl)-1H-pyrazol-4-yl]pyridin-2-yl}carbamat | *3.19* | 408 | - | - | - | B |
| 266 | Ethyl-{4-[1-(2-fluorbenzyl)-3-(4-fluorphenyl)-1H-pyrazol-4-yl]pyridin-2-yl}carbamat | 3.24 | 435 | - | - | - | B |
| 267 | Ethyl-{4-[3-(4-fluorphenyl)-1-(2-methylpropyl)-1H-pyrazol-4-yl]pyridin-2-yl}carbamat | 3.11 | 383 | - | - | - | B |
| 268 | Ethyl-{4-[1-(butan-2-yl)-3-(4-fluorphenyl)-1H-pyrazol-4-yl]pyridin-2-yl}carbamat | 3.08 | 383 | - | - | - | B |
| 269 | N-{4-[1-(Cyclopentyloxy)-3-(4-fluorphenyl)-1H-pyrazol-4-yl]pyridin-2-yl}cyclopropancarboxamid | 3.35 | 407 | - | - | - | A |
| 270 | N-{4-[1-(Cyclopentyloxy)-3-(4-fluorphenyl)-1H-pyrazol-4-yl]pyridin-2-yl}-2-methylpropanamid | 3.65 | 409 | - | - | - | A |
| 271 | N-{4-[3-(4-Fluorphenyl)-1-(propan-2-yloxy)-1H-pyrazol-4-yl]pyridin-2-yl}cyclopropancarboxamid | 2.73 | 381 | - | - | - | A |
| 272 | N-{4-[3-(4-Fluorphenyl)-1-(propan-2-yloxy)-1H-pyrazol-4-yl]pyridin-2-yl}-2-methylpropanamid | 3.01 | 383 | - | - | - | A |
| 273 | N-(Cyclopropylmethyl)-4-[3-(4-fluorphenyl)-1-(2-methylpropyl)-1H-pyrazol-4-yl]pyrimidin-2-amin | 2.55 | 366 | - | - | - | C |
| 274 | Methyl-2-{3-(4-fluorphenyl)-4-[2-(propionylamino)pyridin-4-yl]-1H-pyrazol-1-yl}-3-methylbutanoat | 2.67 | 425 | - | - | - | A |
| 275 | 4-[1-(1,3-Dioxolan-2-ylmethyl)-3-(4-fluorphenyl)-1H-pyrazol-4-yl]pyridin | 1.02 | 326 | - | - | - | A |
| 276 | N-{4-[1-(1,3-Dioxolan-2-ylmethyl)-3-(4-fluorphenyl)-1H-pyrazol-4-yl]pyridin-2-yl}-2-methylpropanamid | 1.99 | 411 | - | - | - | A |
| 277 | Methyl-2-{3-(4-fluorphenyl)-4-[2-(isobutyrylamino)pyridin-4-yl]-1H-pyrazol-1-yl}-3-methylbutanoat | 3.05 | 439 | - | - | - | A |
| 278 | N-{4-[1-(1,3-Dioxolan-2-ylmethyl)-3-(4-fluorphenyl)-1H-pyrazol-4-yl]pyridin-2-yl}propanamid | 1.59 | 397 | - | - | - | B |
| 279 | 4-[3-(4-Fluorphenyl)-1-(2-methylpropyl)-1H-pyrazol-4-yl]-N-(oxetan-3-yl)pyrimidin-2-amin | 2.35 | 368 | - | - | - | C |
| 280 | 7-[3-(4-fluorophenyl)-1-isopropyl-1H-pyrazol-4-yl]-3H-imidazo[4,5-b]pyridine | 1.82 | 322 | - | - | - | C |
| 281 | N-Cyclopropyl-4-[3-(4-fluorphenyl)-1-(2-methylpropyl)-1H-pyrazol-4-yl]pyrimidin-2-amin | 2.41 | 352 | - | - | - | C |
| 282 | 6-[3-(4-Fluorphenyl)-1-isopropyl-1H-pyrazol-4-yl]-9H-purin | 1.79 | 323 | - | - | - | B |
| 283 | 4-[3-(4-fluorophenyl)-1-isopropyl-1H-pyrazol-4-yl]-2-(4-methyl-1H-imidazol-1-yl)pyridine | 2.05 | 362 | - | - | - | C |
| 284 | N-tert-Butyl-4-[3-(4-fluorphenyl)-1-(2-methylpropyl)-1H-pyrazol-4-yl]pyrimidin-2-amin | 2.94 | 368 | - | - | - | C |
| 285 | 4-[3-(4-Fluorphenyl)-1-(2-methylpropyl)-1H-pyrazol-4-yl]-N-(1-methoxypropan-2-yl)pyrimidin-2-amin | 2.39 | 384 | - | - | - | C |
| 286 | N-(Butan-2-yl)-4-[3-(4-fluorphenyl)-1-(2-methylpropyl)-1H-pyrazol-4-yl]pyrimidin-2-amin | 2.68 | 368 | - | - | - | C |
| 287 | 4-[3-(4-Fluorphenyl)-1-(2-methylpropyl)-1H-pyrazol-4-yl]-2-(methylsulfonyl)pyrimidin | 2.90 | 375 | - | - | - | C |
| 288 | 4-[3-(4-Fluorphenyl)-1-isobutyl-1H-pyrazol-4-yl]-7-methylthieno[3,2-d]pyrimidin | 3.85 | 367 | - | - | - | B |
| 289 | 4-[1-(Cyclopropylmethyl)-3-(4-fluorphenyl)-1H-pyrazol-4-yl]-7-methylthieno[3,2-d]pyrimidin | 3.45 | 365 | - | - | - | B |
| 290 | N-{4-[1-Ethyl-3-(4-fluorphenyl)-1H-pyrazol-4-yl]pyridin-2-yl}-2-methylpropanamid | 2.14 | 353 | - | - | - | B |
| 291 | 2-Cyclopropyl-N-{4-[1-ethyl-3-(4-fluorphenyl)-1H-pyrazol-4-yl]pyridin-2-yl}acetamid | 2.29 | 365 | - | - | - | B |
| 292 | N-{4-[3-(4-Fluorphenyl)-1-propyl-1H-pyrazol-4-yl]pyridin-2-yl}propanamid | 2.15 | 353 | - | - | - | B |
| 293 | N-{4-[3-(4-Fluorphenyl)-1-propyl-1H-pyrazol-4-yl]pyridin-2-yl}-2-methoxyacetamid | 2.58 | 369 | - | - | - | B |
| 294 | N-{4-[3-(4-Fluorphenyl)-1-propyl-1H-pyrazol-4-yl]pyridin-2-yl}-2-methylpropanamid | 2.49 | 367 | - | - | - | B |
| 295 | 2-Cyclopropyl-N-{4-[3-(4-fluorphenyl)-1-propyl-1H-pyrazol-4-yl]pyridin-2-yl}acetamid | 2.66 | 379 | - | - | - | B |
| 296 | N-{4-[3-(4-Fluorphenyl)-1-methyl-1H-pyrazol-4-yl]pyridin-2-yl}propanamid | 1.56 | 325 | - | - | - | B |
| 297 | N-{4-[3-(4-Fluorphenyl)-1-methyl-1H-pyrazol-4-yl]pyridin-2-yl}-2-methoxyacetamid | 1.89 | 341 | - | - | - | B |
| 298 | N-{4-[3-(4-Fluorphenyl)-1-methyl-1H-pyrazol-4-yl]pyridin-2-yl}-2-methylpropanamid | 1.83 | 339 | - | - | - | B |
| 299 | 2-Cyclopropyl-N-{4-[3-(4-fluorphenyl)-1-methyl-1H-pyrazol-4-yl]pyridin-2-yl}acetamid | 1.98 | 351 | - | - | - | B |
| 300 | N-(4-{3-(4-Fluorphenyl)-1-[2-(methylsulfanyl)ethyl]-1H-pyrazol-4-yl}pyridin-2-yl)propanamid | 2.12 | 385 | - | - | - | B |
| 301 | N-{4-[1-(1-Cyclopropylethyl)-3-(4-fluorphenyl)-1H-pyrazol-4-yl]pyridin-2-yl}propanamid | 2.54 | 379 | - | - | - | B |
| 302 | N-{4-[1-(3-Cyanbenzyl)-3-(4-fluorphenyl)-1H-pyrazol-4-yl]pyridin-2-yl}propanamid | 2.38 | 426 | - | - | - | B |
| 303 | N-{4-[1-(2-Cyanethyl)-3-(4-fluorphenyl)-1H-pyrazol-4-yl]pyridin-2-yl}propanamid | 1.63 | 364 | - | - | - | B |
| 304 | N-{4-[3-(4-Fluorphenyl)-1-isopropyl-5-(trifluormethyl)-1H-pyrazol-4-yl]pyridin-2-yl}propanamid | 4.07 | 421 | - | - | - | B |
| 305 | 4-[1-(1-Cyclopropylethyl)-3-(4-fluorphenyl)-1H-pyrazol-4-yl]pyridin | 1.59 | 308 | - | - | - | B |
| 306 | N-(4-{3-(4-Fluorphenyl)-1-[2-(methylsulfanyl)ethyl]-1H-pyrazol-4-yl}pyridin-2-yl)-2-methylpropanamid | 2.44 | 399 | - | - | - | B |
| 307 | N-{4-[1-(1-Cyclopropylethyl)-3-(4-fluorphenyl)-1H-pyrazol-4-yl]pyridin-2-yl}-2-methylpropanamid | 2.90 | 393 | - | - | - | B |
| 308 | N-{4-[1-(3-Cyanbenzyl)-3-(4-fluorphenyl)-1H-pyrazol-4-yl]pyridin-2-yl}-2-methylpropanamid | 2.69 | 440 | - | - | - | B |
| 309 | N-{4-[1-(2-Cyanethyl)-3-(4-fluorphenyl)-1H-pyrazol-4-yl]pyridin-2-yl}-2-methylpropanamid | 1.91 | 378 | - | - | - | B |
| 310 | 4-[3-(4-Fluorphenyl)-1-isopropyl-1H-pyrazol-4-yl]-7H-pyrrolo[2,3-d]pyrimidin | 1.61 | 322 | - | - | - | B |
| 311 | 4-[3-(4-Fluorphenyl)-1-isopropyl-1H-pyrazol-4-yl]-5,6,7,8-tetrahydropyrido[2,3-d]pyrimidin | 1.29 | 338 | - | - | - | B |
| 312 | N-{4-[1-(1-Cyanpropan-2-yl)-3-(4-fluorphenyl)-1H-pyrazol-4-yl]pyridin-2-yl}propanamid | 1.87 | 378 | - | - | - | B |
| 313 | N-{4-[1-(1-Cyanpropan-2-yl)-3-(4-fluorphenyl)-1H-pyrazol-4-yl]pyridin-2-yl}-2-methoxyacetamid | 2.17 | 394 | - | - | - | B |
| 314 | N-{4-[1-(1-Cyanpropan-2-yl)-3-(4-fluorphenyl)-1H-pyrazol-4-yl]pyridin-2-yl}-2-methylpropanamid | 1.29 | 392 | - | - | - | B |
| 315 | N-{4-[1-(1-Cyanpropan-2-yl)-3-(4-fluorphenyl)-1H-pyrazol-4-yl]pyridin-2-yl}-2-cyclopropylacetamid | 2.28 | 404 | - | - | - | B |
| 316 | N-{4-[5-(Difluormethoxy)-3-(4-fluorphenyl)-1-methyl-1H-pyrazol-4-yl]pyridin-2-yl}propanamid | 2.33 | 391 | - | - | - | B |
| 317 | 4-[1-(Cyclopropylmethyl)-3-(4-fluorphenyl)-1H-pyrazol-4-yl]-N-(1,1,1-trifluorpropan-2-yl)pyridin-2-amin | 2.33 | 405 | - | - | - | B |
| 318 | 4-[3-(4-Fluorphenyl)-1-isopropyl-1H-pyrazol-4-yl]-N-(1,1,1-trifluorpropan-2-yl)pyridin-2-amin | 2.26 | 393 | - | - | - | B |
| 319 | 4-[3-(4-Fluorphenyl)-1-methyl-1H-pyrazol-4-yl]-N-(1,1,1-trifluorpropan-2-yl)pyridin-2-amin | 1.73 | 365 | - | - | - | B |
| 320 | N-{4-[1-Cyclopropyl-3-(4-fluorphenyl)-1H-pyrazol-4-yl]pyridin-2-yl}propanamid | 1.97 | 351 | - | - | - | B |
| 321 | N-{4-[1-Cyclopropyl-3-(4-fluorphenyl)-1H-pyrazol-4-yl]pyridin-2-yl}-2-methoxyacetamid | 2.37 | 367 | - | - | - | B |
| 322 | N-{4-[1-Cyclopropyl-3-(4-fluorphenyl)-1H-pyrazol-4-yl]pyridin-2-yl}-2-methylpropanamid | 2.32 | 365 | - | - | - | B |
| 323 | 2-Cyclopropyl-N-{4-[1-cyclopropyl-3-(4-fluorphenyl)-1H-pyrazol-4-yl]pyridin-2-yl}acetamid | 2.48 | 377 | - | - | - | B |
| 324 | 4-[3-(4-Fluorphenyl)-1-isopropyl-1H-pyrazol-4-yl]-N-[2,2,2-trifluor-1-(4-fluorphenyl)ethyl]pyridin-2-amin | 3.88 | 473 | - | - | - | B |
| 325 | 4-[3-(4-Fluorphenyl)-1-isopropyl-1H-pyrazol-4-yl]-N-(2,2,2-trifluor-1-phenylethyl)pyridin-2-amin | 3.66 | 455 | - | - | - | B |
| 326 | N-{4-[1-(2-Ethoxyethyl)-3-(4-fluorphenyl)-1H-pyrazol-4-yl]pyridin-2-yl}propanamid | 2.01 | 383 | - | - | - | B |
| 327 | N-{4-[1-(2-Ethoxyethyl)-3-(4-fluorphenyl)-1H-pyrazol-4-yl]pyridin-2-yl}-2-methoxyacetamid | 2.38 | 399 | - | - | - | B |
| 328 | N-{4-[1-(2-Ethoxyethyl)-3-(4-fluorphenyl)-1H-pyrazol-4-yl]pyridin-2-yl}-2-methylpropanamid | 2.32 | 397 | - | - | - | B |
| 329 | 2-Cyclopropyl-N-{4-[1-(2-ethoxyethyl)-3-(4-fluorphenyl)-1H-pyrazol-4-yl]pyridin-2-yl}acetamid | 2.48 | 409 | - | - | - | B |
| 330 | Methyl-6-({4-[1-ethyl-3-(4-fluorphenyl)-1H-pyrazol-4-yl]pyridin-2-yl}amino)-6-oxohexanoat | 2.16 | 425 | - | - | - | A |
| 331 | Methyl-5-({4-[1-ethyl-3-(4-fluorphenyl)-1H-pyrazol-4-yl]pyridin-2-yl}amino)-5-oxopentanoat | 2.02 | 411 | - | - | - | A |
| 332 | Ethyl-3-ethyl-5-({4-[1-ethyl-3-(4-fluorphenyl)-1H-pyrazol-4-yl]pyridin-2-yl}amino)-5-oxopentanoat | 2.90 | 453 | - | - | - | A |
| 333 | 3-(Difluormethyl)-N-{4-[1-ethyl-3-(4-fluorphenyl)-1H-pyrazol-4-yl]pyridin-2-yl}-1-methyl-1H-pyrazol-4-carboxamid | 2.47 | 441 | - | - | - | A |
| 334 | 2-(Difluormethyl)-N-{4-[1-ethyl-3-(4-fluorphenyl)-1H-pyrazol-4-yl]pyridin-2-yl}thiophen-3-carboxamid | 3.27 | 443 | - | - | - | A |
| 335 | N-{4-[1-Ethyl-3-(4-fluorphenyl)-1H-pyrazol-4-yl]pyridin-2-yl}-4-methyl-1,2,3-thiadiazol-5-carboxamid | 2.72 | 409 | - | - | - | A |
| 336 | 3-(4-Chlorphenyl)-N-{4-[1-ethyl-3-(4-fluorphenyl)-1H-pyrazol-4-yl]pyridin-2-yl}propanamid | 3.32 | 449 | - | - | - | A |
| 337 | (1R,2R)-N-{4-[1-Ethyl-3-(4-fluorphenyl)-1H-pyrazol-4-yl]pyridin-2-yl}-2-phenylcyclopropancarboxamid | 3.06 | 427 | - | - | - | A |
| 338 | 2-Cyclopentyl-N-{4-[1-ethyl-3-(4-fluorphenyl)-1H-pyrazol-4-yl]pyridin-2-yl}acetamid | 3.87 | 393 | - | - | - | A |
| 339 | N-{4-[1-Ethyl-3-(4-fluorphenyl)-1H-pyrazol-4-yl]pyridin-2-yl}-3-methylbutanamid | 3.47 | 367 | - | - | - | A |
| 340 | N-{4-[1-Ethyl-3-(4-fluorphenyl)-1H-pyrazol-4-yl]pyridin-2-yl}-2-methylbutanamid | 2.42 | 367 | - | - | - | A |
| 341 | N-{4-[1-Ethyl-3-(4-fluorphenyl)-1H-pyrazol-4-yl]pyridin-2-yl}-2-methylcyclopropancarboxamid | 2.16 | 365 | - | - | - | A |
| 342 | N-{4-[1-Ethyl-3-(4-fluorphenyl)-1H-pyrazol-4-yl]pyridin-2-ylacetamid | 1.57 | 325 | - | - | - | A |
| 343 | N-{4-[3-(4-Fluorphenyl)-1-methyl-1H-pyrazol-4-yl]pyridin-2-yl}-3-phenylpropanamid | 2.54 | 401 | - | - | - | A |
| 344 | 4-Fluor-N-{4-[3-(4-fluorphenyl)-1-methyl-1H-pyrazol-4-yl]pyridin-2-yl}benzamid | 2.61 | 391 | - | - | - | A |
| 345 | N-{4-[3-(4-Fluorphenyl)-1-methyl-1H-pyrazol-4-yl]pyridin-2-yl}-2-(2-methoxyethoxy)acetamid | 1.96 | 385 | - | - | - | A |
| 346 | N-{4-[3-(4-Fluorphenyl)-1-methyl-1H-pyrazol-4-yl]pyridin-2-yl}benzamid | 2.42 | 373 | - | - | - | A |
| 347 | N-{4-[3-(4-Fluorphenyl)-1-methyl-1H-pyrazol-4-yl]pyridin-2-yl}-3-methylbutanamid | 2.12 | 353 | - | - | - | A |
| 348 | N-{4-[3-(4-Fluorphenyl)-1-methyl-1H-pyrazol-4-yl]pyridin-2-yl}-2-methylbutanamid | 2.14 | 353 | - | - | - | A |
| 349 | N-{4-[3-(4-Fluorphenyl)-1-methyl-1H-pyrazol-4-yl]pyridin-2-yl}cyclobutancarboxamid | 1.93 | 351 | - | - | - | A |
| 350 | N-{4-[3-(4-Fluorphenyl)-1-methyl-1H-pyrazol-4-yl]pyridin-2-yl}acetamid | 1.32 | 311 | - | - | - | A |
| 351 | N-{4-[1-Ethyl-3-(4-fluorphenyl)-1H-pyrazol-4-yl]pyridin-2-yl}ethandiamid | 1.99 | 354 | - | - | - | A |
| 352 | (2S)-N-{4-[1-Ethyl-3-(4-fluorphenyl)-1H-pyrazol-4-yl]pyridin-2-yl}-2-hydroxypropanamid | 1.83 | 354 | - | - | - | A |
| 353 | (2S)-N-{4-[3-(4-Fluorphenyl)-1-methyl-1H-pyrazol-4-yl]pyridin-2-yl}-2-hydroxypropanamid | 1.57 | 341 | - | - | - | A |
| 354 | N-{4-[3-(4-Fluorphenyl)-1-(propan-2-yl)-1H-pyrazol-4-yl]pyrimidin-2-yl}-2-methoxyacetamid | 2.37 | 370 | - | - | - | C |
| 355 | N-{4-[3-(4-Fluorphenyl)-1-(propan-2-yl)-1H-pyrazol-4-yl]pyrimidin-2-yl}-2-methylpropanamid | 2.55 | 368 | - | - | - | C |
| 356 | N-{4-[3-(4-Fluorphenyl)-1-(propan-2-yl)-1H-pyrazol-4-yl]pyrimidin-2-yl}cyclopropancarboxamid | 2.49 | 366 | - | - | - | C |
| 357 | N-{4-[3-(4-Fluorphenyl)-1-(2-methylpropyl)-1H-pyrazol-4-yl]pyrimidin-2-yl}acetamid | 2.44 | 354 | - | - | - | C |
| 358 | 4-[3-(4-Fluorphenyl)-1-(2-methoxyethyl)-1H-pyrazol-4-yl]-N-(propan-2-yl)pyrimidin-2-amin | 1.79 | 356 | 1.76 | 356 | 76:23 | C |
| 359 | 4-[1-(2-Chlorethyl)-3-(4-fluorphenyl)-1H-pyrazol-4-yl]-N-(propan-2-yl)pyrimidin-2-amin | 2.05 | 360 | 2.01 | 360 | 70:30 | C |
| 360 | 4-[1-(Butan-2-yl)-3-(4-fluorphenyl)-1H-pyrazol-4-yl]-N-(propan-2-yl)pyrimidin-2-amin | 2.41 | 354 | - | - | - | C |
| 361 | 4-[3-(4-Fluorphenyl)-1-methyl-1H-pyrazol-4-yl]-N-(propan-2-yl)pyrimidin-2-amin | 1.65 | 312 | - | - | - | C |
| 362 | 3-{3-(4-Fluorphenyl)-4-[2-(propan-2-ylamino)pyrimidin-4-yl]-1H-pyrazol-1-yl}propanonitril | 1.74 | 351 | - | - | - | C |
| 363 | 4-[3-(4-Fluorphenyl)-1-(prop-2-in-1-yl)-1H-pyrazol-4-yl]-N-(propan-2-yl)pyrimidin-2-amin | 1.99 | 336 | - | - | - | C |
| 364 | 4-[1-(2,2-Difluorethyl)-3-(4-fluorphenyl)-1H-pyrazol-4-yl]-N-(propan-2-yl)pyrimidin-2-amin | 2.05 | 362 | - | - | - | C |
| 365 | N-{4-[3-(4-Fluorphenyl)-1-(2-methylpropyl)-1H-pyrazol-4-yl]pyrimidin-2-yl}propanamid | 2.70 | 368 | - | - | - | C |
| 366 | 4-[3-(4-Fluorphenyl)-1-(propan-2-yl)-1H-pyrazol-4-yl]pyrimidin-2-amin | 1.54 | 298 | - | - | - | C |
| 367 | 4-[1-(2,2-Difluorethyl)-3-(4-fluorphenyl)-5-methyl-1H-pyrazol-4-yl]pyridin | 1.49 | 318 | 1.43 | 318 | 66:23 | C |
| 368 | 4-[3-(4-Fluorphenyl)-5-methyl-1-(2-methylpropyl)-1H-pyrazol-4-yl]pyridin | 2.07 | 310 | 1.98 | 310 | 64:28 | C |
| 369 | N-Benzyl-4-[3-(4-fluorphenyl)-1-(propan-2-yl)-1 H-pyrazol-4-yl]pyrimidin-2-amin | 3.31 | 388 | - | - | - | C |
| 370 | 4-[1-Ethyl-3-(4-fluorphenyl)-1H-pyrazol-4-yl]-N-(propan-2-yl)pyrimidin-2-amin | 1.91 | 326 | - | - | - | C |
| 371 | 4-[3-(4-Fluorphenyl)-1-(propan-2-yl)-1H-pyrazol-4-yl]-N-(propan-2-yl)pyrimidin-2-amin | 2.92 | 340 | - | - | - | C |
| 372 | N-(1-Cyclopropylethyl)-4-[3-(4-fluorphenyl)-1-(2-methylpropyl)-1H-pyrazol-4-yl]pyrimidin-2-amin | 2.73 | 380 | - | - | - | C |

| | | | | | | | |
|---|---|---|---|---|---|---|---|
| ¹ Bei dem angegebenen logP Wert handelt es sich um den Wert der benannten reinen Verbindung bzw. bei Vorliegen einer Mischung auf das Hauptisomer (1-substituiertes-3-Aryl-1H-pyrazol). ² Bei der angegebenen Masse handelt es sich um den Peak des Isotopenmusters des [M+H]+ Ions mit der höchsten Intensität; falls das [M-H]- Ion detektiert wurde, ist die Massenangabe mit 6 gekennzeichnet. ³ Bei einem angegebenen logP Wert handelt es sich um den Wert des in einer Isomerenmischung (bezogen auf die Pyrazolsubstitution) vorliegenden Minderisomers (1-substituiertes-5-Aryl-1H-pyrazol) ⁴ Das Isomerenverhältnis bezieht sich auf eine gegebenenfalls erhaltene Mischung verschiedener Pyrazolisomerer (Das Hauptisomer ist das 1-substituierte-3-Aryl-1H-pyrazol, das Minderisomer ist das entsprechende 1-substituierte-5-Aryl-1H-pyrazol). Wenn kein Isomerenverhältnis angegeben wurde, liegt ausschliesslich das Hauptisomer vor. Das Isomerenverhältnis bezieht sich auf die Fläche des UV-Chromatogramms (bei einer Wellenlänge von 210nm) des mit der Methode A, B oder C gemessenen LC-MS Spektrums. ⁵ Bei der Bestimmung der logP Werte wurden die unten beschriebenen Methoden angewendet. ⁶ Bei der angegebenen Masse handelt es sich um den Peak des Isotopenmusters des [M-H]- Ions mit der höchsten Intensität. | | | | | | | |

Methode A Anmerkung zur Bestimmung der logP-Werte und Massendetektion: Die Bestimmung der angegebenen logP-Werte erfolgte gemäß EEC-Direktive 79/831 Annex V.A8 durch HPLC (High Performance Liquid Chromatography) an einer Phasenumkehrsäule (C18).Agilent 1100 LC-System; 50*4,6 Zorbax Eclipse Plus C18 1,8 microm; Eluent A: Acetonitril (0,1 % Ameisensäure); Eluent B: Wasser (0,09 % Ameisensäure); linearer Gradient von 10 % Acetonitril bis 95 % Acetonitril in 4,25 min, dann 95% Acetonitril für weitere 1,25 min; Ofentemperatur 55 °C; Fluß:2,0 mL/min. Die Massendetektion erfolgt über ein Agilend MSD-System.

Methode B Anmerkung zur Bestimmung der logP-Werte und Massendetektion: Die Bestimmung der angegebenen logP-Werte erfolgte gemäß EEC-Direktive 79/831 Annex V.A8 durch HPLC (High Performance Liquid Chromatography) an einer Phasenumkehrsäule (C18). HP1100; 50*4,6 Zorbax Eclipse Plus C18 1,8 microm; Eluent A: Acetonitril (0,1 % Ameisensäure); Eluent B: Wasser (0,08 % Ameisensäure); linearer Gradient von 5 % Acetonitril bis 95 % Acetonitril in 1,70 min, dann 95 % Acetonitril für weitere 1,00 min; Ofentemperatur 55°C; Fluß:2,0 mL/min. Die Massendetektion erfolgt über den Massendetektor Micromass ZQ2000 der Firma Waters.

Methode C Anmerkung zur Bestimmung der logP-Werte und Massendetektion: Die Bestimmung der angegebenen logP-Werte erfolgte gemäß EEC-Direktive 79/831 Annex V.A8 durch UPLC (Ultra Performance Liquid Chromatography) an einer Phasenumkehrsäule (C18). HP1100; 50*2,1 Zorbax Eclipse Plus C18 1,8 microm; Eluent A: Acetonitril (0,09 % Ameisensäure); Eluent B: Wasser (0,1 % Ameisensäure); linearer Gradient von 10 % A bis 95 % A in 3,25 min; Ofentemperatur 40°C; Fluß:0,8 mL/min. Die Massendetektion erfolgt über den Massendetektor LCT Premier oder SQD der Firma Waters.

Die Eichung erfolgt mit unverzweigten Alkan-2-onen (mit 3 bis 16 Kohlenstoffatomen), deren logP-Werte bekannt sind (Bestimmung der logP-Werte anhand der Retentionszeiten durch lineare Interpolation zwischen zwei aufeinanderfolgenden Alkanonen).

Die lambda-max-Werte wurden an Hand der UV-Spektren von 200 nm bis 400 nm in den Maxima der chromatographischen Signale ermittelt.

### Anwendungsbeispiele

### Beispiel A: In vivo Test an Peronospora parasitica (Falscher Mehltau an Weißkohl):

Eine wässrige Suspension des aktiven Wirkstoffes wurde durch Homogenisierung einer Mischung aus Aceton/Tween (Dispergenz)/Dimethylsulfoxid (DMSO) und anschließender Verdünnung mit Wasser auf die gewünschte Konzentration hergestellt. Kohlpflanzen (Sorte Eminence) werden in Aufzuchtschalen auf ein Torferde-Puzzolanerde-Substrat (50/50) bei 18-20°C ausgesät und im Keimblattstadium mit der oben beschriebenen wäßrigen Suspension besprüht. Als Kontrolle werden Pflanzen mit einer wäßrigen Lösung ohne Wirkstoff besprüht. Nach 24 Stunden werden die Pflanzen durch Besprühen mit einer wäßrigen Suspension von Peronospora parasitica Sporen (50 000 Sporen pro ml) inokuliert. Die Sporen stammen von infizierten Pflanzen. Die inokulierten Kohlpflanzen werden 5 Tage bei ca. 20°C in feuchter Atmosphäre inkubiert. Nach 5 Tagen wird im Vergleich mit den Kontrollpflanzen bonitiert. Unter diesen Bedingungen wird bei einer Dosis von 500 ppm eine gute (70% Wirkungsgrade) oder vollständige Inhibition für folgende Verbindungen beobachtet:

| Bsp. Verbindung Nr. | **% Wirkungsgrad** | Bsp. Verbindung Nr. | **% Wirkungsgrad** | Bsp. Verbindung Nr. | **% Wirkungsgrad** |
|---|---|---|---|---|---|
| 1 | **100** | 44 | **78** | 122 | **93** |
| 2 | **85** | 46 | **94** | 123 | **83** |
| 3 | **97** | 81 | **89** | 126 | **89** |
| 6 | **70** | 84 | **100** | 127 | **78** |
| 10 | **85** | 85 | **100** | 129 | **78** |
| 15 | **75** | 86 | **100** | 133 | **89** |
| 19 | **100** | 88 | **83** | 134 | **94** |
| 21 | **100** | 91 | **89** | 136 | **94** |
| 22 | **97** | 102 | **70** | 137 | **72** |
| 23 | **100** | 103 | **100** | 140 | **78** |
| 28 | **97** | 106 | **100** | 141 | **89** |
| 32 | **79** | 113 | **73** | 150 | **100** |
| 33 | **93** | 116 | **97** | 171 | **91** |
| 40 | **98** | 117 | **93** | | |
| 41 | **72** | 120 | **85** | | |

### Beispiel B: In vivo Test an Botrytis cinerea (Grauschimmel an Gurken):

Eine wässrige Suspension des aktiven Wirkstoffes wurde durch Homogenisierung einer Mischung aus Aceton/Tween/Dimethylsulfoxid und anschließender Verdünnung mit Wasser auf die gewünschte Konzentration hergestellt. Gurkenpflanzen (Sorte Vert petit de Paris) werden in Aufzuchtschalen auf ein Torferde-Puzzolanerde-Substrat (50/50) bei 18-20°C ausgesät und im Keimblattstadium Z11 mit der oben beschriebenen wäßrigen Suspension besprüht. Als Kontrolle werden Pflanzen mit einer wäßrigen Lösung ohne Wirkstoff besprüht. Nach 24 Stunden werden die Pflanzen durch tropfenweise Beauftragung einer wäßrigen Suspension von Botrytis cinerea Sporen (150 000 Sporen pro ml) auf die Blattoberfläche inokuliert. Die Sporen stammen von einer 15 Tage alten Kultur, die in folgender Nährlösung suspendiert werden:
- 20 g/l Gelatine
- 50 g/l D-Fruktose
- 2 g/l NH₄NO₃
- 1 g/l KH₂PO₄

Die inokulierten Gurkenpflanzen werden 5-7 Tage in einer Klimakammer bei 15-11 °C (Tag/Nacht) und 80%iger Luftfeuchtigkeit gehalten. Nach 5-7 Tagen wird im Vergleich mit den Kontrollpflanzen bonitiert. Unter diesen Bedingungen wird bei einer Dosis von 500 ppm eine gute (70% Wirkungsgrade) oder vollständige Inhibition für folgende Verbindungen beobachtet:

| Bsp. Verbindung Nr. | **% Wirkungsgrad** | Bsp. Verbindung Nr. | **% Wirkungsgrad** |
|---|---|---|---|
| 1 | **100** | 40 | **100** |
| 2 | **74** | 44 | **71** |
| 8 | **94** | 84 | **100** |
| 10 | **73** | 85 | 100 |
| 13 | **100** | 86 | **100** |
| 14 | **100** | 171 | **71** |
| 17 | **83** | | |

### Beispiel C: In vivo Test an Alternaria brassicae (Dürrfleckenkrankheit an Radieschen):

Eine wässrige Suspension des aktiven Wirkstoffes wurde durch Homogenisierung einer Mischung aus Aceton/Tween/Dimethylsulfoxid und anschließender Verdünnung mit Wasser auf die gewünschte Konzentration hergestellt. Radieschenpflanzen (Sorte Pernot) werden in Aufzuchtschalen auf ein Torferde-Puzzolanerde-Substrat (50/50) bei 18-20°C ausgesät und im Keimblattstadium mit der oben beschriebenen wäßrigen Suspension besprüht. Als Kontrolle werden Pflanzen mit einer wäßrigen Lösung ohne Wirkstoff besprüht. Nach 24 Stunden werden die Pflanzen durch Besprühen mit einer wäßrigen Suspension von *Alternaria* brassicae Sporen (40 000 Sporen pro ml) inokuliert. Die Sporen stammen von einer 12 bis 13 Tage alten Kultur. Die inokulierten Radieschenpflanzen werden 6-7 Tage bei ca. 18°C in feuchter Atmosphäre inkubiert. Nach 6-7 Tagen wird im Vergleich mit den Kontrollpflanzen bonitiert. Unter diesen Bedingungen wird bei einer Dosis von 500 ppm eine gute (70% Wirkungsgrade) oder vollständige Inhibition für folgende Verbindungen beobachtet:

| Bsp. Verbindung Nr. | **% Wirkungsgrad** |
|---|---|
| 1 | **94** |
| 2 | **77** |
| 3 | **88** |
| 10 | **85** |
| 84 | **75** |
| 86 | **75** |

### Beispiel D: In vivo Test an Sphaerotheca fuliginea (Echter Mehltau an Gurke):

Eine wässrige Suspension des aktiven Wirkstoffes wurde durch Homogenisierung einer Mischung aus Aceton/Tween/Dimethylsulfoxid und anschließender Verdünnung mit Wasser auf die gewünschte Konzentration hergestellt. Gurkenpflanzen (Sorte Vert petit de Paris) werden in Aufzuchtschalen auf ein Torferde-Puzzolanerde-Substrat (50/50) bei 20/23°C ausgesät und im Keimblattstadium Z10 mit der oben beschriebenen wäßrigen Suspension besprüht. Als Kontrolle werden Pflanzen mit einer wäßrigen Lösung ohne Wirkstoff besprüht. Nach 24 Stunden werden die Pflanzen durch Besprühen mit einer wäßrigen Suspension von Sphaerotheca fuliginea Sporen (100 000 Sporen pro ml) inokuliert. Die Sporen stammen von einer kontaminierten Pflanze. Die inokulierten Gurkenpflanzen werden bei ca. 20/25°C unter einer relativen Luftfeuchtigkeit von 60/70% inkubiert. Nach 12 Tagen wird im Vergleich mit den Kontrollpflanzen bonitiert. Unter diesen Bedingungen wird bei einer Dosis von 500 ppm eine gute (70% Wirkungsgrade) oder vollständige Inhibition für folgende Verbindungen beobachtet:

| Bsp. Verbindung Nr. | **% Wirkungsgrad** | Bsp. Verbindung Nr. | **% Wirkungsgrad** | Bsp. Verbindung Nr. | **% Wirkungsgrad** |
|---|---|---|---|---|---|
| 1 | **100** | 41 | **98** | 114 | **79** |
| 2 | **93** | 44 | **98** | 116 | **89** |
| 3 | **93** | 46 | **81** | 117 | **93** |
| 6 | **95** | 53 | **98** | 122 | **99** |
| 9 | **75** | 81 | **77** | 123 | **86** |
| 10 | **80** | 84 | **100** | 126 | **85** |
| 11 | **93** | 85 | **100** | 127 | **85** |
| 12 | **95** | 86 | **100** | 129 | **92** |
| 13 | **90** | 88 | **86** | 132 | **82** |
| 14 | **80** | 91 | **88** | 133 | **95** |
| 15 | **87** | 92 | **93** | 133 | **91** |
| 17 | **80** | 102 | **71** | 134 | **85** |
| 22 | **71** | 103 | **100** | 136 | **91** |
| 24 | **88** | 105 | **82** | 138 | **77** |
| 26 | **80** | 106 | **100** | 141 | **77** |
| 27 | **73** | 107 | **91** | 150 | **96** |
| 33 | **71** | 108 | **82** | 171 | **100** |
| 37 | **78** | 110 | **79** | | |
| 40 | **100** | 113 | **82** | | |

### Beispiel E: In vivo Test an Pyrenophora teres (Gersten Netzfleckenkrankheit):

Eine wässrige Suspension des aktiven Wirkstoffes wurde durch Homogenisierung einer Mischung aus Aceton/Tween/Dimethylsulfoxid und anschließender Verdünnung mit Wasser auf die gewünschte Konzentration hergestellt. Gerstenpflanzen (Sorte Plaisant) werden in Aufzuchtschalen auf ein Torferde-Puzzolanerde-Substrat (50/50) bei 12°C ausgesät und im Erstblattstadium (10 cm groß) mit der oben beschriebenen wäßrigen Suspension besprüht. Als Kontrolle werden Pflanzen mit einer wäßrigen Lösung ohne Wirkstoff besprüht. Nach 24 Stunden werden die Pflanzen durch Besprühen mit einer wäßrigen Suspension von Pyrenophora teres Sporen (12 000 Sporen pro ml) inokuliert. Die Sporen stammen von einer 12 Tage alten Kultur. Die inokulierten Gerstenpflanzen werden erst für 24 Stunden bei ca. 20°C und 100% relativer Luftfeuchtigkeit und anschließend für 12 Tage bei 80% relativer Luftfeuchtigkeit inkubiert. Nach 12 Tagen wird im Vergleich mit den Kontrollpflanzen bonitiert. Unter diesen Bedingungen wird bei einer Dosis von 500 ppm eine gute (70% Wirkungsgrade) oder vollständige Inhibition für folgende Verbindungen beobachtet:

| Bsp. Verbindung Nr. | **% Wirkungsgrad** | Bsp. Verbindung Nr. | **% Wirkungsgrad** |
|---|---|---|---|
| 1 | **96** | **85** | **75** |
| 7 | **73** | **86** | **81** |
| 19 | **83** | **136** | **71** |
| 21 | **73** | **140** | **71** |
| 24 | **92** | **150** | **86** |

### Beispiel F: In vivo Test an Puccinia recondita (Braunrost des Weizen):

Eine wässrige Suspension des aktiven Wirkstoffes wurde durch Homogenisierung einer Mischung aus Aceton/Tween/Dimethylsulfoxid und anschließender Verdünnung mit Wasser auf die gewünschte Konzentration hergestellt. Weizenpflanzen (Sorte Scipion) werden in Aufzuchtschalen auf ein Torferde-Puzzolanerde-Substrat (50/50) bei 12°C ausgesät und im Erstblattstadium (10 cm groß) mit der oben beschriebenen wäßrigen Suspension besprüht. Als Kontrolle werden Pflanzen mit einer wäßrigen Lösung ohne Wirkstoff besprüht. Nach 24 Stunden werden die Pflanzen durch Besprühen mit einer wäßrigen Suspension von Puccinia recondita Sporen (100 000 Sporen pro ml) inokuliert. Die Sporen stammen von einer 10 Tage alten infizierten Weizenkultur und werden in Wasser mit 2.5 ml/l Tween suspendiert. Die inokulierten Weizenpflanzen werden erst für 24 Stunden bei 20°C und 100% relativer Luftfeuchtigkeit und anschließend für 10 Tage bei 20°C und 70% relativer Luftfeuchtigkeit inkubiert. Nach 10 Tagen wird im Vergleich mit den Kontrollpflanzen bonitiert. Unter diesen Bedingungen wird bei einer Dosis von 500 ppm eine gute (70% Wirkungsgrade) oder vollständige Inhibition für folgende Verbindungen beobachtet:

| Bsp. Verbindung Nr. | **% Wirkungsgrad** | Bsp. Verbindung Nr. | **% Wirkungsgrad** | Bsp. Verbindung Nr. | **% Wirkungsgrad** |
|---|---|---|---|---|---|
| 1 | **89** | 84 | **78** | 126 | **75** |
| 40 | **86** | 91 | **94** | 171 | **94** |
| 44 | **71** | 93 | **83** | | |

### Beispiel G: In vivo Test an Mycosphaerella graminicola (Weizen-Blattfleckenkrankheit):

Eine wässrige Suspension des aktiven Wirkstoffes wurde durch Homogenisierung einer Mischung aus Aceton/Tween/Dimethylsulfoxid und anschließender Verdünnung mit Wasser auf die gewünschte Konzentration hergestellt. Weizenpflanzen (Sorte Scipion) werden in Aufzuchtschalen auf ein Torferde-Puzzolanerde-Substrat (50/50) bei 12°C ausgesät und im Erstblattstadium (10 cm groß) mit der oben beschriebenen wäßrigen Suspension besprüht. Als Kontrolle werden Pflanzen mit einer wäßrigen Lösung ohne Wirkstoff besprüht. Nach 24 Stunden werden die Pflanzen durch Besprühen mit einer wäßrigen Suspension von Mycosphaerella graminicola Sporen (500 000 Sporen pro ml) inokuliert. Die Sporen stammen von einer 7 Tage alten Kultur. Die inokulierten Weizenpflanzen werden erst für 72 Stunden bei 18°C und 100% relativer Luftfeuchtigkeit und anschließend für 21 bis 28 Tage bei 90% relativer Luftfeuchtigkeit inkubiert. Nach 21 bis 28 Tagen wird im Vergleich mit den Kontrollpflanzen bonitiert. Unter diesen Bedingungen wird bei einer Dosis von 500 ppm eine gute (70% Wirkungsgrade) oder vollständige Inhibition für folgende Verbindungen beobachtet:

| Bsp. Verbindung Nr. | **% Wirkungsgrad** | Bsp. Verbindung Nr. | **% Wirkungsgrad** | Bsp. Verbindung Nr. | **% Wirkungsgrad** |
|---|---|---|---|---|---|
| 2 | **98** | 16 | **97** | 81 | **97** |
| 3 | **95** | 17 | **88** | 84 | **98** |
| 6 | **85** | 26 | **88** | 86 | **94** |
| 8 | **92** | 27 | **97** | 92 | **75** |
| 10 | **95** | 40 | **81** | 103 | **75** |
| 12 | **77** | 44 | **81** | 122 | **88** |
| 13 | **95** | 47 | **77** | 132 | **86** |
| 14 | **88** | 53 | **91** | 171 | **93** |
| 15 | **97** | 72 | **83** | | |

### Beispiel H: In vivo Test an Pyricularia grisea (Fleckenkrankheit an Reis):

Eine wässrige Suspension des aktiven Wirkstoffes wurde durch Homogenisierung einer Mischung aus Aceton/Tween/Dimethylsulfoxid und anschließender Verdünnung mit Wasser auf die gewünschte Konzentration hergestellt. Reispflanzen (Sorte Koshihikari) werden in Aufzuchtschalen auf ein Torferde-Puzzolanerde-Substrat (50/50) bei 25°C ausgesät und im Zweitblattstadium (13 bis 15 cm groß) mit der oben beschriebenen wäßrigen Suspension besprüht. Als Kontrolle werden Pflanzen mit einer wäßrigen Aceton/Tween/DMSO Lösung ohne Wirkstoff besprüht. Nach 24 Stunden werden die Pflanzen durch Besprühen mit einer wäßrigen Suspension von Pyricularia grisea Sporen (30 000 Sporen pro ml) inokuliert. Die Sporen stammen von einer 17 Tage alten Kultur und werden in Wasser, welches 2.5g/l Gelatine enthält, suspendiert. Die inokulierten Reispflanzen werden erst für 3 Tage bei ca. 25°C und 100% relativer Luftfeuchtigkeit und anschließend 3 Tage bei 25°C und 80% relativer Luftfeuchtigkeit tagsüber und 20% relativer Luftfeuchtigkeit bei Nacht inkubiert. Nach 6 Tagen wird im Vergleich mit den Kontrollpflanzen bonitiert. Unter diesen Bedingungen wird bei einer Dosis von 500 ppm eine gute (70% Wirkungsgrade) oder vollständige Inhibition für folgende Verbindungen beobachtet:

| Bsp. Verbindung Nr. | **% Wirkungsgrad** |
|---|---|
| 1 | **82** |
| 91 | **75** |
| 148 | **71** |

### Beispiel I: Produktion von Fumonisin FB1 durch Fusarium proliferatum Die Verbindungen wurden in Mikrotiter-Platten in einem Fumonisin induzierenden Flüssig-Medium (0.5g Malzextrakt, 1g Hefeextrakt, 1g Bactopeptone, 20 g Fruktose, 1g KH₂PO₄, 0.3g MgSO₄x7H₂O, 0.3g KCl, 0.05g ZnSO₄x7H₂O und 0.01g CuSO₄x5H₂O pro Liter) mit DMSO (0,5 %) getestet. Die Inokulation erfolgte mit einer konzentrierten Sporen-Suspension von Fusarium proliferatum bei einer Endkonzentration von 2000 Sporen/ml.

### Die Platte wurde bei hoher Luftfeuchtigkeit 5 Tage lang bei 20 °C inkubiert.

Zu Beginn und nach 5 Tagen wurde eine OD-Messung bei OD620 (mehrfache Messung: 3 x 3 Messungen pro Loch) zur Berechnung der Wachstumshemmung vorgenommen.
Nach 5 Tagen wurde eine Probe des flüssigen Mediums entnommen und 1:1000 in 50 %igem Acetonitril verdünnt. Die Konzentration von FB1 der verdünnten Proben wurden mittels HPLC-MS/MS analysiert und die Meßwerte zur Berechnung der Hemmung der Fumonisin FB1 Produktion im Vergleich zu einer wirkstofffreien Kontrolle genutzt.

HPLC-MS/MS wurde mit den folgenden Parametern durchgeführt:
Ionisierungs-Art: ESI positive
Ionen-Spray Spannung: 5500V
Spraygas-Temperatur: 500°C
Dekluster-Potential: 114 V
Kollisions-Energie: 51eV
Kollisions-Gas: N₂
NMR Spur: 722,3 > 352,3; dwell time 100ms

HPLC Säule: Waters Atlantis T3 (trifunktionelle C18 Bindung, verschlossen)
Partikelgröße: 3µm
Säulenmaße: 50 x 2 mm
Temperatur: 40°C
Lösungsmittel A: Wasser+0.1% HCOOH (v/v)
Lösungsmittel B: Acetonitril+0.1% HCOOH (v/v)
Durchfluß: 400 µL/Minute
Injektionsvolumen: 5 µL
Gradient:

| **Time [min]** | **A%** | **B%** |
|---|---|---|
| 0 | 90 | 10 |
| 2 | 5 | 95 |
| 4 | 5 | 95 |
| 4.1 | 90 | 10 |
| 9 | 90 | 10 |

### Beispiele für die Hemmung von Fumonisin FB1 Produktion

Die unten aufgeführten Beispiele zeigten eine > 80 %ige Hemmung der Fumonisin FB1 Produktion bei einer Konzentration von 50 µM. Die Hemmung des Wachst
ums von *Fusarium proliferatum* der genannten Beispiele variierte von 0 bis 99 % bei 50 µM.

| **Bsp. Verbindung Nr.** | **% Inhibition der FB1 produktion bei 50 µM** | **% Inhibition des Pilzwachstums bei 50 µM** |
|---|---|---|
| 19 | 100 | 68 |
| 20 | 100 | 65 |
| 21 | 100 | 55 |
| 23 | 100 | 73 |
| 29 | 82 | 34 |
| 33 | 99 | 99 |
| 40 | 100 | 98 |
| 41 | 100 | 56 |
| 44 | 100 | 33 |
| 47 | 98 | 0 |
| 50 | 89 | 8 |
| 53 | 96 | 0 |
| 54 | 100 | 47 |
| 58 | 89 | 42 |
| 59 | 95 | 0 |
| 60 | 100 | 92 |
| 66 | 85 | 40 |
| 70 | 92 | 0 |
| 84 | 100 | 88 |
| 85 | 100 | 95 |
| 86 | 100 | 96 |
| 87 | 100 | 83 |
| 146 | 100 | 41 |
| 153 | 100 | 48 |
| 155 | 100 | 97 |
| 156 | 100 | 93 |
| 163 | 100 | 72 |
| 164 | 100 | 97 |
| 165 | 100 | 90 |
| 166 | 100 | 98 |
| 189 | 90 | 0 |
| 191 | 96 | 1 |
| 193 | 85 | 0 |
| 195 | 100 | 17 |
| 196 | 100 | 25 |
| 198 | 100 | 99 |
| 200 | 99 | 1 |
| 203 | 100 | 99 |
| 213 | 100 | 56 |
| 214 | 100 | 96 |
| 215 | 100 | 94 |
| 216 | 100 | 97 |
| 217 | 100 | 97 |
| 218 | 100 | 79 |
| 219 | 100 | 97 |
| 220 | 100 | 99 |
| 221 | 100 | 99 |
| 222 | 100 | 96 |
| 223 | 100 | 96 |
| 224 | 100 | 99 |
| 225 | 100 | 93 |
| 5 | 100 | 79 |
| 9 | 96 | 40 |

### Beispiel J: Produktion von DON/Acetyl-DON durch Fusarium graminearum

Die Verbindungen wurden in Mikrotiter-Platten in einem DON induzierenden Flüssig-Medium (1g (NH₄)₂HPO₄, 0.2g MgSO₄x7H₂O, 3g KH₂PO₄, 10g Glycerin, 5g NaCl and 40g Saccharose pro Liter) und DMSO (0,5 %) getestet. Die Inokulation erfolgte mit einer konzentrierten Sporen-Suspension von *Fusarium graminearum* bei einer Endkonzentration von 2000 Sporen/ml.
Die Platte wurde bei hoher Luftfeuchtigkeit 7 Tage lang bei 28 °C inkubiert.

Zu Beginn und nach 3 Tagen wurde eine OD-Messung bei OD620 (mehrfache Messung: 3 x 3 Messungen pro Loch) zur Berechnung der Wachstumshemmung vorgenommen.
Nach 7 Tagen wurden 1 Volumen einer 84/16 Acetonitril/Wasser -Mischung hinzugefügt und aus jedem Loch wurde anschließend eine Probe des flüssigen Mediums entnommen und 1:100 in 10 %igem Acetonitril verdünnt. Die Anteile von DON und Acetyl-DON der Proben wurden mittels HPLC-MS/MS analysiert und die Meßwerte wurden zur Berechnung der Hemmung der DON/AcDON Produktion im Vergleich zu einer wirkstofffreien Kontrolle genutzt.

Die HPLC-MS/MS-Messungen wurden mit folgenden Parametern durchgeführt:
Ionisierungs-Art: ESI negativ
Ionen-Spray Spannung: - 4500 V
Spraygas-Temperatur: 500°C
Dekluster-Potential: - 40 V
Kollisions-Energie: -22eV
Kollisions-Gas: N₂
NMR Spur: 355,0 >264,9;

HPLC Säule: Waters Atlantis T3 (trifunktionelle C18 Bindung, verschlossen)
Partikelgröße: 3µm
Säulenmaße: 50 x 2 mm
Temperatur: 40°C
Lösungsmittel A: Wasser/2.5mM NH₄OAc+0.05% CH₃COOH (v/v)
Lösungsmittel B: Methanol/2.5mM NH₄0Ac+0.05% CH₃COOH (v/v)
Durchfluß: 400 µL/Minute
Injektionsvolumen: 11 µL
Gradient:

| **Time [min]** | **A%** | **B%** |
|---|---|---|
| 0 | 100 | 0 |
| 0.75 | 100 | 0 |
| 1.5 | 5 | 95 |
| 4 | 5 | 95 |
| 5 | 100 | 0 |
| 10 | 100 | 0 |

### Beispiele für DON-Hemmung

Die unten aufgeführten Beispiele zeigten eine >=80%ige Hemmung der DON/AcDON Produktion bei 50 µM. Die Hemmung des Wachstums von *Fusarium graminearum* der genannten Beispiele variierte von 0 bis 100% bei 50 µM.

| **Bsp. Verbindung Nr.** | **% Inhibition der DON/AcDON produktion bei 50 µM** | **% Inhibition des Pilzwachstums bei 50 µM** |
|---|---|---|
| 5 | 100 | 97 |
| 20 | 87 | 21 |
| 37 | 80 | 13 |
| 40 | 100 | 100 |
| 54 | 85 | 31 |
| 84 | 99 | 92 |
| 85 | 99 | 100 |
| 86 | 98 | 94 |
| 87 | 100 | 97 |
| 142 | 89 | 0 |
| 146 | 100 | 97 |
| 150 | 99 | 97 |
| 153 | 100 | 56 |
| 154 | 93 | 0 |
| 155 | 100 | 97 |
| 156 | 81 | 99 |
| 157 | 100 | 100 |
| 159 | 99 | 102 |
| 160 | 100 | 100 |
| 161 | 100 | 104 |
| 162 | 99 | 99 |
| 163 | 100 | 100 |
| 164 | 99 | 100 |
| 165 | 100 | 64 |
| 166 | 100 | 84 |
| 167 | 99 | 100 |
| 169 | 100 | 101 |
| 183 | 99 | 65 |
| 195 | 80 | 0 |
| 198 | 100 | 100 |
| 203 | 100 | 100 |
| 206 | 99 | 75 |
| 207 | 100 | 75 |
| 208 | 100 | 101 |
| 210 | 100 | 85 |
| 211 | 99 | 16 |
| 213 | 100 | 0 |
| 214 | 93 | 100 |
| 219 | 100 | 97 |
| 220 | 99 | 100 |
| 221 | 100 | 100 |
| 222 | 82 | 99 |
| 223 | 97 | 99 |
| 224 | 92 | 100 |
| 225 | 89 | 92 |
| 226 | 100 | 85 |
| 227 | 100 | 100 |
| 229 | 97 | 0 |
| 230 | 97 | 0 |
| 232 | 80 | 0 |
| 233 | 100 | 100 |
| 245 | 99 | 95 |
| 246 | 99 | 88 |
| 247 | 99 | 96 |
| 252 | 99 | 0 |
| 254 | 99 | 16 |
| 255 | 96 | 0 |
| 257 | 94 | 0 |
| 258 | 98 | 79 |
| 260 | 99 | 5 |
| 261 | 99 | 39 |
| 262 | 99 | 35 |
| 264 | 99 | 17 |
| 265 | 98 | 9 |
| 267 | 97 | 59 |
| 268 | 90 | 32 |
| 271 | 99 | 71 |
| 272 | 99 | 33 |
| 326 | 100 | 107 |
| 327 | 100 | 105 |
| 328 | 100 | 101 |
| 329 | 100 | 104 |

### Beispiel K: Produktion von Aflatoxinen durch Aspergillus parasiticus

Die Verbindungen wurden in Mikrotiter-Platten (schwarze 96-Loch Platten mit flachem und transparentem Boden) in einem Aflatoxin induzierenden Flüssigmedium (20g Saccharose, 4g Hefeextrakt, 1g KH₂PO₄ und 0.5g MgSO₄x7H₂O pro Liter) versetzt mit 20mM Cavasol (Hydroxypropyl-beta-cyclodextrin) und 1 % DMSO getestet. Die Inokulation erfolgte mit einer konzentrierten Sporensuspension von *Aspergillus parasiticus* bei einer Endkonzentration von 1000 Sporen/ml.

### Die Platte wurde bei hoher Luftfeuchtigkeit 7 Tage bei 20 °C inkubiert.

Nach 7 Tagen wurde eine OD-Messung bei OD620 (mehrfache Messung: 4 x 4 Messungen pro Loch) zur Berechnung der Wachstumshemmung vorgenommen. Zur gleichen Zeit wurde über den Boden der Platte eine Fluoreszenzmessung mit Em₃₆₀nm und Ex₄₂₆ₙₘ (mehrfache Messung: 3 x 3 Messungen pro Loch) zur Berechnung der Hemmung der Aflatoxin Produktion im Vergleich zu einer wirkstofffreien Kontrolle vorgenommen.

### Beispiele für Hemmung der Aflatoxin Produktion

Die unten aufgeführten Beispiele zeigten eine >80%ige Hemmung der Aflatoxin Produktion bei 50 µM. Die Wachstumshemmung von *Aspergillus parasiticus* bei 50 µM bei diesen Beispielen lag ebenfalls bei > 80%

| **Bsp Verbindung Nr.** | **% Inhibition der Aflatoxin produktion bei 50 µM** | **% Inhibition des Pilzwachstums bei 50 µM** |
|---|---|---|
| 40 | 99 | 88 |
| 84 | 100 | 98 |
| 85 | 100 | 98 |
| 86 | 100 | 97 |
| 87 | 100 | 97 |
| 146 | 100 | 99 |
| 155 | 100 | 100 |
| 203 | 100 | 100 |

### Beispiel L: In vivo Test an Sphaerotheca fuliginea (echter Mehltau an Gurke / protektiv):

| | |
|---|---|
| Lösungsmittel: | 49 Gewichtsteile N, N - Dimethylformamid |
| Emulgator: | 1 Gewichtsteil Alkylarylpolyglykolether |

Zur Herstellung einer zweckmäßigen Wirkstoffzubereitung vermischt man 1 Gewichtsteil Wirkstoff mit den angegebenen Mengen Lösungsmittel und Emulgator und verdünnt das Konzentrat mit Wasser auf die gewünschte Konzentration.

Zur Prüfung auf protektive Wirksamkeit bespritzt man junge Gurkenpflanzen mit der Wirkstoffzubereitung in der angegebenen Aufwandmenge. 1 Tag nach der Behandlung werden die Pflanzen mit einer Sporensuspension von ***Sphaerotheca fuliginea*** inokuliert. Anschließend werden die Pflanzen in einem Gewächshaus bei 70 % relativer Luftfeuchtigkeit und einer Temperatur von 23°C aufgestellt.

7 Tage nach der Inokulation erfolgt die Auswertung. Dabei bedeutet 0 % ein Wirkungsgrad, der demjenigen der Kontrolle entspricht, während ein Wirkungsgrad von 100 % bedeutet, daß kein Befall beobachtet wird.

In diesem Test zeigen die erfindungsgemäßen Verbindungen der nachfolgenden Formeln bei einer Konzentration an Wirkstoff von 500ppm einen Wirkungsgrad von 70 % oder mehr.

### Tabelle

### Sphaerotheca-Test (Gurke) / protektiv

| Bsp. Verbindung Nr. | **% Wirkungsgrad** | Bsp. Verbindung Nr. | **% Wirkungsgrad** | Bsp. Verbindung Nr. | **% Wirkungsgrad** |
|---|---|---|---|---|---|
| 5 | **70** | 217 | **100** | 299 | **84** |
| 9 | **90** | 218 | **100** | 298 | **100** |
| 31 | **70** | 219 | **100** | 297 | **90** |
| 29 | **70** | 220 | **97** | 296 | **84** |
| 15 | **95** | 221 | **100** | 295 | **94** |
| 11 | **73** | 222 | **100** | 294 | **98** |
| 85 | **100** | 223 | **91** | 293 | **94** |
| 86 | **100** | 224 | **99** | 292 | **95** |
| 84 | **100** | 225 | **100** | 291 | **95** |
| 89 | **88** | 165 | **86** | 290 | **100** |
| 136 | **73** | 226 | **95** | 371 | **94** |
| 91 | **100** | 228 | **75** | 369 | **88** |
| 126 | **95** | 229 | **94** | 368 | **94** |
| 150 | **93** | 232 | **93** | 367 | **84** |
| 122 | **95** | 233 | **95** | 366 | **94** |
| 92 | **94** | 236 | **86** | 307 | **100** |
| 116 | **93** | 237 | **88** | 306 | **94** |
| 104 | **70** | 238 | **100** | 301 | **95** |
| 103 | **100** | 239 | **95** | 300 | **90** |
| 146 | **95** | 240 | **96** | 309 | **100** |
| 87 | **100** | 242 | **97** | 303 | **95** |
| 171 | **100** | 243 | **95** | 312 | **100** |
| 174 | **100** | 245 | **88** | 313 | **98** |
| 169 | **89** | 162 | **99** | 314 | **100** |
| 178 | **75** | 249 | **92** | 315 | **95** |
| 179 | **74** | 250 | **84** | 316 | **75** |
| 156 | **94** | 251 | **96** | 320 | **94** |
| 181 | **85** | 253 | **92** | 320 | **89** |
| 183 | **94** | 158 | **97** | 320 | **95** |
| 184 | **95** | 159 | **97** | 322 | **95** |
| 192 | **85** | 254 | **92** | 323 | **86** |
| 198 | **99** | 256 | **92** | 326 | **94** |
| 155 | **95** | 157 | **100** | 328 | **100** |
| 203 | **95** | 258 | **80** | 329 | **94** |
| 166 | **97** | 260 | **95** | 331 | **94** |
| 163 | **93** | 167 | **100** | 335 | **74** |
| 161 | **100** | 267 | **98** | 337 | **88** |
| 160 | **100** | 268 | **100** | 338 | **91** |
| 205 | **100** | 271 | **79** | 339 | **95** |
| 206 | **94** | 272 | **94** | 340 | **95** |
| 207 | **100** | 286 | **89** | 341 | **100** |
| 208 | **100** | 285 | **89** | 342 | **94** |
| 209 | **100** | 284 | **91** | 346 | **95** |
| 210 | **100** | 283 | **78** | 347 | **95** |
| 211 | **93** | 281 | **94** | 348 | **95** |
| 212 | **96** | 280 | **93** | 349 | **95** |
| 213 | **74** | 279 | **100** | 350 | **94** |
| 164 | **100** | 278 | **89** | 351 | **80** |
| 214 | **100** | 277 | **94** | 352 | **73** |
| 215 | **100** | 276 | **99** | | |
| 216 | **89** | 274 | **94** | | |

### Beispiel M: In vivo Test an Alternaria solani (Blattfleckenkrankheit, Tomate / protektiv):

| | |
|---|---|
| Lösungsmittel: | 49 Gewichtsteile N, N - Dimethylformamid |
| Emulgator: | 1 Gewichtsteil Alkylarylpolyglykolether |

Zur Herstellung einer zweckmäßigen Wirkstoffzubereitung vermischt man 1 Gewichtsteil Wirkstoff mit den angegebenen Mengen Lösungsmittel und Emulgator und verdünnt das Konzentrat mit Wasser auf die gewünschte Konzentration.

Zur Prüfung auf protektive Wirksamkeit bespritzt man junge Tomatenpflanzen mit der Wirkstoffzubereitung in der angegebenen Aufwandmenge. 1 Tag nach der Behandlung werden die Pflanzen mit einer Sporensuspension von ***Alternaria solani*** inokuliert und stehen dann 24h bei 100% rel. Feuchte und 22°C. Anschließend stehen die Pflanzen bei 96% rel. Luftfeuchtigkeit und einer Temperatur von 20°C.

7 Tage nach der Inokulation erfolgt die Auswertung. Dabei bedeutet 0 % ein Wirkungsgrad, der demjenigen der Kontrolle entspricht, während ein Wirkungsgrad von 100 % bedeutet, dass kein Befall beobachtet wird.

In diesem Test zeigen die erfindungsgemäßen Verbindungen der nachfolgenden Formeln bei einer Konzentration an Wirkstoff von 500ppm einen Wirkungsgrad von 70% oder mehr.

### Beispiel

### Alternaria-Test (Tomate) / protektiv

| Bsp. Verbindung Nr. | **% Wirkungsgrad** | Bsp. Verbindung Nr. | **% Wirkungsgrad** | Bsp. Verbindung Nr. | **% Wirkungsgrad** |
|---|---|---|---|---|---|
| 30 | **90** | 164 | **95** | 298 | **80** |
| 25 | **90** | 214 | **95** | 297 | **100** |
| 5 | **90** | 215 | **94** | 296 | **100** |
| 9 | **89** | 216 | **95** | 295 | **100** |
| 81 | **95** | 218 | **95** | 294 | **100** |
| 29 | **78** | 220 | **95** | 293 | **100** |
| 2 | **100** | 222 | **90** | 292 | **100** |
| 15 | **95** | 223 | **90** | 291 | **100** |
| 11 | **80** | 224 | **70** | 290 | **100** |
| 72 | **95** | 225 | **95** | 371 | **95** |
| 85 | **100** | 165 | **95** | 370 | **95** |
| 84 | **95** | 226 | **95** | 369 | **90** |
| 89 | **89** | 227 | **95** | 310 | **95** |
| 136 | **95** | 228 | **70** | 368 | **95** |
| 135 | **78** | 229 | **95** | 367 | **95** |
| 134 | **100** | 231 | **95** | 366 | **80** |
| 91 | **95** | 232 | **95** | 365 | **80** |
| 126 | **95** | 233 | **90** | 307 | **100** |
| 150 | **95** | 235 | **94** | 306 | **95** |
| 122 | **95** | 236 | **94** | 305 | **100** |
| 92 | **90** | 237 | **100** | 302 | **95** |
| 116 | **95** | 238 | **94** | 301 | **95** |
| 113 | **89** | 239 | **100** | 300 | **80** |
| 108 | **80** | 240 | **88** | 309 | **100** |
| 106 | **95** | 241 | **94** | 364 | **95** |
| 104 | **90** | 242 | **88** | 363 | **90** |
| 103 | **90** | 243 | **100** | 303 | **100** |
| 102 | **95** | 244 | **94** | 362 | **80** |
| 146 | **100** | 245 | **94** | 361 | **70** |
| 144 | **94** | 247 | **94** | 312 | **100** |
| 142 | **94** | 162 | **94** | 313 | **95** |
| 170 | **78** | 248 | **94** | 314 | **100** |
| 87 | **95** | 249 | **94** | 315 | **95** |
| 153 | **90** | 250 | **94** | 316 | **100** |
| 171 | **95** | 251 | **94** | 317 | **70** |
| 173 | **94** | 253 | **94** | 318 | **80** |
| 174 | **100** | 158 | **94** | 319 | **90** |
| 176 | **100** | 159 | **94** | 320 | **95** |
| 178 | **94** | 254 | **94** | 321 | **70** |
| 179 | **100** | 256 | **94** | 322 | **95** |
| 156 | **94** | 157 | **94** | 323 | **95** |
| 181 | **94** | 257 | **94** | 326 | **95** |
| 183 | **100** | 258 | **95** | 327 | **95** |
| 184 | **95** | 259 | **90** | 328 | **95** |
| 189 | **95** | 260 | **90** | 329 | **95** |
| 192 | **100** | 262 | **95** | 332 | **90** |
| 193 | **93** | 267 | **95** | 337 | **95** |
| 195 | **80** | 268 | **80** | 338 | **80** |
| 196 | **100** | 269 | **89** | 339 | **95** |
| 198 | **93** | 271 | **89** | 340 | **95** |
| 155 | **100** | 272 | **94** | 341 | **95** |
| 200 | **93** | 273 | **90** | 342 | **95** |
| 203 | **93** | 286 | **90** | 347 | **95** |
| 166 | **95** | 285 | **95** | 348 | **95** |
| 163 | **93** | 284 | **100** | 349 | **80** |
| 161 | **95** | 283 | **95** | 350 | **95** |
| 160 | **95** | 282 | **70** | 351 | **95** |
| 205 | **95** | 281 | **95** | 352 | **95** |
| 206 | **90** | 280 | **95** | 353 | **80** |
| 207 | **90** | 279 | **70** | 164 | **95** |
| 208 | **80** | 278 | **95** | 214 | **95** |
| 209 | **95** | 277 | **100** | 215 | **94** |
| 210 | **80** | 276 | **95** | 216 | **95** |
| 211 | **70** | 275 | **90** | 218 | **95** |
| 212 | **95** | 274 | **95** | 220 | **95** |
| 213 | **90** | 299 | **100** | 298 | **80** |
| 297 | **100** | 296 | **100** | 295 | **100** |
| 294 | **100** | 293 | **100** | | |

### Beispiel N: In vivo Test an Plasmopara viticola (falscher Mehltau, Rebe / protektiv):

| | | |
|---|---|---|
| Lösungsmittel: | 24,5 | Gewichtsteile Aceton |
| | 24,5 | Gewichtsteile Dimethylacetamid |
| Emulgator: | 1 | Gewichtsteil Alkyl-Aryl-Polyglykolether |

Zur Herstellung einer zweckmäßigen Wirkstoffzubereitung vermischt man 1 Gewichtsteil Wirkstoff mit den angegebenen Mengen Lösungsmittel und Emulgator und verdünnt das Konzentrat mit Wasser auf die gewünschte Konzentration.

Zur Prüfung auf protektive Wirksamkeit werden junge Pflanzen mit der Wirkstoffzubereitung in der angegebenen Aufwandmenge besprüht. Nach Antrocknen des Spritzbelages werden die Pflanzen mit einer wäßrigen Sporensuspension von ***Plasmopara viticola*** inokuliert und verbleiben dann 1 Tag in einer Inkubationskabine bei ca. 20°C und 100% relativer Luftfeuchtigkeit. Anschließend werden die Pflanzen 4 Tage im Gewächshaus bei ca. 21°C und ca. 90% Luftfeuchtigkeit aufgestellt. Die Pflanzen werden dann angefeuchtet und 1 Tag in eine Inkubationskabine gestellt.

6 Tage nach der Inokulation erfolgt die Auswertung. Dabei bedeutet 0% ein Wirkungsgrad, der demjenigen der Kontrolle entspricht, während ein Wirkungsgrad von 100% bedeutet, daß kein Befall beobachtet wird.

In diesem Test zeigen die nachfolgenden erfindungsgemäßen Verbindungen bei einer Konzentration an Wirkstoff von 100ppm einen Wirkungsgrad von 70% oder mehr.

| Bsp. Verbindung Nr. | **% Wirkungsgrad** | Bsp. Verbindung Nr. | **% Wirkungsgrad** | Bsp. Verbindung Nr. | **% Wirkungsgrad** |
|---|---|---|---|---|---|
| 1 | **100** | 169 | **96** | 219 | **95** |
| 40 | **72** | 183 | **86** | 225 | **90** |
| 85 | **94** | 192 | **94** | 236 | **76** |
| 86 | **84** | 198 | **92** | 237 | **84** |
| 84 | **98** | 161 | **95** | 238 | **88** |
| 89 | **73** | 160 | **79** | 239 | **88** |
| 126 | **81** | 207 | **71** | 240 | **90** |
| 92 | **73** | 212 | **83** | 260 | **70** |
| 174 | **70** | 214 | **88** | | |

### Beispiel O: In vivo Test an Venturia inaequalis (Apfelschorf / protektiv):

| | | |
|---|---|---|
| Lösungsmittel: | 24,5 | Gewichtsteile Aceton |
| | 24,5 | Gewichtsteile Dimethylacetamid |
| Emulgator: | 1 | Gewichtsteil Alkyl-Aryl-Polyglykolether |

Zur Herstellung einer zweckmäßigen Wirkstoffzubereitung vermischt man 1 Gewichtsteil Wirkstoff mit den angegebenen Mengen Lösungsmittel und Emulgator und verdünnt das Konzentrat mit Wasser auf die gewünschte Konzentration.

Zur Prüfung auf protektive Wirksamkeit werden junge Pflanzen mit der Wirkstoffzubereitung in der angegebenen Aufwandmenge besprüht. Nach Antrocknen des Spritzbelages werden die Pflanzen mit einer wäßrigen Konidiensuspension des Apfelschorferregers ***Venturia inaequalis*** inokuliert und verbleiben dann 1 Tag bei ca. 20°C und 100% relativer Luftfeuchtigkeit in einer Inkubations-kabine.

Die Pflanzen werden dann im Gewächshaus bei ca. 21°C und einer relativen Luftfeuchtigkeit von ca. 90% aufgestellt.

10 Tage nach der Inokulation erfolgt die Auswertung. Dabei bedeutet 0% ein Wirkungsgrad, der demjenigen der Kontrolle entspricht, während ein Wirkungsgrad von 100% bedeutet, daß kein Befall beobachtet wird.

In diesem Test zeigen die nachfolgenden erfindungsgemäßen Verbindungen bei einer Konzentration an Wirkstoff von 100ppm einen Wirkungsgrad von 70% oder mehr.

| Bsp. Verbindung Nr. | **% Wirkungsgrad** | Bsp. Verbindung Nr. | **% Wirkungsgrad** | Bsp. Verbindung Nr. | **% Wirkungsgrad** |
|---|---|---|---|---|---|
| 1 | **99** | 160 | **94** | 236 | **94** |
| 40 | **97** | 205 | **91** | 237 | **95** |
| 85 | **88** | 206 | **83** | 238 | **98** |
| 86 | **96** | 207 | **86** | 239 | **98** |
| 84 | **100** | 208 | **78** | 240 | **96** |
| 89 | **100** | 209 | **89** | 242 | **89** |
| 126 | **98** | 210 | **78** | 243 | **98** |
| 92 | **93** | 211 | **92** | 162 | **96** |
| 87 | **95** | 212 | **85** | 249 | **96** |
| 174 | **100** | 164 | **97** | 251 | **84** |
| 169 | **99** | 214 | **88** | 159 | **97** |
| 179 | **100** | 215 | **96** | 254 | **73** |
| 180 | **96** | 217 | **98** | 157 | **94** |
| 156 | **91** | 218 | **95** | 260 | **94** |
| 183 | **73** | 219 | **96** | 167 | **89** |
| 184 | **92** | 220 | **96** | 268 | **98** |
| 189 | **84** | 221 | **91** | 271 | **81** |
| 192 | **95** | 222 | **95** | 272 | **89** |
| 198 | **96** | 224 | **71** | | |
| 161 | **95** | 225 | **96** | | |

### Beispiel P: In vivo Test an Botrytis cinerea (Grauschimmel an Bohne / protektiv):

| | | |
|---|---|---|
| Lösungsmittel: | 24,5 | Gewichtsteile Aceton |
| | 24,5 | Gewichtsteile Dimethylacetamid |
| Emulgator: | 1 | Gewichtsteil Alkyl-Aryl-Polyglykolether |

Zur Herstellung einer zweckmäßigen Wirkstoffzubereitung vermischt man 1 Gewichtsteil Wirkstoff mit den angegebenen Mengen Lösungsmittel und Emulgator und verdünnt das Konzentrat mit Wasser auf die gewünschte Konzentration.

Zur Prüfung auf protektive Wirksamkeit werden junge Pflanzen mit der Wirkstoffzubereitung in der angegebenen Aufwandmenge besprüht. Nach Antrocknen des Spritzbelages werden auf jedes Blatt 2 kleine mit ***Botrytis cinerea*** bewachsene Agarstückchen aufgelegt. Die inokulierten Pflanzen werden in einer abgedunkelten Kammer bei ca. 20°C und 100% relativer Luftfeuchtigkeit aufgestellt.

2 Tage nach der Inokulation wird die Größe der Befallsflecken auf den Blättern ausgewertet. Dabei bedeutet 0% ein Wirkungsgrad, der demjenigen der Kontrolle entspricht, während ein Wirkungsgrad von 100% bedeutet, daß kein Befall beobachtet wird.

In diesem Test zeigen die nachfolgenden erfindungsgemäßen Verbindungen bei einer Konzentration an Wirkstoff von 250ppm einen Wirkungsgrad von 70% oder mehr.

| Bsp. Verbindung Nr. | **% Wirkungsgrad** | Bsp. Verbindung Nr. | **% Wirkungsgrad** | Bsp. Verbindung Nr. | **% Wirkungsgrad** |
|---|---|---|---|---|---|
| 1 | **99** | 155 | **99** | 224 | **99** |
| 40 | **100** | 161 | **100** | 225 | **96** |
| 85 | **100** | 160 | **100** | 225 | **93** |
| 86 | **100** | 205 | **100** | 236 | **100** |
| 84 | **99** | 206 | **100** | 237 | **100** |
| 89 | **100** | 207 | **100** | 238 | **93** |
| 126 | **100** | 208 | **100** | 240 | **99** |
| 92 | **100** | 209 | **100** | 242 | **99** |
| 87 | **100** | 210 | **98** | 243 | **75** |
| 171 | **84** | 211 | **100** | 162 | **100** |
| 174 | **80** | 212 | **98** | 249 | **100** |
| 169 | **100** | 213 | **91** | 251 | **95** |
| 179 | **100** | 214 | **100** | 159 | **98** |
| 180 | **93** | 215 | **98** | 157 | **78** |
| 156 | **98** | 217 | **95** | 260 | **100** |
| 183 | **93** | 218 | **98** | 167 | **100** |
| 184 | **100** | 219 | **100** | 268 | **100** |
| 189 | **93** | 220 | **100** | 271 | **93** |
| 192 | **100** | 221 | **100** | 272 | **100** |
| 198 | **99** | 222 | **85** | | |

### Beispiel Q: In vivo Test an Leptosphaeria nodorum (Spelzenbräune an Weizen / protektiv):

| | |
|---|---|
| Lösungsmittel: | *49* Gewichtsteile *N,N-Dimethylacetamid* |
| Emulgator: | 1 Gewichtsteil Alkylarylpolyglykolether |

Zur Herstellung einer zweckmäßigen Wirkstoffzubereitung vermischt man 1 Gewichtsteil Wirkstoff mit den angegebenen Mengen Lösungsmittel und Emulgator und verdünnt das Konzentrat mit Wasser auf die gewünschte Konzentration.

Zur Prüfung auf protektive Wirksamkeit besprüht man junge Pflanzen mit der Wirkstoffzubereitung in der angegebenen Aufwandmenge.

Nach Antrocknen des Spritzbelages werden die Pflanzen mit Sporen einer Sporensuspension von *Leptosphaeria nodorum* besprüht. Die Pflanzen verbleiben 48 Stunden bei 20°C und 100 % relativer Luftfeuchtigkeit in einer Inkubationskabine.

Die Pflanzen werden in einem Gewächshaus bei einer Temperatur von ca. 22°C und einer relativen Luftfeuchtigkeit von ca. 80 % aufgestellt.

8 Tage nach der Inokulation erfolgt die Auswertung. Dabei bedeutet 0 % ein Wirkungsgrad, der demjenigen der Kontrolle entspricht, während ein Wirkungsgrad von 100 % bedeutet, dass kein Befall beobachtet wird.

In diesem Test zeigen die erfindungsgemäßen nachfolgenden Verbindungen bei einer Konzentration an Wirkstoff von 1000 ppm einen Wirkungsgrad von 70% oder mehr.

| Bsp. Verbindung Nr. | **% Wirkungsgrad** | Bsp. Verbindung Nr. | **% Wirkungsgrad** | Bsp. Verbindung Nr. | **% Wirkungsgrad** |
|---|---|---|---|---|---|
| 1 | **100** | 174 | **93** | 164 | **93** |
| 85 | **100** | 169 | **100** | 214 | **71** |
| 86 | **100** | 184 | **88** | 215 | **71** |
| 84 | **100** | 192 | **86** | 218 | **93** |
| 89 | **83** | 198 | **100** | 225 | **86** |
| 87 | **88** | 212 | **71** | 167 | **92** |

### Beispiel R: In vivo Test an Septoria tritici (Blattfleckenkrankheit (Schwarzfleckigkeit) an Weizen / protektiv):

| | |
|---|---|
| Lösungsmittel: | *49* Gewichtsteile *N,N-Dimethylacetamid* |
| Emulgator: | 1 Gewichtsteil Alkylarylpolyglykolether |

Zur Herstellung einer zweckmäßigen Wirkstoffzubereitung vermischt man 1 Gewichtsteil Wirkstoff mit den angegebenen Mengen Lösungsmittel und Emulgator und verdünnt das Konzentrat mit Wasser auf die gewünschte Konzentration.

Zur Prüfung auf protektive Wirksamkeit werden junge Pflanzen mit der Wirkstoffzubereitung in der angegebenen Aufwandmenge besprüht. Nach Antrocknen des Spritzbelages werden die Pflanzen mit einer Sporensuspension von *Septoria tritici* besprüht. Die Pflanzen verbleiben 48 Stunden bei 20°C und 100 % relativer Luftfeuchtigkeit in einer Inkubationskabine. Danach werden die Pflanzen für weitere 60 Stunden unter eine Klarsichthaube bei 15°C und 100% relativer Luftfeuchte gestellt.

Die Pflanzen werden in einem Gewächshaus bei einer Temperatur von ca. 15°C und einer relativen Luftfeuchtigkeit von 80 % aufgestellt.

21 Tage nach der Inokulation erfolgt die Auswertung. Dabei bedeutet 0% ein Wirkungsgrad, der demjenigen der Kontrolle entspricht, während ein Wirkungsgrad von 100% bedeutet, dass kein Befall beobachtet wird.

In diesem Test zeigen die erfindungsgemäßen nachfolgenden Verbindungen bei einer Konzentration an Wirkstoff von 1000 ppm einen Wirkungsgrad von 70% oder mehr.

| Bsp. Verbindung Nr. | **% Wirkungsgrad** | Bsp. Verbindung Nr. | **% Wirkungsgrad** | Bsp. Verbindung Nr. | **% Wirkungsgrad** |
|---|---|---|---|---|---|
| 1 | **100** | 169 | **100** | 215 | **94** |
| 85 | **100** | 184 | **100** | 218 | **100** |
| 86 | **100** | 192 | **100** | 224 | **100** |
| 84 | **100** | 198 | **100** | 225 | **100** |
| 89 | **100** | 212 | **71** | 240 | **75** |
| 87 | **100** | 164 | **100** | 159 | **100** |
| 174 | **90** | 214 | **100** | 157 | **100** |

### Beispiel S: In vivo Test an Rhynchosporium secalis (Blattfleckenkrankheit an Gerste / protektiv):

| | |
|---|---|
| Lösungsmittel: | *49* Gewichtsteile *N,N-Dimethylacetamid* |
| Emulgator: | 1 Gewichtsteil Alkylarylpolyglykolether |

Zur Herstellung einer zweckmäßigen Wirkstoffzubereitung vermischt man 1 Gewichtsteil Wirkstoff mit den angegebenen Mengen Lösungsmittel und Emulgator und verdünnt das Konzentrat mit Wasser auf die gewünschte Konzentration.

Zur Prüfung auf protektive Wirksamkeit besprüht man junge Pflanzen mit der Wirkstoffzubereitung in der angegebenen Aufwandmenge.

Nach Antrocknen des Spritzbelages werden die Pflanzen mit Sporen mit einer Sporensuspension von *Rhynchosporium secalis* besprüht. Die Pflanzen verbleiben 48 Stunden bei 20°C und 100 % relativer Luftfeuchtigkeit in einer Inkubationskabine.

Die Pflanzen werden in einem Gewächshaus bei einer Temperatur von ca. 20°C und einer relativen Luftfeuchtigkeit von ca. 80 % aufgestellt.

14 Tage nach der Inokulation erfolgt die Auswertung. Dabei bedeutet 0 % ein Wirkungsgrad, der demjenigen der Kontrolle entspricht, während ein Wirkungsgrad von 100 % bedeutet, dass kein Befall beobachtet wird.

In diesem Test zeigen die erfindungsgemäßen nachfolgenden Verbindungen bei einer Konzentration an Wirkstoff von 1000 ppm einen Wirkungsgrad von 70% oder mehr.

| Bsp. Verbindung Nr. | **% Wirkungsgrad** | Bsp. Verbindung Nr. | **% Wirkungsgrad** | Bsp. Verbindung Nr. | **% Wirkungsgrad** |
|---|---|---|---|---|---|
| 86 | **86** | 184 | **71** | 214 | **100** |
| 84 | **90** | 192 | **71** | 215 | **90** |
| 87 | **88** | 198 | **90** | 218 | **100** |
| 169 | **100** | 164 | **100** | 225 | **90** |

### Beispiel T: In vivo Test an Fusarium nivale (var.majus) (Taubährigkeit/ Weissährigkeit an Weizen / protektiv):

| | |
|---|---|
| Lösungsmittel: | *49* Gewichtsteile *N,N-Dimethylacetamid* |
| Emulgator: | 1 Gewichtsteil Alkylarylpolyglykolether |

Zur Herstellung einer zweckmäßigen Wirkstoffzubereitung vermischt man 1 Gewichtsteil Wirkstoff mit den angegebenen Mengen Lösungsmittel und Emulgator und verdünnt das Konzentrat mit Wasser auf die gewünschte Konzentration.

Zur Prüfung auf protektive Wirksamkeit besprüht man junge Pflanzen mit der Wirkstoffzubereitung in der angegebenen Aufwandmenge.

Nach Antrocknen des Spritzbelages werden die Pflanzen mit Sporen einer Sporensuspension von *Fusarium nivale (var.majus)* besprüht.

Die Pflanzen werden in einer Gewächshauskammer unter eine lichtdurchlässige Inkubationshaube bei 10°C und 100 % relativer Luftfeuchtigkeit gestellt.

5 Tage nach der Inokulation erfolgt die Auswertung. Dabei bedeutet 0 % ein Wirkungsgrad, der demjenigen der Kontrolle entspricht, während ein Wirkungsgrad von 100 % bedeutet, dass kein Befall beobachtet wird.

In diesem Test zeigen die erfindungsgemäßen nachfolgenden Verbindungen bei einer Konzentration an Wirkstoff von 1000 ppm einen Wirkungsgrad von 70% oder mehr.

| Bsp. Verbindung Nr. | **% Wirkungsgrad** | Bsp. Verbindung Nr. | **% Wirkungsgrad** | Bsp. Verbindung Nr. | **% Wirkungsgrad** |
|---|---|---|---|---|---|
| 1 | **92** | 192 | **100** | 225 | **100** |
| 85 | **100** | 198 | **100** | 238 | **75** |
| 86 | **100** | 212 | **75** | 240 | **75** |
| 84 | **100** | 164 | **100** | 159 | **75** |
| 89 | **92** | 214 | **100** | 157 | **88** |
| 87 | **92** | 215 | **100** | 167 | **100** |
| 169 | **71** | 218 | **92** | | |
| 184 | **100** | 224 | **100** | | |

### Beispiel T: In vivo Test an Fusarium graminearum (Taubährigkeit/Weissährigkeit an Gerste / protektiv):

| | |
|---|---|
| Lösungsmittel: | *49* Gewichtsteile *N,N-Dimethylacetamid* |
| Emulgator: | 1 Gewichtsteil Alkylarylpolyglykolether |

Zur Herstellung einer zweckmäßigen Wirkstoffzubereitung vermischt man 1 Gewichtsteil Wirkstoff mit den angegebenen Mengen Lösungsmittel und Emulgator und verdünnt das Konzentrat mit Wasser auf die gewünschte Konzentration.

Zur Prüfung auf protektive Wirksamkeit besprüht man junge Pflanzen mit der Wirkstoffzubereitung in der angegebenen Aufwandmenge.

Nach Antrocknen des Spritzbelages werden die Pflanzen mit Sporen einer Sporensuspension von *Fusarium graminearum* besprüht.

Die Pflanzen werden in einer Gewächshauskammer unter eine lichtdurchlässige Inkubationshaube bei 22°C und 100 % relativer Luftfeuchtigkeit gestellt.

5 Tage nach der Inokulation erfolgt die Auswertung. Dabei bedeutet 0 % ein Wirkungsgrad, der demjenigen der Kontrolle entspricht, während ein Wirkungsgrad von 100 % bedeutet, dass kein Befall beobachtet wird.

In diesem Test zeigen die erfindungsgemäßen nachfolgenden Verbindungen bei einer Konzentration an Wirkstoff von 1000 ppm einen Wirkungsgrad von 70% oder mehr.

| Bsp. Verbindung Nr. | **% Wirkungsgrad** | Bsp. Verbindung Nr. | **% Wirkungsgrad** | Bsp. Verbindung Nr. | **% Wirkungsgrad** |
|---|---|---|---|---|---|
| 1 | **100** | 218 | **100** | 87 | **100** |
| 85 | **100** | 224 | **100** | 169 | **100** |
| 86 | **100** | 225 | **100** | 184 | **100** |
| 84 | **100** | 238 | **92** | 192 | **100** |
| 89 | **100** | 251 | **83** | 198 | **100** |
| 164 | **100** | 159 | **100** | 155 | **88** |
| 214 | **100** | 157 | **100** | | |
| 215 | **100** | 167 | **100** | | |

### Beispiel U: In vivo Test an Pythium ultimum (Wurzelfäule/Umfallkrankheit an Baumwolle / Saatgutbehandlung):

Der Test wurde unter Gewächshaus-Bedingungen durchgeführt.

Baumwollsamen, behandelt mit einer erfindungsgemäßen aktiven Verbindung bzw. einer Kombination von erfindungsgemäßen aktiven Verbindungen wurden in 6*6cm grosse Gefäße, in ein Gemisch aus gedämpfter Felderde und Sand (1:1), ausgesät. Die Testverbindung/en wurden in N-methyl-2-pyrrolidon gelöst und mit Wasser auf die gewünschte Konzentration verdünnt. Die Pflanzen wurden bei 10°C angezogen.

Perlite wurde mit Mycelium von Pythium ultimum inokuliert. 1mL des infizierten Perlites wurde zwischen den behandelten Baumwollsamen verteilt. Die Samen wurden mit einer Deckschicht aus Tongranulat bedeckt und im Gewächshaus für 7 Tage bei 20°C und 80% relativer Luftfeuchtigkeit inkubiert.

Die Auswertung erfolgte durch Auszählung des Auflaufs. Dabei bedeutet 0 % ein Wirkungsgrad, der demjenigen der unbehandelten Kontrolle entspricht, während ein Wirkungsgrad von 100 % bedeutet, dass alle Samen gekeimt sind.

In diesem Test zeigten folgende Verbindungen eine Effizienz von 70% und höher bei einer Dosis von 50 g/dt der aktiven erfindungsgemäßen Verbindung.

| Bsp-Verbindung Nr. | **% Wirkungsgrad** |
|---|---|
| 198 | **88** |

### Beispiel V: In vivo Test an Pyricularia oryzae (Reisbräune / protektiv):

| | |
|---|---|
| Lösungsmittel: | 28,5 Gew.-Teile Aceton |
| Emulgator: | 1,5 Gew.-Teil Alkylarylpolyglykolether |

Zur Herstellung einer zweckmäßigen Wirkstoffzubereitung vermischt man 1 Gewichtsteil Wirkstoff mit der angegebenen Menge Lösungsmittel und verdünnt das Konzentrat mit Wasser und der angegebenen Menge Emulgator auf die gewünschte Konzentration.

Zur Prüfung auf protektive Wirksamkeit bespritzt man junge Reispflanzen mit der Wirkstoffzubereitung in der angegebenen Aufwandmenge. 1 Tag nach der Behandlung werden die Pflanzen mit einer wässrigen Sporensuspension von *Pyricularia oryzae* inokuliert. Anschließend werden die Pflanzen in einem Gewächshaus bei 100 % relativer Luftfeuchtigkeit und 25°C aufgestellt.

5 Tage nach der Inokulation erfolgt die Auswertung. Dabei bedeutet 0 % ein Wirkungsgrad, der demjenigen der Kontrolle entspricht, während ein Wirkungsgrad von 100 % bedeutet, dass kein Befall beobachtet wird.

In diesem Test zeigen die erfindungsgemäßen nachfolgenden Verbindungen bei einer Konzentration an Wirkstoff von 250ppm einen Wirkungsgrad von 80% oder mehr.

| Bsp Nr | **% Wirkungsgrad** |
|---|---|
| 1 | **80** |
| 4 | **80** |
| 30 | **80** |
| 40 | **95** |
| 84 | **85** |
| 85 | **80** |
| 86 | **93** |
| 102 | **93** |
| 104 | **80** |
| 108 | **85** |
| 113 | **90** |
| 116 | **80** |
| 122 | **85** |
| 136 | **80** |
| 198 | **85** |
| 217 | **85** |
| 221 | **92** |

### Beispiel W: In vivo Test an Rhizoctonia solani (Stängelfäule an Reis / protektiv):

| | |
|---|---|
| Lösungsmittel: | 28,5 Gew.-Teile Aceton |
| Emulgator: | 1,5 Gew.-Teil Alkylarylpolyglykolether |

Zur Herstellung einer zweckmäßigen Wirkstoffzubereitung vermischt man 1 Gewichtsteil Wirkstoff mit der angegebenen Menge Lösungsmittel und verdünnt das Konzentrat mit Wasser und der angegebenen Menge Emulgator auf die gewünschte Konzentration.

Zur Prüfung auf protektive Wirksamkeit bespritzt man junge Reispflanzen mit der Wirkstoffzubereitung in der angegebenen Aufwandmenge. 1 Tag nach der Behandlung werden die Pflanzen mit Hyphen von *Rhizoctonia solani* inokuliert. Anschließend werden die Pflanzen in einem Gewächshaus bei 100 % relativer Luftfeuchtigkeit und 25°C aufgestellt. 4 Tage nach der Inokulation erfolgt die Auswertung. Dabei bedeutet 0 % ein Wirkungsgrad, der demjenigen der Kontrolle entspricht, während ein Wirkungsgrad von 100 % bedeutet, dass kein Befall beobachtet wird.

In diesem Test zeigen die erfindungsgemäßen nachfolgenden Verbindungen bei einer Konzentration an Wirkstoff von 250ppm einen Wirkungsgrad von 80% oder mehr.

| Bsp Nr | **% Wirkungsgrad** |
|---|---|
| 1 | **100** |
| 4 | **100** |
| 30 | **95** |
| 84 | **100** |
| 85 | **97** |
| 86 | **100** |
| 89 | **92** |
| 101 | **95** |
| 102 | **98** |
| 104 | **95** |
| 108 | **92** |
| 113 | **93** |
| 116 | **97** |
| 120 | **93** |
| 122 | **97** |
| 136 | **97** |
| 169 | **100** |
| 174 | **93** |
| 176 | **97** |
| 198 | **88** |
| 217 | **100** |
| 221 | **100** |

### Beispiel X: In vivo Test an Cochliobolus miyabeanus (Braunfleckigkeit, Reis / protektiv):

| | |
|---|---|
| Lösungsmittel: | 28,5 Gew.-Teile Aceton |
| Emulgator: | 1,5 Gew.-Teil Alkylarylpolyglykolether |

Zur Herstellung einer zweckmäßigen Wirkstoffzubereitung vermischt man I Gewichtsteil Wirkstoff mit der angegebenen Menge Lösungsmittel und verdünnt das Konzentrat mit Wasser und der angegebenen Menge Emulgator auf die gewünschte Konzentration.

Zur Prüfung auf protektive Wirksamkeit bespritzt man Junge Reispflanzen mit der Wirkstoffzubereitung in der angegebenen Aufwandmenge. 1 Tag nach der Behandlung werden die Pflanzen mit einer wässrigen Sporensuspension von *Cochliobolus miyabeanus* inokuliert. Anschließend werden die Pflanzen in einem Gewächshaus bei 100 % relativer Luftfeuchtigkeit und 25°C aufgestellt.

4 Tage nach der Inokulation erfolgt die Auswertung. Dabei bedeutet 0 % ein Wirkungsgrad, der demjenigen der Kontrolle entspricht, während ein Wirkungsgrad von 100 % bedeutet, dass kein Befall beobachtet wird.

In diesem Test zeigen die erfindungsgemäßen nachfolgenden Verbindungen bei einer Konzentration an Wirkstoff von 250ppm einen Wirkungsgrad von 80% oder mehr.

| Bsp Nr | **% Wirkungsgrad** |
|---|---|
| 1 | **85** |
| 40 | **85** |
| 84 | **93** |
| 85 | **90** |
| 86 | **80** |
| 89 | **85** |
| 104 | **80** |
| 108 | **80** |
| 116 | **94** |
| 122 | **90** |
| 198 | **90** |
| 217 | **85** |
| 221 | **80** |

### Beispiel Y: In vivo Test an Gibberella zeae (Weißährigkeit an Reis / protektiv):

| | |
|---|---|
| Lösungsmittel: | 28,5 Gew.-Teile Aceton |
| Emulgator: | 1,5 Gew.-Teil Alkylarylpolyglykolether |

Zur Herstellung einer zweckmäßigen Wirkstoffzubereitung vermischt man I Gewichtsteil Wirkstoff mit der angegebenen Menge Lösungsmittel und verdünnt das Konzentrat mit Wasser und der angegebenen Menge Emulgator auf die gewünschte Konzentration.

Zur Prüfung auf protektive Wirksamkeit bespritzt man junge Reispflanzen mit der Wirkstoffzubereitung in der angegebenen Aufwandmenge. 1 Tag nach der Behandlung werden die Pflanzen mit einer wässrigen Sporensuspension von *Gibberella zeae* inokuliert. Anschließend werden die Pflanzen in einem Gewächshaus bei 100 % relativer Luftfeuchtigkeit und 25°C aufgestellt.

5 Tage nach der Inokulation erfolgt die Auswertung. Dabei bedeutet 0 % ein Wirkungsgrad, der demjenigen der Kontrolle entspricht, während ein Wirkungsgrad von 100 % bedeutet, dass kein Befall beobachtet wird.

In diesem Test zeigen die erfindungsgemäßen nachfolgenden Verbindungen bei einer Konzentration an Wirkstoff von 250ppm einen Wirkungsgrad von 80% oder mehr.

| Bsp Nr | **% Wirkungsgrad** |
|---|---|
| 198 | **80** |

### Beispiel Z: In vivo Test an Phakopsora pachyrhizi (Sojabohnenrost / protektiv):

| | |
|---|---|
| Lösungsmittel: | 28,5 Gew.-Teile Aceton |
| Emulgator: | 1,5 Gew.-Teil Alkylarylpolyglykolether |

Zur Herstellung einer zweckmäßigen Wirkstoffzubereitung vermischt man 1 Gewichtsteil Wirkstoff mit der angegebenen Menge Lösungsmittel und verdünnt das Konzentrat mit Wasser und der angegebenen Menge Emulgator auf die gewünschte Konzentration.

Zur Prüfung auf protektive Wirksamkeit bespritzt man junge Reispflanzen mit der Wirkstoffzubereitung in der angegebenen Aufwandmenge. 1 Tag nach der Behandlung werden die Pflanzen mit einer wässrigen Sporensuspension von *Phakopsora pachyrhizi* inokuliert. Anschließend werden die Pflanzen in einem Gewächshaus bei 80 % relativer Luftfeuchtigkeit und 20°C aufgestellt.

11 Tage nach der Inokulation erfolgt die Auswertung. Dabei bedeutet 0 % ein Wirkungsgrad, der demjenigen der Kontrolle entspricht, während ein Wirkungsgrad von 100 % bedeutet, dass kein Befall beobachtet wird.

In diesem Test zeigen die erfindungsgemäßen nachfolgenden Verbindungen bei einer Konzentration an Wirkstoff von 250ppm einen Wirkungsgrad von 80% oder mehr.

| Bsp Nr | **% Wirkungsgrad** |
|---|---|
| 1 | **91** |
| 84 | **98** |

## Patentansprüche

1. Verbindungen der Formel [I-a], 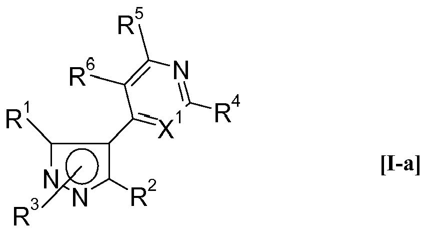 in welcher die Symbole folgende Bedeutungen haben:
X¹ steht für C-H oder N,
R¹ steht für jeweils gegebenenfalls einfach oder mehrfach gleich oder verschieden durch R⁷ substituiertes Phenyl,
R² steht für C₁-C₆-Alkyl, C₁-C₆-Halogenalkyl,
R³ steht für jeweils gegebenenfalls einfach oder mehrfach gleich oder verschieden durch Halogen, Cyano, Hydroxy, C₁-C₆-Alkyl, C₁-C₆-Alkoxy, Halogenalkoxy, Phenyl, Phenoxy substituiertes C₁-C₆-Alkyl, C₂-C₆-Alkenyl, C₂-C₆-Alkinyl, C₃-C₆-Cycloalkyl, C₃-C₆-Cycloalkyl-C₁-C₆-alkyl, C₃-C₆-Cycloalkyloxy, C₁-C₆-Alkoxy, C₁-C₆-Alkoxy-C₁-C₆-alkyl, Acyloxy-C₁-C₆-alkyl, Heteroaryl-C₁-C₆-alkyl, Aryl-C₁-C₆-alkyl, C₁-C₆-Alkylthio-C₁-C₆-alkyl, C₁-C₄-Alkyl-C(O)-C₁-C₆-alkyl, C₃-C₆-Cycloalkyl-C(O)-C₁-C₄-alkyl, C₂-C₉-Heterocycyl-C(O)-C₁-C₄-alkyl, C₁-C₄-Alkyl-C(O)O-C₁-C₆-alkyl, C₁-C₄-Alkyl-C(O)O-C₃-C₆-cycloalkyl, C₁-C₄-Alkyl-C(O)O-heterocyclyl, Heterocyclyl-C₁-C₆-alkyl, Heterocyclyl, OxoHeterocyclyl oder Heteroaryl,
R⁴ steht für Wasserstoff, Halogen, Cyano, -C(O)OR¹², -SR¹², -NR¹²R¹³,-C(O)NR¹²R¹³ oder -NR¹²R¹⁴, -N=C=NR²², -N=C(H)OR²², -N=C(OR²²)R²³,-N=C(SR²²)R²³, -C(=NR²²)NR²²R²³, -SO(=NR²²)R²³, -SO₂R²⁰
oder für jeweils gegebenenfalls einfach oder mehrfach gleich oder verschieden durch R¹¹ substituiertes C₁-C₆-Alkyl, C₃-C₈-Cycloalkyl, C₂-C₆-Alkenyl, C₆-C₁₄-Aryl, C₂-C₉-Heterocyclyl, C₂-C₉-Heteroaryl,
R⁵ und R⁶ stehen unabhängig voneinander für Wasserstoff, Fluor, Chlor, Brom, Cyano, Nitro, -OH, -SH,
oder für jeweils gegebenenfalls einfach oder mehrfach gleich oder verschieden durch R¹¹ substituiertes C₁-C₆-Alkyl, C₃-C₆-Cycloalkyl, C₂-C₆-Alkenyl, C₂-C₆-Alkinyl, C₆-C₁₄-Aryl, -O-(C₁-C₄-Alkyl), -O-(C₆-C₁₄-Aryl), -S-(C₁-C₄-Alkyl), -S(O)-(C₁-C₆-Alkyl) oder -C(O)-(C₁-C₆-Alkyl)
oder bilden außerdem gemeinsam mit dem Kohlenstoffatomen, an das sie gebunden sind, einen gegebenenfalls einfach oder mehrfach, gleich oder verschieden durch Halogen, Sauerstoff, Cyano oder C₁-C₄-Alkyl substituierten Cyclus mit 3 bis 8 Ringatomen, wobei der Cyclus 1 bis 4 Heteroatome aus der Reihe Sauerstoff, Schwefel oder -NR¹⁹ enthalten kann,
R⁷ steht unabhängig voneinander für eine oder mehrere der folgenden Gruppen: Fluor, Chlor, Brom, Cyano, Nitro, -OH, -SH,
oder für jeweils gegebenenfalls einfach oder mehrfach gleich oder verschieden durch Fluor, Chlor, Brom, -OH, Cyano, C₁-C₆-Alkyl oder -O-(C₁-C₄-Alkyl) substituiertes C₁-C₆-Alkyl, C₃-C₆-Cycloalkyl, C₂-C₆-Alkenyl, C₂-C₆-Alkinyl, Tri(C₁-C₄-alkyl)silyl, C₆-C₁₄-Aryl, -O-(C₁-C₄-Alkyl), -O-(C₆-C₁₄-Aryl), -S-(C₁-C₄-Alkyl), -S(O)-(C₁-C₆-Alkyl) oder -S(O)₂-(C₁-C₆-Alkyl),
R¹¹ steht für -OH, Fluor, Chlor, Brom, Cyano, -NH-C(O)R²⁰, -NR²⁰R²¹,-C(O)R²⁰, -C(O)OR²⁰, -C(O)NR²⁰R²¹, -SO₂R²⁰
oder für jeweils gegebenenfalls einfach oder mehrfach gleich oder verschieden durch Fluor, Chlor, Brom, -OH, Carbonyl, Cyano, C₁-C₆-Alkyl oder -O-(C₁-C₄-Alkyl) substituiertes C₁-C₆-Alkyl, C₂-C₆-Alkenyl, C₂-C₆-Alkinyl, C₁-C₁₁-Heteroalkyl, C₃-C₈-Cycloalkyl, -O-(C₁-C₄-Alkyl), -S-(C₁-C₄-Alkyl), -O-(C₃-C₈-Cycloalkyl), -S-(C₃-C₈-Cycloalkyl), C₆-C₁₄-Aryl, -O-(C₆-C₁₄-Aryl), -S-(C₆-C₁₄-Aryl), C₂-C₉-Heterocyclyl oder C₂-C₉-Heteroaryl,
R¹² und R¹³ stehen unabhängig voneinander für eine oder mehrere der folgenden Gruppen: H, -C(S)R¹⁵, -C(O)R¹⁵, -SO₂R¹⁵, -C(O)OR¹⁵, -OR¹⁵ oder-C(O)NR¹⁵R¹⁶
oder für jeweils gegebenenfalls einfach oder mehrfach gleich oder verschieden durch Fluor, Chlor, Brom, -OH, Cyano, C₁-C₆-Alkyl, -O-C(O)R¹¹ , -O-P(O)(OR¹¹)₂, -O-B(OR¹¹)₂ oder -O-(C₁-C₄-Alkyl) substituiertes C₁-C₆-Alkyl, C₃-C₆-Cycloalkyl, C₂-C₆-Alkenyl, C₂-C₆-Alkinyl, C₃-C₈-Cycloalkyl, C₆-C₁₄-Aryl, C₂-C₉-Heterocyclyl oder C₂-C₉-Heteroaryl,
R¹⁴ steht für -CH₂-NR²²R²³, Piperidin-1-ylmethyl oder Morpholin-4-ylmethyl
oder für jeweils gegebenenfalls einfach oder mehrfach gleich oder verschieden durch Fluor, Chlor, Brom, -OH, Cyano substituiertes C₁-C₆-Alkyl oder -O-(C₁-C₄-Alkyl),
R¹⁵ und R¹⁶ stehen unabhängig voneinander für Wasserstoff oder -OH
oder für jeweils gegebenenfalls einfach oder mehrfach gleich oder verschieden durch R¹¹ substituiertes C₁-C₆-Alkyl, C₃-C₆-Cycloalkyl, C₂-C₆-Alkenyl, C₂-C₆-Alkinyl, C₃-C₈-Cycloalkyl, C₆-C₁₄-Aryl, C₂-C₉-Heterocyclyl oder C₂-C₉-Heteroaryl
oder bilden gemeinsam mit dem Stickstoffatom an das sie gebunden sind einen 3- bis 7-gliedrigen Ring, der ein weiteres dem Stickstoff nicht benachbartes Heteroatom aus der Reihe N, O enthalten kann,
R¹⁷ und R¹⁸ stehen unabhängig voneinander für eine oder mehrere der folgenden Gruppen: H, -C(O)OR¹¹
oder für jeweils gegebenenfalls einfach oder mehrfach gleich oder verschieden durch Fluor, Chlor, Brom, -OH, Cyano, C₁-C₆-Alkyl, und -O-(C₁-C₄-Alkyl) substituiertes C₁-C₆-Alkyl, C₃-C₆-Cycloalkyl, C₂-C₆-Alkenyl, C₂-C₆-Alkinyl, C₃-C₈-Cycloalkyl, C₆-C₁₄-Aryl, C₂-C₉-Heterocyclyl oder C₂-C₉-Heteroaryl,
R¹⁹ steht für H, C₁-C₆-Alkyl, C₃-C₆-Cycloalkyl, C₂-C₆-Alkenyl, C₂-C₆-Alkinyl,-C(S)R¹⁵, -C(O)R¹⁵, -SO₂R¹⁵, -C(O)OR¹⁵,
R²⁰ und R²¹ stehen unabhängig voneinander für jeweils gegebenenfalls einfach oder mehrfach gleich oder verschieden durch Fluor, Chlor, Brom, -OH, Cyano substituiertes C₁-C₆-Alkyl, C₃-C₆-Cycloalkyl, C₂-C₆-Alkenyl, C₂-C₆-Alkinyl oder Wasserstoff,
R²² und R²³ stehen unabhängig voneinander für C₁-C₆-Alkyl, C₃-C₆-Cycloalkyl, C₂-C₆-Alkenyl, C₂-C₆-Alkinyl oder Wasserstoff,
sowie agrochemisch wirksame Salze davon,
wobei folgende Verbindungen ausgenommen sind:
a) Verbindungen in denen,
X¹ für N steht,
R¹ für ein gegebenenfalls substituiertes Phenyl steht,
R³ für Butyl oder Propyn-2-yl steht,
R⁴ für -NHR₁₂ steht,
R¹² für gegebenenfalls substituiertes Phenyl steht,
und
b) Verbindungen in denen
X¹ für N steht
R², R⁴, R⁵, R⁶ für H stehen,
R³ für Methyl, Ethyl, Allyl, 2-Methoxyethyl oder Benzyl steht, wenn R¹ für 4-Chlorphenyl steht, oder
R³ für Methyl steht, wenn R¹ für Phenyl, 4-Methoxyphenyl oder 4-Fluorphenyl steht.

2. Verbindungen der Formel [I-a] nach Anspruch 1,
in welcher die Symbole folgende Bedeutungen haben,
R¹ steht für gegebenenfalls einfach oder mehrfach gleich oder verschieden durch R⁷ substituiertes Phenyl,
R² steht für Methyl, Ethyl, Isopropyl,
R³ steht für Propan-2-yl, Isobutyl, Butan-2-yl, 2-Methylpropyl, 2,2-Dimethylpropyl, 3-Methylbut-2-en-1-yl, But-2-en-1-yl, But-3-en-2-yl, Propadienyl, Prop-2-in-1-yl, But-2-in-1-yl, But-3-in-2-yl, 2-Methylbut-3-in-2-yl, 2-Methylbut-3-in-2-yl, Cyanmethyl, 2-Cyanethyl, 1-Cyanpropan-2-yl, Cyclopropyl, Cyclobutyl, Cyclopentyl, Cyclohexyl, (2,2-Dichlorcyclopropyl)methyl, Cyclopropylmethyl, 1-Cyclopropylethyl, Trichlormethyl, Trifluormethyl, 2-Fluorethyl, 2,2-Difluorethyl, 2,2,2-Trifluorethyl, 2,2,2-Trichlorethyl, 2-Chlorethyl, 2-Bromethyl, 2-Fluorpropyl, 3-Fluorpropyl, 2-Chlorpropyl, 3-Chlorpropyl, 1,3-Difluorpropan-2-yl, 2-Fluorbenzyl, 3-Fluorbenzyl, 4-Fluorbenzyl, 2,3-Difluorbenzyl, 2-Chlor-6-fluorbenzyl, 1-(2-Chlorphenyl)ethyl, 1-(3-Chlorphenyl)ethyl, 1-(4-Chlorphenyl)ethyl, 3-Cyanobenzyl, 4-Cyanobenzyl, 4-(Difluormethoxy)benzyl, 2-Cyanobenzyl, 2-(3-Chlorphenyl)ethyl, 2-(2-Chlorphenyl)ethyl, 1-Naphthylmethyl, (Pyridin-3-ylmethyl, 2-Chlor-1,3-thiazol-5-yl)methyl, Methoxymethyl, 2-Methoxyethyl, 2-(Methylsulfanyl)ethyl, 2-(Trifluormethoxy)ethyl, 1-Methoxypropan-2-yl, 2-[2-(2-Methoxyethoxy)ethoxy] ethyl, 2-(2-Methoxyethoxy)ethyl, Tetrahydrofuran-2-ylmethyl, (3-Methyloxetan-3-yl)methyl, 1H-Imidazol-2-ylmethyl, Tetrahydrofuran-3-yl, 2-Oxotetrahydrofuran-3-yl, 2-tert-Butoxy-2-oxoethyl, 1-Methoxy-3-methyl-1-oxobutan-2-yl, 1-Methoxy-1-oxopropan-2-yl oder 3-Ethoxy-3-oxopropyl, Propan-2-yloxy, Methyl, Ethyl, n-Propyl, 2-Ethoxyethyl, 2-Chlorethyl,
R⁴ steht für Wasserstoff oder -NR¹²R¹³ oder -NHR¹³,
R⁵ und R⁶ stehen unabhängig voneinander für Wasserstoff, Fluor oder Cyano
oder bilden gemeinsam mit dem Kohlenstoffatomen, an das sie gebunden sind, einen Monocyclus, wobei sie gemeinsam für gegebenenfalls einfach oder mehrfach gleich
oder verschieden durch R¹¹ substituiertes -(CH=CH-CH=CH)-, -(CH=CH-N(R¹⁹), -(CH=CH-CH=N)-, -(NH-CH=N)- oder -(CH₂-C(O)-N(R¹⁹)-stehen,
R⁷ steht unabhängig voneinander für eine oder mehrere der folgenden Gruppen: Fluor, Chlor, Cyano oder Methyl,
R¹¹ steht für -OH, Fluor, Chlor, Brom, Cyano, -NH-C(O)R²⁰, -C(O)R²⁰,-C(O)OR²⁰, -C(O)NR²⁰R²¹, -SO₂R²⁰
oder für jeweils gegebenenfalls einfach oder mehrfach gleich oder verschieden durch Fluor, Chlor, Brom, -OH, Cyano, C₁-C₆-Alkyl oder -O-(C₁-C₄-Alkyl) substituiertes C₁-C₆-Alkyl, C₃-C₈-Cycloalkyl, C₂-C₆-Alkenyl, C₂-C₆-Alkinyl, -O-(C₁-C₄-Alkyl), -S-(C₁-C₄-Alkyl), -O-(C₃-C₈-Cycloalkyl) oder -S-(C₃-C₈-Cycloalkyl),
R¹² steht für Wasserstoff, -C(S)R¹⁵, -C(O)R¹⁵, -SO₂R¹⁵, -C(O)OR¹⁵, -OR¹⁵ oder-C(O)NR¹⁵R¹⁶,
R¹³ steht für C₁-C₆-Alkyl, C₂-C₆-Alkenyl, C₂-C₆-Alkinyl, C₂-C₉-Heterocyclyl oder C₂-C₉-Heteroaryl oder Wasserstoff,
R¹⁵ steht für jeweils gegebenenfalls einfach oder mehrfach gleich oder verschieden durch Halogen, -OH, Cyano oder C₁-C₄-Alkyl substituiertes C₁-C₆-Alkyl, C₃-C₆-Cycloalkyl, C₂-C₆-Alkenyl, C₂-C₆-Alkinyl, C₃-C₈-Cycloalkyl, C₂-C₉-Heterocyclyl, C₂-C₉-Heteroaryl
oder für Wasserstoff
R¹⁶ steht für Wasserstoff, Methyl, Ethyl oder Propyl,
R¹⁹ steht für H, C₂-C₆-Alkinyl, -C(O)R¹⁵, -SO₂R¹⁵, -C(O)OR¹⁵,
R²⁰ und R²¹ stehen unabhängig voneinander für jeweils gegebenenfalls einfach oder mehrfach gleich oder verschieden durch Fluor, Chlor, Brom, -OH, Cyano substituiertes Methyl, Ethyl, Propyl, iso-Propyl, Cyclopropyl, Cyclobutyl
oder für Wasserstoff,
sowie agrochemisch wirksame Salze davon.

3. Verbindungen der Formel [I-a] nach einem oder mehreren der Ansprüche 1 bis 2,
in welcher die Symbole folgende Bedeutungen haben,
X¹ steht für C-H,
R⁴ steht für Wasserstoff
sowie agrochemisch wirksame Salze davon.

4. Verbindungen der Formel [I-a] nach einem oder mehreren der Ansprüche 1 bis 2,
in welcher die Symbole folgende Bedeutungen haben,
X¹ steht für N,
R⁴ steht für -NHR¹³,
R¹³ steht für jeweils gegebenenfalls einfach oder mehrfach gleich oder verschieden durch Fluor, Chlor, Brom, -OH, Cyano substituiertes C₁-C₆-Alkyl, C₃-C₆-Cycloalkyl, C₂-C₆-Alkenyl, C₂-C₆-Alkinyl, C₂-C₉-Heterocyclyl oder C₂-C₉-Heteroaryl,
sowie agrochemisch wirksame Salze davon.

5. Verbindungen der Formel **[I-b],** 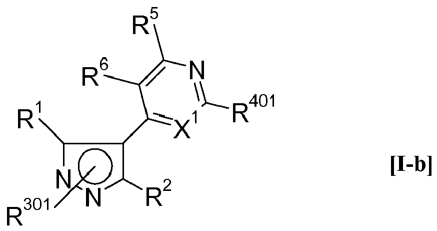 in welcher die Symbole folgende Bedeutungen haben:
X¹ steht für C-H oder N,
R¹ steht für jeweils gegebenenfalls einfach oder mehrfach gleich oder verschieden durch R⁷ substituiertes Phenyl,
R² steht für C₁-C₆-Alkyl, C₁-C₆-Halogenalkyl,
R³⁰¹ steht für -C(O)N(R⁹R¹⁰), -C(O)R⁹, -C(O)OR⁹, -S(O)₂R⁹ oder für jeweils gegebenenfalls einfach oder mehrfach gleich oder verschieden durch R⁸ substituiertes C₁-C₆-Alkyl, C₂-C₆-Alkenyl, C₃-C₆-Allenyl, C₂-C₆-Alkinyl, C₃-C₈-Cycloalkyl, C₁-C₆-Alkoxy, C₂-C₉-Heterocyclyl, C₂-C₉-OxoHeterocyclyl, oder Heteroaryl,
R⁴⁰¹ steht für -NR¹²R¹³, -C(O)NR¹²R¹³ oder -N(R¹²)₂
R⁵ und R⁶ stehen unabhängig voneinander für Wasserstoff, Fluor, Chlor, Brom, Cyano, Nitro, -OH, -SH,
oder für jeweils gegebenenfalls einfach oder mehrfach gleich oder verschieden durch R¹¹ substituiertes C₁-C₆-Alkyl, C₃-C₆-Cycloalkyl, C₂-C₆-Alkenyl, C₂-C₆-Alkinyl, C₆-C₁₄-Aryl, -O-(C₁-C₄-Alkyl), -O-(C₆-C₁₄-Aryl), -S-(C₁-C₄-Alkyl), -S(O)-(C₁-C₆-Alkyl) oder -C(O)-(C₁-C₆-Alkyl),
oder bilden außerdem gemeinsam mit dem Kohlenstoffatomen, an das sie gebunden sind, einen gegebenenfalls einfach oder mehrfach, gleich oder verschieden durch Halogen, Sauerstoff, Cyano oder C₁-C₄-Alkyl substituierten Cyclus mit 3 bis 8 Ringatomen, wobei der Cyclus 1 bis 4 Heteroatome aus der Reihe Sauerstoff, Schwefel oder -N-R¹⁹ enthalten kann,
R⁷ steht unabhängig voneinander für eine oder mehrere der folgenden Gruppen: Fluor, Chlor, Brom, Cyano, Nitro, -OH, -SH,
oder für gegebenenfalls einfach oder mehrfach gleich oder verschieden durch Fluor, Chlor, Brom, -OH, Cyano, C₁-C₆-Alkyl oder -O-(C₁-C₄-Alkyl) substituiertes C₁-C₆-Alkyl, C₃-C₆-Cycloalkyl, C₂-C₆-Alkenyl, C₂-C₆-Alkinyl, Tri(C₁-C₄-alkyl)silyl, C₆-C₁₄-Aryl, -O-(C₁-C₄-Alkyl), -O-(C₆-C₁₄-Aryl), -S-(C₁-C₄-Alkyl), -S(O)-(C₁-C₆-Alkyl), -S(O)₂-(C₁-C₆-Alkyl),
R⁸ steht für -OH, Halogen, NO₂, Cyano, -NR⁹R¹⁰, -C(O)N(R⁹R¹⁰), -C(O)R⁹,-C(O)OR⁹, -O-C(O)R⁹, -(CH₂)ₙC(O)R⁹ wobei n = eine ganze Zahl zwischen 1 und 6 ist
oder für jeweils gegebenenfalls einfach oder mehrfach gleich oder verschieden durch R¹¹ substituiertes C₁-C₆-Alkyl, C₂-C₈-Alkenyl, C₂-C₈-Alkinyl, C₃-C₈-Cycloalkyl, C₁-C₆-Halogenalkyl, C₆-C₁₄-Aryl, C₂-C₉-Heterocyclyl, C₂-C₉-Heteroaryl, -O-(C₁-C₄-Alkyl), -S-(C₁-C₄-Alkyl), -O-(C₃-C₈-Cycloalkyl), -S-(C₃-C₈-Cycloalkyl),
R⁹ und R¹⁰ stehen unabhängig voneinander für jeweils gegebenenfalls einfach oder mehrfach gleich oder verschieden durch R¹¹ substituiertes C₁-C₆-Alkyl, C₂-C₈Alkenyl, C₂-C₈-Alkinyl, C₃-C₈-Cycloalkyl, C₆-C₁₄-Aryl, C₂-C₉-Heterocyclyl, C₂-C₉-Heteroaryl,
oder für Wasserstoff,
R¹¹ steht für -OH, Fluor, Chlor, Brom, Cyano, -NH-C(O)R²⁰, -NR²⁰R²¹ ,-C(O)R²⁰, -C(O)OR²⁰, -C(O)NR²⁰R²¹, -SO₂R²⁰
oder für gegebenenfalls einfach oder mehrfach gleich oder verschieden durch Fluor, Chlor, Brom, -OH, Carbonyl, Cyano, C₁-C₆-Alkyl oder -O-(C₁-C₄-Alkyl) substituiertes
C₁-C₆-Alkyl, C₂-C₆-Alkenyl, C₂-C₆-Alkinyl, C₁-C₁₁-Heteroalkyl, C₃-C₈-Cycloalkyl, -O-(C₁-C₄-Alkyl), -S-(C₁-C₄-Alkyl), -O-(C₃-C₈-Cycloalkyl), S-(C₃-C₈-Cycloalkyl), C₆-C₁₄-Aryl, -O-(C₆-C₁₄-Aryl), -S-(C₆-C₁₄-Aryl), C₂-C₉-Heterocyclyl oder C₂-C₉-Heteroaryl,
R¹² steht für -C(S)R¹⁵, -C(O)R¹⁵, -SO₂R¹⁵, -C(O)OR¹⁵, -OR¹⁵ oder -C(O)NR¹⁵R¹⁶,
R¹³ steht für jeweils gegebenenfalls einfach oder mehrfach gleich oder verschieden durch Fluor, Chlor, Brom, -OH, Cyano, C₁-C₆-Alkyl, -O-C(O)-C₁-C₄-Alkyl, -O-P(O)(O-C₁-C₄-Alkyl)₂, -O-B(O-C₁-C₄-Alkyl)₂ oder -O-(C₁-C₄-Alkyl) substituiertes C₁-C₆-Alkyl, C₃-C₆-Cycloalkyl, C₂-C₆-Alkenyl, C₂-C₆-Alkinyl, C₃-C₈-Cycloalkyl, C₂-C₉-Heterocyclyl, C₂-C₉-Heteroaryl
oder für Wasserstoff,
R¹⁵ und R¹⁶ stehen unabhängig voneinander für Wasserstoff oder -OH,
oder für jeweils gegebenenfalls einfach oder mehrfach gleich oder verschieden durch R¹¹ substituiertes C₁-C₆-Alkyl, C₃-C₆-Cycloalkyl, C₂-C₆-Alkenyl, C₂-C₆-Alkinyl, C₃-C₈-Cycloalkyl, C₆-C₁₄-Aryl, C₂-C₉-Heterocyclyl oder C₂-C₉-Heteroaryl
oder bilden gemeinsam mit dem Stickstoffatom an das sie gebunden sind einen 3 bis 7-gliedrigen Ring, der ein weiteres dem Stickstoff nicht benachbartes Heteroatom aus der Reihe N, O enthalten kann,
R¹⁹ steht für H, C₁-C₆-Alkyl, C₃-C₆-Cycloalkyl, C₂-C₆-Alkenyl, C₂-C₆-Alkinyl,-C(S)R¹⁵, -C(O)R¹⁵, -SO₂R¹⁵, -C(O)OR¹⁵,
R²⁰ und R²¹ stehen unabhängig voneinander für jeweils gegebenenfalls einfach oder mehrfach gleich oder verschieden durch Fluor, Chlor, Brom, -OH, Cyano substituiertes C₁-C₆-Alkyl, C₃-C₆-Cycloalkyl, C₂-C₆-Alkenyl, C₂-C₆-Alkinyl oder Wasserstoff,
sowie agrochemisch wirksame Salze davon,
wobei folgende Verbindungen ausgenommen sind:
a) Verbindungen in denen,
R³⁰¹ für gegebenenfalls substituiertes [1,2,4]Triazolo[4,3-b]pyridazin-6-yl, 7,8-Dihydro[1,2,4]triazolo[4,3-b]pyridazin 6-yl, 6-Oxo-1,6-dihydropyridazin-3-yl, 6-Oxo-1,4,5,6-tetrahydropyridazin-3-yl oder 6-Chlorpyridazin-3-yl steht,
R⁵, R⁶ für H stehen,
b) die Verbindungen 4-{1-[2-(Dimethylamino)ethyl]-3-(4-fluorphenyl)-1H-pyrazol-4-yl}-N,N-dimethylpyridin-2-amin und 1-(4-{4-[1-Ethyl-3-(4-nitrophenyl)-1H-pyrazol-4-yl]-1H-pyrrolo[2,3-b]pyridin-6-yl}phenyl)-N,N-dimethylmethanamin.

6. Verbindungen der Formel [I-b] nach Anspruch 5,
in welcher die Symbole folgende Bedeutungen haben,
R¹ steht für gegebenenfalls einfach oder mehrfach gleich oder verschieden durch R⁷ substituiertes Phenyl,
R² steht für Methyl, Ethyl, Isopropyl,
R³⁰¹ steht für jeweils gegebenenfalls einfach oder mehrfach gleich oder verschieden durch R⁸ substituiertes C₁-C₆-Alkyl, C₂-C₆-Alkenyl, C₃-C₆-Allenyl, C₂-C₆-Alkinyl, C₃-C₈-Cycloalkyl oder C₁-C₆-Alkoxy,
R⁴⁰¹ steht für -NR¹²R¹³,
R⁵ und R⁶ stehen unabhängig voneinander für Wasserstoff, Fluor oder Cyano
oder bilden gemeinsam mit dem Kohlenstoffatomen, an das sie gebunden sind, einen Monocyclus, wobei sie gemeinsam für gegebenenfalls einfach oder mehrfach gleich oder verschieden durch R¹¹ substituiertes -(CH=CH-CH=CH)-, -(CH=CH-N(R¹⁹)-, -(CH=CH-CH=N)- oder -(CH₂-C(O)-N(R¹⁹)-stehen,
R⁷ steht für Fluor, Chlor, Cyano oder Methyl,
R⁸ steht für Fluor, Chlor, Cyano oder für jeweils gegebenenfalls einfach oder mehrfach gleich oder verschieden durch R¹¹ substituiertes C₁-C₆-Alkyl, C₂-C₈-Alkenyl, C₂-C₈-Alkinyl, C₃-C₈-Cycloalkyl, C₆-C₁₄-Aryl, Heterocyclyl, Heteroaryl, -O-(C₁-C₄-Alkyl), -S-(C₁-C₄-Alkyl), -O-(C₃-C₈-Cycloalkyl), -S-(C₃-C₈-Cycloalkyl),
R¹¹ steht für eine oder mehrere der folgenden Gruppen: -OH, Fluor, Chlor, Brom, Cyano, -NH-C(O)R²⁰, -C(O)R²⁰, -C(O)OR²⁰, -C(O)NR²⁰R²¹, -SO₂R²⁰
oder für jeweils gegebenenfalls einfach oder mehrfach gleich oder verschieden durch Fluor, Chlor, Brom, -OH, Cyano, C₁-C₆-Alkyl oder -O-(C₁-C₄-Alkyl) substituiertes C₁-C₆-Alkyl, C₃-C₈-Cycloalkyl, C₂-C₆-Alkenyl, C₂-C₆-Alkinyl, -O-(C₁-C₄-Alkyl), -S-(C₁-C₄-Alkyl), -O-(C₃-C₈-Cycloalkyl) oder -S-(C₃-C₈-Cycloalkyl),
R¹² steht für: -C(S)R¹⁵, -C(O)R¹⁵, -SO₂R¹⁵, -C(O)OR¹⁵, -OR¹⁵ oder-C(O)NR¹⁵R¹⁶,
R¹³ steht für C₁-C₆-Alkyl, C₂-C₆-Alkenyl, C₂-C₆-Alkinyl oder Wasserstoff,
R¹⁵ steht für jeweils gegebenenfalls einfach oder mehrfach gleich oder verschieden durch Halogen, -OH, Cyano oder C₁-C₄-Alkyl substituiertes C₁-C₆-Alkyl, C₃-C₆-Cycloalkyl, C₂-C₆-Alkenyl, C₂-C₆-Alkinyl, C₃-C₈-Cycloalkyl, C₂-C₉-Heterocyclyl, C₂-C₉-Heteroaryl
oder für Wasserstoff,
R¹⁶ steht für Wasserstoff, Methyl, Ethyl oder Propyl,
R¹⁹ steht für H, -C₂-C₆-Alkinyl, -C(O)R¹⁵, -SO₂R¹⁵, -C(O)OR¹⁵,
R²⁰ und R²¹ stehen unabhängig voneinander für jeweils gegebenenfalls einfach oder mehrfach gleich oder verschieden durch Fluor, Chlor, Brom, -OH, Cyano substituiertes Methyl, Ethyl, Propyl, iso-Propyl, Cyclopropyl, Cyclobutyl oder für Wasserstoff,
sowie agrochemisch wirksame Salze davon.

7. Verbindungen der Formel [I-b] nach einem oder mehreren der Ansprüche 5 bis 6,
in welcher die Symbole folgende Bedeutungen haben,
X¹ steht für N,
R¹ steht für Phenyl, 3-Methylphenyl, 2-Fluorphenyl, 3-Fluorphenyl, 4-Fluorphenyl, 3-Trifluormethylphenyl, 4-Chlorphenyl, 2,5-Difluorphenyl, 2,4-Difluorphenyl, 2,6-Difluorphenyl, 3-Methyl-4-fluorphenyl, 3-Cyan-4-fluorphenyl oder 2,4,6-Trifluorphenyl,
R³⁰¹ steht für Methyl, Ethyl, 1-Propyl, Propan-2-yl, Isobutyl, Butan-2-yl, 2-Methylpropyl, 2,2-Dimethylpropyl, 2-(Morpholin-4-yl)ethyl, 2-Cyanethyl, Cyanmethyl, 2-Cyan-2-methylpropyl, 3-Methylbut-2-en-1-yl, But-2-en-1-yl, But-3-en-2-yl, Propadienyl, Prop-2-en-1-yl, Prop-2-in-1-yl, But-2-in-1-yl, But-3-in-2-yl, 2-Methylbut-3-in-2-yl, 2-Methylbut-3-in-2-yl, Cyclopropyl, Cyclobutyl, Cyclopentyl, Cyclohexyl, (2,2-Dichlorcyclopropyl)methyl, Cyclopropylmethyl, 1-Cyclopropylethyl, Trichlormethyl, Trifluormethyl, 2-Fluorethyl, 2,2-Difluorethyl, 2,2,2-Trifluorethyl, 2,2,2-Trichlorethyl, 2-Chlorethyl, 2-Bromethyl, 2-Fluorpropyl, 3-Fluorpropyl, 2-Chlorpropyl, 3-Chlorpropyl, 1,3-Difluorpropan-2-yl, 1,1,1-Trifluorpropan-2-yl, 1,1,1-Trifluor-2-methylpropan-2-yl, 3,3,3-Trifluor-2-hydroxypropyl, 2-Fluorbenzyl, 3-Fluorbenzyl, 4-Fluorbenzyl, 2,3-Difluorbenzyl, 2-Chlor-6-fluorbenzyl, 1-(2-Chlorphenyl)ethyl, 1-(3-Chlorphenyl)ethyl, 1-(4-Chlorphenyl)ethyl, 3-Cyanobenzyl, 4-Cyanobenzyl, 4-(Difluormethoxy)benzyl, 2-Cyanobenzyl, 2-(3-Chlorphenyl)ethyl, 2-(2-Chlorphenyl)ethyl, 1-Naphthylmethyl, (Pyridin-3-ylmethyl, 2-Chlor-1,3-thiazol-5-yl)methyl, Methoxymethyl, 2-Methoxyethyl, 2-Methoxypropyl, 2-(Methylsulfanyl)ethyl, 2-(Trifluormethoxy)ethyl, 1-Methoxypropan-2-yl, 2-[2-(2-Methoxyethoxy)ethoxy]ethyl, 2-(2-Methoxyethoxy)ethyl, 1,3-Dimethoxypropan-2-yl, 2-(Cyclopropyloxy)ethyl, Tetrahydrofuran-2-ylmethyl, (3-Methyloxetan-3-yl)methyl, 1H-Imidazol-2-ylmethyl, Tetrahydrofuran-3-yl, 2-Oxotetrahydrofuran-3-yl, 2-tert-Butoxy-2-oxoethyl, 1-Methoxy-3-methyl-1-oxobutan-2-yl, 1-Methoxy-1-oxopropan-2-yl oder 3-Ethoxy-3-oxopropyl, 1-Cyanpropan-2-yl, Propan-2-yloxy, 2-Ethoxyethyl,
R⁴⁰¹ steht für -NHR¹²,
R¹¹ steht für eine oder mehrere der folgenden Gruppen: -OH, Fluor, Chlor, Cyano, Methyl, Ethyl, Cyclopropyl,
R¹² steht für -C(S)R¹⁵, -SO₂R¹⁵, -C(O)OR¹⁵ oder -C(O)R¹⁵,
R¹⁵ steht für jeweils gegebenenfalls einfach oder mehrfach gleich oder verschieden durch Halogen, OH, Cyano oder C₁-C₄-alkyl substituiertes Methyl, Ethyl, n-Propyl, Isopropyl, sec-Butyl, Isobutyl, Cyclopropyl, Cyclobutyl, Cyclopentyl, Cyclohexyl, 2-Methoxyethyl, (2-Methoxyethoxy)methyl, Cyclopentenyl, Cyclohexenyl, Oxetanyl, Tetrahydrofuran-2-yl, Ethinyl, Prop-1-in-1-yl, Prop-1-en-1-yl, Aminomethyl, Aminoethyl, Aminopropyl, Aminobutyl, Aminoisopropyl, Aminocyclopropyl, Aminocyclobutyl, Aminocyclopentyl, Dimethylamino, Ethyl(methyl)amino, Pyrrolidinyl, Diethylamino, 2-Pyridyl, 3-Pyridyl, 4-Pyridyl, Ethoxycarbonyl, Benzyl, Phenyl, 2-Thienyl, 3-Thienyl
oder für Wasserstoff,
R¹⁹ steht für H, Acetyl, Ethoxycarbonyl, Methoxycarbonyl, Prop-2-in-1-yl, But-2-in-1-yl,
sowie agrochemisch wirksame Salze davon.

8. Verbindungen der Formel [I-b] nach einem oder mehreren der Ansprüche 5 bis 6,
in welcher die Symbole folgende Bedeutungen haben,
X¹ steht für C-H,
R¹ steht für 4-Fluorphenyl, 3-Chlorphenyl, 2,6-Difluorphenyl, 3-Methylphenyl,
R² steht für Methyl,
R⁴⁰¹ steht für Acetylamino, n-Propionylamino, Isobutyrylamino, (Cyclopropylcarbonyl)amino, (Methoxyacetyl)amino, 2-Methoxypropanoyl, (2-Methylbutanoyl)amino, But-2-enoylamino, Prop-2-inoylamino, 3-(Dimethylamino)prop-2-enoyl]amino, 3,3,3-Trifluorpropanoyl)amino, 3,3-Difluorpropanoyl)amino, (Cyclopropylacetyl)amino, Lactoylamino, (Cyclobutylcarbonyl)amino, (Cyclopentylacetyl)amino, 2-Methylcyclopropyl)carbonyl] amino, (3-Methylbutanoyl)amino, (Phenylacetyl)amino, Benzoylamino, (3-Thienylcarbonyl)amino, (2-Thienylcarbonyl)amino (2-Hydroxy-2-methylpropanoyl)amino, [(2-Methoxyethoxy)acetyl] amino, 2,3-Dihydroxypropanoyl)amino,
sowie agrochemisch wirksame Salze davon.

9. Verfahren zur Bekämpfung phytopathogener und Mykotoxin produzierender Pilze, **dadurch gekennzeichnet, dass** man Phenylpyri(mi)dinylazole der Formel [I-a] und/oder [I-b] gemäß einem oder mehreren der Ansprüche 1 bis 8 auf die Pilze und/oder deren Lebensraum ausbringt.

10. Mittel zur Bekämpfung phytopathogener und Mykotoxin produzierender Pilze, **gekennzeichnet durch** einen Gehalt an mindestens einem Phenylpyri(mi)dinylazole der Formel [I-a] und/oder [I-b] gemäß einem oder mehreren der Ansprüche 1 bis 8 neben Streckmitteln und/oder oberflächenaktiven Stoffen.

11. Verbindungen der Formel [X], 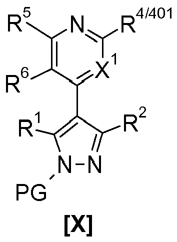 in welcher
PG für Tetrahydro-2H-pyran-2-yl steht,
R¹, R², X¹, R⁶, R⁵, R^{4/401} für die gleichen Restedefinitionen wie für Formel [I-a] und [I-b] in den Ansprüchen 1 bis 8 definiert, stehen,
sowie agrochemisch wirksame Salze davon.

12. Verbindungen der Formel [XI] 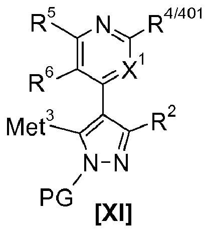 in welcher
Met³ steht für Tributylstannyl, 4,4,5,5-Tetramethyl-1,3,2-dioxaborolan-2-yl,
PG steht für Tetrahydro-2H-pyran-2-yl, 2-(trimethylsilyl)ethoxy]methyl,
R², X¹, R⁶, R⁵, R^{4/401} für die gleichen Restedefinitionen wie für Formel [I-a] und [I-b] in den Ansprüchen 1 bis 8 definiert, stehen,
sowie agrochemisch wirksame Salze davon, wobei die Verbindung 1-({4-[1-(2,2-Difluorethyl)-3-(trimethylstannyl)-1H-pyrazol-4-yl]pyrimidin-2-yl} amino)propan-2-ol ausgenommen ist.

13. Verbindungen der Formel [**III**] 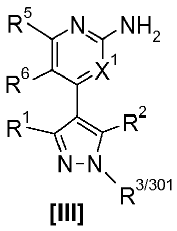 in welcher
R¹, R², X¹, R⁶, R⁵, R^{3/301} für die gleichen Restedefinitionen wie für Formel [I-a] und **[I-b]** in den Ansprüchen 1 bis 8 definiert, stehen,
sowie agrochemisch wirksame Salze davon, wobei die Verbindungen 4-[3-(4-fluorophenyl)-5-methyl-1H-pyrazol-4-yl]pyridin-2-amine 4-[3-(4-chlorophenyl)-5-methyl-1H-pyrazol-4-yl]pyridin-2-amine, 4-[3-(4-methoxyphenyl)-5-methyl-1H-pyrazol-4-yl]pyridin-2-amine, 4-[3-(4-fluorophenyl)-1H-pyrazol-4-yl]pyrimi-din-2-amine; 4-(5-methyl-3-phenyl-1H-pyrazol-4-yl)pyrimidin-2-amine und [4-(2-aminopy-rimidin-4-yl)-3-(3-chloro-5-hydroxyphenyl)-1H-pyrazol-1-yl]acetonitrile ausgenommen sind.

14. Verbindungen der Formel [V] 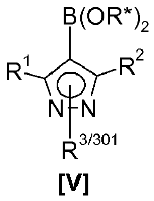 in welcher
B(OR*)₂ für -B(OiPr)₂ oder -B(OH)₂ steht,
R¹, R², R^{3/301} für die gleichen Restedefinitionen wie für Formel [I-a] und [I-b] in den Ansprüchen 1 bis 8 definiert, stehen,
sowie agrochemisch wirksame Salze davon, wobei die Verbindung 1-methyl-3-phenyl-4-(4,4,5,5-tetramethyl-1,3,2-dioxaborolan-2-yl)-1H-pyrazole ausgenommen ist.

15. Verbindungen der Formel [VI] 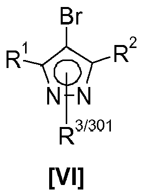 in welcher
R¹, R², R^{3/301} für die gleichen Restedefinitionen wie für Formel [I-a] und [I-b] in den Ansprüchen 1 bis 8 definiert, stehen,
sowie agrochemisch wirksame Salze davon, wobei Verbindungen ausgenommen sind bei denen R^{3/301} = H, CH₃ oder C(CH₃)₃ ist.
